# EUROPEAN PATENT SPECIFICATION

(11) **EP 0 904 262 B1**
(45) Date of publication and mention of the grant of the patent: **21.04.2004**
(21) Application number: 97903805.6
(22) Date of filing: 13.01.1997
(51) Int. Cl.: C07C 217/04, C07D 471/04, C07D 471/10, C07D 233/78, C07D 401/08, C07D 403/08, A61K 31/24, A61K 31/445, A61K 31/415, A61K 31/44, A61K 31/47

(54) **CONFORMATIONALLY RESTRICTED AROMATIC INHIBITORS OF MICROSOMAL TRIGLYCERIDE TRANSFER PROTEIN AND METHOD**
KONFORMATIONSEINGESCHRÄNKTE AROMATISCHE INHIBITOREN DES MICROSOMALEN TRIGLYCERIDTRANSFERPROTEINS UND VERFAHREN
COMPOSES AROMATIQUES A CONFORMATION RESTREINTE SERVANT D'INHIBITEURS DE LA PROTEINE DE TRANSFERT DE TRIGLYCERIDE MICROSOMIQUE ET PROCEDE ASSOCIE

(30) Priority: 16.01.1996 US 10346 P; 09.05.1996 US 17224 P; 05.11.1996 US 30370 P
(43) Date of publication of application: 31.03.1999
(73) Proprietor: BRISTOL-MYERS SQUIBB COMPANY, Princeton, NJ 08543-4000 (US)
(72) Inventor: BILLER, Scott, A., Hopewell, NJ 08525 (US); DICKSON, John, K., Eastampton, NJ 08060 (US); LAWRENCE, R., Michael, Yardley, PA 19067 (US); MAGNIN, David, R., Hamilton, NJ 08690 (US); POSS, Michael, A., Lawrenceville, NJ 08648 (US); ROBL, Jeffrey, A., Newtown, PA 18940 (US); SLUSARCHYK, William, A., Skillman, NJ 08558 (US); SULSKY, Richard, B., Franklin Park, NJ 08823 (US); TINO, Joseph, A., Lawrenceville, NJ 08648 (US)
(74) Representative: VOSSIUS & PARTNER
(86) International application number: PCT/US1997/000587
(87) International publication number: WO 1997/026240

(56) References cited:
- EP-A- 0 643 057
- WO-A-96/26205
- WO-A-96/40640
- AT-B- 368 130
- GB-A- 773 756
- GB-A- 1 147 832
- US-A- 2 838 509
- US-A- 3 843 657
- US-A- 4 197 313
- US-A- 4 277 495
- US-A- 4 282 170
- US-A- 4 864 028
- US-A- 5 173 489
- US-A- 5 272 269

## Description

### Field of the Invention

This invention relates to novel conformationally restricted aromatic compounds which inhibit microsomal triglyceride transfer protein, and to methods for decreasing serum lipids and treating atherosclerosis employing such compounds.

### Background of the Invention

The microsomal triglyceride transfer protein (MTP) catalyzes the transport of triglyceride (TG), cholesteryl ester (CE), and phosphatidylcholine (PC) between small unilamellar vesicles (SUV). Wetterau & Zilversmit, Chem. Phys. Lipids 38, 205-22 (1985). When transfer rates are expressed as the percent of the donor lipid transferred per time, MTP expresses a distinct preference for neutral lipid transport (TG and CE), relative to phospholipid transport. The protein from bovine liver has been isolated and characterized. Wetterau & Zilversmit, Chem. Phys. Lipids 38, 205-22 (1985). Polyacrylamide gel electrophoresis (PAGE) analysis of the purified protein suggests that the transfer protein is a complex of two subunits of apparent molecular weights 58,000 and 88,000, since a single band was present when purified MTP was electrophoresed under nondenaturing condition, while two bands of apparent molecular weights 58,000 and 88,000 were identified when electrophoresis was performed in the presence of sodium dodecyl sulfate (SDS). These two polypeptides are hereinafter referred to as 58 kDa and 88 kDa, respectively, or the 58 kDa and the 88 kDa component of MTP, respectively, or the low molecular weight subunit and the high molecular weight subunit of MTP, respectively.

Characterization of the 58,000 molecular weight component of bovine MTP indicates that it is the previously characterized multifunctional protein, protein disulfide isomerase (PDI). Wetterau et al., J. Biol. Chem. 265, 9800-7 (1990). The presence of PDI in the transfer protein is supported by evidence showing that (1) the amino terminal 25 amino acids of the bovine 58,000 kDa component of MTP is identical to that of bovine PDI, and (2) disulfide isomerase activity was expressed by bovine MTP following the dissociation of the 58 kDa - 88 kDa protein complex. In addition, antibodies raised against bovine PDI, a protein which by itself has no TG transfer activity, were able to immunoprecipitate bovine TG transfer activity from a solution containing purified bovine MTP.

PDI normally plays a role in the folding and assembly of newly synthesized disulfide bonded proteins within the lumen of the endoplasmic reticulum. Bulleid & Freedman, Nature 335, 649-51 (1988). It catalyzes the proper pairing of cysteine residues into disulfide bonds, thus catalyzing the proper folding of disulfide bonded proteins. In addition, PDI has been reported to be identical to the beta subunit of human prolyl 4-hydroxylase. Koivu et al., J. Biol. Chem. 262, 6447-9 (1987). The role of PDI in the bovine transfer protein is not clear. It does appear to be an essential component of the transfer protein as dissociation of PDI from the 88 kDa component of bovine MTP by either low concentrations of a denaturant (guanidine HCl), a chaotropic agent (sodium perchlorate), or a nondenaturing detergent (octyl glucoside) results in a loss of transfer activity. Wetterau et al., Biochemistry 30, 9728-35 (1991). Isolated bovine PDI has no apparent lipid transfer activity, suggesting that either the 88 kDa polypeptide is the transfer protein or that it confers transfer activity to the protein complex.

The tissue and subcellular distribution of MTP activity in rats has been investigated. Wetterau & Zilversmit, Biochem. Biophys. Acta 875, 610-7 (1986). Lipid transfer activity was found in liver and intestine. Little or no transfer activity was found in plasma, brain, heart, or kidney. Within the liver, MTP was a soluble protein located within the lumen of the microsomal fraction. Approximately equal concentrations were found in the smooth and rough microsomes.

Abetalipoproteinemia is an autosomal recessive disease characterized by a virtual absence of plasma lipoproteins which contain apolipoprotein B (apoB). Kane & Havel in The Metabolic Basis of Inherited Disease, Sixth edition, 1139-64 (1989). Plasma TG levels may be as low as a few mg/dL, and they fail to rise after fat ingestion. Plasma cholesterol levels are often only 20-45 mg/dL. These abnormalities are the result of a genetic defect in the assembly and/or secretion of very low density lipoproteins (VLDL) in the liver and chylomicrons in the intestine. The molecular basis for this defect has not been previously determined. In subjects examined, triglyceride, phospholipid, and cholesterol synthesis appear normal. At autopsy, subjects are free of atherosclerosis. Schaefer et al., Clin. Chem. 34, B9-12 (1988). A link between the apoB gene and abetalipoproteinemia has been excluded in several families. Talmud et al., J. Clin. Invest. 82, 1803-6 (1988) and Huang et al., Am. J. Hum. Genet. 46, 1141-8 (1990).

Subjects with abetalipoproteinemia are afflicted with numerous maladies. Kane & Havel, supra. Subjects have fat malabsorption and TG accumulation in their enterocytes and hepatocytes. Due to the absence of TG-rich plasma lipoproteins, there is a defect in the transport of fat-soluble vitamins such as vitamin E. This results in acanthocytosis of erythrocytes, spinocerebellar ataxia with degeneration of the fasciculus cuneatus and gracilis, peripheral neuropathy, degenerative pigmentary retinopathy, and ceroid myopathy. Treatment of abetalipoproteinemic subjects includes dietary restriction of fat intake and dietary supplementation with vitamins A, E and K.

In vitro, MTP catalyzes the transport of lipid molecules between phospholipid membranes. Presumably, it plays a similar role in vivo, and thus plays some role in lipid metabolism. The subcellular (lumen of the microsomal fraction) and tissue distribution (liver and intestine) of MTP have led to speculation that it plays a role in the assembly of plasma lipoproteins, as these are the sites of plasma lipoprotein assembly. Wetterau & Zilversmit, Biochem. Biophys. Acta 875, 610-7 (1986). The ability of MTP to catalyze the transport of TG between membranes is consistent with this hypothesis, and suggests that MTP may catalyze the transport of TG from its site of synthesis in the endoplasmic reticulum (ER) membrane to nascent lipoprotein particles within the lumen of the ER.

Olofsson and colleagues have studied lipoprotein assembly in HepG2 cells. Bostrom et al., J. Biol. Chem. 263, 4434-42 (1988). Their results suggest small precursor lipoproteins become larger with time. This would be consistent with the addition or transfer of lipid molecules to nascent lipoproteins as they are assembled. MTP may play a role in this process. In support of this hypothesis, Howell and Palade, J. Cell Biol. 92, 833-45 (1982), isolated nascent lipoproteins from the hepatic Golgi fraction of rat liver. There was a spectrum of sizes of particles present with varying lipid and protein compositions. Particles of high density lipoprotein (HDL) density, yet containing apoB, were found. Higgins and Hutson, J. Lipid Res. 25, 1295-1305 (1984), reported lipoproteins isolated from Gclgi were consistently larger than those from the endoplasmic reticulum, again suggesting the assembly of lipoproteins is a progressive event. However, there is no direct evidence in the prior art demonstrating that MTP plays a role in lipid metabolism or the assembly of plasma lipoprotein.

Recent reports (Science, Vol. 258, page 999, 1992; D. Sharp et al, Nature, Vol. 365, page 65, 1993) demonstrate that the defect causing abetalipoproteinemia is in the MTP gene, and as a result, the MTP protein. Individuals with abetalipoproteinemia have no MTP activity, as a result of mutations in the MTP gene, some of which have been characterized. These results indicate that MTP is required for the synthesis of apoB containing lipoproteins, such as VLDL, the precursor to LDL. It therefore follows that inhibitors of MTP would inhibit the synthesis of VLDL and LDL, thereby lowering VLDL levels, LDL levels, cholesterol levels, and triglyceride levels in animals and man.

Canadian Patent Application No. 2,091,102 published March 2, 1994 (corresponding to U.S. application Serial No. 117,362, filed September 3, 1993 (file DC21b)) which is incorporated herein by reference), reports MTP inhibitors which also block the production of apoB containing lipoproteins in a human hepatic cell line (HepG2 cells). This provides further support for the proposal that an MTP inhibitor would lower apoB containing lipoprotein and lipid levels in vivo. This Canadian patent application discloses a method for identifying the MTP inhibitors 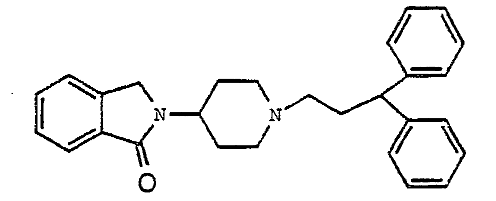 which has the name 2-[1-(3, 3-diphenylpropyl)-4-piperidinyl]-2, 3-dihydro-3-oxo-1H-isoindole hydrochloride and 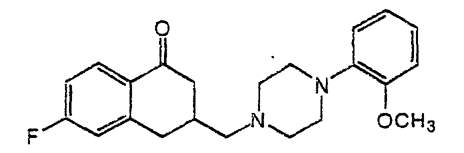 which has the name 1-[3-(6-fluoro-1-tetralanyl)-methyl]-4-O-methoxyphenyl piperazine.

EP 0643057A1 published March 15, 1995, discloses MTP inhibitors of the structure 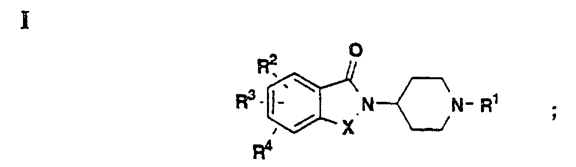 or 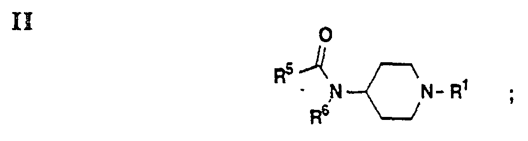 or 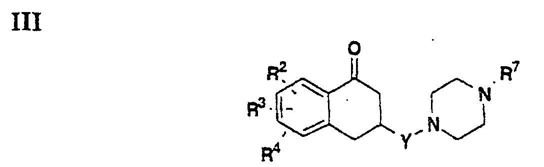 where X is: CHR⁸, 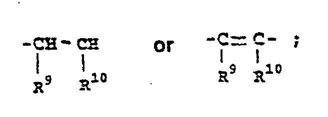 R⁸, R⁹ and R¹⁰ are independently hydrogen, alkyl, alkenyl, alkynyl, aryl, arylalkyl, heteroaryl, heteroarylalkyl, cycloalkyl, or cycloalkylalkyl;
Y is -(CH₂)ₘ- or 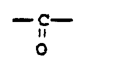 where m is 2 or 3;
R¹ is alkyl, alkenyl, alkynyl, aryl, heteroaryl, arylalkyl (wherein alkyl has at least 2 carbons), diarylalkyl, arylalkenyl, diarylalkenyl, arylalkynyl, diarylalkynyl, diarylalkylaryl, heteroarylalkyl (wherein alkyl has at least 2 carbons), cycloalkyl, or cycloalkylalkyl (wherein alkyl has at least 2 carbons); all of the aforementioned R¹ groups being optionally substituted through available carbon atoms with 1, 2, or 3 groups selected from halo, haloalkyl, alkyl, alkenyl, alkoxy, aryloxy, aryl, arylalkyl, alkylmercapto, arylmercapto, cycloalkyl, cycloalkylalkyl, heteroaryl, fluorenyl, heteroarylalkyl, hydroxy or oxo; or
R¹ is a group of the structure 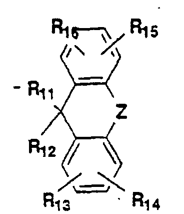 R¹¹ is a bond, alkylene, alkenylene or alkynylene of up to 6 carbon atoms, arylene (for example 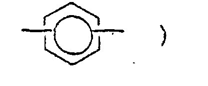 or mixed arylene-alkylene (for example 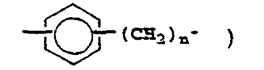 where n is 1 to 6;
R¹² is hydrogen, alkyl, alkenyl, aryl, heteroaryl, haloalkyl, arylalkyl, arylalkenyl, cycloalkyl, aryloxy, alkoxy, arylalkoxy, heteroarylalkyl or cycloalkylalkyl;
Z is a bond, O, S, N-alkyl, N-aryl, or alkylene or alkenylene of from 1 to 5 carbon atoms;
R¹³, R¹⁴, R¹⁵, and R¹⁶ are independently hydrogen, alkyl, halo, haloalkyl, aryl, cycloalkyl, cycloheteroalkyl, alkenyl, alkynyl, hydroxy, alkoxy, nitro, amino, thio, alkylsulfonyl, arylsulfonyl, alkylthio, arylthio, carboxy, aminocarbonyl, alkylcarbonyloxy, alkylcarbonylamino, arylalkyl, heteroaryl, heteroarylalkyl, or aryloxy;
or R¹ is 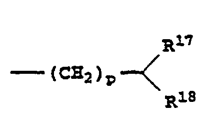 wherein p is 1 to 8 and R¹⁷ and R¹⁸ are each independently H, alkyl, alkenyl, aryl, arylalkyl, heteroaryl, heteroarylalkyl, cycloalkyl or cycloalkylalkyl, at least one of R¹⁷ and R¹⁸ being other than H;
or R¹ is 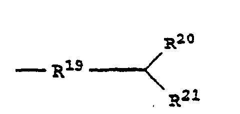 wherein R¹⁹ is aryl or heteroaryl;
R²⁰ is aryl or heteroaryl;
R²¹ is H, alkyl, aryl, alkylaryl, arylalkyl, aryloxy, arylalkoxy, heteroaryl, heteroarylalkyl, heteroarylalkoxy, cycloalkyl, cycloalkylalkyl or cycloalkylalkoxy;
R², R³, R⁴ are independently hydrogen, halo, alkyl, haloalkyl, alkenyl, alkoxy, aryloxy, aryl, arylalkyl, alkylmercapto, arylmercapto, cycloalkyl, cycloalkylalkyl, heteroaryl, heteroarylalkyl, hydroxy or haloalkyl;
R⁵ is alkyl of at least 2 carbons, alkenyl, alkynyl, aryl, heteroaryl, arylalkyl, heteroarylalkyl, cycloalkyl, cycloalkylalkyl, polycycloalkyl, polycycloalkylalkyl, cycloalkenyl, cycloalkenylalkyl, polycycloalkenyl, polycycloalkenylalkyl, heteroarylcarbonyl, all of the R⁵ and R⁶ substituents being optionally substituted through available carbon atoms with 1, 2, or 3 groups selected from hydrogen, halo, alkyl, haloalkyl, alkoxy, haloalkoxy, alkenyl, alkynyl, cycloalkyl, cycloalkylalkyl, cycloheteroalkyl, cycloheteroalky-lalkyl, aryl, heteroaryl, arylalkyl, arylcycloalkyl, arylalkynyl, aryloxy, aryloxyalkyl, arylalkoxy, arylazo, heteroaryloxo, heteroarylalkyl, heteroarylalkenyl, heteroaryloxy, hydroxy, nitro, cyano, amino, substituted amino (wherein the amino includes 1 or 2 substituents which are alkyl, or aryl or any of the other aryl compounds mentioned in the definitions), thiol, alkylthio, arylthio, heteroarylthio, arylthioalkyl, alkylcarbonyl, arylcarbonyl, arylaminocarbonyl, alkoxycarbonyl, aminocarbonyl, alkynylaminocarbonyl, alkylamino-carbonyl, alkenylaminocarbonyl, alkylcarbonyloxy, arylcarbonyloxy, alkylcarbonylamino, arylcarbonylamino, arylsulfinyl, arylsulfinylalkyl, arylsulfonyl, alkylsulfonyl, arylsulfonylamino; with the proviso that when R⁵ is CH₃, R⁶ is not H; and where R⁵ is phenyl, the phenyl preferably includes an ortho hydrophobic substituent such as alkyl, haloalkyl, aryl, aryloxy or arylalkyl;
R⁶ is hydrogen or C₁-C₄ alkyl or C₁-C₄ alkenyl;
R⁷ is alkyl, aryl or arylalkyl wherein alkyl or the alkyl portion is optionally substituted with oxo; and
including pharmaceutically acceptable salts and anions thereof.

In the formula I compounds, where X is CH₂ and R², R³ and R⁴ are each H, R¹ will be other than 3,3-diphenylpropyl.

In the formula III compounds, where one of R², R³ and R⁴ is 6-fluoro, and the others are H, R⁷ will be other than 4-O-methoxyphenyl.

U.S. Application Serial No. 472,067, filed June 6, 1995 (file DC21e) discloses compounds of the structure 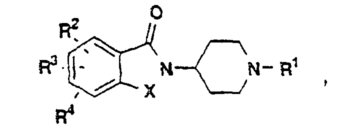 or 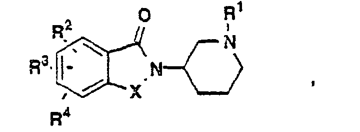 or 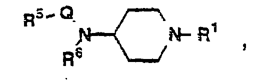 or 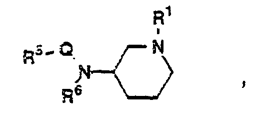 or 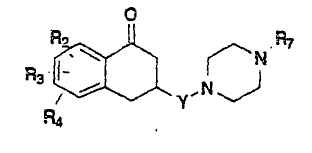 where Q is 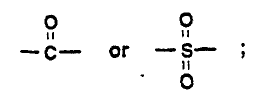 X is: CHR⁸, 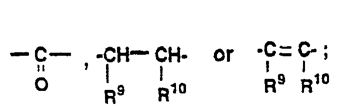 R⁸, R⁹ and R¹⁰ are independently hydrogen, alkyl, alkenyl, alkynyl, aryl, arylalkyl, heteroaryl, heteroarylalkyl, cycloalkyl, or cycloalkylalkyl;
Y is -(CH₂)ₘ- or 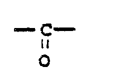 wherein m is 2 or 3;
R¹ is alkyl, alkenyl, alkynyl, aryl, heteroaryl, arylalkyl wherein alkyl has at least 2 carbons, diarylalkyl, arylalkenyl, diarylalkenyl, arylalkynyl, diarylalkynyl, diarylalkylaryl, heteroarylalkyl wherein alkyl has at least 2 carbons, cycloalkyl, or cycloalkylalkyl wherein alkyl has at least 2 carbons, all optionally substituted through available carbon atoms with 1, 2, 3 or 4 groups selected from halo, haloalkyl, alkyl, alkenyl, alkoxy, aryloxy, aryl, arylalkyl, alkylmercapto, arylmercapto, cycloalkyl, cycloalkylalkyl, heteroaryl, fluorenyl, heteroarylalkyl, hydroxy or oxo;
or R¹ is a fluorenyl-type group of the structure 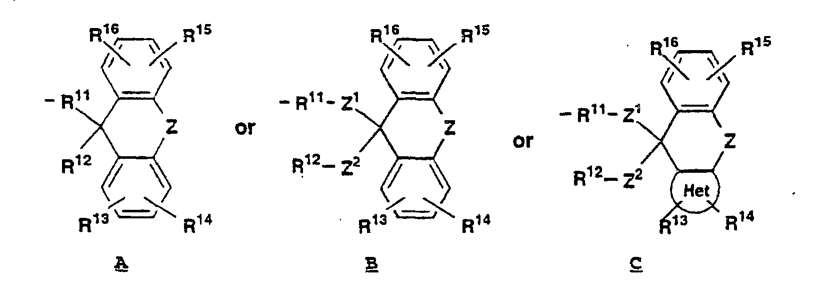 or 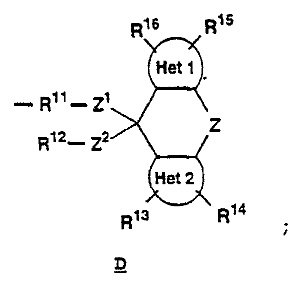 or
R¹ is an indenyl-type group of the structure 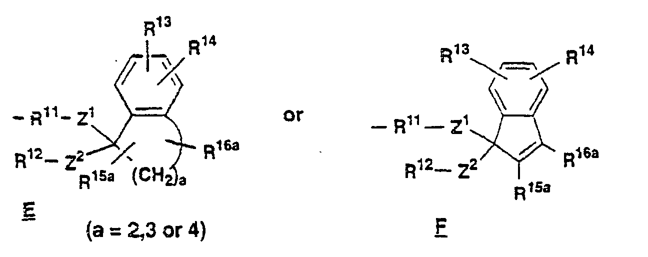 or 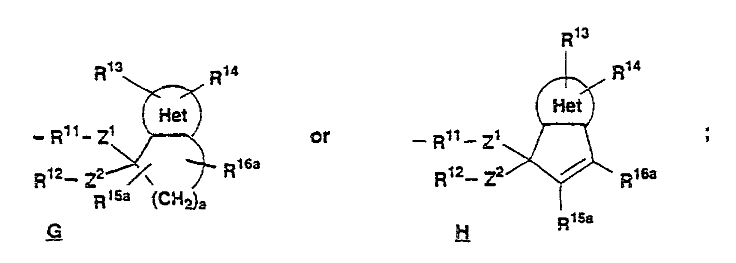 Z¹ and Z² are the same or different and are independently a bond, O, S, 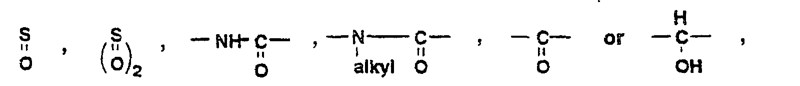 with the proviso that with respect to B, at least one of Z¹ and Z² will be other than a bond; R¹¹ is a bond, alkylene, alkenylene or alkynylene of up to 10 carbon atoms; arylene or mixed arylene-alkylene; R¹² is hydrogen, alkyl, alkenyl, aryl, haloalkyl, trihaloalkyl, trihaloalkylalkyl, heteroaryl, heteroarylalkyl, arylalkyl, arylalkenyl, cycloalkyl, aryloxy, alkoxy, arylalkoxy or cycloalkylalkyl, with the provisos that
(1) when R¹² is H, aryloxy, alkoxy or arylalkoxy, then Z² is 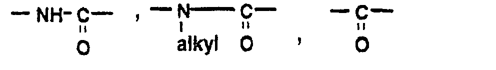 or a bond and
(2) when Z² is a bond, R¹² cannot be heteroaryl or heteroarylalkyl;
Z is bond, O, S, N-alkyl, N-aryl, or alkylene or alkenylene from 1 to 5 carbon atoms; R¹³, R¹⁴, R¹⁵, and R¹⁶ are independently hydrogen, alkyl, halo, haloalkyl, aryl, cycloalkyl, cycloheteroalkyl, alkenyl, alkynyl, hydroxy, alkoxy, nitro, amino, thio, alkylsulfonyl, arylsulfonyl, alkylthio, arylthio, aminocarbonyl, alkylcarbonyloxy, arylcarbonylamino, alkylcarbonylamino, arylalkyl, heteroaryl, heteroarylalkyl or aryloxy;
R^{15a} and R^{16a} are independently hydrogen, alkyl, halo, haloallcyl, aryl, cycloalkyl, cycloheteroalkyl, alkenyl, alkynyl, alkoxy, alkylsulfonyl, arylsulfonyl, alkylthio, arylthio, aminocarbonyl, alkylcarbonyloxy, arylcarbonylamino, alkylcarbonylamino, arylalkyl, heteroaryl, heteroarylalkyl, or aryloxy;
or R¹ is a group of the structure 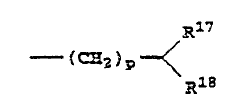 wherein p is 1 to 8 and R¹⁷ and R¹⁸ are each independently H, alkyl, alkenyl, aryl, arylalkyl, heteroaryl, heteroarylalkyl, cycloalkyl or cycloalkylalkyl at least one of R¹⁷ and R¹⁸ being other than H;
or R¹ is a group of the structure 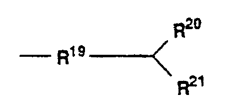 wherein R¹⁹ is aryl or heteroaryl;
R²⁰ is aryl or heteroaryl;
R²¹ is H, alkyl, aryl, alkylaryl, arylalkyl, aryloxy, arylalkoxy, heteroaryl, heteroarylalkyl, heteroarylalkoxy, cycloalkyl, cycloalkylalkyl or cycloalkylalkoxy;
R², R³, R⁴ are independently hydrogen, halo, alkyl, alkenyl, alkoxy, aryloxy, aryl, arylalkyl, alkylmercapto, arylmercapto, cycloalkyl, cycloalkylalkyl, heteroaryl, heteroarylalkyl, hydroxy or haloalkyl;
R⁵ is independently alkyl, alkenyl, alkynyl, aryl, alkoxy, aryloxy, arylalkoxy, heteroaryl, arylalkyl, heteroarylalkyl, cycloalkyl, cycloalkylalkyl, polycycloalkyl, polycycloalkylalkyl, cycloalkenyl, cycloheteroalkyl, heteroaryloxy, cycloalkenylalkyl, polycycloalkenyl, polycycloalkenylalkyl, heteroarylcarbonyl, amino, alkylamino, arylamino, heteroarylamino, cycloalkyloxy, cycloalkylamino, all optionally substituted through available carbon atoms with 1, 2, 3 or 4 groups selected from hydrogen, halo, alkyl, haloalkyl, alkoxy, haloalkoxy, alkenyl, alkynyl, cycloalkyl, cycloalkylalkyl, cycloheteroalkyl, cycloheteroalkylalkyl, aryl, heteroaryl, arylalkyl, arylcycloalkyl, arylalkenyl, arylalkynyl, aryloxy, aryloxyalkyl, arylalkoxy, arylazo, heteroaryloxo, heteroarylalkyl, heteroarylalkenyl, heteroaryloxy, hydroxy, nitro, cyano, amino, substituted amino, thiol, alkylthio, arylthio, heteroarylthio, arylthioalkyl, alkylcarbonyl, arylcarbonyl, arylaminocarbonyl, alkoxycarbonyl, aminocarbonyl, alkynylaminocarbonyl, alkylaminocarbonyl, alkenylaminocarbonyl, alkylcarbonyloxy, arylcarbonyloxy, alkylcarbonylamino, arylcarbonylamino, arylsulfinyl, arylsulfinylalkyl, arylsulfonyl, alkylsulfonyl, arylsulfonylamino, heteroarylcarbonylamino, heteroarylsulfinyl, heteroarylthio, heteroarylsulfonyl, alkylsulfinyl;
R⁶ is hydrogen or C₁-C₄ alkyl or C₁-C₄ alkenyl; all optionally substituted with 1, 2, 3 or 4 groups which may independently be any of the substituents listed in the definition of R⁵ set out above;
R⁷ is alkyl, aryl or arylalkyl wherein alkyl by itself or as part of arylalkyl is optionally substituted with oxo 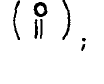 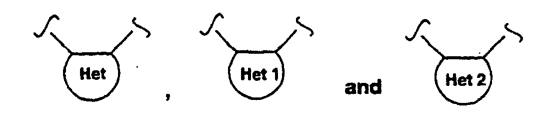 are the same or different and are independently selected from heteroaryl containing 5- or 6-ring members; and
N-oxides 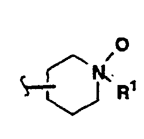 thereof; and pharmaceutically acceptable salts thereof; with the provisos that where in the first formula X is CH₂, and R², R³ and R⁴ are each H, then R¹ will be other than 3,3-diphenylpropyl, and in the fifth formula, where one of R², R³ and R⁴ is 6-fluoro, and the others are H, R⁷ will be other than 4-(2-methoxyphenyl).

EP 643 057 pertains to inhibitors of microsomal triglyceride transfer proteins. GB 773,756 and US 2,838,505 disclose 9-aminoalkyl-9-xanthene-carboxamides and derivatives thereof.

A method for producing novel 9-aminoalkyl-fluorenes and salts thereof is disclosed in AT368130. GB 1,147,832 discloses fluorene xanthene and thioxanthene derivatives. US 4,197,313 pertains to the treatment of cardiac arrhythmias with 9-aminoalkyl-9-(hydroxy, cyano or carbamoyl)-fluorenes.

9-Carbamoyl-9-(2-cyanoethyl)fluorenes as intermediates for the synthesis of 9-aminoalkyl-9-carbamoyl-fluorenes, which are antiarrhytmic agents are disclosed in US 4,282,170.

US 3,843,657 pertains to 9-dialkylaminoalkyl-N-substituted -fluorene-9-carboxamides.

### Summary of the Invention

In accordance with the present invention, novel compounds are provided which are inhibitors of MTP and have the structure 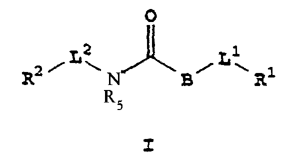 including pharmaceutically acceptable salts thereof, wherein q is 0, 1 or 2; where R⁵ is H or lower alkyl or R⁵ together with R² forms a carbocyclic or heterocyclic ring system containing 4 to 8 members in the ring.

B is a fluorenyl-type group of the structure: 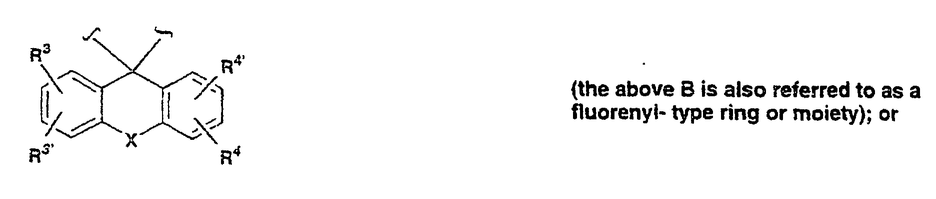 R¹ is H, alkyl, alkenyl, alkynyl, alkoxyl, (alkyl or aryl)₃Si (where each alkyl or aryl group is independent), cycloalkyl, cycloalkenyl, substituted alkylamino, substituted arylalkylamino, aryl, aryl-alkyl, arylamino, aryloxy, cycloheteroalkyl, heteroaryl, heteroarylamino, heteroaryloxy, arylsulfonylamino, heteroarylsulfonylamino, arylthio, arylsulfinyl, arylsulfonyl, alkylthio, alkylsulfinyl, alkylsulfonyl, heteroarylthio, heteroarylsulfinyl, heteroarylsulfonyl, -PO(R¹³) (R¹⁴), (where R¹³ and R¹⁴ are independently alkyl, aryl, alkoxy, aryloxy, heteroaryl, heteroarylalkyl, heteroaryloxy, heteroarylalkoxy, cycloheteroalkyl, cycloheteroalkyl-alkyl, cycloheteroalkoxy, or cycloheteroalkylalkoxy); R¹ can also be aminocarbonyl (where the amino may optionally be substituted with one or two aryl, alkyl or heteroaryl groups); cyano, 1,1-(alkoxyl or aryloxy)₂alkyl (where the two aryl or alkyl substituents can be independently defined, or linked to one another to form a ring, such as 1,3-dioxane or 1,3-dioxolane, connected to L¹ (or L² in the case of R²) at the 2-position); 1,3-dioxane or 1,3-dioxolane connected to L¹ (or L² in the case of R²) at the 4-position.

The R¹ group may have from one to four substituents, which can be any of the R³ groups or R¹ groups, and any of the preferred R¹ substituents set out below.

R¹ may be substituted with the following preferred substituents: alkylcarbonylamino, cycloalkylcarbonylamino, arylcarbonylamino, heteroarylcarbonylamino, alkoxycarbonylamino, aryloxycarbonyl-amino, heteroaryloxylcarbonylamino, uriedo (where the uriedo nitrogens may be substituted with alkyl, aryl or heteroaryl), heterocyclylcarbonylamino (where the heterocycle is connected to the carbonyl group via a nitrogen or carbon atom), alkylsulfonylamino, arylsulfonylamino, heteroarylsulfonylamino, 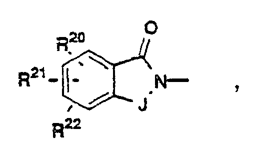 where J is: CHR²³, 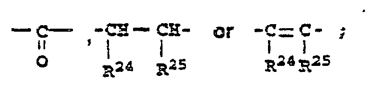 R²³, R²⁴ and R²⁵ are independently hydrogen, alkyl, alkanyl, alkynyl, aryl, arylalkyl, heteroaryl, heteroarylalkyl, cycloalkyl, or cycloalkylalkyl;
R²⁰, R²¹, R²² are independently hydrogen, halo, alkyl, alkenyl, alkoxy, aryloxy, aryl, arylalkyl, alkylmercapto, arylmercapto, cycloalkyl, cycloalkylalkyl, heteroaryl, heteroarylalkyl, hydroxy or haloalkyl; and these preferred substituents may either be directly attached to R¹, or attached via an alkylene chain at an open position.

R² is the same or different from R¹ and is independently any of the groups set out for R¹, H, polyhaloalkyl (such as CF₃CH₂, CF₃CF₂CH₂ or CF₃) or cycloheteroalkyl, and may be substituted with one to four of any of the groups defined for R³, or any of the substituents preferred for R¹.

L¹ is a linking group containing from 1 to 10 carbons in a linear chain (including alkylene, alkenylene or alkynylene), which may contain, within the linking chain any of the following: one or two alkenes, one or two alkynes, an oxygen, an amino group optionally substituted with alkyl or aryl, an oxo group; and may be substituted with one to five alkyl or halo groups (preferably F).

L² may be the same or different from L¹ and may independently be any of the L¹ groups set out above or a singe bond.

R³, R^{3'}, R⁴ and R^{4'} may be the same or different and are independently selected from H, halogen, CF₃, haloalkyl, hydroxy, alkoxy, alkyl, aryl, alkenyl, alkenyloxy, alkynyl, alkynyloxy, alkanoyl, nitro, amino, thiol, alkylthio, alkylsulfinyl, alkylsulfonyl, carboxy, alkoxycarbonyl, aminocarbonyl, alkylcarbonyloxy, alkylcarbonylamino, cycloheteroalkyl, cycloheteroalkylalkyl, cyano, Ar, Ar-alkyl, ArO, Ar-amino, Ar-thio, Ar-sulfinyl, Ar-sulfonyl, Ar-carbonyl, Ar-carbonyloxy or Ar-carbonylamino, wherein Ar is aryl or heteroaryl and Ar may optionally include 1, 2 or 3 additional rings fused to Ar;
X (in the fluorenyl type ring) is a bond, or is the following group:

(2) -o-

with the following provisos
(a) when R¹ is unsubstituted alkyl or unsubstituted arylalkyl, L¹ cannot contain amino;
(b) when R¹ is alkyl, L¹ cannot contain amino and oxo in adjacent positions (to form an amido group);
(d) when R¹ is cyano, L¹ must have more than 2 carbons;
(e) R¹L¹ must contain at least 3 carbons.

With respect to compounds I₇, where R¹ or R² is cycloheteroalkyl, R¹ or R² is exclusive of 1-piperidinyl, 1-pyrrolidinyl, 1-azetidinyl or 1-(2-oxo-pyrrolidinyl).

The pharmaceutically acceptable salts of the compounds of formula I, include alkali metal salts such as lithium, sodium or potassium, alkaline earth metal salts such as calcium or magnesium, as well as zinc or aluminum and other cations such as ammonium, choline, diethanolamine, ethylenediamine, t-butylamine, t-octylamine, dehydroabietylamine, as well as pharmaceutically acceptable anions such as chloride, bromide, iodide, tartrate, acetate, methanesulfonate, maleate, succinate, glutarate, and salts of naturally occurring amino acids such as arginine, lysine, alanine and the like, and prodrug esters thereof.

In addition, in accordance with the present invention, a use of a compound of formula I as defined hereinbefore for the preparation of a pharmaceutical composition for preventing, inhibiting or treating atherosclerosis, pancreatitis or obesity is provided, wherein the compound of formula I is to be administered in an amount which decreases the activity of microsomal triglyceride transfer protein.

Furthermore, in accordance with the present invention, a use of a compound of formula I as defined hereinbefore for the preparation of a pharmaceutical composition for lowering serum lipid levels, cholesterol and/or triglycerides, or inhibiting and/or treating hyperlipemia, hyperlipid-emia, hyperlipoproteinemia, hypercholesterolemia hypertriglyceridemia and/or hyperglycemia, non-insulin dependent diabetes (Type II diabetes), is provided wherein a compound of formula I is to be administered in an amount which decreases the activity of microsomal triglyceride transfer protein.

### Detailed Description of the Invention

The following definitions apply to the terms as used throughout this specification, unless otherwise limited in specific instances.

The term "MTP" refers to a polypeptide or protein complex that (1) if obtained from an organism (e. g., cows, humans, etc.), can be isolated from the microsomal fraction of homogenized tissue; and (2) stimulates the transport of triglycerides, cholesterol esters, or phospholipids from synthetic phospholipid vesicles, membranes or lipoproteins to synthetic vesicles, membranes, or lipoproteins and which is distinct from the cholesterol ester transfer protein [Drayna et al., Nature 327, 632-634 (1987)] which may have similar catalytic properties.

The phrase "stabilizing" atherosclerosis as used in the present application refers to slowing down the development of and/or inhibiting the formation of new atherosclerotic lesions.

The phrase "causing the regression of" atherosclerosis as used in the present application refers to reducing and/or eliminating atherosclerotic lesions.

Unless otherwise indicated, the term "lower alkyl", "alkyl" or "alk" as employed herein alone or as part of another group includes both straight and branched chain hydrocarbons, containing 1 to 40 carbons, preferably 1 to 20 carbons, more preferably 1 to 12 carbons, in the normal chain,such as methyl, ethyl, propyl, isopropyl, butyl, t-butyl, isobutyl, pentyl, hexyl, isohexyl, heptyl, 4,4-dimethylpentyl, octyl, 2,2,4-trimethylpentyl, nonyl, decyl, undecyl, dodecyl, the various branched chain isomers thereof, and the like as well as such groups including 1 to 4 substituents which may be any of the R³ groups, or the R¹ substituents set out herein.

Unless otherwise indicated, the term "cycloalkyl" as employed herein alone or as part of another group includes saturated or partially unsaturated (containing 1 or 2 double bonds) cyclic hydrocarbon groups containing 1 to 3 rings, including monocyclicalkyl, bicyclicalkyl and tricyclicalkyl, containing a total of 3 to 20 carbons forming the rings, preferably 4 to 12 carbons, forming the ring and which may be fused to 1 aromatic ring as described for aryl, which include cyclopropyl, cyclobutyl, cyclopentyl, cyclohexyl, cycloheptyl, cyclooctyl, cyclodecyl and cyclododecyl, cyclohexenyl, 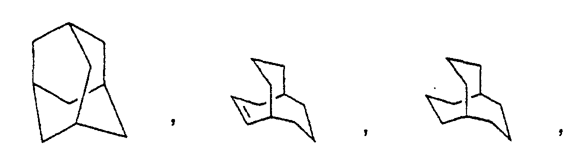 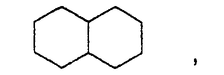 any of which groups may be optionally substituted with 1 to 4 substituents which may be any of the R³ groups, or the R¹ substituents set out herein.

The term "cycloalkenyl" as employed herein alone or as part of another group refers to cyclic hydrocarbons containing 5 to 20 carbons, preferably 6 to 12 carbons and 1 or 2 double bonds. Exemplary cycloalkenyl groups include cyclopentenyl, cyclohexenyl, cycloheptenyl, cyclooctenyl, cyclohexadienyl, and cycloheptadienyl, which may be optionally substituted as defined for cycloalkyl.

The term "polycycloalkyl" as employed herein alone or as part of another group refers to a bridged multicyclic group containing 5 to 20 carbons and containing 0 to 3 bridges, preferably 6 to 12 carbons and 1 or 2 bridges. Exemplary polycycloalkyl groups include [3.3.0]-bicyclooctanyl, adamantanyl, [2.2.1]-bicycloheptanyl, [2.2.2]-bicyclooctanyl and the like and may be optionally substituted as defined for cycloalkyl.

The term "polycycloalkenyl" as employed herein alone or as part of another group refers to a bridged multicyclic group containing 5 to 20 carbons and containing 0 to 3 bridges and containing 1 or 2 double bonds, preferably 6 to 12 carbons and 1 or 2 bridges. Exemplary polycycloalkyl groups include [3.3.0]-bicyclooctenyl, [2.2.1]-bicycloheptenyl, [2.2.2]-bicyclooctenyl and the like and may be optionally substituted as defined for cycloalkyl.

The term "aryl" as employed herein alone or as part of another group refers to monocyclic and bicyclic aromatic groups containing 6 to 10 carbons in the ring portion (such as phenyl or naphthyl) and may optionally include one to three additional rings fused to Ar (such as aryl, cycloalkyl, heteroaryl or cycloheteroalkyl rings) and may be optionally substituted through available carbon atoms with 1, 2, or 3 groups selected from hydrogen, halo, haloalkyl, alkyl, haloalkyl, alkoxy, haloal-koxy, alkenyl, trifluoromethyl, trifluoromethoxy, alkynyl, cyclo-alkylalkyl, cycloheteroalkyl, cycloheteroalkylalkyl, aryl, heteroaryl, arylalkyl, aryloxy, aryloxyalkyl, arylalkoxy, arylthio, arylazo, heteroarylalkyl, heteroarylalkenyl, heteroarylheteroaryl, heteroaryloxy, hydroxy, nitro, cyano, amino, substituted amino wherein the amino includes 1 or 2 substituents (which are alkyl, aryl or any of the other aryl compounds mentioned in the definitions), thiol, alkylthio, arylthio, hetero-arylthio, arylthioalkyl, alkoxyarylthio, alkylcarbonyl, arylcarbonyl, alkyl-aminocarbonyl, arylaminocarbonyl, alkoxycarbonyl, aminocarbonyl, alkylcarbonyloxy, arylcarbonyloxy, alkylcarbonylamino, arylcarbonylamino, arylsulfinyl, arylsulfinylalkyl, arylsulfonylamino or arylsulfon-aminocarbonyl or any of the R³ groups, or the R¹ substituents set out herein.

The term "aralkyl", "aryl-alkyl" or "aryllower alkyl" as used herein alone or as part of another group refers to alkyl groups as discussed above having an aryl substituent, such as benzyl or phenethyl, or naphthylpropyl, or an aryl as defined above.

The term "lower alkoxy", "alkoxy", "aryloxy" or "aralkoxy" as employed herein alone or as part of another group includes any of the above alkyl, aralkyl or aryl groups linked to an oxygen atom.

The term "amino" as employed herein alone or as part of another group may optionally be substituted with one or two substituents such as alkyl, aryl, arylalkyl, heteroaryl, heteroarylalkyl, cycloheteroalkyl, cycloheteroalkylalkyl and/or cycloalkyl.

The term "lower alkylthio", alkylthio", "arylthio" or "aralkylthio" as employed herein alone or as part of another group includes any of the above alkyl, aralkyl or aryl groups linked to a sulfur atom.

The term "lower alkylamino", "alkylamino", "arylamino", or "arylalkylamino" as employed herein alone or as part of another group includes any of the above alkyl, aryl or arylalkyl groups linked to a nitrogen atom.

The term "acyl" as employed herein by itself or part of another group, as defined herein, refers to an organic radical linked to a carbonyl 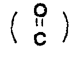 group; examples of acyl groups include alkanoyl, alkenoyl, aroyl, aralkanoyl, heteroaroyl, cycloal-kanoyl.

The term "alkanoyl" as used herein alone or as part of another group refers to alkyl linked to a carbonyl group.

Unless otherwise indicated, the term "lower alkenyl" or "alkenyl" as used herein by itself or as part of another group refers to straight or branched chain radicals of 2 to 20 carbons, preferably 3 to 12 carbons, and more preferably 2 to 8 carbons in the normal chain, which include one to six double bonds in the normal chain, such as vinyl, 2-propenyl, 3-butenyl, 2-butenyl, 4-pentenyl, 3-pentenyl, 2-hexenyl, 3-hexenyl, 2-heptenyl, 3-heptenyl, 4-heptenyl, 3-octenyl, 3-nonenyl, 4-decenyl, 3-undecenyl, 4-dodecenyl, 4,8,12-tetradecatrienyl, and which may be optionally substituted with 1 to 4 substituents, namely, halogen, haloalkyl, alkyl, alkoxy, alkenyl, alkynyl, aryl, arylalkyl, cycloalkyl, amino, hydroxy, heteroaryl, cyclohetero-alkyl, alkanoylamino, alkylamido, arylcarbonylamino, nitro, cyano, thiol, alkylthio or any of the R³ groups, or the R¹ substituents set out herein.

Unless otherwise indicated, the term "lower alkynyl" or "alkynyl" as used herein by itself or as part of another group refers to straight or branched chain radicals of 2 to 20 carbons, preferably 2 to 12 carbons and more preferably 2 to 8 carbons in the normal chain, which include one triple bond in the normal chain, such as 2-propynyl, 3-butynyl, 2-butynyl, 4-pentynyl, 3-pentynyl, 2-hexynyl, 3-hexynyl, 2-heptynyl, 3-heptynyl, 4-heptynyl, 3-octynyl, 3-nonynyl, 4-decynyl,3-undecynyl, 4-dodecynyl, and which may be optionally substituted with 1 to 4 substituents, namely, halogen, haloalkyl, alkyl, alkoxy, alkenyl, alkynyl, aryl, arylalkyl, cycloalkyl, amino, heteroaryl, cycloheteroalkyl, hydroxy, alkanoylamino, alkylamido, arylcarbonylamino, nitro, cyano, thiol, and/or alkylthio, or any of the R³ groups, or the R¹ substituents set out herein.

The term "alkylene" as employed herein alone or as part of another group refers to alkyl groups as defined above having single bonds for attachment to other groups at two different carbon atoms and may optionally be substituted as defined above for "alkyl".

Ther terms "alkenylene" and "alkynylene" as employed herein alone or as part of another group refer to alkenyl groups as defined above and alkynyl groups as defined above, respectively, having single bonds for attachment at two different carbon atoms.

Suitable alkylene, alkenylene or alkynylene groups or (CH₂)ₘ, (CH₂)ₙ or (CH₂)ₚ (which may include alkylene, alkenylene or alkynylene groups) as defined herein, may optionally include 1, 2, or 3 substituents which include any of the R³ groups, or the R¹ substituents set out herein.

Examples of alkylene, alkenylene and alkynylene include

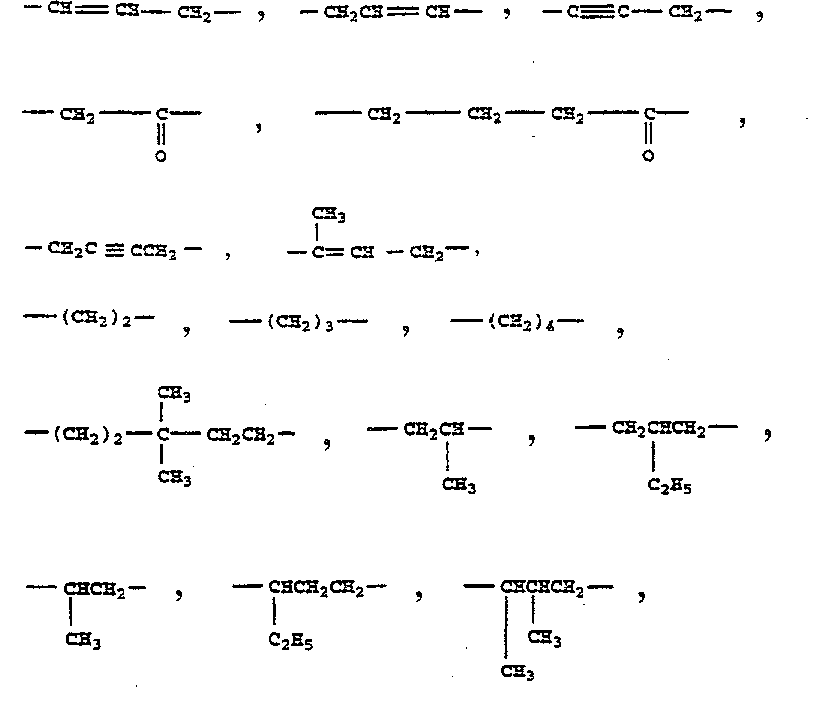

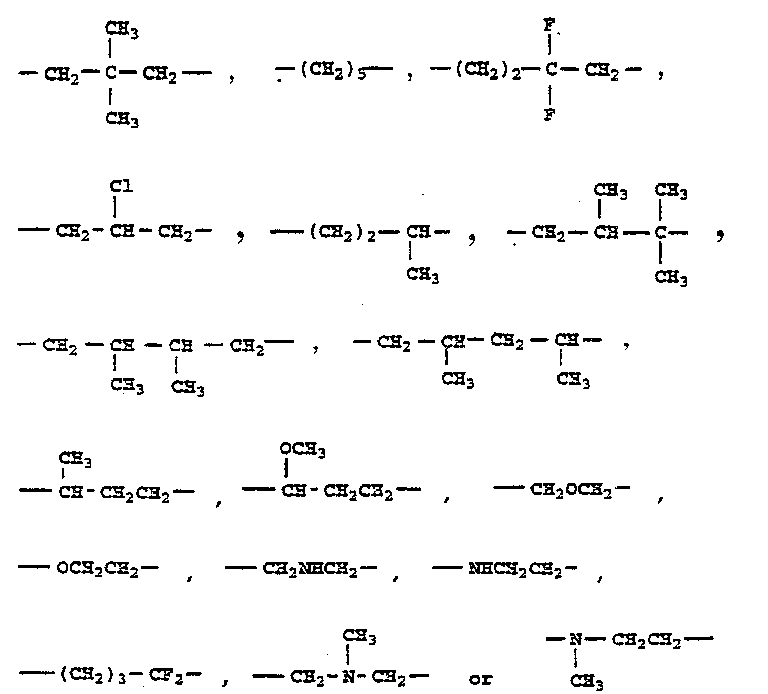

The term "halogen" or "halo" as used herein alone or as part of another group refers to chlorine, bromine, fluorine, and iodine as well as CF₃, with chlorine or fluorine being preferred.

The term "metal ion" refers to alkali metal ions such as sodium, potassium or lithium and alkaline earth metal ions such as magnesium and calcium, as well as zinc and aluminum.

The term "cycloheteroalkyl" as used herein alone or as part of another group refers to a 5-, 6- or 7-membered saturated or partially unsaturated ring which includes 1 to 2 hetero atoms such as nitrogen, oxygen and/or sulfur, linked through a carbon atom or a heteroatom, where possible, optionally via the linker (CH₂)ₚ (which is defined above), such as 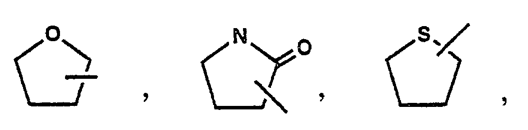 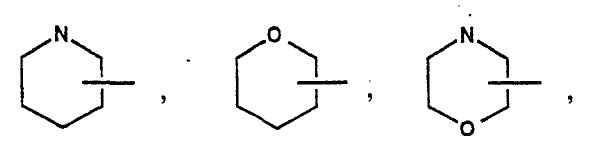 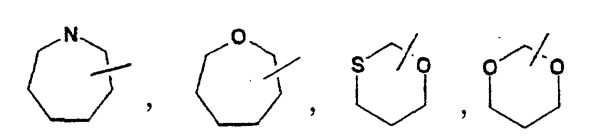 and the like. The above groups may include 1 to 4 substituents such as alkyl, halo, oxo and/or any of of the R³ groups, or the R¹ substituents set out herein. In addition, any of the above rings can be fused to a cycloalkyl, aryl, heteroaryl or cycloheteroalkyl ring.

The term "heteroaryl" as used herein alone or as part of another group refers to a 5- or 6-membered aromatic ring which includes 1, 2, 3 or 4 hetero atoms such as nitrogen, oxygen or sulfur,and such rings fused to an aryl, cycloalkyl, heteroaryl or cycloheteroalkyl ring (e.g. benzothiophenyl, indolyl), and includes possible N-oxides, such as

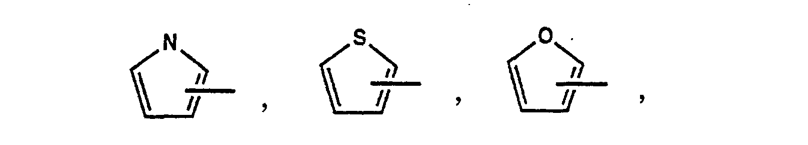

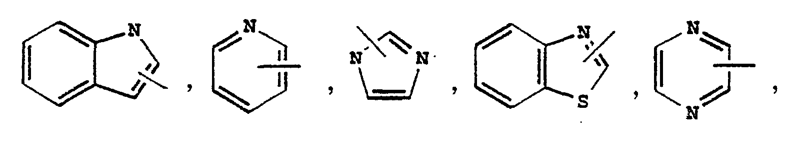

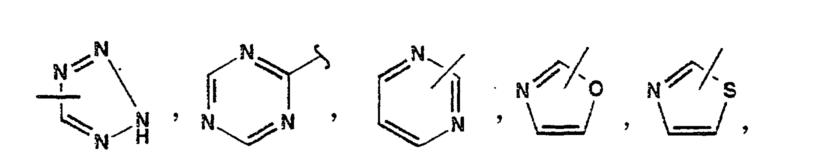

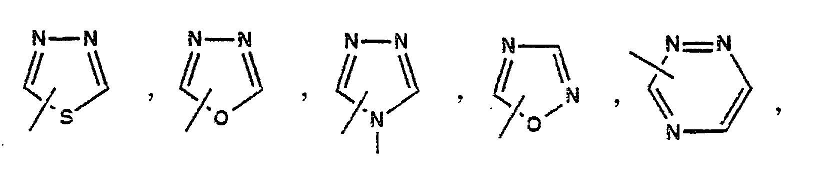

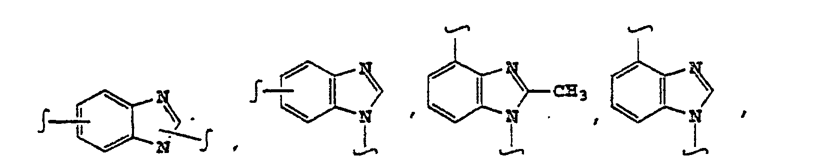

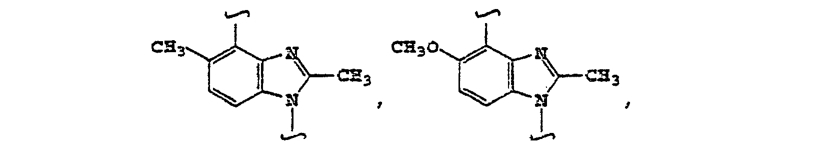

Ar may be either aryl or heteroaryl as defined above. 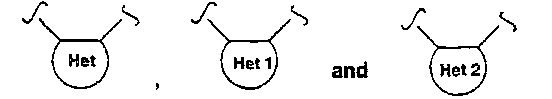 are the same or different, as defined hereinbefore, and are attached to the central ring of the indenyl or fluorenyl type group at adjacent positions (that is, ortho or 1,2-positions). Examples of such groups include 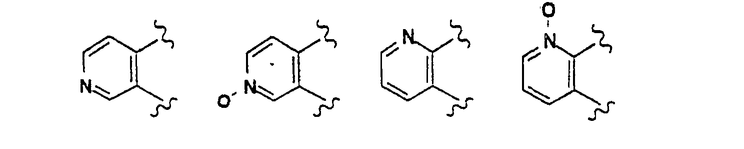 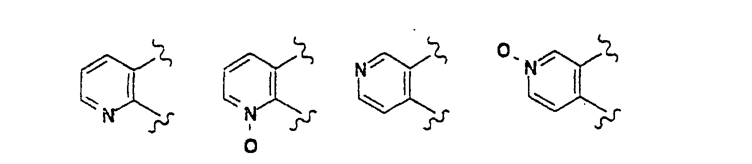 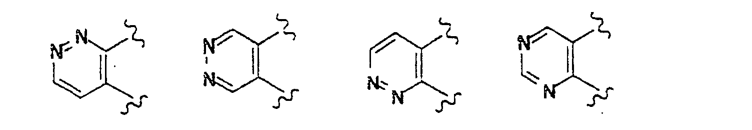 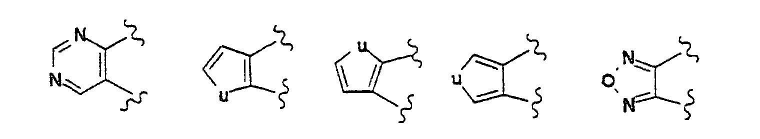 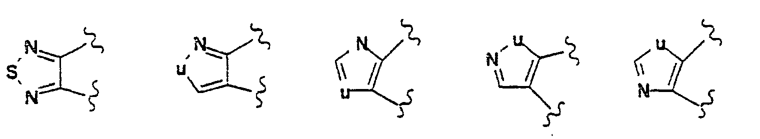 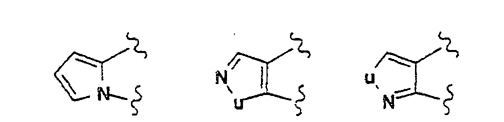 wherein u is selected from O, S, and NR^{7a};
R^{7a} is H, lower alkyl, aryl, -C(O)R^{7b}, -C(O)OR^{7b;}
R^{7b} is alkyl or aryl.

The heteroaryl groups including the above groups may optionally include 1 to 4 substituents such as any of the R³ groups, or the R¹ substituents set out herein. In addition, any of the above rings can be fused to a cycloalkyl, aryl, heteroaryl or cycloheteroalkyl ring.

The term cycloheteroalkylalkyl" as used herein alone or as part of another group refers to cycloheteroalkyl groups as defined above linked through a C atom or heteroatom to a (CH₂)ₚ chain.

The term "heteroarylalkyl" or "heteroarylalkenyl" as used herein alone or as part of another group refers to a heteroaryl group as defined above linked through a C atom or heteroatom to a -(CH₂)ₚ-chain, alkylene or alkenylene as defined above.

The term "polyhaloalkyl" as used herein refers to an "alkyl" group as defined above which includes from 2 to 9, preferably from 2 to 5, halo substituents, such as F or Cl, preferably F, such as CF₃CH₂, CF₃ or CF₃CF₂CH₂.

Preferred are compounds of formula I wherein A is NH,
B is 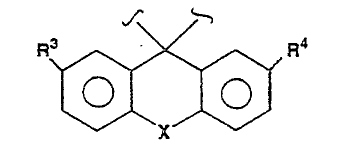 X is a bond, oxygen or sulfur; R³ and R⁴ are independently H or F.

Preferred R¹ groups are aryl, preferably phenyl, heteroaryl, preferably imidazoyl, benzimidazolyl, indolyl, or pyridyl (preferably substituted with one of the preferred R¹ substituents: arylcarbonylamino, heteroarylcarbonyl-amino, cycloalkylcarbonylamino, alkoxycarbonylamino, alkylsulfonylamino, arylsulfonylamino, heteroaryl-sulfonylamino), PO(OAlkyl)₂. heteroarylthio, benzthiazole-2-thio, imidazole-2-thio, alkyl, or alkenyl, cycloalkyl such as cyclohexyl, or 1,3-dioxan-2-yl.

Preferred R² groups are alkyl, polyfluoroalkyl (such as 1,1,1-trifluoroethyl), alkenyl, aryl or heteroaryl (preferably substituted with one of the preferred R¹ substituents above), or PO(OAlkyl)₂.

If R² is alkyl, 1,1,1-trifluoroethyl, or alkenyl, it is preferred that R¹ is other than alkyl or alkenyl.

It is preferred that L¹ contains 1 to 5 atoms in the linear chain and L² is a bond or lower alkylene.

Preferred embodiments of formula IA and formula IB compounds of the invention include those where B, L¹, L², R¹ and R² are as set out with respect to the preferred embodiments of the formula I compounds, q is 0 or 2 and R^{x} is H.

Also preferred are compounds of the structure 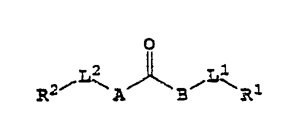 where B is 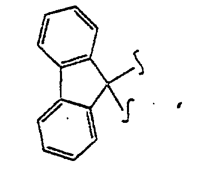 A is NH,
L² is a bond,
R² is CF₃CH_{2,},
L¹ is -CH₂CH₂CH₂- or -CH₂CH₂CH₂CH₂-, and
R¹ is heteroaryl which is a 5-membered aromatic ring which includes 2 nitrogens, which ring is fused to an aryl ring and is substituted on the aryl moiety. Examples of preferred R¹ groups include substituted benzimidazole groups including

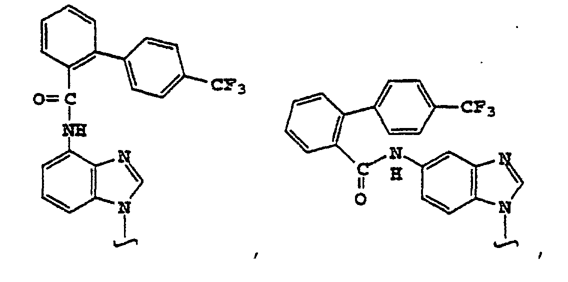

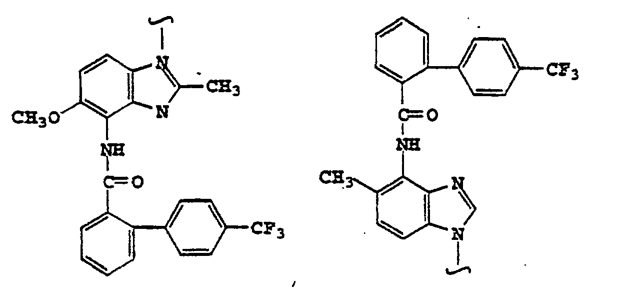

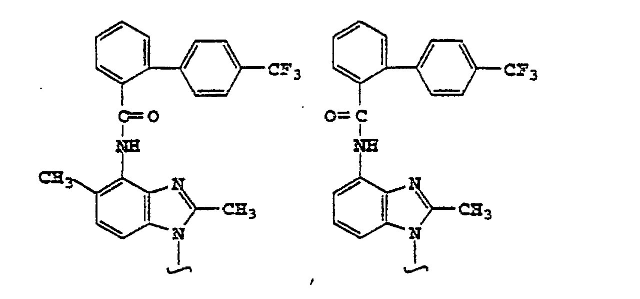

The compounds of formula I, may be prepared by the exemplary processes described in the following reaction schemes. Exemplary reagents and procedures for these reactions appear hereinafter and in the working Examples.

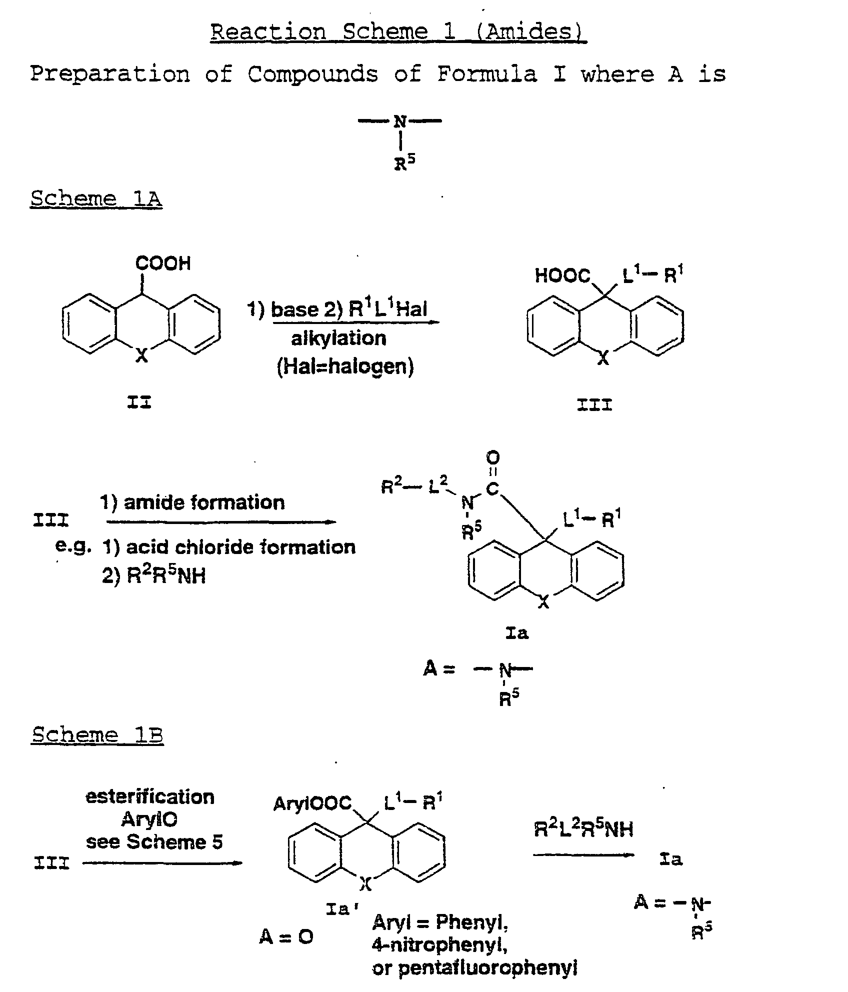

It will be appreciated that in the above reactions and the reactions to follow, unless otherwise indicated, the moiety "B" in the starting materials, intermediates and final products is set out as 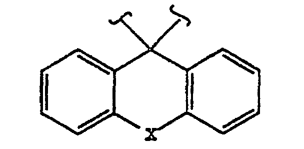 for purposes of illustration only.

It will be appreciated that the "B" moiety in the starting materials, intermediates and final products in all reactions set forth herein, unless indicated to the contrary is a fluorenyl-type group

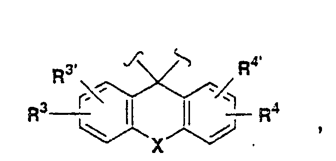

The above B moiety is collect-ively referred to as "fluorenyl-type" moieties. The use of the first fluorenyl-type group (as set out in the previous paragraph) in the Reaction Schemes is for purposes of illustration only; groups as set out above may be employed in any of the Reaction Schemes set out herein in place of

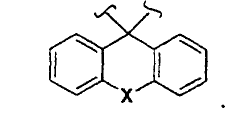

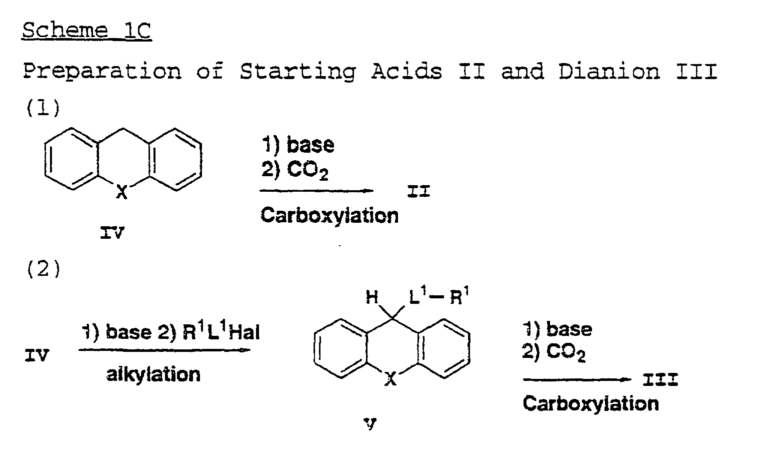

As indicated above, the starting Compound IV may also be 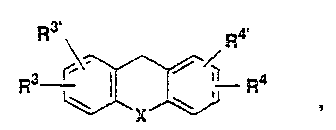 as well as

The above are collectively referred to "fluorenyl-type compounds".

As seen in Scheme 1A, in accordance with another aspect of the present invention, the solution of acid II in an inert organic solvent, such as tetrahydrofuran, dioxane or diethyl ether, at a reduced temperature of within the range of from about -40°C to about room temperature, is treated with base such as potassium hydroxide, potassium tert-butoxide, lithium or potassium bis(trimethylsilylamide), or n-butyllithium in an inert organic solvent such as hexane, tetrahydrofuran or diethyl ether, while maintaining temperature of the reaction mixture below from about -40°C to about room temperature. The reaction mixture is treated with R¹ halide such as an alkylhalide, for example, 3-phenylpropylbromide to form the alkylated product III.

The above dianion formation reaction is carried out employing a molar ratio of R¹halide:acid II of within the range from about 10:1 to about 0.5:1, preferably from about 2:1 to about 0.8:1.

Alternatively, the compound III may be prepared as shown in Scheme 1C(2) wherein fluorenyl-type compound IV is treated with base, such as described above, for example n-butyllithium, and then reacted with R¹halide, such as alkylhalide, as described above, to give compound V. Treatment of V with base, such as described hereinbefore such as n-butyl-lithium, followed by treatment of the reaction mixture with CO₂ (carboxylation) gives III.

As seen in Scheme 1C(1), acid II may be formed by treating fluorenyl-type compound IV with base (as described above with respect to Scheme 1C(2), followed by treatment with CO₂ (carboxylation), to form II.

The amide Ia of the invention is formed by treating III with thionyl chloride or oxalyl chloride in an inert organic solvent such as dichloromethane (optionally in the presence of dimethylformamide (DMF)) to form the acid chloride IIIA 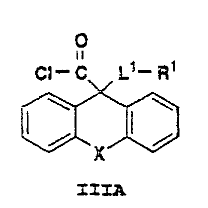 Acid chloride IIIA, without separation from the reaction mixture, is treated with amine (R²L²)R⁵NH at a reduced temperature within the range from about -40°C to about room temperature, to form the amide Ia.

In carrying out the above reaction to form amide Ia, the amine will be employed in a molar ratio to acid chloride IIIA within the range from about 4:1, to about 1:1, optionally in the presence of a tertiary amine base or other acid scavenger.

Alternatively, as seen in Scheme 1B, amide I may be prepared by esterifying III (as shown in Scheme 6) by reacting III with a phenol such as phenol, 4-nitrophenol, or pentafluorophenol and DCC (dicyclo-hexylcarbodiimide) or EDCI (1-(3-dimethylamino-propyl)-3-ethylcarbodiimide), optionally in the presence of HOBT (1-hydroxybenzotriazole) through the intermediary of an aryl ester such as phenyl, p-NO₂-phenyl or pentafluorophenyl, followed by treatment with a primary or secondary amine to give Ia.

In carrying out the above reaction, the amine will be employed in a molar ratio to ester within the range form about 10:1, to about 1:1.

Alternative formation of amide Ia from acid III and R²R⁵NH can be carried out via standard literature procedures.

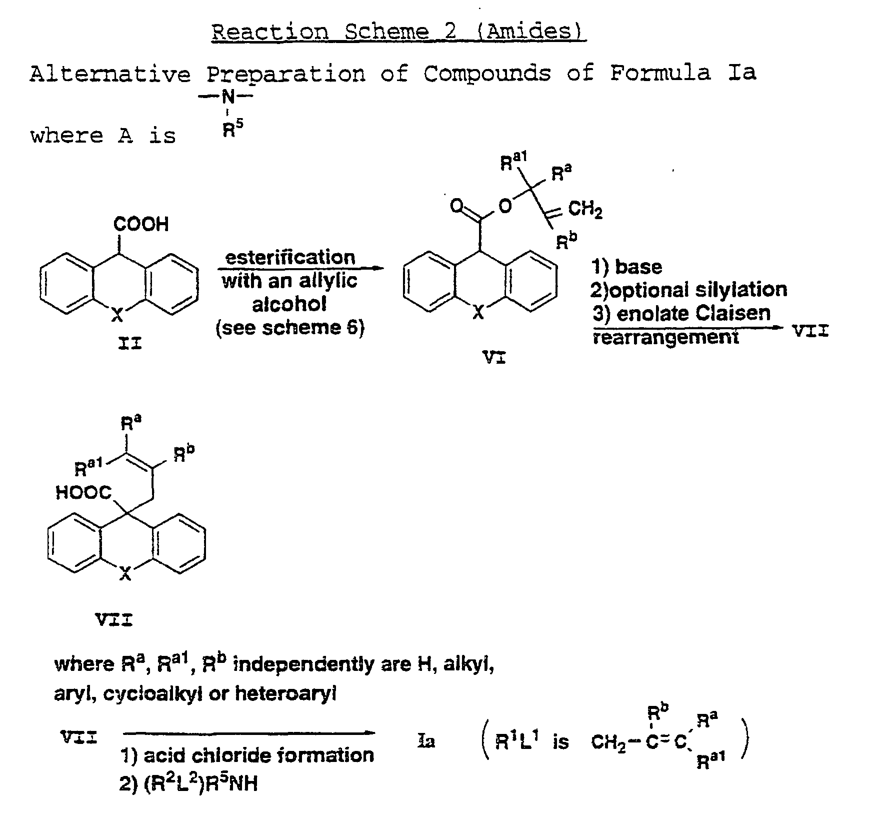

As seen in Reaction Scheme 2, amides of the invention of structure I can also be prepared by esterifying acid II with an allylic alcohol (as described in Scheme 5), to form ester VI which is treated with base, such as lithium diisopropyl amide or potassium bis(trimethylsilylamide) (optionally in the presence of a triorganosilylchloride, such as trimethylsilylchloride), to give the enolate-Claisen rearrangement acid product VII. Acid VII is then converted to amide Ia of the invention employing conditions as described with respect to Scheme 1.

In carrying out the above reaction, the base treatment and enolate-Claisen rearrangement were performed at a temperature within the range of from about -20 to about 100°C, preferably from about 25° to about 80°C, to form Ia where R¹L¹ is as defined above in Scheme 2.

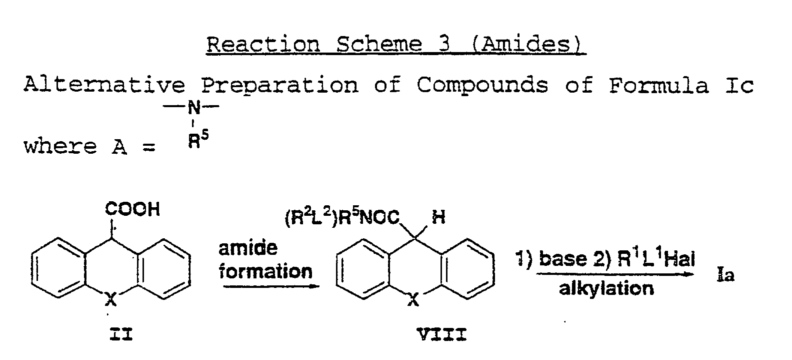

As seen in Reaction Scheme 3, compounds of structure I of the invention can be prepared optionally through amide formation (as described in Reaction Scheme 1 or via other known coupling procedures) from acid II to give compounds of formula VIII. Treatment of VIII with base, such as lithium diisopropylamide or n-BuLi, or potassium bis(trimethylsilyl)amide, followed by quenching the anion with an alkyl halide gives compounds of the formula I. In the specific case where R⁵ is H, a dianion can be prepared requiring ≥ two equivalents of base; the dianion can be trapped with an alkyl halide to give I.

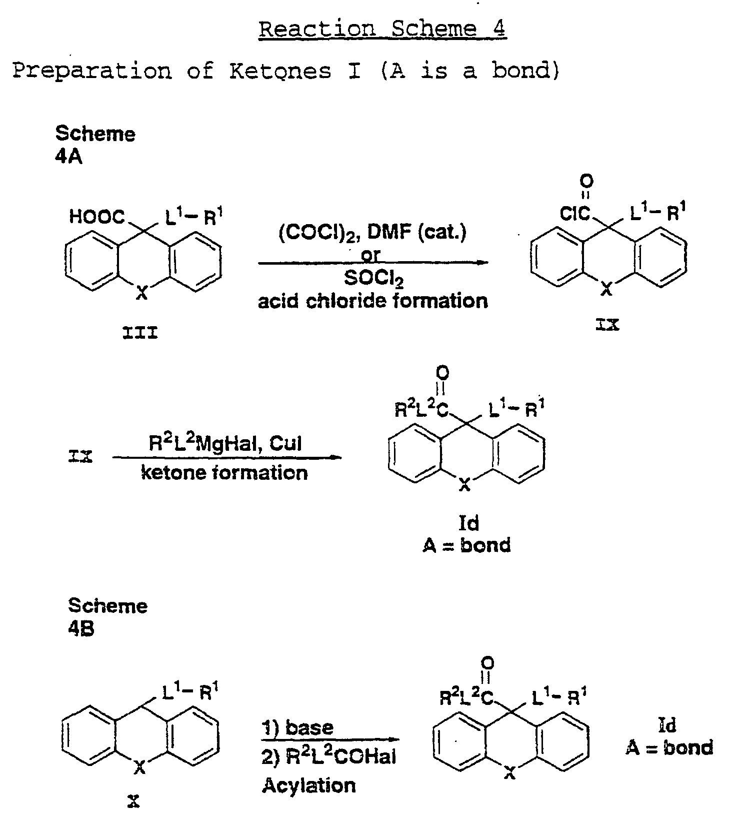

Compounds of the formula I of the invention wherein A = bond can be prepared as shown in Reaction Schemes 4A and 4B.

As seen in Scheme 4A, acid chloride formation under standard methods gives compound IX, which can be reacted with Grignard reagents and copper (I) iodide to give the compound of the invention I.

As seen in Scheme 4B, optionally, ketones can be formed by treatment of X with base, followed by acylation with an acid halide (R²L²COHal), preferably chloride or fluoride, to give compounds of the invention I.

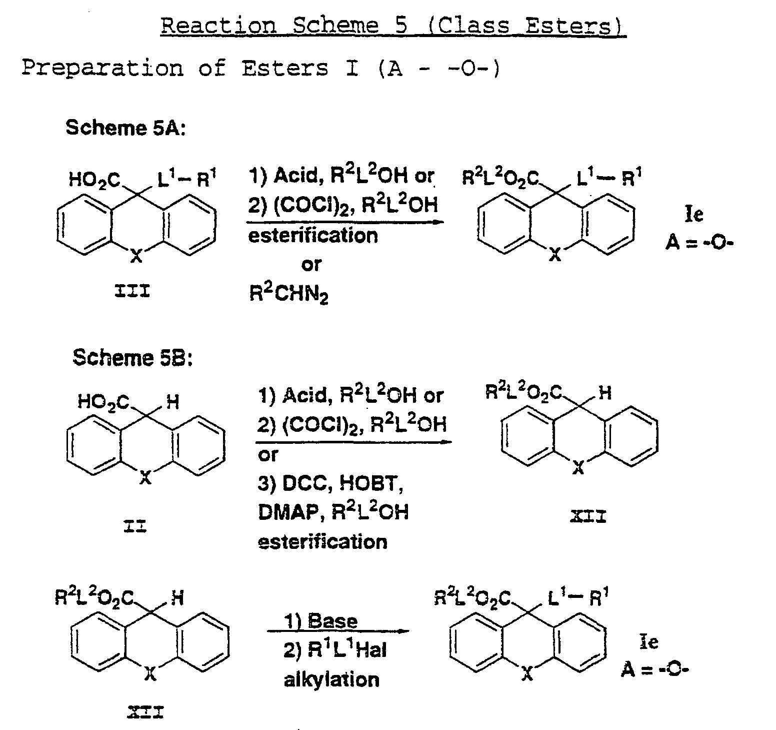

As seen in Reaction Scheme 5A, compounds of formula I of the invention wherein A = oxygen can be prepared by an acid catalyzed esterification of acid III employing an acid such as H₂SO₄ or p-toluene-sulfonic acid in the presence of an alcohol such as allyl alcohol, ethanol or methanol. Alternatively, activation of the acid III to the acid chloride (with oxaly chloride or thionyl chloride) followed by treatment with an alcohol optionally in the presence of a tertiary amine base or other acid scavenger, gives compounds of formula I.

Various additional methods of activation include mixed anhydride formation ((CF₃COO)₂ or i-BuOCOCl) or formation of the acylimidazole (carbonyldiimidazole) or with DCC and HOBT in the presence of DMAP (4-dimethylaminopyridine). These activated intermediates readily form esters upon treatment with alcohols.

Scheme 5B involves esterification of acids II to compound XII which is subjected to alkylation to give Ie.

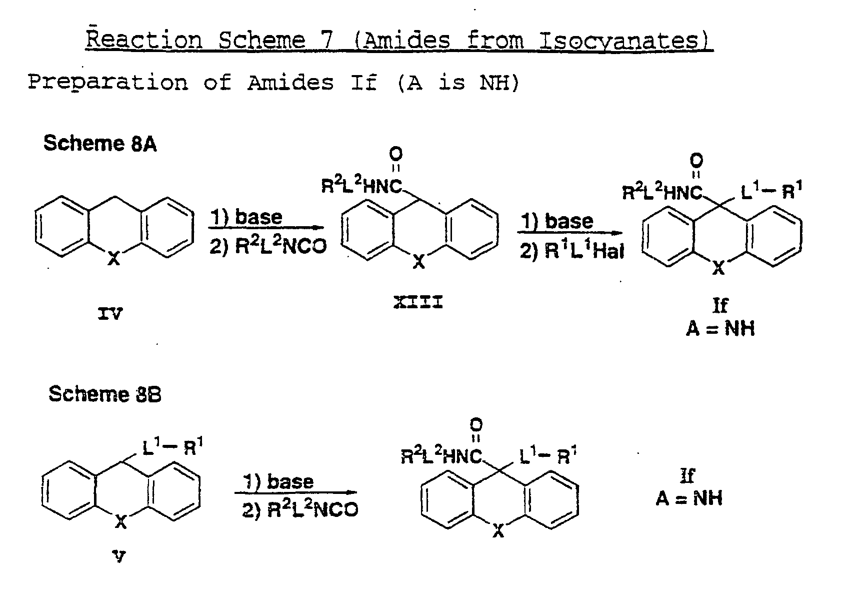

Compounds of formula I where A is -NH-(amides) can be prepared by the methods shown in Reaction Scheme 7A from known compound IV. Treatment of compound IV with base, such as n-BuLi, followed by reacting the anion with an isocyanate gives compound XIII. Compound XIII can be further transformed to compounds of the formula If as shown above.

In a similar manner, as seen in Scheme 7B, compound V can be transformed to compounds of the formula If.

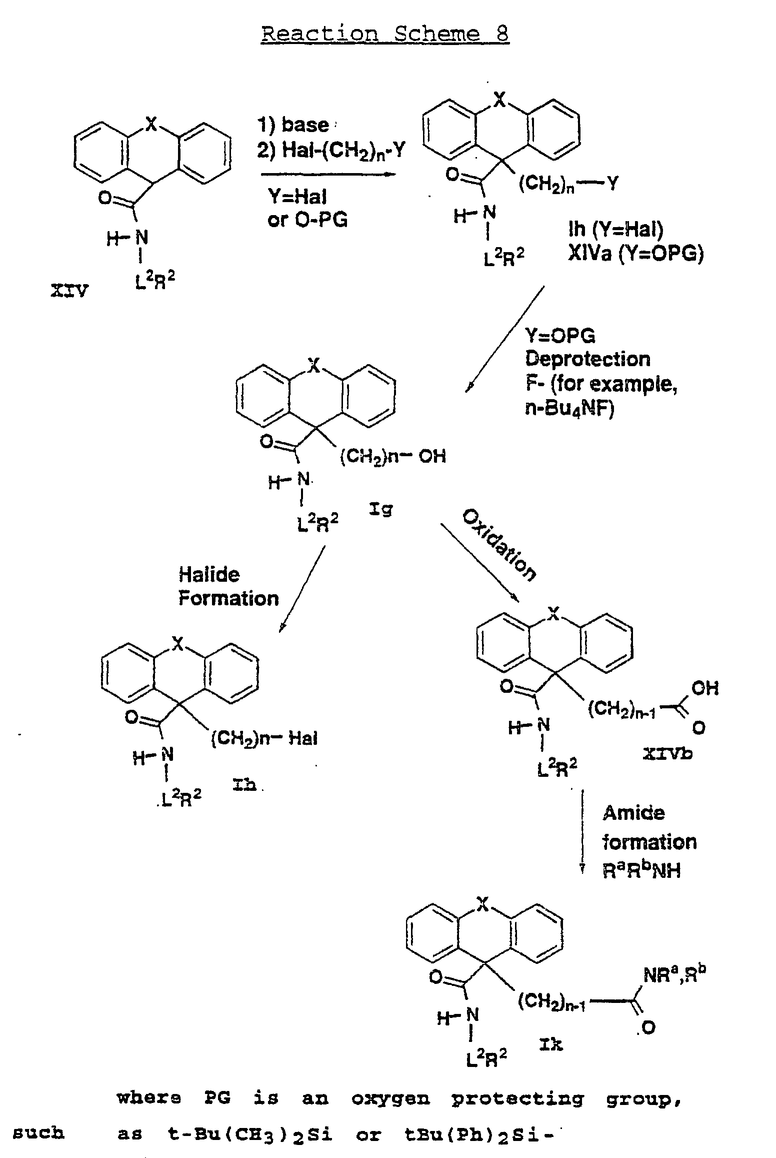

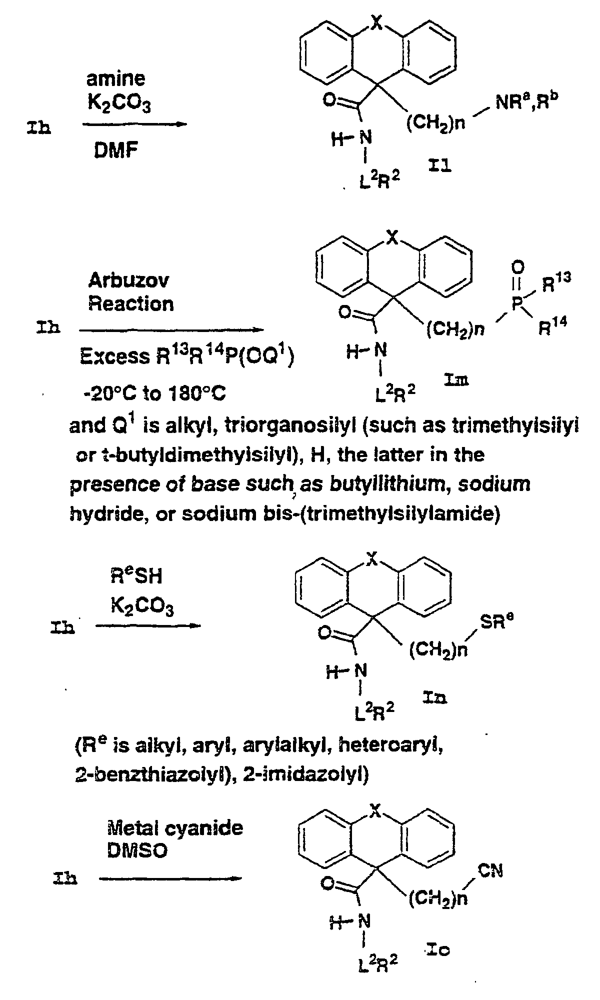

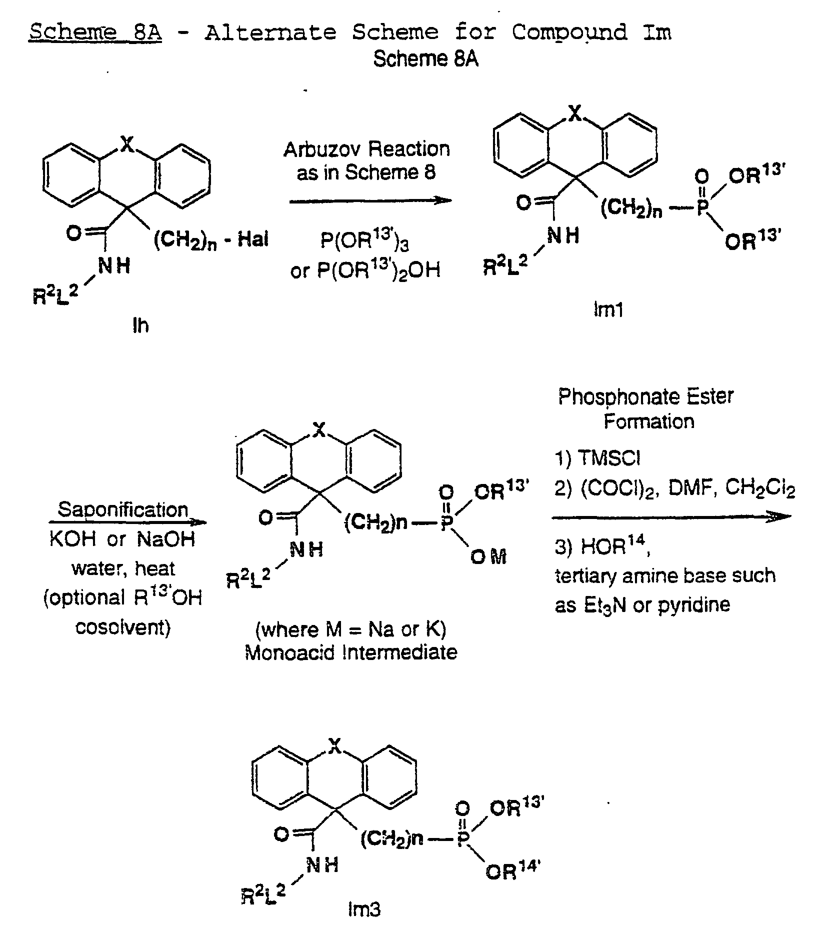

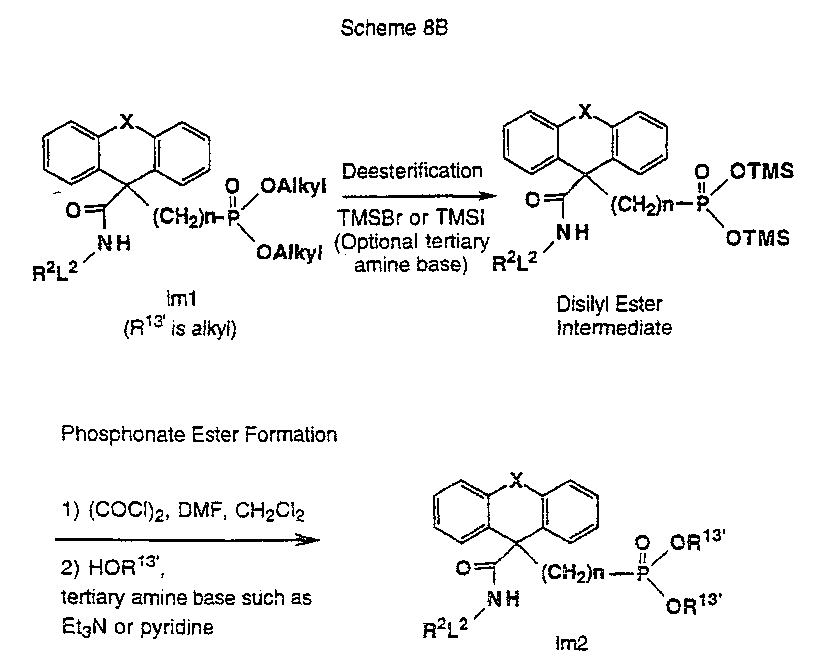

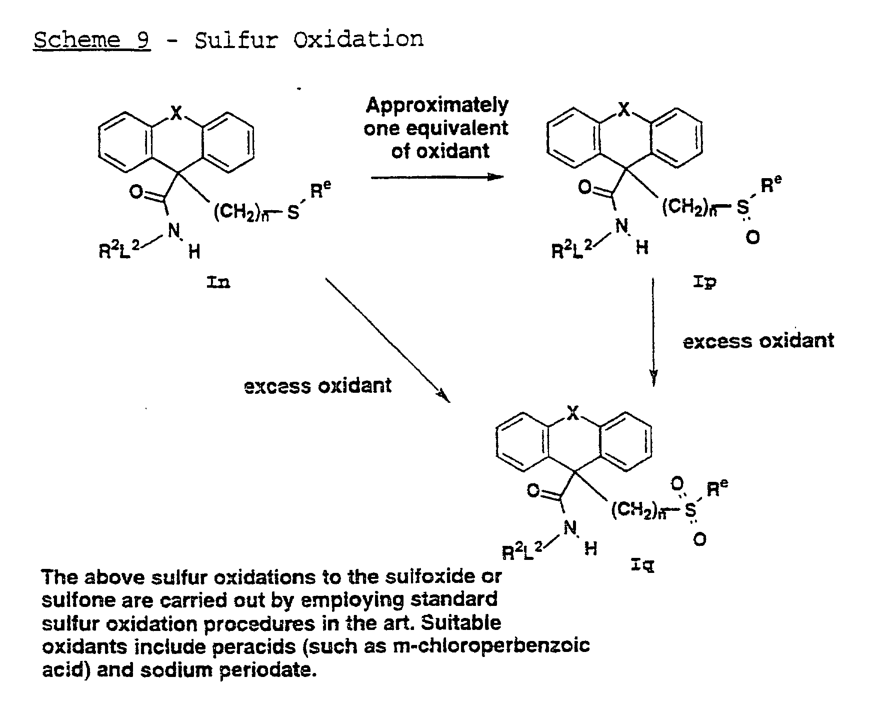

Compounds I of the invention may be modified by the various transformations set out in Reaction Scheme 8. Protected alcohol XIVa can be converted into a wide variety of functional groups through the intermediacy of a halide Ih. For example; the alcohol Iq can be converted to the halide Ih of the invention by either activation through the sulfonate ester (tosyl chloride, or mesyl chloride) and iodide displacement (NaI or KI in acetone or 2-butanone), or by reaction with triphenylphosphine, I₂ and imidazole. The iodide Ih can undergo an Arbuzov reaction to form phosphonates, phosphinates and phosphine oxides of the invention Im. The Arbuzov reaction can be accomplished with phosphites, phosphinites, and phosphonites (for example, R¹³R¹⁴POalkyl or R¹³R¹⁴POSi(alkyl)₃ or R¹³R¹⁴POH, the latter being in the presence of a base such as butyllithium, sodium hydride or sodium bis(trimethyl-silylamide)) at temperatures within the range from about -20°C to about 180°C. Alternately, displacement reactions to form amines Il, thioethers In or nitriles Io can be easily accomplished. To form amines Il, iodide Ih, can be treated with amines in DMF with or without K₂CO₃. Thioethers In can also be formed under similar conditions. The nitriles If are prepared from either KCN or NaCN in hot DMSO. The alcohol can also be oxidized to a carboxylic acid. The acids can also be used as intermediates to form amides of the invention Ik by methods previously described. The sulfur atom of In can be oxidized under standard conditions to sulfoxide Ip or sulfone Iq.

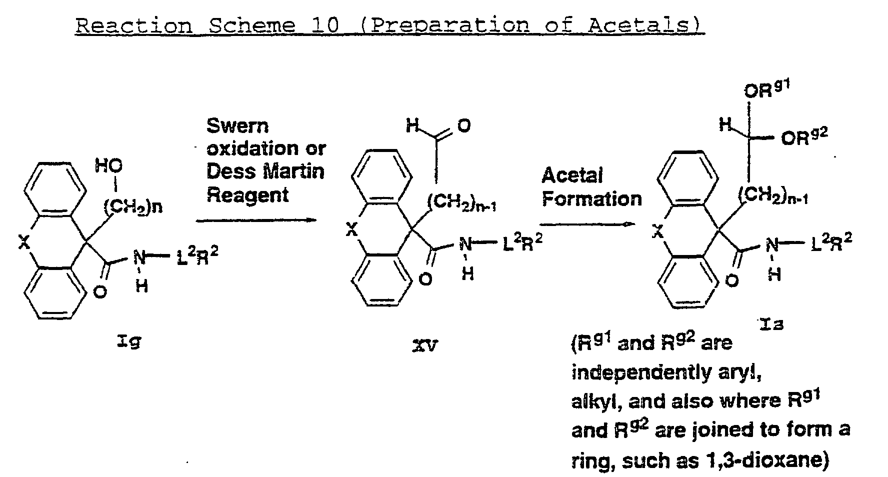

Acetals of the invention Is can be prepared from alcohol Ig by oxidation of the alcohol to the aldehyde XV. Prefered reagents to accomplish the transformation are either the Swern oxidation ((COCl)₂, DMSO, triethylamine) or Dess-Martin Periodinane. The aldehyde XV can be converted to the acetal Is with excess alcohol such as 1,3-propanediol or ethylene glycol in the presence of a catalytic amount of acid such as H₂SO₄ or p-toluenesulfonic acid, optionally in the presence of a dehydrating agent such as 4A sieves or trimethyl orthoformate.

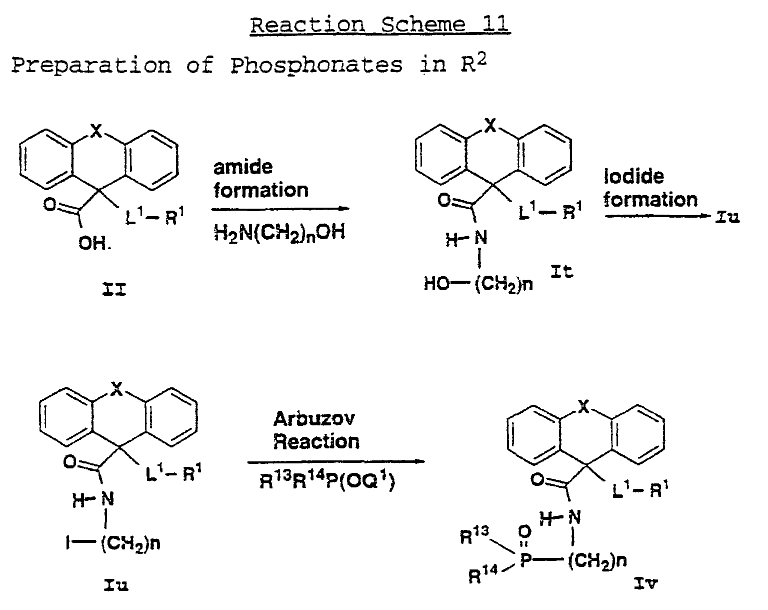

An addition procedure to incorporate the phosphonate in the N-alkyl chain is shown in Scheme 11. Carboxylic acid II is converted to the amide of the invention It as follows. Activation of the acid II to the acid chloride (with oxalyl chloride or thionyl chloride) followed by treatment with an aminoalcohol such as 1,5-aminopentanol or 1,3-aminopropanol gives amide of the invention It. Various additional methods of activation include mixed anhydride formation ((CF₃COO)₂ or i-BuOCOCl) or formation of the acylimidazole (carbonyldiimidazole) or with DCC and HOBT in the presence of DMAP. These activated intermediates readily form amides upon treatment with aminoalcohols. The alcohol It can then be converted to the iodide Iu by either activation through the sulfonate ester (tosyl chloride or mesyl chloride) and iodide displacement (NaI or KI in acetone or 2-butanone) or by reaction with triphenylphosphine, I₂ and imidazole. The iodide Iu can be reacted with a phosphorus (III) derivative R¹³R¹⁴P(OQ¹), for example triethylphosphite, tributylphosphite or (phenyl)₂POC₂H₅, in an Arbuzov reaction to give the phosphonate of the invention Iv.

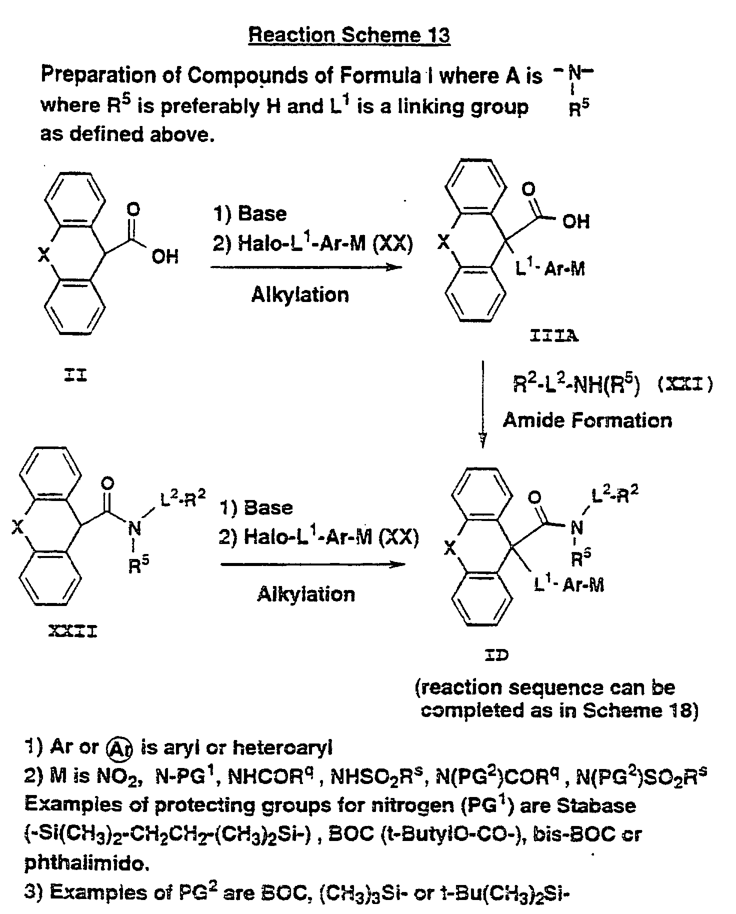

Compounds of the invention of formula I where A is 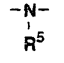 and R⁵ is preferably H, and L¹ is a linking group as defined above can be prepared as shown in Reaction Scheme 13.

As seen in Scheme 13, acid II is treated with base and alkylated by reaction with halide XX, as described with respect to Scheme 1, to form alkylated intermediate IIIA. IIIA is reacted with amine XXI (using the amide formation procedure as described in Scheme 1) to form amide of the invention ID.

Where M in ID is NO₂, NHCOR^{q} or NHSO₂R^{s}, ID represents a final product.

Where M includes a protecting group, the protecting group may be removed as shown in Scheme 18.

Where desired, acid II may undergo amide formation by reaction with amine XXI to form amide XXII via various known procedures, which is then alkylated to form ID.

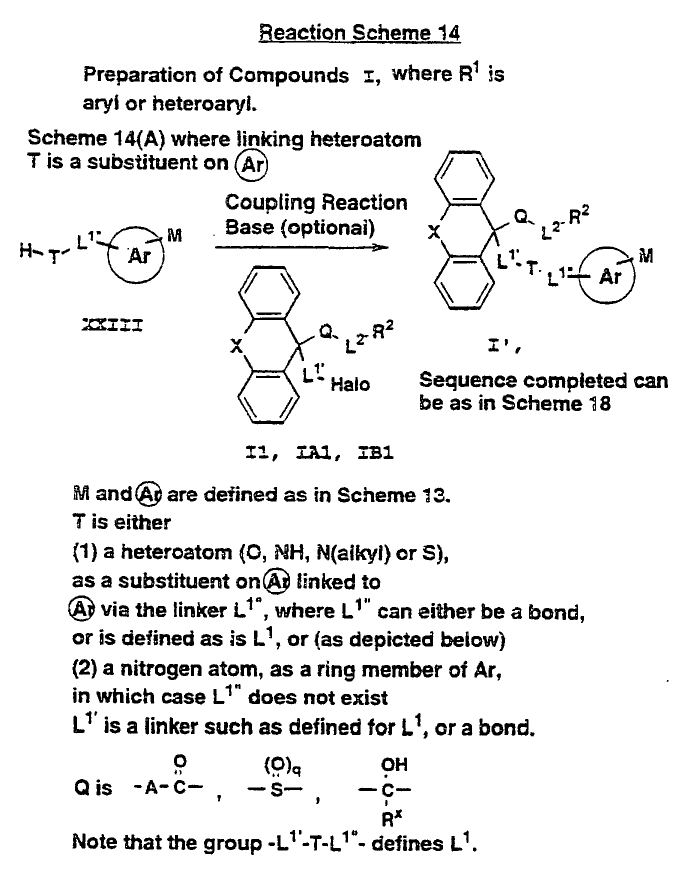

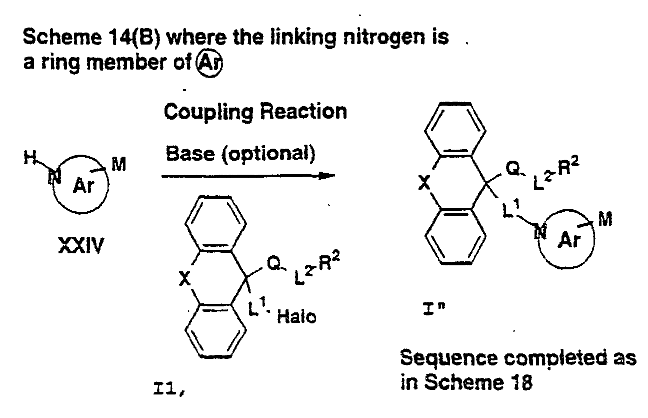

Compounds of the invention of formula I, where R¹ is aryl or heteroaryl may be prepared as shown in Reaction Schemes 14(A) and 14(B) .

In Scheme 14(A) compounds of formula I', (where R¹ is aryl or heteroaryl) may be prepared by coupling compound XXIII with compound Il, respectively, optionally in the presence of a base as described with respect to Scheme 1.

Compounds I', I", may be subjected to deprotection and/or further converted, where necessary as shown in Scheme 18.

In Scheme 14(B) compounds of formula I", (where R¹ is heteroaryl and 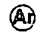 is linked to L¹ via a ring nitrogen)) may be prepared by coupling XXIV with I1, optionally in the presence of a base.

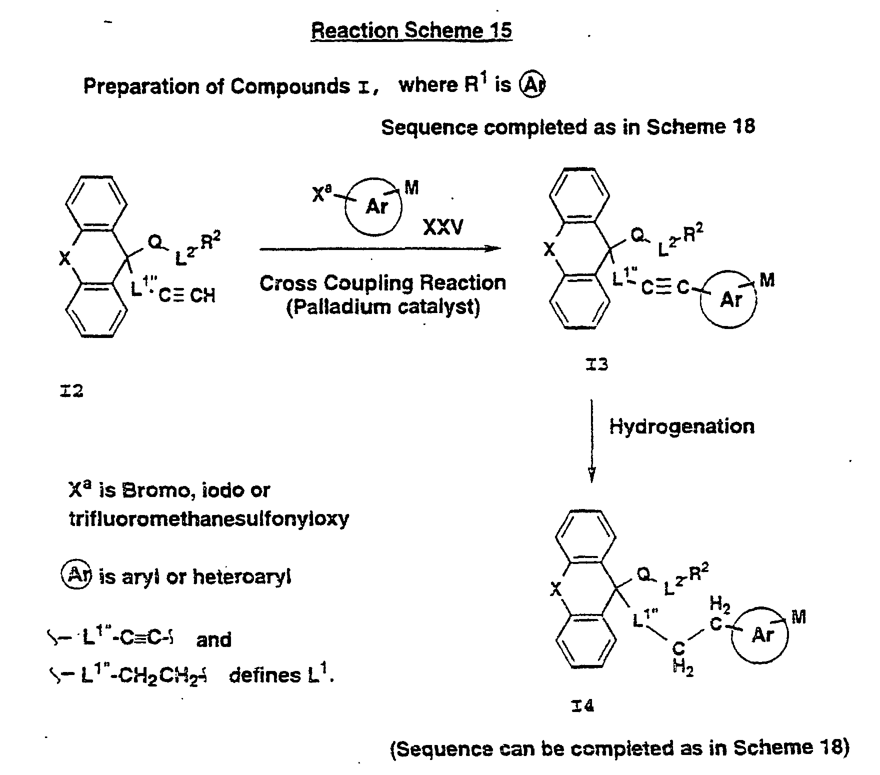

Compounds of the invention of formula I, where R¹ is 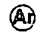 may be prepared as shown in Reaction Scheme 15.

In Scheme 15, acetylenic starting compound I2, is made to undergo a Castro-Stevens cross coupling with XXV in the presence of a catalyst, such as palladium, Pd(Ph₃P)₄ or Pd(Ph₃P)₂Cl₂ in the presence of an amine (e.g. BuNH₂, Et₃N) and a Copper (I) salt (e.g. CuI) to form compound of the invention I3, respectively, and subjecting I3, to hydrogenation to form compound of the invention I4.

Compound I3, or I4, may be subjected to deprotection and further conversion if necessary, as described in Reaction Scheme 18.

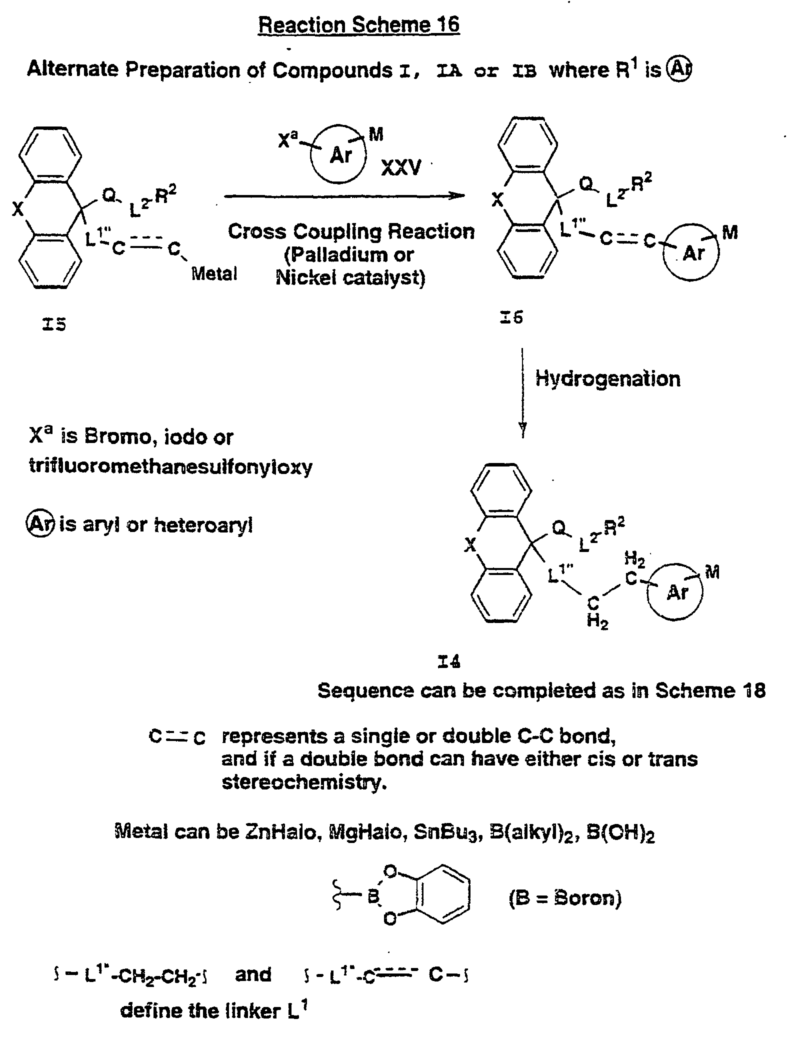

In an alternative procedure as shown in Reaction Scheme 16 compound I4, may be prepared starting with compound I5, respectively, which is made to undergo a cross coupling reaction with XXV in the presence of a palladium or nickel catalyst, to form I6, respectively, which is hydrogenated to form I4, respectively.

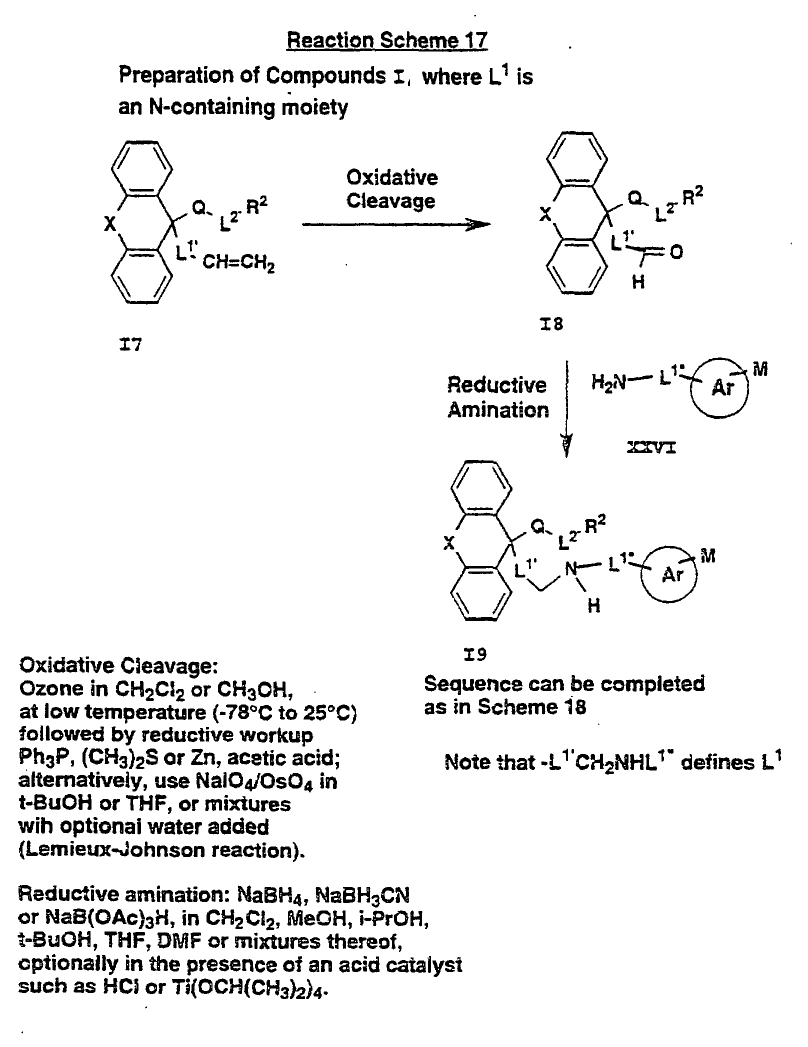

Compounds of the invention of formula I, where L¹ is an N-containing moiety may be prepared as shown in Reaction Scheme 17 wherein starting compound I7, is made to undergo oxidative cleavage, as described above, to form aldehyde I8, respectively, which is subjected to reductive amination by reaction with amine XXVI, as described above, to form compound of the invention I9, respectively.

Compound I9, may undergo deprotection, if necessary, as shown in Scheme 18.

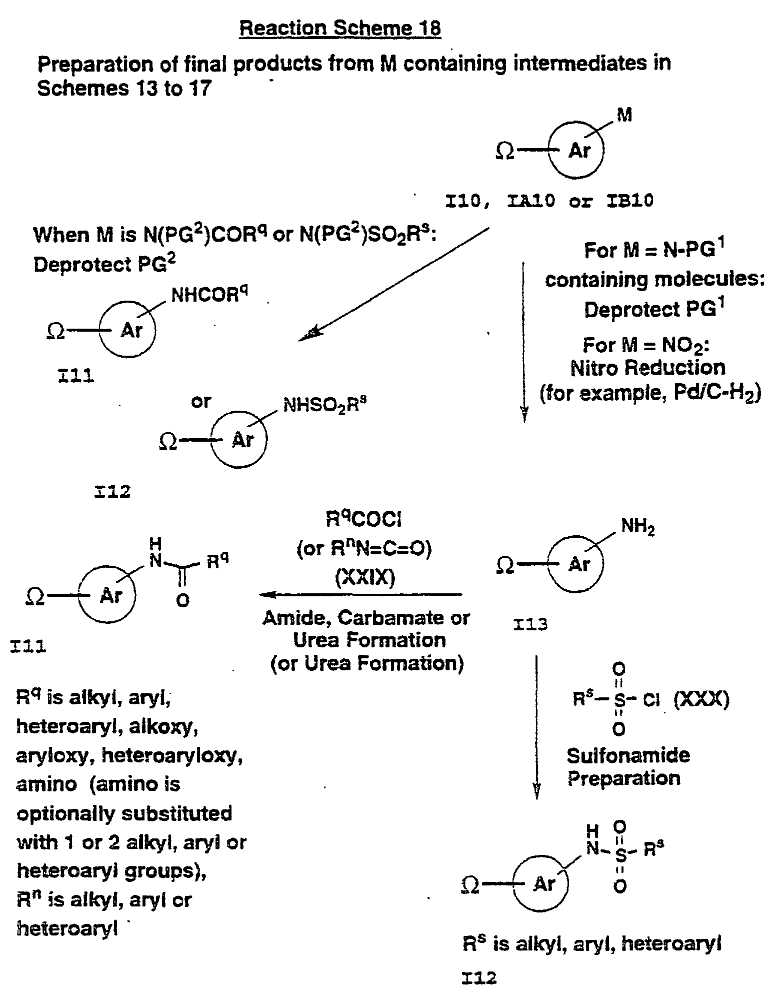

In a preferred method, superior yields of final products (I11, I12) are obtained when the intermediate I13, is reacted with R^{q}COCl, RⁿN=C=O or R^{s}SO₂Cl immediately after formation of I13, preferably in situ.

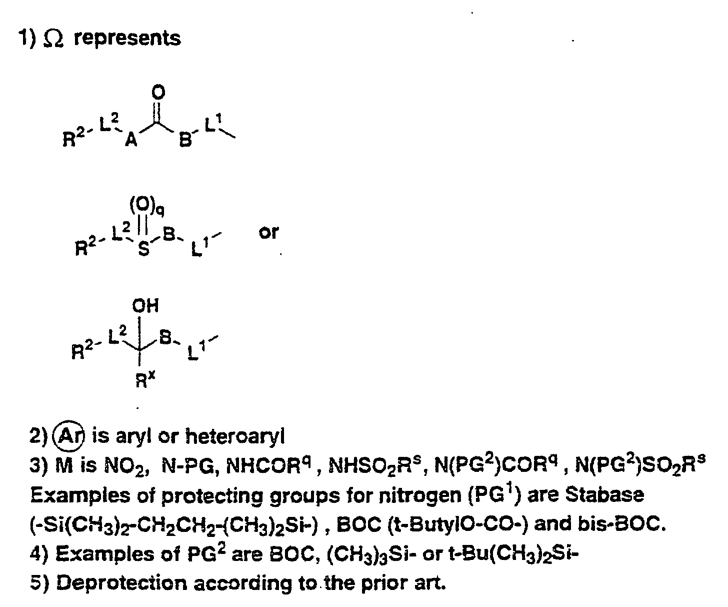

The compounds of the invention may be employed in preventing, stabilizing or causing regression of atherosclerosis in a mammalian species by administering a therapeutically effective amount of a compound to decrease the activity of MTP.

The compounds of the invention can be tested for MTP inhibitory activity employing the procedures set out in U.S. application Serial No. 117,362 filed September 3, 1993, employing MTP isolated from one of the following sources:
(1) bovine liver microsomes,
(2) HepG₂ cells (human hepatoma cells) or
(3) recombinant human MTP expressed in baculovirus.

The compounds of the invention may also be employed in lowering serum lipid levels, such as cholesterol or triglyceride (TG) levels, in a mammalian species, by administering a therapeutically effective amount of a compound to decrease the activity of MTP.

The compounds of the invention may be employed in the treatment of various other conditions or diseases using agents which decrease activity of MTP. For example, compounds of the invention decrease the amount or activity of MTP and therefore decrease serum cholesterol and TG levels, and TG, fatty acid and cholesterol absorption and thus are useful in treating hypercholesterolemia, hypertriglyceridemia, hyperlipidemia, pancreatitis, hyperglycemia and obesity.

The compounds of the present invention are agents that decrease the activity of MTP and can be administered to various mammalian species, such as monkeys, dogs, cats, rats, humans, etc., in need of such treatment. These agents can be administered systemically, such as orally or parenterally.

The agents that decrease the activity or amount of MTP can be incorporated in a conventional systemic dosage form, such as a tablet, capsule, elixir or injectable formulation. The above dosage forms will also include the necessary physiologically acceptable carrier material, excipient, lubricant, buffer, antibacterial, bulking agent (such as mannitol), anti-oxidants (ascorbic acid or sodium bisulfite) or the like. Oral dosage forms are preferred, although parenteral forms are quite satisfactory as well.

The dose administered must be carefully adjusted according to the age, weight, and condition of the patient, as well as the route of administration, dosage form and regimen, and the desired result. In general, the dosage forms described above may be administered in amounts of from about 5 to about 500 mg per day in single or divided doses of one to four times daily.

The following Examples represent preferred embodiments of the invention. All temperatures are in °C unless indicated otherwise.

Where structures are set in the following Examples which include hetero atoms with unfilled valency, it will be understood that hydrogen is attached to such hetero atoms to fulfill valency requirements.

### Example 1

### N-(Phenylmethyl)-9-(3-phenylpropyl)-9H-fluorene-9-carboxamide

### A. N-(Phenylmethyl)-9H-fluorene-9-carboxamide

A solution of 9-fluorene carboxylic acid (2.10 g, 10.0 mmol) in 50 mL of CH₂Cl₂ was treated with oxalyl chloride in dichloromethane (6.0 mL, 12.0 mmol) and two drops of DMF. After 0.75 h, the mixture was concentrated under reduced pressure to give a white solid. The solid was diluted with 50 mL of CH₂Cl₂, cooled to 0°C, treated with benzylamine (1.17 g, 11.0 mmol) and pyridine (0.87 g, 11 mmol). The transparent yellow solution was stirred for 3 h at room temperature and diluted with ethyl acetate and water. The organic fraction was dried over Na₂SO₄ and concentrated to a white solid. The solid purified by trituration with hexanes and recrystalization from hot methanol to give 2.60 g (86%) of title compound as white flakes. mp 195-200°C.
TLC Silica gel (3:7 ethyl acetate/hexane) R_{f}= 0.30. Mass Spec. (CI-NH₃, + ions) m/z 300 (M+H), 317 (M+NH4).

| | | | |
|---|---|---|---|
| Anal. Calc'd for C₂₁H₁₇NO | C, 84.25; | H, 5.72; | N, 4.68 |
| Found | C, 83.96; | H, 5.68; | N, 4.54. |

### B. N-(Phenylmethyl)-9-(3-phenylpropyl)-9H-fluorene-9-carboxamide

To a suspension of Part A compound (0.35 g, 1.17 mmol) in THF (10 mL) at 0°C was added n-butyllithium in hexanes (1.0 mL, 2.4 mmol) dropwise at such at rate to maintain the internal temperature near 0°C. The resulting bright orange solution was stirred at 0°C for 0.5 h and treated with 1-bromo-3-phenylpropane (0.26 g, 1.30 mmol). The mixture was slowly warmed to room temperature and stirred for 3 h and diluted with NH₄Cl (20 mL) and ethyl acetate (50 mL). The layers were separated, the organic fraction dried (Na₂SO₄) and concentrated. The remainder was purified by column chromatography on silica gel (30 g) with 2:8 ethyl acetate/hexane to give 0.33 g (67%) of title compound as a white solid. The solid was recrystalized from hot hexane to give 0.25 g (51%) of title compound as white flakes. mp 94°C.
TLC Silica gel (3:7 ethyl acetate/hexane) R_{f}= 0.70.
Mass Spec. (CI-NH₃, + ions) m/z 418 (M+H), 435 (M+NH₄).

| | | | |
|---|---|---|---|
| Anal. Calc'd for C₃₀H₂₇NO | C, 86.30; | H, 6.52; | N, 3.35 |
| Found | C, 85.99; | H, 6.47; | N, 3.21. |

Examples 2-4 were prepared from Example 1 Part A by the method described in Example 1, Part B.

### Example 2

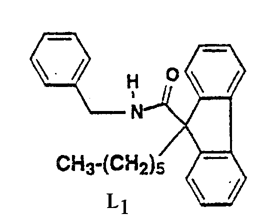

MS (Cl-NH₃, + ions) m/e 384 (M+H).
mp: 79-82°

| | | | |
|---|---|---|---|
| Anal. Cald'd for C₂₇H₂₉NO | C, 84.56; | H, 7.62; | N, 3.65 |
| Found | C, 84.22; | H, 7.72; | N, 3.65. |

### Example 3

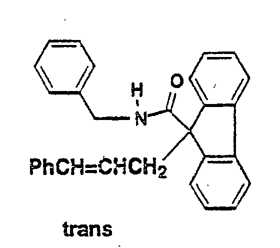

MS (Cl-NH₃, + ions) m/e 416 (M+H).
mp: 134°

| | | | |
|---|---|---|---|
| Anal. Cald'd for C₃₀H₂₅NO | C, 86.72; | H, 6.06; | N, 3.37 |
| Found | C, 86.61; | H, 6.23; | N, 3.31. |

### Example 4

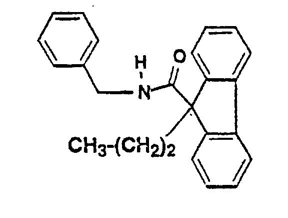

MS (Cl-NH₃, + ions) m/e 342 (M+H), 359 (M+NH₄).
mp: 96°

| | | | |
|---|---|---|---|
| Anal. Cald'd for C₂₄H₂₃NO | C, 84.42; | H, 6.79; | N, 4.10 |
| Found | C, 84.29; | H, 6.72; | N, 3.96. |

### Example 5

### (E)-N-Ethyl-9-(3-phenyl-2-propenyl)-9H-fluorene-9-carboxamide

### A.

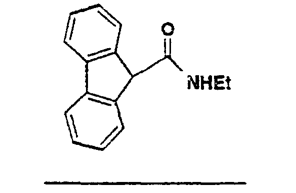

A solution of 9-fluorene carboxylic acid (2.10 g, 10.0 mmol) in 50 mL of CH₂Cl₂ was treated with oxalyl chloride in dichloromethane (6.0 mL, 12.0 mmol) and two drops of DMF. After 0.75 h, the mixture was concentrated under reduced pressure to give a white solid. The solid was diluted with 50 mL of CH₂Cl₂, cooled to 0°C, treated with ethylamine (1.0 g, 22 mmol). The transparent yellow solution was stirred for 3 h at room temperature and diluted with ethyl acetate and water. The organic fraction was dried over Na₂SO₄ and concentrated to a white solid. The solid purified by trituration with hexanes and recrystalization from hot methanol to give 2.60 g (86%) of title compound as white flakes. mp 233-234°C.

### B. (E)-N-Ethyl-9-(3-phenyl-2-propenyl)-9H-fluorene-9-carboxamide

To a suspension of Part A compound (1.00 g, 4.21 mmol) in THF (25 mL) at 0°C was added n-butyllithium in hexanes (3.53 mL, 8.84 mmol) dropwise at such at rate to maintain the internal temperature near 0°C. The resulting bright yellow solution was stirred at 0°C for 0.5 h and treated with cinnamyl chloride (0.79 g, 4.63 mmol). The mixture was slowly warmed to room temperature and stirred for 2 h when it was diluted with water (40 mL) and ethyl acetate (40 mL). The layers were separated, the organic fraction dried (Na₂SO₄) and concentrated. The remainder was triturated with hexanes and the resulting solid recrystalized from hot methanol to give 1.20 g (79%) of title compound as white needles. mp 144°C.
TLC Silica gel (3:7 ethyl acetate/hexane) R_{f}=0.6.

| | | | |
|---|---|---|---|
| Anal. Calc'd for C₂₅H₂₃NO | C, 84.95; | H, 6.56; | N, 3.96 |
| Found | C, 84.53; | H, 6.74; | N, 3.95. |

Example 6-10 can be prepared from Example 5 Part A compound by the method described in Example 5 Part B.

### Example 6

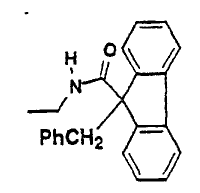

MS (Cl-NH₃, + ions) m/e 328 (M+H).
mp: 126-128°

| | | | |
|---|---|---|---|
| Anal. Cald'd for C₂₃H₂₁NO | C, 84.37; | H, 6.46; | N, 4.29 |
| Found | C, 84.22; | H, 6.42; | N, 4.58. |

### Example 7

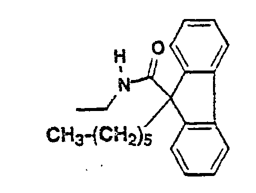

MS (Cl-NH₃, + ions) m/e 322 (M+H).
mp: 70°

| | | | |
|---|---|---|---|
| Anal. Cald'd for C₂₂H₂₇NO | C, 82.20; | H, 8.47; | N, 4.36 |
| Found | C, 82.07; | H, 8.55; | N, 4.74. |

### Example 8

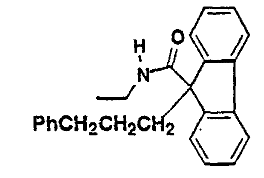

MS (Cl, + ions) m/z 356 (M+H).
mp: 72-73°

| | | | |
|---|---|---|---|
| Anal. Cald'd for C₂₅H₂₅NO + 0.3 H₂O | C, 83.08; | H, 7.16; | N, 3.88 |
| Found | C, 82.84; | H, 7.89; | N, 3.78. |

### Example 9

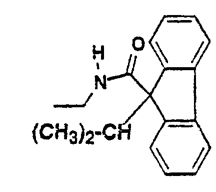

MS (Cl-NH₃, + ions) m/e 280 (M+H).
mp: 66-67°

| | | | |
|---|---|---|---|
| Anal. Cald'd for C₁₉H₂₁NO | C, 81.68; | H, 7.58; | N, 5.01 |
| Found | C, 81.60; | H, 7.87; | N, 5.08. |

### Example 10

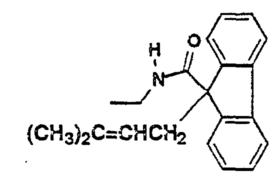

MS (Cl-NH₃, + ions) m/e 306 (M+H).
mp: 78°

| | | | |
|---|---|---|---|
| Anal. Cald'd for C₂₁H₂₃NO | C, 82.59; | H, 7.59; | N, 4.59 |
| Found | C, 82.37; | H, 7.74; | N, 4.57. |

### Example 11

### 9-[4-(Dibutoxyphosphinyl)butyl]-N-propyl-9H-fluorene-carboxamide

### A. N-Propyl-9-fluorene-carboxamide

A solution of 9-fluorene carboxylic acid (20.0 g, 95 mmol) in 200 mL of CH₂Cl₂ was treated with oxalyl chloride (12.5 g, 105 mmol) and 0.2 mL of DMF. After 0.75 h, the mixture was concentrated under reduced pressure to give a white solid. The solid was diluted with 100 mL of THF cooled to -40°C, treated with propylamine (11.8 g, 200 mmol). The suspension was stirred for 3 h at room temperature and diluted with ethyl acetate and water. The organic fraction was dried over Na₂SO₄ and concentrated to a white solid. The solid purified by trituration with hot hexanes and recrystalization from hot methanol to give 17.5 g (87%) of title compound as white flakes. mp 197-199°C .
TLC Silica gel (3:7 ethyl acetate/hexane) R_{f}= 0.30.
MS (CI-NH₃, + ions) m/e 252 (M+H).

### B. Dibutyl (4-bromobutyl)phosphonate

A mixture of 1,4-dibromobutane (129 g, 600 mmol) and tributyl phosphite (15.0 g, 60 mmol) was heated to 118°C (bath temperature) for 6 h. The volatiles were removed by short path distillation (0.4 mm Hg, 40°C) to leave 20 g (100%) of part b compound as an amber colored oil. The oil can be purified by flash column chromatography on silica gel with 1:9 acetone/dichloromethane.
TLC: (1:9 acetone/dichloromethane) R_{f}=0.55.
¹³C NMR (d₆-acetone) δ 64.4 (d, J=6 Hz), 33.1, 33.0 (d, J=22 Hz), 32.4 (d, J=6 Hz), 24.0 (J=140 Hz), 21.1 (J=5 Hz), 18.5, 13.0 ppm.

### C. Dibutyl (4-Iodobutyl)phosphonate

A mixture of Part B compound (4.8 g, 14.58 mmol), potassium iodide (20.0 g, 120 mmol) and acetone (200 mL) was heated to reflux for 2.5 h and cooled to room temperature. The solids were filtered and the filtrate concentrated. The remainder was diluted with ether and filtered. The ether fraction was concentrated to give 5.32 g (97%) of title compound as a pale yellow oil.
TLC: (1:9 acetone/dichloromethane) R_{f}=0.55.
¹³C NMR (CDCl₃) δ 65.2 (d, J=7 Hz), 33.7 (d, J=17 Hz), 32.4 (d, J=6 Hz), 24.2 (J=140 Hz), 18.6, 13.5, 5.5 ppm.

### D. 9-[4-(Dibutoxyphosphinyl)butyl]-N-propyl-9H-fluorene-9-carboxamide

A solution of Part A compound (3.00 g, 11.95 mmol) in 30 mL of THF at -40° was treated with n-BuLi (5.20 mL, 13 mmol) in hexanes at such a rate to maintain the internal temperature below -35°. The orange yellow solution was stirred for 0.5 h and treated with Part C compound (4.30 g, 11.50 mmol). The mixture was warmed to room temperature over 0.5 h and after 2 h at room temperature was quenched with 100 mL of NH₄Cl solution and 100 mL of ethyl acetate. The organic fraction was dried (MgSO₄) and concentrated. The remainder was purified by column chromatography on silica gel (400 g) with 1:9 acetone/dichloromethane to give 4.30 g (75%) of title compound as a colorless oil.
TLC Silica gel (7:3 ethyl acetate/hexane) R_{f}= 0.5. Mass Spec. (ES, + ions) m/e 500 (M+H).

| | | | | |
|---|---|---|---|---|
| Anal. Calc'd for C₂₉H₄₂NO₄P + 0.6 H₂O | C, 68.29; | H, 8.53; | N, 2.75; | P, 6.07 |
| Found | C, 68.34; | H, 8.45; | N, 2.70; | P, 6.03. |

### Example 12

### (E)-9-(3-Phenyl-2-propenyl)-N-propyl-9H-fluorene-9-carboxamide

To a suspension of 500 mg (1.99 mmol) of Example 11 Part A compound in 10 mL of THF, at 0°C under argon, was added dropwise 2.5 mL (3.98 mmol) of n-BuLi (1.6 M in hexanes). The resulting orange solution was stirred at 0°C for 0.5 h at which time 305 µL (2.19 mmol) of cinnamyl chloride was added. The reaction was warmed to RT and allowed to stir for 1 h at which time it was diluted with 1:1 ethyl acetate/water (30 mL). The organics were dried (NaSO₄) and evaporated to dryness. Purification by crystallization from hot methanol provided 350 mg (48%) of title compound as a white solid.
mp 95-97°C.
TLC Silica gel (1:1 hexanes/ethyl acetate) R_{f} = 0.59.
MS (CI-NH₃, + ions) m/e 368 (M+H).

| | | | |
|---|---|---|---|
| Anal. calcd. for C₂₆H₂₅NO + 0.62 mol H₂O | C, 82.47; | H, 6.98; | N, 3.70 |
| Found | C, 82.67; | H, 6.92; | N, 3.50. |

Examples 13-21 can be prepared from Example 11 Part A by the method in Example 11 Part D or Example 12 Part A.

### Example 13

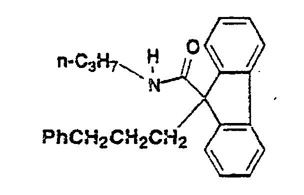

MS (Cl-NH₃, + ions) m/e 370 (M+H).
mp: 57-59°

| | | | |
|---|---|---|---|
| Anal. Cald'd for C₂₆H₂₇NO | C, 84.51; | H, 7.36; | N, 3.79 |
| Found | C, 84.53; | H, 7.41; | N, 3.70. |

### Example 14

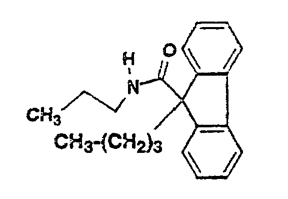

MS (Cl-NH₃, + ions) m/e 308 (M+H).
mp: 60-62°

| | | | |
|---|---|---|---|
| Anal. Cald'd for C₂₁H₂₅NO + 0.05 mol C₆H₁₄ | C, 82.07; | H, 8.32; | N, 4.49 |
| Found | C, 82.12; | H, 8.76; | N, 4.65. |

### Example 15

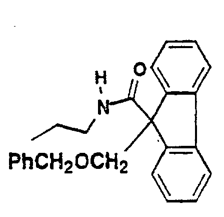

MS (Cl-NH₃, + ions) m/e 372 (M+H).

| | | | |
|---|---|---|---|
| Anal. Cald'd for C₂₅H₂₅NO₂ | C, 80.83; | H, 6.78; | N, 3.77 |
| Found | C, 80.48; | H, 6.90; | N, 3.71. |

### Example 16

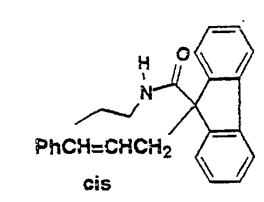

MS (Cl-NH₃, + ions) m/e 368 (M+H).

| | | | |
|---|---|---|---|
| Anal. Cald'd for C₂₆H₂₅NO + 0.31 mol H₂O | C, 83.71; | H, 6.92; | N, 3.75 |
| Found | C, 83.84; | H, 6.95; | N, 3.62. |

### Example 17

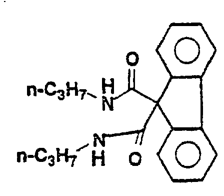

MS (Cl-NH₃, + ions) m/e 337 (M+H).

| | | | |
|---|---|---|---|
| Anal. Cald'd for C₂₁H₂₄N₂O₂ | C, 74.97; | H, 7.19; | N, 8.33 |
| Found | C, 74.94; | H, 7.17; | N, 7.80. |

### Example 18

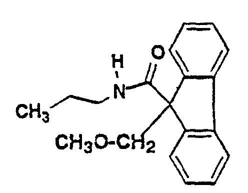

MS (Cl-NH₃, + ions) m/e 296 (M+H).
mp: 69-73°

| | | | |
|---|---|---|---|
| Anal. Cald'd for C₁₉H₂₁NO₂ + 0.09 mol C₂₁H₂₅NO₃ | C, 76.98; | H, 7.19; | N, 4.68 |
| Found | C, 76.71; | H, 7.42; | N, 4.65. |

### Example 19

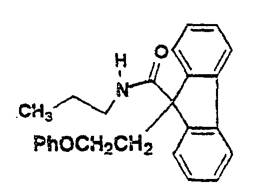

MS (Cl-NH₃, + ions) m/e 372 (M+H).

| | | | |
|---|---|---|---|
| Anal. Cald'd for C₂₅H₂₅NO₂ + 0.86 mol H₂O | C, 77.60; | H, 6.96; | N, 3.62 |
| Found | C, 77.92; | H, 6.54; | N, 3.88. |

### Example 20

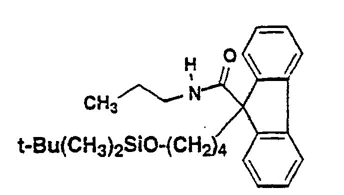

MS (Cl-NH₃, + ions) m/e 438 (M+H).
mp: 45-47°

| | | | |
|---|---|---|---|
| Anal. Cald'd for C₂₇H₃₉NSiO₂ | C, 74.09; | H, 8.98; | N, 3.20 |
| Found | C, 73.83; | H, 9.34; | N, 3.25. |

### Example 21

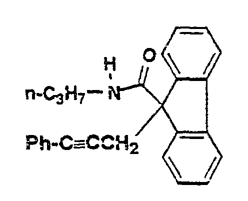

MS (ES, + ions) m/z 366 (M+H). mp: 120-123°

| | | | |
|---|---|---|---|
| Anal. Cald'd for C₂₆H₂₃NO + 0.15 mol H₂O | C, 84.76; | H, 6.38; | N, 3.80 |
| Found | C, 84.81; | H, 6.29; | N, 3.75. |

### Example 22

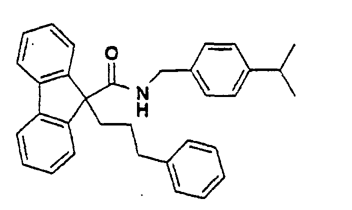

### A. 9-(3-Phenylpropyl)-9H-fluorene-9-carboxylic acid

To a solution of 10 g (48 mmol, 1 eq) of (9H)-flourene-9-carboxylic acid in 200 mL of THF at 0°C was added 40 mL (100 mmol, 2.1 eq) of a 2.5 M solution of n-butyllithium in hexanes dropwise over 15 min. (First equivalent resulted in precipitation of Li salt of the carboxylate; solution became homogeneous as dianion formed.) The resulting green solution of dianion was stirred at 0°C for 10 min and 10.1 mL (66 mmol, 1.4 eq) of 1-bromo-3-phenylpropane was added quickly over 3 min. The reaction was stirred at 0°C and allowed to warm to RT as the ice bath melted. After 16 h, the basic reaction mixture (pH ~14) was extracted with water (1 x 200 mL, 2 x 50 mL). The combined aqueous layers were acidified (to pH ~1) with 5 N HCl and extracted with ether (3 x 100 mL). The combined ether solutions were dried (MgSO₄), filtered and concentrated to afford 16.4 g of a viscous golden oil. Flash chromatography of the oil on silica gel (250 g) eluted with 20% acetone in toluene containing 0.1 % acetic acid afforded 12.6 g of a yellow oil. The product was crystallized by slow evaporation of an ether/hexanes solution and then recrystallized from ether/hexanes to afford 10.5 g (67%) of title compound as a white crystalline solid.
m.p. 123-125°C.
TLC (silica gel, 10% MeOH in CH₂Cl₂, UV and I₂)
R_{f} = 0.67.

### B. 9-(3-Phenylpropyl)-9H-fluorene-9-carboxylic acid, 4-nitrophenyl ester

To a solution of 10 g (30.4 mmol, 1 eq) of Part A compound in 100 mL of CH₂Cl₂ was added 100 µL of DMF. The solution was cooled to 0°C and 22.8 mL (45.7 mmol, 1.5 eq) of a 2.0 M oxalyl chloride solution in CH₂Cl₂ was added over 5 min. The resulting bubbling solution was stirred at 0°C for 1.5 h (until bubbling had ceased). The solution was concentrated and the residual oil was taken up in 50 mL of CH₂Cl₂ and reconcentrated. The resulting oil was dissolved in 150 mL of CH₂Cl₂ and 188 mg (1.5 mmol, 0.05 eq) of 4-dimethylaminopyridine was added. The solution was cooled to 0°C and 5.1 mL (36.5 mmol, 1.2 eq) of triethylamine was added. To the resulting dark brown cloudy solution was added 12.7 g (91.3 mmol, 3 eq) of p-nitrophenol as a solid. Upon addition the reaction quickly became clear and the resulting clear reaction mixture was allowed to warm to RT as the ice bath melted. (TLC indicated the reaction was essentially complete after 40 min.) After 15 h, the reaction was washed with 100 mL of ice-cold 1 N HCl. The organic solution was filtered through cotton and concentrated to afford 24.84 g of a viscous golden-brown oil which was adsorbed onto silica gel (25 g) and chromatographed on silica gel (200 g) eluted with 10% ethyl acetate in hexanes to afford 13.54 g of a yellow solid. The solid was further purified by recrystallization from ether/hexanes to provide 13.2 g (97%) of title compound as a pale yellow crystalline solid. m.p. 110-112°C.
TLC (silica gel, 25% EtOAc in hexanes, UV and I₂)
R_{f} = 0.39.
MS(CI, pos. ions): *m*/*z* 467 (M + NH₄), 450 (M + H).

| | | | |
|---|---|---|---|
| Anal. Calcd. for C₂₉H₂₃NO₄ | C, 77.49; | H, 5.16; | N, 3.12 |
| Found | C, 77.27; | H. 4.90; | N, 2.99. |

### C.

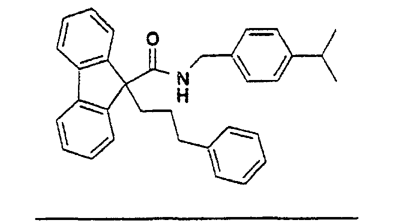

The title compound was prepared via an automated procedure carried out on a Zymark Benchmate® Workstation using the following procedure.

The Benchmate® delivered 1 mL (80 mg, 0.18 mmol, 1 eq) of a stock solution of Part B compound in THF (80 mg/mL) to a 16 mm x 100 mm culture tube. The tube was removed and placed on a balance where 40 mg (0.27 mmol, 1.5 eq) of 4-isopropylbenzylamine was added manually by a Pipetman. The reaction was allowed to proceed until all reactions in the run were complete as indicated by disappearance of Part B compound by TLC (silica gel, 2% MeOH in CH₂Cl₂,
R_{f} 0.88, visualized by UV and I₂).

The product was purified via solid phase extraction using a Varian SAX anion exchange column (1 g of sorbent, chloride form) on the Benchmate® by the procedure outlined below:
1) Syringe washed with 5 mL 300 mM KOH in MeOH.
2) Syringe washed with 5 mL 300 mM KOH in MeOH.
3) Column conditioned with 10 mL of 300 mM KOH(aq) in MeOH (0.25 mL/sec).
4) Column conditioned with 10 mL of MeOH (0.25 mL/sec).
5) Column conditioned with 10 mL of CH₂Cl₂ (0.25 mL/sec).
6) THF (1 mL) added to reaction mixture.
7) Reaction mixture loaded onto SAX column (0.05 mL/sec) and effluent collected into a second tube.
8) Column rinsed with 1 mL of THF and effluent collected into second tube.
9) Column rinsed with 2 mL of CH₂Cl₂ and effluent collected into second tube.
10) Syringe washed with 10 mL of CH₂Cl₂.
11) Syringe washed with 5 mL of MeOH.
12) Syringe washed with 4 mL of 300 mM KOH(aq) in MeOH.
13) Syringe washed with 4 mL of 300 mM KOH(aq) in MeOH.

This procedure was followed by a second solid phase extraction using a Varian SCX cation exchange column (500 mg of sorbent) on the Benchmate® by the procedure outlined below:
1) Column conditioned with 10 mL of CH₂Cl₂ (0.25 mL/sec).
2) Reaction mixture loaded onto SCX column (0.05 mL/sec) and effluent collected into product tube (tared).
3) Column rinsed with 2 mL of CH₂Cl₂ and effluent collected into product tube.
4) Syringe washed with 5 mL of CH₂Cl₂.
5) Syringe washed with 5 mL of CH₂Cl₂.

The product solution (approx. 5 mL) was concentrated using a speed vacuum for 14 h to afford 78 mg (94%) of title compound as a pale yellow oil.
HPLC Purity = 94%; retention time = 9.5 minutes. Column: YMC-Pack ODS 6.0 x 150 mm C18 with a 4 x 23 mm OSDA S-5 µm guard column. Buffer: 10 mM KH₂PO₄ (pH 5.4, unadjusted). Elution: Isocratic at 85:15 buffer:actetonitrile for 5 minutes; linear gradient from 85:15 to 5:95 buffer:acetonitrile over 9 minutes followed by isocratic 5:95 buffer:acetonitrile for 2 minutes with return to 85:15 buffer:acetonitrile over 2 minutes.
MS (CI, + ions): m/z 460 (M + H).

### Example 23 to 58

Examples 23-58 can be prepared from Example 22 Part B compound by the method in Example 22, Part C.

### Example 23

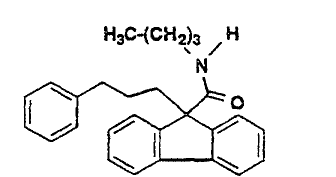

mp 73-75°C
MS (CI, pos. ions) 384 (M+H).

| | | | |
|---|---|---|---|
| Anal. Cald'd for C₂₇H₂₉NO + 0.04 H₂O | C, 84.40; | H, 7.63; | N, 3.65 |
| Found | C, 84.02; | H, 7.73; | N, 3.66. |

### Example 24

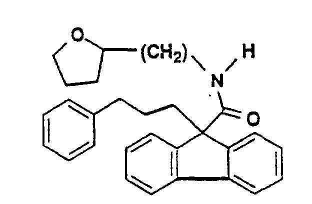

MS (CI, pos. ions) 412 (M+H).

### Example 25

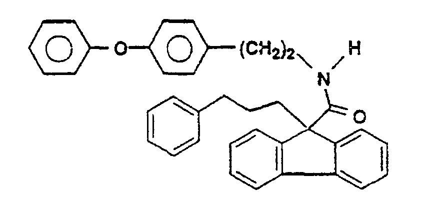

MS (CI, pos. ions) 524 (M+H).

### Example 26

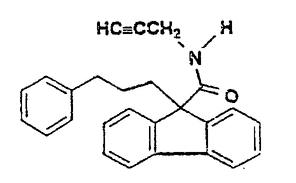

MS (CI, pos. ions) 366 (M+H).

### Example 27

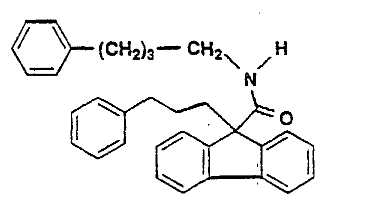

MS (CI, pos. ions) 460 (M+H).

### Example 28

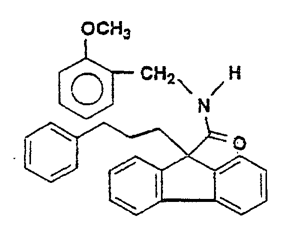

MS (CI, pos. ions) 448 (M+H).

### Example 29

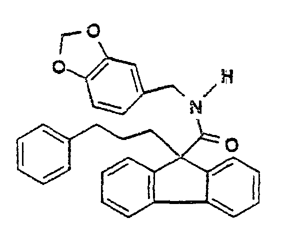

MS (electrospray, pos. ions) 462 (M+H).

### Example 30

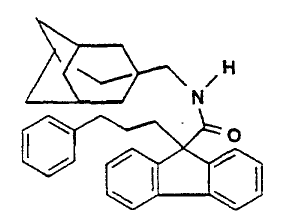

MS (electrospray, pos. ions) 476 (M+H).

### Example 31

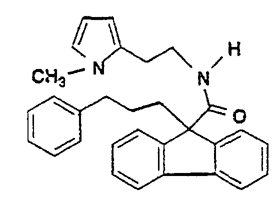

MS (electrospray, pos. ions) 435 (M+H).

### Example 32

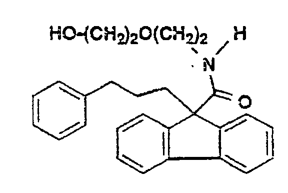

MS (electrospray, pos. ions) 416 (M+H).

### Example 33

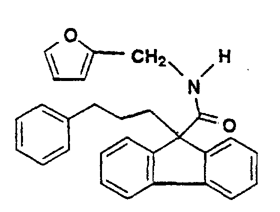

MS (electrospray, pos. ions) 408 (M+H).

### Example 34

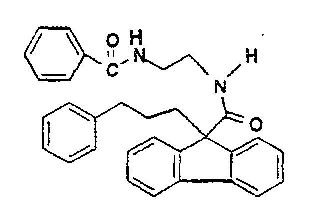

MS (electrospray, pos. ions) 475 (M+H).

### Example 35

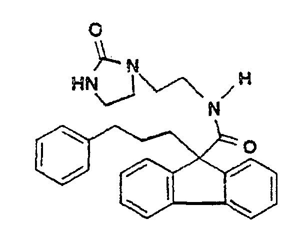

MS (electrospray, pos. ions) 440 (M+H).

### Example 36

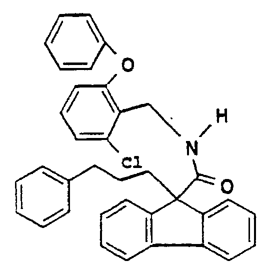

MS (electrospray, pos. ions) 544 (M+H).

### Example 37

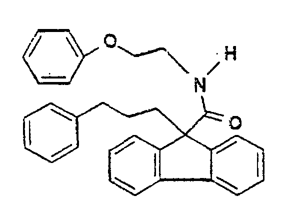

MS (electrospray, pos. ions) 448 (M+H).

### Example 38

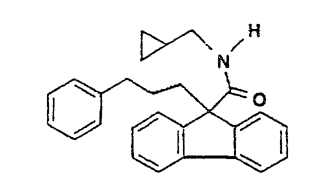

MS (electrospray, pos. ions) 382 (M+H).

### Example 39

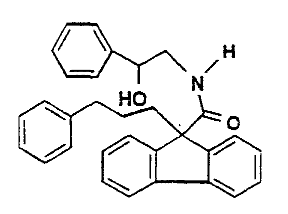

MS (electrospray, pos. ions) 448 (M+H).

### Example 40

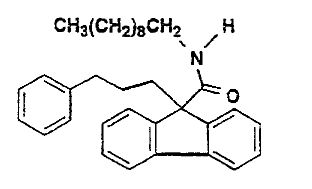

MS (electrospray, pos. ions) 468 (M+H).

### Example 41

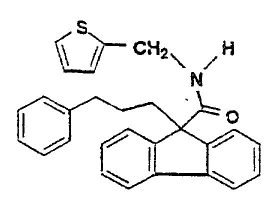

MS (electrospray, pos. ions) 424 (M+H).

### Example 42

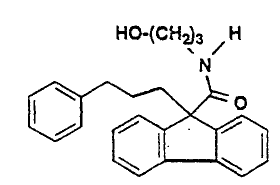

MS (electrospray, pos. ions) 386 (M+H).

### Example 43

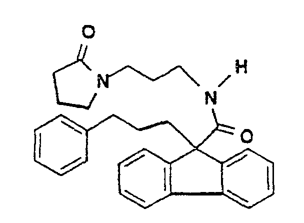

MS (electrospray, pos. ions) 453 (M+H).

### Example 44

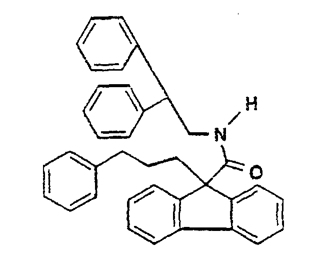

MS (electrospray, pos. ions) 508 (M+H).

### Example 45

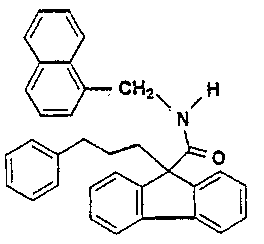

MS (electrospray, pos. ions) 468 (M+H).

### Example 46

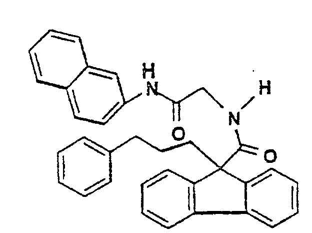

MS (electrospray, pos. ions) 511 (M+H).

### Example 47

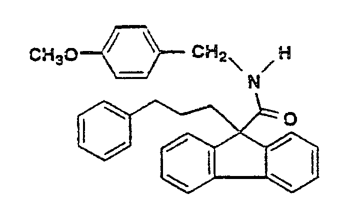

M. P. 105-107°C
MS (Cl,+ ions) m/z 448

| | | | |
|---|---|---|---|
| Anal. Cald'd for C₃₁H₂₉NO₂ + 0.15 H₂O | C, 82.69; | H, 6.56; | N, 3.11 |
| Found: | C, 82.36; | H, 6.37; | N, 2.99. |

### Example 48

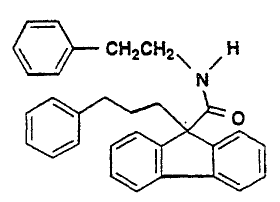

M.P. 104-105°C
MS (Cl,+ ions) m/z 432

| | | | |
|---|---|---|---|
| Anal. Cald'd for C₃₁H₂₉NO | C, 86.27; | H, 6.77; | N, 3.25 |
| Found | C, 85.87; | H, 6.60; | N, 3.14. |

### Example 49

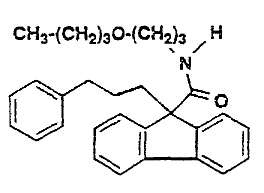

MS (Cl,+ ions) m/z 442

| | | | |
|---|---|---|---|
| Anal. Cald'd for C₃₀H₃₅NO₂ | C, 81.59; | H, 7.99; | N, 3.17 |
| Found | C, 81.93; | H, 8.11; | N, 3.04. |

### Example 50

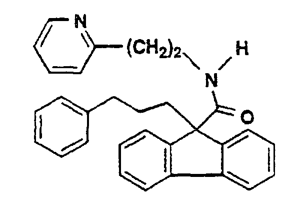

MS (electrospray, pos. ions) 433 (M+H)

### Example 51

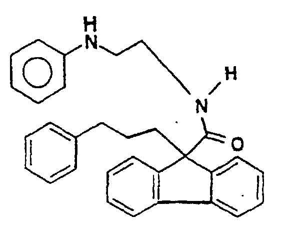

MS (electrospray, pos. ions) 447 (M+H)

### Example 52

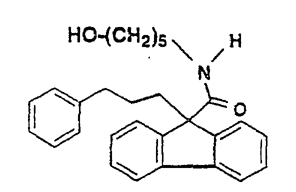

MS (Cl, + ions) m/z 414 (M+H)

| | | | |
|---|---|---|---|
| Anal. Cald'd for C₂₈H₃₁NO₂ + 0.1 CH₂Cl₂ | C, 79.97; | H, 7.45; | N, 3.32 |
| Found | C, 80.29; | H, 7.57; | N, 3.27. |

### Example 53

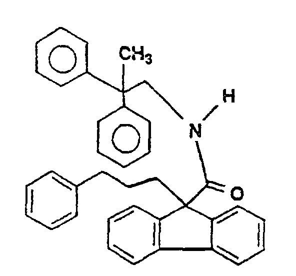

MS (electrospray, pos. ions) 458 (M+H)

### Example 54

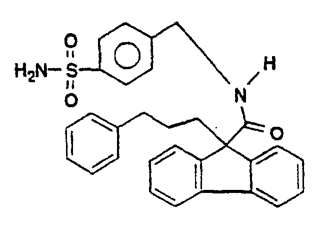

MS (electrospray, pos. ions) 497

### Example 55

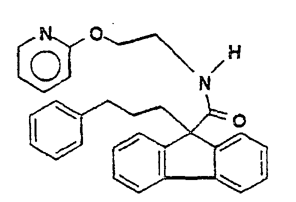

MS (electrospray, pos. ions) 449 (M+H)

### Example 56

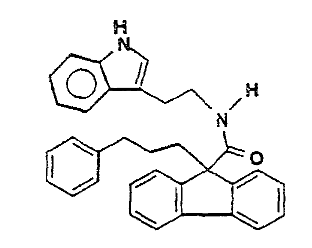

MS (electrospray, pos. ions) 471 (M+H)

### Example 57

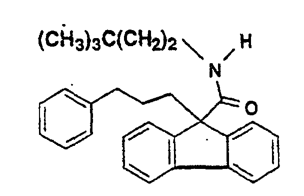

MS (electrospray, pos. ions) 412 (M+H)

### Example 58

### 9-(3-Phenylpropyl)-N-(2,2,2-trifluoroethyl)-9H-fluorene-9-carboxamide

A solution of oxalyl chloride in dichloromethane (1 mL, 2.0 mmol) was added to a stirred suspension of Example 22 Part A compound (0.30 g 0.90 mmol) in 5 mL of dichloromethane. The reaction mass was treated with 1 drop of DMF, allowed to stir for 2 h and concentrated. The remainder was diluted with 10 mL of THF, cooled to -40° and treated with 2,2,2-trifluoroethylamine (0.44 g, 7.5 mmol) and warmed to RT over 3 h. The reaction mixture was diluted with 20 mL of water and 50 mL of ethyl acetate. The organic fraction was extracted with 15 mL of 1 M KOH, dried (MgSO₄) and concentrated. The remainder was purified by column chromatography on silica gel (50 g) with hexanes (100 mL) followed by 2:8 ethyl acetate/hexane (300 mL) to give 0.28 g (88%) of title compound as a white solid. The resulting solid was recrystalized from 1.5 mL of a 10:1 ethanol/water solution to give 0.19 g (52%) of title compound as needles.mp 86-88°C.
TLC Silica gel (3:7 ethyl acetate/hexane) R_{f}= 0.7.
Mass Spec. (ES, + ions) m/z 410 (M+H).

| | | | |
|---|---|---|---|
| Anal. Calc'd for C₂₅H₂₂NOF₃ | C, 73.34; | H, 5.42; | N, 3.42 |
| Found | C, 72.98; | H, 4.94; | N, 3.35. |

### Example 59

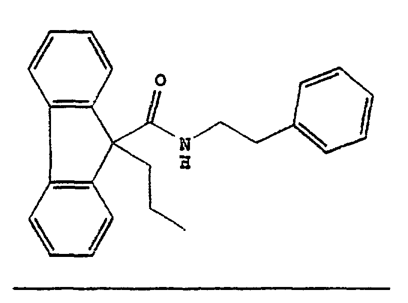

### A.

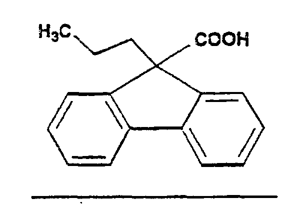

A solution of (9H)-9-fluorenecarboxylic acid (12 g, 57 mmol) in 250 ml of THF was cooled to 0°C under an argon atmosphere and 2 equiv. (71.25 ml) of a 1.6 M n-butyl lithium solution in hexane was added followed by the addition of n-propyl iodide (7.5 ml, 13.1 g, 77 mmol). The reaction mixture was stirred at 0°C for 6 hrs. TLC, silica, MeOH:CH₂Cl₂ (1:9) showed starting acid still present, therefore, an additional 1 ml of n-propyl iodide was added and the reaction stirred for 4 hrs at 0°C. The reaction was quenched by adding 75 ml of water and the pH was adjusted to pH 1 with 3 N HCl. The reaction mixture was extracted with hexane (3x200ml) and the hexane extract washed with water, brine and dried over anhy. sodium sulfate. The solvents were evaporated yielding the crude product as a yellow oil which was dissolved in ~250 ml of ethanol and heated at reflux with Darco G-60, filtered through Celite and concentrated to approximately one half of the original volume. Water was slowly added until the mixture became cloudy. The mixture was reheated and slowly allowed to cool to room temperature yielding 10.5 grams (73%) of title compound as colorless crystals. m.p.120-122°C.

| | | |
|---|---|---|
| Anal Calc'd for C₁₇H₁₆O₂ (MW 252.3) | C, 80.93; | H, 6.39 |
| Found | C, 81.01; | H, 6.22. |

### B.

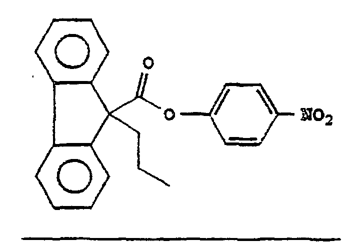

Example 59 Part B was prepared analogously to Example 22 Part B starting with Example 59 Part A (1.5 g, 5.95 mmol), 4.5 mL (8.92 mmol) of oxalyl chloride, 6 drops (catalytic) of dimethylformamide, 2.5 g (17.8 mmol) of 4-nitrophenol, and 1 mL (7.14 mmol) of triethylamine.

### C.

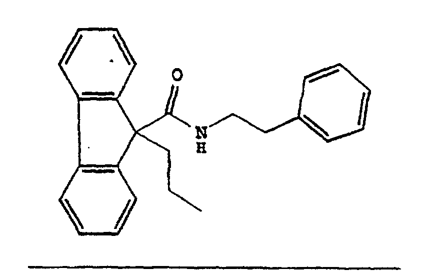

Example 59 compound was prepared via an automated procedure carried out on a Zymark Benchmate® Workstation using the following procedure.

The Benchmate® delivered 1 mL (44 mg, 0.11 mmol, 1 eq) of a stock solution of Example 59 Part B in THF (44 mg/mL) to a 16 mm x 100 mm culture tube. The tube was removed and placed on a balance where phenethyl amine (24 mg, 0.17 mmol) was added manually. The reaction was allowed to proceed until all reactions in the run were complete as indicated by disappearance of Example 59 Part B compound by TLC (silica gel, 2% MeOH in CH₂Cl₂, visualized by UV and I₂).

The product was purified in an analogous manner to Example 22, Part C, to give title compound as a colorless solid in 81% yield. MS (electrospray, + ions) m/z 356 (M+H).

### Example 66

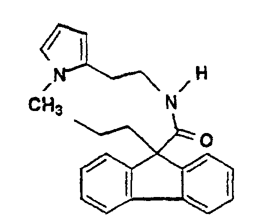

MS (electrospray, pos. ions) 359 (M+H)

### Example 67

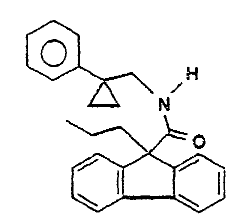

MS (electrospray, pos. ions) 382 (M+H)

### Example 68

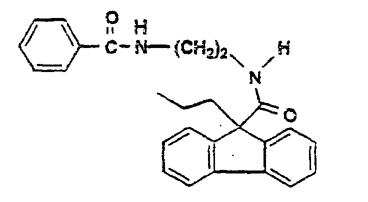

MS (electrospray, pos. ions) 399 (M+H)

### Example 69

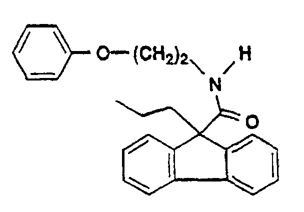

MS (electrospray, pos. ions) 372 (M+H)

### Example 70

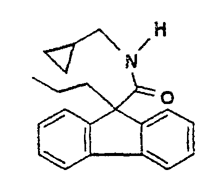

MS (electrospray, pos. ions) 306 (M+H)

### Example 71

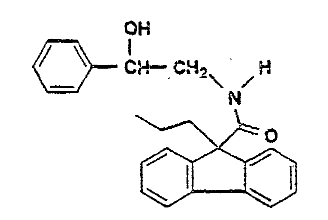

MS (electrospray, pos. ions) 372 (M+H)

### Example 72

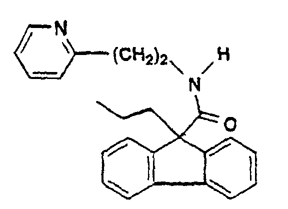

MS (electrospray, pos. ions) 357 (M+H)

### Example 73

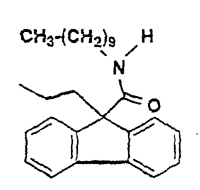

MS (electrospray, pos. ions) 392 (M+H)

### Example 74

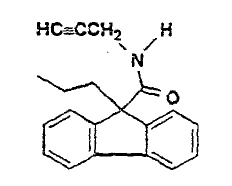

MS (electrospray, pos. ions) 291 (M+H)

### Example 75

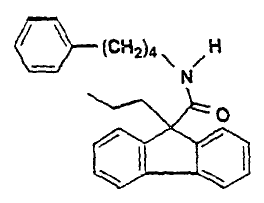

MS (electrospray, pos. ions) 384 (M+H)

### Example 76

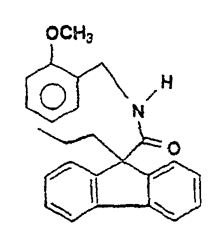

MS (electrospray, pos. ions) 372 (M+H)

### Example 77

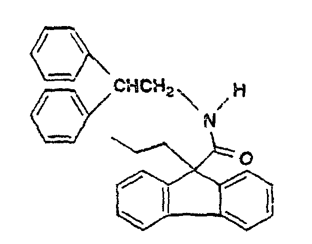

MS (electrospray, pos. ions) 432 (M+H)

### Example 78

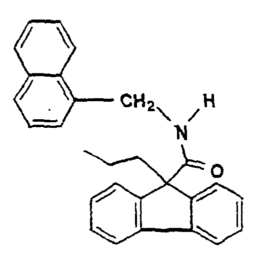

MS (electrospray, pos. ions) 392 (M+H)

### Example 79

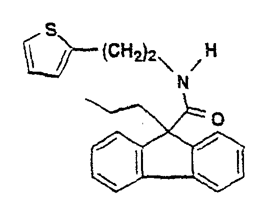

MS (electrospray, pos. ions) 362 (M+H)

### Example 80

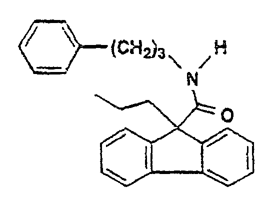

MS (electrospray, pos. ions) 370 (M+H)

### Example 81

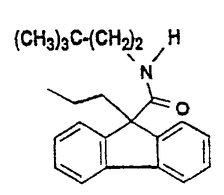

MS (electrospray, pos. ions) 336 (M+H)

### Example 82

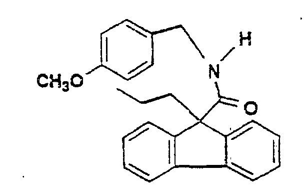

MS (electrospray, pos. ions) 372 (M+H)

### Example 83

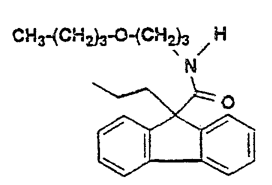

MS (electrospray, pos. ions) 366 (M+H)

### Example 84

### N-Methyl-N-(phenylmethyl)-9-propyl-9H-fluorene-9-carboxamide

### A.

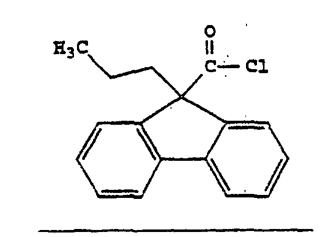

A solution of Example 59 Part A compound (2.02 g, 8 mmol) in 15 ml of dry dichloromethane was cooled to 0°C under an argon atmosphere. N,N-Dimethylform-amide (50µl) was added to the reaction mixture followed by the addition of oxalyl chloride (0.77 ml, 1.12 g, 8.8 mmol) over a 10 minute period. After stirring for 15 min at 0°C the reaction was allowed to warm to room temperature and stir for 1 hr. The volatiles were removed under vacuum and the oily residue was redissolved several times in dichloro-methane and evaporated yielding the title acid chloride as a colorless solid which was used without any further purification.

### B. N-Methyl-N-(phenylmethyl)-9-propyl-9H-fluorene-9-carboxamide

A solution of Example 84 Part A compound (1 mmol) in 8 ml of dry THF was cooled to 0°C under an argon atmosphere and 2.1 equiv. of N-methyl-N-benzylamine (255 mg, 2.1 mmol) was added. After stirring at ambient temperature for 2 hrs. the reaction was diluted with 25 ml of ethyl acetate and washed with sat. sodium bicarbonate solution. The ethyl acetate extract was washed with sodium bicarbonate, water, brine and dried over anhy. sodium sulfate. The crude product was purified by flash chromatography on Merck EM silica gel eluting with 5% EtOAc/hexane yielding 186 mg (53%) of pure title product as a colorless solid. m.p. 73-74°C.

| | | | |
|---|---|---|---|
| Anal Calc'd for C₂₅H₂₅NO (FW 355.48) | C, 84.47; | H, 7.09; | N, 3.94 |
| Found | C, 84.57; | H, 7.16; | N, 3.90. |

### Examples 85 to 92

Examples 85 to 92 can be prepared from Example 84 Part A compound by the method in Example 84, Part B.

### Example 85

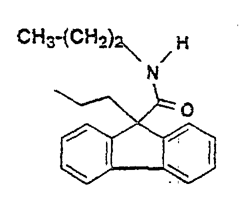

M.P. 96-98°C
Mass Spec. (CI) (M+H)⁺=308⁺

| | | | |
|---|---|---|---|
| Anal. Cald'd for C₂₁H₂₅NO | C, 82.04; | H, 8.20; | N, 4.56 |
| Found | C, 82.06; | H, 8.46; | N, 4.48. |

### Example 86

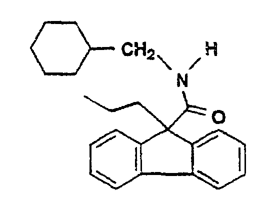

M.P. 106-107°C
Mass Spec. (CI) (M+H)⁺=348

| | | | |
|---|---|---|---|
| Anal. Cald'd for C₂₄H₂₉NO | C, 82.95; | H, 8.41; | N, 4.03 |
| Found | C, 82.71; | H, 8.22; | N, 3.82. |

### Example 87

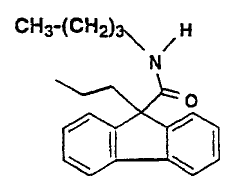

M.P. 60-62°C
Mass Spec. (CI) (M+H)=308

| | | | |
|---|---|---|---|
| Anal. Cald'd for C₂₁H₂₅NO | C, 82.04; | H, 8.20; | N, 4.56 |
| Found | C, 82.09; | H, 8.35; | N, 4.42. |

### Example 88

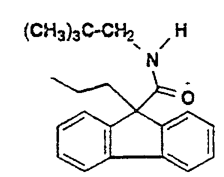

M. P. 62-64°C
Mass Spec. (CI) (M+H) = 322

| | | | |
|---|---|---|---|
| Anal. Cald'd for C₂₂H₂₇NO | C, 82.20; | H, 8.47; | N, 4.36 |
| Found | C, 81.86; | H, 8.19; | N, 4.41. |

### Example 89

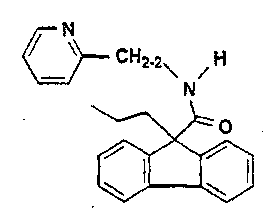

M.P. 102-103°C
Mass Spec. (CI) (M+H) = 343

| | | | |
|---|---|---|---|
| Anal. Cald'd for C₂₃H₂₂N₂O | C, 80.67; | H, 6.48; | N, 8.18 |
| Found | C, 80.51; | H, 6.46; | N, 8.04. |

### Example 90

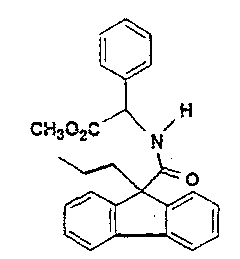

Mass Spec. (CI) (M+H) = 400

| | | | |
|---|---|---|---|
| Anal. Cald'd for C₂₆H₂₅NO₃ + 0.1 H₂O | C, 77.87; | H, 6.33; | N, 3.49 |
| Found | C, 77.87; | H, 6.35; | N, 3.53. |

### Example 91

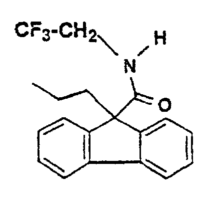

M.P. 113-115°C
MS (CI, + ions) m/z 334 (M+H)

| | | | | |
|---|---|---|---|---|
| Anal. Cald'd for C₁₉H₁₈NOF₃ | C, 68.46; | H, 5.44; | N, 4.20; | F, 17.10 |
| Found | C, 68.24; | H, 5.70; | N, 4.18; | F, 17.22. |

### Example 92

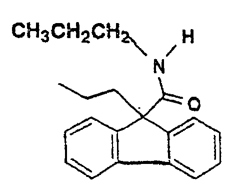

M.P. 75-77°C
MS (CI, + ions) m/z 294 (M+H)

| | | | |
|---|---|---|---|
| Anal. Cald'd for C₂₀H₂₃NO | C, 81.87; | H, 7.90; | N, 4.77 |
| Found | C, 81.88; | H, 8.18; | N, 4.70. |

### Example 93

### 9-(2-Propenyl)-N-(2-pyridinylmethyl)-9H-fluorene-9-carboxamide

### A.

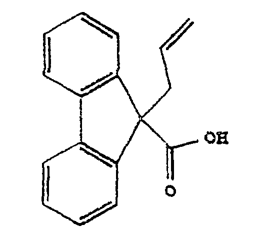

To a methoxyethanol solution (100 ml) of 9H-fluorene-9-carboxylic acid (10.83 g, 0.0515 mol) under argon was added solid KOH (6.8 g, 0.103 mol). After about 15 min the KOH had dissolved resulting in a blue-green colored solution. Allyl bromide (8.9 ml, 0.526 mol) was then added and stirred at room temperature for 2 h. The reaction mixture was partitioned between EtOAc/H₂O and the aqueous layer extracted twice with EtOAc. The aqueous layer was brought to pH 2 with 1N HCl, extracted twice with EtOAc, and the combined organics were dried over Na₂SO₄. Evaporation *in vacuo* gave 11.63 g of a brown colored oily-solid. The residue was co-evaporated with CH₂Cl₂, Et₂O, EtOAc, and hexanes to give an orange colored solid 9.19 g (70% recovery). A portion of the material (400 mg) was purified by flash chromatography (twice, 3x13 cm), eluting with 3%MeOH:CH₂Cl₂ to give title compound as a colorless solid (160 mg).
m.p. 128-130°C.
MS: (CI, M+NH₄⁺): m/z 268.

| | | |
|---|---|---|
| Anal. Calc. for C₁₇H₁₄O₂ • 0.13 H₂O | C, 80.80; | H, 5.69 |
| Found | C, 80.80; | H, 5.61. |

### Alternative Preparation of Part A Compound

To a THF (15 ml) supension of 9-fluorene carboxylic acid (5.28 g, 0.025 mol) at 0°C under argon was added sodium hexamethyldisilizane (50 mL, 0.05 mol, 1M in THF), initial solid formation, and the final greenish-brown solution stirred for 5 min.. Allyl bromide (2.3 mL, 0.0265 mol) was added and after 1 h the mixture was poured into cold water. The aqueous layer was extracted with EtOAc and the organic layer washed with water. The combined aqueous layers were brought to pH 1 with 3N HCl and extracted with EtOAc. The organics were washed with brine, dried over Na₂SO₄, and the volatiles removed *in vacuo* to give an oily-solid residue (6.96 g). The residue was crystallized from EtOH/water to give 2.81 g colorless solid. After concentrating the mother liquor, a second crop (1.04 g) and third crop (0.5 g) were obtained of Part A compound (4.35 g, 69% yield). mp 128-130°C.

### B.

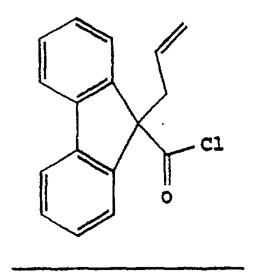

To a CH₂Cl₂ (40 ml) solution of Part A compound (3.83 g, 0.015 mol) at 0°C under argon was added oxalyl chloride (2 ml, 0.023 mol) then DMF (90 µL). After 15 min. at 0°C and 1.5 h at room temperature, the volatiles were removed *in vacuo* and the residue co-evaporated with CH₂Cl₂ to give title compound, which was used directly.

### C. 9-(2-Propenyl)-N-(2-pyridinylmethyl)-9H-fluorene-9-carboxamide

To a THF (35 ml) solution of Part B acid chloride (0.015 mol) at -5°C under argon was added 2-(aminomethyl)pyridine (3.4 mL, 0.033 mol), with extra THF (10 mL) added to improve stirring. After 15 min, the mixture was brought to room temperature for 4 h. At 0°C, the reaction mixture was quenched with saturated NaHCO₃, the aqueous layer extracted 3 times with EtOAc, the combined organic layers were washed with H₂O, brine and dried over Na₂SO₄. The volatiles were removed *in vacuo* to give a colored solid (5.1 g). The residue was purified by flash column chromatography (SiO₂, 10 by 20 cm), eluting with 2.5% MeOH:CH₂Cl₂, to give title compound (2.67 g, 51% yield) as a colorless solid.
m.p. 110-111°C.
MS: (CI, (M+H)⁺): 341 m/z.

| | | | |
|---|---|---|---|
| Anal. Calc. for C₂₃H₂₀N₂O | C, 81.15; | H,. 5.92; | N, 8.23 |
| Found | C, 80.95; | H, 5.99; | N, 8.21. |

### Examples 94 to 102

Example 94 to 102 can be prepared from Example 93 Part B compound by the method in Example 93 Part C.

### Example 94

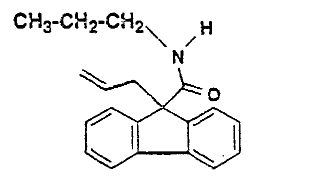

mp 85.5-86.5°C
MS (CI, (M+H)⁺) m/z 292

| | | | |
|---|---|---|---|
| Anal. Cald'd for C₂₀H₂₁NO | C, 82.44; | H, 7.26; | N, 4.81 |
| Found | C, 82.31; | H, 7.44; | N, 4.77. |

### Example 95

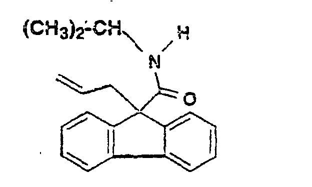

mp 74-75.5°C.
MS (CI, (M+H)⁺) m/z 292 •

| | | | |
|---|---|---|---|
| Anal. Cald'd for C₂₀H₂₁NO•0.09 H₂O | C, 81.98; | H, 7.29; | N, 4.78 |
| Found | C, 82.02; | H, 7.33; | N, 4.74. |

### Example 96

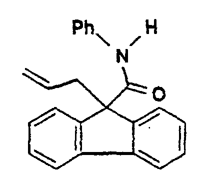

mp 112.5-114°C
MS (CI, (M+H)⁺) m/z 326

| | | | |
|---|---|---|---|
| Anal. Cald'd for C₂₃H₁₉NO•0.12 H₂O | C, 84.32; | H, 5.92; | N, 4.27 |
| Found | C, 84.35; | H, 5.76; | N, 4.24. |

### Example 97

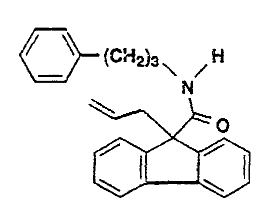

mp 74.5-75.5°C
MS (CI, (M+H)⁺) m/z 368

| | | | |
|---|---|---|---|
| Anal. Cald'd for C₂₆H₂₅NO•0.13 H₂O | C, 84.42; | H, 6.88; | N, 3.79 |
| Found | C, 84.48; | H, 6.84; | N, 3.73. |

### Example 98

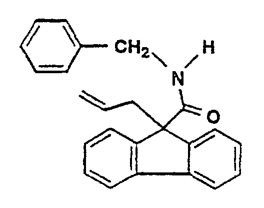

mp 80.5-81.5°C
MS (CI, (M+H)⁺) m/z 340

| | | | |
|---|---|---|---|
| Anal. Cald'd for C₂₄H₂₁NO | C, 84.92; | H, 6.24; | N, 4.13 |
| Found | C, 84.58; | H, 6.15; | N, 4.10. |

### Example 99

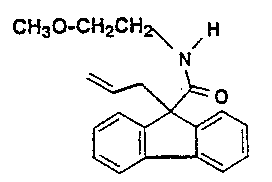

mp 87-88.5°C
MS (CI, (M+H)⁺) m/z 308

| | | | |
|---|---|---|---|
| Anal. Cald'd for C₂₀H₂₁NO₂ | C, 78.15; | H, 6.89; | N, 4.56 |
| Found | C, 78.05; | H, 6.83; | N, 4.47. |

### Example 100

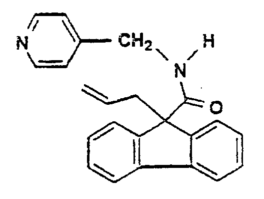

mp 127-128°C
MS (CI, (M+H)⁺) m/z 341

| | | | |
|---|---|---|---|
| Anal. Cald'd for C₂₃H₂₀N₂O | C, 81.15; | H, 5.92; | N, 8.23 |
| Found | C, 81.27; | H, 5.88; | N, 8.11. |

### Example 101

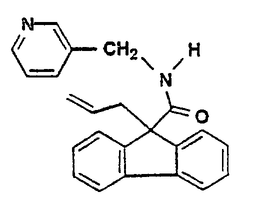

mp 68-71°C
MS (CI, (M+H)⁺) m/z 341

| | | | |
|---|---|---|---|
| Anal. Cald'd for C₂₃H₂₀N₂O | C, 81.15; | H, 5.92; | N, 8.23 |
| Found | C, 81.11; | H, 5.86; | N, 8.12. |

### Example 102

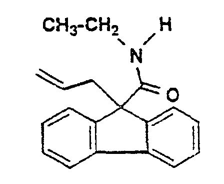

mp 87.5-88.5°C

| | | | |
|---|---|---|---|
| Anal. Cald'd for C₁₉H₁₉NO•0.13 H₂O | C, 81.57; | H, 6.94; | N, 5.01 |
| Found | C, 81.58; | H, 6.79; | N, 5.00. |

### Example 103

### 9-(1-Piperidinylcarbonyl)-9-(2-propenyl)-9H-fluorene

To a 0°C suspension under argon of Example 93 Part A compound (0.495 g, 1.98 mmol), piperidine (0.39 ml, 3.94 mmol), hydroxybenzotriazole hydrate (0.40 g, 2.96 mmol), and N-methylmorpholine (0.22 ml, 2.00 mmol) in DMF (6 ml) was added EDCI (0.44 g, 2.27 mmol) and the reaction was allowed to come to room temperature overnight. After 24 h, the reaction was quenched with saturated NaHCO₃, the aqueous layer extracted twice with EtOAc, and the combined organics dried over Na₂SO₄ overnight. The volatiles were removed *in vacuo* to give an oil (600 mg). The residue was purified by flash column chromatography (SiO₂, 3 by 17 cm), eluting with CH₂Cl₂ to give title compound (0.265 g, 42% yield) as a colorless solid. m.p. 64-66°C.
MS: (CI, + ions): m/z 318 (M+H).

| | | | |
|---|---|---|---|
| Anal. Calc. for C₂₂H₂₃NO | C, 83.24; | H, 7.30; | N, 4.41 |
| Found | C, 83.25; | H, 7.32; | N, 4.36. |

### Example 104

### N-Butyl-9-(2-propenyl)-9H-fluorene-9-carboxamide

To a CH₂Cl₂ (8 ml) and pyridine (0.28 ml) solution of Example 93 Part A compound (400 mg, 1.60 mmol) under argon was added cyanuric fluoride (0.27 mL, 3.20 mmol). After 1.5 h, the cloudy reaction mixture was partitioned between ice-water and CH₂Cl₂. The organics were dried over Na₂SO₄, and the volatiles removed *in vacuo* to give an oily-solid residue (420 mg). The crude residue was used directly in the subsequent reaction.

To a THF (7 ml) solution of the above crude residue (1.5 mmol) at 0°C under argon was added n-butylamine (0.3 mL, 3.04 mmol) and the reaction brought to room temperature. After 16 h, the mixture was quenched with saturated NaHCO₃, the aqueous layer extracted 2 times with EtOAc, and the combined organic layers were washed with brine and dried over Na₂SO₄. The volatiles were removed in vacuo to give an oily-solid (470 mg). The residue was purified by flash column chromatography (SiO₂, 5 by 6 cm), eluting with 12.5% EtOAc:hexanes, to give title compound (362 mg, 79% yield) as a colorless solid. m.p. 62.5-64°C.
MS: (CI, M+H⁺): m/z 306.

| | | | |
|---|---|---|---|
| Anal. Calc. for C₂₁H₂₃NO | C, 82.59; | H, 7.59; | N, 4.59 |
| Found | C, 82.72; | H, 7.45; | N, 4.46. |

### Example 105

### 9-[[2,2-Bis(trifluoromethyl)-1,3-dioxolan-4-yl]-methyl-N-ethyl-9H-fluorene-9-carboxamide

To a CH₂Cl₂ (0.5 ml) solution of Example 102 compound (35 mg, 0.125 mmol) and hexafluoroacetone hydrate (40 mg, 0.207 mmol) was added 30% H₂O₂ (25 µl). After several hours, MgSO₄ was added and the reaction stirred for 24 h, when a second amount of the ketone and 30% H₂O₂ were added. After 48 h total, the reaction was quenched with aqueous sodium thiosulfate and sat. NaHCO₃. The aqueous layer was extracted twice with CH₂Cl₂ and the combined organics were dried over Na₂SO₄. The organics were concentrated *in vacuo* and the residue was purified by flash column chromatography (SiO₂, 2 by 6 cm), eluting with 1% EtOAc: CH₂Cl₂, to give title compound (20 mg, 34% yield) as a colorless solid. m.p. 91-93°C.
MS: (CI, M+H⁺): m/z 460.

| | | | |
|---|---|---|---|
| Anal. Calc. for C₂₂H₁₉F₆NO₃ | C, 57.52; | H, 4.17; | N, 3.05 |
| Found | C, 57.51; | H, 4.00; | N, 2.93. |

### Example 106

### 9-(2,3-Dihydroxypropyl)-N-ethyl-9H-fluorene-9-carboxamide

To an acetone:H₂O (4 ml, 9:1) suspension of Example 102 compound (191 mg, 0.689 mmol) and N-methylmorpholine-N-oxide ( 215 mg, 1.59 mmol) under argon was added OsO₄ (several small crystals). After stirring at room temperature overnight, the reaction was cooled and then quenched with aq. sodium metabisulfite. The reaction mixture was stirred 15 min. and the aqueous layer extracted twice with EtOAc. The organics were washed with brine, dried over Na₂SO₄, and concentrated to an oil (220 mg). The residue was purified by flash column chromatography (SiO₂, 3 by 9 cm), eluting with 4:1 EtOAc:CH₂Cl₂, to give title compound (106 mg, 49% yield) as a colorless, hygroscopic foam.
MS: (CI, M+H⁺): m/z 312.

| | | | |
|---|---|---|---|
| Anal. Calc. for C₁₉H₂₁NO₃ • 0.4 H₂O | C, 71.64; | H, 6.90; | N, 4.40 |
| Found | C, 71.68; | H, 6.84; | N, 4.36. |

### Example 107

### 9-(3-Phenylpropyl)-N-(3-hydroxy)propyl-9H-xanthene-9-carboxamide

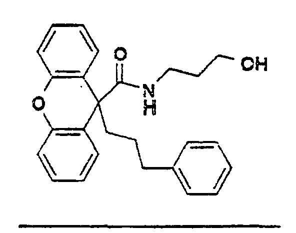

### A. 9-(3-Phenylpropyl)-9H-xanthene-9-carboxylic acid

To a solution of 10 g (44 mmol, 1 eq) of 9-xanthenylcarboxylic acid in 200 mL of THF at 0°C was added 37.2 mL (93 mmol, 2.1 eq) of a 2.5 M solution of n-butyllithium in hexanes dropwise over 15 min. (First equivalent resulted in precipitation of Li salt of the carboxylate; solution became homogeneous as dianion formed.) The resulting orange solution of dianion was stirred at 0°C for 10 min and 9.4 mL (62 mmol, 1.4 eq) of 1-bromo-3-phenylpropane was added quickly over 3 min. The reaction was stirred at 0°C and allowed to warm to RT as the ice bath melted. After 16 h, the basic reaction mixture (pH -14) was extracted with water (3 x 100 mL). The combined aqueous layers were acidified (to pH ~1) with 6 N HCl and extracted with ether (3 x 100 mL). The combined ether solutions were dried (MgSO₄), filtered and concentrated to afford 17.04 g of a viscous golden oil. The oil was dissolved in hot hexanes using a small amount of CH₂Cl₂ to effect complete dissolution. Concentration of this solution resulted in a yellow solid which was recrystallized from ether/hexanes to afford' 13.3 g (88%) of title compound as a white crystalline solid,
m.p. 137-138°C.
TLC (silica gel, 10% MeOH in CH₂Cl₂, UV and I₂)
R_{f} = 0.52.

### B. 9-(3-Phenylpropyl)-9H-xanthene-9-carboxylic acid, 4-nitrophenyl ester

To a solution of 10 g (29.0 mmol, 1 eq) of Part A compound in 100 mL of CH₂Cl₂ was added 100 µL of DMF. The solution was cooled to 0°C and 22.0 mL (43.6 mmol, 1.5 eq) of a 2.0 M oxalyl chloride solution in CH₂Cl₂ was added over 5 min. The resulting bubbling solution was stirred at 0°C for 1.5 h (until bubbling had ceased). The solution was concentrated and the residual oil was taken up in 50 mL of CH₂Cl₂ and reconcentrated. The resulting oil was dissolved in 150 mL of CH₂Cl₂ and 188 mg (1.52 mmol, 0.05 eq) of 4-dimethylaminopyridine was added. The solution was cooled to 0°C and 4.9 mL (34.8 mmol, 1.2 eq) of triethylamine was added. To the resulting dark brown cloudy solution was added 12.1 g (87.1 mmol, 3 eq) of p-nitrophenol as a solid. Upon addition the reaction quickly became clear and the resulting clear reaction mixture was allowed to warm to RT as the ice bath melted. (TLC indicated the reaction was essentially complete after 40 min.) After 15 h, the reaction was washed with 100 mL of ice-cold 1 N HCl. The organic solution was filtered through cotton and concentrated to afford 24.22 g of a viscous golden-brown oil which was chromatographed on silica gel (200 g) eluted with 25% hexanes in CH₂Cl₂ to afford 13.45 g of a viscous golden oil. The product was cystallized by concentrating down a ether/hexane solution and the crude solid was then recrystallized from ether/hexanes to afford 11.8 g (87%) of title compound as an off-white crystalline solid, m.p. 93-94°C.
TLC (silica gel, 25% EtOAc in hexanes, UV and I₂)
R_{f} = 0.39.
MS(CI, pos. ions): *m*/*z* 483 (M + NH₄), 466 (M + H).

| | | | |
|---|---|---|---|
| Anal. Calcd. for C₂₉H₂₃NO₅ | C, 74.83; | H, 4.98; | N, 3.01 |
| Found | C, 74.61; | H, 4.71; | N, 2.88. |

### C. 9-(3-Phenylpropyl)-N-(3-hydroxy)propyl-9H-xanthene-9-carboxamide

The title compound was prepared via an automated procedure carried out on a Zymark Benchmate® Workstation using the following procedure.

The Benchmate® delivered 1 mL (80 mg, 0.18 mmol, 1 eq) of a stock solution of title compound in THF (80 mg/mL) to a 16 mm x 100 mm culture tube. The tube was removed and placed on a balance where 3-amino-1-propanol (24 mg, 0.27 mmol) was added manually. The reaction was allowed to proceed untiI all reactions in the run were complete as indicated by disappearance of title compound by TLC (silica gel, 2% MeOH in CH₂Cl₂, visualized by UV and I₂).

The product was purified in an analogous manner to Example 22, Part C, to give title compound as a pale oil (55 mg) in 69% yield.
MS (electrospray, pos. ions) = 402 (M+H).

### Examples 108-140

Examples 108 to 140 can be prepared from Example 107 Part B compound by the method in Example 107, Part C.

### Example 108

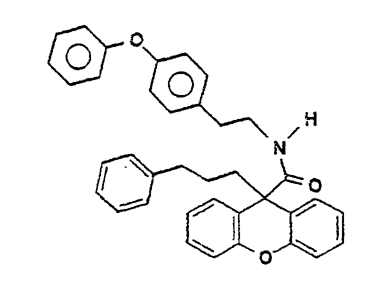

MS (CI, pos. ions) 540 (M+H)

### Example 109

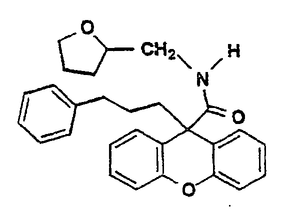

MS (CI, pos. ions) 428 (M+H)

### Example 110

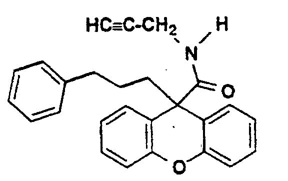

MS (CI, pos. ions) 382 (M+H)

### Example 111

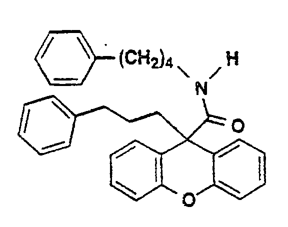

MS (CI, pos. ions) 476 (M+H)

### Example 112

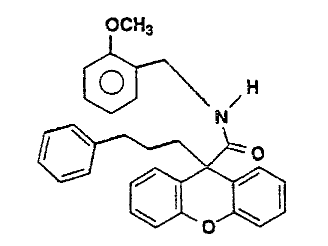

MS (CI, pos. ions) 464 (M+H)

### Example 113

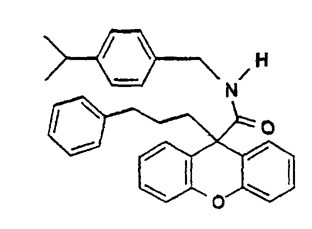

MS (CI, pos. ions) 476 (M+H).

### Example 114

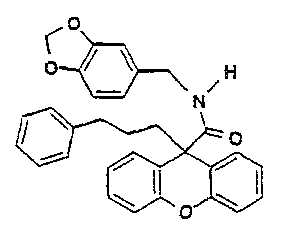

MS (electrospray, pos. ions) 478 (M+H).

### Example 115

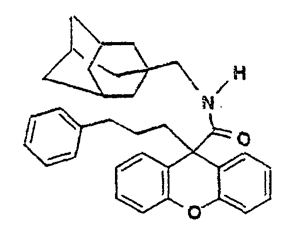

MS (electrospray, pos. ions) 492 (M+H).

### Example 116

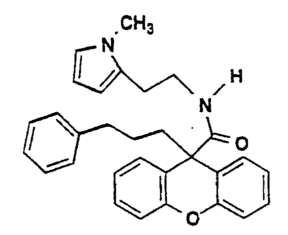

MS (electrospray, pos. ions) 451 (M+H).

### Example 117

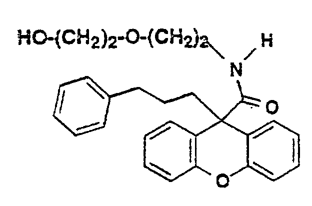

MS (electrospray, pos. ions) 432 (M+H).

### Example 118

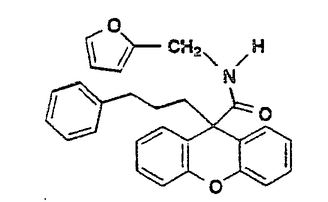

MS (electrospray, pos. ions) 424 (M+H).

### Example 119

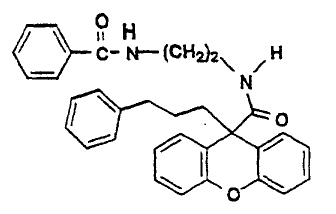

MS (electrospray, pos. ions) 491 (M+H).

### Example 120

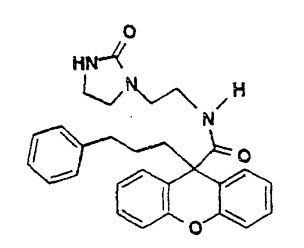

MS (electrospray, pos. ions) 456 (M+H).

### Example 121

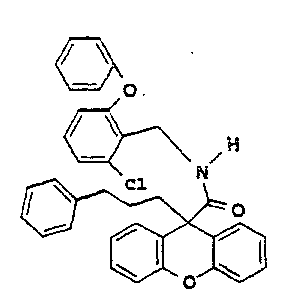

MS (electrospray, pos. ions) 560 (M+H).

### Example 122

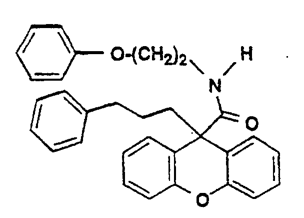

MS (electrospray, pos. ions) 464 (M+H).

### Example 123

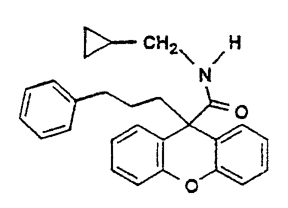

MS (electrospray, pos. ions) 398 (M+H).

### Example 124

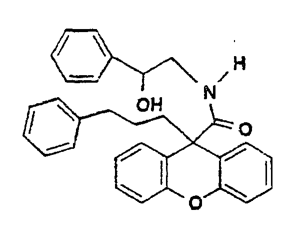

MS (electrospray, pos. ions) 464 (M+H).

### Example 125

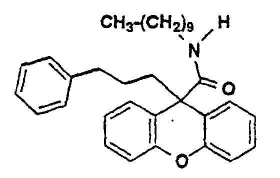

MS (electrospray, pos. ions) 484 (M+H).

### Example 126

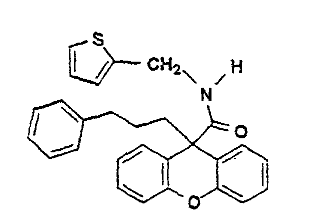

MS (electrospray, pos. ions) 440 (M+H).

### Example 127

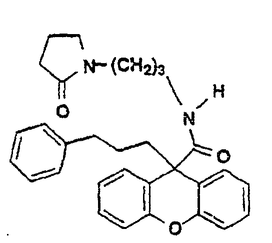

MS (electrospray, pos. ions) 469 (M+H).

### Example 128

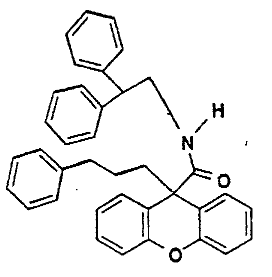

MS (electrospray, pos. ions) 524 (M+H)

### Example 129

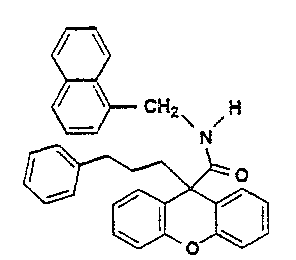

MS (electrospray, pos. ions) 484 (M+H).

### Example 130

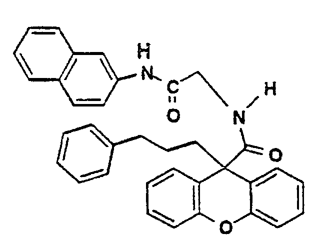

MS (electrospray, pos. ions) 527 (M+H).

### Example 131

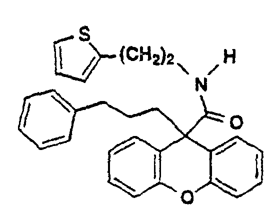

MS (electrospray, pos. ions) 454 (M+H).

### Example 132

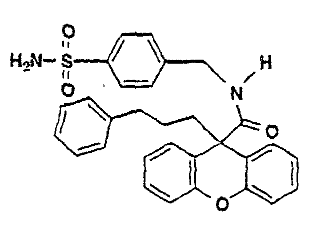

MS (electrospray, pos. ions) 513 (M+H)

### Example 133

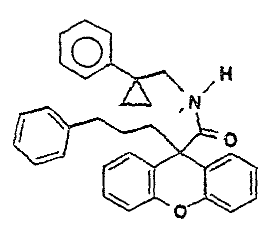

MS (electrospray, pos. ions) 474 (M+H).

### Example 134.

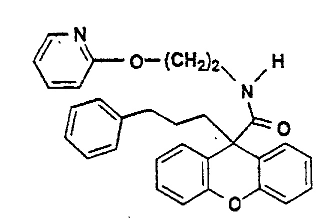

MS (electrospray, pos. ions) 465 (M+H).

### Example 135

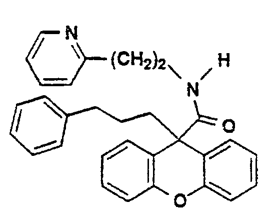

MS (electrospray, pos. ions) 449 (M+H).

### Example 136

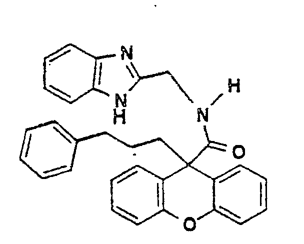

MS (electrospray, pos. ions) 474 (M+H).

### Example 137

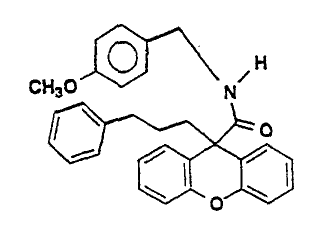

MS (electrospray, pos. ions) 464 (M+H).

### Example 138

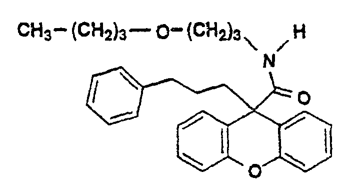

MS (electrospray, pos. ions) 458 (M+H).

### Example 139

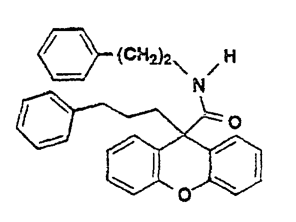

MS (electrospray, pos. ions) 448 (M+H).

### Example 140

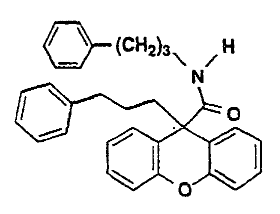

MS (electrospray, pos. ions) 462 (M+H).

### Example 141

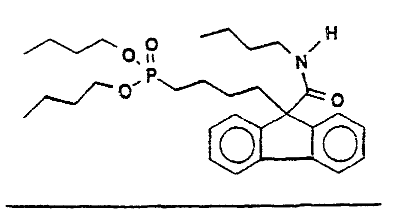

### A.

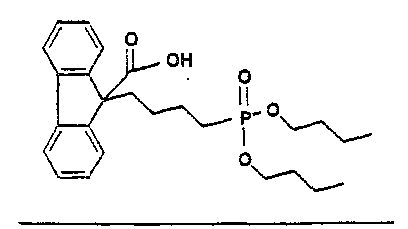

To a suspension of fluorene-(9H)-9-carboxylic acid (0.45 g, 2.18 mmol) in THF (5 mL) at -78°C was added n-butyllithium in hexanes (1.70 mL, 4.20 mmol) dropwise at such a rate to maintain the internal temperature below -40°C. The resulting bright yellow solution was stirred at - 40°C for 0.5 h and treated with compound Example 11, Part B (0.60 g, 1.82 mmol). The mixture was slowly warmed to room temperature and stirred for 6 h when the mixture was treated with 0.1 g (10 mol%) of tetrabutylammonium iodide and allowed to stir overnight. The mixture was diluted with 0.1N HCl (25 mL, 2.50 mmol) and ethyl acetate (50 mL). The layers were separated, the organic fraction dried (Na₂SO₄) and concentrated to give 1 g of crude oil. This material could be purified by flash chromatography (silica gel, eluting with 5% MeOH:ethyl acetate) and crystallization from hexane/ethyl acetate/methylene chloride to gave title compound as a colorless solid. mp 123-125°C.
TLC Silica gel (3:7:1 acetone/dichloromethane/acetic acid) R_{f}= 0.45.

### B.

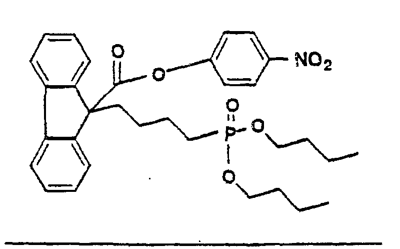

Part B compound was prepared as described for Example 22 Part B compound, using 7.59 g (16.5 mmol) of Example 144 Part A compound, 12.4 mL (24.9 mmol) of oxalyl chloride, 100 µL (catalytic) of dimethyl-formamide, 101 mg (0.8 mmol) of 4-dimethylamino-pyridine, 2.01 g (19.8 mmol) of triethylamine, and 6.91 g (49.6 mmol) of 4-nitrophenol in CH₂Cl₂ (ml). The crude product was purified by flash chromato-graphy on silica gel (400 g) eluted with methylene chloride (3 L), followed by 2% methanol in methylene chloride. The product was further purified flash chromatography on silica gel (150 g) eluted with 7:3 hexanes:ethyl acetate (3 L) followed by 6:4 hexanes:ethyl acetate (3 L), to provide 6.29 g (73%) of title compound, as a pale yellow oil.
TLC Silica gel (9:1 toluene:acetone, visualization by UV, I₂) R_{f} = 0.27.

### C.

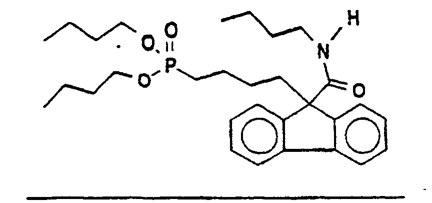

A solution of 104 mg (0.18 mmol) of Part B compound in 1 mL of THF was treated with 20 mg (0.36 mmol) of n-butylamine for 16 hours. The product was purified via solid phase extraction using a Varian SAX anion exchange column (1 g of sorbent, chloride form) by the procedure outlined below:
1) Column conditioned with 10 mL of 300 mM KOH(aq) in MeOH.
2) Column conditioned with 10 mL of MeOH.
3) Column conditioned with 10 mL of CH₂Cl₂ .
4) Reaction mixture loaded onto SAX column and effluent collected into a product tube.
5) Column rinsed with 1 mL of THF and effluent collected into product tube.
6) Column rinsed with 2 mL of CH₂Cl₂ and effluent collected into product tube.

This procedure was followed by a second solid phase extraction using a Varian SCX cation exchange column (1 mg of sorbent) by the procedure outlined below:
1) Column conditioned with 10 mL of CH₂Cl₂.
2) Reaction mixture loaded onto SCX column and effluent collected into product tube (tared).
3) Column rinsed with 2 mL of CH₂Cl₂ and effluent collected into product tube.

The product solution (approx. 5 mL) was concentrated using a speed vac for 14 h to afford 59 mg (63%) of title compound as a clear oil.
HPLC Purity = 90%; retention time = 13.0 minutes. Column: EM Lichropshere C8 Select-B 250 mm. Solvent A: 10% methanol:90% water:0.2% H₃PO₄. Solvent B: 90% methanol:10% water:0.2% H₃PO₄. Elution: Linear gradient from 30:70 A:B over 10 minutes followed by isocratic 100%B for 10 minutes.
MS (Electrospray, + ions): m/z 598 (M + H).

### Examples 142 to 185

Examples 142 to 175 can be prepared from Example 141 Part B compound by the method in Example 141 Part C. For examples where the starting amine is a salt, the amine was free based by partitioning between THF and aqueous saturated sodium bicarbonate or by adding an equimolar amount of triethylamine.
Note, Bu stands for n-butyl.

### Example 142

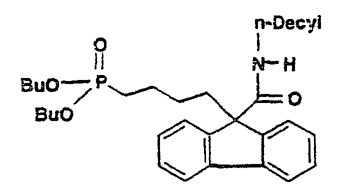

MS (ES, + ions) m/z 598 (M+H).

### Example 143

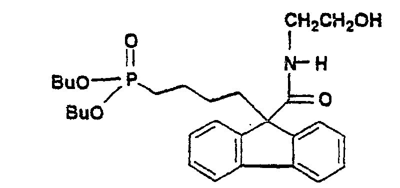

MS (ES, + ions) 501 (M+H).

### Example 144

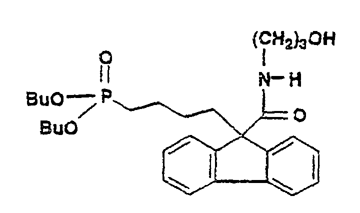

MS (ES, + ions) 516 (M+H).

### Example 145

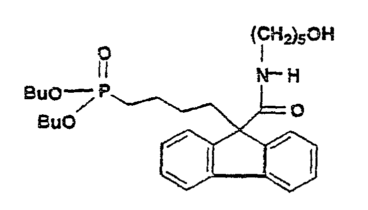

MS (ES, + ions) 544 (M+H).

### Example 146

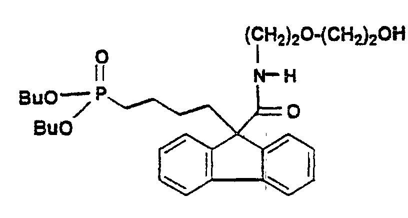

MS (ES, + ions) 546 (M+H).

### Example 147

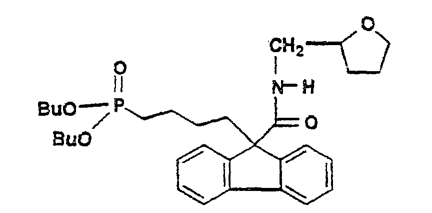

MS (ES, + ions) 542 (M+H).

### Example 148

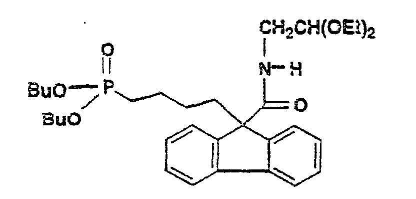

MS (ES, + ions) 596 (M+Na).

### Example 149

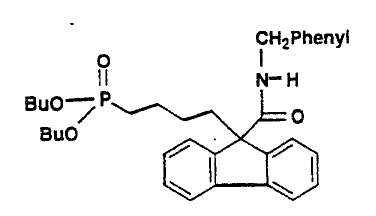

MS (ES, + ions) 548 (M+H).

### Example 150

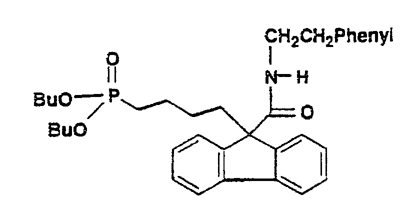

MS (ES, + ions) 562 (M+H).

### Example 151

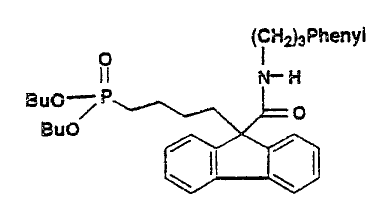

MS (ES, + ions) 576 (M+H).

### Example 152

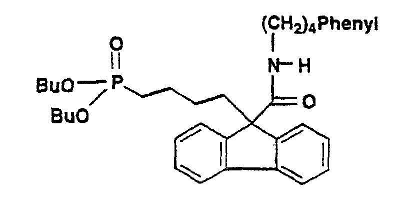

MS (ES, + ions) 590 (M+H).

### Example 153

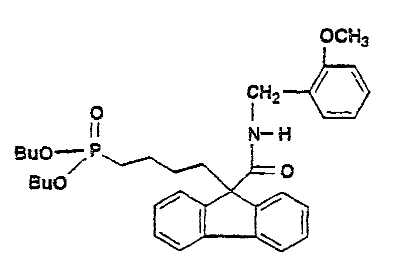

MS (ES, + ions) 578 (M+H).

### Example 154

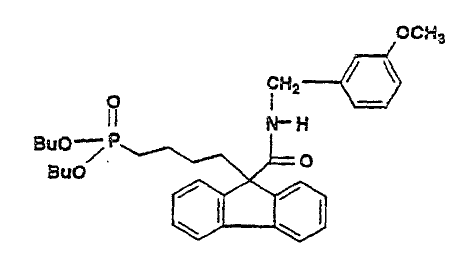

MS (ES, + ions) 578 (M+H).

### Example 155

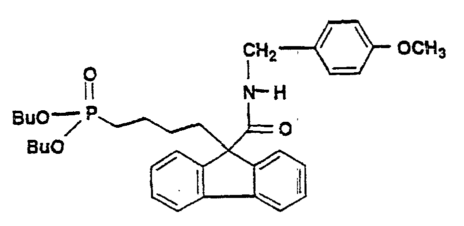

MS (ES, + ions) 578 (M+H).

### Example 156

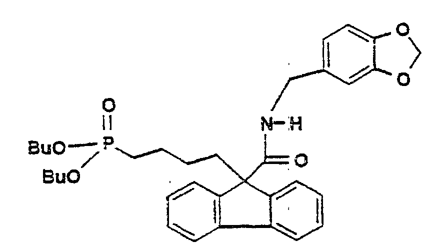

MS (ES, + ions) 592 (M+H).

### Example 157

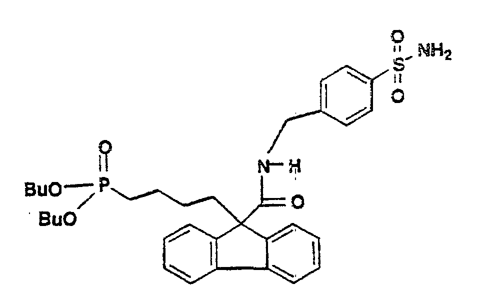

MS (ES, + ions) 627 (M+H).

### Example 158

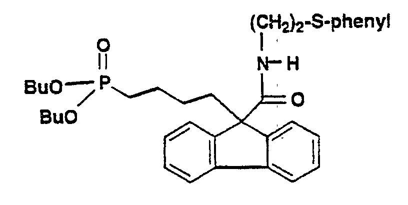

MS (ES, + ions) 594 (M+H).

### Example 159

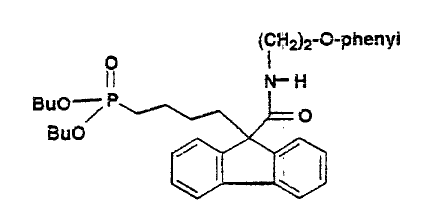

MS (ES, + ions) 578 (M+H).

### Example 160

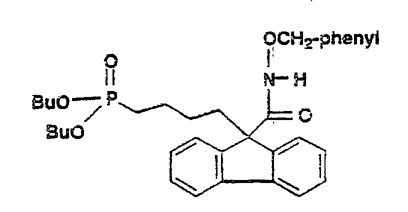

MS (ES, + ions) 564 (M+H).

### Example 161

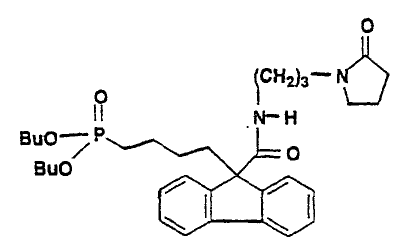

MS (ES, + ions) m/z 583 (M+H).

### Example 162

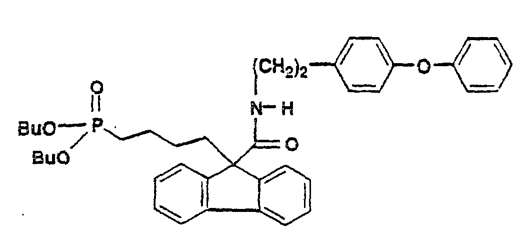

MS (ES, + ions) 654 (M+H).

### Example 163

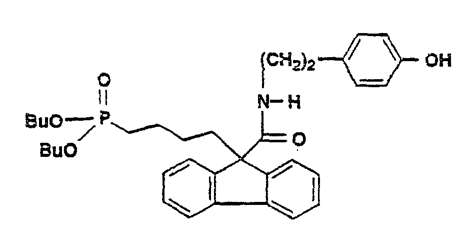

MS (ES, + ions) 578 (M+H).

### Example 164

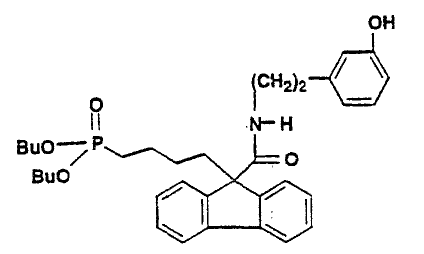

MS (ES, + ions) 578 (M+H).

### Example 165

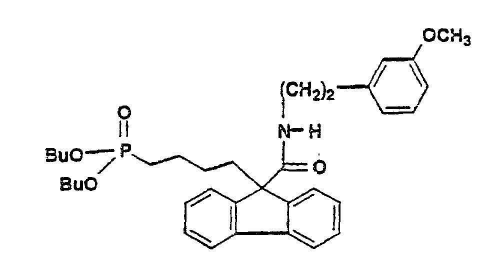

MS (ES, + ions) 592 (M+H).

### Example 166

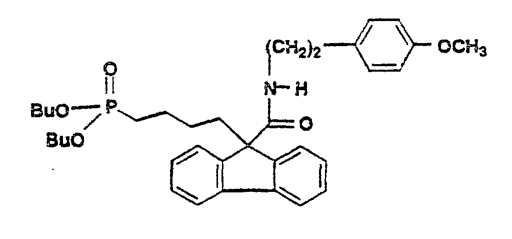

MS (ES, + ions) 592 (M+H).

### Example 167

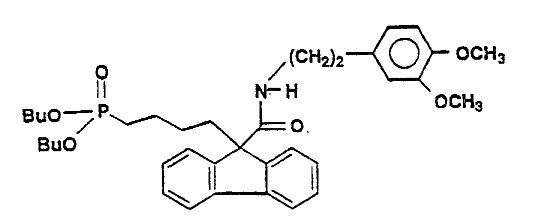

MS (ES, + ions) 622 (M+H).

### Example 168

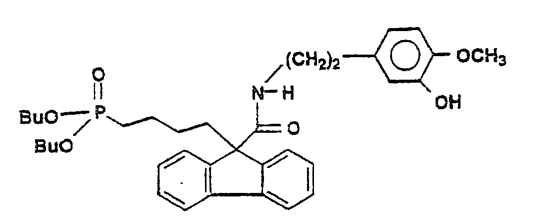

MS (ES, + ions) 608 (M+H).

### Example 169

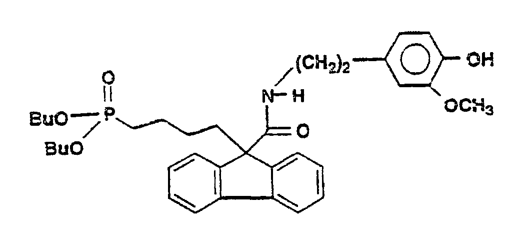

MS (ES, + ions) 608 (M+H).

### Example 170

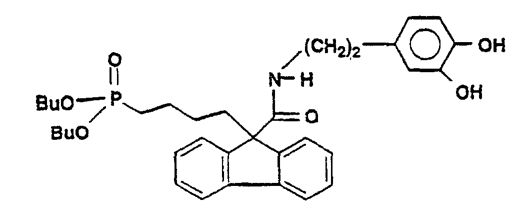

MS (ES, + ions) 594 (M+H).

### Example 171

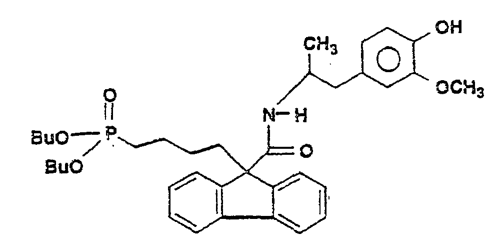

MS (ES, + ions) 622 (M+H).

### Example 172

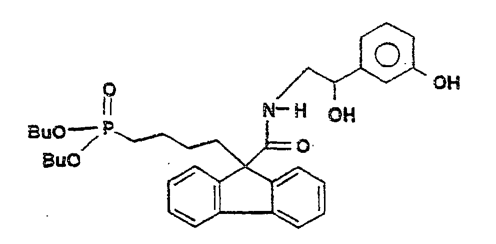

MS (ES, + ions) 594 (M+H).

### Example 173

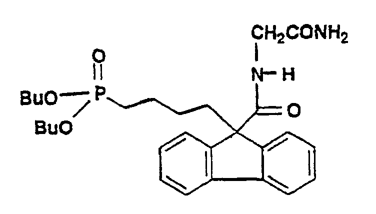

MS (ES, + ions) 515 (M+H).

### Example 174

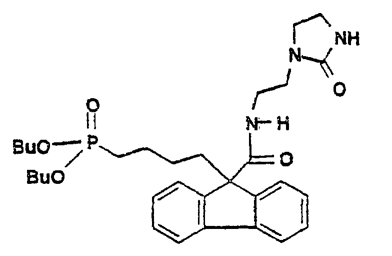

MS (ES, + ions) 570 (M+H).

### Example 175

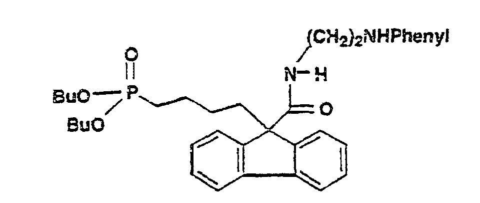

A solution of 104 mg (0.18 mmol) of Example 141 Part B compound in 1 mL of THF was treated with 22 mg (0.16 mmol, 0.9 eq) of N-phenethylaminediamine for 48 hours. The product was purified via solid phase extraction using a Varian SCX anion exchange column (1 g of sorbent, 0.6 meq/g) by the procedure outlined below:
1) Column conditioned with 10 mL of CH₂Cl₂ (0.25 mL/sec).
2) Reaction mixture loaded onto SCX column (0.05 mL/sec).
3) Column rinsed with 10 mL of methanol.
4) Column rinsed with 4 mL of 1M NH₃/methanol and effluent collected into product tube.
5) Syringe washed with 2 mL of methanol.

This procedure was followed by a second solid phase extraction using a Varian SAX cation exchange column (1 g of sorbent, 0.7 meq/g) on the Benchmate® by the procedure outlined below:
1) Syringe washed with 4 mL of methanol.
2) Column conditioned with 10 mL of CH₂Cl₂ (0.25 mL/sec).
3) Product solution from SCX column loaded onto SAX
   column (0.05 mL/sec) and effluent collected into
   product tube (tared).
4) Column rinsed with 2 mL of CH₂Cl₂ and effluent collected into product tube.
5) Syringe washed with 4 mL of methanol.

The product solution (approx. 5 mL) was concentrated using a speed vac for 14 h to afford 66 mg (72%) of the title compound as a yellow semi-solid.
MS (Electrospray, + ions): m/z 577 (M + H).

Examples 176 to 185 can be prepared from Example 141 Part B compound by the method in Example 175.

### Example 176

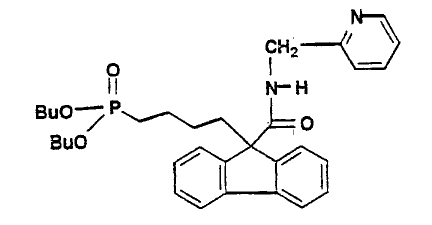

MS (ES, + ions) 549 (M+H).

### Example 177

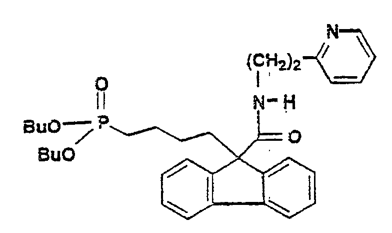

MS (ES, + ions) 563 (M+H).

### Example 178

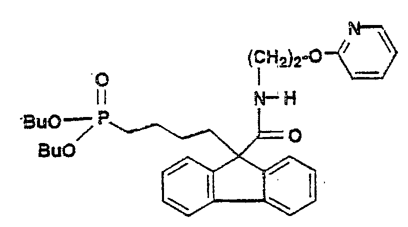

MS (ES, + ions) 579 (M+H).

### Example 179

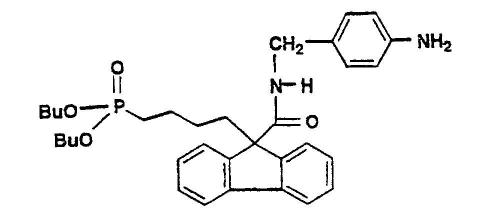

MS (ES, + ions) 563 (M+H).

### Example 180

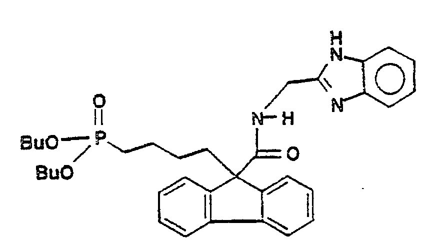

MS (ES, + ions) 588 (M+H).

### Example 181

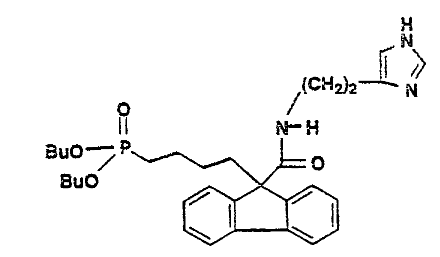

MS (ES, + ions) 552 (M+H).

### Example 182

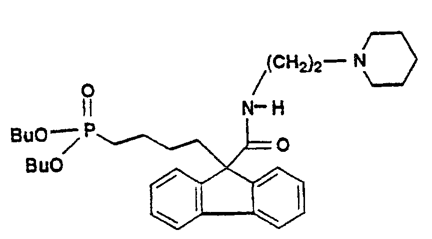

MS (ES, + ions) 569 (M+H).

### Example 183

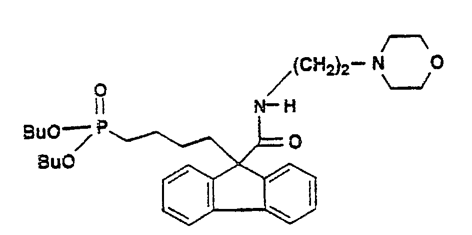

MS (ES, + ions) 571 (M+H).

### Example 184

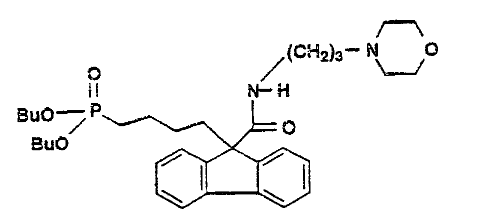

MS (ES, + ions) 585 (M+H).

### Example 185

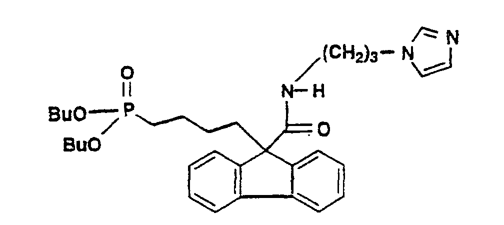

MS (ES, + ions) 566 (M+H).

### Example 186

### 9-[4-(Dibutoxyphosphinyl)butyl]-N-(2,2,2-trifluoroethyl)-9H-fluorene-9-carboxamide

A solution of Example 141 Part A compound (0.90 g, 2 mmol) in 5 mL of CH₂Cl₂ was treated with oxalyl chloride in dichloromethane (1.5 mL, 3.00 mmol) and two drops of DMF. After 0.5 h, the mixture was concentrated under reduced pressure to give a yelow oil. The oil was diluted with 10 mL of tetrahydro-furan, cooled to 0°C and treated with 2,2,2-trifluo-roethylamine (0.39 g, 4.00 mmol) and triethylamine (0.2 g, 2.0 mmol). The mixture was stirred for 3 h at room temperature and diluted with ethyl acetate (50 mL) and water (50 mL). The organic fraction was washed with 1N HCl (5 mL) dried over Na₂SO₄ and concentrated to a yellow oil. The oil was purified by flash column chromatography on silica gel (100 g) with 1:9 acetone/dichloromethane to give 0.69 g (59% overall yield) of title compound as a clear oil.
TLC Silica gel (1:9 acetone/dichloromethane) R_{f}= 0.3.
Mass Spec. (CI-NH_{3,} + ions) m/e 540 (M+H).

| | | | | | |
|---|---|---|---|---|---|
| Anal. Calc' d for C₂₈H₃₇F₃NO₄P + 0.3 H₂O | C, 61.76; | H, 6.95; | N, 2.57; | F, 10.47; | P, 5.69 |
| Found | C, 61.71; | H, 6.78; | N, 2.62; | F, 10.66; | P, 5.47. |

### Alternate Example 186

### 9-[4-(Dibutoxyphosphinyl)butyl]-N-(2,2,2-trifluoroethyl)-9H-fluorene-9-carboxamide

### A.

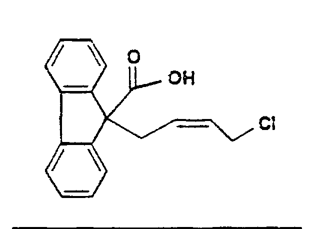

Butyllithium (8.4 mL, 2.5M in hexane, 21 mmol) was added dropwise over 10 min to a solution of 9-fluorenecarboxylic acid (2.10 g, 10 mmol) in THF (50 mL) at 0 °C under argon. During addition of the first equivalent of BuLi, the reaction became thick with a white precipitate which became yellow and cleared after addition of the second equivalent. The reaction was stirred at 0 °C for 20 min, then *cis*-1,4-dichloro-2-butene (1.2 mL, 11 mmol) was added dropwise over 5 min. The reaction lightened in color during addition and was stirred at 0 °C for 3 h, then poured into 1N HCl (50 mL) and extracted with CH₂Cl₂ (3 x 50 mL). The combined organic layers were washed with brine (30 mL) then dried over MgSO₄. Evaporation provided 3.5 g of a yellow oil containing crystalline solid. The crude residue was triturated with hexane (20 mL). The supernatant was decanted, and the residue pumped under high vacuum to give 2.93 g of title compound as a tan solid.

### B.

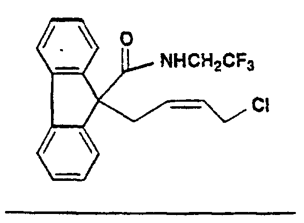

To a stirred solution of 10.0 g (33.5 mmol) of Part A compound in 100 mL of dichloromethane at RT was added 20.0 mL (40 mmol) of 2M oxalyl chloride in dichloromethane followed by 30 µL of DMF. The reaction was allowed to stir at RT for 2 h when the solvent was evaporated and the semisolid residue pumped (= 1 mm pressure) for 0.5 h. The residue was dissolved by adding 300 mL of ether and cooled to 0°C. The mixture was treated with 7.30 g (67 mmol) of 2,2,2-trifluoroethylamine and warmed to room temperature. The mixture was diluted with 150 mL of ethyl acetate and 100 mL of 0.5 M HCL. The layers were separated, the organics dried (Na₂SO₄) and concentrated. The remainder was purified by flash column chromatography on silica gel (250 g) eluting with 1:9 ethyl acetate/hexanes (800 mL) followed by 1:5 ethyl acetate/hexanes (1L). Pure fractions were pooled and concentrated to give 9.25 g (73%) of title compound as a white solid. mp: 87-89°C.

### C.

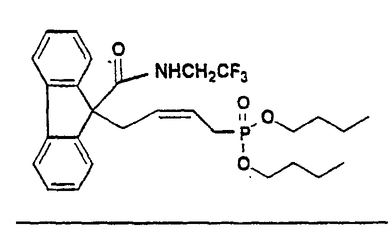

A mixture of Part B compound (7.60 g, 20 mmol) and tributylphosphite (25 g, 100 mmol) was warmed to 120°C for 24 h. The volitals were removed by short path distillation (0.2 mm Hg, 118°C) to leave 11.5 g of a colorless oil. The oil was purified by flash column chromatography on silica gel (500 g) eluting with 5:95 acetone/dichloromethane (1 L) followed by 1:5 acetone/dichloromethane (1L). Pure fractions were pooled to give 8.80 g (82%) of title compound as a colorless oil which gradually turned to a waxy solid.
TLC Silica gel (1:5 acetone/dichloromethane) R_{f}= 0.5.

### D. 9-[4-(Dibutoxyphosphinyl)butyl]-N-(2,2,2-trifluoroethyl)-9H-fluorene-9-carboxamide

A suspension of 8.50 g (15.8 mmol) of Part C compound in 200 mL of ethanol was warmed to 40°C for a few minutes to completely dissolve the crystalline solids. The resulting colorless solution was treated with 0.5 g of 10% Pd/carbon and the reaction vessel placed under an atmosphere of H₂ (balloon pressure). The reaction mixture was stirred for 25 h when it was filtered through a pad of celite. The colorless filtrate was filtered through a pad of celite and concentrated to give 8.3 g (95%) of title compound as a colorless oil.
The oil gradually turned to white solid on standing. mp: 71-74°C.
TLC Silica gel (1:5 acetone/dichloromethane) R_{f}= 0.5.
MS (ES, + ions) m/z 540 (M+H).

| | | | | | |
|---|---|---|---|---|---|
| Anal. Calc'd for C₂₈H₃₇F₃NO₄P | C, 62.33; | H, 6.91; | F, 10.56; | N, 2.60; | P, 5.74 |
| Found | C, 62.36; | H, 7.00; | F, 10.63; | N, 2.56; | P, 5.86. |

### Example 187

### 9-(2-Propenyl)-9H-fluorene-9-carboxylic acid, ethyl ester

An ethanol (7 ml) solution of Example 93 Part B (275 mg, 1.04 mmol) was stirred at room temperature for 1h, then stored at -20°C overnight. After warming, the volatiles were removed in vacuo to give an oil (300 mg). The residue was purified by flash column chromatography (SiO₂, 3 by 9 cm), eluting with 5%EtOAc:hexanes to give title compound (211 mg, 73% yield) as a colorless oil.
MS: (CI) : m/z 296 (M+NH₄)⁺.

### Example 188

### 9-(4-Cyanobutyl)-N-propyl-9H-fluorene-9-carboxamide

To a solution of 400 mg (0.92 mmol) of Example 11 Part C compound in 1 mL of DMSO, under argon at RT, was added 180 mg (2.77 mmol) of potassium cyanide (KCN). The mixture was stirred at RT for 18 h, at which time the reaction was diluted with ether and washed with sodium bisulfite, NaHCO₃, water, brine, dried (Na₂SO₄) and evaporated. Recrystallization was attained from hot hexanes to provide 225 mg (74%) of title compound as a white solid.
mp 102-104°C.
TLC Silica gel (95:5 dichloromethane/isopropanol)
R_{f} = 0.43.
MS (CI-NH₃, + ions) m/e 333 (M+H).

| | | | |
|---|---|---|---|
| Anal. Calcd. for C₂₂H₂₄N₂O₁ | C, 79.48; | H, 7.28; | N, 8.43 |
| Found | C, 79.17; | H, 7.40; | N, 8.34. |

### Example 189

### 1-[9-(3-Phenylpropyl)-9H-fluorene-9-yl]-1-butanone

A solution of Example 22 Part B acid chloride (4 mmol) in 15 ml of tetrahydrofuran was cooled to -20°C under an argon atmosphere and anhy. copper iodide (50 mg) was added. A 2 M solution of n-propyl magnesium chloride in ether (2 ml, 4 mmol) was added over a 5 minute period. The reacton was stirred at -20°C for 2.5 hrs. and then at 0°C for 30 min. The reaction was quenched with a saturated solution of ammonium chloride and extracted with ethyl acetate (3x20ml). The ethyl acetate extract was washed with water, brine and dried over anhy. sodium sulfate. The crude ketone was purified on a Merck EM silica column eluting with 5% ethyl acetate/hexane yielding 850 mg (64%) of title compound as a colorless oil.
MS (CI, + ions) 355 (M+H)

| | | |
|---|---|---|
| Anal Calc'd for C₂₆H₂₆O | C, 87.74; | H, 7.41 |
| Found | C, 87.70; | H, 7.45. |

### Example 190

### 9-(3-Phenylpropyl)-α-propyl-9H-fluorene-9-methanol

A solution of Example 189 compound (400 mg, 1.13 mmol) in 25 ml of methanol was cooled to 0°C under an argon atmosphere. Sodium borohydride (93 mg, 2.45 mmol) was added portion wise over 10 minutes and the mixture was then stirred for 30 min. longer at 0°C. The reaction was diluted with 0.1 N hydrochloric acid to pH 4. The reaction mixture was diluted with 30 ml of water and extracted with ethyl acetate (3x20 ml). The ethyl acetate extract was washed with water, brine and dried over sodium sulfate. The crude product was purified on a Merck EM silica column eluting with 10% ethyl acetate / hexane yielding 345 mg (86%) of title compound as a colorless oil.
MS (CI, + ions) 374 (M+NH₄).

| | | |
|---|---|---|
| Anal Calc'd for C₂₆H₂₈O+0.65 H₂O (FW 368.21) | C, 84.79; | H, 8.02 |
| Found | C, 84.83; | H, 7.94. |

### Example 191

### 4-Hydroxy-1-(9-propyl-9H-fluoren-9-yl)butanone

A solution of Example 59 Part B compound (1.07 g, 3.97 mmol) in THF (10 mL) under argon was cooled to 0°C. Copper (I) iodide (38 mg, 0.20 mmol) was added followed by dropwise addition of 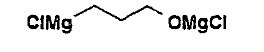 (prepared analogously to Umio, et al, J. Med. Chem. 1972, 15, 855) (14.5 mL, 0.3M in THF, 4.37 mmol) over 10 min. Upon addition, a deep red color appeared but quickly dissipated with stirring. The opaque yellow reaction was stirred at 0 °C for 45 min, then quenched by addition of saturated NH₄Cl (10 mL). The reaction was diluted with water (10 mL) and extracted with EtOAc (3 x 30 mL). The combined organic layers were washed with saturated NH₄Cl, water, and brine (10 mL each), then dried over MgSO₄. Evaporation gave 1.3 g of a yellow oil, which was purified by flash chromatography on silica gel (150 g), loading in 50% EtOAc/hexane, and eluting with 25% EtOAc/hexane to provide title compound (885 mg, 76%) as a colorless oil.

| | | |
|---|---|---|
| Anal. Calcd. for C₂₀H₂₂O₂ • 0.5 H₂O | C, 79.19; | H, 7.64. |
| Found | C, 79.07; | H, 7.32. |

### Example 192

### N-[3-(Dibutoxyphosphinyl)propyl]-9-propyl-9H-fluorene-9-carboxamide

### A.

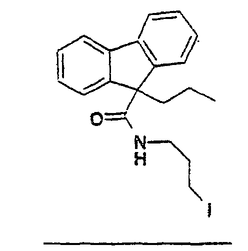

A solution of oxalyl chloride in dichloromethane (1 mL, 2.0 mmol) was added to a stirred suspension of Example 59 Part A compound (0.44 g 1.74 mmol) in 10 mL of dichloromethane. The reaction mass was treated with 1 drop of DMF, allowed to stir for 0.5 h and concentrated. The remainder was diluted with 10 mL of THF, cooled to -40° and treated with 1,3-propanolamine (0.26 g, 3.50 mmol) and warmed to RT over 3 h. The reaction mixture was diluted with 20 mL of water and 50 mL of ethyl acetate. The organic fraction was extracted with water (3X), dried (MgSO₄) and concentrated. The crude alcohol was carried on to the next step without further characterization.

To a stirred solution of 0.50 g (1.58 mmol) of the crude alcohol, 0.46 g (1.74 mmol) of triphenyl-phosphine, and 0.21 g (3.15 mmol) of imidazole in 10 mL of THF under argon at room temperature was added a solution of 0.44 g (1.74 mmol) of iodine in 10 mL of THF, dropwise over 15 min. After the addition was complete, the reaction was stirred at RT for 2 h and diluted with 100 mL of ethyl acetate and washed with a saturated solution of Na₂SO₃. The organic phase was dried (MgSO₄) and concentrated. The residue was purified by flash chromatography on silica gel (100 g) eluted with 15:85 ethyl acetate/hexanes to give 0.42 g (64%) of title compound as a white solid.
TLC Silica gel (1:3 ethyl acetate/hexanes) R_{f}=0.6. Mass Spec (CI-NH₃, + ions) m/e 420 (M+H).

### B. N-[3-(Dibutoxyphosphinyl)propyl]-9-propyl-9H-fluorene-9-carboxamide

A mixture of Part A compound (0.35 g, 0.83 mmol) and tributylphosphite (1.2 mL, 1.9 mmol) was warmed to 120°C for 18 h. The mixture was purified by short path distillation (0.2 mm Hg, 110°C) to leave 0.34 g of title compound as a colorless oil. The oil was purified by flash chromatography on silica gel (50 g) eluting with 1:9 isopropanol/dichloromethane to give 0.30 g (78%) of title compound as a colorless oil.
TLC Silica gel (5:95 2-propanol/dichloromethane)
R_{f}= 0.3.
Mass Spec. (ES, + ions) m/z 486 (M+H).

| | | | |
|---|---|---|---|
| Anal. Calc'd for C₂₈H₄₀NO₄P + 0.90 H₂O | C, 67.04; | H, 8.39; | N, 2.79 |
| Found | C, 67.09; | H, 8.54; | N, 2.72. |

### Example 193

### N-[5-(Dibutoxyphosphinyl)pentyl-9-propyl-9H-fluorene-9-carboxamide

### A.

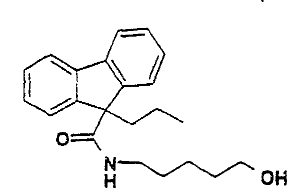

### N-(5-Hydroxypentyl)-9-propyl-9H-fluorene-9-carboxamide

A solution of oxalyl chloride in dichloromethane (1 mL, 2.0 mmol) was added to a stirred suspension of Example 59 Part A compound (0.40 g 1.58 mmol) in 10 mL of dichloromethane. The reaction mass was treated with 1 drop of DMF, allowed to stir for 0.5 h and concentrated. The remainder was diluted with 10 mL of THF, cooled to -78° and treated with 1,5-pentanolamine (0.41 g, 4 mmol) and warmed to RT over 3 h. The reaction mixture was diluted with 20 mL of water and 50 mL of ethyl acetate. The organic fraction was extracted with water (3X), dried (MgSO₄) and concentrated. The remainder was purified by column chromatography on silica gel (100 g) with 1:1 ethyl acetate/hexanes (500 mL) followed by 7:3 ethyl acetate/hexane (400 mL) to give 0.53 g (98%) of title compound as an oil. The resulting oil gradually solidified (4 days standing) to a white solid.
mp 48-51°.
TLC Silica gel (1:1 ethyl acetate/hexane) R_{f}= 0.3.
Mass Spec. (CI, + ions) m/z 338 (M+H).

| | | | |
|---|---|---|---|
| Anal. Calc'd for C₂₂H₂₇NO₂ + 0.3 H₂O | C, 77.13; | H, 8.11; | N, 4.09 |
| Found | C, 77.10; | H, 8.23; | N,. 4.00. |

### B.

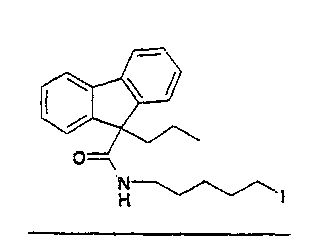

To a stirred solution of 0.50 g (1.50 mmol) of Part A compound, 0.47 g (1.80 mmol) of triphenyl-phosphine, and 0.20 g (3.00 mmol) of imidazole in 10 mL of THF under argon at room temperature was added a solution of 0.46 g (1.8 mmol) of iodine in 10 mL of THF, dropwise over 15 min. After the addition was complete, the reaction was stirred at RT for 2 h and diluted with 100 mL of ethyl acetate and washed with a saturated solution of Na₂SO₃. The organic phase was dried (MgSO₄) and concentrated. The residue was purified by flash chromatography on silica gel (100 g) eluted with 15:85 ethyl acetate/hexanes to give 0.58 g (87%) of title compound as a colorless oil.
TLC Silica gel (1:9 ethyl acetate/hexanes) R_{f}=0.3. Mass Spec (CI-NH₃, + ions) m/e 448 (M+H).

### C. N-[5-(Dibutoxyphosphinyl)pentyl]-9-propyl-9H-fluorene-9-carboxamide

A mixture of Part B compound (0.28 g, 0.63 mmol) and tributylphosphite (2 mL, 8 mmol) was warmed to 120°C for 18 h. The volitals were removed by short path distillation (0.2 mm Hg, 110°C) to leave 0.30 g (88%) of title compound as a colorless oil.
TLC Silica gel (5:95 2-propanol/dichloromethane)
R_{f}= 0.3.
Mass Spec. (ES, + ions) m/z 536 (M+Na), 514 (M+H).

| | | | | |
|---|---|---|---|---|
| Anal. Calc'd for C₃₀H₄₄NO₄P + 1.0 H₂O | C, 67.62; | H, 8.73; | N, 2.63; | P, 5.81 |
| Found | 'C, 67.31; | H, 8.33; | N, 2.94; | P, 6.05. |

### Example 194

### N-[[4-(1,3-Dihydro-1-oxo-2H-isoindol-2-yl)phenyl]-methyl]-9-propyl-9H-fluorene-9-carboxamide

### A.

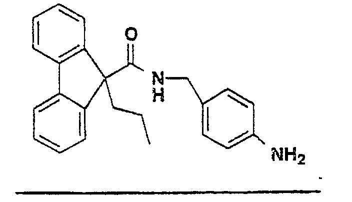

To a stirred solution of Example 59 Part A compound (1.0 g, 3.91 mmol) and triethylamine (0.6 mL, 4.30 mmol) in THF (10 mL) at -20°C was added dropwise isobutyl chloroformate (0.56 mL, 4.30 mmol). After stirring at -20°C for 30 min, the reaction containing a white precipitate was filtered through a fritted funnel to obtain a clear solution. To a stirred solution of 4-aminobenzylamine (0.49 mL, 4.30 mmol) in THF (10 mL) at -20°C was added dropwise the mixed anhydride solution over 30 min. The reaction was stirred at -20°C for 3 hrs, then warmed to RT. Dichloromethane (300 mL) was added to dilute the reaction. The resulting solution was washed with H₂O (2 x 50 mL), saturated sodium bicarbonate solution (2 x 50 mL), brine (2 x 50 mL) and dried over MgSO₄. The volatiles were removed under reduced pressure to afford title compound (1.2 g, 85%) as a solid. (mp 96-99°C, recrystallized from isopropanol/hexane).

### B.

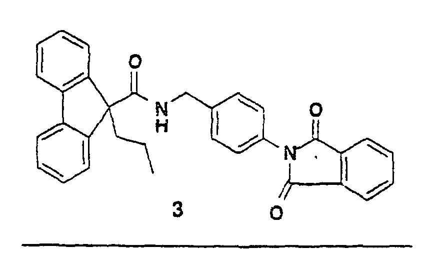

A mixture of Part A compound (500 mg, 1.39 mmol) and phthalic anhydride (206 mg, 1.39 mmol) was heated at 150°C for 30 min then cooled to RT. The reaction was triturated with methanol (5 mL), and the solid filtered and dried under vacuum to give title compound (440 mg, 65%) as a yellow solid.

### C. N-[[4-(1,3-Dihydro-1-oxo-2H-isoindol-2-yl)phenyl]methyl]-9-propyl-9H-fluorene-9-carboxamide

To stirred solution of Part B compound (420 mg, 0.86 mmol) in THF/MeOH (1:1, 8 mL) at 0°C was added sodium borohydride (33 mg, 0.86 mmol). The reaction was stirred at 0 °C for 30 min then warmed to RT. Stirring was continued for 2 h. The reaction was quenched with acetic acid until the reaction pH = 5. Dichloromethane (150 mL) was added to dilute the reaction and the solution was washed with saturated sodium bicarbonate (2 x 30 mL), H₂O (2 x 30 mL), brine (2 x 30 mL) and dried over MgSO₄. Evaporation gave a yellow solid. The residue was dissolved in trifluoroacetic acid (4 mL) at RT. Triethylsilane (0.42 mL, 2.58 mmol) was added. The reaction was stirred at RT for 30 min then evaporated to dryness. The residue was triturated with methanol (2 mL), filtered and dried to give title compound (260 mg, 64%) as a white powder.
mp 238-240°C.

| | | | |
|---|---|---|---|
| Anal. Calc. for C₃₂H₂₈N₂O₂ • 0.4H₂O | C, 80.11; | H, 6.05; | N, 5.84 |
| Found | C, 79.96; | H, 5.84; | N, 5.85. |

### Example 195

### (E)-9-[4-(Dibutoxyphosphinyl)-2-butenyl]-2,7-difluoro-N-propyl-9H-fluorene-9-carboxamide

### A.

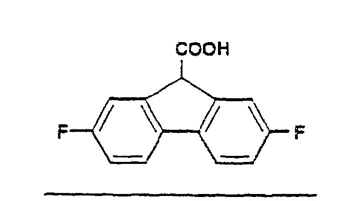

### A(1).

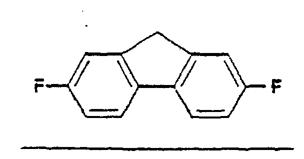

To a THF (25 ml) supension of 2,7-diaminofluorene (7.17 g, 0.036 mol) at -10°C under argon was added aqueous HBF₄ (71 mL, 1.13 mol, 48-50%). Near the end of addition stirring became difficult due to solid formation, although most of the solid went into solution upon complete addition of acid. A saturated aqueous solution of sodium nitrite (7.1 g in 11 mL, 0.103 mol) was added and after 1.5 h the mixture was filtered, washing with 5% aq. HBF₄, MeOH, then ether, and the collected solid dried briefly on the fliter flask. The resulting brown solid (9.7 g) was used in the subsequent reaction.

The above solid was suspended in xylenes (100 ml) and heated to 110°C for 2 h, with gas evolution observed, then brought to reflux for an additional 2 h. The solution was decanted from a black tar in the reaction flask and the volatiles removed under high vacuum to give a dark tan solid (7.5 g). The solid was crystallized from hot EtOH to give title compound (1.4 g) as a colorless solid. An ether wash of the black tar was combined with the mother liquor and concentrated *in vacuo*. The oily-solid residue (4.3 g) was purified by flash column chromatography (SiO₂, 9 by 16 cm), eluting with hexanes then 2.5% EtOAc:hexanes, to give title compound (2.44 g, total 3.84 g, 52% yield).as a colorless solid.

### A(2).

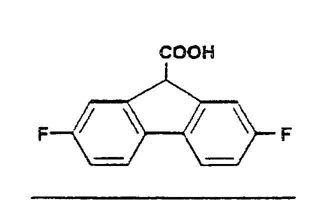

To a THF (15 ml) solution of Part A(1) compound (1.38 g, 6.82 mmol) at -5°C (ice/brine bath) under argon was added dropwise n-BuLi (3.4 ml, 8.50 mmol, 2.5 M in hexanes). After 1.15 h, crushed solid CO₂ (excess) was added, followed by Et₂O (∼5 ml), and the reaction allowed to stir at room temperature for 19 h. The brown colored reaction mixture was cooled to 0°C, quenched with 2N HCl, and the aqueous layer extracted twice with EtOAc. The combined organics were dried over Na₂SO₄ and evaporated *in vacuo* to give crude title compound (1.64 g, 98% recovery, contaminated with A(1), seen by ¹H NMR), as a colorless solid suitable for the next reaction. Trituration with hexanes can remove unreacted starting material Compound A(1).

### B.

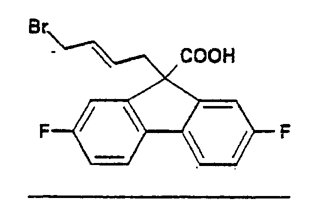

A solution of Part A 2,7-difluorofluorene-9-carboxylic acid (500mg, 2.05 mmol) in 5 ml of THF was cooled to -30°C under an argon atmosphere and 2 equiv. of a 2.5 M solution of n-butyl lithium in hexane (1.64 ml, 4.1 mmol) was added. The mixture was stirred for 5 min. at -30°C and was then added to a cold (-30°C) solution of 1,4-dibromo-2-butene (2.14 g, 10 mmol) in 4 ml of THF. The reaction mixture was stirred at -30°C for 30 min and was then quenched with 1 N HCl and extracted with ethyl acetate (3x10 ml). The ethyl acetate extract was washed with water, brine and dried over anhy. sodium sulfate. The crude title material was purified on a Merck EM silica column eluting with 5% isopropanol/dichloro-methane yielding 480 mg (62%) as a colorless solid, m.p. 142-146°C. (Mass Spec. M+H = 380).

### C.

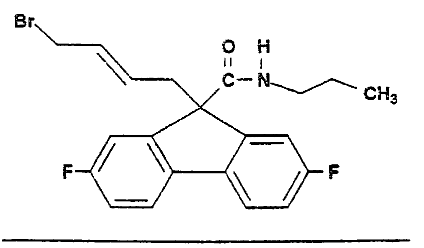

The Part B carboxylic acid (476 mg, 1 mmol) was dissolved in 12 ml of dichloromethane and DMF (50 µl) was added. The mixture was cooled to 0°C under an argon atmosphere and oxalyl chloride (178 mg, 1.4 mmol) was added and the mixture allowed to warm to ambient temperature and stir for 2.5 hrs. The mixture was evaporated several times from dichlormethane yielding the crude acid chloride as a pale yellow solid.

The acid chloride was dissolved in 8 ml of THF and cooled to 0°C under an argon atmosphere. Triethylamine (152 mg, 1.5 mmol) was added followed by the addition of n-propyl amine (77 mg, 1.3 mmol). The reaction was allowed to warm to ambient temperature and stir overnight. The reaction was quenched by adding sat. sodium bicarbonate and extracted with dichloromethane (4x20 ml). The crude product was purified on a Merck EM silica column eluting wiith 5% ethyl acetate/hexane yielding 420 mg (80%) of title compound as a pale yellow oil, (Mass Spec, M+H = 421).

### D. (E)-9-[4-(Dibutoxyphosphinyl)-2-butenyl]-2,7-difluoro-N-propyl-9H-fluorene-9-carboxamide

A solution of Part C compound (400 mg, 0.95 mmol) in tributyl phosphite (1.8 ml) was heated at 90°C overnight. Excess tributyl phosphite was removed under vacuum at 100°C and the oily residue was purified on a Merck EM silica column eluting with 3% isopropanol / dichloromethane yielding 353 mg (70%) of title compound as a colorless oil.
MS (CI, + ions) 534 (M+H).

| | | | |
|---|---|---|---|
| Anal Calc'd for C₂₉H₃₈NF₂PO₄+0.3 H₂O | C, 64.61; | H, 7.22; | N, 2.60 |
| Found | C, 64.69; | H, 7.50; | N, 2.52. |

### Example 196

### 9-[4-(Dibutoxyphosphinyl)butyl]-2,7-difluoro-N-propyl-9H-fluorene-9-carboxamide

An ethanol solution of Example 195 compound (260 mg, 0.49 mmol) containing 50 mg of 10% palladium on carbon was stirred under a hydrogen atmosphere (balloon) for 14 hrs. The reaction was filtered through a 0.2 µm nylon filter to remove the catalyst and the solvent evaporated yielding 235 mg (90%) of title compound as a colorless oil.
MS (CI, + ions) 536 (M+H).

| | | | |
|---|---|---|---|
| Anal Calc'd for C₂₉H₄₀NF₂PO₄+0.5 H₂O | C, 64.73; | H, 7.54; | N; 2.60 |
| Found | C, 64.78; | H, 7.50; | N, 2.55. |

### Example 197

### 9-[4-(Diethoxyphosphinyl)butyl]-N-propyl-9H-fluorene-9-carboxamide

To 400 mg (0.92 mmol) of Example 11 Part C compound was added 475 µL (2.77 mmol) of triethylphosphite (neat). The mixture was heated to 120°C for 18 h and bulb to bulb distilled (5 mm, 100°C) to remove lower boiling impurities and provide a yellow oil. Flash chromatography was performed on 50 g of silica gel eluting with 97:3 dichloromethane/isopro-panol to provide 300 mg (75%) of title compound as a pale yellow oil.
TLC Silica gel (95:5 dichloromethane/isopropanol)
R_{f} = 0.38.
MS (CI-NH₃, + ions) m/e 444 (M+H).

| | | | | |
|---|---|---|---|---|
| Anal. Calcd. for C₂₅H₃₄NO₄P + 0.75 mol H₂O | C, 65.20; | H, 7.85; | N, 3.04; | P, 6.73 |
| Found | C, 65.30; | H, 7.57; | N, 2.94; | P, 6.53. |

### Example 198

### 9-[4-(Diphenylphosphinyl)butyl]-N-propyl-9H-fluorene-9-carboxamide

To 400 mg (0.92 mmol) of Example 11 Part C compound was added 600 µL (2.77 mmol) of ethyldiphenyl phosphinite (neat, Aldrich). The mixture was heated to 120°C for 18 h. Flash chromatography was performed on 100 g of silica gel eluting with 97:3 dichloromethane/isopropanol to provide a white solid, which was further purified by crystalization from hot methanol triturated with water to provide 100 mg (22%) of title compound as a white solid. mp 163-165°C.
TLC Silica gel (95:5 dichloromethane/isopropanol)
R_{f} = 0.34.
MS (CI-NH₃, + ions) m/e 508 (M+H).

| | | | | |
|---|---|---|---|---|
| Anal. Calcd. for C₃₃H₃₄NO₂P | C, 78.08; | H, 6.75; | N, 2.76; | P, 6.10 |
| Found | C, 77.75; | H, 6.76; | N, 2.73; | P, 5.97. |

¹³C NMR (75 MHz, CDCl₃) is consistent with the indicated compound.

### Example 203

### (E)-9-[4-(Dibutoxyphosphinyl)-2-butenyl]-2,7-difluoro-N-(2,2,2-trifluoroethyl)-9H-fluorene-9-carboxamide

### A.

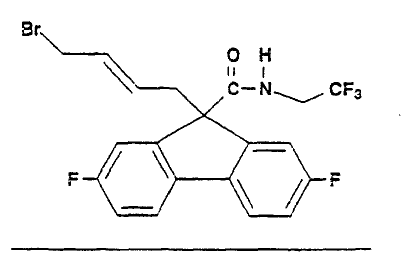

The Example 195 Part B carboxylic acid (465 mg, 1.23 mmol) was dissolved in 10 ml of dichloromethane and DMF (50 µl) was added. The mixture was cooled to 0°C under an argon atmosphere and oxalyl chloride (165 mg, 1.3 mmol) was added and the mixture allowed to warm to ambient temperature and stir for 2.5 hrs. The mixture was evaporated several times from dichlormethane yielding the crude acid chloride as a pale yellow solid.

The acid chloride was dissolved in 5 ml of THF and cooled to 0°C under an argon atmosphere. Triethylamine (142 mg, 1.4 mmol) was added followed by the addition of 2,2,2-trifluoroethylamine (139 mg, 1.4 mmol). The reaction was allowed to warm to ambient temperature and stir overnight. The reaction was quenched by adding sat. sodium bicarbonate and extracted with ethyl acetate (3x20 ml). The crude product was purified on a Merck EM silica column eluting wiith 10% ethyl acetate /hexane yielding 230 mg (38%) of title compound as a pale yellow solid, (Mass Spec, M+H = 461).

### B. (E)-9-[4-(Dibutoxyphosphinyl)-2-butenyl]-2,7-difluoro-N-(2,2,2-trifluoroethyl)-9H-fluorene-9-carboxamide

A solution of Part A compound (230 mg, 0.5 mmol) in tributyl phosphite (3 ml) was heated at 110°C overnight. Excess tributyl phosphite was removed under vacuum at 100°C and the oily residue was purified on a Merck EM silica column eluting with 3% isopropanol/dichloromethane yielding 186 mg (68%) of title compound as a colorless solid, m.p. 142-144°C.
MS (CI, + ions) 574 (M+H).

| | | | | | |
|---|---|---|---|---|---|
| Anal Calc'd for C₂₈H₃₃NF₅PO₄+0.3 H₂O | C, 58.63; | H, 5.80; | N, 2.44; | F, 16.56; | P, 5.40 |
| Found | C, 58.91; | H, 5.88; | N, 2.47; | F, 16.24; | P, 5.50. |

### Example 204

### 9-[4-[4-(1,3-Dihydro-1,3-dioxo-2H-isoindol-2-yl)-phenyl]butyl]-N-propyl-9H-fluorene-9-carboxamide

### A. 9-[4-(4-Aminophenyl)butyl]-N-propyl-9H-fluorene-9-carboxamide

### A(1). 9-[4-(4-Nitrophenyl)butyl]-N-propyl-9H-fluorene-9-carboxamide

### A(1)a.

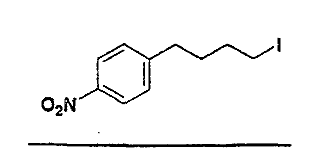

A solution of iodine (1.40 g, 5.5 mmol) in THF (5 mL) was added dropwise over 5 min to a solution of 4-(4-nitrophenyl)-1-butanol (975 mg, 5 mmol), triphenylphosphine (1.44 g, 5.5 mmol), and imidazole (749 mg, 11 mmol) in THF (10 mL) under argon at RT. The dark orange solution was stirred at RT for 15 min, diluted with hexane (50 mL), then washed with 10% sodium bisulfite, saturated NaHCO₃, and brine (20 mL each). The organic layer was dried over MgSO₄ and filtered. To the filtrate was added silica gel (4 g) and the mixture was concentrated in vacuo to give a yellow powder, which was purified by flash chromatography on silica gel (120 g) eluting with 25% CH₂Cl₂/hexane to give title compound (1.33 g, 87%) as a pale yellow crystalline solid (mp 44-45°C).

### A(1)b. 9-[4-(4-Nitrophenyl)butyl]-N-propyl-9H-fluorene-9-carboxamide

Butyllithium (1.8 mL, 2.5M in hexane, 4.4 mmol) was added to a solution of 9-fluorenecarboxylic acid (purchased from Aldrich Chemical Co.) (420 mg, 2.0 mmol) in THF (10 mL) at 0°C under argon over 5 min. The reaction went from a clear solution to a white suspension then to a yellow solution during addition. The reaction was stirred at 0°C for 20 min, whereupon a solution of Part A(1)a iodide (671 mg, 2.2 mmol) in THF (4 mL) was added dropwise over 5 min. The reaction was stirred at 0°C for 1.5 h, warmed to RT, then stirred at RT for 3.5 h. The reaction was quenched with 1N HCl to pH <2, diluted with water (10 mL), then extracted with EtOAc (2 x 20 mL). The combined organic layers were washed with water and brine (10 mL each), then dried over MgSO₄. Evaporation gave a residue, which was azeotroped with toluene (10 mL) to give 870 mg of a dark foam.

To a solution of the crude acid prepared above containing 3 drops of DMF in CH₂Cl₂ (6 mL) at RT under argon was added oxalyl chloride (1.5 mL, 2.0M in CH₂Cl₂, 3.0 mmol). The reaction bubbled for 10 min, then was allowed to stir at RT for 1.5 h. The reaction was concentrated in vacuo to provide a dark oil, which was diluted with CH₂Cl₂ (5 mL) and cooled to 0°C under argon. Propylamine (493 µL, 6.0 mmol) was added dropwise over 2 min, and the reaction was stirred at 0°C for 15 min. The reaction was partitioned between EtOAc (30 mL) and water (10 mL). The organic layer was washed with 1N HCl (2 x 5 mL) and brine (5 mL), then dried over MgSO₄. Evaporation gave 974 mg of a brown oil, which was dissolved in a minimal amount of CH₂Cl₂ and purified by flash chromatography on silica gel (75 g) eluting with 20% EtOAc/hexane to afford title compound (705 mg, 82%) as a waxy, yellow solid.
mp 109-110°C.

| | | | |
|---|---|---|---|
| Anal. Calcd. for C₂₇H₂₈N₂O₃ | C, 75.68; | H, 6.59; | N, 6.54 |
| Found | C, 75.70; | H, 6.58; | N, 6.57. |

### A(2). 9-[4-(4-Aminophenyl)butyl]-N-propyl-9H-fluorene-9-carboxamide

A mixture of Part A(1) compound (628 mg, 1.47 mmol) and 10% palladium on carbon (74 mg, 0.07 mmol) in EtOAc (5 mL) was hydrogenated (balloon) at RT for 5 h, filtered through Celite with the aid of EtOAc, then concentrated in vacuo to give a residue, which was pumped under high vacuum to provide title compound (588 mg, 100%) as a yellow gum.
MS (CI, + ions) m/z 399 (M+H).

| | | | |
|---|---|---|---|
| Anal. Calcd. for C₂₇H₃₀N₂O • 0.3 H₂O | C, 80.28; | H, 7.64; | N, 6.93 |
| Found | C, 80.37; | H, 7.53; | N, 7.34. |

### B. 9-[4-[4-(1,3-Dihydro-1,3-dioxo-2H-isoindol-2-yl)phenyl]butyl]-N-propyl-9H-fluorene-9-carboxamide

A mixture of Part A compound (342 mg, 0.859 mmol) and phthalic anhydride (127 mg, 0.859 mmol) was heated neat at 140 °C. The reaction bubbled (water evolution) for 10 min, then the reaction was allowed to stir for an additional 15 min. The reaction was cooled to RT, and the resulting glassy solid was dissolved in a minimum amount of CH₂Cl₂ and purified by flash chromatography on silica gel (50 g) eluting with 35% EtOAc/hexane to provide title compound (380 mg, 84%) as a yellow oil.
MS (CI, + ions) m/z 529 (M+H).

| | | | |
|---|---|---|---|
| Anal. Calcd. for C₃₅H₃₂N₂O₃ • 0.2 CH₂Cl₂ | C, 77.48; | H, 5.99; | N, 5.13. |
| Found | C, 77.18; | H, 6.20; | N, 4.87. |

### Example 205

### 9-[4-[4-[[(2-Phenoxyphenyl)carbonyl]amino]phenyl]-butyl]-N-propyl-9H-fluorene-9-carboxamide

To a solution of 2-phenoxybenzoic acid (Aldrich Chemical Co.) (111 mg, 0.518 mmol) and DMF (2 drops) in CH₂Cl₂ (1.5 mL) was added oxalyl chloride (389 µL, 2.0M in CH₂Cl₂, 0.777 mmol). The reaction bubbled for 10 min, then was stirred at RT under argon for 1.5 h. The reaction was concentrated in vacuo, and the resulting residue was dissolved in CH₂Cl₂ (1.5 mL) and added dropwise to a solution of Example 204 Part A compound (172 mg, 0.432 mmol) and triethylamine (90 µL, 0.648 mmol) in CH₂Cl₂ (1.5 mL) at 0°C under argon. The reaction was stirred at 0°C for 10 min, diluted with CH₂Cl₂ (20 mL), washed with saturated NaHCO₃ (5 mL) and brine (5 mL), then dried over Na₂SO₄. Evaporation gave a yellow oil, which was dissolved in a minimum amount of CH₂Cl₂ and purified by flash chromatography on silica gel (50 g) eluting with 30% EtOAc/hexane to provide title compound (211 mg, 82%) as a yellow gum.
MS (CI, + ions) m/z 595 (M+H).

| | | | |
|---|---|---|---|
| Anal. Calcd. for C₄₀H₃₈N₂O₃ • 0.4 CH₂Cl₂ | C, 77.18; | H, 6.22; | N, 4.46 |
| Found | C, 77.18; | H, 6.20; | N, 4.87. |

### Example 206

### 9-[4-[4-(1,3-Dihydro-1-oxo-2H-isoindol-2-yl)-phenyl]-butyl]-N-propyl-9H-fluorene-9-carboxamide

Sodium borohydride (22 mg, 0.574 mmol) was added to a solution of Example 204 compound (303 mg, 0.574 mmol) in THF/EtOH (3:7, 5 mL) at 0°C under argon. The reaction was stirred at 0°C for 30 min, then allowed to warm to RT overnight. The reaction was adjusted to slightly acidic pH with glacial acetic acid (few drops), then concentrated in vacuo. The resulting residue was partitioned between CH₂Cl₂ (20 mL) and saturated NaHCO₃ (5 mL). The organic layer was washed with brine (5 mL) then dried over Na₂SO₄. Evaporation gave 285 mg of a yellow foam.

To the hydroxylactam prepared above was added triethylsilane (137 µL, 0.861 mmol) followed by trifluoroacetic acid (2 mL). The reaction was stirred at RT under argon for 20 min, then concentrated in vacuo. The resulting.orange oil was purified by flash chromatography on silica gel (50 g) eluting with 4% EtOAc/CH₂Cl₂ to afford title compound (243 mg, 82%) as a white solid.
mp 147-148.5°C.
MS (CI, + ions) m/z 515 (M+H).

| | | | |
|---|---|---|---|
| Anal. Calcd. for C₃₅H₃₄N₂O₂ | C, 81.68; | H, 6.66; | N, 5.44 |
| Found | C, 81.54; | H, 6.65; | N, 5.45. |

### Example 207

### 9-[3-[4-(1,3-Dihydro-1,3-dioxo-2H-isoindol-2-yl)-phenyl]propyl]-N-propyl-9H-fluorene-9-carboxamide

### A. 9-[3-(4-Aminophenyl)propyl]-N-propyl-9H-fluorene-9-carboxamide

### A(1). 9-[3-(4-Nitrophenyl)-2-propenyl]-N-propyl-9H-fluorene-9-carboxamide

### A(1)a.

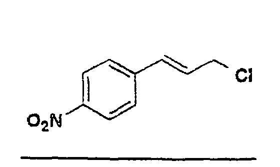

To a solution of N-chlorosuccinimide (2.23 g, 16.7 mmol) in dichloromethane (40 mL) at -40°C was added dropwise methyl sulfide (1.64 mL, 22.3 mmol). The reaction was stirred at -40°C for 30 min, then warmed to RT for 60 min. The reaction was recooled to -40°C, and a solution of 4-nitrocinnamyl alcohol (2.50 g, 13.9 mmol) in dichloromethane (4 mL) was added dropwise. The reaction was stirred at -40°C for 2 h then warmed to RT overnight. Ethyl acetate (200 mL) was added to dilute the reaction and the solution was washed with water (2 x 50 mL), brine (2 x 50 mL) and dried over MgSO₄. Evaporation gave title compound (2.50 g, 91%) as a crude oil.

### A(1)b.

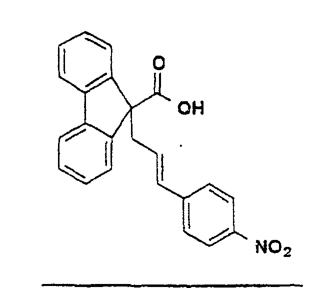

### 9-[3-(4-Nitrophenyl)-2-propenyl]-9-fluorenecarboxylic acid

To a solution of 9-fluorenecarboxylic acid (1.0 g, 4.76 mmol) in THF (20 mL) at 0°C was added dropwise a solution of n-butyllithium (2.5M, 4.2 mL, 10.5 mmol) in THF. The dark reaction was stirred at 0°C for 20 min, then a solution of Part A(1)a chloride (1.04 g, 5.24 mmol) in THF (2 mL) was added dropwise over 5 min. The reaction was stirred at 0°C for 4.5 h and the dark color faded away gradually. Hydrochloric acid (1.0M, 2 mL) was added to quench the reaction. Ethyl acetate (200 mL) was added and the organic layer was washed with water (2 x 50 mL), brine (2 x 50 mL) and dried over MgSO₄. Evaporation gave title compound (1.7 g, 87%) as a yellowish oil.

### A(1)c. 9-[3-(4-Nitrophenyl)-2-propenyl]-N-propyl-9H-fluorene-9-carboxamide

To a solution of Part A(1)b compound (1.65 g, 4.45 mmol) and DMF (1 drop) in dichloromethane (15 mL) at RT was added dropwise a solution of oxalyl chloride in dichloromethane (2.0M, 3.34 mL, 6.67 mmol). Bubbling of escaping gasses continued for 10 min after addition. The reaction was stirred at RT for 60 min, then concentrated in vacuum to give a dark oil. The crude acid chloride was dissolved in dichloromethane (10 mL) and cooled to 0°C under argon. Propylamine (1.1 mL, 13.4 mmol) was added dropwise over 3 min. The reaction was stirred at 0°C for 30 min. Ethyl acetate (100 mL) was added to dilute the reaction and the resulting solution was washed with H₂O (2 x 30 mL), HCl (1.0M, 2 x 30 mL), saturated sodium carbonate solution (2 x 30 mL), brine (2 x 30 mL) and dried over MgSO₄. Evaporation gave a crude gum. Purification was performed by flash chromatography on silica gel (100 g), loaded and eluted with 20% ethyl acetate in hexane. Pure fractions were combined and evaporated to give a yellow solid (1.10 g, 60%). A portion of the resulting product (300 ma) was recrystallized from ethyl acetate/hexane to give title compound (200 mg, 67%) as a yellow solid.
m.p. 143-146°C.
MS (CI, + ions) m/z 413 (M+H).

| | | | |
|---|---|---|---|
| Anal. Calc. for C₂₆H₂₄N₂O₃ • 0.3H₂O | C, 74.73; | H, 5.93; | N, 6.70 |
| Found | C, 74.54; | H, 5.75; | N, 6.67. |

### A(2). 9-[3-(4-Aminophenyl)propyl]-N-propyl-9H-fluorene-9-carboxamide

To a solution of Part A(1) compound (911 mg, 2.21 mmol) in ethyl acetate (10 mL) at RT was added palladium on activated carbon (10%, 60 mg) under argon. The reaction was hydrogenated (balloon) at RT for 18 h. The reaction was filtered and the filtrate was evaporated to give 720 mg of a white solid. A portion of the product (500 mg) was recrystallized from ethyl acetate/hexane to give title compound (350 mg, 60%) as a white solid.
m.p. 138-140°C.
MS (CI, + ions) m/z 385 (M+H).

| | | | |
|---|---|---|---|
| Anal. Calc. for C₂₆H₂₈N₂O • 0.3H₂O | C, 80.09; | H, 7.39; | N, 7.18 |
| Found | C, 80.01; | H, 7.31; | N, 7.17. |

### B. 9-[3-[4-(1,3-Dihydro-1,3-dioxo-2H-isoindol-2-yl)-phenyl]propyl]-N-propyl-9H-fluorene-9-carboxamide

Following the procedure in Example 194 Part A compound (360 mg, 0.94 mmol) was reacted with phthalic anhydride (140 mg, 0.94 mmol) to give 450 ma of a colorless oil. The product was crystallized from MeOH/H₂O to give title compound (380 mg, 79%) as a white solid.
m.p. 148-151°C.
MS (CI, + ions) m/z 515 (M+H).

| | | | |
|---|---|---|---|
| Anal. Calc. for C₃₄H₃₀N₂O₃ • 0.9H₂O | C, 76.93; | H, 6.04; | N, 5.28 |
| Found | C, 76.88; | H, 5.73; | N, 5.23. |

### Example 208

### 9-[3-[4-(Benzoylamino)]phenyl]-N-propyl-9H-fluorene-9-carboxamide

To a solution of Example 207 Part A compound (100 mg, 0.26 mmol) and triethylamine (0.04 mL, 0.39 mmol) in dichloromethane at 0°C was added dropwise a solution of benzoyl chloride (0.04 mL, 0.31 mmol) in dichloromethane (1 mL). The reaction was stirred at 0 °C for 20 min. Ethyl acetate (50 mL) was added and the solution was washed with saturated sodium bicarbonate solution (2 x 30 mL), water (2 x 30 mL), brine (2 x 30 mL) and dried over MgSO₄. Purification was performed by flash chromatography on silica gel (50 g), loaded and eluted with 30% ethyl acetate in hexane. Pure fractions were combined and evaporated to give a solid. The resulting solid was recrystallized from ethyl acetate/hexane to give title compound (52 mg, 41%) as a white solid.
m.p. 187-190°C.
MS (CI, + ions) m/z 489 (M+H).

| | | | |
|---|---|---|---|
| Anal. Calc. for C₃₃H₃₂N₂O₂ • 1.0 H₂O | C, 78.23; | H, 6.76; | N, 5.53 |
| Found | C, 78.44; | H, 6.54; | N, 5.43. |

### Example 209

### 9-[3-[(1,3-Dihydro-1-oxo-2H-isoindol-2-yl)phenyl]-propyl]-N-propyl-9H-fluorene-9-carboxamide

Following the procedure in Example 194, Example 207 Part (A2) compound (350 mg, 0.68 mmol) was reacted to give 300 mg of a colorless oil. The product was crystallized from MeOH/H₂O to give title compound (160 mg, 47%) as a white solid.
m.p. 122-125°C.
MS (CI, + ions) m/z 501 (M+H).

| | | | |
|---|---|---|---|
| Anal. Calc. for C₃₄H₃₂N₂O₂ • 0.8H₂O | C, 79.29; | H, 6.58; | N, 5.44 |
| Found | C, 79.28; | H, 6.51; | N, 5.29. |

### Example 210

### 9-[5-[(6-Ethoxy-2-benzothiazolyl)thio]pentyl]-N-propyl-9H-fluorene-9-carboxamide

### A.

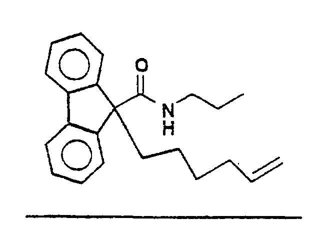

To a mixture of 3.0 g (11.95 mmol) of Example 11 Part C compound in 30 mL of THF, under argon at 0°C, was added 9.4 mL (23.90 mmol) of n-BuLi (2.5 M in hexanes) dropwise. The dianion was stirred for 0.5 h at which time 1.9 mL (14.34 mmol) of 6-bromo-1-hexene (Aldrich) was added dropwise. The reaction gradually warmed to RT and was stirred for 6 days. The reaction was diluted with a 1:1 mixture of ethyl acetate/water and separated. The organics were washed with brine, dried (Na₂SO₄) and evaporated. Flash chromatography was performed on 200a of silica gel eluting with 4:1 hexanes/ethyl acetate to provide 3.0 g (77%) of title compound as a pale yellow solid.
mp 54-56°C.
TLC Silica gel (4:1 hexanes/ethyl acetate) R_{f}=0.27. MS (CI-NH₃, + ions) m/e 334 (M+H).

| | | | |
|---|---|---|---|
| Anal. Calc. for C₂₃H₂₇NO | C, 82.84; | H, 8.16; | N, 4.20 |
| Found | C, 82.90; | H, 8.18; | N, 4.59. |

### B.

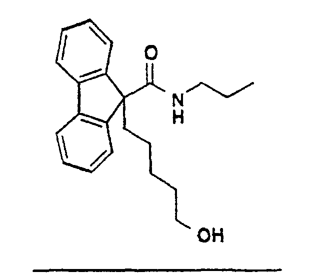

To a solution of 2.0 g (6.00 mmol) of Part A compound in 20 mL of methanol, under nitrogen at - 78°C, was bubbled O₃ for 0.5 h. The solution was purged with nitrogen and treated with 718 mg (18.89 mmol) of sodium borohydride (~ 5 pellets). The mixture was gradually warmed to room temperature and was stirred for 18 h, at which time the reaction was diluted with ether and quenched with NH₄Cl. The organics were washed with water, brine, dried (Na₂SO₄) and evaporated. Flash chromatography was performed on 200 g of silica gel eluting with 1:1 hexanes/ethyl acetate to provide 1.6 g (80%) of title compound as a colorless oil.
TLC Silica gel (1:1 hexanes/ethyl acetate) R_{f}=0.13.

| | | | |
|---|---|---|---|
| Anal. Calcd. for C₂₂H₂₇NO₂ + 0.40 mol H₂O + 0.15 mol CH₂Cl₂ | C, 74.44; | H, 7.92; | N, 3.92 |
| Found | C, 74.50; | H, 7.62; | N, 3.73. |

### C.

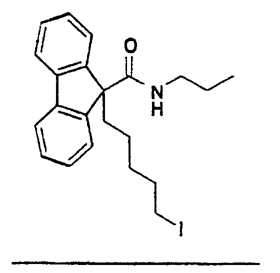

To a solution of 1.4 g (4.15 mmol) of Part B compound in 20 mL of THF, under argon at 0°C, was added 620 mg (9.13 mmol) of imidazole and 1.4 g (5.40 mmol) of triphenylphosphine. This mixture was stirred at 0°C for 0.5 h, at which time 1.4 g (5.40 mmol) of iodine in 10 mL of THF was added dropwise. The reaction was stirred for 1.5 h, at 0°C, at which time it was diluted with hexanes and washed with sodium bisulfite, NaHCO₃, brine, dried (Na₂SO₄) and evaporated. Flash chromatography was performed on 50 g of silica gel eluting with 1:1 hexanes/ethyl acetate to provide 1.57 g (84%) of title compound as a white solid.
TLC: Silica gel (1:1 hexanes/ethyl acetate)
R_{f} = 0.63.

MS (ES, + ions) m/e 448 (M+H).

### D. 9-[5-[(6-Ethoxy-2-benzothiazolyl)thio]-pentyl]-N-propyl-9H-fluorene-9-carboxamide

To a solution of 200 mg (0.45 mmol) of Part C compound in 5 mL of DMF, under argon at RT, was added 125 mg (0.90 mmol) of K₂CO₃ followed by 114 mg (0.54 mmol) of 6-ethoxy-2-mercaptobenzothiazole. The reaction was stirred for 18 h at which time it was diluted with ether and the organics were washed with water, brine, dried (Na₂SO₄) and evaporated. Flash chromatography was performed on 50 g of silica gel eluting with 95:5 dichloromethane/isopropanol to provide 120 mg (50%) of title compound as a biege solid.
mp 67-70°C .
TLC Silica gel (95:5 dichloromethane/isopropanol)
R_{f} = 0.35.
MS (CI-NH₃, + ions) m/e 531 (M+H).

| | | | | |
|---|---|---|---|---|
| Anal. Calcd. for C₃₁H₃₄N₂O₂S₂ | C, 70.15; | H, 6.46; | N, 5.28; | S, 12.08 |
| Found | C, 69.95; | H, 6.20; | N, 5.22; | S, 12.11. |

### Example 211

### 9-[4-[4-(Benzoylamino)phenyl]butyl]-N-propyl-9H-fluorene-9-carboxamide

Benzoyl chloride (156 µL, 1.35 mmol) was added dropwise to a solution of Example 207 Part A compound (490 mg, 1.23 mmol) and triethylamine (257 µL, 1.85 mmol) in CH₂Cl₂ (4 mL) at 0°C under argon. The reaction was stirred at 0°C for 30 min, diluted with CH₂Cl₂ (20 mL) and CHCl₃ (20 mL), washed with 1N KOH (2 x 10 mL) and water (10 mL), then dried over MgSO₄. Evaporation gave a yellow solid, which was adsorbed onto silica gel (10 g), then purified by flash chromatography on silica gel (150 g) eluting with 5% EtOAc/CH₂Cl₂ to give a solid. The product was dried under high vacuum at 50°C overnight to provide title compound (412 mg, 67%) as a white solid.
mp 171-173°C.

| | | | |
|---|---|---|---|
| Anal. Calcd. for C₃₄H₃₄N₂O₂ • 0.4 H₂O | C, 81.24; | H, 6.82; | N, 5.57 |
| Found | C, 80.88; | H, 6.83; | N, 5.33. |

### Example 212

### 9-[5-(Dibutoxyphosphinyl)pentyl]-N-propyl-9H-fluorene-9-carboxamide

To 400 mg (0.89 mmol) of Example 209 Part A compound, under argon, was added 1.2 mL (4.45 mmol) of tributylphosphite (neat). The mixture was heated to 120°C for 18 h and bulb to bulb distilled (5 mm, 100°C) to remove lower boiling impurities and provide a pale yellow oil. Flash chromatography was performed on 75 g of silica gel eluting with 95:5 dichloromethane/isopropanol to provide 440 mg (96%) of title compound as a pale yellow oil.
TLC Silica gel (95:5 dichloromethane/isopropanol)
R_{f} = 0.29.
IR 3434, 2959, 2934, 2872, 1665, 1508, 1449, 1244, 1024, 978, 743 cm⁻¹.
¹H NMR (300 MHz, CDCl₃) is consistent with the indicated compound.
MS (CI-NH₃, + ions) m/e 514 (M+H).

| | | | |
|---|---|---|---|
| Anal. Calcd. for C₃₀H₄₄NO₄P | C, 70.15; | H, 8.63; | P, 6.03 |
| Found | C, 70.60; | H, 8.80; | P, 5.86. |

¹³C NMR (75 MHz, CDCl₃) is consistent with the indicated compound.

The following compounds were prepared employing procedures as described hereinbefore.

### Example 213

### N,N-Diethyl-9-(2-propenyl)-9H-fluorene-9-carboxamide

MS (CI, M+H)⁺ m/z 306

| | | | |
|---|---|---|---|
| Anal. Calcd for C₂₁H₂₃NO•0.14 H₂O | C, 81.90; | H, 7.62; | N, 4.55 |
| Found | C, 82.11; | H, 7.52; | N, 4.34. |

mp 84-86°C.

### Example 214

### N-Ethyl-9-propyl-9H-fluorene-9-carboxamide

MS (CI, M+H)⁺ m/z 280

| | | | |
|---|---|---|---|
| Anal. Calcd for C₁₉H₂₁NO | C, 81.68; | H, 7.58; | N, 5.01 |
| Found | C, 81.45; | H, 7.77; | N, 5.06. |

mp 96-97.5°C.

### Example 215

### N-Ethyl-9-(2-propenyl)-9H-xanthene-9-carboxamide

MS (CI-NH₃, + ions) m/e 311 (M+NH₄), 294 (M+H).

| | | | |
|---|---|---|---|
| Anal. Calcd for C₁₉H₁₉O₂N | C, 77.79; | H, 6.53; | N, 4.77 |
| Found | C, 77.87; | H, 6.57; | N, 4.77. |

mp 111-112°C.

### Example 216

### N-Ethyl-9-(3-phenylpropyl)-9H-xanthene-9-carboxamide

MS (CI-NH₃, + ions) m/e 372 (M+H).

| | | | |
|---|---|---|---|
| Anal. Calcd for C₂₅H₂₅NO₂ | C, 80.83; | H, 6.78; | N, 3.77 |
| Found | C, 80.77; | H, 6.88; | N, 3.83. |

mp 130°C.

### Example 217

### 9- [(4-Morpholinyl)carbonyl]-9-propyl-9H-fluorene

CI-Mass Spec. (M+H)=322.

| | | | |
|---|---|---|---|
| Anal. Calcd for C₂₁H₂₃NO₂ | C, 78.47; | H, 7.21; | N, 4.36 |
| Found | C, 78.43; | H, 7.11; | N, 4.18. |

mp 92-94°C.

### Example 218

### 9-Hexyl-N-propyl-9H-xanthene-9-carboxamide

MS (CI-NH₃, + ions) m/e 352 (M+H).

| | | | |
|---|---|---|---|
| Anal. Calcd for C₂₃H₂₉NO₂ | C, 78.60; | H, 8.32; | N, 3.98 |
| Found | C, 78.64; | H, 8.46; | N, 3.96. |

mp 76-77.5°C.

### Example 219

### N-Methoxy-N-methyl-9-propyl-9H-fluorene-9-carboxamide

CI-Mass Spec. (M+H)=296.

| | | | |
|---|---|---|---|
| Anal. Calcd for C₁₉H₂₁NO₂ | C, 77.26; | H, 7.17; | N, 4.74 |
| Found | C, 77.12; | H, 7.04; | N, 4.68. |

mp 73.75°C.

### Example 220

### 10,11-Dihydro-5-(3-phenyl-2-propenyl)-N-propyl-5H-dibenzo[a,d]cycloheptene-5-carboxamide

MS (CI-NH₃, + ions) m/e 396 (M+H).

| | | | |
|---|---|---|---|
| Anal. Calcd for C₂₈H₂₉NO | C, 85.02; | H, 7.39; | N, 3.54 |
| Found | C, 84.66; | H, 7.46; | N, 3.46. |

mp 159°C.

### Example 221

### N-Methyl-9-propyl-9H-fluorene-9-carboxamide

CI-Mass Spec. (M+H)=266.

| | | | |
|---|---|---|---|
| Anal. Calcd for C₁₈H₁₉NO+0.12 H₂O | C, 80.82; | H, 7.25; | N, 5.24 |
| Found | C, 80.90; | H, 7.26; | N, 5.16. |

mp 145-146°C.

### Example 222

### 1-(9-Propyl-9H-fluoren-9-yl)-1-pentanone

CI-Mass Spec. (M+H)=293.

| | | |
|---|---|---|
| Anal. Calcd for C₂₁H₂₄O | C, 86.20; | H, 8.24 |
| Found | C, 85.86; | H, 8.14. |

mp 56-58°C.

### Example 223

### α-Butyl-9-propyl-9H-fluorene-9-methanol

CI-Mass Spec. (M+NH₄)=312⁺.

| | | |
|---|---|---|
| Anal. Calcd for C₂₁H₂₆O+0.12 H₂O | C, 85.05; | H, 8.92 |
| Found | C, 85.05; | H, 8.87. |

mp 88-90°C.

### Example 224

### 1-(9-Propyl-9H-fluoren-9-yl)-1-butanone

CI-Mass Spec. (M+H)=279.

| | | |
|---|---|---|
| Anal. Calcd for C₂₀H₂₂O+0.1 H₂O | C, 85.79; | H, 7.98 |
| Found | C, 85.79; | H, 8.15. |

mp 65-67°C.

### Example 225

### α,9-Dipropyl-9H-fluorene-9-methanol

CI-Mass Spec. (M+NH₃)=298.

| | | |
|---|---|---|
| Anal. Calcd for C₂₀H₂₄O+0.1 H₂O | C, 85.15; | H, 8.64 |
| Found | C, 85,15; | H, 8.72. |

mp 83-85°C.

### Example 226

### 10,11-Dihydro-5-(2-propenyl)-N-propyl-5H-dibenzo-[a,d]cycloheptene-5-carboxamide

MS (CI-NH₃, + ions) m/e 320 (M+H).

| | | | |
|---|---|---|---|
| Anal. Calcd for C₂₂H₂₅NO | C, 81.98; | H, 7.92; | N, 4.35 |
| Found | C, 82.01; | H, 7.91; | N, 4.32. |

mp 76-79°C.

### Example 227

### 9-(3-Phenylpropyl)-N-propyl-9H-thioxanthene-9-carboxamide

MS (CI-NH₃, + ions) m/e 402 (M+H).

| | | | |
|---|---|---|---|
| Anal. Calcd for C₂₆H₂₇NOS | C, 77.77; | H, 6.78; | N, 3.49 |
| Found | C, 77.60; | H, 6.83; | N, 3.42. |

mp 130-131°C.

### Example 228

### N,9-Dipropyl-9H-thioxanthene-9-carboxamide

MS (CI-NH₃, + ions) m/e 326 (M+H).

| | | | |
|---|---|---|---|
| Anal. Calcd for C₂₀H₂₃NOS | C, 73.81; | H, 7.12; | N, 4.30 |
| Found | C, 73.84; | H, 7.36; | N, 4.24. |

mp 132-133°C.

### Example 229

### 10,11-Dihydro-5-(3-phenylpropyl)-N-propyl-5H-dibenzo-[a,d]cycloheptane-5-carboxamide

MS (CI, NH₃, + ions) m/z 398 (M+H).

| | | | |
|---|---|---|---|
| Anal. Calcd for C₂₈H₃₁NO+0.4 H₂O | C, 82.90; | H, 7.93; | N, 3.45 |
| Found | C, 82.99; | H, 7.95; | N, 3.36. |

mp 109-112°C.

### Example 230

### (E)-2,7-Difluoro-9-(3-phenyl-2-propenyl)-N-propyl-9H-fluorene-9-carboxamide

MS (CI, M+H)⁺ m/z 404.

| | | | |
|---|---|---|---|
| Anal. Calcd for C₂₆H₂₃NF₂O | C, 77.40; | H, 5.75; | N, 3.47 |
| Found | C, 77.32; | H, 5.70; | N, 3.33. |

mp 124-126°C.

### Example 231

### 9-(3-Phenylpropyl)-N-(2-pyridinylmethyl)-9H-fluorene-9-carboxamide

CI-Mass Spec. (M+H)=419.

| | | | |
|---|---|---|---|
| Anal. Calcd for C₂₉H₂₆N₂O | C, 83.22; | H, 6.26; | N, 6.70 |
| Found | C, 83.42; | H, 6.31; | N, 6.62. |

mp 115-116°C.

### Example 232

### 2,7-Difluoro-9-(3-phenylpropyl)-N-propyl-9H-fluorene-9-carboxamide

MS (CI, M+H)⁺ m/z 406.

| | | | | |
|---|---|---|---|---|
| Anal. Calcd for C₂₆H₂₅F₂NO•0.12 H₂O | C, 76.62; | H, 6.24; | N, 3.44; | F, 9.32 |
| Found | C, 76.64; | H, 6.33; | N, 3.42; | F, 9.12. |

mp 99-100.5°C.

### Example 233

### 2,7-Difluoro-9-(3-phenylpropyl)-N-(4-pyridinylmethyl)-9H-fluorene-9-carboxamide

MS (electrospray, M+H)⁺ m/z 455⁺.

| | | | |
|---|---|---|---|
| Anal. Calcd for C₂₉H₂₄N₂F₂O•0.25 H₂O | C, 75.88; | H, 5.38; | N, 6.10 |
| Found | C, 75.93; | H, 5.15; | N, 6.04. |

mp 60-62°C.

### Example 234

### 9-(Butylthio)-9-propyl-9H-fluorene

MS (CI-NH₃, + ions) m/e 297 (M+H), 207 (M+H-C₄H₁₀S).

| | | | |
|---|---|---|---|
| Anal. Calcd for C₂₀H₂₄S | C, 81.03; | H, 8.16; | N, 10.81 |
| Found | C, 81.40; | H, 8.47; | N, 10.85. |

### Example 235

### 9-(Butylsulfinyl)-9-propyl-9H-fluorene

MS (ES, + ions) m/e 625 (2M+H), 313 (M+H).

| | | | |
|---|---|---|---|
| Anal. Calcd for C₂₀H₂₄SO | C, 76.88; | H, 7.74; | N, 10.26 |
| Found | C, 77.12; | H, 7.78; | N, 9.93. |

mp 57-59°C.

### Example 236

### 9-(4-Hydroxybutyl)-N-propyl-9H-fluorene-9-carboxamide

MS (CI-NH₃, + ions) m/e 324 (M+H).

| | | | |
|---|---|---|---|
| Anal. Calcd for C₂₁H₂₅NO₂ | C, 77.99; | H, 7.79; | N, 4.33 |
| Found | C, 77.89; | H, 7.92; | N, 4.35. |

mp 73-75°C.

### Example 237

### 9-[4-(Phenylthio)butyl]-N-propyl-5H-fluorene-9-carboxamide

MS (CI-NH₃, + ions) m/e 416 (M+H).

| | | | | |
|---|---|---|---|---|
| Anal. Calcd for C₂₇H₂₉NOS | C, 78.03; | H, 7.03; | N, 3.37; | S, 7.71 |
| Found | C, 77.70; | H, 7.26; | N, 3.35; | S, 7.51. |

mp 50-53°C.

### Example 238

### 9-[3-(1,3-Dioxan-2-yl)propyl]-N-propyl-9H-fluorene-9-carboxamide

MS (CI-NH₃, + ions) m/e 380 (M+H).

| | | | |
|---|---|---|---|
| Anal. Calcd for C₂₄H₂₉NO₃ + 0.32 mol H₂O | C, 74.82; | H, 7.75; | N, 3.64 |
| Found | C, 74.75; | H, 7.33; | N, 3.64. |

mp 127-128°C.

### Example 239

### 9-[3-(1,3-Dioxolan-2-yl)propyl]-N-propyl-9H-fluorene-9-carboxamide

MS (CI-NH₃, + ions) m/e 366 (M+H).

| | | | |
|---|---|---|---|
| Anal. Calcd for C₂₃H₂₇NO₃ | C, 75.59; | H, 7.45; | N, 3.83 |
| Found | C, 75.23; | H, 7.63; | N, 3.76. |

mp 88-90°C.

### Example 240

### cis-N,9-Dipropyl-1H-thioxanthene-9-carboxamide, 10-oxide

MS (CI-NH₃, + ions) m/e 342 (M+H).

| | | | |
|---|---|---|---|
| Anal. Calcd for C₂₀H₂₃NO₂S | C, 70.35; | H, 6.79; | N, 4.10 |
| Found | C, 70.25; | H, 6.86; | N, 4.10. |

mp 201-204°C.

### Example 241

### 5-(2-Propenyl)-N-propyl-5H-indeno[1,2-b]pyridine-5-carboxamide

MS (CI, M+H)⁺ m/z 293⁺.

| | | | |
|---|---|---|---|
| Anal. Calcd for C₁₉H₂₀N₂O • 0.1 H₂O | C, 77.58; | H, 6.92; | N, 9.52 |
| Found | C, 77.50; | H, 6.84; | N, 9.57. |

mp 131-133.5°C.

### Example 242

### (E)-5-(3-Phenyl-2-propenyl)-N-propyl-5H-indeno[1,2-b]pyridine-5-carboxamide

mp 153-154.5
MS (CI, M+H)⁺ m/z 369⁺.

| | | | |
|---|---|---|---|
| Anal. Calcd for C₂₅H₂₄N₂O | C, 80.32; | H, 6.63; | N, 7.49 |
| Found | C, 80.26; | H, 6.51; | N, 7.55. |

### Example 243

### N-Ethyl-N-methyl-9-(2-propenyl)-9H-fluorene-9-carboxamide

MS (CI, M+H)⁺ m/z 292.

| | | | |
|---|---|---|---|
| Anal. Calcd for C₂₀H₂₁NO • 0.06 dioxane | C, 81.94; | H, 7.30; | N, 4.72 |
| Found | C, 81.76; | H, 7.39; | N, 4.68. |

### Example 244

### N,9-Dipropyl-9H-thioxanthene-9-carboxamide, 10,10-dioxide

MS (CI-NH₃, + ions) m/z 380 (M+Na) 375 (M+NH₄), 358 (M+H).

| | | | |
|---|---|---|---|
| Anal. Calcd for C₂₀H₂₃NO₃S + 0.6 CH₂Cl₂ | C, 60.58; | H, 5.97; | N, 3.43 |
| Found | C, 60.58; | H, 5.79; | N, 3.39. |

mp 264-266°C.

### Example 245

### trans-N,9-Dipropyl-9H-thioxanthene-9-carboxamide, 10-oxide

MS (CI-NH₃, + ions) m/z 342 (M+H).

| | | | |
|---|---|---|---|
| Anal. Calcd for C₂₀H₂₃NO₂S + 0.4 H₂O | C, 68.92; | H, 6.88; | N, 4.02 |
| Found | C, 68.96; | H, 7.18; | N, 3.98. |

mp 147-150°C.

### Example 246

### 9-[3-(Dibutoxyphosphinyl)propyl]-N-(2-pyridinylmethyl)-9H-fluorene-9-carboxamide

CI-Mass Spec. (M+H)=535.

| | | | | |
|---|---|---|---|---|
| Anal. Calcd for C₃₁H₃₉N₂PO₄•0.5 H₂O | C, 68.48; | H, 7.42; | N, 5.15; | P, 5.70 |
| Found | C, 68.28; | H, 7.23; | N, 5.28; | P, 5.50. |

### Example 247

### 1-(9-Propyl-9H-fluorene-9-yl)-2-(1-piperidinyl)-ethanone, monohvdrochloride

MS (ES) 334 (M+H).

| | | | |
|---|---|---|---|
| Anal. Calcd for C₂₃H₂₈ClNO • H₂O | C, 71.21; | H, 7.79; | N, 3.61 |
| Found | C, 71.01; H, | 7.75; | N, 3.93. |

### Example 248

### N-(5-Hydroxypentyl)-9-propyl-9H-fluorene-9-carboxamide

MS (CI, + ions) m/z 338 (M+H).

| | | | |
|---|---|---|---|
| Anal. Calcd for C₂₂H₂₇NO₂ + 0.3 H₂O | C, 77.13; | H, 8.11; | N, 4.09 |
| Found | C, 77.10; | H, 8.23; | N, 4.00. |

mp 48.51°C.

### Example 249

### 9-(3-Cyanopropyl)-N-propyl-9H-fluorene-9-carboxamide

MS (ES, + ions) m/z 319 (M+H).

| | | | |
|---|---|---|---|
| Anal. Calcd for C₂₁H₂₂N₂O | C, 79.21; | H, 6.96; | N, 8.80 |
| Found | C, 78.98; | H, 6.89; | N, 8.68. |

mp 80-83°C.

### Example 250

### N-[[4-[[(9-Propyl-9H-fluoren-9-yl)carbonyl]amino]-phenyl]methyl]-9-propyl-9H-fluorene-9-carboxamide

MS (CI, + ions) 591 (M+H).

| | | | |
|---|---|---|---|
| Anal. Calcd for C₄₁H₃₈N₂O₂ • 0.3 H₂O | C, 82.60; | H, 6.53; | N, 4.70 |
| Found | C, 82.62; | H, 6.44; | N, 4.64. |

mp 188-190°C.

### Example 251

### N-[4-(4-Aminophenyl)methyl]-9-propyl-9H-fluorene-9-carboxamide

MS (ES, + ions) 357 (M+H).

| | | | |
|---|---|---|---|
| Anal. Calcd for C₂₄H₂₄N₂O • 0.7 H₂O | C, 78.10; | H, 6.94; | N, 7.59 |
| Found | C, 78.26; | H, 6.70; | N, 7.48. |

mp 96-99°C.

### Example 252

### 9-[3-(Dibutoxyphosphinyl)propyl]-N-propyl-9H-fluorene-9-carboxamide

MS (CI-NH₃, + ions) m/e 486 (M+H).

| | | | | |
|---|---|---|---|---|
| Anal. Calcd for C₂₈H₄₀NO₄P + 0.75 mol • H₂O | C, 67.37; | H, 8.38; | N, 2.81; | P, 6.21 |
| Found | C, 67.49; | H, 8.28; | N, 2.69; | P, 6.45. |

### Example 253

### 4-(1-Piperidinyl)-1-(9-propyl-9H-fluoren-9-yl)-1-butanone, monohvdrochloride

MS (ES)362 (M+H).

| | | | | |
|---|---|---|---|---|
| Anal. Calcd for C₂₅H₃₂ClNO | C, 75.45; | H, 8.10; | N, 3.52; | Cl, 8.91 |
| Found | C, 75.41; | H, 8.18; | N, 3.36; | Cl, 8.72. |

mp 148-150°C.

### Example 254

### N-Methyl-9-(3-phenylpropyl)-9H-fluorene-9-carboxamide

MS (CI, + ions) m/z 342 (M+H).

| | | | |
|---|---|---|---|
| Anal. Calcd for C₂₄H₂₃NO + 0.2 H₂O | C, 83.51; | H, 6.84; | N, 4.06 |
| Found | C, 83.55; | H, 6.69; | N, 4.02. |

mp 101-102°C.

### Example 255

### 2-(Dimethylamino)-9-(3-phenylpropyl)-N-propyl-9H-fluorene-9-carboxamide

MS (CI, M+H)⁺ m/z 413⁺.

| | | | |
|---|---|---|---|
| Anal. Calcd for C₂₈H₃₂N₂O · 0.34 H₂O | C, 80.32; | H, 7.87; | N, 6.69 |
| Found | C, 80.30; | H, 7.74; | N, 6.71. |

### Example 256

### 9-[4-(Dibutoxyphosphinyl)-2-butenyl]-N-propyl-9H-fluorene-9-carboxamide

MS (ES) 498 (M+H).

| | | | | |
|---|---|---|---|---|
| Anal. Calcd for C₂₉H₄₀NO₄P | C, 70.00; | H, 8.10; | N, 2.81; | P, 6.22 |
| Found | C, 69.85; | H, 8.15; | N, 3.13; | P, 6.19. |

### Example 257

### 9-[4-(4-Nitrophenyl)butyl]-N-propyl-9H-fluorene-9-carboxamide

MS (ES) 429 (M+H).

| | | | |
|---|---|---|---|
| Anal. Calcd for C₂₇H₂₈N₂O₃ | C, 75.68; | H, 6.59; | N, 6.54 |
| Found | C, 75.70; | H, 6.58; | N, 6.57. |

mp 109-110°C.

### Example 258

### 9-[3-(4-Nitrophenyl)-2-propenyl]-N-propyl-9H-fluorene-9-carboxamide

MS (CI, + ions) 413 (M+H).

| | | | |
|---|---|---|---|
| Anal. Calcd for C₂₆H₂₄N₂O₃ • 0.3 H₂O | C, 74.73; | H, 5.93; | N, 6.70 |
| Found | C, 74.54; | H, 5.75; | N, 6.67. |

mp 143-146°C.

### Example 259

### 5-(3-Phenylpropyl)-N-propyl-5H-indeno[1,2-b]pyridine-5-carboxamide

MS (CI, M+H)⁺ m/z 371⁺.

| | | | |
|---|---|---|---|
| Anal. Calcd for C₂₅H₂₆N₂O | C, 81.05; | H, 7.07; | N, 7.56 |
| Found | C, 80.97; | H, 7.12; | N, 7.51. |

mp 124.5-126°C.

### Example 260

### 9-[4-(4-Aminophenyl)butyl]-N-propyl-9H-fluorene-9-carboxamide

MS (CI) 399 (M+H).

| | | | |
|---|---|---|---|
| Anal. Calcd for C₂₇H₃₀N₂O • 0.3 H₂O | C, 80.28; | H, 7.64; | N, 6.93 |
| Found | C, 80.37; | H, 7.53; | N, 7.34. |

### Example 261

### 9-[3-(4-Aminophenyl)propyl]-N-propyl-9H-fluorene-9-carboxamide

MS (CI, + ions) 385 (M+H).

| | | | |
|---|---|---|---|
| Anal. Calcd for C₂₆H₂₈N₂O • 0.3 H₂O | C, 80.09; | H, 7.39; | N, 7.18 |
| Found | C, 80.01; | H, 7.31; | N, 7.17. |

mp 138-140°C.

### Example 262

### 9-[4-(Dibutoxyphosphinyl)butyl]-9H-fluorene-9-carboxvlic acid, methvl ester

MS (CI, + ions) m/z 473 (M+H).

| | | | |
|---|---|---|---|
| Anal. Calcd for C₂₇H₃₇O₅P | C, 68.63; | H, 7.89; | N, 6.55 |
| Found | C, 68.37; | H, 7.96; | N, 6.21. |

### Example 263

### N,N-Dibutyl-9-[(propylamino)carbonyl]-9H-fluorene-9-butanamide

MS (CI-NH₃, + ions) m/e 449 (M+H).

| | | | |
|---|---|---|---|
| Anal. Calcd for C₂₉H₄₀N₂O₂ + 0.29 mol H₂O | C, 76.75; | H, 9.01; | N, 6.17 |
| Found | C, 76.71; | H, 8.92; | N, 6.21. |

mp 109-111°C.

### Example 264

### 9-(5-Cyanopentyl)-N-propyl-9H-fluorene-9-carboxamide

MS (ES, + ions) m/e 347 (M+H).

| | | | |
|---|---|---|---|
| Anal. Calcd for C₂₃H₂₆N₂O | C, 79.73; | H, 7.56; | N, 8.09 |
| Found | C, 79.25; | H, 7.55; | N, 7.76. |

mp 92-94°C.

### Example 265

### 9-[2-[[[4-(1,3-Dihydro-1,3-dioxo-2H-isoindol-2-yl)-phenyl]sulfonyl]amino]ethyl]-N-(2,2,2-trifluoroethyl)-9H-fluorene-9-carboxamide

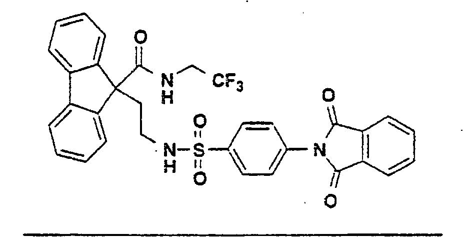

### A.

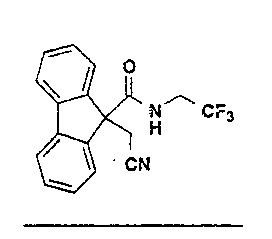

Butyllithium (18 mL, 2.5M in hexanes, 44 mmol) was added dropwise over 10 min to a solution of 9-fluorenecarboxylic acid (4.2 g, 20 mmol) in THF (200 mL) at 0°C under argon. The slightly heterogeneous dark yellow reaction was stirred at 0°C for 30 min, then chloroacetonitrile (1.5 mL, 24 mmol) was added dropwise over 3 min. The orange reaction was stirred at 0°C for 30 min, warmed to RT and stirred for 3 h. The reaction was extracted with water (2 x 100 mL) and the combined aqueous extracts were washed with Et₂O (100 mL). The aqueous layer was acidified to pH<2 with 1N HCl and extracted with CH₂Cl₂ (3 x 50 mL). The combined organic extracts were dried over MgSO₄, filtered, and concentrated in vacuo to give 4.7 g of a light yellow solid (mp 138-145°C).

A portion (2.63 g) of the crude carboxylic acid was dissolved in CH₂Cl₂ (30 mL) under argon. N,N-Dimethylformamide (40 µL, 0.53 mmol) was added followed by oxalyl chloride (8.0 mL, 2.0M in CH₂Cl₂, 15.9 mmol). The reaction bubbled for a few minutes and was allowed to stir at RT for 1.5 h. The reaction was concentrated in vacuo then pumped under high vacuum to give the crude acid chloride. Triethylamine (4.4 mL, 31.8 mmol) was added to a suspension of 2,2,2-trifluoroethylamine hydrochloride (1.71 g, 12.7 mmol) in CH₂Cl₂ (20 mL) at 0°C under argon. The resulting thick slurry was stirred at 0 °C for 5 min, then a solution of the crude acid chloride in CH₂Cl₂ (10 mL) was added dropwise over 5 min. The reaction was stirred at 0°C for 10 min, diluted with CH₂Cl₂ (50 mL), washed with 1N HCl (2 x 20 mL) and saturated NaHCO₃ (30 mL), then dried over Na₂SO₄. Evaporation gave 3.5 g of a yellow foam which was purified by flash chromatography on silica (150 g) eluting with CH₂Cl₂ to give title compound (2.74 g, 76%) as a white solid (mp 159-159.5).

### B.

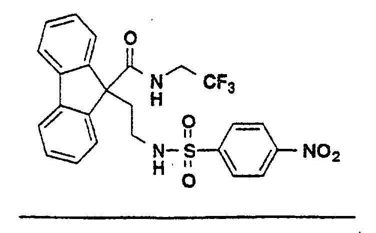

Platinum (IV) oxide (107 mg, 0.472 mmol) was added to a solution of Part A compound (1.50 g, 4.72 mmol) and chloroform (750 µL, 9.44 mmol) in MeOH (15 mL). The reaction mixture was hydrogenated (balloon) at RT for 3.5 days, filtered through Celite, and concentrated in vacuo to provide 1.71 g of the crude amine hydrochloride.

To a solution of the crude amine hydrochloride and triethylamine (800 µL, 5.80 mmol) in CH₂Cl₂ (7 mL) at 0°C under argon was added a solution of 4-nitrobenzenesulfonyl chloride (612 mg, 2.77 mmol) (recrystallized from hexane prior to use) in CH₂Cl₂ (1 mL). The cloudy reaction was stirred at 0°C for 15 min, diluted with CH₂Cl₂ (10 mL), washed with saturated NaHCO₃ (2 x 5 mL), then dried over MgSO₄. Evaporation gave 1.36 g of a yellow foam which was dissolved in 1:1 CH₂Cl₂:30% EtOAc/hexane and purified by flash chromatography on silica (150 g) eluting with a step gradient of 30-50% EtOAc/hexane to give title compound (783 mg, 59%) as a white solid (mp 164.5-165.5).

### C.

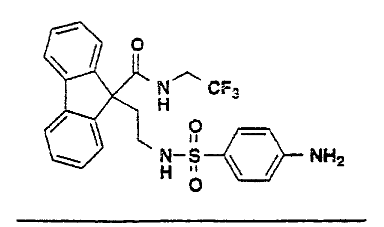

A mixture of Part B compound (760 mg, 1.46 mmol) and 10% palladium on carbon (77 mg, 0.073 mmol) in EtOAc (8 mL) was hydrogenated (balloon) at RT for 2.5 h, filtered through Celite with the aid of EtOAc (50 mL), and concentrated in vacuo to provide title compound (728 mg, 100%) as a white foam. A sample of title compound was diluted with CH₂Cl₂, concentrated in vacuo, and pumped under high vacuum to give title compound as a white solid (mp 184-186°C).

### D. 9-[2-[[[4-(1,3-Dihydro-1,3-dioxo-2H-isoindol-2-yl)-phenyl]sulfonyl]amino] ethyl]-N-(2,2,2-trifluoroethyl)-9H-fluorene-9-carboxamide

A solution of Part C compound (290 mg, 0.593 mmol) and phthalic anhydride (92 mg, 0.623 mmol) in N,N-dimethylacetamide (1 mL) was heated at 150°C under argon for 9 h, then cooled to RT. The solvent was distilled off under high vacuum and the amber oily residue was purified by flash chromatography on silica gel (50 g) eluting with 5% EtOAc/CH₂CH₂ to provide title compound (300 mg, 82%) as a white solid.
mp 235-237°C

| | | | | | |
|---|---|---|---|---|---|
| Anal. Calcd. for C₃₂H₂₄F₃N₃O₅S • 0.4 H₂O | C, 61.31; | H, 3.99; | N, 6.78; | F, 9.20; | S, 5.17 |
| Found | C, 61.37; | H, 3.85; | N, 6.64; | F, 8.81; | S, 5.36. |

### Example 266

### (Z)-9-[4-[(6-Ethoxy-2-benzothiazolyl)thio]-2-butenyl]-N-propyl-9H-fluorene-9-carboxamide

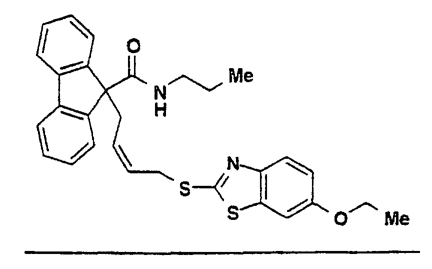

### A.

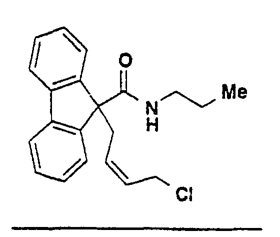

Butyllithium (8.4 mL, 2.5M in hexane, 21 mmol) was added dropwise over 10 min to a solution of 9-fluorenecarboxylic acid (2.10 g, 10 mmol) in THF (50 mL) at 0°C under argon. During addition of the first equivalent of BuLi, the reaction became thick with a white precipitate which became yellow and cleared after addition of the second equivalent. The reaction was stirred at 0°C for 20 min, then cis-1,4-dichloro-2-butene (1.2 mL, 11 mmol) was added dropwise over 5 min. The reaction lightened in color during addition and was stirred at 0°C for 3 h, then poured into 1N HCl (50 mL) and extracted with CH₂Cl₂ (3 x 50 mL). The combined organic layers were washed with brine (30 mL) then dried over MgSO₄. Evaporation provided 3.5 g of a yellow oil containing crystalline solid. The crude residue was triturated with hexane (20 mL). The supernatant was decanted, and the residue pumped under high vacuum to give 2.93 g of a tan solid.

To a suspension of the crude acid prepared above (1.42g, 4.77 mmol) and N,N-dimethylformamide (5 drops) in CH₂Cl₂ (15 mL) at room temperature under argon was added oxalyl chloride (3.6 mL, 2.0M in CH₂Cl₂, 7.16 mmol). The reaction bubbled for 10 min, then the reaction was stirred at room temperature for 1.5 h, at which time all solids had dissolved. The reaction was concentrated in vacuo to give an orange oil. The crude acid chloride was dissolved in CH₂Cl₂ (15 mL) and cooled to 0°C. Propylamine (1.2 mL, 14.3 mmol) was added dropwise over 1 min, and the reaction was stirred at 0°C for 10 min. The reaction was partitioned between EtOAc (50 mL) and water (20 mL). The organic layer was washed with 1N HCl (2 x 20 mL) and brine (20 mL), then dried over MgSO₄. Evaporation gave 1.7 g of an orange oil, which was purified by flash chromatography on silica gel (150 g) eluting with CH₂Cl₂ to give title compound (1.38 g, 84%) as a pale yellow oil.

### B. (Z)-9-[4-[(6-Ethoxy-2-benzothiazolyl)-thio]-2-butenyl]-N-propyl-9H-fluorene-9-carboxamide

To a solution of 500 mg (1.47 mmol) of Part A compound in 5 mL of DMF, under argon at RT, was added 400 mg (2.94 mmol) of K₂CO₃ followed by 466 mg (2.20 mmol) of 6-ethoxy-2-mercaptobenzothiazole. The reaction was stirred for 5 h at RT, at which time it was heated to 50°C for 16 h. The reaction was diluted with ether and the organics were washed with water (2x), brine, dried (Na₂SO₄) and evaporated. Flash chromatography was performed on 100 g of silica gel eluting with 3:2 hexanes/ethyl acetate to provide 450 mg (60%) of title compound as a biege solid.
mp 135-137°C.

| | | | | |
|---|---|---|---|---|
| Anal. Calcd. for C₃₀H₃₀N₂O₂S₂ + 0.55 mol H₂O | C, 68.68; | H, 5.98; | N, 5.34; | S, 12.22 |
| Found | C, 68.88; | H, 5.77; | N, 5.14; | S, 12.26. |

### Example 267

### 9-[4-(Dibutoxyphosphinyl)butyl]-N-(2,2,2-trifluoropropyl)-9H-xanthene-9-carboxamide

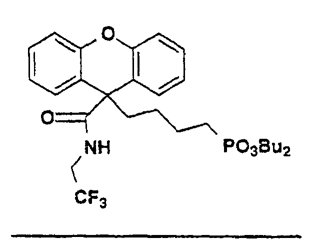

### A.

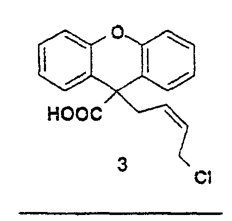

To a stirred solution of 5.00 g (22.1 mmol) of xanthene carboxylic acid in 100 mL of THF at 0°C was added 19.5 mL (48.7 mmol) of 2.5 M butyllithium in hexanes followed by 3.05 g (24.32 mmol) of cis-1,4-dichloro-2-butene. The reaction was allowed to stir at 0°C for 24 h when the mixture was diluted with 250 mL of ethyl acetate and 100 mL or 0.5 M HCl. The layers were separated, the organics dried (Na₂SO₄) and concentrated. The remainder was purified by flash column chromatography on silica gel (250 g) eluting with 30:70:0.5 ethyl acetate/hexanes/acetic acid to give 4.6 g (66%) of title compound as a white solid. mp 134-135°C.

### B.

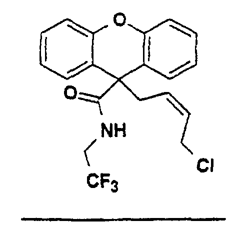

To a stirred solution of 2.00 g (6.35 mmol) of Part A compound in 100 mL of dichloromethane at RT was added 3.6 mL (7.2 mmol) of 2M oxalyl chloride in dichloromethane followed by 2 drops of DMF. The reaction was allowed to stir at RT for 2.5 h when the solvent was evaporated and the semisolid residue pumped (= 1 mm pressure) for 0.5 h. The residue was dissolved by adding 300 mL of THF and cooled to 0°C. The mixture was treated with 0.9 g (7 mmol) of trifluoroethylamine hydrochloride and 1.41 g (14 mmol) of triethylamine and warmed to room temperature. The mixture was stirred overnight and diluted with 150 mL of ethyl acetate and 50 mL of 0.5 M HCl. The layers were separated, the organics dried (Na₂SO₄) and concentrated. The remainder was purified by trituration with hot methanol to give 1.30 g (52%) of title compound as a white solid.
mp 153-159°C.

### C.

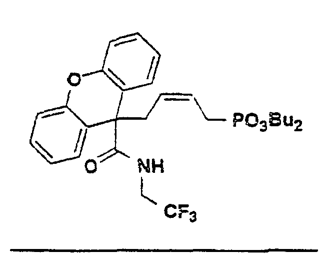

A mixture of Part B compound (0.53 g, 1.34 mmol) and tributylphosphite (3.00 g, 12 mmol) was heated to 115-120°C for 24 h. The mixture was concentrated by bulb-to-bulb distillation to leave an amber colored oil. The remainder was purified by flash column chromatography on silica gel (60 g) eluting with 9:1 dichloromethane/acetone to give 0.65 g (86%) of title compound as a colorless oil.
TLC Silica gel (9:1 dichloromethane/acetone)
R_{f}= 0.4.

### D. 9-[4-(Dibutoxyphosphinyl)butyl]-N-(2,2,2-trifluoropropyl)-9H-xanthene-9-carboxamide

A solution of Part C compound (0.60 g, 1.06 mmol) in ethanol (10 mL) was treated ith 40 mg of 10% Pd/Carbon and placed under an atm of H₂ for 18 h. The mixture was diluted with 25 mL of ethanol and filtered through a pad of Celite. The filtrate was concentrated to an oil which gradually solidified to give 0.32 g (91%) of title compound as a colorless oil which gradually turned to a white solid on standing. mp 102-105°C.
Mass Spec. (ES, + ions) m/z 573 (M+NH₄), 556 (M+H)

| | | | | |
|---|---|---|---|---|
| Anal. Calc'd for C₂₈H₃₇NO₅PF₃ + 0.65 H₂O | C, 59.25; | H, 6.81; | N, 2.47; | P, 5.46 |
| Found | C, 59.59; | H, 6.53; | N, 2.14; | P, 5.03. |

### Example 270

### 9-[4-(Dibutoxyphosphinyl)butyl)-2,7-difluoro-N-(2,2,2-trifluoroethyl)-9H-fluorene-9-carboxamide

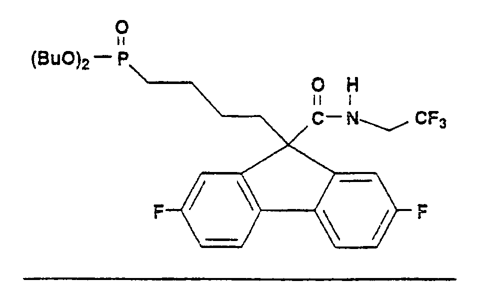

A solution of Example 203 compound (574 mg, 1 mmol) in 25 ml of absolute ethanol containing 250 mg of 10% Pd on carbon as catalyst was stirred under a hydrogen atmosphere (balloon) for 48 hours. The reaction was filtered after stirring 24 hrs and fresh catalyst added. The reaction was filtered through a 0.45 µm nylon filter and the solvent evaporated yielding 538 mg (94%) of title compound as a colorless oil.
Mass Spec (CI) _{°} m/z 576 (M+H).

| | | | | | |
|---|---|---|---|---|---|
| Anal Calc'd for C₂₈H₃₅NF₅PO₄ | C, 58.43; | H, 6.13; | N, 2.43; | F, 16.50; | P, 5.38 |
| Found | C, 58.54; | H, 5.86; | N, 2.39; | F, 16.41; | P, 5.39. |

### Example 272

### (E)-9-[4-(Dibutoxyphosphinyl)-2-butenyl]-N-propyl-9H-fluorene-9-carboxamide

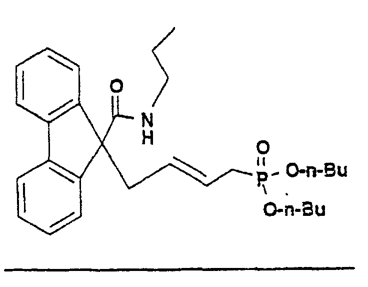

### A.

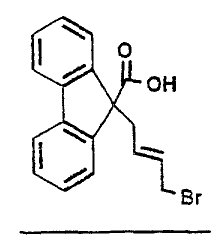

To a THF (150 ml) suspension of 9-fluorenecarboxylic acid (10 g, 0.048 mol) at 0°C under argon was added dropwise sodium bis(trimethylsilyl)amide (100 ml, 1 M in THF). After 30 min, 1,4-trans-2-butene (10.2 g, 0.048 mol) was added and the reaction allowed to stir for 1 h. The reaction mixture was quenched with 1N HCl and the aqueous layer extracted 3 times with EtOAc. The combined organics were dried over Na₂SO₄ and evaporated *in vacuo* to give an oily-solid residue (18 g). The residue was purified by flash column chromatography (SiO₂, 10 by 25 cm), eluting with 6.5% MeOH:CH₂Cl₂ to give title compound (2.48 g, 15% yield) as an oily solid. MS: (CI, M+NH₄⁺): m/z 360⁺.

### B.

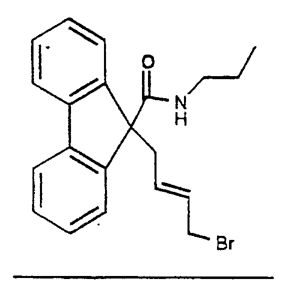

To a CH₂Cl₂ (30 ml) solution at 0°C of Part A compound (2.48 g, 7.22 mmol) under argon was added oxalyl chloride (1.46 g, 11.4 mmol) and DMF (0.1 ml). The reaction mixture was stirred at room temperature for 2.5 h and the volatiles were removed *in vacuo*. The crude residue containing acid chloride was co-evaporated with CH₂Cl₂ and used directly in the following reaction.

To a THF (26 ml) solution of the acid chloride (7.22 mmol) at 0°C under argon was added n-propyl-amine (0.899 g, 15.2 mmol) and the reaction was stirred for 1.45 h. After warming to room temperature for 15 min, the mixture was stored at -80°C overnight. The reaction mixture was partitioned between EtOAc and water, the aqueous layer extracted twice with EtOAc, the combined organics washed with brine, dried over Na₂SO₄, and evaporated to give title compound (2.79 g, >100% crude recovery, containing EtOAc) as a slightly orange colored oil. MS: (CI, M+H⁺): m/z 384⁺.

### C. (E)-9-[4-(Dibutoxyphosphinyl)-2-butenyl]-N-propyl-9H-fluorene-9-carboxamide

A solution of Part B compound (977 mg, 2.54 mmol) and tri-*n*-butyl phosphite (2.75 ml) under argon was heated at 120°C for 17 h. The volatiles were removed *in vacuo* to give an oil (1.26 g). The residue was purified by flash column chromatography (SiO₂, 5 by 10 cm), eluting with 2.5% MeOH:CH₂Cl₂, to give after heating at 70°C *in vacuo* overnight title compound (120 mg, 10% yield from Part A compound) as a colorless oil. The bulk of title compound was isolated as colorless oil containing residual tri-*n*-butyl phosphite (1.07 g).
MS: (CI, M+H⁺): m/z 498.

| | | | |
|---|---|---|---|
| Anal. Calc. for C₂₉H₄₀NO₄P • 0.90 H₂O | C, 67.78; | H, 8.20; | N, 2.73 |
| Found | C, 67.75; | H, 7.91; | N, 2.76. |

### Example 273

### 9-[4-[4-(Benzoylamino)-1H-imidazol-1-yl]butyl]-N-(2,2,2-trifluoroethyl)-9H-fluorene-9-carboxamide

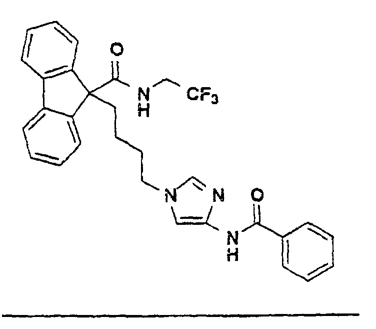

### A.

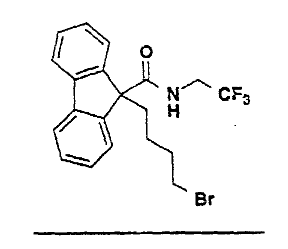

### A(1).

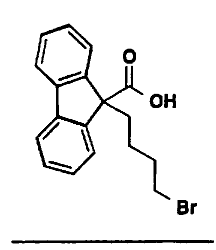

To a solution of 9-fluorenecarboxylic acid (50 g, 240 mmol) in THF (1200 mL) at 0°C was added dropwise a solution of n-butyllithium (2.5M, 211 mL, 530 mmol) in THF. The yellow reaction was stirred at 0°C for 1 h, then 1,4-dibromobutane (31.3 mL, 260 mmol) was added dropwise over 30 min. The reaction was stirred at 0°C for 30 min, then the reaction was warmed to RT for 30 h. The reaction was extracted with water (3 x 750 mL). The combined aqueous layers were extracted with ethyl ether (800 mL). The aqueous layer was made acidic with HCl solution (1N, 500 mL), then extracted with dichloromethane (3 x 750 mL). The combined organic layers were dried over MgSO₄. Evaporation gave title compound (71 g, 85%) as a white solid.

### A(2).

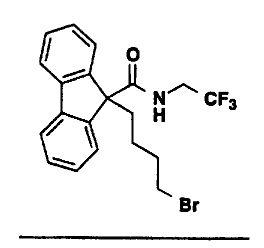

To a solution of Part A(1) acid (60 g, 173 mmol) and DMF (100 µL) in CH₂Cl₂ (600 mL) under argon at 0°C was added oxalyl chloride (104 mL, 2.0M in CH₂Cl₂, 208 mmol) dropwise. The reaction was stirred at 0°C for 10 min, then warmed to room temperature and stirred for 1.5 h. The reaction was concentrated in vacuo to give the crude acid chloride as a yellow oil. To a suspension of 2,2,2-trifluoroethylamine hydrochloride (25.9 g, 191 mmol) in CH₂Cl₂ (500 mL) at 0°C under argon was added triethylamine (73 mL, 521 mmol) followed by dropwise addition of a solution of the crude acid chloride in CH₂Cl₂ (15 mL). The reaction was stirred at 0°C for 1 h, diluted with CH₂Cl₂ (500 mL), and washed with water (2 x 300 mL), 1N HCl (2 x 300 mL), saturated NaHCO₃ (2 x 300 mL), and brine (2 x 300 mL), then dried over MgSO₄. Evaporation gave 80 g of a oil which was purified by flash chromatography on silica gel (2.5 kg). The crude product was loaded in a mixture of CH₂Cl₂ and hexane, and eluted with a step gradient of 10% EtOAc/hexane (4L) to 15% EtOAc/hexane (2L) to 20% EtOAc/hexane (4L). Pure fractions were combined and evaporated to give title compound (52.5 g, 71%) as a white solid (mp 88-92°C).

### B.

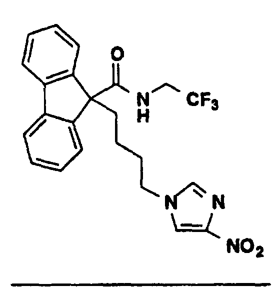

A mixture of Part A (1.55 g, 3.64 mmol), 4-nitroimidazole (452 mg, 4.00 mmol), and anhydrous potassium carbonate (552 mg, 4.00 mmol) in DMF (5 mL) was heated at 50°C under argon for 6 h, cooled to RT, and the solvent was removed in vacuo. The yellow residue was partitioned between EtOAc (50 mL) and water (10 mL). The aqueous layer was extracted with EtOAc (3 mL). The combined organic extracts were washed with water (3 x 10 mL) and brine (20 mL), then dried over Na₂SO₄. Evaporation gave 1.77 g of a foamy gum, which was purified by flash chromatography on silica gel (120 g) eluting with 15% EtOAc/CH₂CH₂ to provide title compound (1.51 g, 91%) as a white foam.

### C. 9-[4-[4-(Benzoylamino)-1H-imidazol-1-yl]butyl]-N-(2,2,2-trifluoroethyl)-9H-fluorene-9-carboxamide

Palladium on carbon (10%) (35 mg, 0.033 mmol) was added to a solution of Part B compound (300 mg, 0.655 mmol) in dry EtOAc (2 mL), and the mixture was hydrogenated (balloon) at RT overnight. The reaction was degassed with argon, cooled to 0°C, and benzoyl chloride (83 µL, 0.72 mmol) was added dropwise. The reaction was stirred at 0 °C for 20 min, filtered through Celite, and washed with EtOAc (5 mL). The brown filtrate was washed with saturated NaHCO₃ (2 x 2 mL) and brine (1 mL), then dried over Na₂SO₄. Evaporation gave 282 mg of a dark brown oil, which was purified by flash chromatography on silica gel (50 g) eluting with 2% MeOH/CH₂CH₂ to provide title compound (253 mg, 73%) as a brown foam.
MS (ES): 533 [M+H]

| | | | | |
|---|---|---|---|---|
| Anal. Calcd. for C₃₀H₂₇F₃N₄O₂ • 0.5 H₂O | C, 66.53; | H, 5.21; | N, 10.35; | F, 10.52 |
| Found | C, 66.60; | H, 5.13; | N, 10.19; | F, 10.86. |

### Example 274

### 9-[4-[5-(Benzoylamino)-2-pyridinyl]butyl]-N-(2,2,2-trifluoroethyl)-9H-fluorene-9-carboxamide

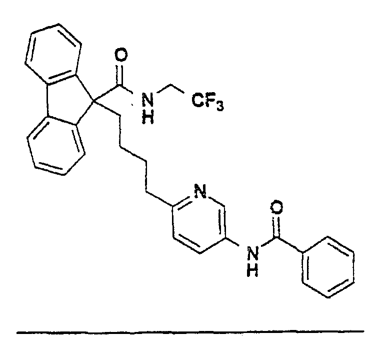

### A.

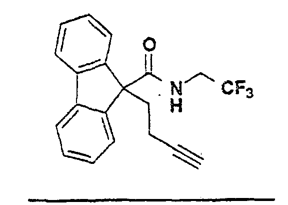

Butyllithium (12.6 mL, 31.5 mmmol) was added dropwise over 5 min to a solution of 9-fluorene-carboxylic acid (3.0 g, 14.3 mmol) in THF (150 mL) at 0°C under argon. The reaction went cloudy during addition, then cleared upon completion. The reaction was stirred at 0°C for 30 min, then 3-butynyl p-toluenesulfonate (9.6 g, 42.9 mmol) was added dropwise. The amber reaction was warmed to RT, then stirred for 24 h. The reaction solution was extracted with water (2 x 75 mL). The combined aqueous layers were washed with Et₂O (50 mL), then acidified with 1N HCl (30 mL). The cloudy mixture was extracted with CH₂Cl₂ (2 x 50 mL), and the combined organic layers were dried over MgSO₄. Evaporation gave 1.85 g of a crude orange gummy solid.

A portion (1.75 g) of crude acid product was dissolved in CH₂Cl₂ (20 mL) under argon. A catalytic amount of DMF (26 µL, 0.33 mmol) was added, followed by oxalyl chloride (5.0 mL, 2.0 M in CH₂Cl₂, 10 mmol) slowly. After bubbling for a few minutes, the reaction was stirred at RT for 1.5 h, then concentrated in vacuo. The crude acid chloride was dissolved in CH₂Cl₂ (20 mL) and added dropwise to a suspension of 2,2,2-trifluoroethylamine hydrochloride (1.08 g, 8.02 mmol) and triethylamine (2.8 mL, 20 mmol) in CH₂Cl₂ (30 mL) at 0°C under argon. The reaction was stirred at 0°C for 10 min, diluted with CH₂Cl₂ (50 mL), washed with 1N HCl (2 x 20 mL) and saturated NaHCO₃ (20 mL), then dried over Na₂SO₄. Evaporation gave 2.24 g of a dark orange semi-solid, which was dissolved in 2:1 CH₂Cl₂:10% EtOAc/hexane and purified by flash chromatography on silica gel (175 g) eluting with 10% EtOAc/hexane to provide title compound (1.16 g, 22%) as a yellow solid (mp 109-113°C).

### B.

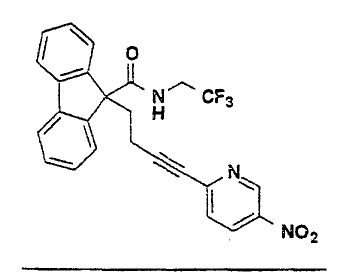

Copper (I) iodide (4 mg, 0.02 mmol) was added to a solution of Part A compound (343 mg, 1 mmol) and 2-bromo-5-nitropyridine (203 mg, 1 mmol) in a mixture of triethylamine (3 mL) and DMF (2 mL). The yellow solution was degassed with argon then cooled to 0°C. Bis(triphenylphosphine)-palladium (II) chloride (14 mg, 0.02 mmol) was added and the reaction was stirred at 0°C for 10 min then at RT for 6 h. The reaction was diluted with water (20 mL) and extracted with EtOAc (2 x 20 mL). The combined organic layers were washed with water (3 x 10 mL) then dried over K₂CO₃. Evaporation gave 520 mg of a brown foamy gum, which was purified by flash chromatography on silica gel (65 g) eluting with 20% EtOAc/hexane to provide title compound (342 mg, 74%). as a yellow foam.

### C. 9-[4-[5-(Benzoylamino)-2-pyridinyl]-butyl]-N-(2,2,2-trifluoroethyl)-9H-fluorene-9-carboxamide

A mixture of Part B compound (334 mg, 0.718 mmol) and 10% palladium on carbon (38 mg, 0.036 mmol) in EtOAc (2 mL) was hydrogenated (balloon) at RT for 6 h, filtered through Celite with the aid of EtOAc (30 mL), then concentrated in vacuo to give 292 mg of the aminopyridine as a brown gum.

A portion of amine (262 mg, 0.597 mmol) was dissolved in CH₂Cl₂ (3 mL), cooled to 0°C under argon, then treated sequentially with triethylamine (125 µL, 0.896 mmol) and benzoyl chloride (77 µL, 0.658 mmol) dropwise. The reaction was stirred at 0°C for 1 h, diluted with CH₂Cl₂ (5 mL), washed with saturated NaHCO₃ (2 x 1 mL) and brine (1 mL), then dried over Na₂SO₄. Evaporation gave 360 mg of a green foam, which was purified by flash chromatography on silica gel (50 g) eluting with 50% EtOAc/hexane to give 192 mg of impure product as a yellow glassy foam. The product was further purified by recrystallization from EtOAc/hexane. The first two crops were combined and dried in a vacuum oven at 50°C overnight to afford title compound (90 mg, 21%) as an off-white solid.
mp 166-169°C.
MS (ES): 544 [M+H].

| | | | |
|---|---|---|---|
| Anal. Calcd. for C₃₂H₂₈F₃N₃O₂ • 0.3 H₂O | C, 70.01; | H, 5.25; | N, 7.65 |
| Found | C, 70.06; | H, 4.98; | N, 7.33. |

### Example 275

### 9-[4-[4-[(2-Phenoxybenzoyl)amino]-1H-imidazol-1-yl]-butyl]-N-(2,2,2-trifluoroe,thyl)-9H-fluorene-9-carboxamide

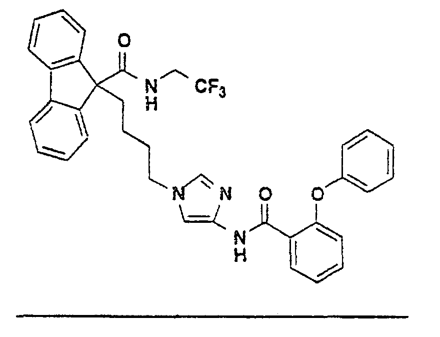

### A. 2-Phenoxybenzoic Acid Chloride

To a solution of 2-phenoxybenzoic acid (500 mg, 2.33 mmol) and DMF (1 drop) in CH₂Cl₂ (10 mL) under argon was added oxalyl chloride (1.3 mL, 2.0M in CH₂Cl₂, 2.6 mmol) dropwise. Bubbling of escaping gasses continued for 5 min after addition. The reaction was stirred at room temperature for 1 h, then concentrated in vacuo to give the title compound as a crude pale yellow oil.

### B. 9-[4-[4-[(2-Phenoxybenzoyl)amino]-1H-imidazol-1-yl]butyl]-N-(2,2,2-trifluoroethyl)-9H-fluorene-9-carboxamide

Palladium on carbon (10%) (74 mg, 0.07 mmol) was added to a solution of Example 273 Part B compound (640 mg, 1.4 mmol) in dry EtOAc (5 mL), and the mixture was hydrogenated (balloon) at RT overnight. The reaction was degassed with argon, cooled to 0°C, and triethylamine (290 µL, 2.10 mmol) was added. A solution of Part A acid chloride in CH₂Cl₂ (2 mL) was added dropwise over 5 min. The resulting thick reaction was stirred at 0°C for 30 min and filtered through Celite. The filter cake was rinsed with CH₂Cl₂ (3 x 20 mL). The filtrate was washed with saturated NaHCO₃ (10 mL) and brine (10 mL), then dried over MgSO₄. Evaporation gave 1.0 g of a dark brown foam, which was purified by flash chromatography on silica gel (75 g) eluting with 2% MeOH/CH₂Cl₂ to provide title compound (670 mg, 77%) as a yellow foam.
MS (ES): 625 [M+H].

| | | | | |
|---|---|---|---|---|
| Anal. Calcd. for C₃₆H₃₁F₃N₄O₃ | C, 69.22; | H, 5.00; | N, 8.97; | F, 9.12 |
| Found | C, 68.84; | H, 4.90; | N, 8.80; | F, 8.80. |

### Example 276

### 9-[4-[(2-Bromo-5-pyridinyl)amino]butyl]-N-propyl-9H-fluorene-9-carboxamide

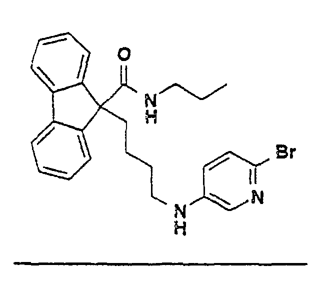

### A.

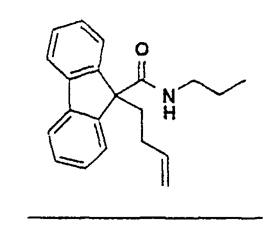

The title compound was prepared from 9-fluorenecarboxylic acid (4.2g, 20 mmol) and 4-bromo-1-butene (2.2 mL, 22 mmol) according to the procedure for Part A compound in Example 274 to give title compound (5.1 g, 84%) as a white solid
(mp 67-69°C).

### B. 9-[4-[(2-Bromo-5-pyridinyl)amino]-butyl]-N-propyl-9H-fluorene-9-carboxamide

A solution of Part A compound (500 mg, 1.64 mmol) in THF (2 mL) was added to a solution of 9-borabicyclo[3.3.1]nonane (3.3 mL, 0.5M in THF, 1.64 mmol) at 0°C under argon. The clear, colorless reaction was stirred at RT for 5 h, then diluted further with dioxane (10 mL). Anhydrous potassium phosphate anhydrous (316 mg, 1.49 mmol) was added, followed by tetrakis(triphenylphosphine)palladium (52 mg, 0.045 mmol). 2-Bromo-5-nitropyridine (302 mg, 1.49 mmol) was added and the reaction was stirred at 60'C overnight, then cooled to RT. Water (30 mL) was added and the reaction was stirred vigorously in the air for 2 h. The reaction mixture was extracted with EtOAc (100 mL, then 20 mL), and the combined organic layers were washed with brine (2 x 20 mL), then dried over MgSO₄. Evaporation gave 1.2 g of a brown oil, which was dissolved in a minimum amount of CH₂Cl₂ and purified by flash chromatography on silica gel (75 g) eluting with 40% EtOAc/hexane to provide 200 mg of impure product as a yellow foam. Additional chromatography eluting with 50% EtOAc/hexane gave title compound (147 mg, 19%) as a yellow solid.
mp 139-141°C.
MS (ES): 478/480 [M+H].

| | | | |
|---|---|---|---|
| Anal. Calcd. for C₂₆H₂₈BrN₃O • 0.3 H₂O | C, 64.54; | H, 5.96; | N, 8.68 |
| Found | C, 64.61; | H, 5.88; | N, 8.66. |

### Examples 277 to 286

The following additional compounds were prepared following procedures, set out hereinbefore.

### Example 277

### 9-[2-[[[4-(Benzoylamino)phenyl]sulfonyl]amino]ethyl]-N-(2,2,2-trifluoroethyl)-9H-fluorene-9-carboxamide

mp 235-236°C
MS (ES) 594 (M+H); 1187 (2M+H)

| | | | | | |
|---|---|---|---|---|---|
| Anal. Calc'd for C₃₁H₂₆F₃N₃O₄S | C, 62.72; | H, 4.41; | N, 7.08; | F, 9.60; | S, 5.40 |
| Found | C, 62.56; | H, 4.45; | N, 7.00; | F, 9.54; | S, 5.21. |

### Example 278

### 9-(4-Phenylbutyl)-N-propyl-9H-fluorene-9-carboxamide

mp 88-90°C
MS (CI) 384 (M+H)

| | | | |
|---|---|---|---|
| Anal. Calc'd for C₂₇H₂₉NO | C, 84.56; | H, 7.62; | N, 3.65 |
| Found | C, 84.62; | H, 7.66; | N, 3.72. |

### Example 279

### 3-[(9-Propyl-9H-fluoren-9-yl)sulfonyl]propanoic acid, methyl ester

mp 74-77°C
MS (FAB, + ions) m/z 376 (M+NH₄) m/z 359 (M+H)

| | | | |
|---|---|---|---|
| Anal. Calc'd for C₂₀H₂₂O₄S • 0.29 H₂O | C, 66.04; | H, 6.26; | N, 8.81 |
| Found | C, 66.04; | H, 6.11; | N, 8.45. |

### Example 280

### 9-[4-[(6-Ethoxy-2-benzothiazolyl)thio]butyl]-N-propyl-9H-fluorene-9-carboxamide

mp 109-111°C
MS (ES, + ions) m/z 517 (M+H)

| | | | | |
|---|---|---|---|---|
| Anal. Calc'd for C₃₀H₃₂N₂O₂S₂ + 0.40 mol H₂O | C, 68.78; | H, 6.31; | N, 5.35; | S, 12.24 |
| Found | C, 68.56; | H, 6.07; | N, 5.57; | S, 12.23. |

### Example 281

### 9-[3-[(6-Ethoxy-2-benzothiazolyl)thio]propyl]-N-propyl-9H-fluorene-9-carboxamide

mp 82-85°C
MS (ES, + ions) m/z 503 (M+H)

| | | | | |
|---|---|---|---|---|
| Anal. Calc'd for C₂₉H₃₀N₂O₂S₂ + 0.56 mol H₂O | C, 67.93; | H, 6.12; | N, 5.46; | S, 12.50 |
| Found | C, 68.03; | H, 5.83; | N, 5.36; | S, 12.51, |

### Example 282

### (Z)-9-[4-(Diethoxyphosphinyl)-2-butenyl]-N-(2,2,2-trifluoroethyl)-9H-fluorene-9-carboxamide

mp 88-91°C
MS (CI-NH₃, + ions) m/z 482 (M+H)

| | | | | | |
|---|---|---|---|---|---|
| Anal. Calc'd for C₂₄H₂₇NO₄PF₃ | C, 59.87; | H, 5.65; | N, 2.91; | P, 6.43; | F, 11.84 |
| Found | C, 59.52; | H, 5.61; | N, 2.89; | P, 6.92; | F, 11.94. |

### Example 283

### 9-[4-(Diethoxyphosphinyl)butyl]-N-(2,2,2-trifluoroethyl)-9H-fluorene-9-carboxamide

mp 87-89°C
MS (FAB) m/z 484 (M+H)

| | | | | | |
|---|---|---|---|---|---|
| Anal. Calc'd for C₂₄H₂₉NO₄PF₃ + 0.13 mol H₂O | C, 59.33; | H, 6.07; | N, 2.88; | P, 6.37; | F, 11.73 |
| Found | C, 59.09; | H, 5.98; | N, 2.95; | P, 6.51; | F, 11.92. |

### Example 284

### 9-[4-(Dibutoxyphosphinyl)butyl]-N-(2,2,3,3,3-pentafluoropropyl)-9H-fluorene-9-carboxamide

mp 56-57°C
MS (ES, + ions) m/z 590 (M+H)

| | | | | | |
|---|---|---|---|---|---|
| Anal. Calc'd for C₂₉H₃₇NO₄F₅P | C, 59.08; | H, 6.33; | N, 2.38; | P, 5.25; | F, 16.11 |
| Found | C, 58.80; | H, 6.34; | N, 2.26; | P, 5.05; | F, 15.90. |

### Example 285

### 9-[4-(Dibutoxyphosphinyl)butyl]-N-propyl-9H-xanthene-9-carboxamide

mp 64-67°C
MS (ES, + ions) m/z 516 (M+H)

| | | | | |
|---|---|---|---|---|
| Anal. Calc'd for C₂₉H₄₂O₅NP | C, 67.55; | H, 8.21; | N, 2.72; | P, 6.01 |
| Found | C, 67.25; | H, 8.17; | N, 2.68; | P, 5.99. |

### Example 286

### 9-[4-(Dibutoxyphosphinyl)butyl]-N-(2,2,3,3,4,4,4-heptafluorobutyl)-9H-fluorene-9-carboxamide

MS (ES, + ions) m/z 657 (M+NH₄), 640 (M+H)

| | | | | | |
|---|---|---|---|---|---|
| Anal. Calc'd for C₃₀H₃₇NF₇PO₄ | C, 56.34; | H, 5.83; | N, 2.19; | F, 20.79; | P, 4.84 |
| Found | C, 56.03; | H, 5.91; | N, 2.15; | F, 20.74; | P, 4.77. |

The following compounds of the invention may be prepared following the procedures described hereinbefore and in the working Examples.

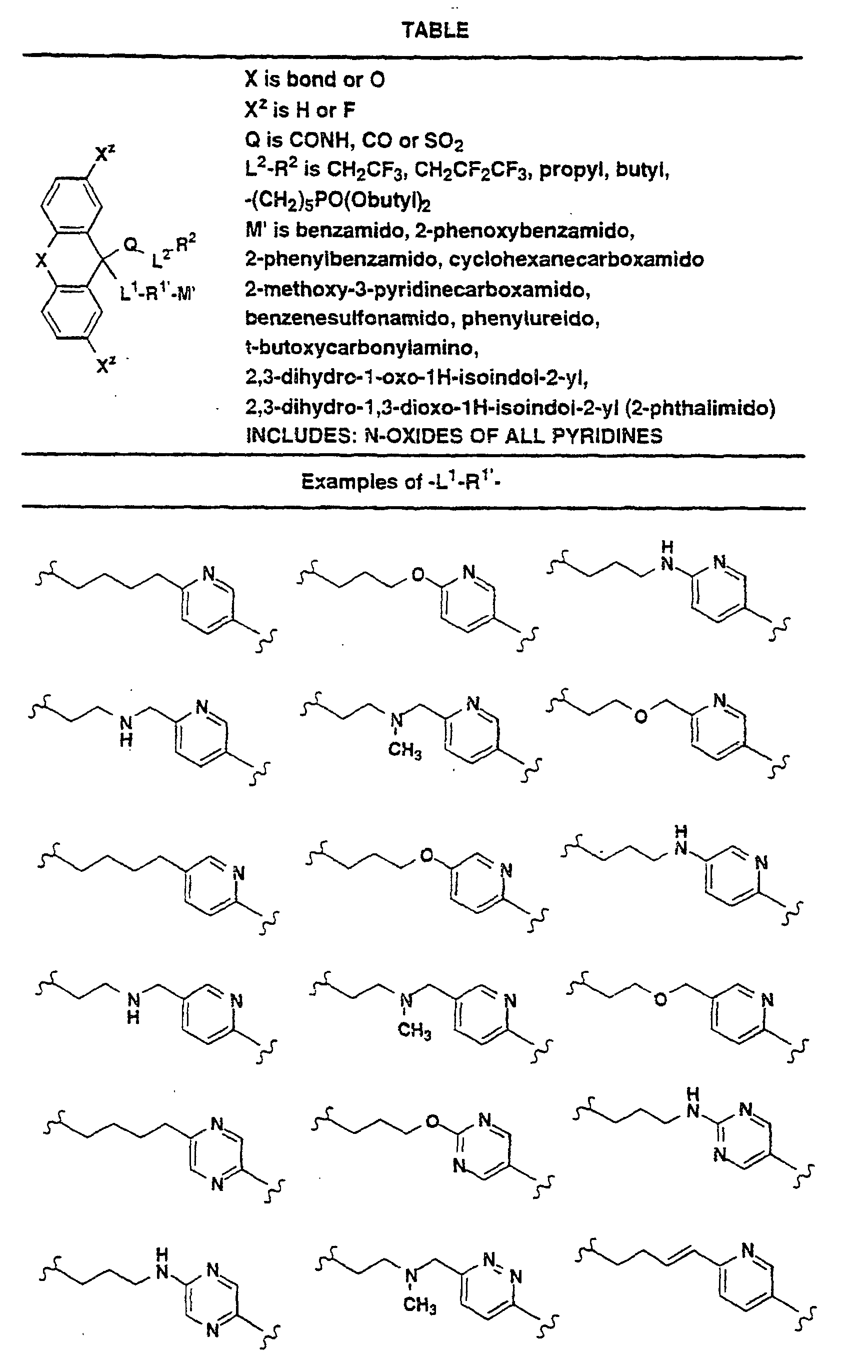

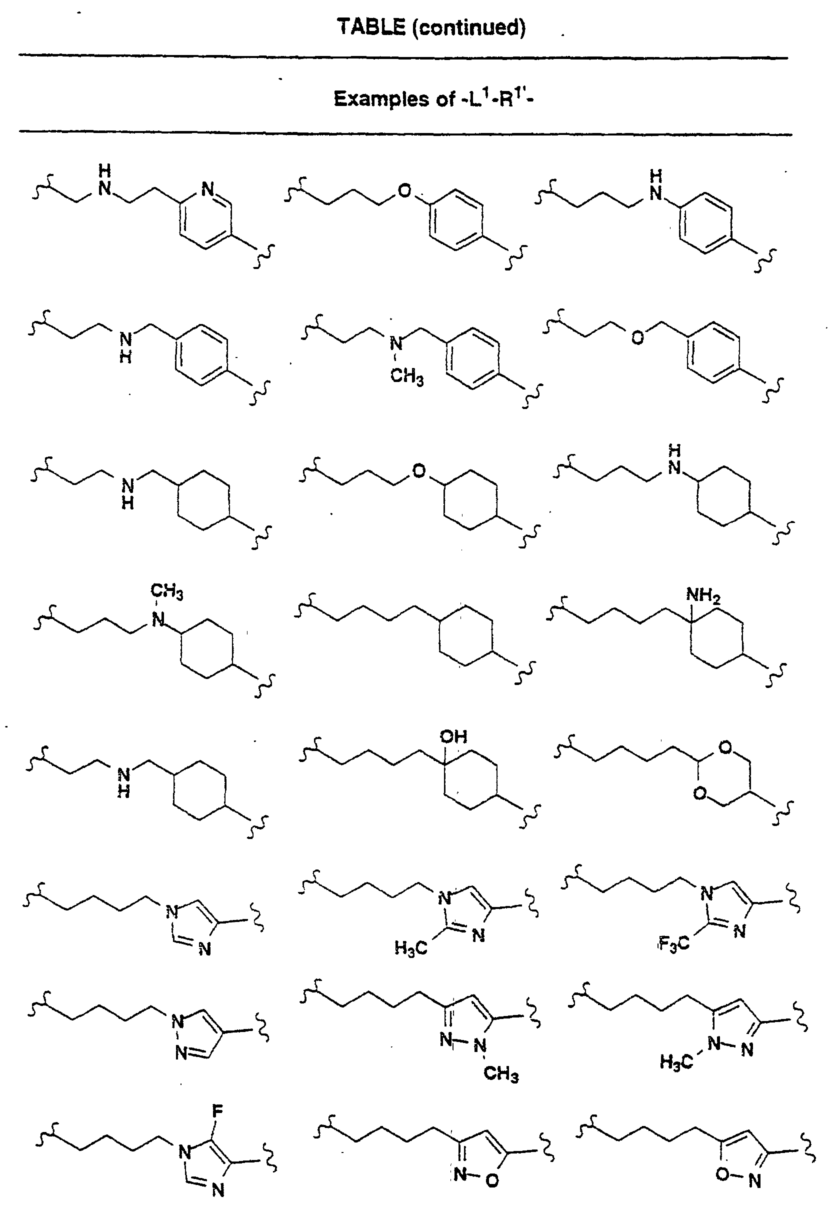

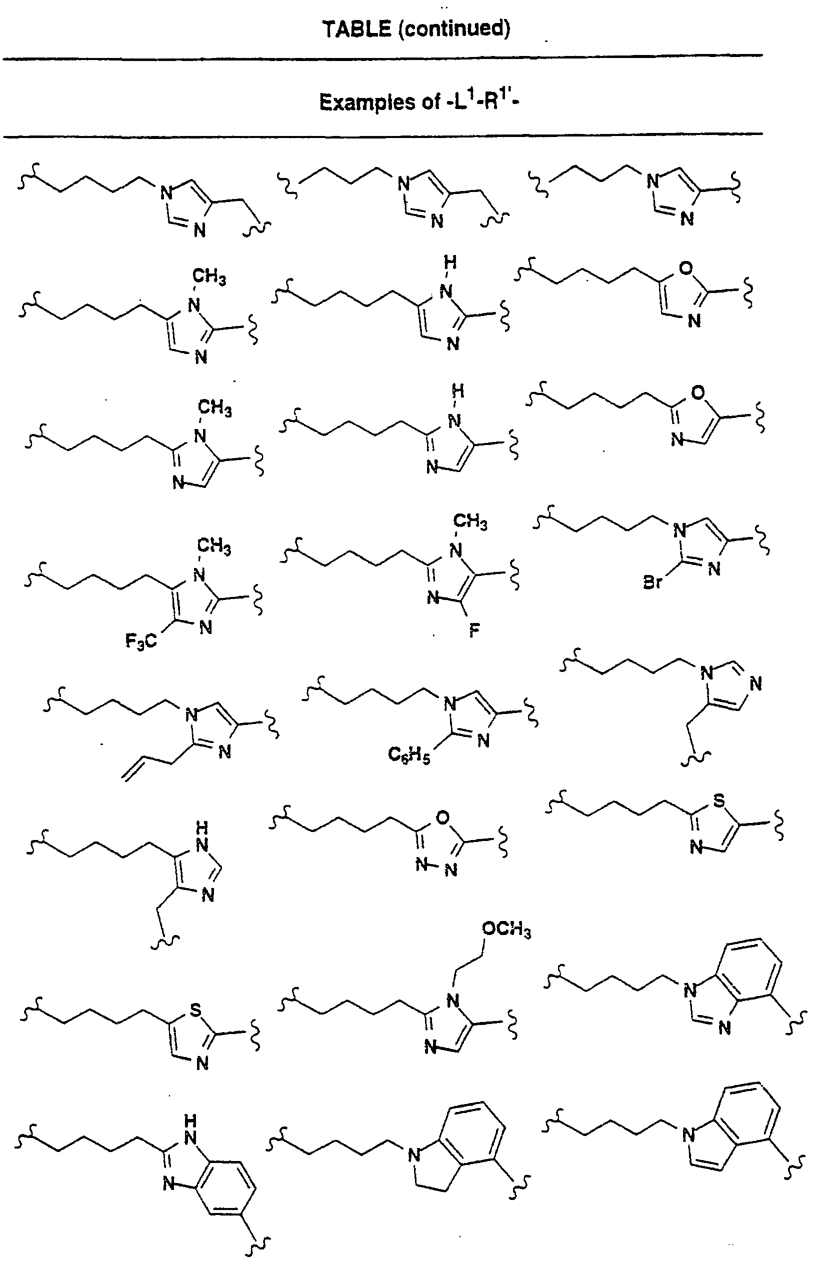

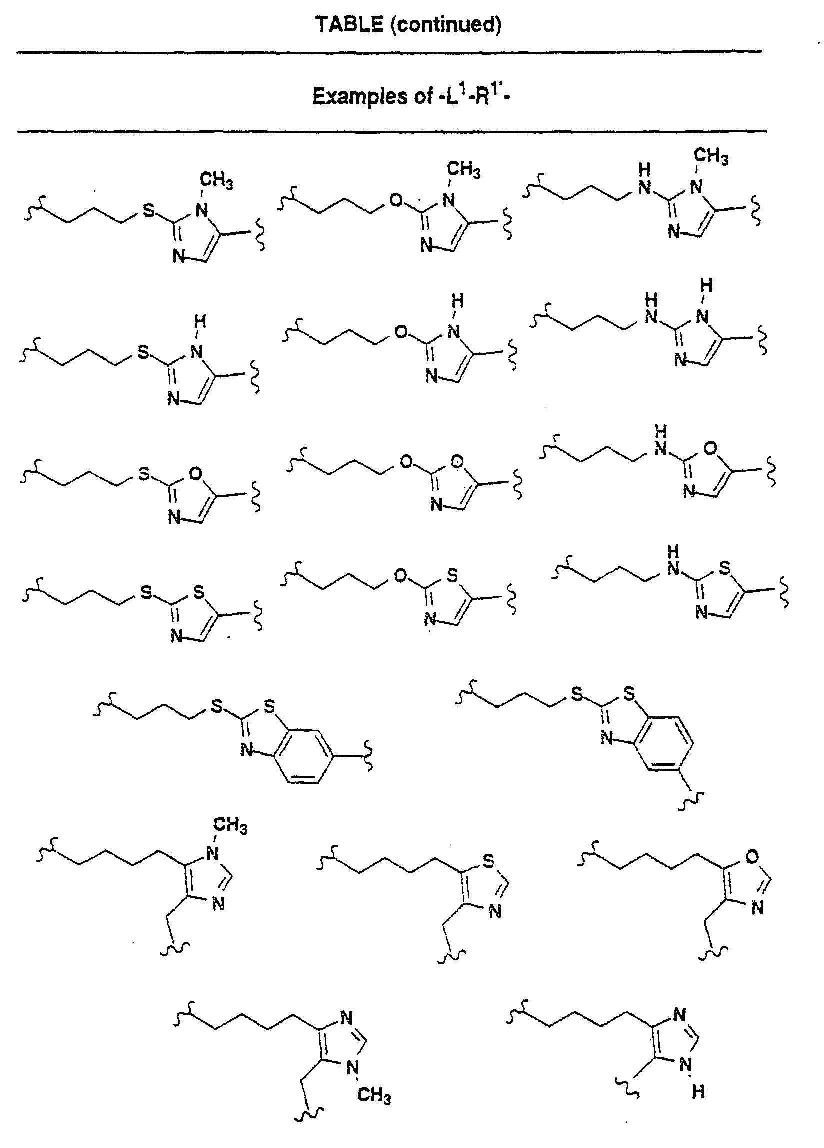

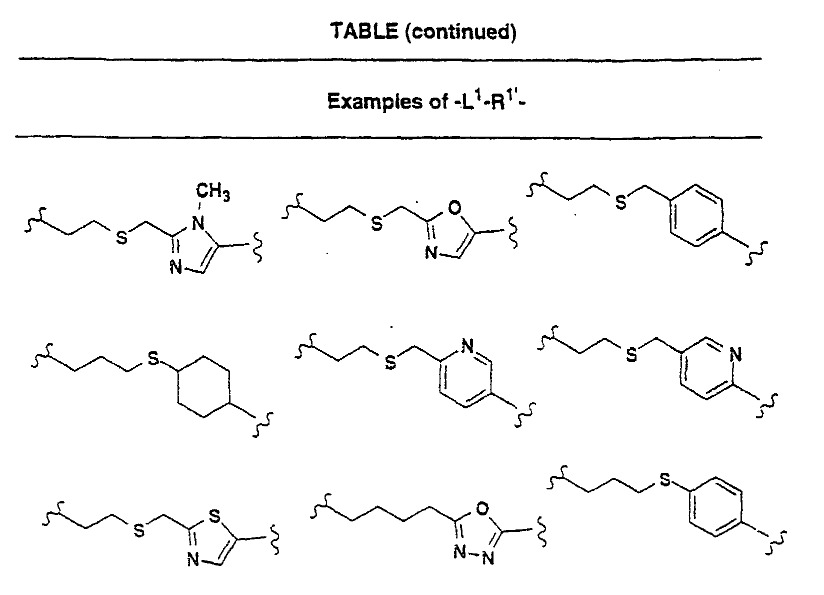

### Example 288

### 9-[4-[4-[(Phenylsulfonyl)amino]phenyl]butyl]-N-(2,2,2-trifluoroethyl)-9H-fluorene-9-carboxamide

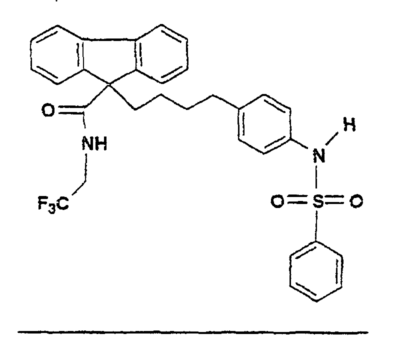

### A.

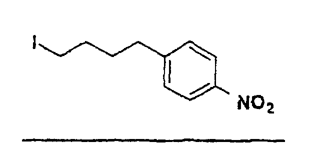

A solution of iodine (1.40 g, 5.5 mmol) in THF (5 mL) was added dropwise over 5 min to a solution of 4-(4-nitrophenyl)-1-butanol (975 mg, 5 mmol), triphenylphosphine (1.44 g, 5.5 mmol), and imidazole (749 mg, 11 mmol) in THF (10 mL) under argon at room temperature. The dark orange solution was stirred at room temperature for 15 min, diluted with hexane (50 mL), then washed with 10% sodium bisulfite, saturated NaHCO₃, and brine (20 mL each). The organic layer was dried over MgSO₄ and filtered. To the filtrate was added silica gel (4 g) and the mixture was concentrated in vacuo to give a yellow powder, which was purified by flash chromatography on silica gel (120 g) eluting with 25% CH₂Cl₂/hexane to give title iodide (1.33 g, 87%) as a pale yellow crystalline solid (mp 44-45°C).

### B.

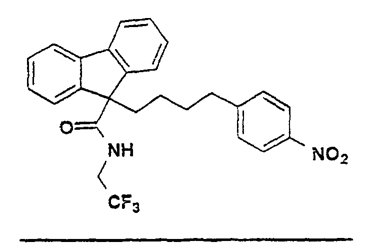

Butyllithium (2.0 mL, 2.5M in hexane, 5.0 mmol) was added to a solution of 9-fluorenecarboxylic acid (480 mg, 2.3 mmol) in The (10 mL) at 0°C under argon over 5 min. The reaction went from a clear solution to a white suspension then to a yellow solution during addition. The reaction was stirred at 0°C for 20 min, whereupon a solution of Part A iodide (671 mg, 2.2 mmol) in THF (4 mL) was added dropwise over 5 min. The reaction was stirred at 0 °C for 1.5 h, warmed to room temperature, then stirred at room temperature for 3.5 h. The reaction was quenched with 1N HCl to pH ≈ 3, diluted with water (10 mL), then extracted with EtOAc (2 x 20 mL). The combined organic layers were washed with water and brine (10 mL each), then dried over MgSO₄. Evaporation gave a residue, which was azeotroped'with toluene (10 mL) to give crude acid in the form of a dark foam

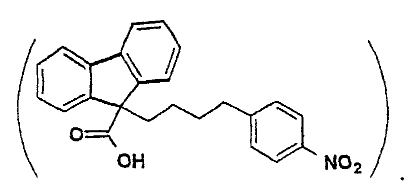

To a solution of the crude acid prepared above containing 3 drops of DMF in CH₂Cl₂ (6 mL) at room temperature under argon was added oxalyl chloride (3 mL, 2.0M in CH₂Cl₂, 6.0 mmol). The reaction was allowed to stir at room temperature for 1.5 h. The reaction was concentrated in vacuo to provide a dark oil, which was diluted with THF (5 mL) and cooled to 0°C under argon. Trifluoroethylamine (0.63 g, 8 mmol) was added dropwise over 2 min, and the reaction was stirred at 0°C for 3 h. The reaction was partitioned between EtOAc (30 mL) and water (10 mL). The organic layer was washed with 1N HCl (7 mL) and brine (5 mL), then dried over MgSO₄. Evaporation gave 974 mg of a brown oil, which was dissolved in CH₂Cl₂ and purified by flash chromatography on silica gel (75 g) eluting with 15:85 EtOAc/hexane to afford title compound (0.75 g, 69%) as a thick oil.

### C. 9-[4-[4-[(Phenylsulfonyl)amino]phenyl]-butyl]-N-(2,2,2-trifluoroethyl)-9H-fluorene-9-carboxamide

A mixture of Part B compound (220 mg, 0.47 mmol) and 10% palladium on carbon (20 mg) in EtOAc (15 mL) was hydrogenated (balloon pressure) at room temperature for 18 h, filtered through Celite with the aid of EtOAc, then concentrated in vacuo to give a residue, which was pumped under high vacuum to provide a thick oil.

Phenylsulfonyl chloride (80 mg, 0.46 mmol) was added to a solution of the crude amine (≈ 0.45 mmol) and pyridine (35 mg, 0.46 mmol) in CH₂Cl₂ (4 mL) at room temperature under argon. The reaction was stirred for 2 h, diluted with ethyl acetate (50 mL), washed with 1N HCl (10 mL) and water (10 mL), then dried over MgSO₄. Evaporation gave an oil, which was adsorbed onto silica gel (10 g), then purified by flash chromatography on silica gel (50 g) eluting with 30% EtOAc/hexane to give 0.23 g (88%) of title compound as a pink solid.
mp : 130-132°C.

| | | | | | |
|---|---|---|---|---|---|
| Anal Calc'd for C₃₂H₂₉N₂SO₃F₃ + 0.2 CH₂Cl₂ | C, 64.93; | H, 4.98; | N, 4.70; | S, 5.38; | F, 9.57 |
| Found | C, 65.16; | H, 5.08; | N, 4.55; | S, 5.52; | F, 9.17. |

### Example 290

### 9-[5-(Dibutoxyphosphinyl)pentyl]-N-(2,2,2-trifluoroethyl)-9H-fluorene-9-carboxamide

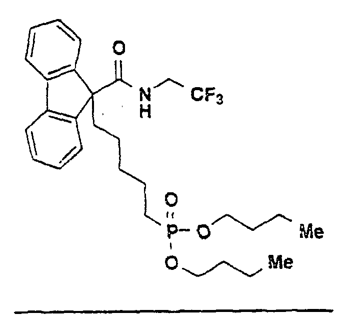

### A.

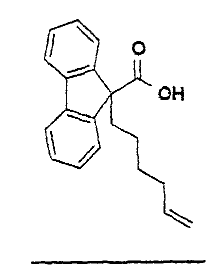

To a solution of 3.0 g (14.30 mmol) of 9-fluorenecarboxylic acid in 50 mL of THF, under argon at 0°C, was added dropwise 11.4 mL (28.60 mmol) of n-BuLi (2.5 M in hexanes). The anion was stirred for 0.5 h at which time 2.3 mL (17.16 mmol) of 6-bromo-1-hexene was added dropwise. The reaction gradually warmed to room temperature and was stirred for 18 h, at which time it was diluted with a 1:1 mixture of ethyl acetate/water (200 mL). The organics were washed with NaHCO₃, water, brine, dried (Na₂SO₄) and evaporated. Flash chromatography was performed on 200 g of silica gel eluting with 95:5 dichloro-methane/isopropanol to provide 900 mg (22%) of title compound as a pale yellow solid.
MS (CI-NH₃, + ions) m/z 310 (M + NH₄), 293 (M + H).

### B.

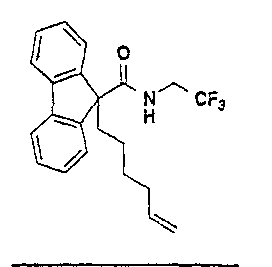

To a solution of 800 mg (2.74 mmol) of Part A compound in 10 mL of CH₂Cl₂, under argon at room temperature, was added dropwise two drops of DMF and 2.0 mL (4.11 mmol) of oxalyl chloride (2.0 M in CH₂Cl₂). The reaction was stirred for 45 min. when it was evaporated to dryness.

In another flask, 446 mg (3.29 mmol) of 2,2,2-trifluoroethylamine in 10 mL of CH₂Cl₂, under argon at 0°C, was added 1.1 mL (8.22 mmol) of triethylamine. This slurry was stirred for 15 min at which time the above acid chloride, in 5 mL of CH₂Cl₂, was added dropwse. The reaction gradually warmed to room temperature and was stirred for 18 h, at which time it was diluted with ether and washed with water, 1N HCl, NaHCO₃, water, brine, dried (Na₂SO₄) and evaporated. Flash chromatography was performed on 100 g of silica gel eluting with 6:4 hexanes/ethyl acetate to provide 740 mg (74%) of title compound as a pale yellow solid.
MS (ES NH₃, - ions) m/z 372 (M - H).

### C.

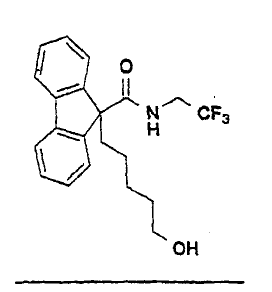

250 mg (0.67 mmol) of Part B compound in 2 mL of methanol, at -78°C, was treated with a stream of O₂/O₃ for 0.5 h, at which time the reaction was purged with N₂ and treated with 76 mg (2.0 mmol) of sodium borohydride pellets. The reaction gradually warmed to room temperature and was stirred for 18 h, at which time it was diluted with ether and washed with NH₄Cl, water, brine, dried (Na₂SO₄) and evaporated. Flash chromatography was performed on 100 g of silica gel eluting with 3:2 hexanes/ethyl acetate to provide 200 mg (79%) of title compound as a white solid.
MS (ES NH₃, - ions) m/z 376 (M - H).

### D.

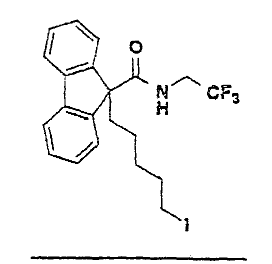

To a solution of 200 mg (0.53 mmol) of Part C compound in 3 mL of THF, under argon at 0°C, was added 76 mg (1.12 mmol) of imidazole followed by 180 mg (0.69 mmol) of triphenylphosphine. This mixture was stirred for 0.5 h at which time 175 mg (0.69 mmol) of iodine in 3 mL of THF was added dropwise. The reaction was stirred at 0°C for 1 h, at room temperature for 1 h, then diluted with hexanes and washed with fresh sodium bisulfite solution. The organics were washed with NaHCO₃, water, brine, dried (Na₂SO₄) and evaporated. Flash chromatography was performed on 50 g of silica gel eluting with 9:1 hexanes/ethyl acetate to provide 200 mg (78%) of title compound as a white solid.

### E. 9-[5-(Dibutoxyphosphinyl)pentyl]-N-(2,2,2-trifluoroethyl)-9H-fluorene-9-carboxamide

To 200 mg (0.41 mmol) of Part D compound was added 555 µL (2.05 mmol) of tributylphosphite (neat). The mixture was heated to 120°C for 18 h and bulb to bulb distilled (5 mm, 100°C) to remove lower boiling impurities and provide 234 mg (98%) of title compound as a white solid.
mp 88-91°C.
MS (ES NH₃, + ions) m/z 571 (M+NH₄), 554 (M+H).

| | | | | |
|---|---|---|---|---|
| Anal. Calcd. for C₂₉H₃₉NO₄PF₃ + 0.3 H₂O | C, 62.31; | H, 7.14; | N, 2.51; | P, 5.54 |
| Found | C, 62.35; | H, 7.21; | N, 2.38; | P, 5.76. |

### Example 291

### 9-[3-[[5-[(2-Phenoxybenzoyl)amino]-2-pyridinyl]-oxy]-propyl]-N-(2,2,2-trifluoroethyl)-9H-fluorene-9-carboxamide, monohydrochloride

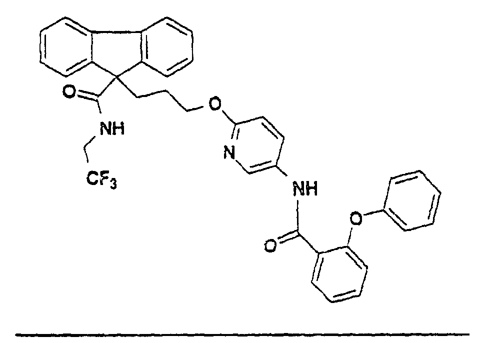

### A.

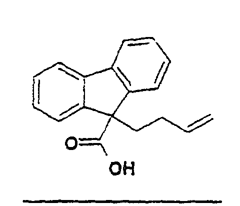

To a stirred solution of 12.6 g (60 mmol) of 9-fluorenecarboxylic acid in 600 mL of dry THF at 0° under argon was added, over 20 min, 53 mL of 2.5 M n-butyllithium in hexane (132.5 mmol). The mixture was stirred for 30 min and then 7.3 mL (72 mmol) of 4-bromo-1-butene were added. The reaction was sirred at 0°C for 10 min and then at room temperature for 2 days. Additional 4-bromo-1-butene (3.0 mL, 30 mmol) was added and stirring was continued for 2 days longer. Water (100 mL) was added and the mixture was concentrated to remove THF. Additional water was added and the mixture was extracted with ether (2 x 200 mL). The aqueous layer was layered with CH₂Cl₂ and acidified with 1N HCl (pH <2). After three extractions with CH₂Cl₂, the combined CH₂Cl₂ fraction was washed with water (2x), dried (MgSO₄), and concentrated to give 14.5 g (92%) of title compound as an amorphous pale yellow solid.

### B.

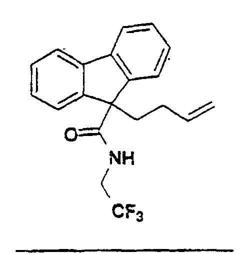

Part A compound (9.1 g, 34.5 mmol) was dried by concentration *in vacuo* from dry THF and dry toluene (2x) and then *in vacuo* overnight. To a solution of this acid in 100 mL of dry CH₂Cl₂ and 133 µL of DMF under nitrogen was slowly added 26 mL of 2.0 M oxalyl chloride in CH₂Cl₂ (52 mmol). The reaction was stirred at room temperature for 1.5 h and then concentrated *in vacuo* and dried for 1 h at 0.5 mm to aive the crude acid chloride of Part A compound. Triethylamine (14.5 mL, 104 mmol) was added to a stirred suspension of 2,2,2-trifluoro-ethyiamine hydrochloride in 70 mL of dry CH₂Cl₂ at 0°C under argon and the slurry was stirred at 0°C for 10 min. A solution of the crude acid chloride of Part A compound in 35 mL of CH₂Cl₂ was added over 15 min keeping the internal temperature. < 12°C. The reaction was stirred at 0°C for 1 h and then it was diluted with 175 mL of CH₂Cl₂. The CH₂Cl₂ was washed with 1N HCl (2x70 mL), water (175 mL), 5% NaHCO₃ (110 mL) and water (2x175 mL), dried (Na₂SO₄), and concentrated to give crude title compound as a solid (11.4 g). This solid was combined with an additional 6.54 g of crude title compound, and the combined crude title compound was chromatographed over 700 g of silica gel using CH₂Cl₂ to provide 15.5 g (82%) of title compound as a solid having mp 105-107°C.

### C.

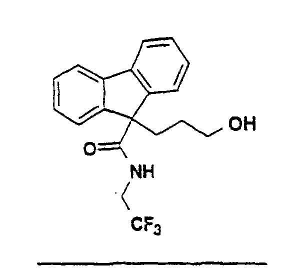

A solution of Part B compound (0.50 g, 1.44 mmol) in 20 mL of 1:1 dichloromethane/methanol at - 78°C was treated with a stream of O₂/O₃ until the solution turned light blue. The mixture was treated with NaBH₄ (1 pellet, 0.2 g, 5.26 mmol) and stirred for 18 h. The resulting colorless solution was diluted with 1:1 NH₄Cl solution/ethyl acetate (150 mL) and the layers separated. The organic fraction was dried (MgSO₄), filtered, and concentrated to give 0.44 g (89%) of title compound as a white solid.
mp 111-114°C.

### D.

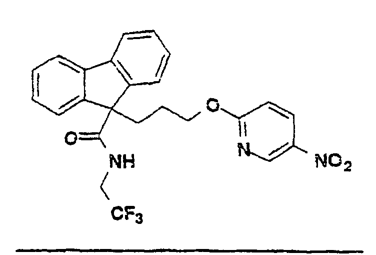

A solution of Part C compound (0.50 g, 1.43 mmol) in THF (7 mL) was treated with NaH (38 mg, 1.57 mmol) and stirred for 0.5 h. After all of the gray solid was consumed, 2-bromo-5-nitropyridine (0.32 g, 1.57 mmol) was added to the reaction mixture. The resulting dark orange solution was stirred at room temperature for 18 h, diluted with 1:1 water/ethyl acetate (150 mL) and the layers separated. The organic fraction was dried (MgSO₄), filtered, and concentrated. The remainder was purified by flash chromatography on silica gel (50 g) eluting with 1:4 ethyl acetate/hexane to give title compound (0.81 g, 99%) as a pale yellow yellow oil.

### E. 9-[3-[[5-[(2-Phenoxybenzoyl)amino]-2-pyridinyl]oxy]propyl]-N-(2,2,2-trifluoroethyl)-9H-fluorene-9-carboxamide, monohydrochloride

A mixture of Part D compound (0.78 g, 1.65 mmol) and 10% palladium on carbon (80 mg) in EtOAc (20 mL) was hydrogenated (balloon pressure) at room temperature for 18 h. 2-Phenoxybenzoyl chloride (0.46 g, 2.00 mmol) was added to the solution of the crude amine (= 1.65 mmol) and pyridine (0.14 g, 1.78 mmol). The reaction was stirred for 2 h, diluted with ethyl acetate (50 mL), washed with NaHCO₃ solution (20 mL), and dried over MgSO₄. Evaporation gave an oil, which was purified by flash chromato-graphy on silica gel (75 g) eluting with 40% EtOAc/hexane to give 0.78 g (75%) of a white foam. The foam was diluted with ether and treated with 4N HCl in dioxane. A white solid formed which was collected by filtration. The solid was dried under vacuum (20 mm Hg) at room temperature for 18 h to give (0.70 g, 63%) of title compound (HCl salt) as a white solid.
mp 110-115°C.
MS (FAB, + ions) m/z 638(M + H).

| | | | | |
|---|---|---|---|---|
| Anal Calc'd for C₃₈H₃₀N₃O₄ + 1.0 H₂O + 1.0 HCl | C, 64.21; | H, 4.81; | N, 6.07; | F, 8.23 |
| Found | C, 64.46; | H, 4.88; | N, 5.86; | F, 8.13. |

### Example 292

### [6-[9-[[(2,2,2-Trifluoroethyl)amino)carbonyl]-9H-fluoren-9-yl]hexyl]phosphonic acid, dibutyl ester

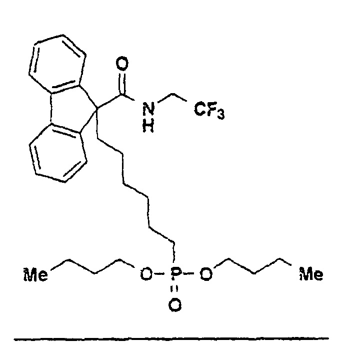

### A.

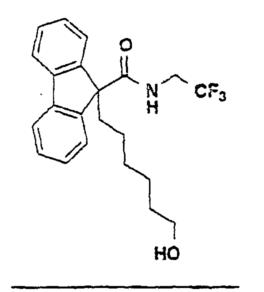

To 400 mg (1.07 mmol) of Example 290 Part B compound was added 3.7 mL (1.87 mmol) of 9-BBN (9-borabicyclo[3.3.1]nonane, 0.5 M in THF). The reaction was stirred for 18 h, at which time it was cooled to 0°C and treated dropwise with 1.25 mL (3.74 mmol) of 3N NaOH and 432 µL (3.74 mmol) of 30% H₂O₂ simultaneously. The biphasic mixture was stirred vigorously for 18 h, at which time it was extracted with ethyl acetate and the organic layer was washed with H₂O, brine, dried (Na₂SO₄) and evaporated. Flash chromatography was performed on 100 g of silica gel eluting with 1:1 hexanes/ethyl acetate to provide 320 mg (77%) of title compound as a white solid.
MS (ES NH₃, + ions) m/z 409 (M + NH₄).

### B.

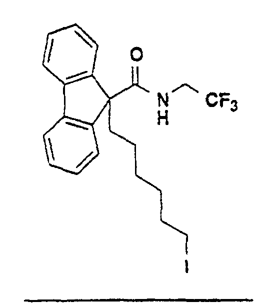

To a solution of 310 mg (0.793 mmol) of Part A compound in 5 mL of THF, under argon at 0°C, was added 118 mg (1.74 mmol) of imidazole followed by 270 mg (1.03 mmol) of triphenylphosphine. The mixture was stirred for 0.5 h at which time 262 mg (1.03 mmol) of iodine in 3 mL of THF was added dropwise. The reaction was stirred at 0°C for 1 h, room temperature for 1 h then diluted with hexanes. The organics were washed with fresh sodium bisulfite solution, NaHCO₃, water, brine, dried (Na₂SO₄) and evaporated. Flash chromatography was performed on 25 g of silica gel eluting with 9:1 hexanes/ethyl acetate to provide 310 mg (78%) of title compound as a white solid.

### C. [6-[9-[[(2,2,2-Trifluoroethyl)amino]-carbonyl]-9H-fluoren-9-yl]hexyl]phosphonic acid, dibutyl ester

To 150 mg (0.30 mmol) of Part B compound was added 405 µL (1.50 mmol) of tributylphosphite (neat). The mixture was heated to 120°C for 18 h and bulb to bulb distilled (5 mm, 100°C) to remove lower boiling impurities and provide 165 mg (98%) of title compound as a pale yellow oil.
MS (ES NH₃, + ions) m/z 568 (M + H).

| | | | | | |
|---|---|---|---|---|---|
| Anal. Calcd. for C₃₀H₄₁NO₄PF₃ + 0.24 CH₂Cl₂ | C, 61.77; | H, 7.11; | N, 2.38; | P, 5.27; | F, 9.69 |
| Found | C, 61.80; | H, 7.20; | N, 2.36; | P, 5.15; | F, 9.60. |

### Example 293

### 9-[4-[5-[(2-Phenoxybenzoyl)amino]-2-pyridinyl]-butyl]-N-(2,2,2-trifluoroethyl)-9H-fluorene-9-carboxamide, monohydrochloride

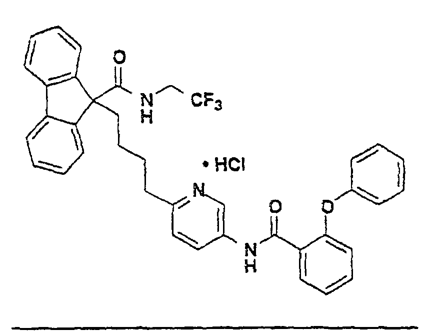

Following the procedure in Example 274 Part C, Example 274 Part B compound (1.02 g, 2.19 mmol) was reacted with Example 275 Part A compound (prepared from 563 mg (2.63 mmol) of 2-phenoxybenzoic acid) to provide 712 mg of product as the free amine.

A portion of the desired product (317 mg) was dissolved in MeOH (2 mL) and a solution of 1.1N HCl/Et₂O (0.9 mL, 1.0 mmol) was added. The solution was concentrated in vacuo and the residue was triturated with Et₂O to give a foamy solid, which was pumped under high vacuum overnight to afford title compound (302 mg, 47%) as a foamy beige solid.
MS (ES, + ions) m/z 636 (M+H)

| | | | | | |
|---|---|---|---|---|---|
| Anal. Calcd for C₃₈H₃₃Cl₃N₃O₃ + 0.5H₂O | C, 67.01; | H, 5.03; | N, 6.17; | Cl, 5.20; | F, 8.37 |
| Found | C, 67.04; | H, 5.02; | N, 6.03; | Cl, 5.55; | F, 8.20. |

### Example 294

### 9-[4-[4-(Benzoylamino)-2-methyl-1H-imidazol-1-yl]-butyl]-N-(2,2,2-trifluoroethyl)-9H-fluorene-9-carboxamide

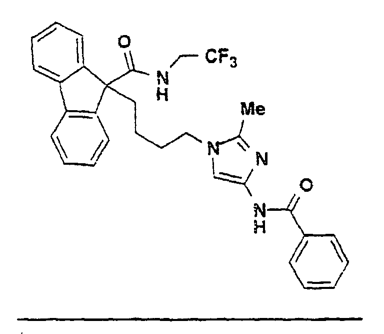

### A.

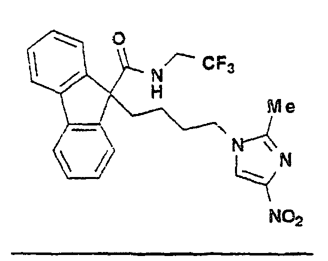

To a solid mixture of Example 273 Part A(2) compound (1.00 g, 2.35 mmoL), 2-methyl-5-nitroimidazole (400 mg, 3.15 mmol), and K₂CO₃ (2.82 mmol) was added DMF (5 mL) and the mixture was stirred at room temperature for 3 days. The reaction was partitioned between EtOAc and saturated NaHCO₃ and the organic layer was washed successively with H₂O and brine. The solution was dried (Na₂SO₄), filtered, and stripped. The residue was triturated with Et₂O/EtOAc/hexane to give title compound (973 mg, 88%) as a white solid. mp 145-147°C.

### B. 9-[4-[4-(Benzoylamino)-2-methyl-1H-imidazol-1-yl]-butyl]-N-(2,2,2-trifluoroethyl)-9H-fluorene-9-carboxamide

A solution of compound Part A (171 mg, 0.36 mmol) in dry 1,4-dioxane (3.9 mL) was hydrogenated (balloon) over 10% Pd/C (35 mg) at room temperature for 5 hours. Additional 10% Pd/C (40 mg) was added and stirring over H₂ was continued for an additional 16 hours. The reaction flask was evacuated and the atmosphere was replaced with air. To this slurry was added triethylamine (TEA) (200 µL, 145 mg, 1.4 mmol) followed by benzoyl chloride (100 µL). After one hour at room temperature, the mixture was filtered through Celite, diluted with EtOAc and subsequently washed with saturated NaHCO₃, H₂O, and brine, then dried (Na₂SO₄), filtered, and stripped to give a brown oil. The residue was partially purified by flash chromatography on silica gel (2/98-MeOH/CH₂Cl₂ as eluant). Further flash chromatographic separation (EtOAc as eluant) afforded title compound which was isolated as a light yellow solid foam by trituration and stripping from EtOAc/hexanes (88 mg, 45%).

| | | | | |
|---|---|---|---|---|
| Anal. Calc'd for C₃₁H₂₉F₃N₄O₂·0.2H₂O+0.2C₆H₁₄ | C, 68.16; | H, 5.72; | N, 9.87; | F, 10.04 |
| Found | C, 68.02; | H, 5.76; | N, 9.61; | F, 9.65. |

### Example 295

### 9-[4-[4-[(2-Phenoxybenzoyl)amino]-2-methyl-1H-imidazol-1-yl]butyl]-N-(2,2,2-trifluoroethyl)-9H-fluorene-9-carboxamide, monohydrochloride

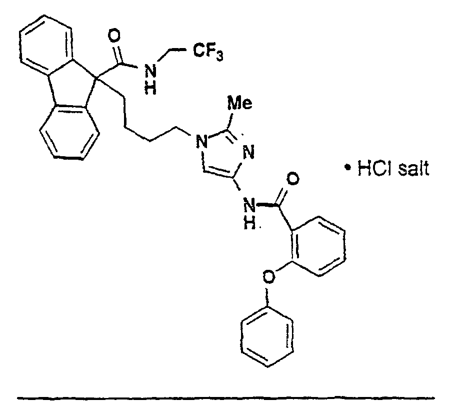

### A. and B.

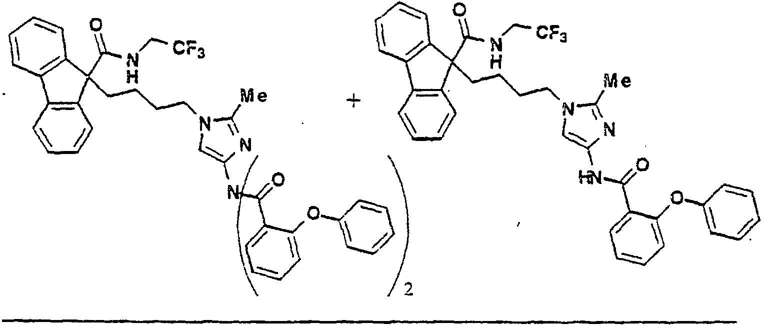

A solution of Example 294 Part A compound (350 mg, 0.65 mmol) in dry 1,4-dioxane (7 mL) was hydrogenated (balloon) over 10% Pd/C (126 mg) at room temperature for 28 hours. The reaction flask was evacuated and the atmosphere was replaced with air. To this slurry was added triethylamine (TEA) (300 µL, 218 mg, 2.15 mmol) followed by 2-phenoxybenzoic acid chloride (320 mg, 1.37 mmol) in dry THF (2 mL). After 1.5 hours at room temperature, the mixture was filtered through Celite, diluted with EtOAc and subsequently washed with saturated NaHCO₃, H₂O, and brine, then dried (Na₂SO₄), filtered, and stripped to give a brown oil. The residue was purified by flash chromatography on Merck SiO₂ (1:1-acetone:hexanes as eluant) to give a R_{f} 0.36 (1:1-acetone:hexanes) as a light brown foam (≈400 mg).

The mixture was separated by preparative HPLC (YMC-Pack ODS-A, 250 x 30 mm column, eluted with B:A solvent mixture, 50 to 100% B over a 20 minute linear gradient followed by 100% B (solvent A: 90% H₂O-10% MeOH-0.1% trifluoroacetic acid (TFA); solvent B: 10% H₂O-90% MeOH-0.1% TFA); flow rate 25 mL/min detecting at 254 nm). The desired fractions were stripped and the residues were partitioned between EtOAc and saturated NaHCO₃. The organic extracts were washed with brine, dried (Na₂SO₄), flitered and stripped to afford Part A compound (182 mg) and Part B compound (87 mg) as foams.

### C. 9-[4-[4-[(2-Phenoxybenzoyl)amino]-2-methyl-1H-imidazol-1-yl]butyl]-N-(2,2,2-trifluoroethyl)-9H-fluorene-9-carboxamide, monohydrochloride

Part A compound (≈180 mg) was dissolved in MeOH (6 mL) and treated with K₂CO₃ (62 mg). HPLC analysis after 5 hours indicated that all of Part A compound was converted to Part B compound and 2-phenoxybenzoic acid methyl ester. The mixture was partitioned between EtOAc and H₂O. The organic layer was washed with H₂O and brine, then dried (Na₂SO₄), filtered and stripped. The residue was combined with Part B compound from above and flash chromatographed (SiO₂, 7/3-EtOAc/hexanes as eluant) to afford pure Part B compound as a pale yellow foam (210 mg, 51% from Example 294 Part A compound).

The foam was dissolved in THF (400 µL), diluted with Et₂O (5 mL) and treated with 140 µL of 4 N HCl in 1,4-dioxane. The resulting precipitate was collected by filtration and dried in vacuo to afford title compound as a white solid (212 mg, 48% from Example 294 Part A compound).
mp 200-202°C.
MS (ESI, + ions) m/z 639 (M+H)⁺; (ESI, - ions) m/z 637 (M-H)⁻.

### Example 296

### 9-[3-[[2-(Benzoylamino)-5-pyridinyl]amino]propyl]-N-(2,2,2-trifluoroethyl)-9H-fluorene-9-carboxamide, monohydrochloride

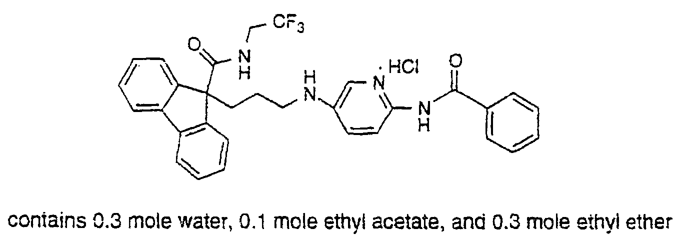

### A.

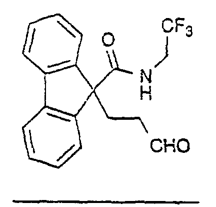

Ozone (Welsbach generator) was bubbled through a stirred solution of 2.07 g (6 mmol) of Example 291 Part B compound in 25 mL of dry MeOH at -65°C for 45 min. Nitrogen was bubbled through the solution for 10 min, 5 mL of dimethyl sulfide was added, and the reaction was warmed to room temperature. The solvent was removed and the residue was taken up in EtOAc. The EtOAc was washed with water (3x), dried (Na₂SO₄) and concentrated to an oil (2.21 g). Chromatography of the oil over 150 g of silica gel packed in 1% EtOAc in CH₂Cl₂, by elution with 2% EtOAc in CH₂Cl₂, afforded 1.11 g (53%) of title compound as an oily residue.

### B.

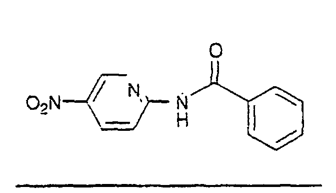

Benzoyl chloride (8.2 mL, 70 mmol) was added to a stirred suspension of 7.5 g (54 mmol) of 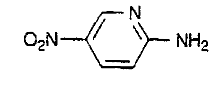 and 13 mL (160 mmol) of dry pyridine in 50 mL of dry THF and the mixture was stirred for 20 h at room temperature. The reaction was filtered and the filtrate was concentrated to a gummy residue, which was slurried with CH₂Cl₂, water, and 10% aq. NaHCO₃ to give crystals. The crystals were collected by filtration, washed with CH₂Cl₂, and dried to give 7.44 g pale yellow crystals, which were recrystal-lized from hot 95% EtOH to give 7.18 g of pale yellow crystalline title compound (55%) having mp 169-170°C.

### C.

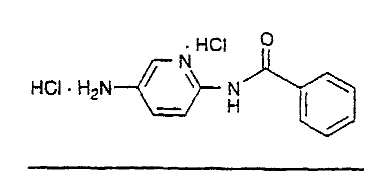

Part B compound (2.92 g, 12 mmol) was hydrogenated with 360 mg of 10% Pd/C in 50 mL of AcOH at 1 atmosphere for 1.5 h. Concentrated HCl (2.1 mL, 24.5 mmol) was added and the solids were collected by filtration. Trituration of the wet moist solid with EtOH and then filtration through a 45 µ nylon filter gave a filtrate, which was concentrated to a 25 mL yellow slurry. Et₂O (150 mL) was added and the solids were collected, washed with Et₂O, and dried for 2 h to give 2.77 g (81%) of title compound as a solid.

### D.

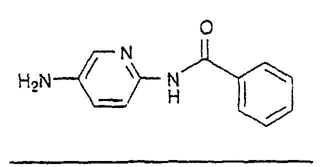

Part C compound (286 mg, 1 mmol) was dissolved in water and layered with CH₂Cl₂. Aqueous 5% NaHCO₃ was added and after extracting, the CH₂Cl₂ layer was washed with 5% NaHCO₃ and then water (2x), dried (Na₂SO₄), and concentrated to give 189 mg (89%) of title compound as an amorphous pale yellow solid.

### E.

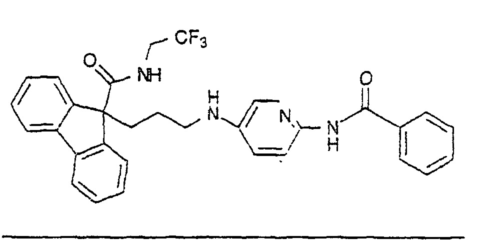

Acetic acid (0.29 mL, 5.1 mmol) was added to a stirred suspension of 180 mg (0.85 mmol) of Part D compound and 297 mg (0.85 mmol) of Part A compound in 5 mL of 1,2-dichloroethane. After 5 min, NaBH(OAc)₃ (540 mg, 2.55 mmol) was added to the clear solution and the reaction was stirred for 16 h at room temperature. The reaction was diluted with CH₂Cl₂ and 5% NaHCO₃ and the layers were separated. The CH₂Cl₂ was washed with 5% NaHCO₃ and water (2x), dried (Na₂SO₄), and concentrated to a foam (479 mg). Chromatography of this foam over a column of silica gel (40 g) packed in CH₂Cl₂, by eluting with CH₂Cl₂-MeOH (97:3), gave 429 mg of impure title compound. Chromatography of the 429 mg sample over 40 g of silica gel using CH₂Cl₂-EtOAc (8:2) gave 246 mg (53%) of title compound as a gummy residue.

### F. 9-[3-[[2-(Benzoylamino)-5-pyridinyl]-amino]propyl]-N-(2,2,2-trifluoroethyl)-9H-fluorene-9-carboxamide, monohydrochloride

To a solution of Part E compound (243 mg, 0.446 mmol) in 3 mL of dry THF was added 0.4 mL of 4 N HCl in dioxane (1.6 mmol). Ether was added to the clear solution and the precipitate was collected, washed with Et₂O, and dried at 40°C/0.5 mm for 4 h to give 225 mg (82%) title compound as a pale yellow solid having mp 120-126°C.
MS (ESI-NH₃, + ions) 545 (M+H); (- ions) 543 (M-H).

| | | | | | |
|---|---|---|---|---|---|
| Anal. Calcd for C₃₁H₂₇F₃N₄O₂ + HCl + 0.3 H₂O + 0.1 EtOAc + 0.3 Et₂O | C, 63.41; | H, 5.29; | N, 9.07; | Cl, 5.74; | F, 9.23 |
| Found | C, 63.40; | H, 5.25; | N, 8.88; | Cl, 5.60; | F, 9.10. |

### Example 297

### [[4-(Benzoylamino)phenyl]methyl][2-[9-[[(2,2,2-trifluoroethyl)amino]carbonyl]-9H-fluoren-9-yl]ethyl]-carbamic acid, 1,1-dimethylethyl ester

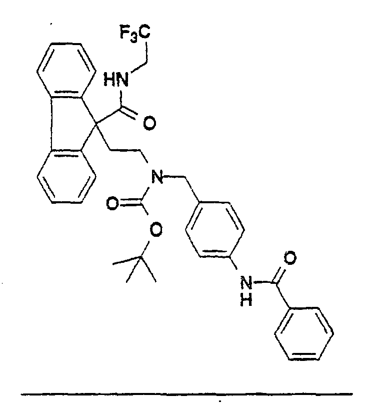

### A.

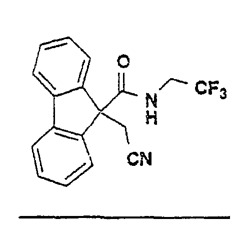

Butyllithium (18 mL, 2.5M in hexanes, 44 mmol) was added dropwise over 10 min to a solution of 9-fluorenecarboxylic acid (4.2 g, 20 mmol) in THF (200 mL) at 0°C under argon. The slightly heterogeneous dark yellow reaction was stirred at 0°C for 30 min, then chloroacetonitrile (1.5 mL, 24 mmol) was added dropwise over 3 min. The orange reaction was stirred at 0°C for 30 min, warmed to room temperature and stirred for 3 h. The reaction was extracted with water (2 x 100 mL) and the combined aqueous extracts were washed with Et₂O (100 mL). The aqueous layer was acidified to pH<2 with 1N HCl and extracted with CH₂Cl₂ (3 x 50 mL). The combined organic extracts were dried over MgSO₄, filtered, and concentrated in vacuo to give 4.7 g of a light yellow solid (mp 138-145°C).

A portion (2.63 g) of the crude carboxylic acid was dissolved in CH₂Cl₂ (30 mL) under argon. N,N-Dimethylformamide (40 µL, 0.53 mmol) was added followed by oxalyl chloride (8.0 mL, 2.0M in CH₂Cl₂, 15.9 mmol). The reaction bubbled for a few minutes and was allowed to stir at room temperature for 1.5 h. The reaction was concentrated in vacuo then pumped under high vacuum to give the crude acid chloride. Triethylamine (4.4 mL, 31.8 mmol) was added to a suspension of 2,2,2-trifluoroethylamine hydrochloride (1.71 g, 12.7 mmol) in CH₂Cl₂ (20 mL) at 0°C under argon. The resulting thick slurry was stirred at 0°C for 5 min, then a solution of the crude acid chloride in CH₂Cl₂ (10 mL) was added dropwise over 5 min. The reaction was stirred at 0°C for 10 min, diluted with CH₂Cl₂ (50 mL), washed with 1N HCl (2 x 20 mL) and saturated NaHCO₃ (30 mL), then dried over Na₂SO₄. Evaporation gave 3.5 g of a yellow foam which was purified by flash chromato-graphy on silica (150 g) eluting with CH₂Cl₂ to give title compound (2.74 g, 76%) as a white solid (mp 159-159.5).

### B.

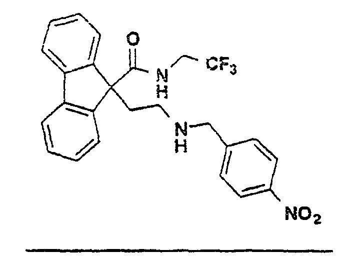

To a solution of Part A compound (2.7 g, 8.2 mmol) in methanol (30 ml) and chloroform (1.3 ml, 16 mmol) was added platinum oxide (186 mg, 0.82 mmol). The reaction mixture was hydrogenated (balloon) for 3.5 days, filtered through Celite and concentrated *in vacuo* to give 3.13 g of the crude amine hydrochloride.

4-Nitrobenzyl bromide (1.57 g, 7.3 mmol) was added to a stirred solution of the crude amine hydrochloride (2.7 g, 7.3 mmol) and triethylamine (1.0 ml, 7.3 mmol) in THF (15 ml) at 0°C. The reaction stirred under argon in a melting ice bath overnight. Reaction mixture partitioned between ethyl acetate and saturated sodium bicarbonate solution. Aqueous layer extracted one time with ethyl acetate. The combined organic layers were dried (Na₂SO₄), and the solvent removed *in vacuo* to give a yellow oil which was purified by flash chromatography (SiO₂, 400g) packed and run with 30% EtOAc in methylene chloride to give title compound as a clear oil (940 mg, 27.5% yield).

### C.

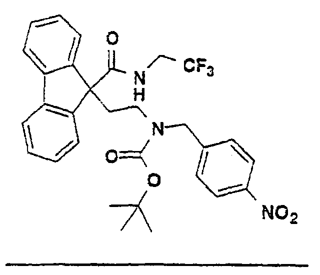

To the yellow solution of Part B compound (900 mg, 1.9 mmol) and 4-dimethylaminopyridine (280 mg, 2.3 mmol) in methylene chloride (10 ml) was added di-tert-butyldicarbonate (500 mg, 2.3 mmol) and the reaction stirred under argon at room temperature 1.5 h. More di-tert-butyldicarbonate (85 mg, 0.46 mmol) was added and the reaction stirred 1 h. The reaction was partitioned between methylene chloride and brine. The organic layer was dried (Na₂SO₄) and the solvent removed in *vacuo* to give a yellow oil which was purified by flash chromatagraphy (SiO₂, 100g) packed and run with 5% EtOAc in methylene chloride to give title compound as a solid white foam (944 mg, 86.6% yield).

### D. [[4-(Benzoylamino)phenyl]methyl][2-[9-[[(2,2,2-trifluoroethyl)amino]carbonyl]-9H-fluoren-9-yl]ethyl]carbamic acid, 1,1-dimethylethyl ester

10% Palladium on carbon (200 mg, catalyst) was added to a solution of Part C compound (860 mg, 1.5 mmol) in EtOAc (10ml) and the mixture hydrogenated (balloon) for 2h. The reaction was filtered through Celite and the Celite rinsed with EtOAc. A portion of the resulting amine solution (32 ml) was used in the next reaction.

To the amine solution (15 ml, -0.71 mmol) cooled to -5°C was added triethylamine (99 µl, 0.71 mmol) followed by benzoyl chloride (82 µl, 0.71 mmol). The reaction was stirred at -5°C under argon for 2 h. The reaction mixture was partitioned between ethyl acetate and water. The organic layer was dried (Na₂SO₄) and the solvent removed *in vacuo* to give a clear oil which was purified by flash chromatagraphy (SiO₂, 50 g) packed and run with 30% EtOAc in hexanes to give title compound as a solid white foam (369 mg, 80.9% yield).
mp 96-98°C.
MS (ESI, + ions) m/z 644 (M + H).

| | | | |
|---|---|---|---|
| Anal. calc'd for C₃₇H₃₆F₃N₃O₄ | C, 69.04; | H, 5.64; | N,6.53 |
| Found | C, 68.94; | H, 5.65; | N,6.27. |

### Example 298

### 9-[2-[[[4-(Benzoylamino)phenyl]methyl]amino]ethyl]-N-(2,2,2-trifluoroethyl)-9H-fluorene-9-carboxamide, monohydrochloride

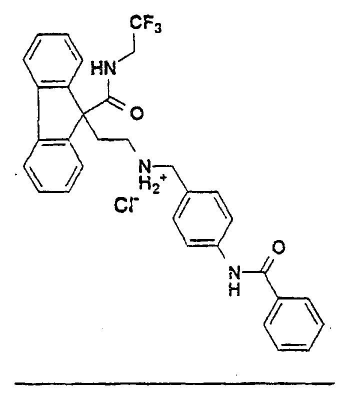

A solution of Example 297 compound (264 mg, 0.41 mmol) in 1.1 ml 4.0M HCl in dioxane was stirred under argon at room temperature for 2h. The solvent was removed in vacuo at 30°C. The residue was mixed with toluene, and the toluene removed *in vacuo* to give title compound as a white solid (193 mg, 81.1% yield).
mp 135-38°C.
MS (ESI, + ions) m/z 544 (M + H) ; 1087 (2M + H).

| | | | |
|---|---|---|---|
| Anal. calc'd for C₃₂H₂₈F₃N₃O₂ + 1HCl + 0.1 dioxane + 0.1 toluene | C, 65.49; | H, 5.25; | N,6.92 |
| Found | C, 65.54; | H, 5.50; | N,6.66. |

### Example 299

### 9-[4-[Butoxy(tetrahydrofuran-2-ylmethoxy)-phosphinyl]-butyl]-N-(2,2,2-trifluoroethyl)-9H-fluorene-9-carboxamide

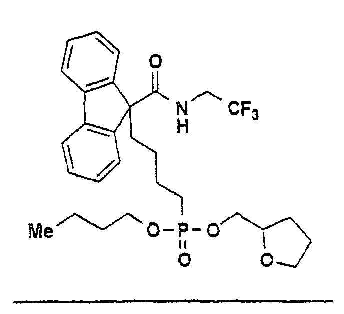

### A.

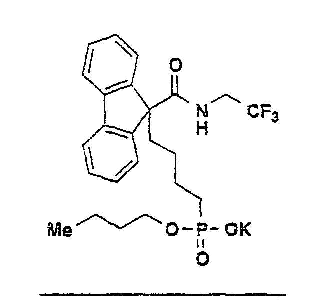

To a solution of 1 g (1.85 mmol) of Example 186 compound in 10 mL of a 3:7 water/n-butanol solution was added 1 g (18.50 mmol) of KOH pellets. The mixture was heated to 100°C for 5 days, at which time it was evaporated to remove n-butanol and freeze dried. The residue was purified by MPLC on a column of CHP20P gel (2.5 cm diam. X 20 cm height) eluting with water (1 L) followed by a gradient created by the gradual addition of 500 mL of acetonitrile to a reservoir of 700 mL of water. Fractions #34 to 40 were pooled. The acetonitrile was removed under reduced pressure and the aqueous solution was freeze dried to provide 695 mg (72%) of title compound as a white lyophilate.
TLC: silica gel (8:1:1 n-propanol/water/aqueous NH₃) R_{f}=0.63.
MS((ES-NH₄OH, + ions) m/z 525 (M+H+CH₃CN), 501 (M+NH₄), 484 (M+H)

| | | | | |
|---|---|---|---|---|
| Anal. Calcd for C₂₄H₂₈NO₄PF₃K + 0.93 H₂O | C, 53.56; | H, 5.59; | N, 2.60; | P, 5.75 |
| Found | C, 53.60; | H, 5.56; | N, 2.56; | P, 5.78. |

### B. 9-[4-[Butoxy(tetrahydrofuran-2-ylmethoxy)phosphinyl]-butyl]-N-(2,2,2-trifluoroethyl)-9H-fluorene-9-carboxamide

To a solution of 200 mg (0.38 mmol) of Part A compound in 3 mL of toluene, under argon at room temperature, was added dropwise 53 µL (0.73 mmol) of triethylamine followed by 146 µL (1.15 mmol) of chlorotrimethylsilane. The reaction was stirred for 1 h at which time it was evaporated to dryness to provide a pale yellow solid. The solid was dissolved in 3 mL of dichloromethane, under argon at room temperature, and treated with two drops of DMF followed by the dropwise addition of 283 µL (0.57 mmol) of oxalyl chloride (2.0 M in dichloromethane). The reaction was stirred for 0.5 h at which time it was evaporated to dryness to provide a yellow solid. The solid was dissolved in 3 mL of THF, under argon at room temperature, and treated dropwise with 58 µL (0.57 mmol) of tetrahydrofurfuryl alcohol and 31 µL (0.38 mmol) of pyridine. The reaction was stirred for 18 h at which time it was diluted with ether and washed with NaHCO₃, brine, dried (Na₂SO₄) and evaporated. Flash chromatography was performed on 75 g of silica gel eluting with 97:3 dichloromethane/isopropanol to provide 75 mg (35%) of title compound as a pale yellow oil.
MS (FAB, ± ions) m/z 568 (M + H), (FAB, - ion) 566 (M - H).
HRMS molecular ion calcd for C₂₉H₃₈NO₅PF₃ (M + H)
568.24398, found 568.2440.

### Example 300

### 9-[4-[Butoxy(2-pyridinylmethoxy)phosphinyl]butyl]-N-(2,2,2-trifluoroethyl)-9H-fluorene-9-carboxamide

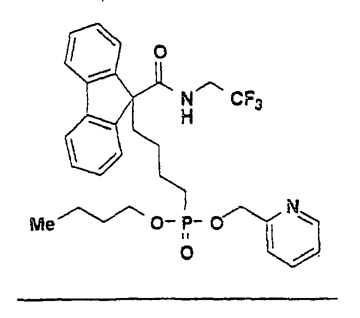

To a solution of 200 mg (0.38 mmol) of Example 299 Part A compound in 3 mL of toluene, under argon at room temperature, was added dropwise 53 µL (0.73 mmol) of triehtylamine followed by 146 µL (1.15 mmol) of chlorotrimethylsilane. The reaction was stirred for 1 h at which time it was evaporated to dryness to provide a pale yellow solid. The solid was dissolved in 3 mL of dichloromethane, under argon at room temperature, and treated with two drops of DMF followed by the dropwise addition of 290 µL (0.58 mmol) of oxalyl chloride (2.0 M in dichloromethane). The reaction was stirred for 0.5 h at which time it was evaporated to dryness to provide a yellow solid. The solid was dissolved in 3 mL of THF, under argon at RT, and treated dropwise with 73 µL (0.77 mmol) of 2-pyridylcarbinol. The reaction was stirred for 18 h at which time it was diluted with ether and washed with NaHCO₃, brine, dried (Na₂SO₄) and evaporated. Flash chromatography was performed on 65 g of silica gel eluting with 97:3 dichloromethane/isopropanol to provide 160 mg (73%) of title compound as a pale yellow oil.
MS (ES-NH₄OH, ± ions) m/z 575 (M + H).

| | | | | | |
|---|---|---|---|---|---|
| Anal. Calcd. for C₃₀H₃₄N₂O₄PF₃ + 0.65H₂O | C, 61.46; | H, 6.07; | N, 4.78; | F, 9.72; | P, 5.28. |
| Found: | C, 61.07; | H, 5.88; | N, 5.00; | F, 9.55; | P, 5.26. |

The following additional compounds of the invention were prepared following the procedures set out herein.

### Example 301

### 9-[4-(Dipropoxyphosphinyl)butyl]-N-(2,2,2-trifluoro-ethyl)-9H-fluorene-9-carboxamide

MS (ES-NH₄OH, + ions) m/z 529 (M+NH₄), 512 (M+H).

| | | | | |
|---|---|---|---|---|
| Anal. Calc'd for C₂₆H₃₃N₄PF₃ + 0.23 CH₂Cl₂ | C, 59.32; | H, 6.35; | N, 2.64; | P, 5.83 |
| Found | C, 59.31; | H, 6.46; | N, 2.88; | P, 5.68. |

### Example 302

### 9-[4-[4-[[(4-Nitrophenyl)sulfonyl]amino]phenyl]-butyl]-N-(2,2,2-trifluoroethyl)-9H-fluorene-9-carboxamide

mp 136-138°C.
MS (ES, - ions) m/z 622 (M-H).

| | | | | |
|---|---|---|---|---|
| Anal. Calc'd for C₃₂H₂₈N₃SO₅F₃ + 2.00 CH₂Cl₂ | C, 51.60; | H, 4.06; | N, 5.30; | S, 4.04 |
| Found | C, 51.70; | H, 4.00; | N, 5.20; | S, 4.17. |

### Example 303

### 9-[4-[4-[[(2-Nitrophenyl)sulfonyl]amino]phenyl]-butyl]-N-(2,2,2-trifluoroethyl)-9H-fluorene-9-carboxamide

mp 60-64°C.
MS (ES, - ions) m/z 622 (M-H).

| | | | | |
|---|---|---|---|---|
| Anal. Calc'd for C₃₂H₂₈N₃SO₅F₃ + 0.5 CH₂Cl₂ | C, 58.60; | H, 4.39; | N, 6.31; | S, 4.81 |
| Found | C, 58.61; | H, 4.41; | N, 6.14; | S, 4.88. |

### Example 304

### 9-[4-(Dibutoxyphosphinyl)butyl)-3,6-difluoro-N-(2,2,2-trifluoroethyl)-9H-fluorene-9-carboxamide

MS (ESI, M+H)⁺ = 576 m/z⁺.

| | | | |
|---|---|---|---|
| Anal. Calc'd for C₂₈H₃₅F₅NO₄P • 0.25 H₂O | C, 57.98; | H, 6.17; | N, 2.41 |
| Found | C, 57.95; | H, 6.22; | N, 2.23. |

### Example 305

### 9-[3-[[5-[(2-Phenoxybenzoyl)amino]-2-pyridinyl]-oxy]propyl]-N-propyl-9H-fluorene-9-carboxamide

mp 104-108°C.
MS (FAB, + ions) m/z 598 (M+H).

| | | | |
|---|---|---|---|
| Anal. Calc'd for C₃₈H₃₅N₃O₄ | C, 76,36; | H, 5.90; | N, 7.03 |
| Found | C, 75.86; | H, 5.80; | N, 6.96. |

### Example 306

### 9-[6-[(6-Ethoxy-2-benzothiazolyl)thio]hexyl]-N-(2,2,2-trifluoroethyl)-9H-fluorene-9-carboxamide

MS (FAB, + ions) m/z 585 (M+H).

| | | | | |
|---|---|---|---|---|
| Anal. Calc'd for C₃₁H₃₁N₂O₂S₂F₃ | C, 63.68; | H, 5.34; | N, 4.79; | F, 9.75 |
| Found | C, 63.43; | H, 5.37; | N, 4.61; | F, 9.78. |

### Example 307

### [4-[9-[[(2,2,2-Trifluoroethyl)amino]carbonyl]-9H-fluoren-9-yl]-butyl]phosphonic acid, di(1-methylethyl) ester

mp 91-94°C.
MS (ES-NH₄OH, + ions) m/z 512 (M+H).

| | | | | | |
|---|---|---|---|---|---|
| Anal. Calc'd for C₂₆H₃₃NO₄PF₃ + 0.13 CH₂Cl₂ | C, 60.06; | H, 6.42; | N, 2.68; | P, 5.93; | F, 10.91 |
| Found | C, 60.21; | H, 6.70; | N, 2.68; | P, 6.00; | F, 10.64. |

### Example 308

### [[4-[(2-Phenoxybenzoyl)amino]phenyl]methyl][2-[9-[[(2,2,2-trifluoroethyl)amino]carbonyl]-9H-fluoren-9-yl]ethyl]carbamic acid, 1,1-dimethylethyl ester

mp 83-85°C.
MS (ESI, + ions) m/z 753 (M+NH₄).

| | | | |
|---|---|---|---|
| Anal. Calc'd for C₄₃H₄₀F₃N₃O₅ + 1.4 H₂O | C, 67.87; | H, 5.67; | N, 5.52 |
| Found | C, 67.85; | H, 5.34; | N, 5.42. |

### Example 309

### 9-[2-[[[4-[(2-Phenoxybenzoyl)amino]phenyl]methyl]-amino]ethyl]-N-(2,2,2-trifluoroethyl)-9H-fluorene-9-carboxamide, monohydrochloride

mp 260-62°C.
MS (ESI, + ions) m/z 636 (M+H).

| | | | |
|---|---|---|---|
| Anal. Calc'd for C₃₈H₃₂F₃N₃O₃ • HCl | C, 67.90; | H, 4.95; | N, 6.25 |
| Found | C. 56.06; | H, 4.07; | N, 4.93. |

### Example 310

### [1-[4-[9-[[(2,2,2-Trifluoroethyl)amino]carbonyl]-9H-fluoren-9-yl]butyl]-1H-imidazol-4-yl]carbamic acid, 1,1-dimethylethyl ester

MS (ESI, + ions) m/z 543 (M+H)⁺; (ESI, - ions) m/z 541 (M-H)⁻.

| | | | | |
|---|---|---|---|---|
| Anal. Calc'd for C₂₉H₃₃F₃N₄O₃ + 0.1 C₆H₁₄ | C, 64.50; | H, 6.29; | N, 10.16; | F, 10.34 |
| Found | C, 64.18; | H, 6.39; | N, 9.86; | F, 9.54. |

The following Examples 311 to 313 describe preparation of compounds of the invention employing solid phase synthesis techniques as described hereinafter.

### Example 311

### 9-[4-[(6-Ethoxy-2-benzothiazolyl)thio]butyl]-N-propyl-9H-fluorene-9-carboxamide

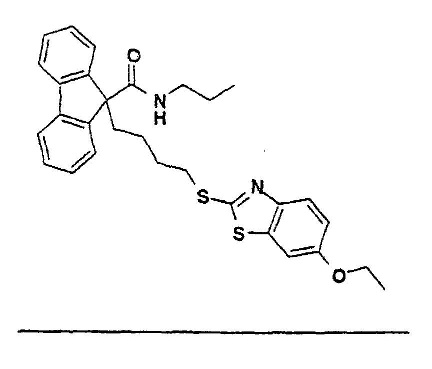

### A.

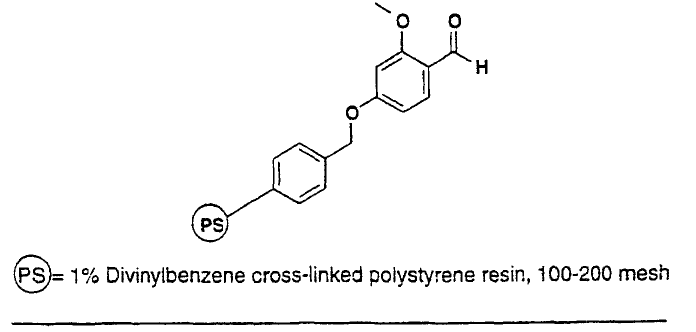

To a magnetically stirred suspension of 4.8 g (120 mmol, 10 eq) of sodium hydride (60% mineral oil dispersion) in 30 mL of dimethylformamide (DMF) at 0 °C was added a solution of 18.2 g (120 mmol, 10 eq) of 4-hydroxy-2-niethoxybenzaldehyde in 50 mL of DMF dropwise over 75 min. The reaction was allowed to warm to room temperature (RT) and stirred for an additional 75 min. The stirbar was removed and 10 g (12 mmol, 1 eq) of Merrifield resin (loading of 1.2 mmol/g, Advanced Chemtech) was added. The flask was placed in a heating mantel mounted on a vortex mixer and heated at 70°C (internal temperature) while vortexing for 26 h. The contents of the reaction vessel were transferred to a large filter funnel with a scintered-glass frit (porosity C) and rinsed sequentially with DMF (3 x 100 mL), 1:1 DMF:water (3 x 100 mL), water (2 x 100 mL) and MeOH (5 x 100 mL). The resin was dried under high vacuum (0.1 mm Hg) for 72 h to afford 11.16 g (98% of expected weight) of title product as a tacky non-freeflowing tan resin. The resin was characterized by gel-phase ¹³C-NMR and elemental analysis (chlorine and oxygen).
Elemental Analysis:
Chlorine: Expected 0% Cl for 100% loading; found 0.21%. Starting Cl content of resin was 4.26%. Residual Cl consistent with 95% resin loading.
Oxygen: Expected 5.76% for 100% loading; found 6.21%.

### B.

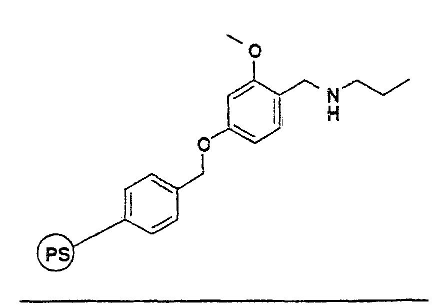

To a 25 mL Varian polypropylene tube fitted with a polyethylene frit and a luer stopcock was added 500 mg of Part A resin. The tube was sealed with a 19 mm Aldrich Suba septa and the resin was swollen in 5 mL of dry DMF, mixed by vortexing for 1 min and the DMF was removed using vacuum and N₂ pressure in order to maintain the vessel under inert atmosphere. Trimethyl orthoformate (1 mL) was added followed by 3.2 mL of DMF and 0.8 mL (10.0 mmol, 18 eq) of n-propylamine. The reaction mixture was vortexed for 18 h at room temperature. After removal of the reaction solution by nitrogen pressure and vacuum, 5 mL of a 200 mg/mL solution of sodium triacetoxyboro-hydride in DMF (1 g, 4.7 mmol, 8 eq) and 100 µL of acetic acid were added. The reaction mixture was vortexed for 8 h at room temperature. The reaction solution was removed and the resin was rinsed with DMF (4 x 5 mL), 1:1 DMF:water (2 x 5 mL), water (1 x 5 mL), DMF (3 x 5 mL) and dichloromethane (CH₂Cl₂) (4 x 5 mL). The last CH₂Cl₂ rinse was done with dry CH₂Cl₂ in the tube with the septa in place using nitrogen gas and vacuum to filter away the solvent and keep the reaction vessel under inert atmosphere. The title resin was used in the next step without characterization.

### C.

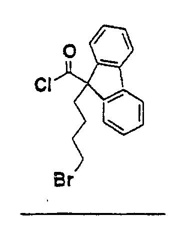

To 3.45 g (10 mmol, 1 eq) of Example 273 Part A(1) compound in 15 mL of CH₂Cl₂ was added 100 µL of DMF. The resulting solution was cooled to 0°C and 7.5 mL (15 mmol, 1.5 eq) of a 2.0 M oxalyl chloride solution in CH₂Cl₂ was added. The bubbling reaction mixture was stirred at 0°C for 15 min and then allowed to warm to room temperature. After 2 h, the reaction mixture was concentrated to afford the crude acid title chloride as a yellowish orange solid/oil mixture which was dissolved in CH₂Cl₂ and used without purification.

### D.

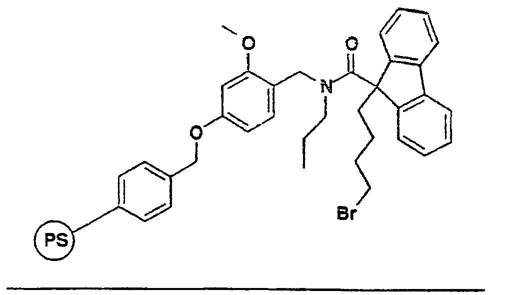

To the Part B resin in the polypropylene tube were added 1 mL of diisopropylethyl amine (5.7 mmol, 10 eq) and 1 mL of CH₂Cl₂ and the resulting mixture was mixed for 2 min. The tube was cooled to 0°C in an ice bath and 4 mL (2.2 mmol, 4 eq) of a solution of Part C acid chloride in CH₂Cl₂ was added. The resulting orange reaction mixture was mixed by vortexing at room temperature for 19 h. and then rinsed with CH₂Cl₂ (4 x 5 mL) to afford title resin which was used in the next step without characterization.

### E.

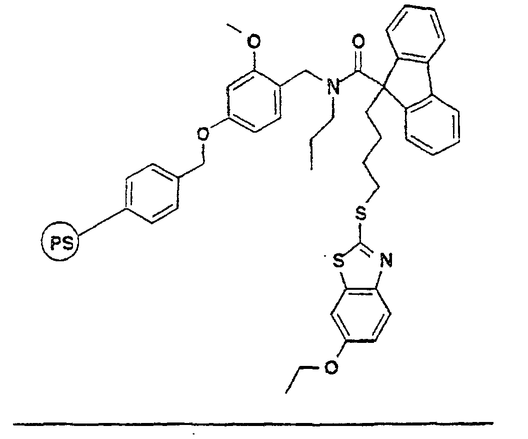

The Part D resin in the sealed polypropylene tube was swollen in 5 mL of dry DMF and vortexed for 2 min. The solvent was removed with N₂ and vacuum and a solution of 1.16 g (5.5 mmol, 10 eq) of 6-ethoxy-2-mercaptobenzothiazole in 4 mL of DMF was added to the resin followed by 5 mL (5 mmol, 9 eq) of a 1.0 M solution of sodium bistrimethylsilylamide in THF. Vortexing was initiated and the reaction mixture was mixed for 17 h at room temperature. The reaction solution was filtered away and the title resin was rinsed with DMF (4 x 5 mL), 1:1 DMF:water (2 x 5 mL), water (1 x 5 mL), DMF (3 x 5 mL) and dichloromethane (CH₂Cl₂) (4 x 5 mL).

### F. 9-[4-[(6-Ethoxy-2-benzothiazolyl)thio]-butyl]-N-propyl-9H-fluorene-9-carboxamide

The Part E resin was treated with 5 mL of 100% trifluoroacetic acid and vortexed for 90 min. The reaction solution was collected, the resin was rinsed with CH₂Cl₂ (3 x 1 mL) and the combined reaction solution and rinses were concentrated. The products from 3 parallel reactions were each redissolved in 15 mL of CH₂Cl₂, pooled and reconcentrated to afford 393 mg (46% crude) of an off-white solid. Recrystallization from MeOH afforded 339 mg (40%) of title compound as a white solid.
mp 112-113.5°C.
MS (electrospray, pos. ions): m/z 517 (M+H).

| | | | | |
|---|---|---|---|---|
| Anal. Calcd for C₃₀H₃₂N₂O₂S₂ | C, 69.73; | H, 6.24; | N, 5.42; | S, 12.41 |
| Found | C, 69.48; | H, 6.22; | N, 5.39; | S, 12.25. |

### Example 312

### 9-[4-[(4,5-Diphenyl-1H-imidazol-2-yl)thio]butyl]-N-[2-(4-methoxyphenyl)ethyl]-9H-fluorene-9-carboxamide

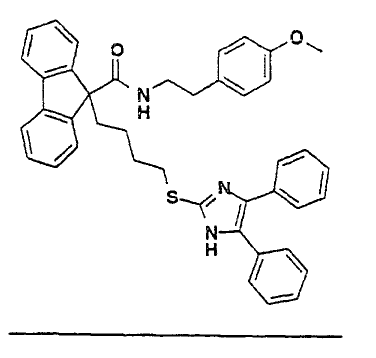

### A.

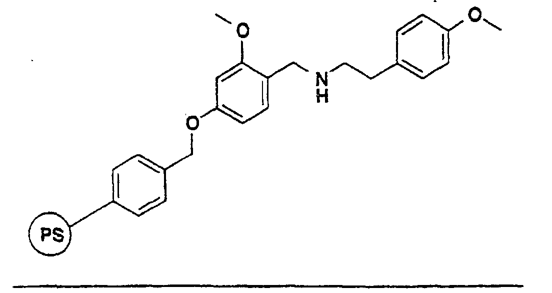

To a 25 mL Varian polypropylene tube fitted with a polyethylene frit and a luer stopcock was added 500 mg of Example 311 Part A resin. The tube was sealed with a 19 mm Aldrich Suba septa and the resin was swollen in 5 mL of dry DMF, mixed by vortexing for 1 min and the DMF was removed using vacuum and N₂ pressure in order to maintain the vessel under inert atmosphere. Trimethyl orthoformate (1 mL) was added followed by 2.6 mL of DMF and 1.46 mL (1.51 g, 10.0 mmol, 18 eq) of *p*-methoxyphenethylamine. The reaction mixture was vortexed for 18 h at RT. After removal of the reaction solution by nitrogen pressure and vacuum, 5 mL of a 200 mg/mL solution of sodium triacetoxyborohydride in DMF (1 g, 4.7 mmol, 8 eq) and 100 µL of acetic acid were added. The reaction mixture was vortexed for 8 h at room temperature. The reaction solution was removed and the resin was rinsed with DMF (4 x 5 mL), 1:1 DMF:water (2 x 5 mL), water (1 x 5 mL), DMF (3 x 5 mL) and dichloromethane (CH₂Cl₂) (4 x 5 mL). The last CH₂Cl₂ rinse was done with dry CH₂Cl₂ in the tube with the septa in place using nitrogen gas and vacuum to filter away the solvent and keep the reaction vessel under inert atmosphere. The title resin was used in the next step without characterization.

### B.

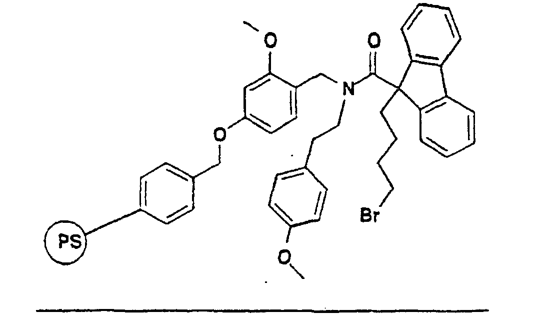

To the Part A resin in the polypropylene tube were added 1 mL of diisopropylethyl amine (5.7 mmol, 10 eq) and 1 mL of CH₂Cl₂ and the resulting mixture was mixed for 2 min. The tube was cooled to 0°C in an ice bath and 4 mL (2.2 mmol, 4 eq) of a solution of Example 311 Part C acid chloride in CH₂Cl₂ was added. The resulting orange reaction mixture was mixed by vortexing at room temperature for 19 h and then rinsed with CH₂Cl₂ (4 x 5 mL) to afford title resin which was used in the next step without characterization.

### c.

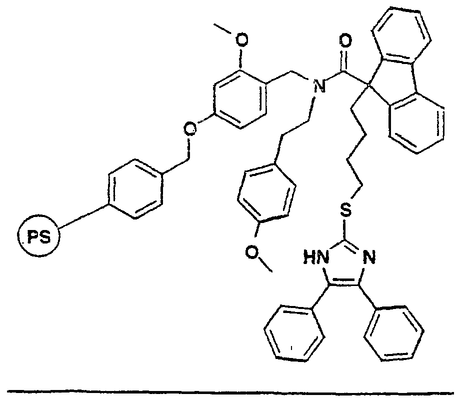

The Part B resin in the sealed polypropylene tube was swollen in 5 mL of dry DMF and vortexed for 2 min. The solvent was removed with N₂ and vacuum. To a suspension of 1.4 g (5.5 mmol, 10 eq) of 4,5-diphenyl-2-imidazolethiol in 5 mL of DMF was added 5 mL (5 mmol, 9 eq) of a 1.0 M solution of sodium bistrimethylsilylamide in THF. The resulting solution of thiolate anion was added to the resin, vortexing was initiated and the reaction mixture was mixed for 17 h at RT. The reaction solution was filtered away and the title resin was rinsed with DMF (4 x 5 mL), 1:1 DMF:water (2 x 5 mL), water (1 x 5 mL), DMF (3 x 5 mL) and dichloromethane (CH₂Cl₂)
(4 x 5 mL) and used in the next step without characterization.

### D. 9-[4-[(4,5-Diphenyl-1H-imidazol-2-yl)-thio]butyl]-N-[2-(4-methoxyphenyl)ethyl]-9H-fluorene-9-carboxamide

The Part C resin was treated with 5 mL of 100% trifluoroacetic acid and vortexed for 90 min. The reaction solution was collected, the resin was rinsed with CH₂Cl₂ (3 x 1 mL) and the combined reaction solution and rinses were concentrated. The products from 3 parallel reactions were each redissolved in 15 mL of CH₂Cl₂, pooled and reconcentrated to afford 729 mg (68% crude) of a yellow oil. Flash chromatography on silica gel (50 g) eluted with 2% MeOH in CH₂Cl₂ (1 L), followed by 5% MeOH in CH₂Cl₂
(1 L) afforded 208 mg (19%) of title compound as a white foam:
MS(electrospray, pos. ions): m/z 650 (M + H).

| | | | | |
|---|---|---|---|---|
| Anal. Calc'd for C₄₂H₃₉N₃O₂S + 0.63 CH₂Cl₂ | C, 71.72; | H, 5.59; | N, 5.97; | S, 4.56 |
| Found | C, 71.96; | H, 5.64; | N, 5.94; | S, 4.76. |

### Example 313

### 9-[4-(2-Thiazolylthio)butyl]-N-(2,2,2-trifluoroethyl)-9H-fluorene-9-carboxamide

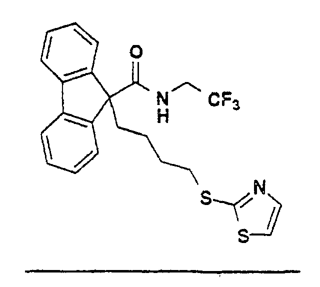

### A.

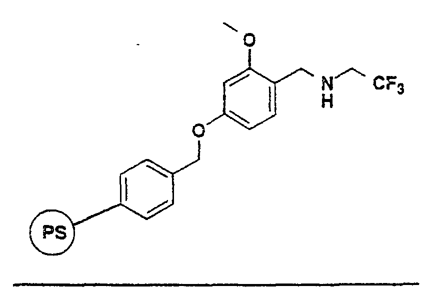

To a 25 mL Varian polypropylene tube fitted with a polyethylene frit and a luer stopcock was added 500 mg of Example 311 Part A resin. The tube was sealed with a 19 mm Aldrich Suba septa and the resin was swollen in 5 mL of dry DMF, mixed by vortexing for 1 min and the DMF was removed using vacuum and N₂ pressure in order to maintain the vessel under inert atmosphere. Trimethyl orthoformate (1 mL) was added followed by 3.2 mL of DMF and 796 µL (991 mg, 10.0 mmol, 18 eq) of 2,2,2-trifluoroethylamine. The reaction mixture was vortexed for 18 h at room temperature. After removal of the reaction solution by nitrogen pressure and vacuum, 5 mL of a 200 mg/mL solution of sodium triacetoxyboro-hydride in DMF (1 g, 4.7 mmol, 8 eq) and 100 µL of acetic acid were added. The reaction mixture was vortexed for 8 h at room temperature. The reaction solution was removed and the resin was rinsed with DMF (4 x 5 mL), 1:1 DMF:water (2 x 5 mL), water (1 x 5 mL), DMF (3 x 5 mL) and dichloromethane (CH₂Cl₂) (4 x 5 mL). The last CH₂Cl₂ rinse was done with dry CH₂Cl₂ in the tube with the septa in place using nitrogen gas and vacuum to filter away the solvent and keep the reaction vessel under inert atmosphere. The title resin was used in the next step without characterization.

### B.

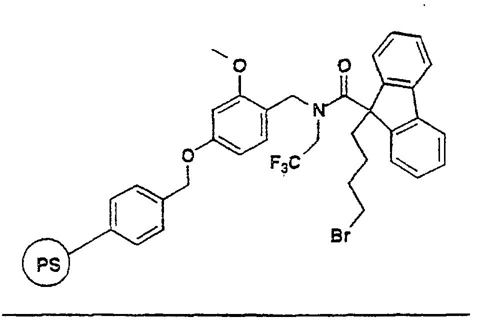

To the Part A resin in the polypropylene tube were added 1 mL of diisopropylethyl amine (5.7 mmol, 10 eq) and 1 mL of CH₂Cl₂ and the resulting mixture was mixed for 2 min. The tube was cooled to 0°C in an ice bath and 4 mL (2.2 mmol, 4 eq) of a solution of Example 311 Part C acid chloride in CH₂Cl₂ was added. The resulting orange reaction mixture was mixed by vortexing at RT for 19 h. and then rinsed with CH₂Cl₂ (4 x 5 mL) to afford title resin which was used in the next step without characterization.

### C.

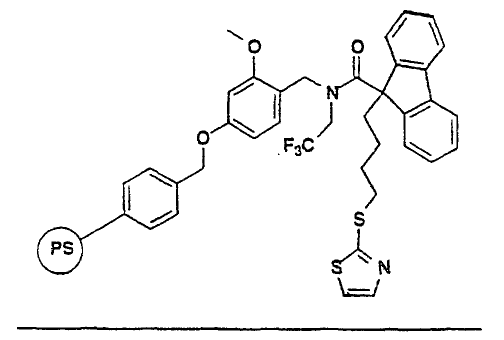

The Part B resin in the sealed polypropylene tube was swollen in 5 mL of dry DMF and vortexed for 2 min. The solvent was removed with N₂ and vacuum and a solution of 644 mg (5.5 mmol, 10 eq) of 2-mercaptothiazole in 4 mL of DMF was added to the resin followed by 5 mL (5 mmol, 9 eq) of a 1.0 M solution of sodium bistrimethylsilylamide in THF. Vortexing was initiated and the reaction mixture was mixed for 17 h at RT. The reaction solution was filtered away and the title resin was rinsed with DMF (4 x 5 mL), 1:1 DMF:water (2 x 5 mL), water (1 x 5 mL), DMF (3 x 5 mL) and dichloromethane (CH₂Cl₂) (4 x 5 mL).

### D. 9-[4-(2-Thiazolylthio)butyl]-N-(2,2,2-trifluoroethyl)-9H-fluorene-9-carboxamide

The Part C resin was treated with 5 mL of 100% trifluoroacetic acid and vortexed for 90 min. The reaction solution was collected, the resin was rinsed with CH₂Cl₂ (3 x 1 mL) and the combined reaction solution and rinses were concentrated. The products from 3 parallel reactions were each redissolved in 15 mL of CH₂Cl₂, pooled and reconcentrated to afford 395 mg (52% crude) of an off-white solid. Recrystal-lization from MeOH afforded 342 mg (45%) of title compound as a white solid.
mp 143-144°C.
MS(electrospray, pos. ions): m/z 463 (M + H).

| | | | | |
|---|---|---|---|---|
| Anal. Calcd for C₂₃H₂₁N₂O₂S₂F₃ | C, 59.72; | H, 4.58; | N, 6.06; | S, 13.86 |
| Found | C, 59.65; | H, 4.58; | N, 6.01; | S, 13.64. |

The following additional compounds were prepared employing solid phase synthesis techniques as described in Examples 311 to 313.

### Example 314

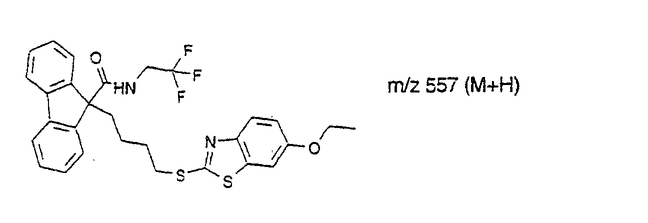

### Example 315

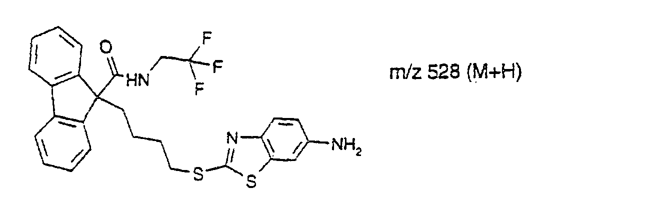

### Example 316

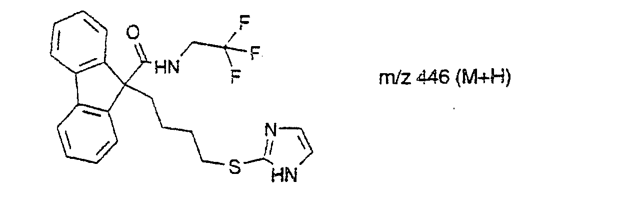

### Example 317

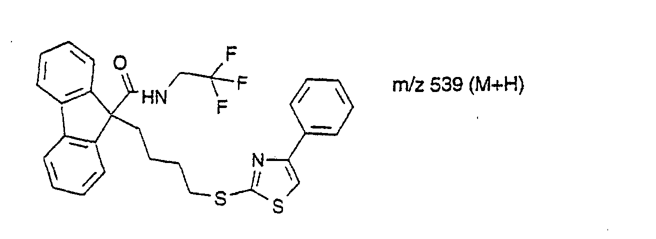

### Example 318

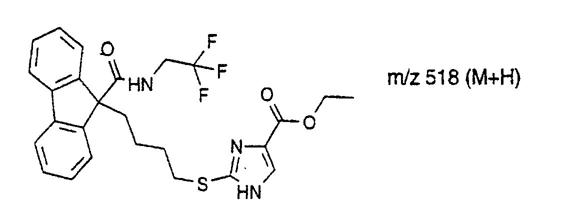

### Example 319

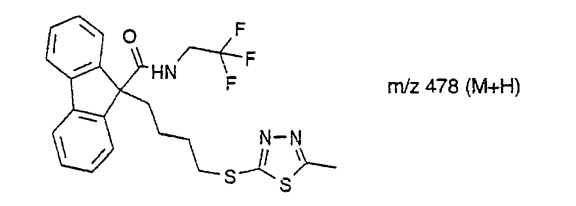

### Example 320

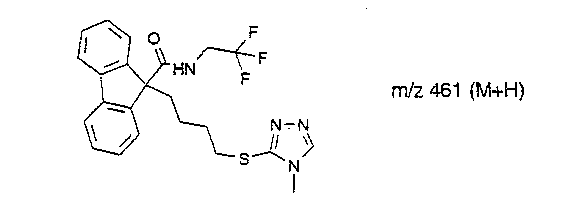

### Example 321

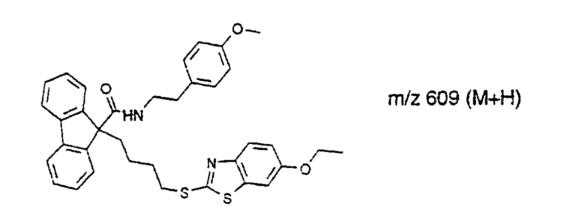

### Example 322

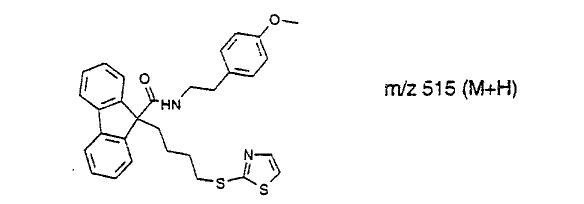

### Example 323

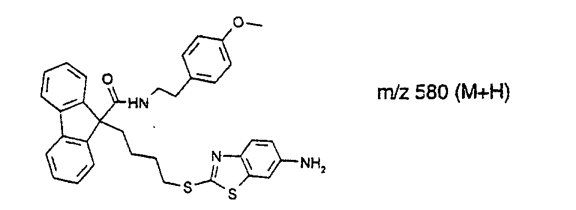

### Example 324

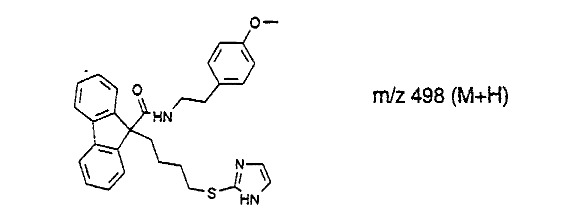

### Example 325

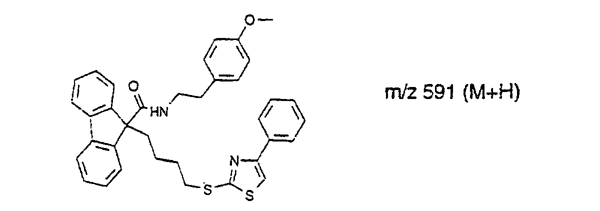

### Example 326

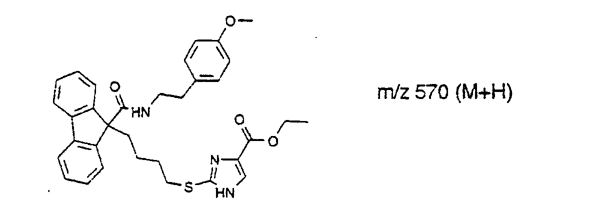

### Example 327

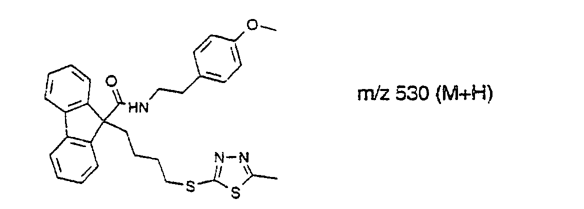

### Example 328

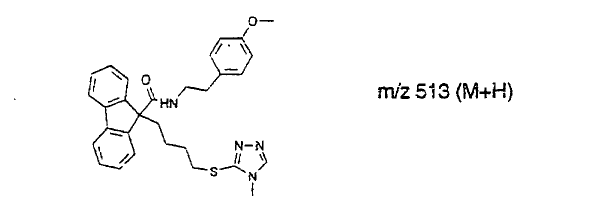

### Example 329

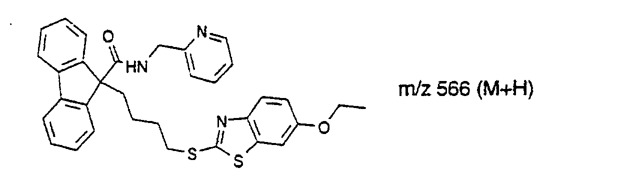

### Example 330

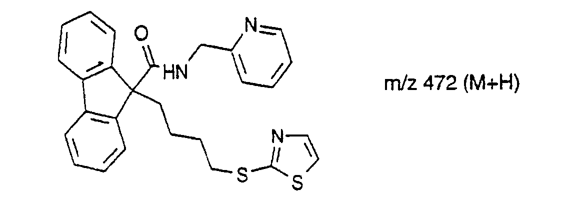

### Example 331

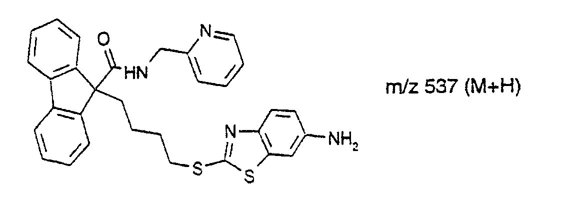

### Example 332

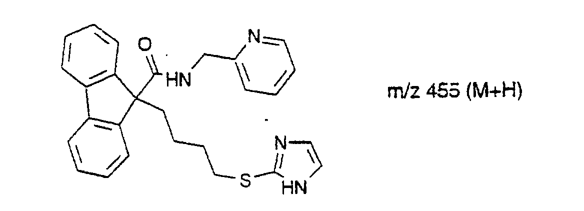

### Example 333

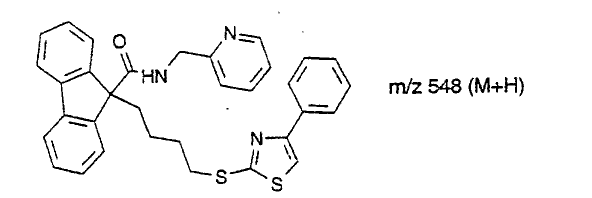

### Example 334

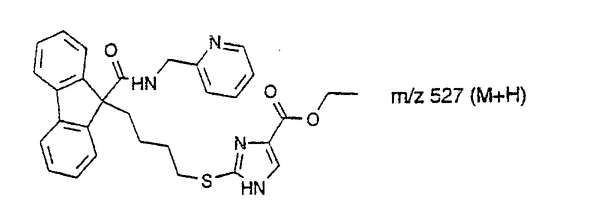

### Example335

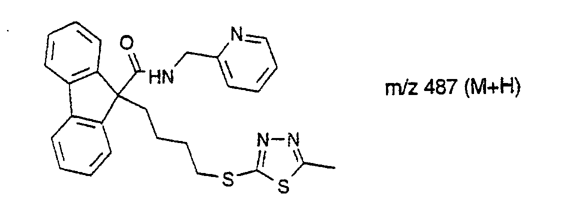

### Example 336

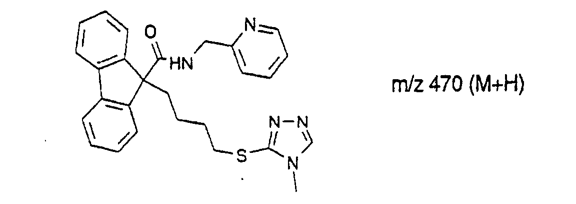

### Example 337

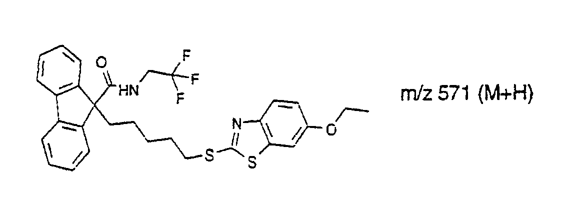

### Example 338

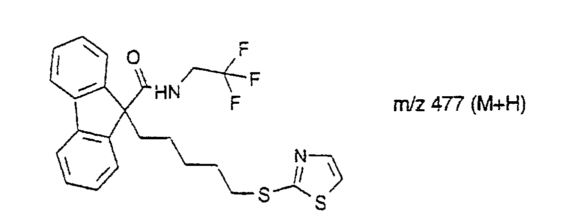

### Example 339

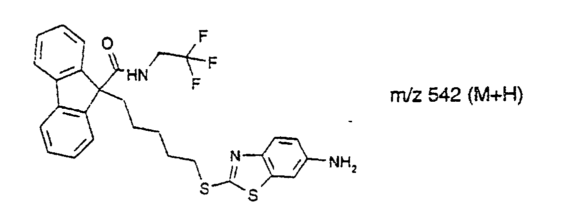

### Example 340

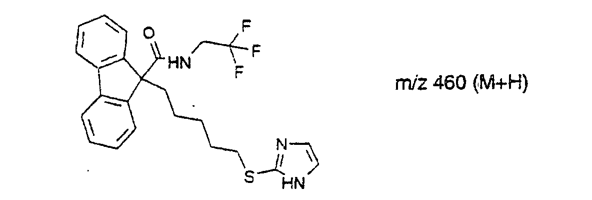

### Example 341

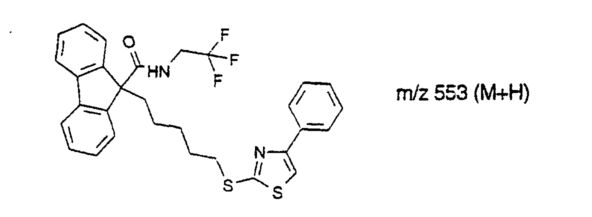

### Example 342

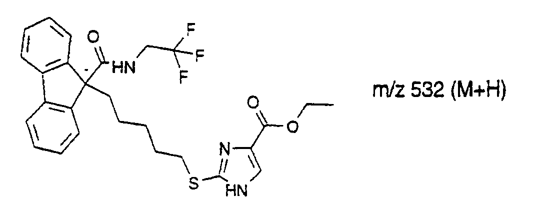

### Example 343

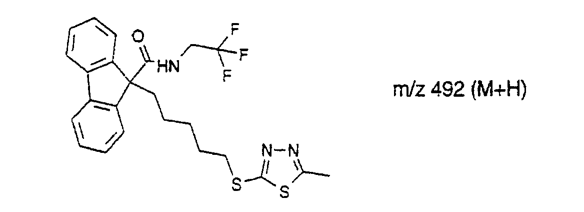

### Example 344

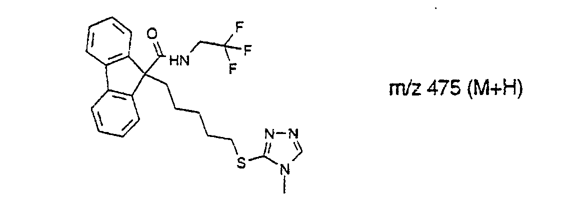

### Example 345

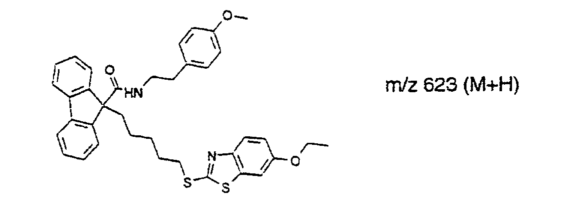

### Example 346

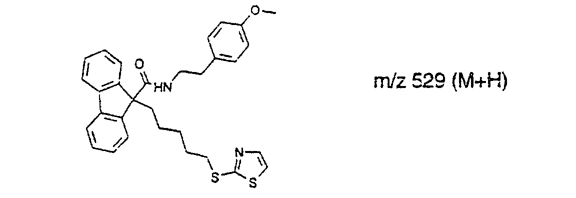

### Example 347

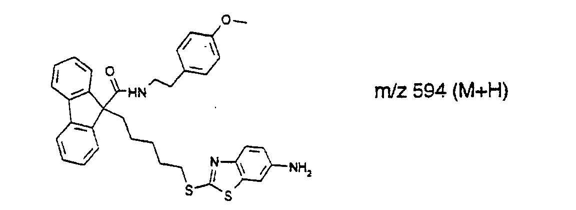

### Example 348

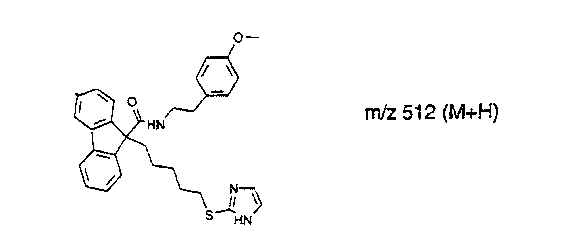

### Example 349

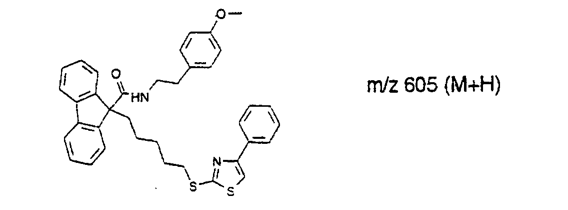

### Example 350

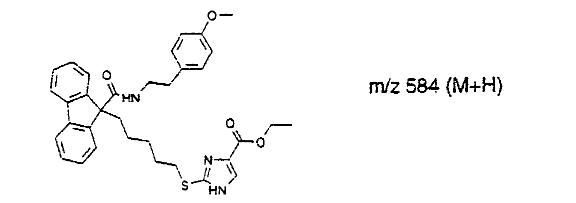

### Example 351

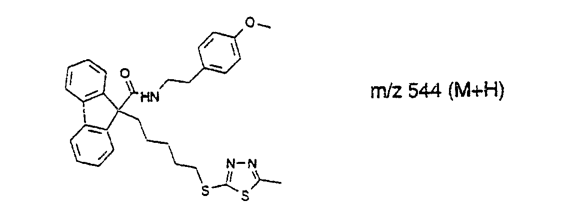

### Example 352

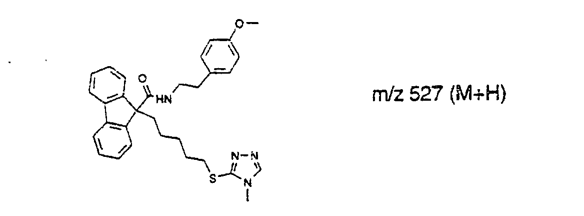

### Example 353

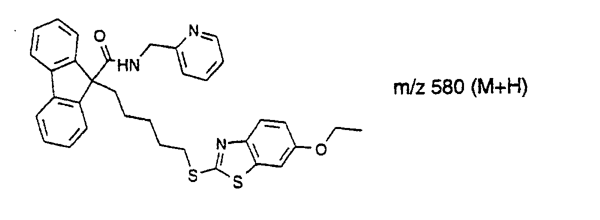

### Example 354

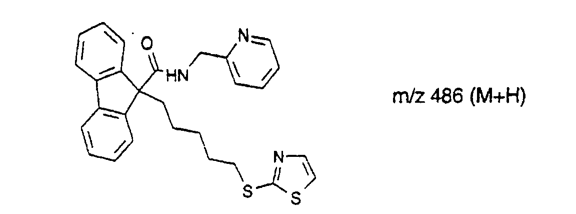

### Example 355

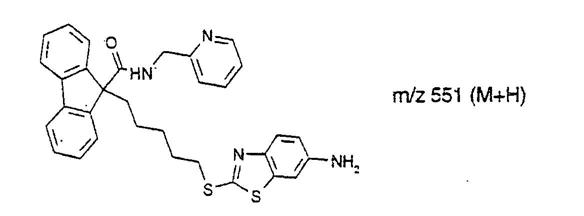

### Example 356

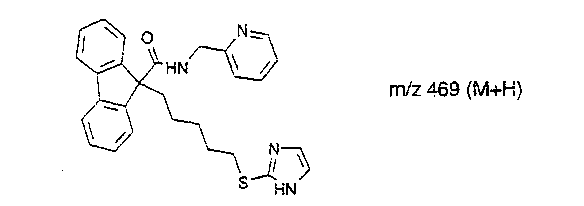

### Example 357

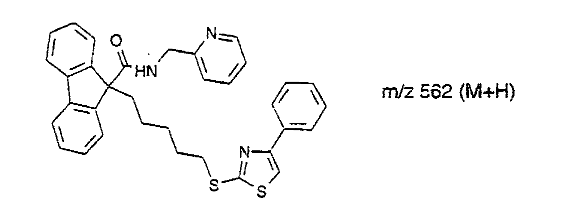

### Example 358

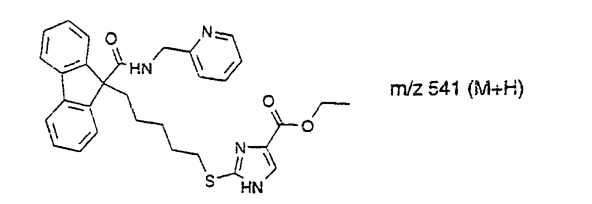

### Example 359

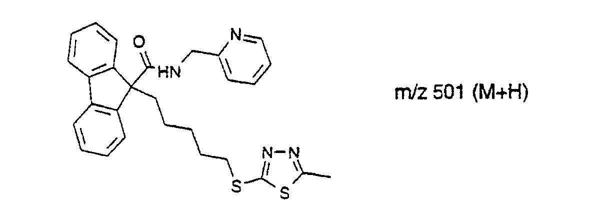

### Example 360

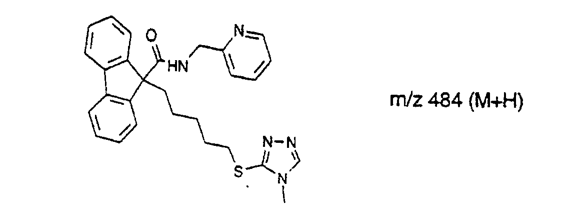

### Example 361

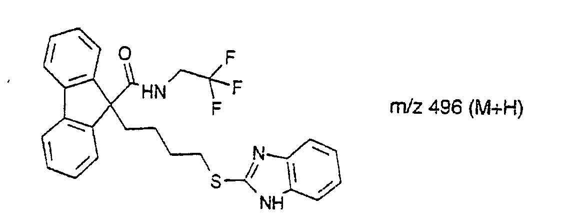

### Example 362

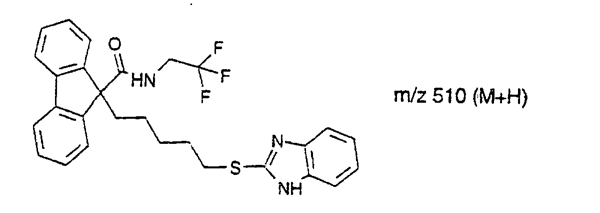

### Example 363

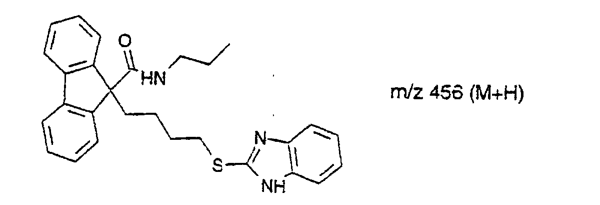

### Example 364

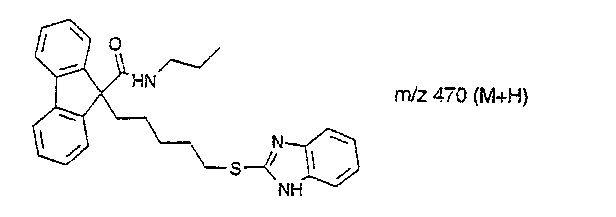

### Example 365

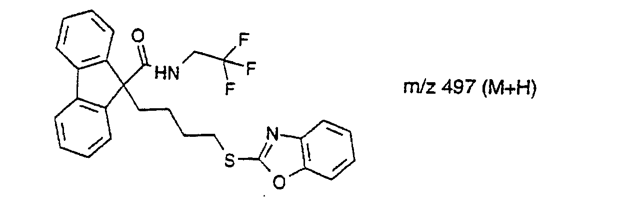

### Example 366

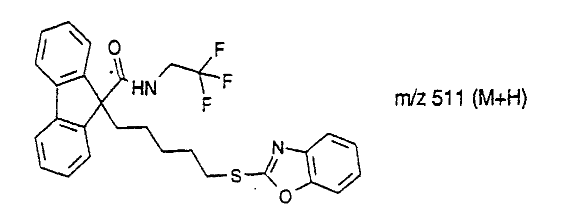

### Example 367

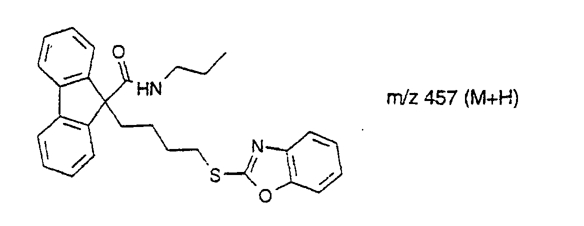

### Example 368

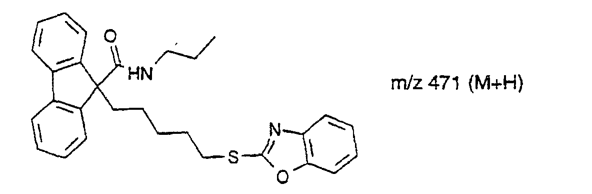

### Example 369

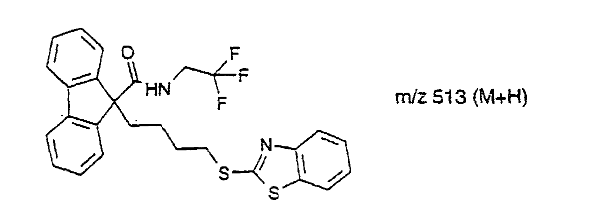

### Example 370

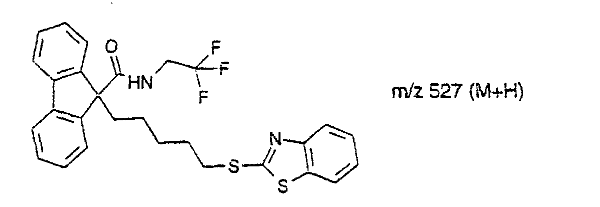

### Example 371

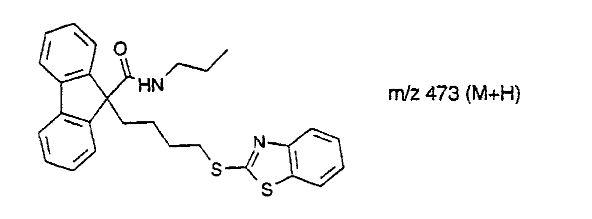

### Example 372

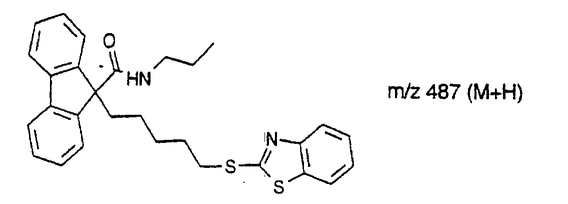

### Example 373

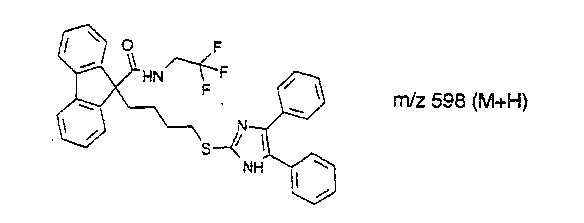

### Example 374

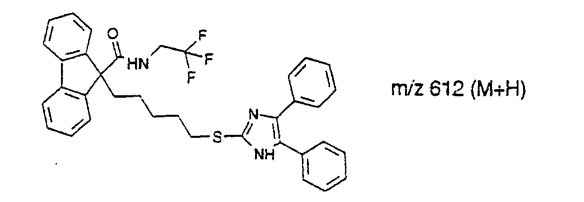

### Example 375

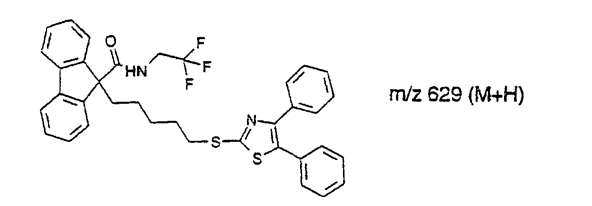

### Example 376

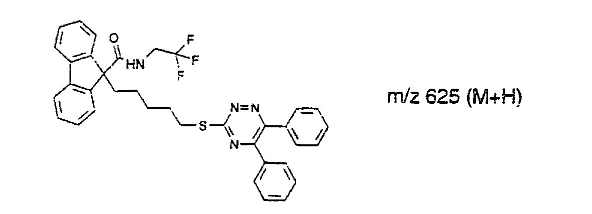

### Example 377

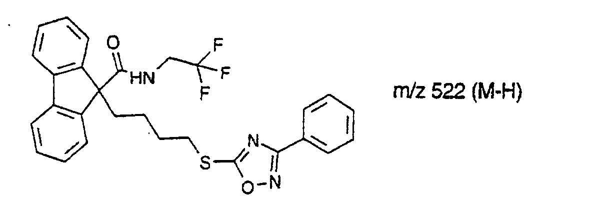

### Example 378

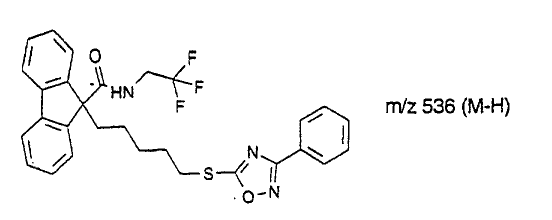

### Example 379

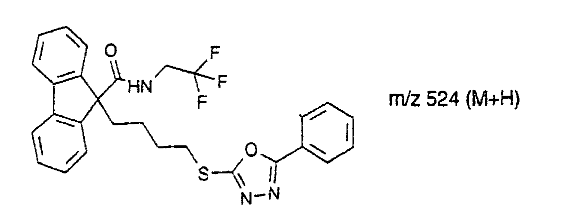

### Example 380

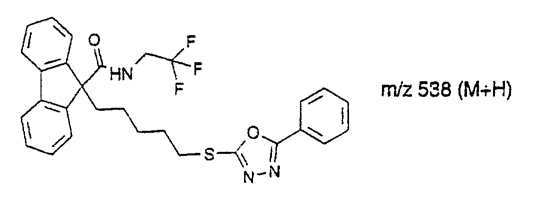

### Example 381

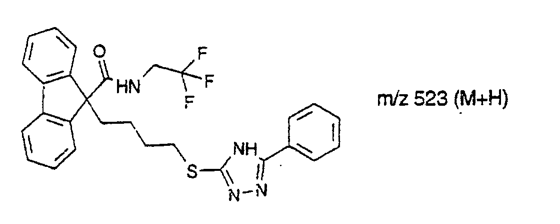

### Example 382

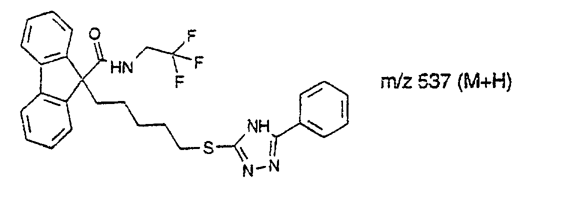

### Example 383

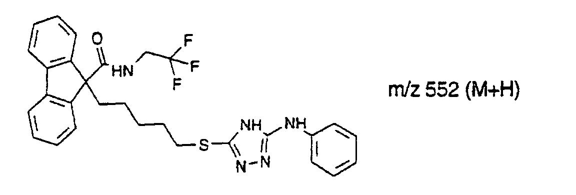

### Example 384

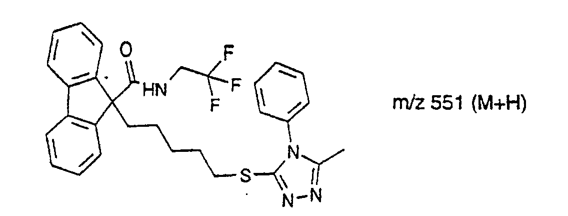

### Example 385

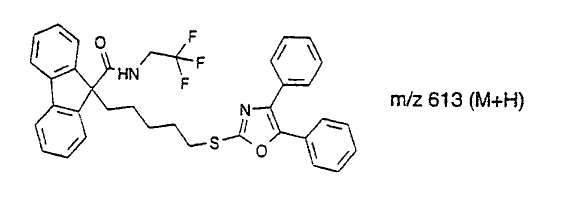

### Example 386

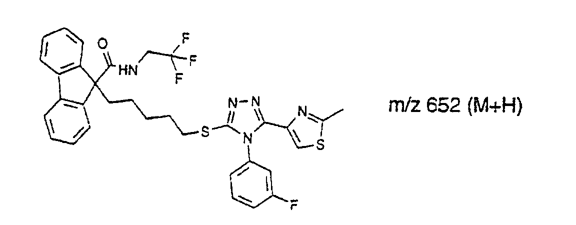

### Example 387

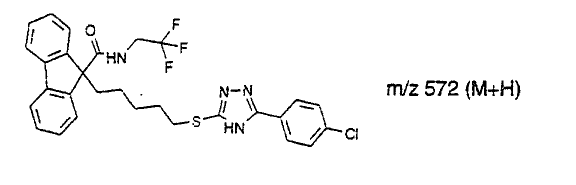

### Example 388

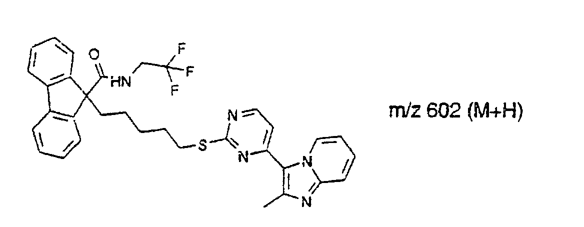

### Example 389

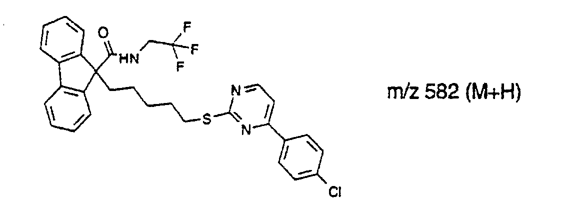

### Example 390

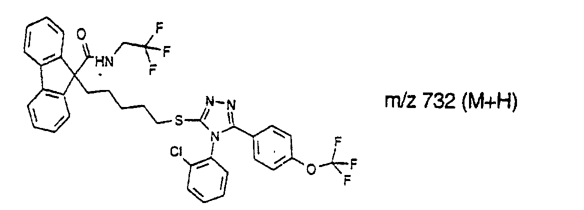

### Example 391

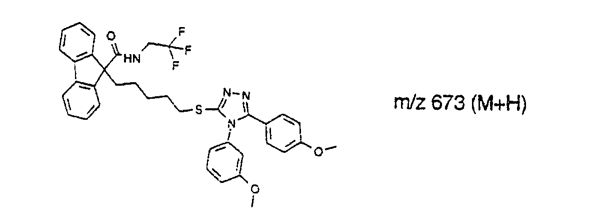

### Example 392

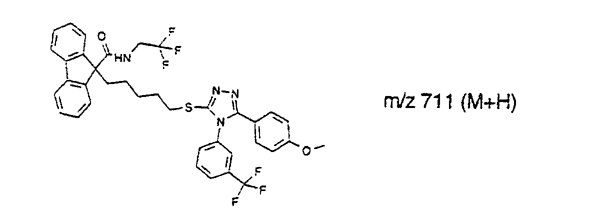

### Example 393

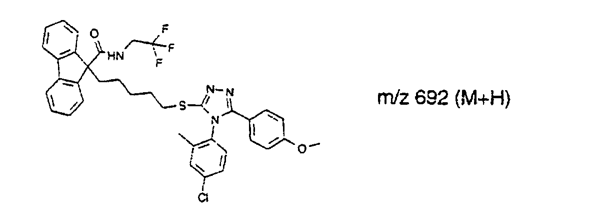

### Example 394

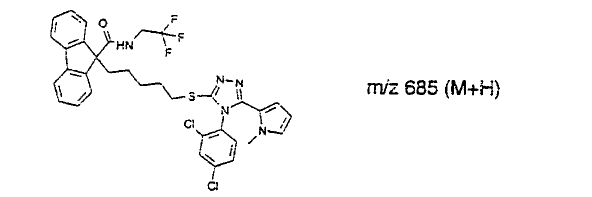

### Example 395

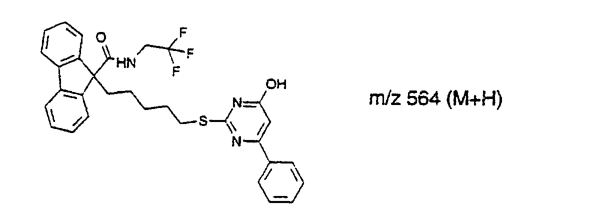

### Example 396

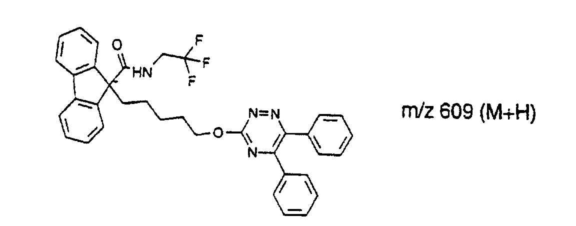

### Example 397

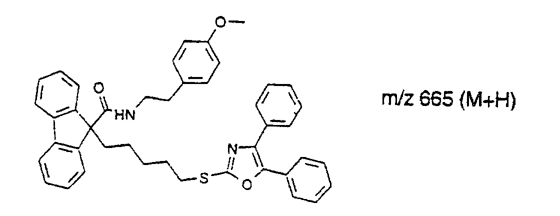

### Example 398

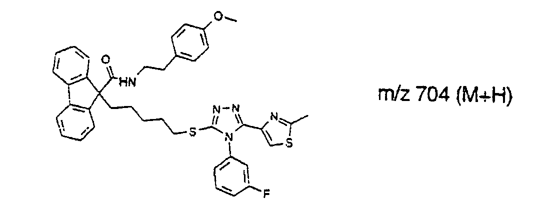

### Example 399

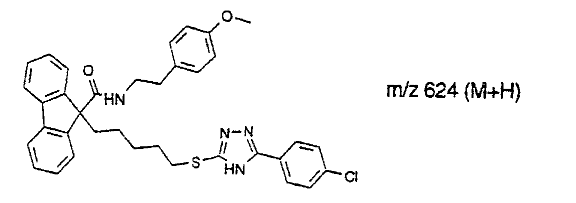

### Example 400

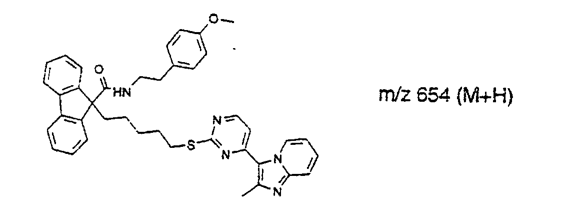

### Example 401

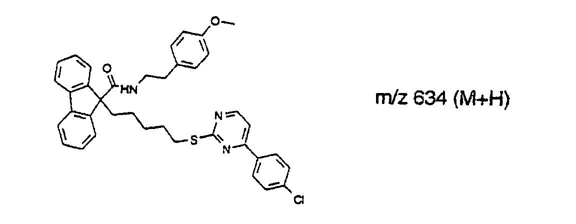

### Example 402

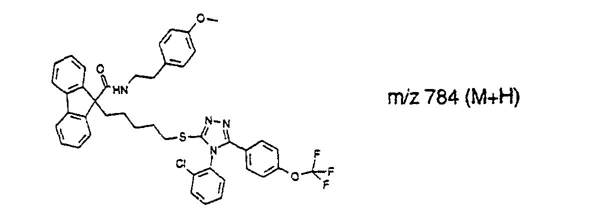

### Example 403

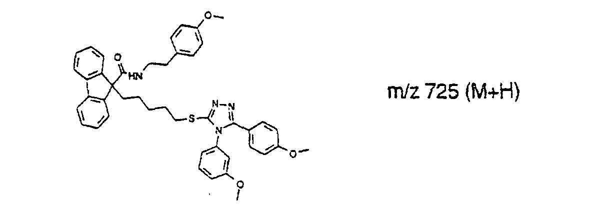

### Example 404

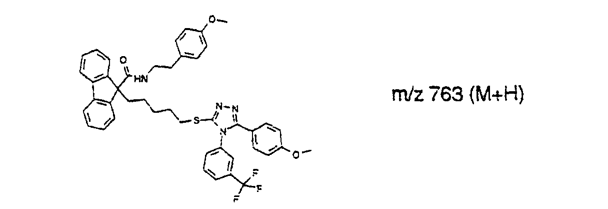

### Example 405

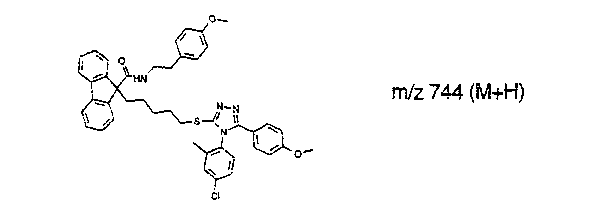

### Example 406

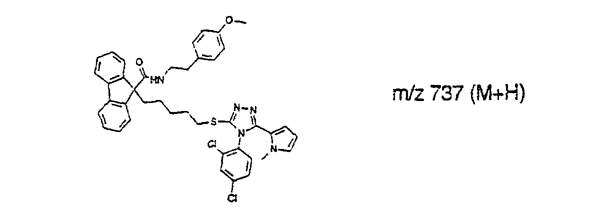

### Example 407

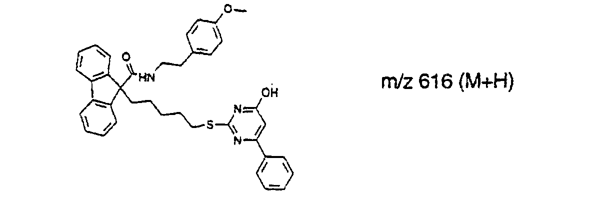

### Example 408

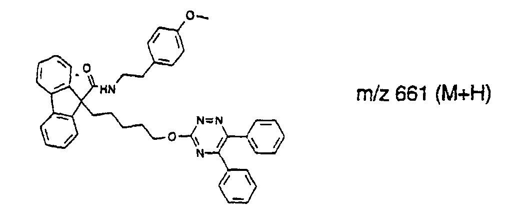

### Example 409

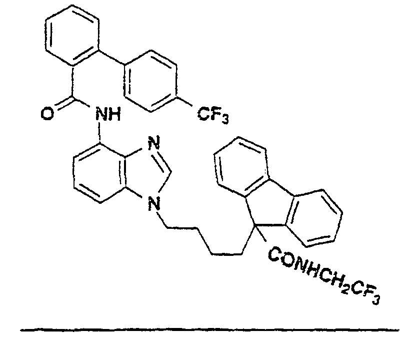

NOTE: The phrase "flash chromatography" refers to chromatography performed on EM Industries Silica Gel 60 (catalog #9385-9), 230-400 mesh under 10-20 psi of nitrogen pressure:

### A.

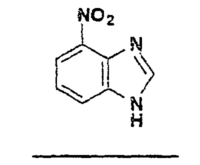

A stirred solution of 7.53 g (50.0 mmol) of 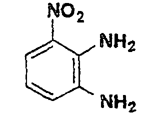 in 100 mL of 98% formic acid was set to reflux under argon for 3 hours. The reaction mixture was cooled and evaporated. The resulting solid residue was stirred with 100 mL of concentrated ammonium hydroxide for 30 min. The solids were collected, washed with 20 mL of water and dried *in vacuo* at 40°C to give title compound as a white solid, 7.76 g, 95%, mp 238-240°C.

### B.

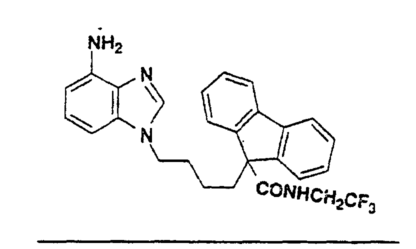

To a stirred solution of 2.50 g (15.0 mmol) of Part A compound in 30 mL of DMF at room temperature under argon was added 3.0 g (22 mmol) of potassium carbonate and, after 30 min, 6.80 g (16.0 mmol) of 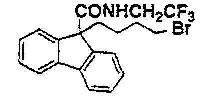 (prepared in Example 273 part A (2)). After 24h, the reaction mixture was quenched with 200 mL of water. The gummy solid that formed was collected, washed with water and dissolved in dichloromethane. This solution was washed twice with water, once with brine, dried (MgSO₄) and evaporated. The resulting semi-solid was triturated with cold ether and collected. Without characterization, a stirred slurry of this material and 200 mg of 10% palladium-on-charcoal in 50 mL of ethanol was purged with argon and evacuated three times. Hydrogen was introduced to the partially evacuated solution via a bladder. After 20 h, the reaction mixture was purged with argon, passed through a 0.45 µ nylon filter, washing with dichloromethane and evaporated. The oily product was purified by flash chromatography on silica gel (5x25 cm columm, 3:97 methanol/ethyl acetate) to give title compound as a white amorphous solid, 3.02 g, 42% overall yield from Part A compound.

### C.

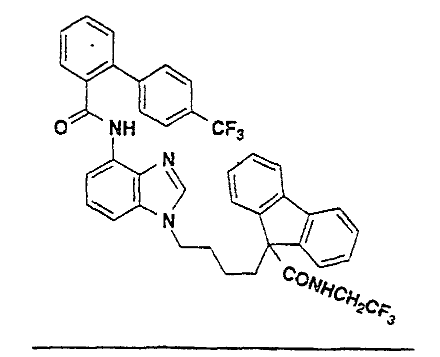

To a solution of 1.50 g (3.13 mmol) of Part B compound, 835 mg (3.13 mmol) of 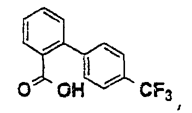 425 mg of HOAt (3.13 mmol) and 220 µL of triethylamine (1.58 mmol) in 10 mL of dichloromethane was added 680 mg (3.6 mmol) of EDAC. After 48 h, the reaction mixture was quenched with saturated sodium bicarbonate solution and extracted twice with ethyl acetate. The extracts were combined, dried (MgSO₄) and evaporated. Purification by flash chromatography on silica gel (5x20 cm column, 8:17 hexanes/ethyl acetate) gave title compound as a white amorphous solid, 1.43 g, 63%.

| | | | | |
|---|---|---|---|---|
| MICROANALYSIS: Calculated for C₄₁H₃₂F₆N₄O₂+0.5 EtOAc | C, 67.01; | H, 4.71; | N, 7.27; | F, 14.79 |
| Found | C, 66.95; | H, 4.36; | N, 7.36; | F, 14.76. |

MS (electrospray, + ions) m/e 727 (M+H).

### Example 410

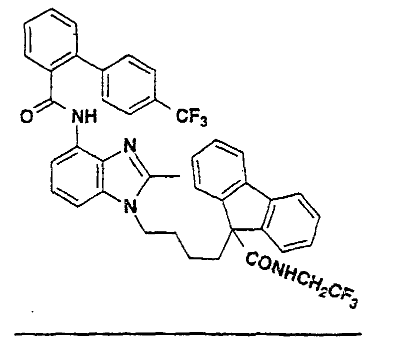

NOTE: The phrase "flash chromatography" refers to chromatography performed on EM Industries Silica Gel 60 (catalog #9385-9), 230-400 mesh under 10-20 psi of nitrogen pressure.

### A.

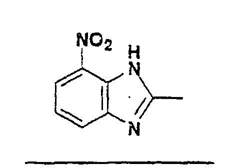

To a refluxing solution of 1.53 g (10.00 mmol) of 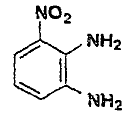 in 45 mL of ethanol and 12 mL of 5 M hydrochloric acid under argon was added 2.00 g (20.0 mmol) of 2,4-pentanedione over the course of 5 min. After an additional 25 min at reflux, the reaction was cooled, neutralized with saturated sodium bicarbonate solution and partially evaporated to remove ethanol. The residual mass was extracted twice with ethyl acetate. The extracts were combined, dried (MgSO₄) and evaporated to give title compound as a tan solid, 1.35 g, 76%, mp 215-217°C.

### B.

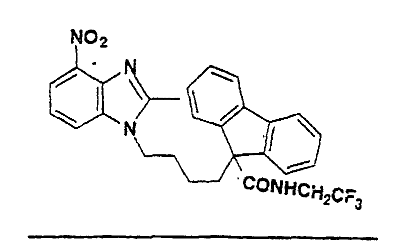

To a stirred slurry of 1.00 g of Part A compound (5.64 mmol) in 10 mL of DMF at room temperature under argon was added 1.00 g (7.2 mmol) of potassium carbonate. After 30 min. 2.55 g (6.0 mmol) of 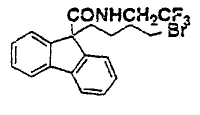 (prepared in Example 273 Part A(2)) was added and the reaction stirred for 86 h. The reaction mixture was quenched with 30 mL of water. The resulting solids were filtered, washed with water and dissolved in dichloromethane. The organic extract was washed with water, dried (MgSO₄) and evaporated onto 10 g of silica gel. Purification by flash chromatography (5x25 cm column, 3:7 ethyl acetate/dichloromethane) gave title compound as a white solid, mp 187-189°C, 2.03 g, 69%.

### C.

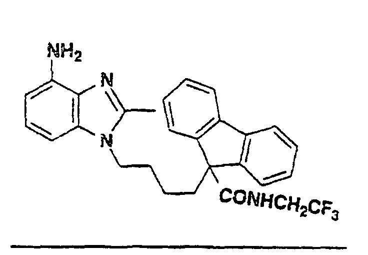

A stirred slurry of 1.00 g (1.91 mmol) of Part B compound and 200 mg of 10% palladium-on-charcoal in 25 mL of ethanol was purged with argon and evacuated three times. Hydrogen was introduced to the partially evacuated solution via a bladder. After 14 h, the reaction mixture was purged with argon and passed through a 0.45 µ nylon filter, washing with dichloromethane. The filtrate was evaporated and then re-evaporated twice from dichloromethane to give title compound as a white foam. The material was used in the next reaction without purification or characterization.

### D.

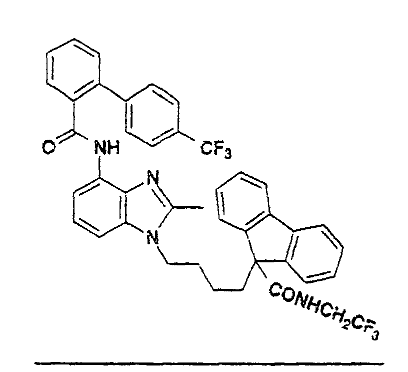

To all of Part C compound, was added 508 mg (1.90 mmol) of 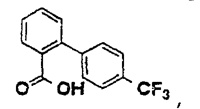 260 mg of HOAt (1.91 mmol) and 132 µL of triethylamine (0.95 mmol) in 10 mL of dichloromethane was added 230 mg (2.2 mmol) of EDAC. After 70 h, the reaction mixture was quenched with saturated sodium bicarbonate solution and extracted twice with dichloromethane. The extracts were combined, dried (MgSO₄) and evaporated. Purification by flash chromatography on silica gel (5x20 cm column, 1:4 ether/dichloromethane) gave title compound as a white solid, 1.10 g, 78%, mp 110-112°C.

| | | | | |
|---|---|---|---|---|
| MICROANALYSIS: Calculated for C₄₂H₃₄F₆N₄O₂ | C, 68.10; | H, 4.63; | N, 7.56; | F, 15.39 |
| Found | C, 67.82; | H, 4.69; | N, 7.31; | F, 15.44. |

MS (electrospray, + ions) m/e 741 (M+H).

### Example 411

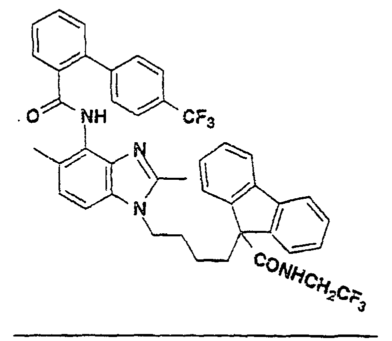

Preparation of compounds Parts A, B and C were by modifications of the procedures found in the following references:
1. S. Grivas, W. Tian, E. Ronne, S. Lindström and K. Olsson; *Acta Chem*. *Scand*., 47 521 (1993);
2. W. Tian and S. Grivas; *Synthesis* 29 1305 (1992).
NOTE: The phrase "flash chromatography" refers to chromatography performed on EM Industries Silica Gel 60 (catalog #9385-9), 230-400 mesh under 10-20 psi of nitrogen pressure.

### A.

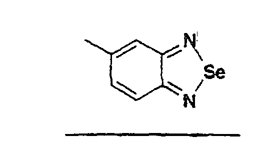

To a stirred solution of 48.95 g (0.400 mol) of 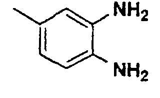 in 500 mL of 2.4 M hydrochloric acid at 80°C under argon, was added a warm solution of 88.77 g (0.800 mol) of selenium dioxide in 300 mL of water dropwise over the course of 30 min. After an additional 90 min, the reaction was cooled to room temperature and the solids were collected, washing with water. The brown solids were dried in vacuo at 50°C to give title compound, 75.10 g, 95% yield, mp 67-69°C.

### B.

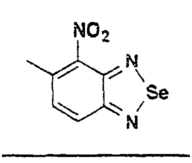

To a stirred solution of 72.00 g (0.365 mol) of Part A compound in 180 mL of 98% sulfuric acid at 10°C was added a cold solution of 108.0 mL of 2:1 98% sulfuric acid/70% nitric acid over 1 h. The temperature of the reaction mixture was not allowed to rise above 20°C. After an additional 60 min, the reaction was poured as a thin stream into 750 g of ice with rapid stirring. The fine yellow slurry was filtered and the collected solids were washed five times with 200 mL portions of cold water. The moist cake was heated in 500 mL of ethanol to near boiling and then cooled to room temperature and the solid collected. Drying *in vacuo* at 50°C gave title compound as a yellow solid, 80.70 g, 91% yield, mp 190-192°C.

| | | | | |
|---|---|---|---|---|
| MICROANALYSIS: Calculated for C₇H₅N₃O₂Se | C, 34.73; | H, 2.08; | N, 17.36; | Se, 32.61 |
| Found | C, 34.96; | H, 1.97; | N, 17.35; | Se, 32.59. |

### C.

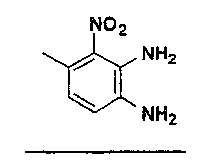

To a stirred solution of hydriodic acid (25.0 mL, 57%, 189 mmol, Aldrich catalog #21,002-1, stabilized with 1.5% hypophosphorous acid) at room temperature in argon was added 5.00 g (20.7 mmol) of Part B compund. The reaction vessel was placed in an oil bath pre-heated to 50°C and the resulting deep red solution was vigorously stirred for 2 h. After cooling to room temperature the reaction mixture was poured into a stirred slurry of 24 g (0.2 mol) of sodium hydrogen sulfite in 50 mL of water. The resulting light yellow slurry was treated with an ice-cold solution of sodium hydroxide (7.5 g, 188 mmol) in 50 mL of water. Additional 6 M sodium hydroxide was added until the aqueous slurry was brought to pH 8. The resulting deep red slurry was filtered and the filtrate extracted three times with 200 mL portions of chloroform. The solids from the filtration were dissolved in 300 mL of chloroform and washed once with 50 mL of water. The organic extracts were combined, dried (Na₂SO₄) and evaporated to give title compound as a deep red solid, 3.04 g, 88% yield, mp 132-133°C.

### D.

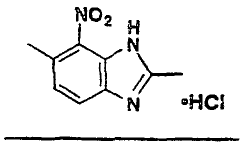

To a refluxing solution of 1.00 g (6.00 mmol) of Part C compound in 27 mL of ethanol and 7.2 mL of 5 M hydrochloric acid under argon was added 1.20 g (12.0 mmol) of 2,4-pentanedione over the course of 5 min. After an additional 60 min at reflux, the reaction was cooled and partially evaporated to remove ethanol. The resulting precipitate was filtered, washed with water and dried *in vacuo* at 40°C to give title compound as a tan solid, 1.12 g, 98%, mp 232-234°C.

### E.

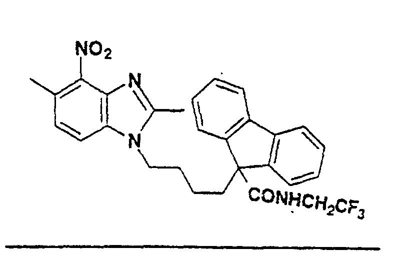

To a stirred slurry of 1.80 g of the free base of Part D compound (9.41 mmol) in 15 mL of DMF at room temperature under argon was added 1.75 g (33 mmol) of potassium carbonate. After 1 h, 4.26 g (10.0 mmol) of 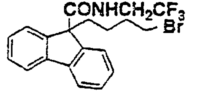 (prepared in Example 273 Part A(2)) was added and the reaction stirred for 86 h. The reaction mixture was quenched with 30 mL of water. The liquids were decanted away from the formed gummy solid, which was then washed with water. The semi-solid residue was triturated with 40 mL of ether. The resulting granular solid was chilled and filtered. The collected solid cake was washed with water, transferred to a round bottom flask and evaporated from toluene. The dried residual solid was triturated with hot ethyl acetate and filtered to give 4.02 g of title compound (80%) as a white solid, mp 181-183°C. Analytical HPLC indicated that the compound was 98.7% pure.

### F.

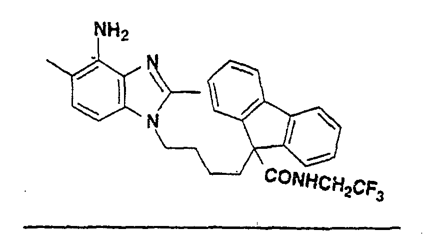

A stirred slurry of 1.05 g (1.96 mmol) of Part E compound and 200 mg of 10% palladium-on-charcoal in 40 mL of ethanol was purged with argon and evacuated three times. Hydrogen was introduced to the partially evacuated solution via a bladder. After 14 h, the reaction mixture was purged with argon and passed through a 0.45 µ nylon filter, washing with dichloromethane. The filtrate was evaporated and then re-evaporated twice from dichloromethane to give title compound as a white foam, 0.958 g, 99%.

### G.

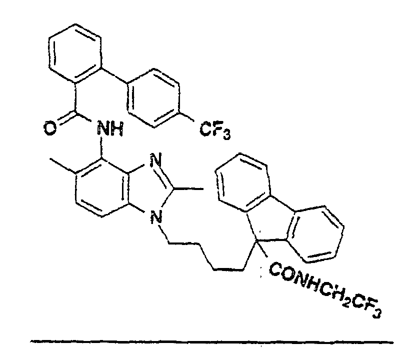

To a solution of 536 mg (1.00 mmol) of Part F compound, 270 mg (1.02 mmol) of 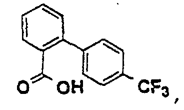 136 mg of HOAt (1.00 mmol) and 70 µL of triethylamine (0.5 mmol) in 2 mL of dichloromethane was added 230 mg (1.2 mmol) of EDAC. After 70 h, the reaction mixture was quenched with saturated sodium bicarbonate solution and extracted twice with dichloromethane. The extracts were combined, dried (MgSO₄) and evaporated. Purification by flash chromatography on silica gel (5x20 cm column, 1:9 hexanes/ethyl acetate) gave title compound as a white amorphous solid, 440 mg, 58%.

| | | | |
|---|---|---|---|
| MICROANALYSIS: Calculated for C₄₃H₃₆F₆N₄O₂+1.4 H₂O+0.2 EtOAc | C, 65.96; | H, 5.11; | N, 7.02 |
| Found | C, 65.95; | H, 4.72; | N, 7.08. |

MS (electrospray, + ions) m/e 755 (M+H).

### Preparation of G [ALTERNATIVE] :

To a stirred slurry of 1.72 g (6.47 mmol) of 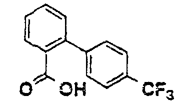 in 15 mL of dichloromethane (protected from atmospheric moisture by a Drierite-filled tube) was added 0.85 mL (9.74 mmol) of oxalyl chloride and then 0.1 mL of DMF. Gas evolves and, within a few minutes, a colorless solution formed. After 1 h, IR indicated that complete reaction had occurred. The reaction was evaporated twice from dichloromethane and then rediluted with 10 mL of dichloromethane. This solution was added dropwise to a solution of 3.21 g of Part F compound and 1.00 mL (7.17 mmol) of triethylamine at 0°C under argon. Total addition took 20 min and then the reaction was warmed to room temperature. After 90 min, the reaction mixture was quenched with saturated sodium bicarbonate solution and extracted twice with dichloromethane. The extracts were combined, dried (MgSO₄) and evaporated. Recrystallization from ethyl acetate/hexanes provided title compound as a white solid, mp 126-128°C, 3.86 g, 81% yield.

### Example 412

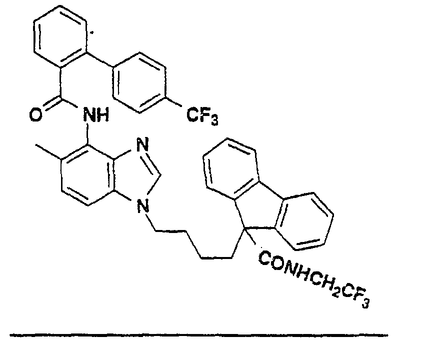

NOTE: The phrase "flash chromatography" refers to chromatography performed on EM Industries Silica Gel 60 (catalog #9385-9), 230-400 mesh under 10-20 psi of nitrogen pressure.

### A.

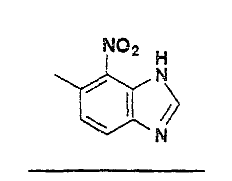

A refluxing solution of 1.586 g (9.49 mmol) of Example 411 Part C in 19 mL of 98% formic acid under argon was stirred for 90 min. The reaction mixture was cooled and evaporated. The syrupy residue was cautiously treated with 20 mL of concentrated ammonium hydroxide solution and stirred for 15 min. The resulting tan solid was collected, washed with 20 mL of cold water and dried in vacuo at 40°C to give title compound as a tan solid, 1.63 g, 97%, mp 237-239°C.

| | | | |
|---|---|---|---|
| MICROANALYSIS: Calculated for C₈H₇N₃O₂+0.12 H₂O | C, 53.58; | H, 4.07; | N, 23.43 |
| Found | C, 53.66; | H, 3.88; | N, 23.62. |

### B.

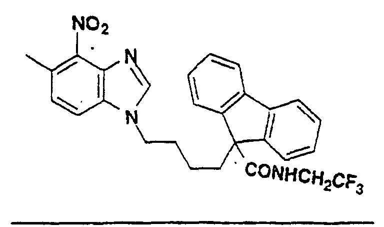

To a stirred slurry of 1.587 g of Part A compound (8.96 mmol) in 15 mL of DMF at room temperature under argon was added 1.50 g (10.9 mmol) of potassium carbonate. After 1 h, 4.26 g (10.0 mmol) of 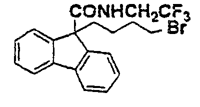 (prepared in Example 273 Part A(2)) was added and the reaction stirred for 20 h. The reaction mixture was quenched with water. The liquids were decanted away from the formed gummy solid, which was then washed with water. The semi-solid residue was dissolved in ethyl acetate, washed twice with water, once with brine and dried (MgSO₄). Two purifications by flash chromatography on silica gel (5x20 cm column, 57:43 ethyl acetate/hexanes) gave 3.05 g of title compound (45%) as a white amorphous solid.

### C.

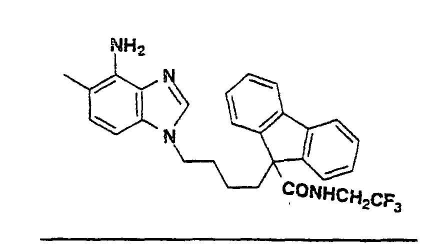

A stirred slurry of 500 mg (0.96 mmol) of Part B compound and 200 mg of 10% palladium-on-charcoal in 20 mL of ethanol was purged with argon and evacuated three times. Hydrogen was introduced to the partially evacuated solution via a bladder. After 14 h, the reaction mixture was purged with argon and passed through a 0.45 µ nylon filter, washing with dichloromethane. The filtrate was evaporated and then re-evaporated twice from dichloromethane to give title compound as a white foam, 0.455 g, 97%.

### D.

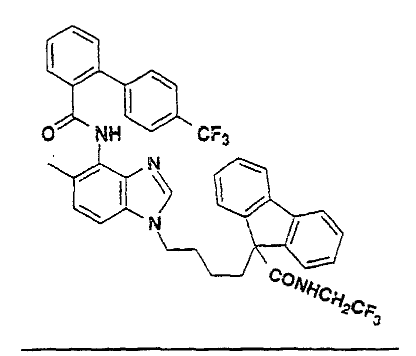

To a solution of 411 mg (0.834 mmol) of Part C compound, 222 mg (0.85 mmol) of 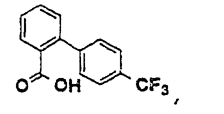 114 mg of HOAt (0.838 mmol) and 58 µL of triethylamine (0.4 mmol) in 4 mL of dichloromethane was added 190 mg (1.0 mmol) of EDAC. After 66 h, the reaction mixture was quenched with saturated sodium bicarbonate solution and extracted twice with dichloromethane. The extracts were combined, dried (MgSO₄) and evaporated. Purification by flash chromatography on silica gel (5x20 cm column, 2 L 1:4 hexanes/ethyl acetate, then 1:5 hexanes/ethyl acetate) gave title compound as a white amorphous solid, 258 mg, 42%.

| | | | |
|---|---|---|---|
| MICROANALYSIS: Calculated for C₄₂H₃₄F₆N₄O₂+0.5 H₂O + 0.5 EtOAc | C, 66.58; | H, 4.95; | N, 7.06 |
| Found | C, 66.63; | H, 4.67; | N, 7.28. |

MS (electrospray, + ions) m/e 741 (M+H).

### Example 413

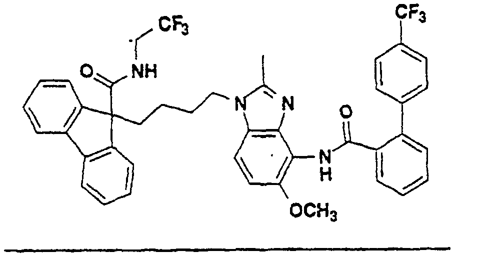

Preparation of compounds of Parts A, B and C were by modifications of the procedures found in.the following references:
1. S. Grivas, W. Tian, E. Ronne, S. Lindstrom and K. Olsson; Acta Cehm. Scand., 47 521 (1993).
2. W. Tian and S. Grivas; Synthesis 29 1305 (1992).
NOTE: The phrase "flash chromatography" refers to chromatography performed on EM Industries Silica Gel 60 (catalog #9385-9), 230-400 mesh under 10-20 psi of nitrogen pressure.

### A.

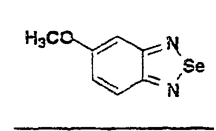

To a stirred solution of 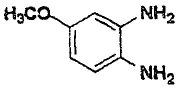 (5.30 g, 25.0 mmol) in 75.0 mL of 1 M HCl at 80°C under argon, was added a solution of selenium dioxide (5.55 g, 50.0 mmol) in 37.5 mL of water dropwise over the course of 0.5 h. Some solid was formed. The reaction was stirred an additional 0.5 h at 80°C and then cooled to 0°C. The resulting solid was collected, washed with water, and dried in vacuum at 50°C. The filtrate was extracted with ethyl acetate (2x80 mL). The combined extracts were washed twice with brine, dried (Na₂SO₄) and evaporated to give additional solid. The solids were combined to provide title compound as a brown solid, 5.09 g (95.5%), mp 108-9°C.

### B.

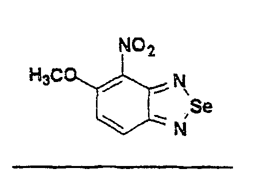

To a stirred solution of Part A compound (4.70 g, 22.1 mmol) in 98% H₂SO₄ (40 mL) at 5°C was added a cold solution of 98% H₂SO₄ (8 mL) and 70% HNO₃ (4 mL), dropwise over 0.5 h. After an additional 1 h at 5°C, the reaction mixture was poured into ice (40 g). Some yellow solid was formed. The solution was neutralized to pH 10-11 by 1 N NaOH, extracted with ethyl acetate, washed twice with brine, dried (Na₂SO₄) and evaporated to give title compound, 5.25 g (92.0%) as a yellow solid (mp 234-5°C).

### C.

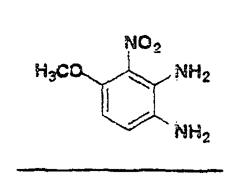

To a stirred solution of Part B compound (5.10 g, 19.8 mmol) in concentrated HCl (60 mL) at room temperature under argon was added a solution of 57% HI (6 mL), dropwise over 15 minutes. After an additional 2 h, a solution of 5% NaHSO₃ (60 mL) was added and the reaction mixture was heated to 80°C for 0.5 h. After cooling,to room temperature, the dark mixture was added to ethyl acetate (200 mL) and stirred for 0.5 h. The mixture was neutralized to pH 9-10 by 4 N NaOH at 5°C and filtered through Celite. The ethyl acetate layer was washed twice with brine, dried (Na₂SO₄) and evaporated to give title compound, 2.07 g (57.1%) as a red solid (mp 114-6°C).

### D.

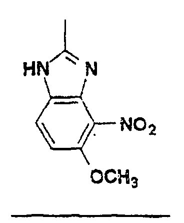

To a stirred refluxing solution of Part C compound (1.00 g, 5.46 mmol) in 5 M HCl (6 mL) and EtOH (40 mL) under argon was added 2,4-pentanedione (1.10 g, 11.0 mmol). After refluxing 0.5 h, the reaction mixture was cooled in an ice bath and neutralized with saturated NaHCO₃ solution. The resulting yellow precipitate was filtered, washed with water and ethyl ether. The resulting solid was then dissolved in hot ethyl acetate, dried (Na₂SO₄) and evaporated to give title compound, 0.827g (73.0%) as a yellow solid (mp 200-1°C).

| | | | |
|---|---|---|---|
| MICROANALYSIS: Calculated for C₉H₉N₃O₃+0.36Et₂O | C, 53.62; | H, 5.43; | N, 17.97 |
| Found | C, 54.04; | H, 5.08; | N, 18.35. |

### E.

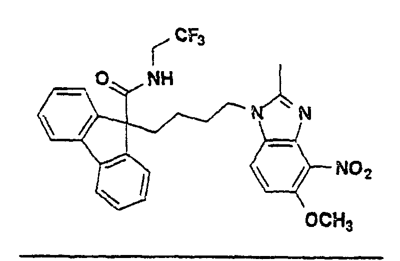

A solution of Part D compound (0.800 g, 3.86 mmol) and K₂CO₃ (0.680 g, 4.94 mmol) in DMF (5 mL) under argon was stirred for 0.5 h at room temperature. To the mixture was added 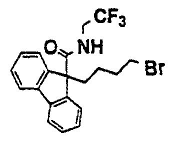 (prepared as in Example 273 Part A(2)) (1.75 g, 4.11 mmol). After 16 h, water (50 mL) was added to the reaction mixture. The resulting yellow precipitate was filtered. The solid was then dissolved in CH₂Cl₂, washed with water, dried (Na₂SO₄) and evaporated. The residue was purified by flash chromatography on silica gel (5x18 cm column, ethyl acetate) to give title compound, 1.42 g (66.6%) as a yellow solid (mp 87-9°C).

| | | | | |
|---|---|---|---|---|
| MICROANALYSIS: Calculated for C₂₉H₂₇F₃N₄O₄+0.25AcOEt | C, 62.71; | H, 5.09; | N, 9.75; | F, 9.92 |
| Found | C, 62.33; | H, 4.86; | N, 9.67; | F, 10.17. |

### F.

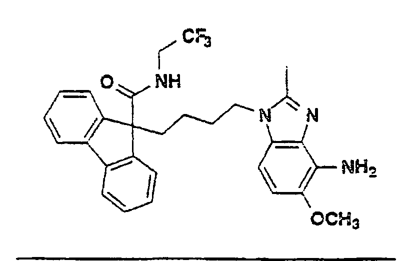

To 10% palladium-on-charcoal (0.230 g, 9.56% mmol) under argon was added EtOH (35 mL) and Part E compound (1.25 g, 2.26 mmol). Hydrogen was introduced to the solution via a bladder at room temperature. After stirring 16 h, the reaction mixture was filtered through Celite and concentrated to give title compound, 1.09 g (92.4%) as a light yellow solid (mp 80-1°C).

| | | | | |
|---|---|---|---|---|
| MICROANALYSIS: Calculated for C₂₉H₂₉F₃N₄O₂+0.55H₂O | C, 65.41; | H, 5.70; | N, 10.52; | F, 10.70 |
| Found | C, 65.12; | H, 5.56; | N, 10.72; | F, 11.15. |

### G.

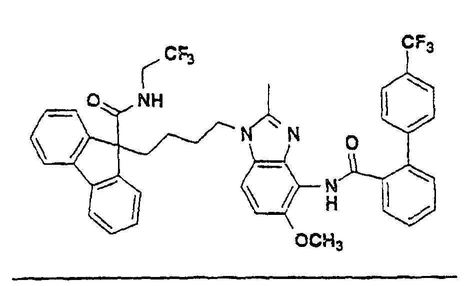

To a solution of Part F compound (0.870 g, 1.58 mmol), 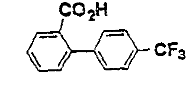 (0.420 g, 1.58 mmol) and HOAt (0.240 g, 1.74 mmol) in CH₂Cl₂ (2 mL) under argon was added EDAC (0.330 g, 1.74 mmol) and Et₃N (0.080 g, 0.790 mmol). After stirring 24 h at room temperature, additional CH₂Cl₂ (1 mL) was added and stirring was continued for an additional 12 h. Saturated NaHCO₃ solution was added to the reaction mixture which was extracted with ethyl acetate, washed with water, dried (Na₂SO₄) and concentrated. The residue was purified by flash chromatography on silica gel (5x18 cm column, ethyl acetate followed by 1:99 methanol/ethyl acetate) to give title compound,
0.512 g (42.0%) as a white amorphous solid (mp 132-4°C).

| | | | | |
|---|---|---|---|---|
| MICROANALYSIS: Calculated for C₄₃H₃₆F₆N₄O₃+0.3 AcOEt+0.5 H₂O | C, 65.85; | H, 4.93; | N, 6.95; | F, 14.14 |
| Found | C, 65.93; | H, 4.69; | N, 6.90; | F, 14.44. |

### Example 414

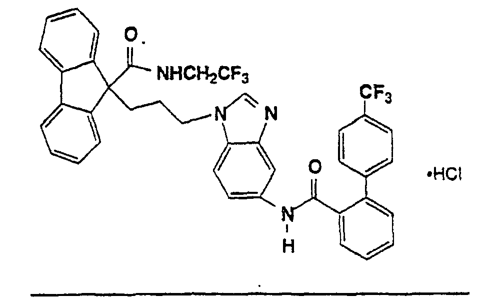

### A.

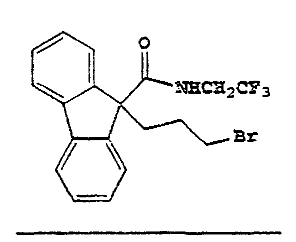

A solution of (9-fluorenecarboxylic acid (20.0 g, 92.3 mmoles) in dry THF (90 ml) was placed under vacuum for 20 minutes to remove dissolved oxygen then cannulated into a cooled (0°C, ice-salt bath) solution of 1.0 M lithium t-butoxide in THF (212 ml, 2.23 eq). The ice-bath was removed and the reaction mixture stirred at room temperature for 1.0 hr. after which the green suspension was treated with 1,3-dibromopropane (18.5 ml., 1.96 eq) via syringe. The dark mixture was stirred at room temperature for 19 hours then partitioned between 30% Heptane in EtOAc (300 ml) and H₂O (250 ml), re-extracting the aqueous phase with H₂O (3 x 70 ml). The combined aqueous extracts were acidified with 2.0 N HCl to pH 2.0, extracted with CH₂Cl₂ (4 x 190 ml) and the combined CH₂Cl₂ extracts were dried (anhydrous MgSO₄), filtered, evaporated to dryness and dried *in vacuo* to give the crude acid as a syrup (32 g).

The acid was dissolved in dry CH₂Cl₂ (190 ml), cooled to 0°C (ice-salt bath), treated with dry DMF (0.32 ml, 0.4 eq) and (COCl)₂ (8.2 ml, 94 mmoles), stirred at 0°C for 5 minutes then at room temperature for 2.0 hours. Meanwhile, trifluoroethylamine hydrochloride (13.8 g, 102 mmoles) was dissolved in dry CH₂Cl₂ (225 ml), cooled to 0°C (ice-salt bath), treated with Et3N (51.5 ml) and stirred for 10 minutes. The acid mixture was cannulated into the amine solution, and stirred at 0°C, allowing the reaction mixture to come to room temperature overnight. The reaction mixture was washed sequentially with H₂O (2 x 190 ml), 1.0 N HCl (320 ml), H₂O (190 ml) and saturated NaHCO₃ (190 ml), dried (anhydrous MgSO₄), filtered, evaporated to dryness and dried *in vacuo*. The crude product mixture was chromatographed on a silica gel column (Merck, 4" x 13"), eluting the column with EtOAc:Hexane (1:4) to give title compound as a solid foam (22 g, 57.8 %). R_{f} 0.38 (Silica gel; EtOAc:Hexane-1:4; UV, PMA); m.p. 106-108°C.

### B.

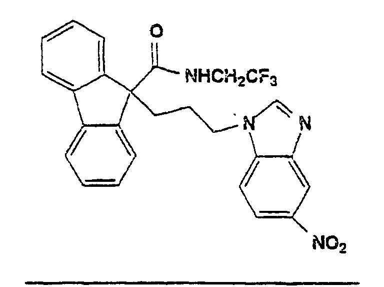

A mixture of Part A compound (2.0 g, 4.85 mmoles), 5-nitrobenzimidazole (870 mg, 5.33 mmoles), and anhydrous K₂CO₃ (737 mg, 5.34 mmoles) in dry DMF (7.0 ml) was stirred at room temperature for 3 days then concentrated *in vacuo*. The residual syrup was partitioned between EtOAc (2 x 50 ml) and H₂O (13 ml), and the combined organic extracts were washed with H₂O (3 x 13 ml) and brine (13 ml), dried (anhydrous Na₂SO₄), filtered, evaporated to dryness and dried *in vacuo.* The crude product mixture was triturated with hot CH₃CN (2 x 25 ml), and filtered while hot to give a white solid (584 mg). The crude filtrate was concentrated to a solid mixture and chromatographed twice on a silica gel column (Merck, 200 g), eluting each column with CH₂Cl₂:EtOAc (3:1-4.0 L) to give diastereomerically enriched title compound (1.197 g, 50.3 %, m.p. 207-8°C ).
TLC : R_{f} 0.37 (Silica gel; EtOAc:CH₂Cl₂-6:4; UV).

### C.

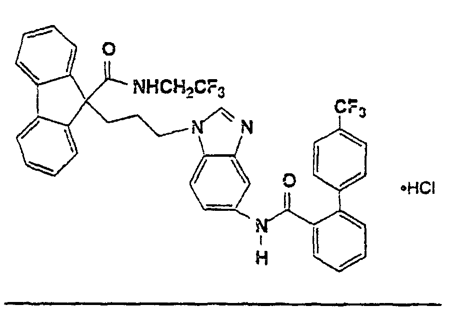

A solution of Part B compound (200 mg, 0.4 mmole) in dry CH₃OH (10 ml) was treated with 10 % Pd/C (40 mg) and hydrogenated (balloon) at room temperature for 20 hours. The reaction mixture was diluted with CH₃OH (10 ml) and filtered through a celite pad in a millipore unit, washing the pad well with CH₃OH (3 x 10 ml). The combined filtrates were evaporated to dryness and dried *in vacuo* to give the crude amine as a syrup (196 mg).

The amine was dissolved in dry CH₂Cl₂ (5.0 ml), treated with the 4'-(trifluoromethyl)-2-biphenylcarboxylic acid (110 mg, 0.42 mmole), FiOBt·H₂O (57 mg, 0.42 mmole) and EDAC (88 mg, 0.46 mmole) and stirred at room temperature for 20 hours. The reaction mixture was partitioned between EtOAc (2 x 15 ml) and saturated NaHCO₃ (3.0 ml) and the combined organic extracts were washed with H₂O (3 x 3.0 ml) and brine (3.0 ml), dried (anhydrous Na₂SO₄), filtered, evaporated to dryness and dried *in vacuo*. The crude product mixture was chromatographed on a silica gel column (Merck, 70 g), eluting the column with EtOAc:Hexane (1:2), EtOAc and CH₂Cl₂:MeOH (100:3) to give the clean free base (207 mg).

This adduct (207 mg) was dissolved in dry dioxane (2.6 ml), treated with 4.0 M HCl/dioxane (0.21 ml, 2.83 eq), swirled for a few minutes then diluted with dry Et₂O (35 ml), scratching the solids as they formed. The supernatant was decanted and the solids washed with dry Et₂O (2 x 15 ml) to give title compound as a solid (163.8 mg, 53.6 %; m.p. 155-165°C, shrinking commencing at 150°C)).

| | | | |
|---|---|---|---|
| Anal. Calc'd for C₄₀H₃₀F₆N₄O₂•HCl•0.8 H₂O (Eff. Mol. Wt.=763.57) | C, 62.92; | H, 4.30; | N, 7.34; |
| Found | C, 62.93; | H, 4.37; | N, 7.11. |

### Example 415

### N-(2,2,2-Trifluoroethyl)-9-[3-[[2-[[[4'-(3,3,3-trifluoromethyl)[1,1'-biphenyl]-2-yl]carbonyl]amino]-5-pyridinyl]amino]propyl]-9H-fluorene-9-carboxamide, monohydrochloride.

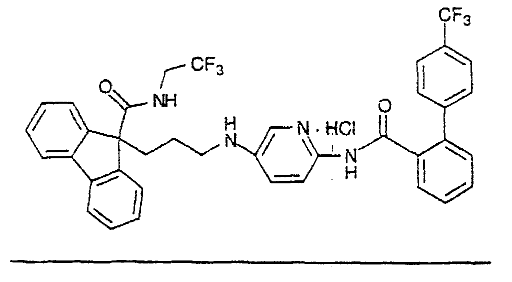

### A.

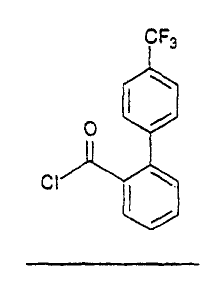

To a stirred solution of 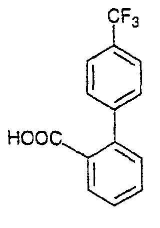 (5.32 g., 20 mmol) in 40 mL of dry CH₂Cl₂ and 40 mL of DMF at room temperature under nitrogen was slowly added 15.0 mL of 2 M oxalyl chloride in CH₂Cl₂ (30 mmol). The reaction was stirred at room temperature for 2 h and concentrated to an oil, which was dried in vacuo for 2 h and then stored at - 40°C overnight to give crude title compound as an amorphous solid.

### B.

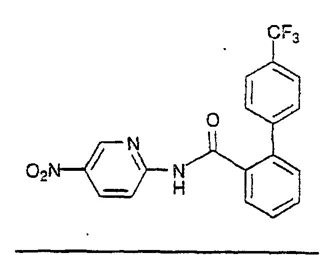

A mixture of 3.41 g (12 mmol) of Part A compound, 1.25 g (9 mmol) of 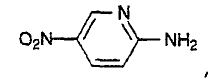 and 2.9 mL (36 mmol) of dry pyridine in 15 mL of dry THF was stirred at room temperature under argon for 20 h and filtered. Evaporation of the filtrate gave a residue which was taken up in CH₂Cl₂, water, and 10% Na₂CO₃. The CH₂Cl₂ was washed with dilute Na₂CO₃⁻ (2x) and water (2x), dried (Na₂SO₄), and concentrated to a yellow gummy residue (4.72 g). Chromatography of this residue over 450 g of silica gel using CHCl₃, concentration, and then concentration from EtOAc afforded 2.63 g (57%) of title compound as a white solid.

### C.

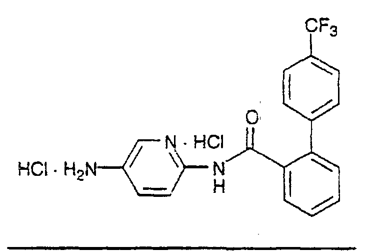

Part B compound (2.45 g, 6.33 mmol) was hydrogenated at 1 atmosphere with 350 mg of 10% Pd/C in 60 mL of glacial AcOH for 1.5 h. Concentrated HCl (1.1 mL, 13 mmol) was added, the mixture was filtered, and the filtrate was concentrated to a residual oil. Concentration of the oil from 95% EtOH and trituration of the oily residue from Et₂O gave 2.41 g (89%) of title compound as a solid.

### D.

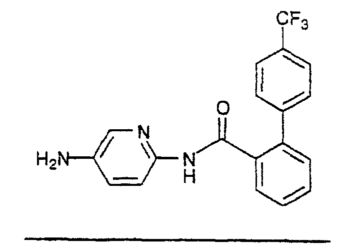

Part C compound (430 mg, 1 mmol) was shaken with CH₂Cl₂ and 5% NaHCO₃. The CH₂Cl₂ extract was washed with 5% NaHCO₃ (2x) and then water (2x), dried (Na₂SO₄), and concentrated to give 342 mg (96%) of title compound as a yellow foam.

### D(1).

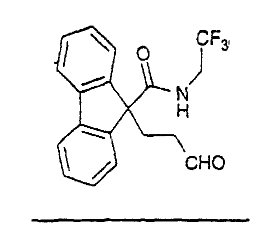

The Part D(1) compound is prepared as described in Example 296 Part A.

### E.

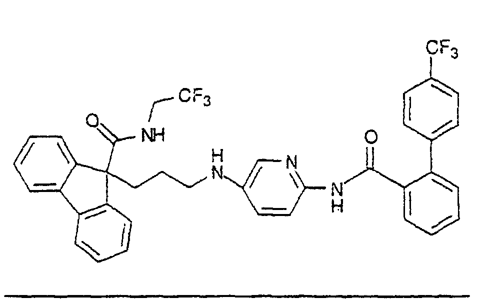

A mixture of Part D compound (342 mg, 0.96 mml), Part D(1) compound (335 mg, 0.96 mmol), glacial AcOH (0.33 mL, 5.8 mmol) and NaBH(OAc)₃ (610 mg, 2.88 mmol) in 6 mL of 1,2-dichloroethane was stirred at room temperature under argon for 17 h. The mixture was diluted with CH₂Cl₂ and the organics were washed with 5% NaHCO₃ (3x) and then water (2x), dried (Na₂SO₄) and concentrated to a foamy residue (772 mg). Chromatography of this residue over 70 g of silica gel packed in CH₂Cl₂-EtOAc (85:15) by eluting with this solvent and then CH₂Cl₂-EtOAc (80:20) afforded 329 mg (50%) of title compound as a residue. F.

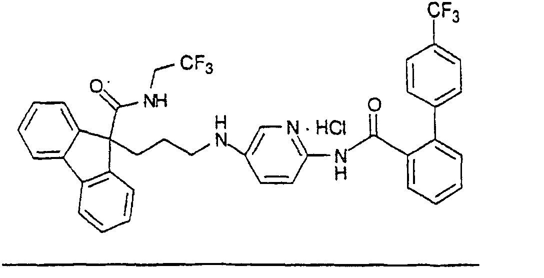

To a solution of Part E compound (320 mg, 0.46 mmol) in 4 mL of dry THF was added 0.5 mL of 4 N HCl in dioxane and then Et₂O. The precipitate was collected, washed with Et₂O, and dried in vacuo at 40°C for 1 h to give 251 mg (75%) of title compound as a pale yellow solid having mp 128-132°C.

| | | | | | |
|---|---|---|---|---|---|
| Anal. Calcd for C₃₈H₃₀F₆N₄O₂ + HCl+0.75 H₂O+0.15 Et₂O | C, 61.84; | H, 4.57; | N, 7.47; | Cl, 4.73; | F, 15.20 |
| Found | C, 61.91; | H, 4.41; | N, 7.40; | Cl, 4.81; | F, 15.48. |

MS (ESI-NH₃, + ions) 689 (M+H); (- ions) 687 (M-H). TLC (silica gel): Rf=0.50, CH₂Cl₂: CH₃OH (19:1).

### Example 416

### N-(2,2,2-Trifluoroethyl)-9-[3-[5-[[[4'-(trifluoromethyl)[1,1'-biphenyl]-2-yl]carbonyl]amino]-1,3-dioxan-2-yl]propyl]-9H-fluorene-9-carboxamide

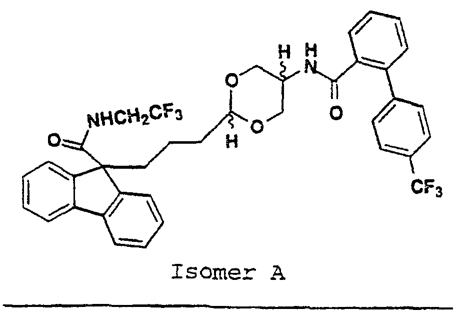

### Example 416A

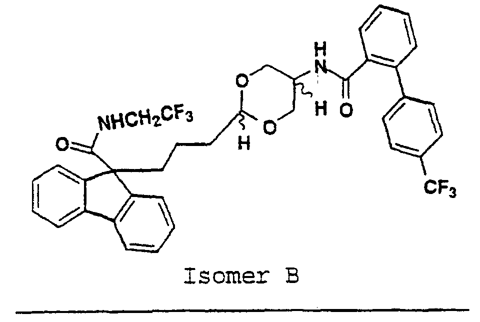

NOTE: The phrase "flash chromatography" refers to chromatography performed on EM Industries Silica Gel 60, 230-400 mesh under 10-20 psi of nitrogen pressure.

### A.

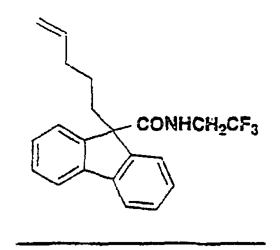

A solution of 9H-fluorene carboxylic acid (5.00 g, 23.7 mmol) in 24 mL of THF at -12°C was purged and evacuated with argon three times. The solution was added via canula to an argon-purged solution of 50 mL of lithium t-butoxide (1 M in THF, 50.0 mmol) at -12°C over 5 min. After 1 h, the solution was warmed to room temperature and Br(CH₂)₃CH=CH₂ (5.6 mL, 48 mmol) was added in a steady stream. After 70 h, the reaction was quenched with 1 M hydrochloric acid and extracted twice with ethyl acetate. The organic extracts were combined, dried (MgSO₄) and evaporated.

The white solid was stirred and slurried in 25 mL of dichloromethane at room temperature while oxalyl chloride (3.5 mL, 40 mmol) and DMF (0.2 mL) were added. After 1 h, the yellow solution was evaporated twice from dichloromethane and redissolved in 20 mL of dichloromethane. This solution was added to a stirred solution of 1,1,1-trifluoroethylammonium chloride (4.10 g, 30.0 mmol) and Et₃N (12.5 mL, 89.7 mmol) in 30 mL of dichloromethane at 0°C under argon. After 1 h, the reaction was quenched with 10% citric acid solution. The organic extract was dried (MgSO₄) and evaporated. Purification by flash Chromatography on silica gel (5x20 cm column, 1:1 hexane/dichlo-romethane) gave, after trituration in hexane, title compound, 5.40 g, 63% yield, as a white solid, mp 47-49°C.

### B.

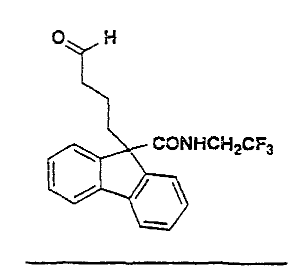

A solution of Part A compound (3.59 g, 10.0 mmol) in 100 mL of dichloromethane, protected by a Drierite-filled tube, at -78°C was treated with a stream of ozone/oxygen generated from a Welsbach Ozonizer for 20 min until a blue color persisted. Solid triphenylphosphine (2.70 g, 10.1 mmol) was added and the reaction was warmed to room temperature. After 24 h, the reaction mixture was partially evaporated and purified by flash chromatography on silica gel (5 x 20 cm column, 3:197 ether/dichloromethane) to give title compound as a low-melting solid, 3.40 g, 94%. C.

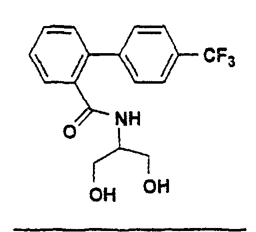

To a stirred solution of 1.33 g (5.00 mmol) of 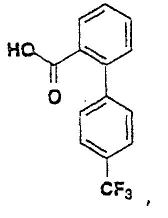 0.455 g (5.00 mmol) of 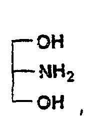 0.750 g (5.0 mmol) of HOBt and 0.5 mL (3.6 mmol) of triethylamine in 10 mL of dichloromethane at room temperature under argon, was added 1.0 g (5.25 mmol) of EDAC, portion-wise, over 3 min. After 16 h, the reaction mixture was diluted with ethyl acetate, washed once with saturated sodium bicarbonate solution, once with brine and once with 10% citric acid solution, dried (MgSO₄) and evaporated. Purification by flash chromatography on silica gel (5 x 15 cm column, ethyl acetate) provided title compound as a white solid, mp 146-148°C, 1.23 g, 72% yield.

### D.

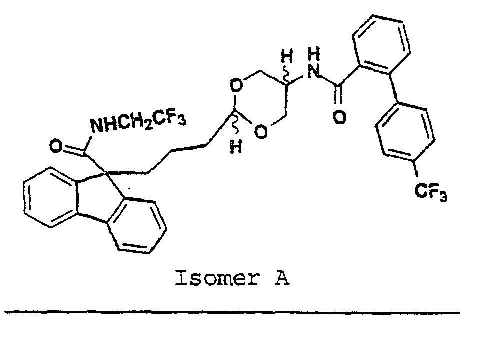

### E.

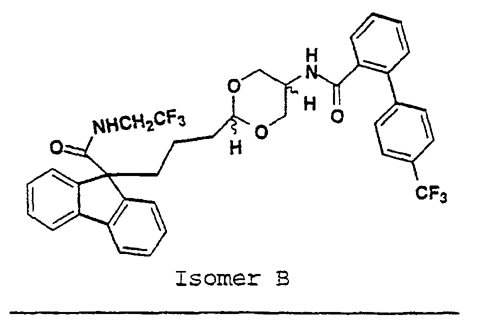

To a stirred slurry of Part C compound (340 mg, 1.00 mmol) and Part B compound (362 mg, 1.00 mmol) in 2 mL of dichloromethane at room temperature under argon was added 98% methanesulfonic acid (10 µL, 0.15 mmol). After 14 h, the resulting colorless solution was quenched with saturated sodium bicarbonate solution and extracted twice with dichloromethane. The organic extracts were combined, dried (Na₂SO₄) and evaporated. The oily residue was partially purified by flash chromatography on silica gel (5 x 25 cm column, 1:1 EtOAc/hexanes) to give two fractions:
**Isomer A** (Example 416)
   80 mg, 12% yield.
   TLC: R_{f} = 0.46 (3:2 EtOAc/hexane on Silica Gel 60).
   Melting point: 210-212°C.
**Isomer B** (Example 416A)
   420 mg, 62% yield.
   TLC: R_{f} = 0.37 (3:2 EtOAc/hexane on Silica Gel 60).
   Melting point: 85-88°C.
   Mass Spectrometry: (electrospray, + ions) m/z 700 (M+NH₄⁺), 683 (M+H).

| | | | | |
|---|---|---|---|---|
| MICROAnal. Calcd for C₃₇H₃₃F₆N₂O₅P | C, 65.10;. | H, 4.73; | N, 4.10; | F, 16.70 |
| Found | C, 65.19; | H, 4.91; | N, 3.86; | F, 16.52. |

### Example 417

### N-(2,2,2-Trifluoroethyl)-9-[3-[[5-[[[4'-(trifluoromethyl)[1,1'-biphenyl]-2-yl]carbonyl]amino]-2-pyridinyl]oxy]propyl]-9H-fluorene-9-carboxamide, trifluoroacetate.

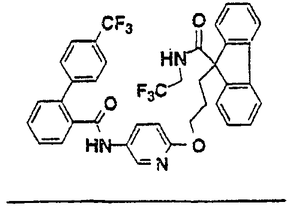

### A.

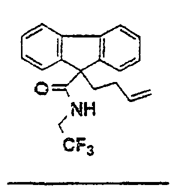

To a solution of the 9H-fluorene carboxylic acid (8.0 g, 38 mmol) in THF at 0°C (150 ml) was added a 1 M solution of lithium tert-butoxide (76 ml, 76 mmol) in THF. Following the addition of base, the reaction mixture was stirred vigorously at RT for 2h. The reaction mixture was treated with 1-bromo-3-butene (8.00 g, 60 mmol) and stirred overnight. TLC indicated a trace of starting acid was still present. The reaction mixture was treated with an additional 5 mL (5 mmol) of lithium tert-butoxide and the mixture stirred overnight. The mixture was quenched with NH₄Cl solution and the pH adjusted to 2 with KHSO₄ solution. The mixture was diluted with ethyl acetate (400 mL) and washed with water. The organic layer was dried (MgSO₄), and the solvent was removed *in vacuo* to give an off-white foam which was partially purified by trituration with hexane to give a white solid (9.5 g) of the structure

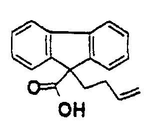

To a solution of the above crude acid (9.5 g, 36 mmol) in dichloromethane (200 mL) was added a 2 M solution of oxalyl chloride (23 ml, 46 mmol) in dichloromethane followed by a 2 drops of DMF. The reaction (bubbled vigorously) was stirred under argon at RT for 2 h. The solvent was evaporated in vacuo and the residue was dissolved in THF (150 ml). The mixture was treated with CF₃CH₂NH₂ HCl salt (5.4 g, 40 mmol) and triethylamine (8.00 g, 78 mmol) and stirred at RT for 6 h. The reaction mass was diluted with ethyl acetate (300 mL) and washed 1N HCl and saturated K₂CO₃ solution. The organic layer was dried (MgSO₄), and the solvent was removed in vacuo to give an off-white solid which was purified by recrystalization from methanol to give 4.5 g of title compound as a white solid. The filtrate was concentrated and the residue purified by flash column chromatography to give an additional 3.5 g of title compound as a white solid (overall yield 8.0 g. 64%).

### B.

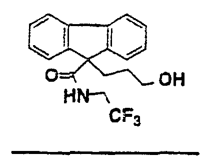

A solution of Part A compound (3.00 g, 8.7 mmol) in a mixture of 50 mL 1:1 dichloromethane/methanol at -78° C was treated with a stream of ozone in oxygen for 35 min. The mixture turned light gray and TLC indicated that the starting olefin was consumed. The reaction mixture was treated with NaBH₄ pellets (1.03 g, 27 mmol) and stirred overnight at RT. The mixture was quenched with 50 mL of NH₄Cl solution and 150 mL ethyl acetate. The layers were equilibrated and separated. The organic fraction was dried (MgSO₄) and concentrated. The residue was purified by flash column chromatography on silica gel with 1:1 ethyl acetate/hexanes to give 2.6 g (85%) of title compound as a white solid.
mp: 112-114°C

### C.

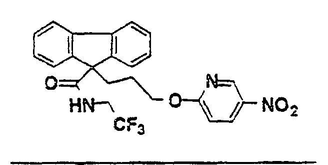

A solution of Part B compound (2.50 g, 7.16 mmol) in THF was treated with NaH (192 mg, 8 mmol) at 0°C. After 1 h the alkoxide was treated with 1.30 g (8 mmol) of 2-bromo-5-nitropyridine. The mixture was stirred at RT overnight and an additional 36 mg (1.5 mmol) of NaH was added. After stirring for an additional 4 hours the reaction mixture was quenched with NaHCO₃ solution and extracted with ethyl acetate. The organic fraction was dried (MgSO₄) and concentrated. The residue was purified by flash column chromatography on silica gel with 6:12:1 ethyl acetate/hexanes/dichloromethane to give 3.12 g (92%) of title compound as a white solid.

### D.

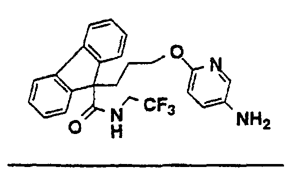

A solution of Part C compound (3.00 g, 6.4 mmol) in ethyl acetate (50 mL) was treated with 200 mg of 10% Pd/carbon and placed under an atmosphere of H₂ (balloon pressure). After stirring overnight the mixture was filtered through a pad of celite and the filtrate concentrated to title compound in the form of a thick oil (3.00 g, ≈ 100%).

### E.

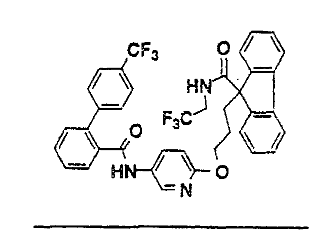

The crude Part D amine (3.0 g, 6.3 mmol) was stripped from toluene (2 X 20 mL) and pumped to ensure complete drying. The amine was diluted with 100 mL of THF and cooled to 0°C. The solution was treated with the Example 415 Part A acid chloride (1.75 g, 6.1 mmol) in 10 mL of dichloromethane. The mixture was then treated with triethylamine (0.64 g, 6.3 mmol) and a slurry resulted. The thick mixture was stirred for 1 hour at RT and diluted with 50 mL NaHCO₃ solution and 100 mL of ethyl acetate. The layers were equilibrated and separated. The organic fraction was dried (MgSO₄), concentrated and purified by flash column chromatography on silica gel with 3:7 ethyl acetate/hexanes followed by 1:1 ethyl acetate/hexanes to give 4.00 g (92%) of title compound as an off white solid.
mp: 115-120°C
TLC Silica gel (3:7 ethyl acetate/hexane) R_{f}=0.50.
Mass Spec. (ES-NH₃, + ions) m/z 690 (M+H).

| | | | | |
|---|---|---|---|---|
| Anal. Calc'd for C₃₈H29N₃O₃F₆ + 0.5 H₂O + HCl | C, 61.34; | H, 4.33; | N, 5.65; | Cl, 4.76 |
| Found | C, 60.90; | H, 4.30; | N, 5.36; | Cl, 4.97. |

### Example 418

### N-(2,2,2-Trifluoroethyl)-9-[4-[4-[[[4'-(trifluoromethyl)[1,1'-biphenyl]-2-yl]carbonyl]amino]-1H-indol-1-yl]butyl]-9H-fluorene-9-carboxamide.

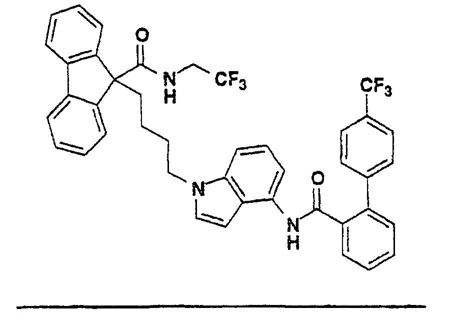

### A.

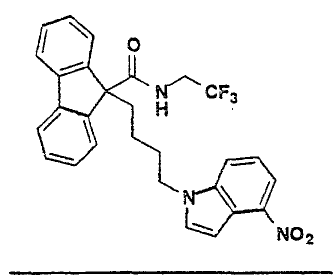

A solution of 4-nitroindole (4.0 g, 24.7 mmol) in DMF (20 mL) was added slowly over 5 min to a suspension of unwashed sodium hydride (1.09 g, 60 wt.% in mineral oil, 27.2 mmol) in DMF (50 mL) at 0°C. An immediate color change to deep red occurred with bubbling of escaping gasses. The reaction mixture was stirred at 0°C for 5 min and then at RT for 40 min. A solution of Example 273 Part A(2) compound (12.6 g, 29.6 mmol) in DMF (20 mL) was added and the reaction mixture was stirred at RT over a weekend (64 h total). The solvent was removed under high vacuum on a rotary evaporator, and the resulting orange residue was partitioned between EtOAc (200 mL) and H₂O (50 mL). The organic layer was washed with H₂O (2 x 50 mL) and brine (50 mL), dried over MgSO₄, and concentrated to give a yellow foam. The crude product was purified by flash chromatography on silica gel (600 g) eluting with a step gradient of 20% to 25% to 30% EtOAc/hexane to give title compound (10.9 g, 73%) as a yellow foam.

### B.

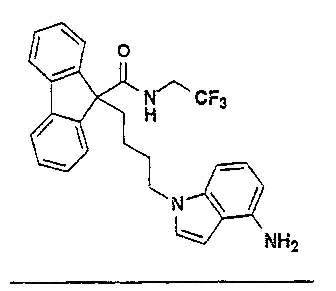

A mixture of Part A compound (7.47 g, 14.7 mmol) and 10% palladium on carbon (780 mg, 0.737 mmol) in EtOAc (50 mL) was hydrogenated under a balloon of H₂ at RT for 5 h, filtered through Celite®, and washed with EtOAc (2 x 50 mL). The filtrate was concentrated and dried under high vacuum to give title compound (7.12 g, 100%) as a white foam.

### C.

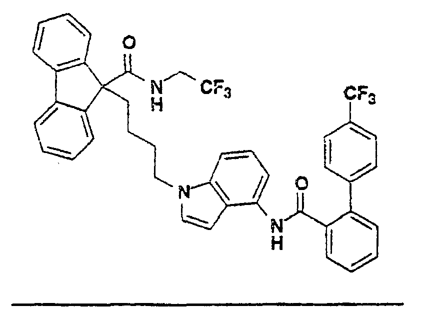

To a solution of Part B compound (5.2 g, 10.9 mmol) and triethylamine (2.0 mL, 14.2 mmol) in CH₂Cl₂ (30 mL) at 0°C was added Example 415 Part A compound (12 mL, 1.0M in CH₂Cl₂, 12.0 mmol) over 5 min. The cloudy reaction mixture was stirred at 0°C for 10 min, diluted with EtOAc (200 mL), washed with saturated NaHCO₃ (2 x 50 mL) and brine (50 mL), dried over MgSO₄, and concentrated to give a golden foam. The crude product was dissolved in a minimal amount of CH₂Cl₂ and then purified by flash chromatography on silica gel (400 g) eluting with a step gradient of 30% to 40% EtOAc/hexane to give title compound (7.74 g, 89%) as a pale yellow foam. NMR shows product to contain EtOAc.

| | | | | |
|---|---|---|---|---|
| Anal. Calcd for C₄₂H₃₃F₆N₃O₂ + 0.5 C₄H₈O₂ | C, 68.55; | H, 4.84; | N, 5.46; | F, 14.81 |
| Found | C, 68.38; | H, 4.55; | N, 5.44; | F, 14.82. |

### Example 419

### N-(2,2,2-Trifluoroethyl)-9-[3-[[2-[[[4'-(trifluoromethyl)[1,1'-biphenyl]-2-yl]carbonyl]amino]-5-pyridinyl]oxy]propyl]-9H-fluorene-9-carboxamide

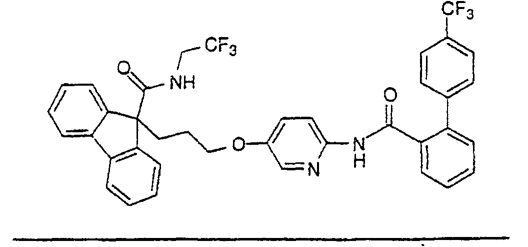

### A.

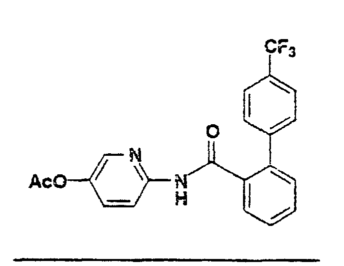

Sodium nitrite (587 mg, 8.5 mmol) was added in portions to a stirred solution of 2.02 g (5.66 mmol) of Example 415 Part D compound in 40 mL of glacial AcOH at room temperature under N₂. The reaction was stirred at room temperature for 45 minutes, then 408 mg (6.8 mmol) of urea was added to destroy excess HONO and stirring was continued for 2 hours. The reaction was gradually heated to 90°C (N₂ evolution) and then 115°C, over the course of 3 hours, and then cooled to room temperature. The solvent was removed in vacuo and the residue was taken up in CH₂Cl₂ and dilute NaHCO₃. The CH₂Cl₂ was washed with dilute NaHCO₃ (2x) and water (2x), dried (Na₂SO₄), and concentrated to an oily residue (2.29 g). Flash chromatography over 200 g of silica gel packed in CHCl₃ by eluting with title compound (fraction A, 265 mg and fraction B, 763 mg), which was used without further purification.

### B.

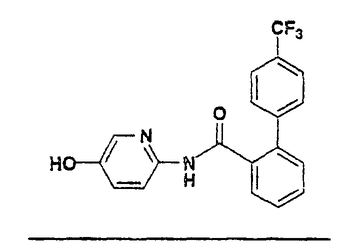

A solution of Part A compound (763 mg) in 10 mL of CH₃OH and 6 mL of 2N KOH was stirred at room temperature for 20 hours and concentrated to a residue, which was taken up in Et₂O and water and extracted twice with Et₂O. The aqueous phase was layered with Et₂O and adjusted to pH 5.2 with dilute HCl. After two extractions with Et₂O, the acidic Et₂O extract was dried (Na₂SO₄) and concentrated to a residue. Crystallization of this residue from CH₂Cl₂ gave 439 mg of title compound. Similar treatment of the above 265 mg fraction of Part A compound provided an additional 87 mg of title compound for a total of 526 mg ( 26%, 2 steps) of title compound.

### C.

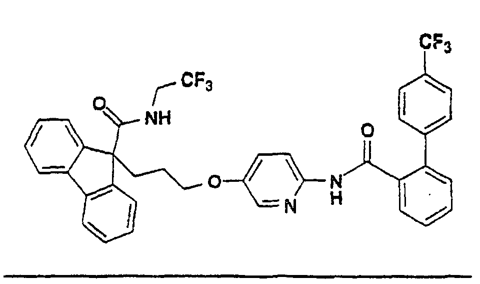

50 mg (0.143 mmol) of Example 417 Part B compound, 64 mg (0.179 mmol) of Part B compound and 41 mg of triphenylphosphine were azeotropically evaporated with toluene (3X), then dried in vacuo for 2 hours before dissolved in 0.5 mL of freshly distilled THF. To above solution cooled at 0°C was added dropwise diethylazodicarboxylate (24.8 µL, 0.257 mmol), and the resulting mixture was stirred at room temperature under argon for 18 hours, then diluted with EtOAc, washed with water, brine, dried over MgSO₄, The filtrate was concentrated, absorbed on Celite, flash chromatographed eluting with 20-30% EtOAc/hexane to give 76.4 mg of the product as an oily residue, Further purication using preparative HPLC, after lyophilization afforded 56.5 mg (57% yield) of the pure title product as a white powder.

| | | | | |
|---|---|---|---|---|
| MICROANALYSIS: Calculated for C₃₈H₂₉N₃F₆O₃ + 0.60 H₂O | C, 65.16; | H, 4.35; | N, 6.00; | F, 16.27 |
| Found | C, 64.86; | H, 4.04; | N, 5.77; | F, 16.59. |

MS: (electrospray, + ions) m/e @ 690 (M+H).

### Example 420

### 9-[3-[[3-Methyl-5-[[[4'-(trifluoromethyl)[1,1'-biphenyl]-2-yl]carbonyl]amino]-2-pyridinyl]oxy]propyl]-N-(2,2,2-trifluoroethyl)-9H-fluorene-9-carboxamide, monohydrochloride.

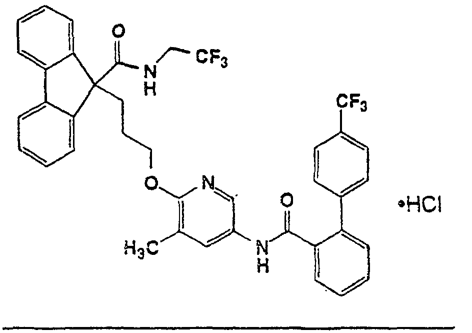

### A.

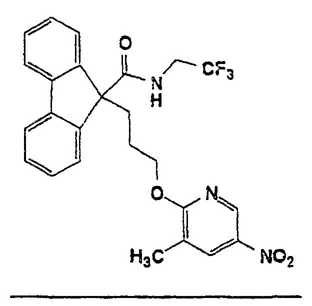

A solution of Example 417 Part B compound (1.25 g, 3.58 mmol) in THF (5 mL) was treated with NaH (173 mg, 60% mineral oil dispersion, 4.3 mmol) and stirred for 15 min at RT. After all the gray solid was consumed, 2-chloro-3-methyl-5-nitropyridine (742 mg, 4.3 mmol) was added to the reaction mixture. The resulting black mixture was stirred at RT for 18 h. Additional 2-chloro-3-methyl-5-nitropyridine (74 mg, 0.43 mmol) was added and stirring was continued for 6 h longer. The mixture was diluted with 5% aq. NaHCO3 (10 mL) and extracted with EtOAc (3 x 50 mL). The combined organic extracts were washed with H₂O (10 mL) and brine (10 mL), dried over Na₂SO₄ and concentrated to give a foam. Flash chromatography on Merck silica gel K-60 (50 g) eluting with EtOAc/hexane (0.5:9.5 to 1:4) to give title compound (1.53 g, 90%) as a solid, m.p. 102-104°C.

### B.

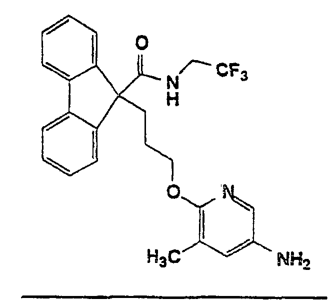

A mixture of Part A compound (250 mg, 0.51 mmol) and 10% palladium on carbon (15 mg) in ethyl acetate (5 mL) was hydrogenated (balloon pressure) at RT for 24 h. The catalyst was removed by filtration through nylon 66 filter, and concentrated in vacuo to give crude title amine (240 mg, quantitative) as an oil.

### C.

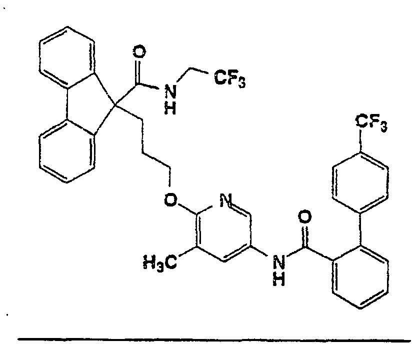

To a solution of crude Part B compound (240 mg, 0.50 mmol) and triethylamine (221 µl, 1.5 mmol) in CH₂Cl₂ (5 mL) at 0°C was added dropwise 540 µl (0.54 mmol) of 1.0 M 4'-(trifluoromethyl)-2-biphenyl carboxylic acid chloride (Example 415 Part A) solution in CH₂Cl₂. The reaction was stirred at 0°C for 1 h. Dichloromethane (20 mL) was added and the solution was washed with sat. NaHCO₃ solution (2 x 10 mL), then dried over Na₂SO₄ and concentrated to give an oil. Purification by flash chromatography on Merck silica gel K-60 (20 g) eluting with CH₂Cl₂/MeOH (10:0 to 9.8:0.2) to give 300 mg of title compound as a free base. To the stirred solution of free base title compound (281 mg, 0.4 mmol) in THF was added 4N HCl in dioxane (415 µl, 1.6 mmol). After stirring for 3 min, the clear solution was diluted with Et₂O (50 mL). The separated solid was collected and dried in vacuo (0.5 mm) at RT for 2 h to give title compound (260 mg, 90%) as off white solid.
MS (ESI, + ions) m/z 704 (M + H).

### Example 421

### 9-[3-[[3-(Dimethylamino)-5-[[[4'-(trifluoromethyl)[1,1'-biphenyl]-2-yl]carbonyl]amino]-2-pyridinyl]oxy]propyl]-N-(2,2,2-trifluoroethyl)-9H-fluorene-9-carboxamide.

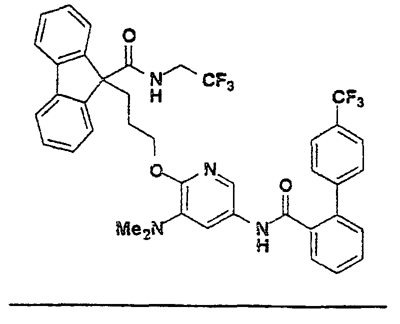

For compounds of Part A(1) and Part A(2), the procedure described in J. Med. Chem. **1992** 35, 1895, was followed.

### A.

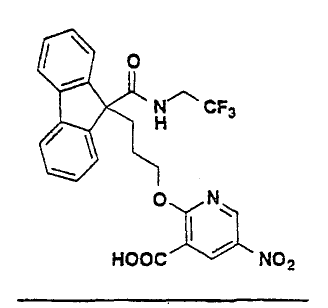

### A(1).

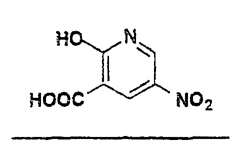

Fuming nitric acid (10 mL, 240 mmol) was added to a suspension of 2-hydroxynicotinic acid (13.9 g, 100 mmol) in concentrated sulfuric acid (40 mL) and the reaction mixture was heated gradually to 50°C, at which point all solids had dissolved. After 5 min at 50°C, the reaction mixture began to exotherm violently, whereupon the heating bath was removed. The reaction mixture turned dark red and emitted red fumes, and within a few minutes, began to cool down. Once at RT (HPLC indicated complete reaction), the yellow solution was poured into ice water (600 mL), and the resulting solid was filtered, washed with ice water (2 x 100 mL), and air-dried for 1 h to give 12.1 g of a yellow solid. The crude product was recrystallized from H₂O (200 mL) and then dried in a vacuum oven at 90 °C to give title compound (10.4 g, 57%) as a yellow solid (mp 238.5-240.5°C, lit mp 240°C).

### A(2).

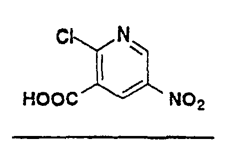

A suspension of Part A(l) compound (7.0 g, 38 mmol) in phosphorus oxychloride (20 mL) was heated at reflux for 2 h, cooled to RT, and added slowly to H₂O (100 mL) with stirring, maintaining the temperature below 40'C with added ice. Following addition, the mixture was stirred at RT for 30 min, whereupon a precipitate formed. The mixture was extracted with Et₂O/THF (2:1, 2 x 200 mL), and the combined organic extracts were washed with brine (100 mL), dried over Na₂SO₄, and concentrated to give an oily yellow solid. The crude product was taken up in hot Et₂O/hexane (1:1, 200 mL), filtered, and the filtrate was concentrated to give title compound (5.78 g, 75%) as a yellow solid (mp 140-141°C, lit mp 142-143°C).

### A(3).

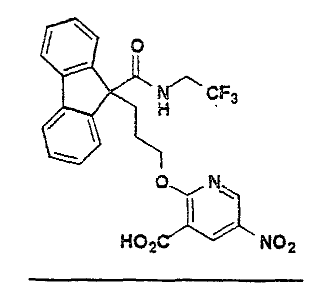

Sodium hydride (124 mg, 60 wt% in mineral oil, 3.09 mmol) was added all at once to a solution of Example 417 Part B compound (430 mg, 1.23 mmol) in DMF (2 mL). After evolution of gasses, the reaction mixture was stirred for 30 min at RT, followed by addition of Part A(2) compound (208 mg, 1.03 mmol) all at once. Bubbling ensued and the reaction mixture was stirred at RT for 30 min, diluted with H₂O, and then acidified with 1N HCl (3 mL). The solid mass that formed was extracted with EtOAc (20 mL), washed with a large amount of brine, dried over Na₂SO₄, and concentrated to give 750 mg crude title carboxylic acid as a yellow oil.

### B.

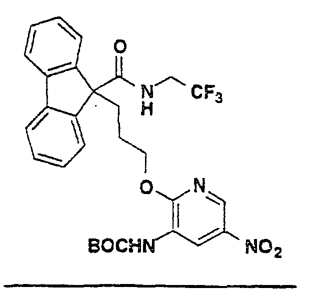

Diphenylphosphoryl azide (477 µL, 2.22 mmol) was added to a solution of Part A compound (955 mg, 1.85 mmol) and triethylamine (385 µL, 2.78 mmol) in freshly distilled *tert*-butanol. The reaction mixture was heated at 80°C for 2 h, cooled to RT, and concentrated to give an orange oil. The oil was dissolved in EtOAc (25 mL), washed with saturated NaHCO₃ (2 x 5 mL), H₂O (5 mL), and brine (5 mL), dried over MgSO₄, and concentrated to give 1.33 g of an orange thick oil. The crude product was purified by flash chromatography on silica gel (100 g) eluting with a step gradient of 15% to 20% EtOAc/hexane to give title compound (355 mg, 33%) as a yellow foam.

### C.

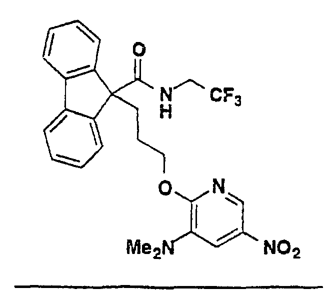

A solution of Part B compound (343 mg, 0.585 mmol) in 4N HCl/dioxane (3 mL) was allowed to stand at RT for 5 h, then concentrated to give the crude amine. To a mixture of the crude free amine, formalin (950 µL, 37%, 11.7 mmol), and AcOH (1 mL, 17.6 mmol) in MeOH (3 mL) was added sodium cyanoborohydride (370 mg, 5.85 mmol) all at once. The reaction mixture was stirred at RT overnight, concentrated, and azeotroped with toluene (15 mL). The residue was dissolved in EtOAc (50 mL), washed with saturated NaHCO₃ (2 x 10 mL) and brine (10 mL), dried over MgSO₄, and concentrated to give 400 mg of an orange oil. The crude product was purified by flash chromatography on silica gel (50 g) eluting with 15% EtOAc/hexane to give title compound (230 mg, 76%) as a yellow glass.

### D.

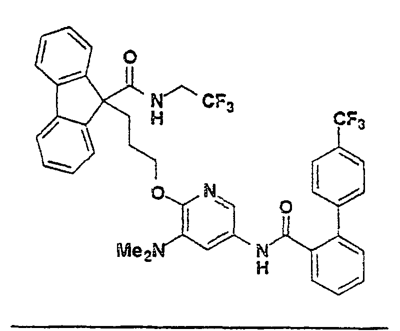

Following the procedure in Example 418 Part C compound(230 mg, 0.447 mmol) was hydrogenated and then acylated with Example 415 Part A compound to give title compound (234 mg, 72%) as a white foam.
MS (ES, + ions) m/z 733 [M+H].

| | | | | |
|---|---|---|---|---|
| Anal. Calcd for C₄₀H₃₄F₆N₄O₃ + 0.5 H₂O | C, 64.77; | H, 4.76; | N, 7.55; | F, 15.37 |
| Found | C, 64.70; | H, 4.60; | N, 7.28; | F, 15.16. |

### Example 422

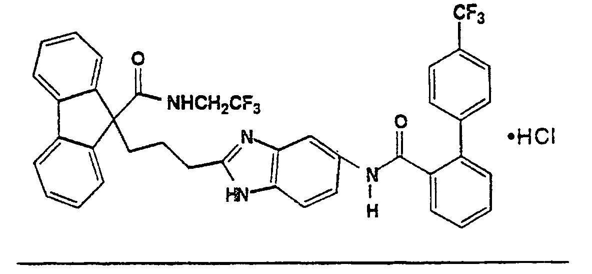

### A.

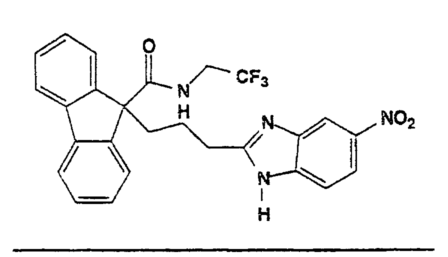

A mixture of Example 416 Part B compound (400 mg, 1.11 mmoles), 5-nitrophenyldiamine (173 mg, 1.11 mmoles) and 2,3-dichloro-5,6-dicyano-1,4-benzoquinone (DDQ) (256.3 mg, 1.11 mmoles) in dry CH3CN (5.0 ml) was stirred at room temperature for 25 hours and stripped to dryness. The crude mixture chromatographed on a silica gel column (Merck), eluting the column with CH₂Cl₂:EtOAc (3:1) to give title compound as a light brick-red solid foam (313 mg, 57.1 %).
TLC : R_{f} 0.47 (Silica gel; EtOAc:CH₂Cl₂-6:4; UV)

### B.

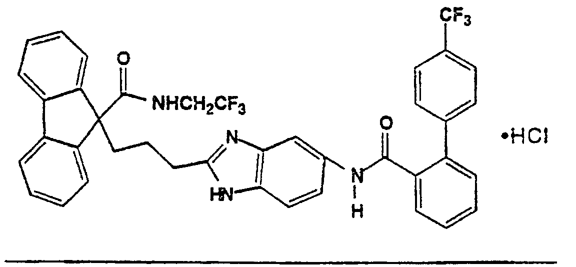

A solution of Part A compound (308 mg, 0.62 mmole) in dry CH₃OH (15 ml) was treated with 10% Pd/C (60 mg) and hydrogenated (balloon) at room temperature for 19 hours. The reaction mixture was diluted with CH₃OH (15 ml) and filtered through a celite pad in a millipore unit, washing the pad well with CH₃OH (3x). The combined filtrates were evaporated to dryness and dried in vacuo to give the crude amine as a syrup (281.7 mg).

The amine was dissolved in dry CH₂Cl₂ (8.0 ml), treated with 4'-(trifluoromethyl)-2-biphenylcarboxylic acid (167 mg, 0.65 mmole), HOBt·H₂O (86 mg, 0.64 mmole) and EDAC (133.4 mg, 0.68 mmole) and stirred at room temperature for 20 hours. The reaction mixture was partitioned between EtOAc (2 x 25 ml) and saturated NaHCO₃ (4.5 ml) and the combined organic extracts were washed with H₂O (3x) and brine, dried (anhydrous Na₂SO₄), filtered, evaporated to dryness and dried in vacuo. The crude product mixture was chromatographed on a silica gel column (Merck), eluting the column with EtOAc:Hexane mixtures (1:2; 4:1) to give the clean free base (165.7 mg, 37.3%).

This adduct (136 mg, 0.19 mmole) was dissolved in dry dioxane (1.7 ml), treated with 4.0 M HCl/dioxane (0.17 ml, 3.5 eq), swirled for a few minutes then diluted with dry Et₂O (25 ml), scratching the solids as they formed. The mixture was filtered and the solids washed with dry Et₂O (2x) to give title compound as a solid (123 mg, m.p. 170-180°C, shrinking commencing at 150°C).
MS: (M + H)⁺ = 713.

| | | | | | |
|---|---|---|---|---|---|
| Anal. Calc'd for C₄₀H₃₀F₆N₄O₂•HCl•0.9 H₂O | C, 62.77; | H, 4.32; | N, 7.32; | Cl, 4.63; | F, 14.89 |
| Found | C, 62.73; | H, 4.00; | N, 7.22; | Cl, 4.60; | F, 14.51 |

### Example 423

### 9-[3-[[4-Methyl-5-[[[4'-(trifluoromethyl)[1,1'-biphenyl]-2-yl]carbonyl]amino]-2-pyridinyl]oxy]propyl]-N-(2,2,2-trifluoroethyl)-9H-fluorene-9-carboxamide.

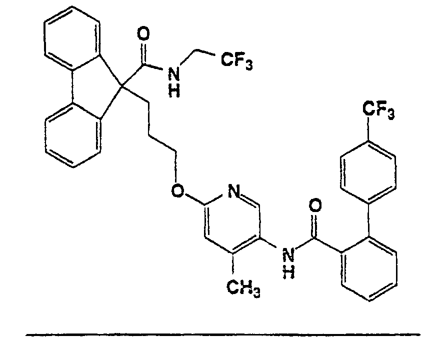

### A.

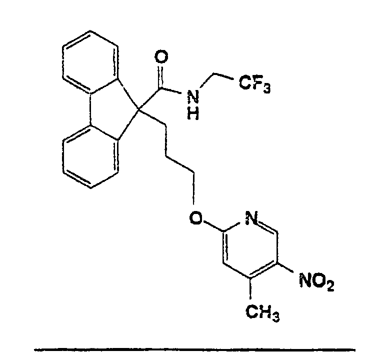

To a stirred solution of Example 417 Part B compound (7.0 g, 20.0 mmol, dried with toluene) in 200 mL of dry THF at 0°C under argon was added triphenylphosphine (7.9 g, 30.0 mmol) and 2-hydroxy-4-methyl-5-nitropyridine (3.7 g, 24.0 mmol) followed by the dropwise addition of diisopropyl azodicar-boxylate (DIAD) (5.9 mL, 30.0 mmol). The reaction mixture was stirred at 0°C for 1 h and quenched with sat. NaHCO₃ (70 mL) and concentrated to remove THF. Water (300 mL) was added and the mixture was extracted with EtOAc (3 x 150 mL). The combined organic layers were washed with H₂O (100 mL) and brine (100 mL), dried over Na₂SO₄ and concentrated in vacuo to give a viscous oil. Flash chromatography on Merck silica gel K-60 (800 g) eluting with EtOAc/hexane (0.5:9.5 to 1:4) provide 4.0 g (41%) of title compound as foam.

### B.

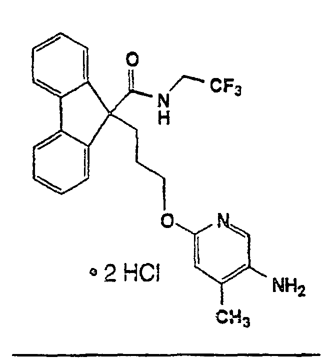

A mixture of Part A compound (1.5 g, 3.09 mmol) and 10% palladium on carbon (200 mg) in ethyl acetate (30 mL) was hydrogenated (balloon pressure) at RT for 24 h. TLC showed the presence of some starting material; therefore an additional quantity of 10% Pd/C (25 mg) was added and hydrogenation was continued for 12 h longer. The catalyst was removed by filtration through nylon 66 filter, and concentrated in vacuo to give crude amine. To the stirred solution of clear amine in Et₂O (100 mL) was added 4N HCl in dioxane (2.8 mL, 10.7 mmol). The separated solid was diluted with Et₂O (50 mL) and collected, dried in vacuo (0.5 mm) at RT for 3 h to give title compound (1.53 g, 94%) as off white solid.

### C.

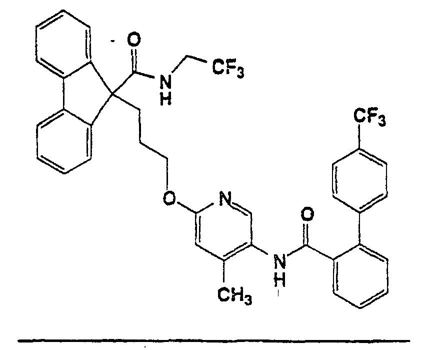

To a solution of crude Part B compound (106 mg, 0.2 mmol) and triethylamine (150 µl, 1.0 mmol) in CH₂Cl₂ (5 mL) at 0°C was added dropwise 220 µl of 1.0 M 4'-(trifluoromethyl)-2-biphenyl acid chloride solution in CH₂Cl₂ (0.22 mmol). The reaction was stirred at 0°C for 1 h. Dichloromethane (20 mL) was added and the solution was washed with sat. NaHCO₃ solution (2 x 5 mL), then dried over Na₂SO₄ and concentrated to give 190 mg of foam. Purification by flash chromatography on Merck silica gel K-60 (5 g) eluting with EtOAc/hexane (1:4 to 3:7) provided title compound (110 mg, 78%) as foam.
MS (ESI, + ions) m/z 704 (M + H).

### Example 424

### 9-[4-[2-(4-Morpholinyl)-4-[[[4'-(trifluoromethyl)[1,1'-biphenyl]-2-yl]carbonyl]amino]-1H-benzimidazol-1-yl]butyl]-N-(2,2,2-trifluoroethyl)-9H-fluorene-9-carboxamide.

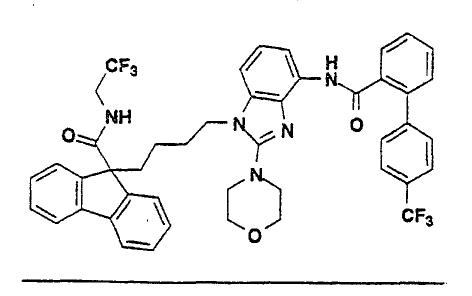

### A.

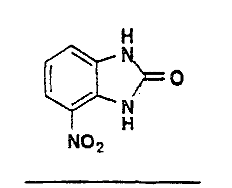

To a solution of 3-nitro-1,2-benzenediamine (5.36 g, 35 mmol) in 300 mL of dry THF cooled at 0°C was added Et₃N (10.95 mL), followed by dropwise addition of phosgene/toluene (1.93 M, 20 mL, 38.5 mmol). After addition, the resulting suspension was stirred at room temperature overnight, then filtered. The collected solid was washed with H₂O (4X), dried over P₂O₅ in vacuo for 2 days to give 3.98 g (63% yield) of title compound as a brown solid.

### B.

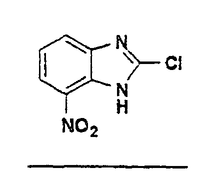

A suspension of Part A compound (3.583 g, 20 mmol) in 70 mL of POCl₃ was refluxed at 120°C for 3 hours, then a stream of HCl gas was bubbled through a gently refluxed suspension for 2 more hours. After cooling to room temperature, the reaction mixture was concentrated in vacuo to dryness. The obtained residue was dissolved in H₂O, adjusted pH to 6 with 10% aqueous NH₄OH, then extracted with EtOAc (3X). The combined EtOAc extracts were washed with H₂O (2X), brine, dried over MgSO₄. The filtrate was concentrated and the residue was absorbed on Celite, then chromatographed eluting with 25% EtOAc/hexane to give 2.785 g (71% yield) of title compound as a light yellow solid.

### C.

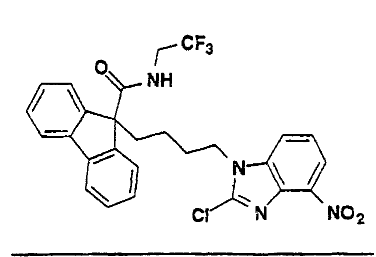

To a solution of Part B compound (2.785 g, 14.10 mmol) in 30 mL of anhydrous DMF was added 7.20 g (16.92 mmol) of Example 273 Part A(2) compound, followed by potassium carbonate (3.90g, 28.20 mmol). The resulting suspension was stirred at room temperature under argon for 64 hours, then partitioned between EtOAc/H₂O. The aqueous phase was extracted with EtOAc (3X), the combined EtOAc extracts washed with water (3X), brine, dried over MgSO₄. The filtrate was concentrated in vacuo to give a beige colored solid, which was triturated with EtOAc (2X), dried in air to yield 2.3 g of title compound as an off-white solid. The EtOAc washings were concentrated and the residue triturated with EtOAc, and the process repeated to afford 1.9 g more of title compound. The EtOAc washings from last trituration were concentrated and the residue absorbed on Celite, then chromatographed eluting with 20-50% EtOAc/hexane to give additional 0.4 g of title compound (total 4.6 g, 60% yield) as a light yellow solid.

### D.

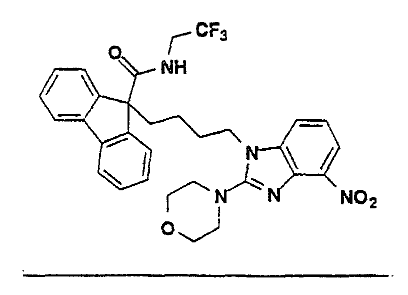

A solution of Part C compound (109 mg, 0.20 mmol) in neat morpholine (1 mL) was heated at 45°C under argon for 20 hours, then concentrated to dryness, the residue chromatographed eluting with 50-70% EtOAc/hexane to give 123 mg (100% yield) of title compound as a yellow foam.

### E.

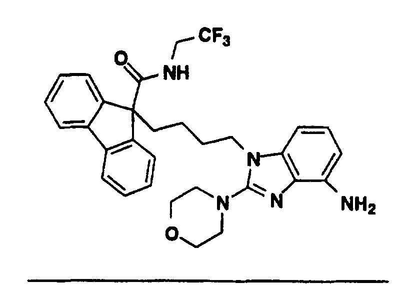

A suspension of Part D compound (115 mg, 0.2 mmol) and 45 mg of 10% Pd/C in EtOH/EtOAc (1:1, 4 mL) was hydrogenated under a hydrogen balloon for 3.5 hours, then filtered. The filtrate was concentrated, the residue stripped with CH₂Cl₂ (3X), dried in vacuo to give 110 mg (100% yield) of title compound as a white foam.

### F.

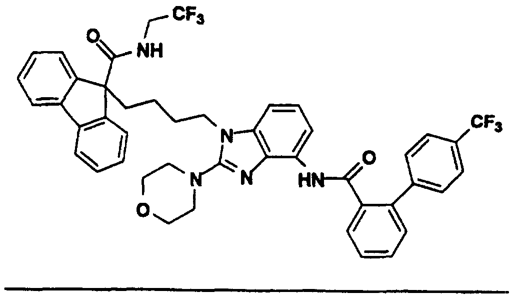

To a solution of Part E compound (110 mg, 0.2 mmol) in 0.5 mL of CH₂Cl₂ cooled at 0°C was added a 1.0 M solution of Example 415 Part A compound in CH₂Cl₂ (0.24 mL), followed by Et₃N (35 µL). The resulting mixture was stirred at room temperature under argon overnight, then diluted with EtOAc, washed with water, brine, dried over MgSO₄. The filtrate was concentrated in vacuo, the obtained residue absorbed on Celite, chromatographed eluting with 20-60% EtOAc/hexane to give 110 mg of title compound as a white foam, which was lyophilized in MeOH/H₂O to give 100 mg (61% yield) of title compound as a white powder.

| | |
|---|---|
| MS | (electrospray, + ions) m/e @ 812 (M+H). |
| MS | (high resolution) Calcd for C₄₅H₄₀N₅F₆O₃ (M+H), 812.3055 |
| Found | 812.2994. |

### Example 425

### 9-[4-[2-Methyl-4-[methyl[[4'-(trifluoromethyl)[1,1'-biphenyl]-2-yl]carbonyl]amino]-1H-benzimidazol-1-yl]butyl]-N-(2,2,2-trifluoroethyl)-9H-fluorene-9-carboxamide.

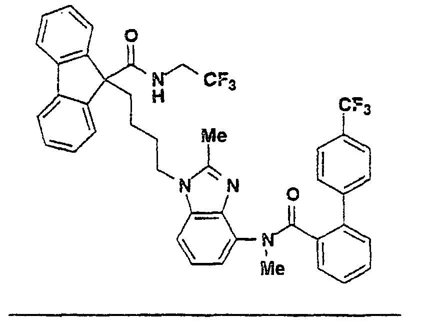

### A

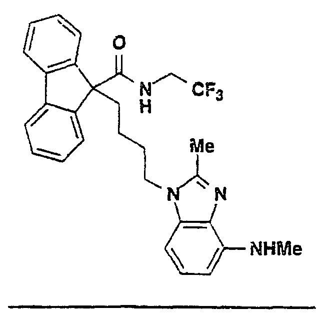

Acetic anhydride (472 µL, 5 mmol) was added to formic acid (5.0 mL) at 0°C. The reaction mixture was stirred at 0°C for 30 min, and a portion (1.9 mL, 1.9 mmol) was added slowly to a solution of Example 410 Part C compound (300 mg, 0.61 mmol) in THF (0.5 mL) at 0°C. After 30 min, the reaction mixture was partitioned between EtOAc (20 mL) and saturated NaHCO₃ (20 mL), and the organic layer was washed with saturated NaHCO₃ (5 mL) and brine (5 mL), dried over Na₂SO₄, and concentrated to give 189 mg of the formamide.

Lithium aluminum hydride (515 µL, 1.0M in THF, 0.515 mmol) was added dropwise to a solution of a portion of the formamide (312 mg) in THF (3 mL) at 0°C. The cooling bath was removed, and the reaction mixture was stirred at RT for 30 min. Following a quench with H₂O (0.5 mL), 1M sodium potassium tartrate (5 mL) was added, and the reaction mixture was stirred at RT vigorously for 2 h. The reaction mixture was extracted with EtOAc (2 x 10 mL), and the organic extracts were washed with brine (5 mL), dried over Na₂SO₄, and concentrated to give 110 mg of an opaque oil. The crude product was purified by flash chromatography on silica gel (35 g) eluting with a step gradient of 60% to 80% EtOAc/hexane to give title compound (280 mg, 89%) as a yellow foam.

### B.

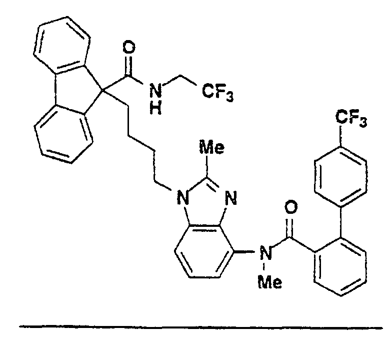

Following the procedure in Example 418 Part C, Part A compound (218 mg, 0.431 mmol) was acylated with Example 415 Part A compound to give title compound (289 mg, 89%) as a white foam.

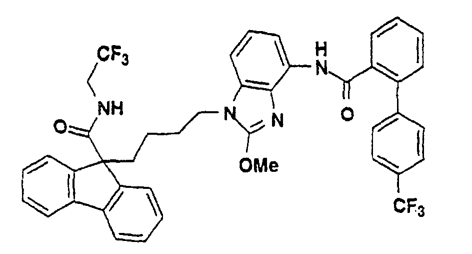

### Example 547

### 9-[4-[2-(Methylthio)-4-[[[4'-(trifluoromethyl)[1,1'-biphenyl]-2-yl]carbonyl]amino]-1 H-benzimidazol-1-yl]butyl]-N-(2,2,2-trifluoroethyl)-9H-fluorene-9-carboxamide.

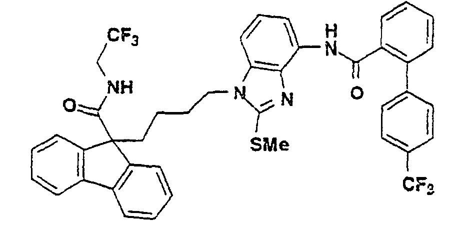

### Example 548

### 9-[4-(2-Chloro-4-[[[4'-(trifluoromethyl)[1,1'-biphenyl]-2-yl]carbonyl]amino]-1H-benzimidazol-1-yl]butyl]-N-(2,2,2-trifluoroethyl)-9H-fluorene-9-carboxamide.

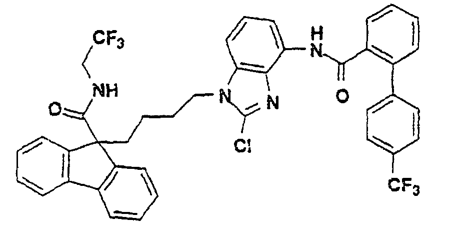

### Example 549

### [[[2-[9-[[(2,2,2-Trifluoroethyl)amino]carbonyl]-9H-fluoren-9-yl]ethyl]amino]methyl]phosphonic acid, bis(1-methylethyl) ester.

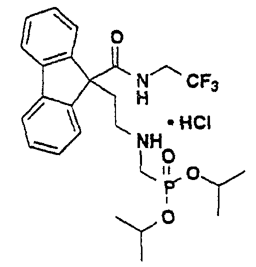

MS (ES, + ions) m/z 513 [M+H].

### Example 550

### N-(2,2,2-Trifluoroethyl)-9-[4-[5-[[[4'-(trifluoromethyl)[1,1'-biphenyl]-2-yl]carbonyl]amino]-1H-indol-1-yl]butyl]-9H-fluorene-9-carboxamide.

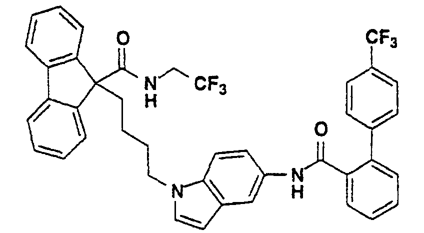

MS (ES, + ions) m/z 726 [M+H].

### Example 551

### 9-[4-[5-(Benzoylamino)-1H-indol-1-yl]butyl]-N-(2,2,2-trifluoroethyl)-9H-fluorene-9-carboxamide.

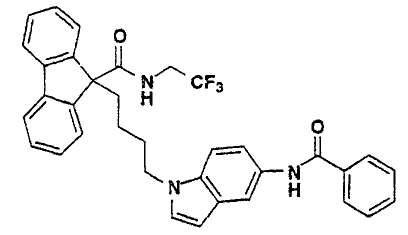

MS (ES, + ions) m/z 582 [M+H].

### Example 552

### N-(2,2,2-Trifluoroethyl)-9-[4-[5-[[[4'-(trifluoromethyl)[1,1'-biphenyl]-2-yl]carbonyl]amino]-2-pyridinyl]-3-butenyl]-9H-fluorene-9-carboxamide.

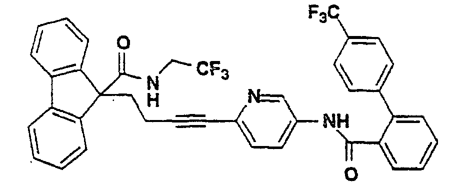

MS (ES, + ions) m/z 684 (M+H).

### Example 552A

### N-(2,2,2-Trifluoroethyl)-9-[4-[5-[[[4'-(trifluoromethyl)[1,1'-biphenyl]-2-yl]carbonyl]amino]-2-pyridinyl]-3-butenyl]-9H-fluorene-9-carboxamide, trifluoroacetate.

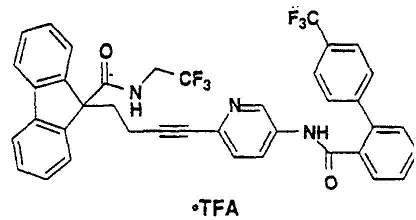

MS (ES, + ions) m/z 684 (M+H).

### Example 553

### 2-[3-[9-[[(2,2,2-Trifluoroethyl)amino]carbonyl]-9H-fluoren-9-yl]propoxy]-5-[[[4'-(trifluoromethyl)[1,1'-biphenyl]-2-yl]carbonyl]amino]-3-pyridinecarboxylic acid, methyl ester.

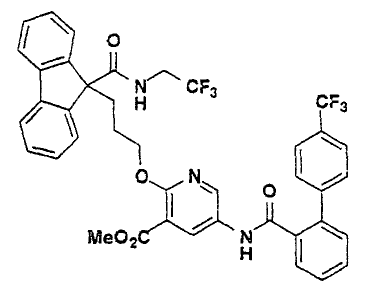

MS (ES, + ions) m/z 748 [M+H].

### Example 554

### 2-[3-[9-[[(2,2,2-Trifluoroethyl)amino]carbonyl]-9H-fluoren-9-yl]propoxy]-5-[[[4'-(trifluoromethyl)[1,1'-biphenyl]-2-yl]carbonyl]amino]-3-pyridinecarboxylic acid.

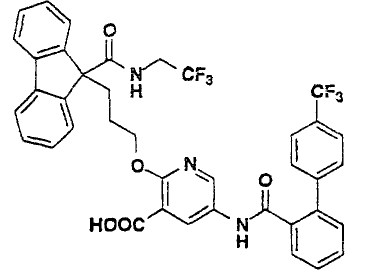

MS (ES, + ions) m/z 734 [M+H].

### Example 555

### N-(2,2,2-Trifluoroethyl)-9-[4-[4-[[[4'-(trifluoromethyl)[1,1'-biphenyl]-2-yl]sulfonyl]amino]-1H-indol-1-yl]butyl]-9H-fluorene-9-carboxamide.

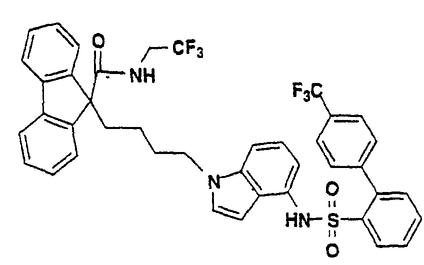

MS (ES, + ions) m/z 762 (M+H).

### Example 556

### N-(2,2,2-Trifluoroethyl)-9-[4-[4-[[[4'-(trifluoromethyl)[1,1'-biphenyl]-2-yl]carbonyl]amino]-1H-benzotriazol-1-yl]butyl]-9H-fluorene-9-carboxamide.

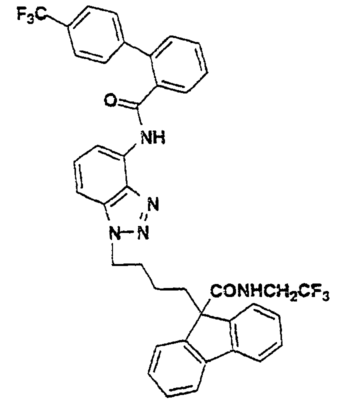

MS: (electrospray, + ions) m/z 728 (M+H).

### Example 557

### N-(2,2,2-Trifluoroethyl)-9-[5-[4-[[[4'-(trifluoromethyl)[1,1'-biphenyl]-2-yl]-carbonyl]amino]-1H-benzimidazol-1-yl]pentyl]-9H-fluorene-9-carboxamide.

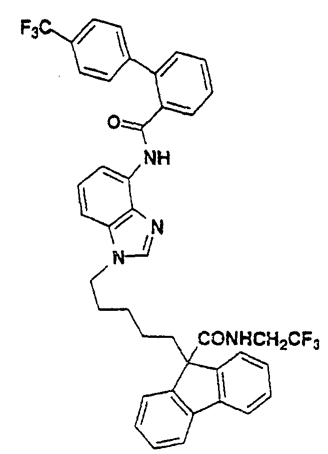

MS: (electrospray, + ions) m/z 741 (M+H).

### Example 558

### 9-[4-[4-[Methyl[[4'-(trifluoromethyl)[1,1'-biphenyl]-2-yl]carbonyl]amino]-1H-benzimidazol-1-yl]butyl]-N-(2,2,2-trifluoroethyl)-9H-fluorene-9-carboxamide.

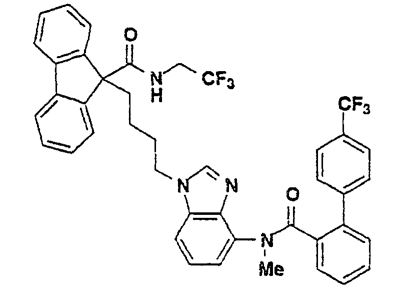

MS (ES, + ions) m/z 741 [M+H].

### Example 559

### 9-[3-[5-[Methyl[[4'-(trifluoromethyl)[1,1'-biphenyl]-2-yl]carbonyl]amino]-1H-benzimidazol-1-yl]propyl]-N-(2,2.2-trifluoroethyl)-9H-fluorene-9-carboxamide.

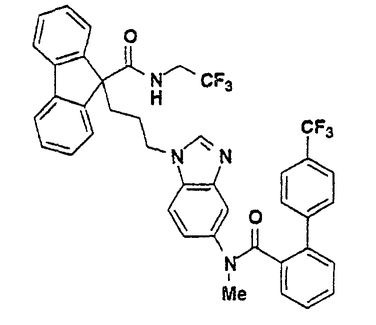

MS (ES, + ions) m/z 727 [M+H].

### Example 560

### N-(2,2,2-Trifluoroethyl)-9-[4-[4-[[[4'-(trifluoromethyl)[1,1'-biphenyl]-2-yl]carbonyl]amino]-6H-pyrrolo[2,3-c]pyridin-6-yl]butyl]-9H-fluorene-9-carboxamide.

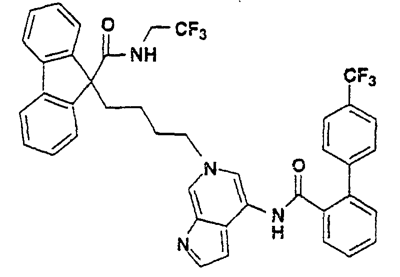

MS (ES, + ions) m/z 727 (M+H).

### Example 561

### 9-[4-[2-(1-Methylethyl)-4-[[[4'-(trifluoromethyl)[1,1'-biphenyl]-2-yl]carbonyl]amino]-1H-benzimidazol-1-yl]butyl]-N-(2,2,2-trifluoroethyl)-9H-fluorene-9-carboxamide.

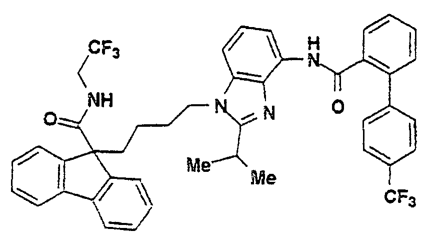

MS: m/z 769 (M+H)⁺.

### Example 562

### 9-[3-[2-(Diethylamino)-5-[[[4'-(trifluoromethyl)[1,1'-biphenyl]-2-yl]carbonyl]amino]-1H-benzimidazol-1-yl]propyl]-N-(2,2,2-trifluoroethyl)-9H-fluorene-9-carboxamide.

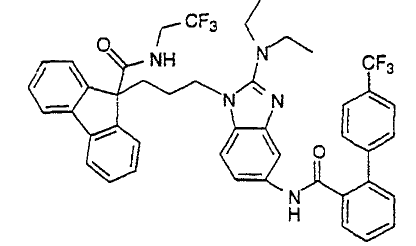

MS: (M+H)⁺.@ 784.

### Example 563

### N-(2,2,2-Trifluoroethyl)-9-[4-[4-[[[4'-(1,1,1-trifluoromethyl)[1,1'-biphenyl]-2-yl]carbonyl]amino]-1H-imidazol-1-yl]butyl]-9H-fluorene-9-carboxamide, monohydrochloride.

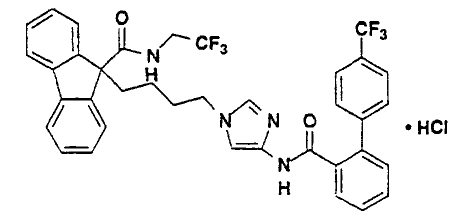

MS: (M+H)⁺. = 677.

### Example 564

### N-(2,2,2-Trifluoroethyl)-9-[3-[[5-[[[4'-(trifluoromethyl)[1,1'-biphenyl]-2-yl]carbonyl]amino]-2-pyridinyl]amino]propyl]-9H-fluorene-9-carboxamide, trifluoroacetate.

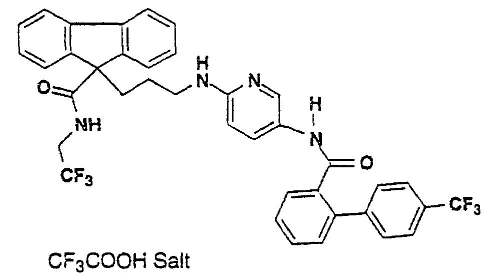

MS (ES, NH₃, + ions) m/ z 689 (M+H).

### Example 565

### [4-[9-[[(2,2,2-Trifluoroethyl)amino]carbonyl]-9H-fluoren-9-yl]butyl]phosphonic acid, butyl 3-[[[4'-(trifluoromethyl)[1,1'-biphenyl]-2-yl]carbonyl]amino]propyl ester.

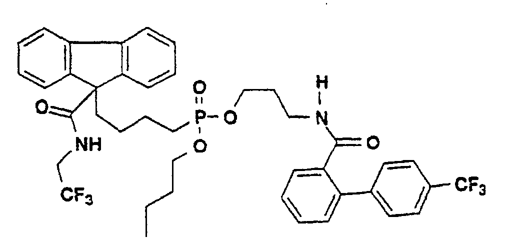

MS (ES, NH₃, + ions) m/z 806 (M+NH₄), 789 (M+H).

### Example 566

### [4-[9-[[(2,2,2-Trifluoroethyl)amino]carbonyl]-9H-fluoren-9-yl]butyl]phosphonic acid, butyl 2-[[[4'-(trifluoromethyl)[1,1'-biphenyl]-2-yl]carbonyl]amino]ethyl ester.

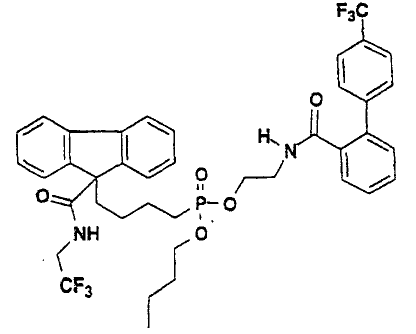

MS (ES, NH₃, + ions) m/z 792 (M+NH₄), 775 (M+H).

### Example 567

### 9-[3-[[5-(Benzoylamino)-2-pyridinyl]amino]propyl]-N-(2,2,2-trifluoroethyl)-9H-fluorene-9-carboxamide, monohydrochloride.

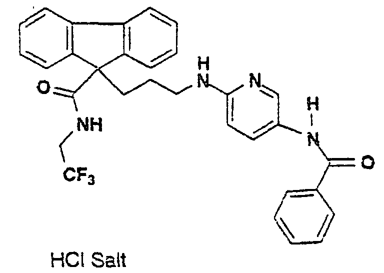

MS (ES, NH₃, + ions) m/z 545 (M+H).

### Example 568

### 9-[3-[[5-[[2-(2-Benzothiazolyl)benzoyl]amino]-2-pyridinyl]oxy]propyl]-N-(2,2,2-trifluoroethyl)-9H-fluorene-9-carboxamide, monohydrochloride.

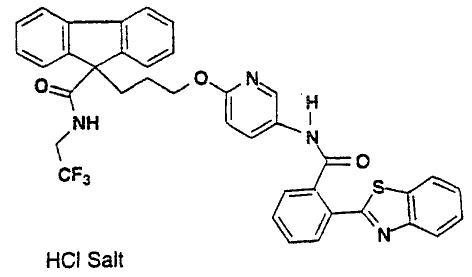

MS (ES, NH₃, + ions) m/z 679 (M+H).

### Example 569

### 9-[3-[[5-[[2-(2-Pyridinyl)benzoyl]amino]-2-pyridinyl]oxy]propyl]-N-(2,2,2-trifluoroethyl)-9H-fluorene-9-carboxamide, dihydrochloride.

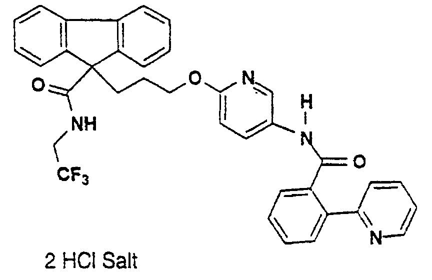

MS (ES, NH₃, + ions) m/z 623 (M+H).

### Example 570

### 9-[3-[[5-[[2-(4-Morpholinyl)benzoyl]amino]-2-pyridinyl]oxy]propyl]-N-(2,2,2-trifluoroethyl)-9H-fluorene-9-carboxamide, dihydrochloride.

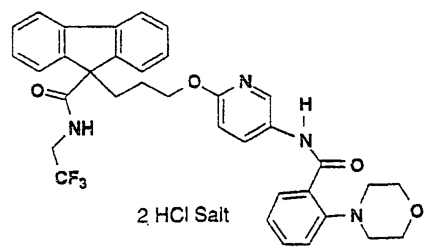

MS (ES, NH₃, + ions) m/z 631 (M+H).

### Example 571

### 1-(Phenylmethyl)-N-[2-[3-[9-[[(2,2,2-trifluoroethyl)amino]carbonyl]-9H-flluoren-9-yl]propoxy]-5-pyridinyl]-2-piperidinecarboxamide, dihydrochloride.

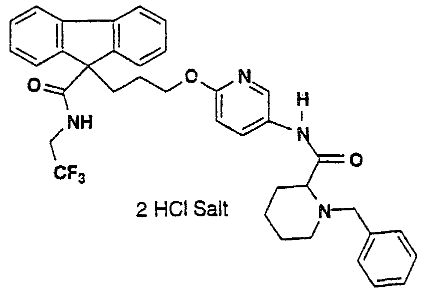

MS (ES, NH₃, + ions) m/z 643 (M+H).

### Example 572

### N-(2,2,2-Trifluoroethyl)-9-[5-[[5-[[[4'-(trifluoromethyl)[1,1'-biphenyl]-2-yl]carbonyl]amino]-2-pyridinyl]oxy]pentyl]-9H-fluorene-9-carboxamide.

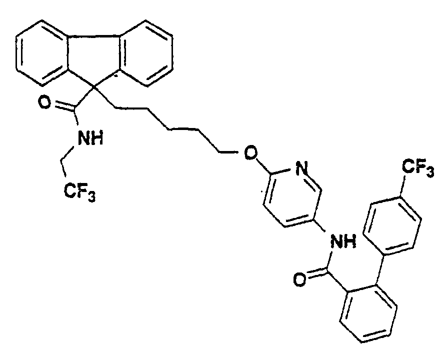

MS (ES, NH₃, + ions) m/z 718 (M+H).

### Example 573

### 9-[5-[[5-(Benzoylamino)-2-pyridinyl]oxy]pentyl]-N-(2,2,2-trifluoroethyl)-9H-fluorene-9-carboxamide.

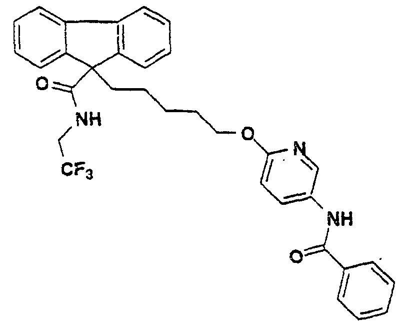

MS (ES, NH₃, + ions) m/z 574 (M+H).

### Example 574

### 9-[3-[5-[[(4'-Chloro[1,1'-biphenyl]-2-yl)carbonyl]amino]-1H-benzimidazol-1-yl]propyl]-N-(2,2,2-trifluoroethyl)-9H-fluorene-9-carboxamide.

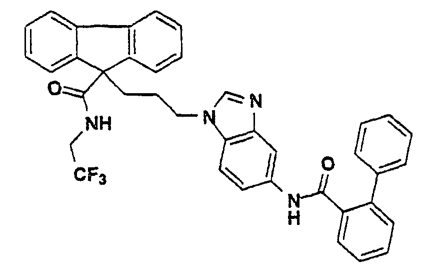

MS (ES, NH₃, + ions) m/z 680 (M+H).

### Example 575

### 9-[3-[[5-[[(4'-Chloro[1,1'-biphenyl]-2-yl)carbonyl]amino]-2-pyridinyl]oxy]propyl]-N-(2,2,2-trifluoroethyl)-9H-fluorene-9-carboxamide, hydrochloride.

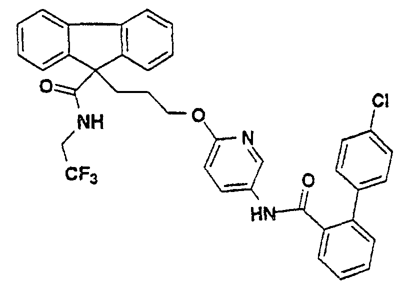

MS (ES, NH₃, + ions) m/z 656 (M).

### Example 576

### 9-[4-[4-[[(4'-Chloro[1,1'-biphenyl]-2-yl)carbonyl]amino]-1H-benzimidazol-1-yl]butyl]-N-(2,2,2-trifluoroethyl)-9H-fluorene-9-carboxamide.

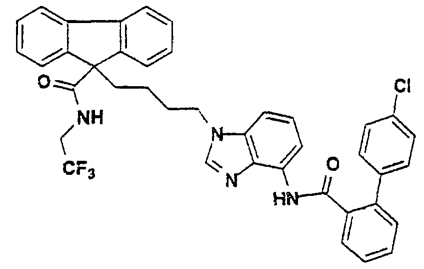

MS (ES, NH₃, + ions) m/z 693 (M).

### Example 577

### 9-[4-[4-[[(4'-Chloro[1,1'-biphenyl]-2-yl)carbonyl]amino]-1H-indol-1-yl]butyl]-N-(2,2,2-trifluoroethyl)-9H-fluorene-9-carboxamide.

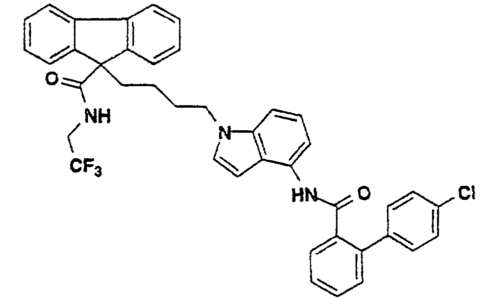

MS (ES, NH₃, + ions) m/z 692 (M).

### Example 578

### N-(2,2,2-Trifluoroethyl)-9-[3-[5-[[[4'-(1,1,1-trifluoromethyl)[1,1'-biphenyl]-2-yl]carbonyl]amino]-2H-indazol-2-yl]propyl]-9H-fluorene-9-carboxamide.

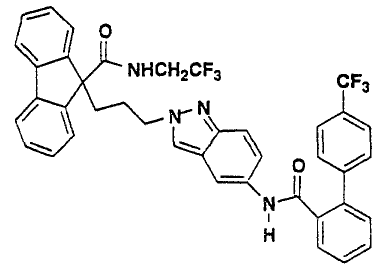

MS (M+H)⁺ = 713.

### Example 579

### 9-[4-[[5-Amino-1-[[4'-(trifluoromethyl)[1,1'-biphenyl]-2-yl]carbonyl]-1H-1,2,4-triazol-3-yl]thio]butyl]-N-(2,2,2-trifluoroethyl)-9H-fluorene-9-carboxamide.

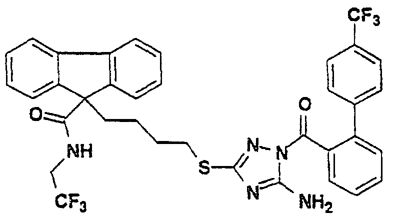

MS (ES, NH₃, + ions) m/z 710 (M+H).

### Example 580

### 9-[4-[[5-Amino-1-[(4'-chloro[1,1'-biphenyl]-2-yl)carbonyl]-1H-1,2,4-triazol-3-yl]thio]butyl]-N-(2,2,2-trifluoroethyl)-9H-fluorene-9-carboxamide.

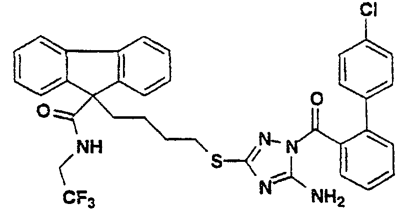

MS (ES, NH₃, + ions) m/z 676 (M+H).

### Example 581

### 9-[3-[[5-Amino-1-[[4'-(trifluoromethyl)[1,1'-biphenyl]-2-yl]carbonyl]-1H-1,2,4-triazol-3-yl]thio]propyl]-N-(2,2,2-trifluoroethyl)-9H-fluorene-9-carboxamide.

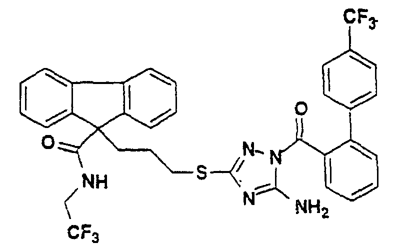

MS (ES, NH₃, + ions) m/z 696 (M+H).

### Example 582

### 9-[3-[[5-Amino-1-[(4'-chloro[1,1'-biphenyl]-2-yl)carbonyl]-1H-1,2,4-triazol-3-yl]thio]propyl]-N-(2,2,2-trifluoroethyl)-9H-fluorene-9-carboxamide.

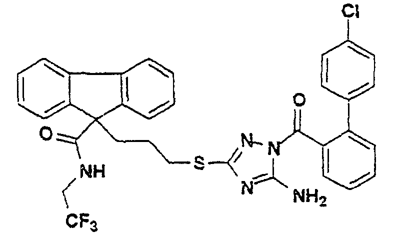

MS (ES, NH₃, + ions) m/z 662 (M+H).

### Example 583

### N-(2,2,2-Trifluoroethyl)-9-[4-[4-[[[4'-(trifluoromethyl)[1,1'-biphenyl]-2-yl]carbonyl]amino]-6H-pyrrolo[2,3-c]pyridin-6-yl]butyl]-9H-fluorene-9-carboxamide.

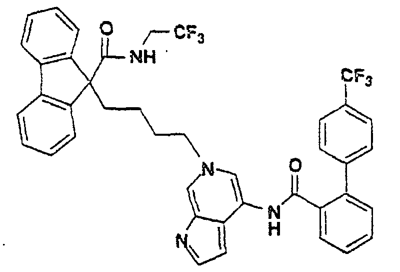

MS (ES, + ions) m/z 727 (M+H).

### Example 584

### N-(2,2,2-Trifluoroethyl)-9-[4-[4-[[[4'-(trifluoromethoxy)[1,1'-biphenyl]-2-yl]carbonyl]amino]-1H-benzimidazol-1-yl]butyl]-9H-fluorene-9-carboxamide.

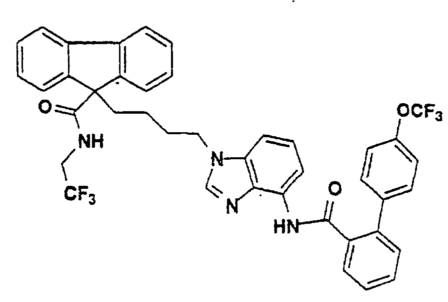

MS (ES, NH₃, + ions) m/z 743 (M+H).

### Example 585

### 9-[4-[4-[[[3',5'-Bis(trifluoromethyl)[1,1'-biphenyl]-2-yl]carbonyl]amino]-1H-benzimidazol-1-yl]butyl]-N-(2,2,2-trifluoroethyl)-9H-fluorene-9-carboxamide.

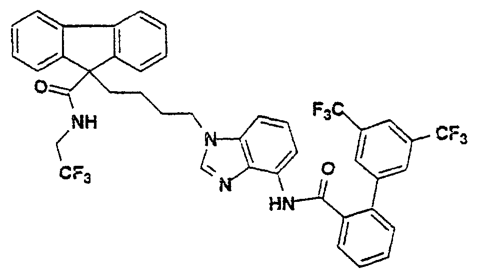

MS (ES, NH₃, + ions) m/z 795 (M+H).

### Example 586

### N-(2,2,2-Trifluoroethyl)-9-[4-[4-[[[3'-(trifluoromethyl)[1,1'-biphenyl]-2-yl]carbonyl]amino]-1H-benzimidazol-1-yl]butyl]-9H-fluorene-9-carboxamide.

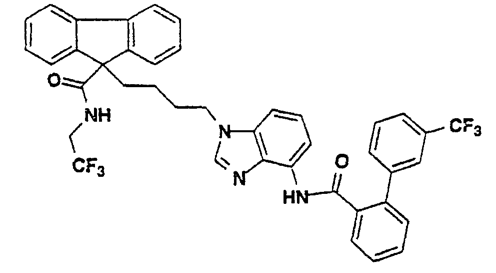

MS (ES, NH₃, + ions) m/z 727 (M+H).

### Example 587

### 9-[3-[2-(4-Morpholinyl)-5-[[[4'-(1,1,1-trifluoromethyl)[1,1'-biphenyl]-2-yl]carbonyl]amino]-1H-benzimidazol-1-yl]propyl]-N-(2,2,2-trifluoroethyl)-9H-fluorene-9-carboxamide.

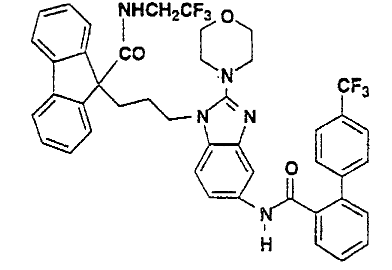

MS (M+H) ⁺ = 798.

### Example 588

### 9-[4-[2-Methyl-4-[[[3'-(trifluoromethyl)[1,1'-biphenyl]-2-yl]carbonyl]amino]-1H-benzimidazol-1-yl]butyl]-N-(2,2,2-trifluoroethyl)-9H-fluorene-9-carboxamide.

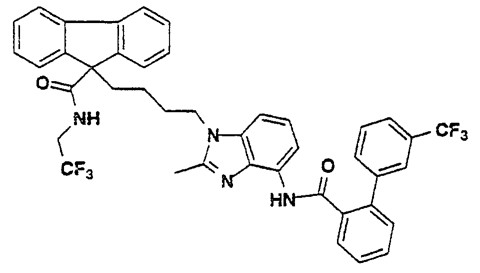

MS (ES, NH₃, + ions) m/z 741 (M+H).

### Example 589

### 9-[4-[1-Methyl-5-[[[4'-(trifluoromethyl)[1,1'-biphenyl]-2-yl]carbonyl]amino]-1H-benzimdazol-2-yl]butyl]-N-(2,2,2-trifluoroethyl)-9H-fluorene-9-carboxamide, and 9-[4-[1-methyl-6-[[[4'-(trifluoromethyl)[1,1'-biphenyl]-2-yl]carbonyl]amino]-1H-benzimidazol-2-yl]butyl]-N-(2,2,2-trifluoroethyl)-9H-fluorene-9-carboxamide (1:1).

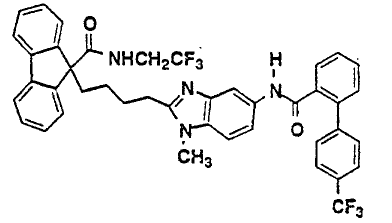

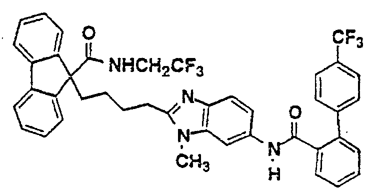

MS: (M+H)⁺ = 741.

### Example 590

### 9-[4-[(6-Ethoxy-2-benzothiazolyl)thio]butyl]-N-propyl-9H-fluorene-9-carboxamide.

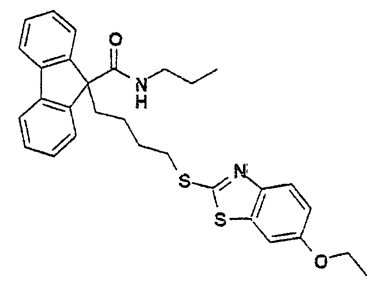

MS (ES) 517 (M+H).

### Example 591

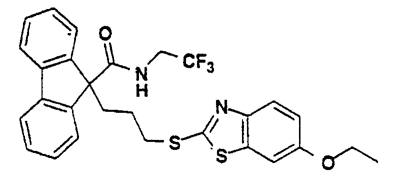

MS (ESI, + ions): m/z 543 (M+H).

### Example 592

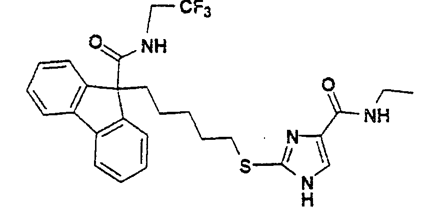

MS (eletrospray, pos. ions): m/z 531 (M+H).

### Example 593

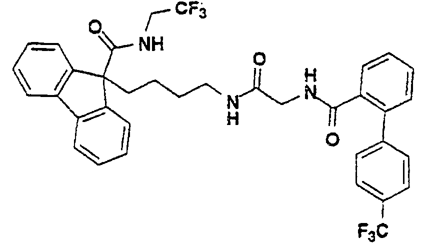

MS (eletrospray, pos. ions): m/z 668 (M+H).

### Example 594

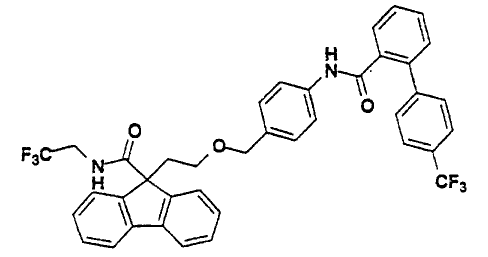

MS: (ESI, + ions) m/z 689 (M+H), 706 (M+NH₄).

### Example 595

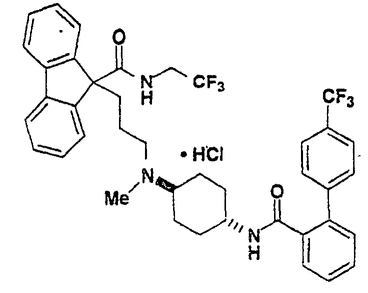

MS (ES, + ions) m/z 708 [M+H].

### Example 596

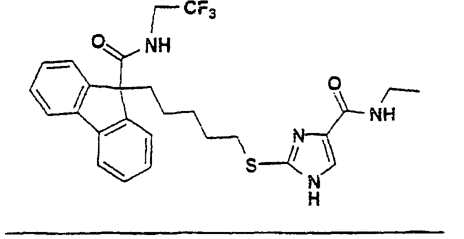

The reaction sequence for preparation of title compound was carried out in batch mode until the final amide coupling which was carried out using a Varian Vac Elute SPS 24 as one of a 24 compound run. During the amide formation and cleavage all mixing was done by having the Vac Elute SPS 24 mounted to an orbital shaker. Mixing was done at 265 rpm unless otherwise noted.

### A.

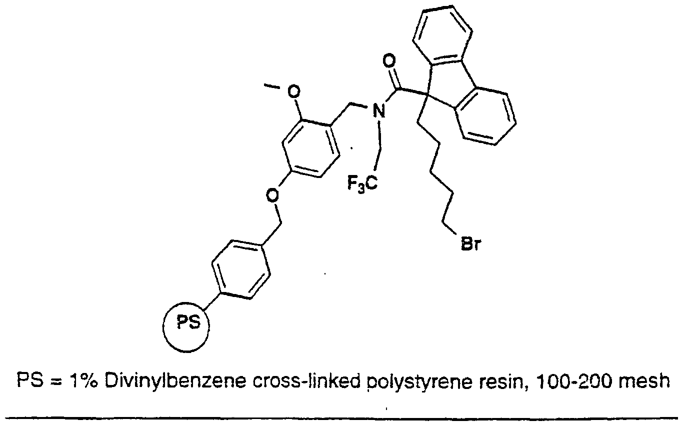

Title resin was prepared as described for Example 688 Part E except that 9-(5-bromopentyl)-9H-fluorene carboxylic acid chloride was used for the acylation with Example 689 Part A resin.

### B.

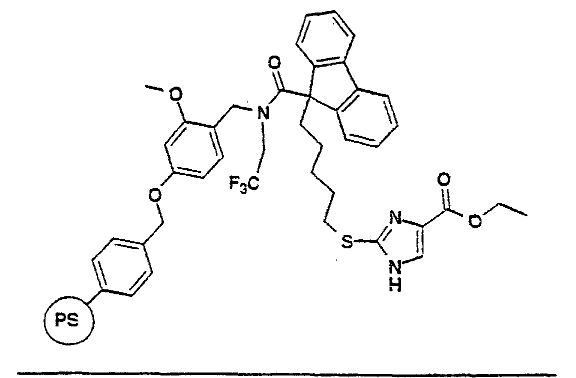

Title resin was prepared as descibed for Example 689 Part D compound employing 4-ethoxycarbonylimidazole-2-thiol (Maybridge Chemical Co.).

### C.

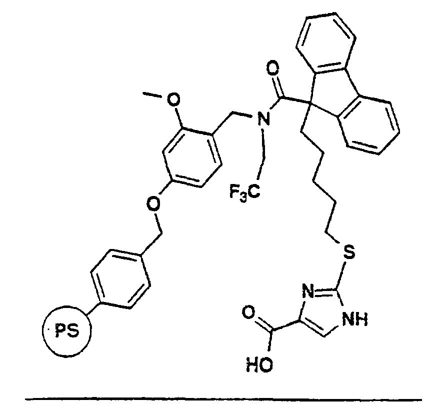

Part B resin (6.6 mmol) was swollen in 40 mL of THF, followed by draining of the solvent using nitrogen pressure. The resin was treated with a solution of 5.6 g (99 mmol, 15 eq) of KOH in 15 mL of water, 30 mL of MeOH and 30 mL of THF. The reaction mixture was heated at 50'C and vortexed for 4 days. The reaction mixture was cooled to RT and the reaction solution was removed. The resin was rinsed with 1:1 THF:water (3 x 50 mL), THF (3 x 50 mL) , 5% acetic acid in THF (3 x 30 mL), THF (3 x 50 mL), CH₂Cl₂ (3 x 50 mL) and MeOH (3 x 50 mL). The title resin was used in the next step without characterization.

### D.

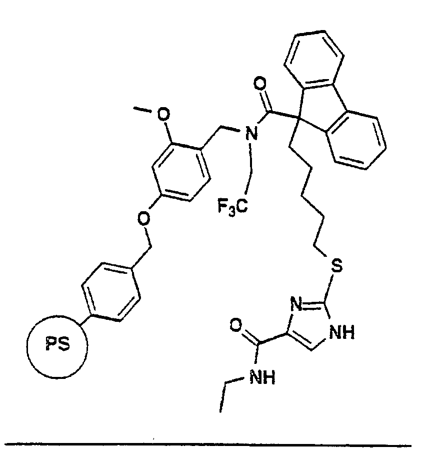

### Method A.

Part C resin (300 mg, 0.28 mmol) in a 25 mL polypropylene tube was swollen in 3 mL of CH₂Cl₂ and drained. The resin was suspended in 3 mL of a 1:1 CH₂Cl₂:DMF solution and treated with 376 mg (1.9 mmol, 7 eq) of 1-(3-dimethylaminopropyl)-3-ethylcarbodi-imide hydrochloride (EDC) and 267 mg (1.9 mmol, 7 eq) of 1-hydroxy-7-azabenzotriazole (HOAt). Diethylamine gas (was then bubbled into the reaction mixture for 5 min (≥10 eq). The reaction mixture was shaken for 18 h, the reaction solution was drained and the resin was retreated under the same conditions. After 72 h, the reaction solution was again drained and the resin was rinsed with DMF (4 x 5 mL) and CH₂Cl₂ (4 x 5 mL). The title resin was used in the next step without characterization.

### Method B

The Part C resin was swollen in 3 mL of CH₂Cl₂ and drained. The resin was suspended in 3 mL of a 1:1 CH₂Cl₂:DMF solution and treated with 307 µL (247 mg, 1.9 mmol, 7 eq) diisopropylcarbodiimide and 342 mg (2.8 mmol, 10 eq) of 4-dimethylaminopyridine (DMAP). The required amine (10 eq) was and the reaction mixture was shaken for 18 h. The reaction solution was drained and the resin was retreated under the same conditions. After 72 h, the reaction solution was again drained and the resin was rinsed with DMF (4 x 5 mL) and CH₂Cl₂ (4 x 5 mL). The resin was used in the next step without characterization.

### E.

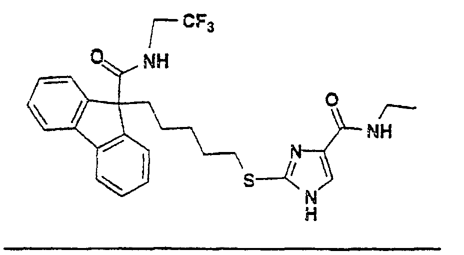

The Part D resin was treated with 2 mL of 100% trifluoroacetic acid and shaken for 90 min. The cleavage solution was collected, the resin was rinsed with CH₂Cl₂ (2 x 1 mL) and the combined cleavage solution and rinses were concentrated on a Speed Vac at RT. After 18 h, the sample was reconstituted in 4 mL of CH₂Cl₂ and reconcentrated on the Speed Vac. After 18 h, the sample was again reconstituted in 4 mL of CH₂Cl₂ and aliquots were removed for HPLC and MS analysis. The tube was concentrated again on the Speed Vac at ∼40°C followed by exposure to high vacuum (1 mm Hg) on a lyophilizer for 14 h to afford 161 mg of crude product mixture of which **6** was 26%. The desired product was purified by preparative HPLC using a YMC-Pack ODS-A 250 x 30 mm, S-5 µm, 120 A column with a 70-100 %B gradient over 30 min, holding at 100% B for 15 min at a flow of 25 mL/min (Solvent A: 90% H₂O/10% MeOH with 0.1% TFA; Solvent B: 90% MeOH/10% H₂O with 0.1% TFA) to provide 25 mg (17% based on starting aldehyde resin) of title compound as a cloudy oil.
HPLC: retention time: 4.7 min; 90% purity. HPLC conditions: YMC S3 ODS 4.6 x 50 mm
Rapid Resolution column; linear gradient from 50% B to 100% B over 8 min and held at 100% B for 2 min (method name: SMET4); flow rate 2.5 mL/min; detection at 215 nm; Solvent A: 90% H₂O/10% MeOH with 0.2% H₃PO₄; Solvent B: 90% MeOH/10% H₂O with 0.2% H₃PO₄.
MS(electrospray, pos. ions): m/z 531 (M + H)

### Example 597

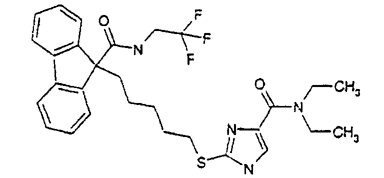

MS: m/z 559 (M+H)

### Example 598

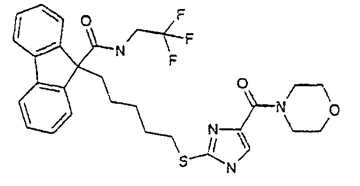

MS: m/z 573 (M+H)

### Example 599

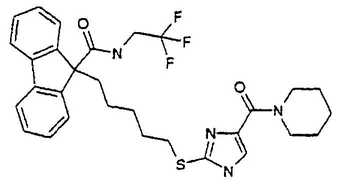

MS: m/z 571 (M+H)

### Example 600

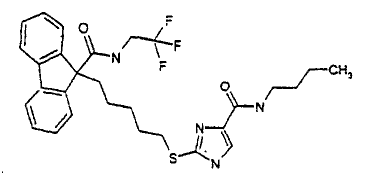

MS: m/z 559 (M+H)

### Example 601

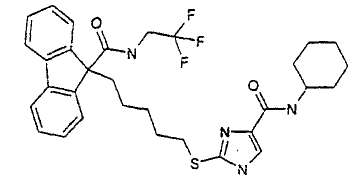

MS: m/z 585 (M+H)

### Example 602

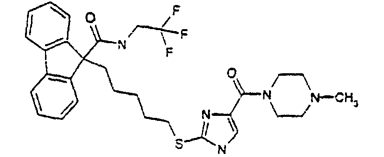

MS: m/z 586 (M+H)

### Example 603

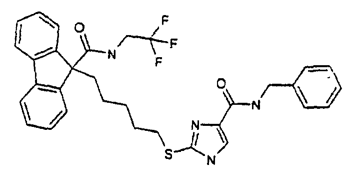

MS: m/z 593 (M+H)

### Example 604

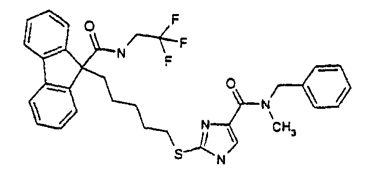

MS: m/z 607 (M+H)

### Example 605

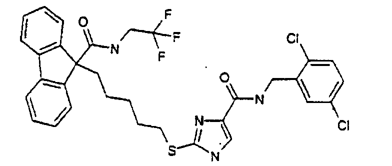

MS: m/z 661 (M+H)

### Example 606

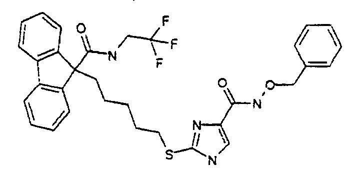

MS: m/z 609 (M+H)

### Example 607

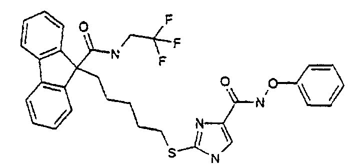

MS: m/z 595 (M+H)

### Example 608

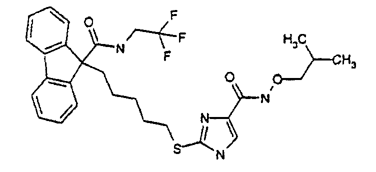

MS: m/z 575 (M+H)

### Example 609

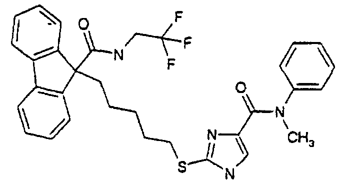

MS: m/z 593 (M+H)

### Example 610

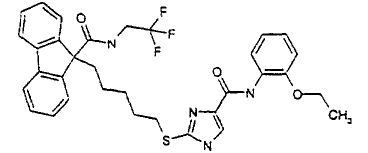

MS: m/z 623 (M+H)

### Example 611

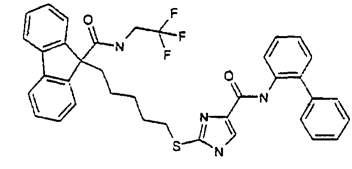

MS: m/z 655 (M+H)

### Example 612

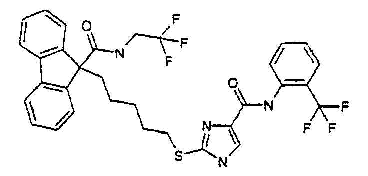

MS: m/z 647 (M+H)

### Example 613

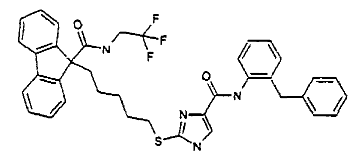

MS: m/z 669 (M+H)

### Example 614

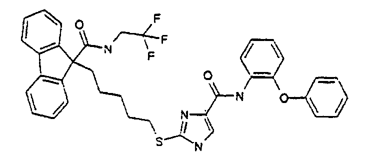

MS: m/z 671 (M+H)

### Example 615

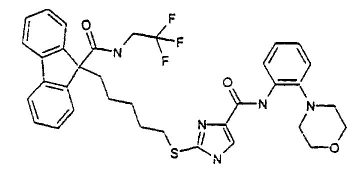

MS: m/z 664 (M+H)

### Example 616

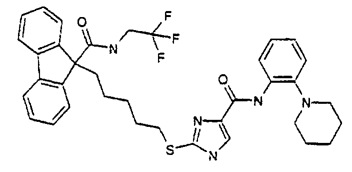

MS: m/z 662 (M+H)

### Example 617

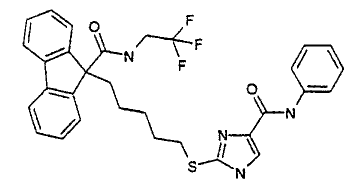

MS: m/z 579 (M+H)

### Example 618

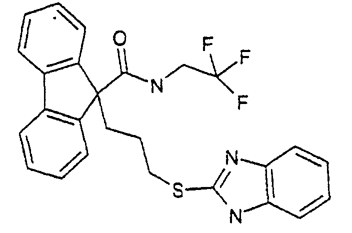

MS: m/z 482 (M+H)

### Example 619

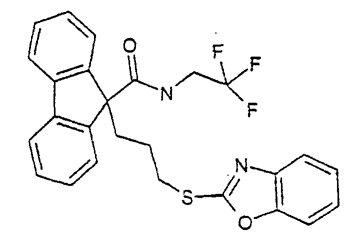

MS: m/z 483 (M+H)

### Example 620

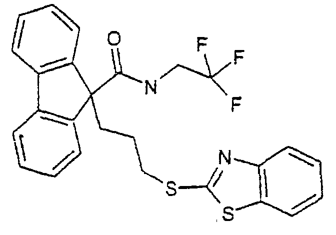

MS: m/z 499 (M+H)

### Example 621

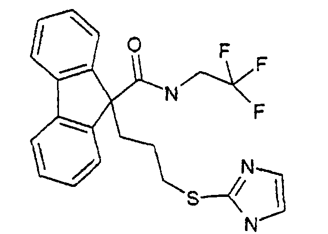

MS: m/z 432 (M+H)

### Example 622

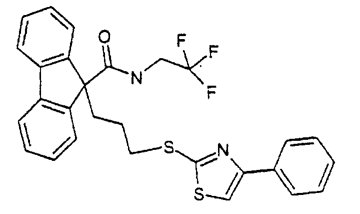

MS: m/z 525 (M+H)

### Example 623

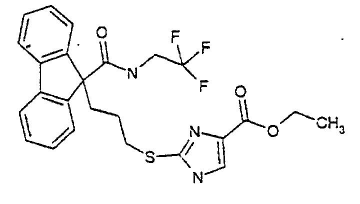

MS: m/z 504 (M+H)

### Example 624

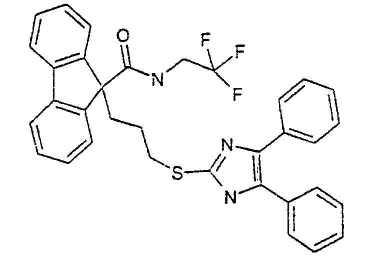

MS: m/z 584 (M+H)

### Example 625

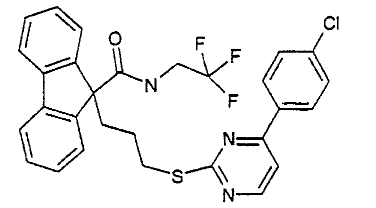

MS: m/z 554 (M+H)

### Example 626

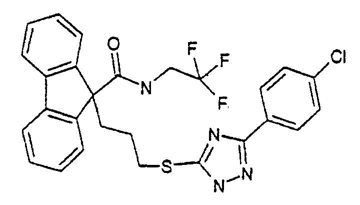

MS: m/z 543 (M+H)

### Example 627

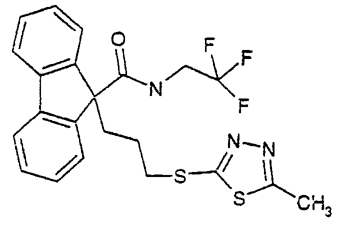

MS: m/z 464 (M+H)

### Example 628

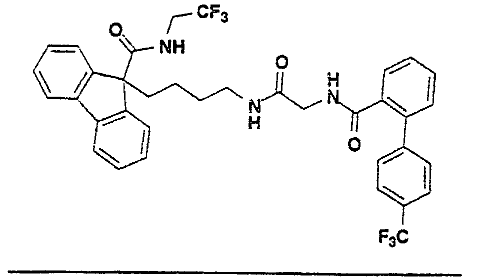

The reaction sequence for preparation of title compound was carried out using the 48-Weller solid phase reactor mounted to an orbital shaker as part of a 48 compound run. Shaking was done at 300 rpm.

### A.

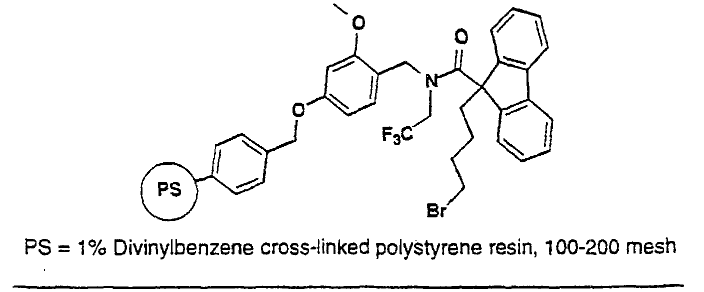

The title resin was prepared as described for Example 688 Part E except that 9-(4-bromobutyl)-9H-fluorene carboxylic acid chloride was used for the acylation with Example 689 Part A resin.

### B.

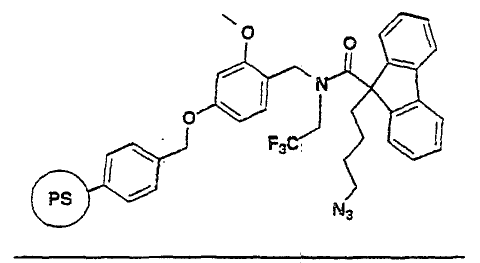

Part A resin (0.2 mmol) was swollen in 2 mL of dry DMF and drained using argon pressure. The resin was suspended in 1 mL of dry DMF and a solution of 284 mg (1 mmol, 5 eq) of tetrabutylammonium azide in 1 mL of DMF was added. After shaking for 16 h at RT, the reaction solution was drained and the title resin was rinsed with DMF (2 x 2 mL) and THF (2 x 2 mL). The title resin was used in the next step without characterization.

### C.

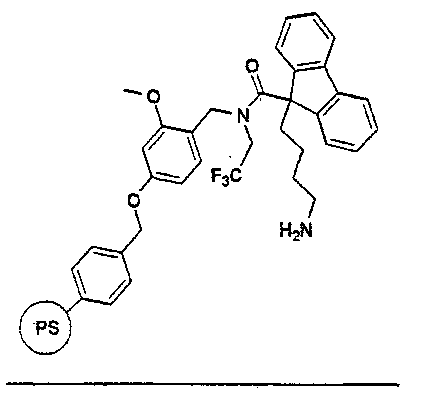

To the THF swollen Part B resin was added a solution of 262 mg (1 mmol, 5 eq) of triphenylphosphine and 1.26 mL (1.4 mmol, 7 eq) of water in 2 mL of THF. After shaking for 7 h at RT, the reaction solution was drained and the resin was rinsed with THF (3 x 2 mL) and DMF (2 x 2 mL). The title resin was used in the next step without characterization.

### D.

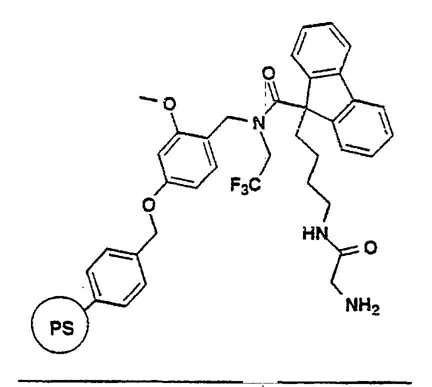

To the DMF swollen Part C resin were added a solution of 135 mg (1 mmol, 5 eq) of N-hydroxybenzotriazole and 293 mg (1 mmol, 5 eq) of FMOC-glycine in 1.5 mL of DMF and a solution of 126 mg (1 mmol, 5 eq) of diisopropylcarbodiimide in CH₂Cl₂. After shaking for 12 h at RT, the reaction solution was drained and the resin was retreated under the same conditions for 3 h. The reaction solution was drained and the resin was rinsed with DMF (1 x 2 mL), CH₂Cl₂ (2 x 2 mL) and DMF (2 x 2 mL). The resin was then treated with 3 mL of 30% piperidine in DMF. After shaking at RT for 30 min, the reaction solution was drained and the resin was treated again with 3 mL of 30% piperidine in DMF. After draining the reaction solution, the title resin was rinsed with DMF (3 x 2 mL). The title resin was used in the next step without characterization.

### E.

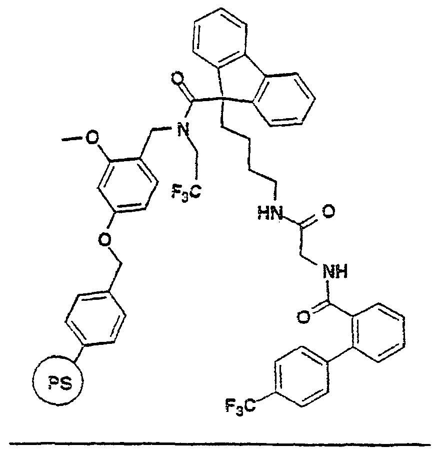

To the DMF swollen Part D resin were added solutions of 135 mg (1 mmol, 5 eq) of N-hydroxybenzotriazole in 1 mL of DMF, 266 mg (1 mmol, 5 eq) of 4'-(trifluoromethyl)-2-biphenylcarboxylic acid in 1 mL of DMF and 126 mg (1 mmol, 5 eq) of diisopropylcarbodiimide in 0.5 mL of CH₂Cl₂. After shaking for 72 h at RT, the reaction solution was drained and the title resin was rinsed with DMF (1 x 2 mL) and CH₂Cl₂ (4 x 2 mL). The title resin was used in the next step without characterization.

### F.

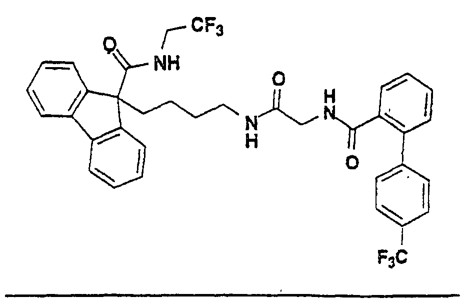

The Part E resin was treated with 2 mL of 100% triflucroacetic acid and shaken for 1 h. The cleavage solution was collected, the resin was rinsed with CH₂Cl₂ (2 x 1 mL) and the combined cleavage solution and rinses were concentrated on a Speed Vac at RT. After 18 h, the sample was reconstituted in 4 mL of CH₂Cl₂ and reconcentrated on the Speed Vac. After 18 h, the sample was again reconstituted in 4 mL of CH₂Cl₂ and aliquots were removed for HPLC and MS analysis. The tube was concentrated again on the Speed Vac followed by exposure to high vacuum (1 mm Hg) on a lyophilizer for 14 h to afford 110 mg (82% yield based on starting aldehyde resin) of title compound as clear yellow oil.
HPLC: retention time: 7.7 min; 86% purity. HPLC conditions: YMC S3 ODS 4.6 x 50 mm
Rapid Resolution column; linear gradient from 20% B to 100% B over 8 min and held at 100% B for 2 min (method name: SMET2); flow rate 2.5 mL/min; detection at 215 nm; Solvent A: 90% H₂O/10% MeOH with 0.2% H₃PO₄; Solvent B: 90% MeOH/10% H₂O with 0.2% H₃PO₄.
MS (electrospray, pos. ions): m/z 668 (M + H)

### Example 629

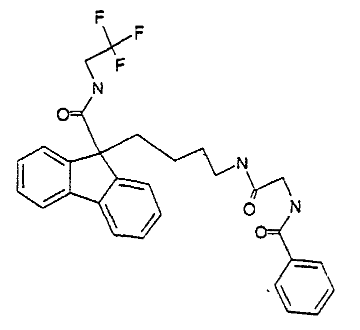

MS: m/z 524 (M+H)

### Example 630

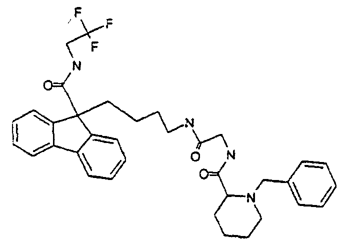

MS: m/z 621 (M+H)

### Example 631

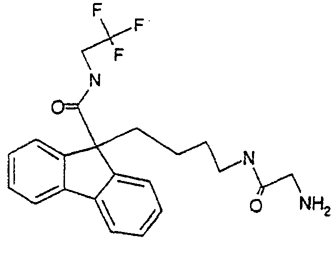

MS: m/z 420 (M+H)

### Example 632

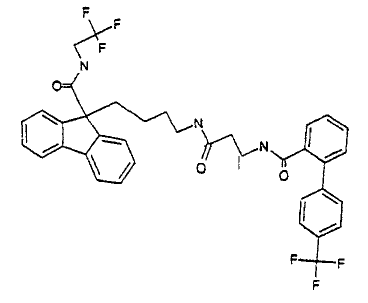

MS: m/z 682 (M+H)

### Example 633

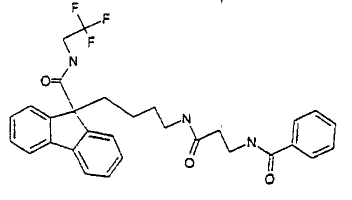

MS: m/z 538 (M+H)

### Example 634

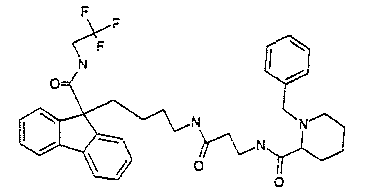

MS: m/z 635 (M+H)

### Example 635

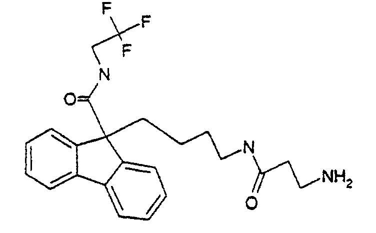

MS: m/z 434 (M+H)

### Example 636

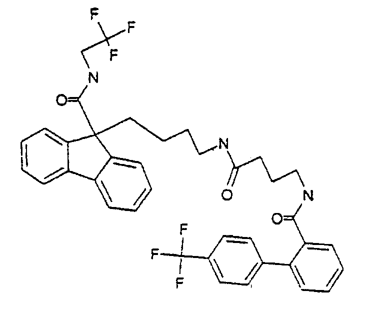

MS: m/z 696 (M+H)

### Example 637

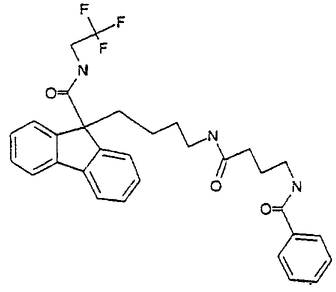

MS: m/z 552 (M+H)

### Example 638

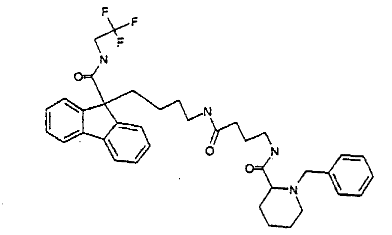

MS: m/z 649 (M+H)

### Example 639

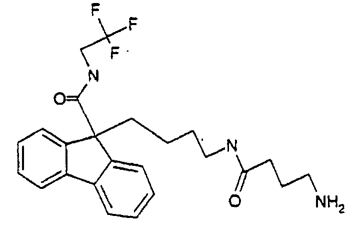

MS: m/z 448 (M+H)

### Example 640

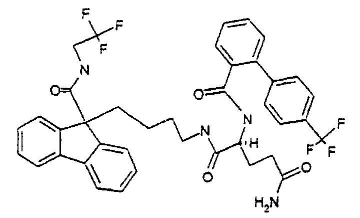

MS: m/z 739 (M+H)

### Example 641

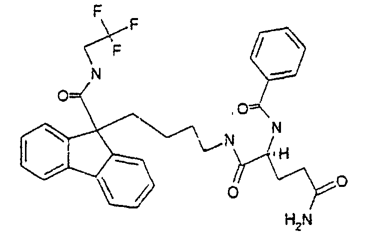

MS: m/z 595 (M+H)

### Example 642

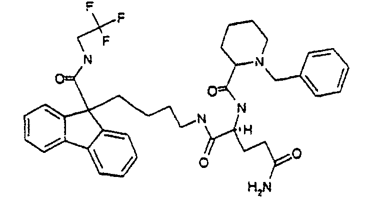

MS: m/z 692 (M+H)

### Example 643

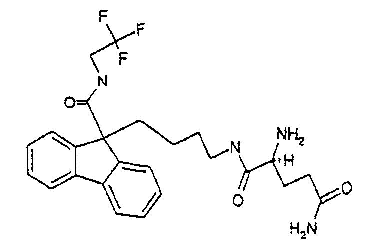

MS: m/z 491 (M+H)

### Example 644

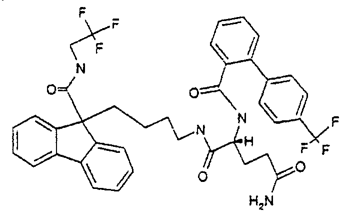

MS: m/z 739 (M+H)

### Example 645

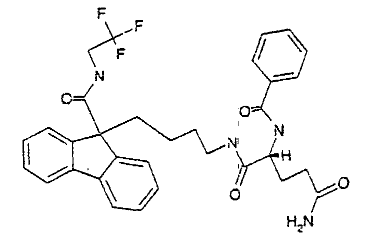

MS: m/z 595 (M+H)

### Example 646

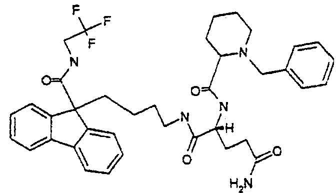

MS: m/z 692 (M+H)

### Example 647

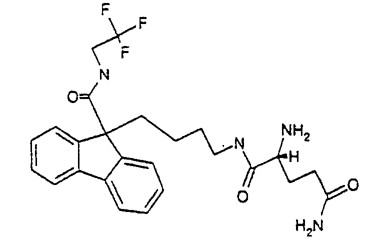

MS: m/z 491 (M+H)

### Example 648

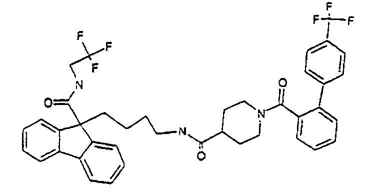

MS: m/z 722 (M+H)

### Example 649

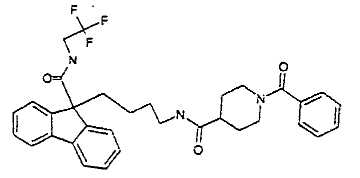

MS: m/z 578 (M+H)

### Example 650

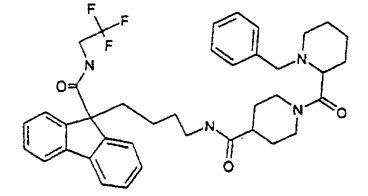

MS: m/z 675 (M+H)

### Example 651

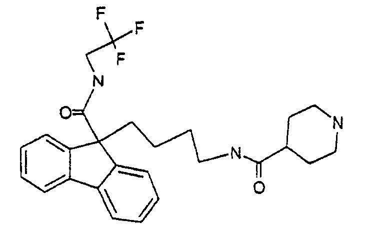

MS: m/z 474 (M+H)

### Example 652

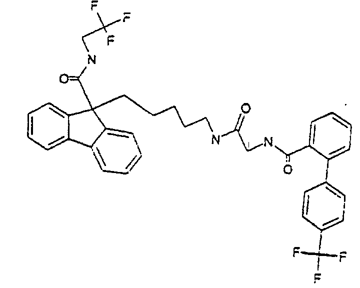

MS: m/z 682 (M+H)

### Example 653

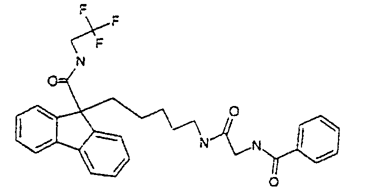

MS: m/z 538 (M+H)

### Example 654

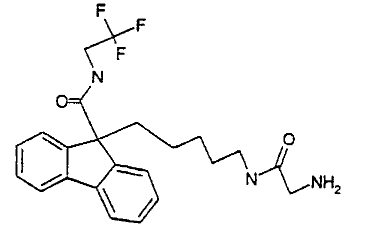

MS: m/z 434 (M+H)

### Example 655

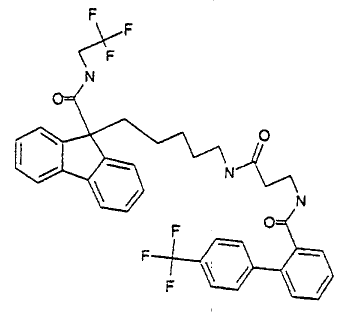

MS: m/z 696 (M+H)

### Example 656

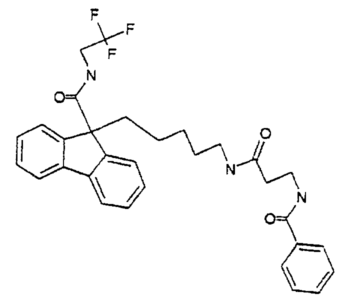

MS: m/z 552 (M+H)

### Example 657

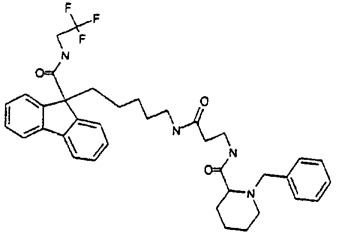

MS: m/z 649 (M+H)

### Example 658

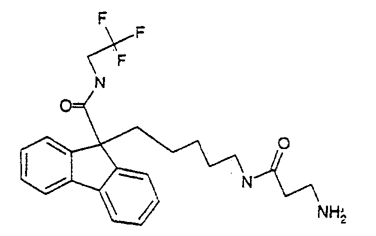

MS: m/z 448 (M+H)

### Example 659

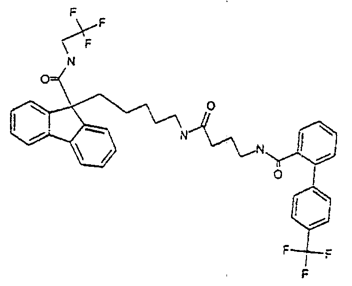

MS: m/z 710 (M+H)

### Example 660

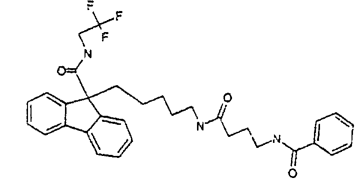

MS: m/z 566 (M+H)

### Example 661

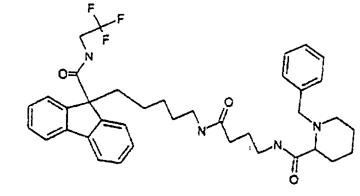

MS: m/z 663 (M+H)

### Example 662

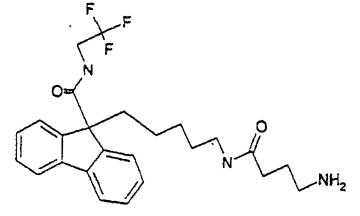

MS: m/z 462 (M+H)

### Example 663

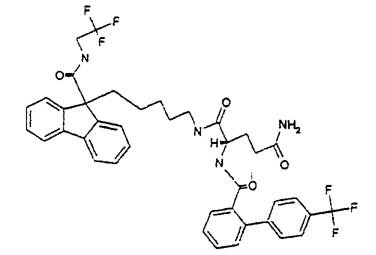

MS: m/z 753 (M+H)

### Example 664

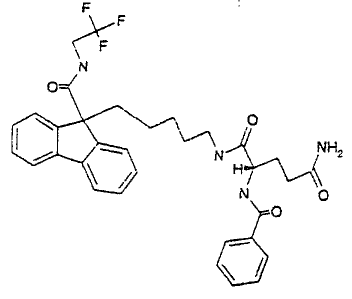

MS: m/z 609 (M+H)

### Example 665

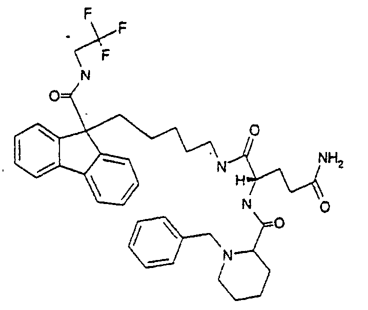

MS: m/z 706 (M+H)

### Example 666

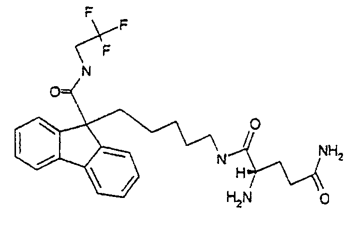

MS: m/z 505 (M+H)

### Example 667

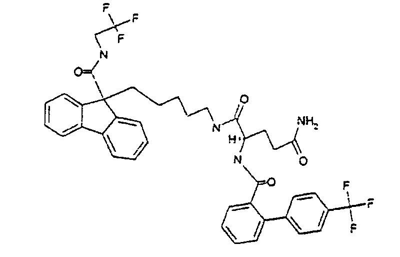

MS: m/z 753 (M+H)

### Example 668

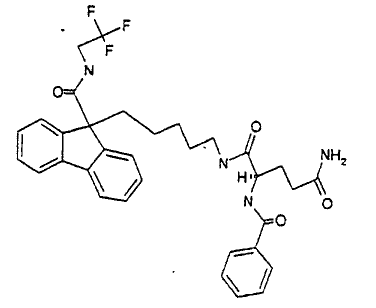

MS: m/z 609 (M+H)

### Example 669

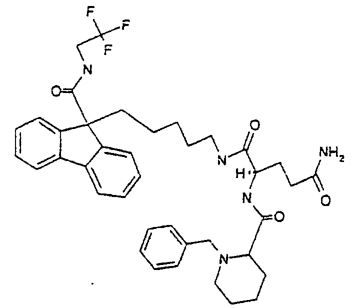

MS: m/z 706 (M+H)

### Example 670

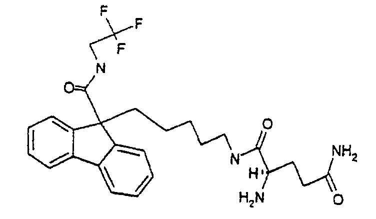

MS: m/z 505 (M+H)

### Example 671

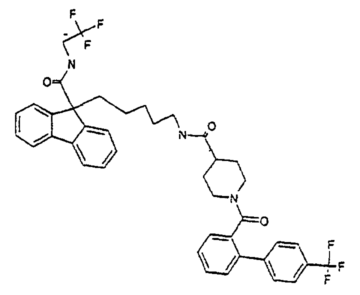

MS: MS: m/z 736 (M+H)

### Example 672

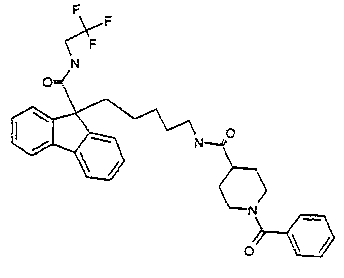

MS: m/z 592 (M+H)

### Example 673

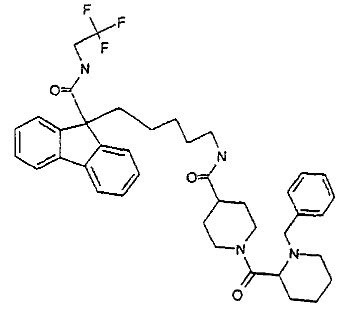

MS: m/z 689 (M+H)

### Example 674

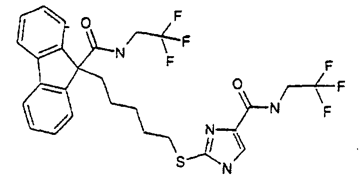

MS: m/z 585 (M+H)

### Example 675

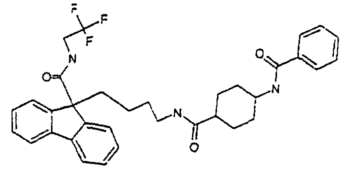

MS: m/z 592 (M+H)

### Example 676

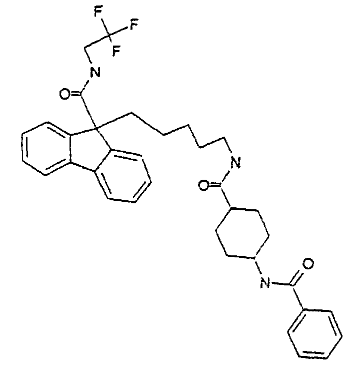

MS: m/z 606 (M+H)

### Example 677

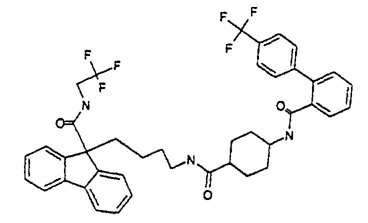

MS: m/z 736 (M+H)

### Example 678

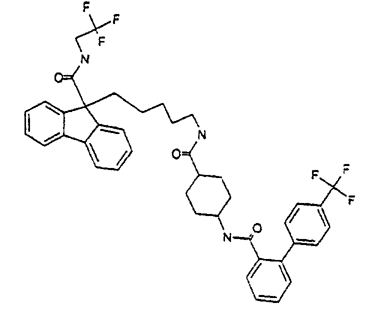

MS: m/z 750 (M+H)

### Example 679

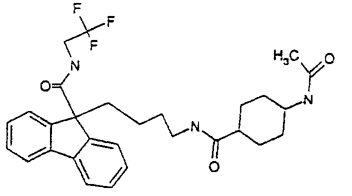

MS: m/z 530 (M+H)

### Example 680

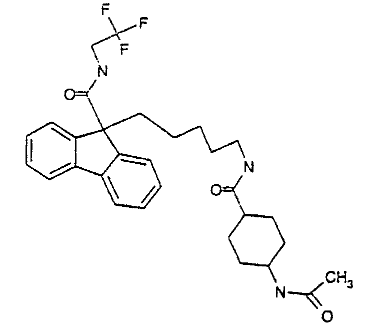

MS: m/z 544 (M+H)

### Example 681

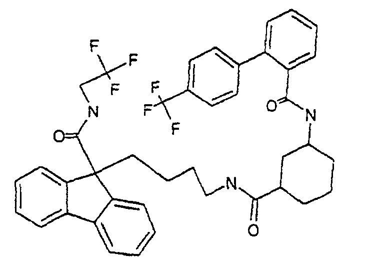

MS: m/z 736 (M+H)

### Example 682

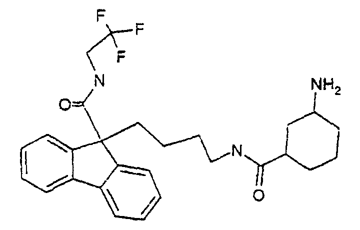

MS: m/z 488 (M+H)

### Example 683

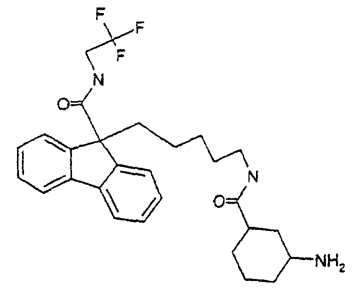

MS: m/z 502 (M+H)

### Example 684

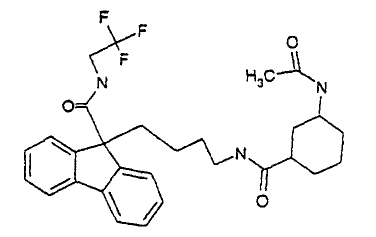

MS: m/z 530 (M+H)

### Example 685

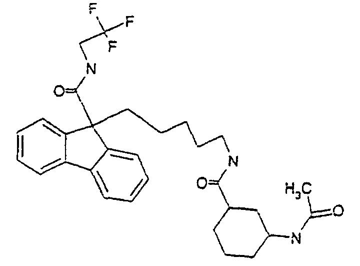

MS: m/z 544 (M+H)

### Example 686

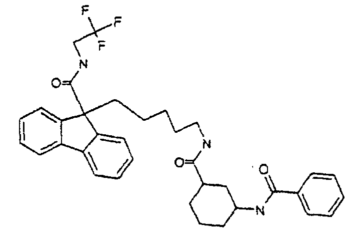

MS: m/z 606 (M+H)

### Example 687

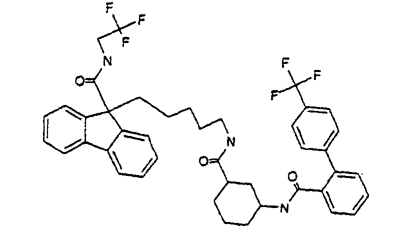

MS: m/z 750 (M+H)

### Example 688

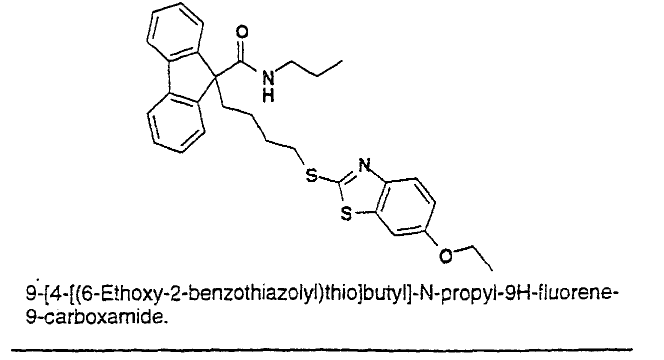

### A.

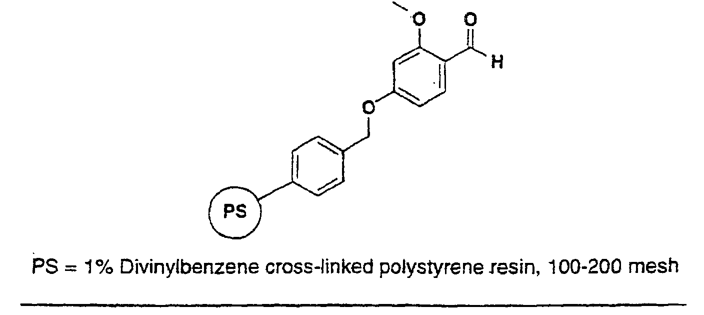

To a magnetically stirred suspension of 4.8 g (120 mmol, 10 eq) of sodium hydride (60% mineral oil dispersion) in 30 mL of dimethylformamide (DMF) at 0 °C was added a solution of 18.2 g (120 mmol, 10 eq) of 4-hydroxy-2-methoxybenzaldehyde in 50 mL of DMF dropwise over 75 min. The reaction was allowed to warm to room temperature (RT) and stirred for an additional 75 min. The stirbar was removed and 10 g (12 mmol, 1 eq) of Merrifield resin (with a loading of 1.2 mmol/g (Advanced Chemtech)) was added. The flask was placed in a heating mantel mounted on a vortex mixer and heated at 70°C (internal temper-ature) while vortexing for 26 h. The contents of the reaction vessel were transferred to a large filter funnel with a scintered-glass frit (porosity C) and rinsed sequentially with DMF (3 x 100 mL), 1:1 DMF:water (3 x 100 mL), water (2 x 100 mL) and MeOH (5 x 100 mL). The resin was dried under high vacuum (0.1 mm Hg) for 72 h to afford 11.16 g (98% of expected weight) of title compound as a tacky non-freeflowing tan resin. The resin was characterized by gel-phase ¹³C-NMR and elemental analysis (chlorine and oxygen).
Elemental Analysis:
Chlorine: Expected 0% Cl for 100% loading; found 0.21%. Starting Cl content of resin was 4.26%. Residual Cl consistent with 95% resin loading.
Oxygen: Expected 5.76% for 100% loading; found 6.21%.

### B.

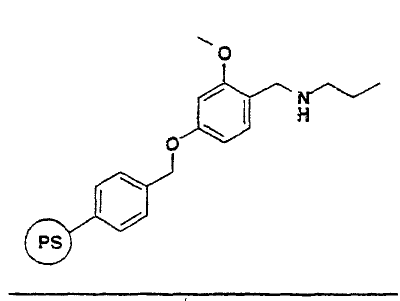

To a 25 mL Varian polypropylene tube fitted with a polyethylene frit and a luer stopcock was added 500 mg of Part A resin. The tube was sealed with a 19 mm Aldrich Suba septa and the resin was swollen in 5 mL of dry DMF, mixed by vortexing for 1 min and the DMF was removed using vacuum and N₂ pressure in order to maintain the vessel under inert atmosphere. Trimethyl orthoformate (1 mL) was added followed by 3.2 mL of DMF and 0.8 mL (10.0 mmol, 18 eq) of n-propylamine. The reaction mixture was vortexed for 18 h at RT. After removal of the reaction solution by nitrogen pressure and vacuum, 5 mL of a 200 mg/mL solution of sodium triacetoxy-borohydride in DMF (1 g, 4.7 mmol, 8 eg) and 100 µL of acetic acid were added. The reaction mixture was vortexed for 8 h at RT. The reaction solution was removed and theso-formed title resin was rinsed with DMF (4 x 5 mL), 1:1 DMF:water (2 x 5 mL), water (1 x 5 mL), DMF (3 x 5 mL) and dichloromethane (CH₂Cl₂) (4 x 5 mL). The last CH₂Cl₂ rinse was done with dry CH₂Cl₂ in the tube with the septa in place using nitrogen gas and vacuum to filter away the solvent and keep the reaction vessel under inert atmosphere. The title resin was used in the next step without characterization.

### C.

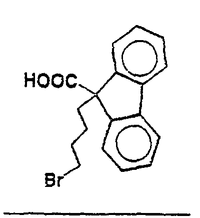

The title compound was prepared as described in Example 273 Part A(1).

### D.

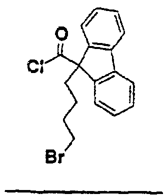

To 3.45 g (10 mmol, 1 eq) of 9-(4-bromobutyl)-9H-fluorene carboxylic acid (Part C) in 15 mL of CH₂Cl₂ was added 100 µL of DMF. The resulting solution was cooled to 0°C and 7.5 mL (15 mmol, 1.5 eq) of a 2.0 M oxalyl chloride solution in CH₂Cl₂ was added. The bubbling reaction mixture was stirred at 0°C for 15 min and then allowed to warm to RT. After 2 h, the reaction mixture was concentrated to afford the title crude acid chloride as a yellowish orange solid/oil mixture which was dissolved in CH₂Cl₂ and used without purification.

### E.

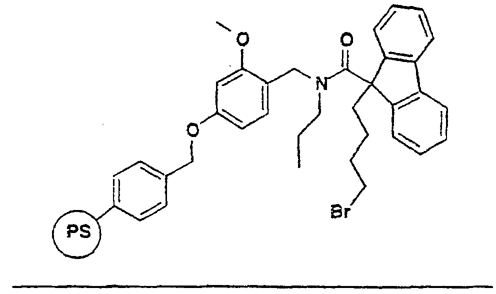

To the Part B resin in the polypropylene tube were added 1 mL of diisopropylethyl amine (5.7 mmol, 10 eq) and 1 mL of CH₂Cl₂ and the resulting mixture was mixed for 2 min. The tube was cooled to 0°C in an ice bath and 4 mL (2.2 mmol, 4 eq) of a solution of Part D acid chloride in CH₂Cl₂ was added. The resulting orange reaction mixture was mixed by vortexing at RT for 19 h. and then rinsed with CH₂Cl₂ (4 x 5 mL) to afford title resin which was in the next step without characterization.

### F.

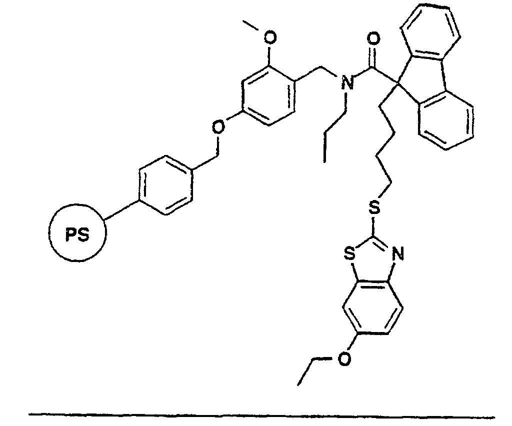

The Part E resin in the sealed. polypropylene tube was swollen in 5 mL of dry DMF and vortexed for 2 min. The solvent was removed with N₂ and vacuum and a solution of 1.16 g (5.5 mmol, 10 eq) of 6-ethoxy-2-mercaptobenzothiazole (Aldrich) in 4 mL of DMF was added to the resin followed by 5 mL (5 mmol, 9 eq) of a 1.0 M solution of sodium bistrimethyl-silylamide in THF.
Vortexing was initiated and the reaction mixture was mixed for 17 h at RT. The reaction solution was filtered away and the title resin was rinsed with DMF (4 x 5 mL), 1:1 DMF:water (2 x 5 mL), water (1 x 5 mL), DMF (3 x 5 mL) and dichloromethane (CH₂Cl₂) (4 x 5 mL).

### G.

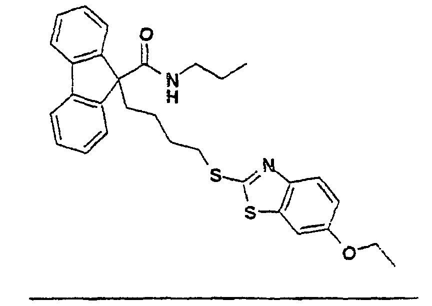

The Part F resin was treated with 5 mL of 100% trifluoroacetic acid and vortexed for 90 min. The reaction solution was collected, the resin was rinsed with CH₂Cl₂ (3 x 1 mL) and the combined reaction solution and rinses were concentrated. The products from 3 parallel reactions were each redissolved in 15 mL of CH₂Cl₂, pooled and reconcentrated to afford 393 mg (46% crude) of an off-white solid. Recrystallization from MeOH afforded 339 mg (40%) of title compound as a white solid.
m.p. 112-113.5°C
TLC (silica gel, 5% MeOH in CH₂Cl₂, UV and I₂)
R_{f} = 0.75;
IR(KBr): 3343, 2924, 1653, 1522, 1449, 1225, 739 cm⁻¹;
MS(electrospray, pos. ions): m/z 517 (M + H);

| | | | | |
|---|---|---|---|---|
| Anal. Calcd for C₃₀H₃₂N₂O₂S₂ | C, 69.73; | H, 6.24; | N, 5.42; | S, 12.41 |
| Found | C, 69.48; | H, 6.22; | N, 5.39; | S, 12.25. |

### Example 689

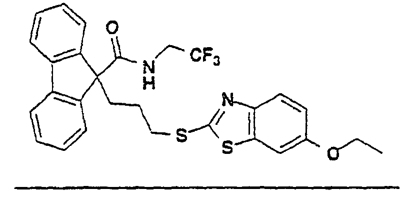

### A.

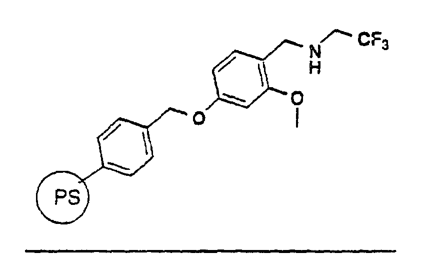

Example 688 Part A resin (250 mg, 0.3 mmol) was swollen in 3.0 mL of dimethylformamide (DMF). The solvent was drained. and 406 mg (3.0 mmol, 10 eq) of trifluoroethylamine, 261 µL (1.5 mmol, 5 eq) of diisopropylethylamine, 0.5 mL of trimethylortho-formate and 1.8 mL of DMF were added. The reaction mixture was shaken on a vortex mixer for 3.5 hours. The reaction solution was drained and 2.5 mL of a 200 mg/mL solution of sodium triacetoxyborohydride (500 mg) and 100 µL of acetic acid were added. The mixture was shaken for 16 hours. The resin was rinsed with 3 x 3 mL of the following: DMF, 1:1 DMF:H₂O, H₂O, DMF, followed by 5 x 3 mL each of CH₂Cl₂ and CH₃OH. The resin was dried under vacuum to provide 262 mg of title compound as a white resin.

### B.

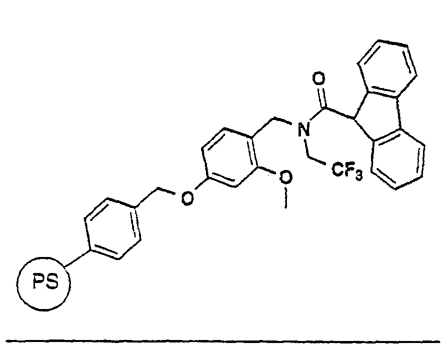

The Part A resin (262 mg, 0.3 mmol) was swollen in 3.0 mL of methylene chloride. A solution of 204 mg of 1-hydroxy-7-azabenzotriazole (1.5 mmol, 5 eq) and 315 mg of 9-fluorenecarboxylic acid (1.5 mmol, 5 eq) in 1.0 mL of DMF and 2.0 mL of methylene chloride was treated with 235 µL of diisopropyl-carbodiimide (1.5 mmol, 5 eq). The resin was drained, the reagent solution was added and the mixture was shaken for 17 hours. The reaction solution was drained and rinsed with 3 x 3 mL of the following: DMF, 1:1 DMF:H₂O, H₂O, DMF, followed by 5 x 3 mL each of CH₂Cl₂ and CH₃OH. The resin was dried under vacuum to provide 356 mg of title compound as a yellow resin.

### C.

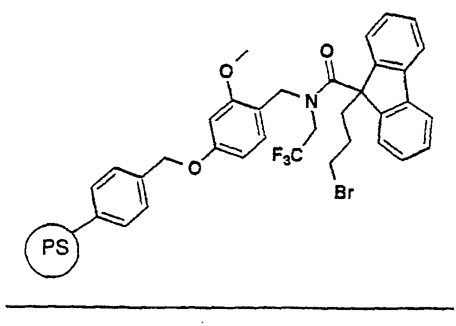

The Part B resin (323 mg, 0.27 mmol) was swollen in 3.0 mL of DMF (new Sure-Seal) and then drained under an atmosphere of argon. DMF (2.5 mL) was added, followed by the dropwise addition of 324 µL (3.2 mmol, 1.2 eq) of a 1.0 M solution of sodium bis(trimethylsilyl)amide in tetrahydrofuran (THF). The reaction mixture was shaken under argon for two hours. The reaction solution was drained and the resin was rinsed with 6 x 3 mL of DMF maintaining an argon atmosphere. The resin was suspended in 2.5 mL of DMF and 137 µL of 1,3 dibromopropane (1.35 mmol, 5 eq) was added. The mixture was shaken for 4 hours. The reaction solution was drained and the resin was rinsed with 3 x 3 mL of the following: DMF, 1:1 DMF:H₂O, H₂O, followed by 4 x 3 mL of DMF to provide title resin, used as is in the next step.

### D.

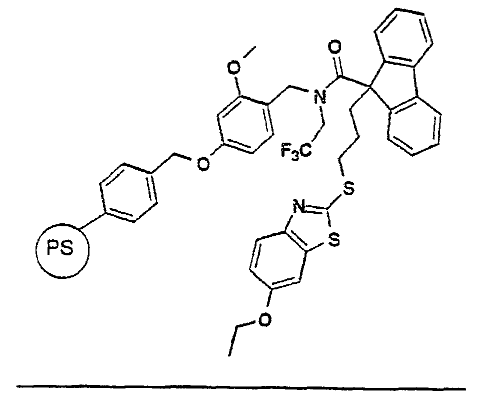

The Part C resin (0.27 mmol) was swollen in 3.0 mL of DMF. The solvent was drained and a solution of 570 mg of 6-ethoxy-2-mercaptobenzothiazole (2.7 mmol, 10 eq) in 3.0 mL of DMF was added, followed by the dropwise addition of 2.7 mL (2.7 mmol, 10 eq) of a 1.0 M solution of sodium bis(trimethylsilyl)amide in THF. After the addition was completed, the mixture was shaken for 14 hours. The resin was rinsed with 3 x 3 mL of the following: DMF, 1:1 DMF:H₂O, H₂O, DMF, followed by 8 x 3 mL of CH₂Cl₂ to provide title resin, used as is in the next step.

### E.

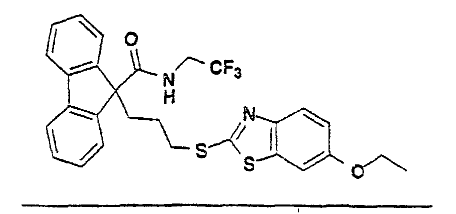

The Part D resin (0.27 mmol) was treated with 3.0 mL of trifluoroacetic acid for 90 minutes and then rinsed with methylene chloride, and the solutions were concentrated to provide 86 mg (58%) of a brown solid. This solid was combined with another batch of product prepared by the same route and purified by flash chromatography on silica gel (50 g) packed, loaded, and eluted with 25% hexane in methylene chloride followed by 100% methylene chloride. The 100% methylene chloride fractions were concentrated to provide 198 mg of title compound as an off-white foam.
TLC Silica gel (9:1 methylene chloride/hexane, visualization by UV) R_{f} = 0.25.
HPLC Purity = 97%. Retention time = 9.0 min. Column: Zorbax SB- C18 Rapid Resolution 4.6 x 75 mm. Solvent A: 10% methanol:90% water:0.2% H₃PO₄. Solvent B: 90% methanol:10% water:0.2% H3PO4. Elution: Linear gradient from 20 to 100% B over 8 minutes followed by isocratic 100% B for 2 minutes (Short Method 2-SMET2).
MS (ESI, + ions): m/z 543 (M + H).
IR (KBr) 2930, 1684, 1601, 1512, 1449, 1273, 1223, 1163, 1038, 997, 745 cm⁻¹.

| | | | | | |
|---|---|---|---|---|---|
| Anal. Calcd for C₂₈H₂₅N₂O₂S₂F₃ | C, 61.98; | H, 4.64; | N, 5.16; | S, 11.82; | F, 10.50 |
| Found | C, 61.90; | H, 4.72; | N, 5.06; | S, 12.09; | F, 10.23. |

## Claims

1. A compound which has the structure 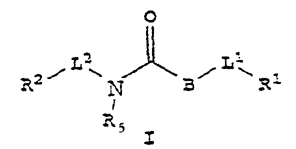 including pharmaceutically acceptable salts thereof, N-oxides thereof,
wherein q is 0, 1 or 2;
where R⁵ is H or C₁-C₁₂ alkyl, or R⁵ together with R² forms a carbocyclic or heterocyclic ring system containing 4 to 8 members in the ring;
B is a fluorenyl-type group of the structure 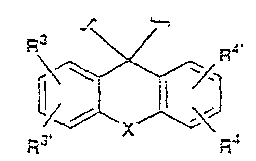 R¹ is H, alkyl, alkenyl, alkynyl, alkoxyl, (alkyl or aryl)₃Si (where each alkyl or aryl group is independent), cycloalkyl, cycloalkenyl, substituted alkylamino, substituted arylalkylamino, aryl, arylalkyl, arylamino, aryloxy, heteroaryl, heteroarylamino, heteroaryloxy, arylsulfonylamino, heteroarylsulfonylamino, arylthio, arylsulfinyl, arylsulfonyl, alkylthio, alkylsulfinyl, alkylsulfonyl, cycloheteroalkyl, heteroarylthio, heteroarylsulfinyl, heteroarylsulfonyl, -PO(R¹³)(R¹⁴), (where R¹³ and R¹⁴ are independently alkyl, aryl, alkoxy, aryloxy, heteroaryl, heteroarylalkyl, heteroaryloxy, heteroarylalkoxy, cycloheteroalkyl, cycloheteroalkylalkyl, cycloheteroalkoxy, or cycloheteroalkylalkoxy); aminocarbonyl (where the amino may optionally be substituted with one or two aryl, alkyl or heteroaryl groups); cyano, 1,1-(alkoxyl or aryloxy)₃alkyl (where the two aryl or alkyl substituents can be independently defined, or linked to one another to form a ring connected to L¹ (or L² in the case of R²) at the 2-position); 1,3-dioxane or 1,3-dioxolane connected to L¹ (or L² in the case of R²) at the 4-position; the R¹ group may optionally be substituted with 1, 2, 3 or 4 substituents, which can be any of the R³ or R¹ groups or alkylcarbonylamino, cycloalkylcarbonylamino, arylcarbonylamino, heteroarylcarbonylamino, alkoxycarbonylamino, aryloxycarbonylamino, heteroaryloxylcarbonylamino, uriedo (where the uriedo nitrogens may optionally be substituted with alkyl, aryl or heteroaryl), heterocyclylcarbonylamino (where the heterocycle is connected to the carbonyl group via a nitrogen or carbon atom), alkylsulfonylamino, arylsulfonylamino, heteroarylsulfonylamino, 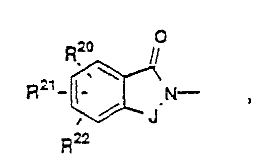 where J is: CHR²³, 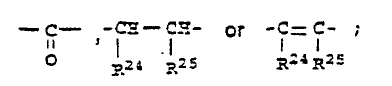 R²³, R²⁴ and R²⁵ are independently hydrogen, alkyl, alkenyl, alkynyl, aryl, arylalkyl, heteroaryl, heteroarylalkyl, cycloalkyl, or cycloalkylalkyl;
R²⁰, R²¹, R²² are independently hydrogen, halo, alkyl, alkenyl, alkoxy, aryloxy, aryl, arylalkyl, alkylmercapto, arylmercapto, cycloalkyl, cycloalkylalkyl, heteroaryl, heteroarylalkyl, hydroxy or haloalkyl; and these substituents may either be directly attached to R¹, or attached via an alkylene at an open position;
R² is independently any of the groups set out for R¹, H, polyhaloalkyl, or cycloheteroalkyl, and may be optionally substituted with one to four of any of the groups defined for R³ or substituents defined for R¹;
L¹ is a linking group containing from up to 10 carbons in a linear chain including alkylene, alkenylene or alkynylene, which may contain, within the linking chain any of the following: one or two alkenes, one or two alkynes, an oxygen, an amino group, an oxo group, and may be substituted with one to five alkyl or halo groups;
L² may be the same or different, from L¹ and may independently be any of the L¹ groups set out above or a singe bond;
R³, R³', R⁴ and R⁴' may be the same or different and are independently selected from H, halogen, CF₃, haloalkyl, hydroxy, alkoxy, alkyl, aryl, alkenyl, alkenyloxy, alkynyl, alkynyloxy, alkanoyl, nitro, amino, thiol, alkylthio, alkylsulfinyl, alkylsulfonyl, carboxy, alkoxycarbonyl, aminocarbonyl, alkylcarbcnyloxy, alkylcarbonylamino, cycloheteroalkyl, cycloheteroalkylalkyl, cyano, Ar-, Ar-alkyl, ArO, Ar-amino, Ar-thio, Ar-sulfinyl, Ar-sulfonyl, Ar-carbonyl, Ar-carbonyloxy or Ar-carbonylamino, wherein Ar is aryl or heteroaryl and Ar may optionally include 1, 2 or 3 additional rings fused to Ar;
X is a bond, or is one of the following groups:
(2) -O- ;
with the following provisos
(a) when R¹ is unsubstituted alkyl or unsubstituted arylalkyl, L¹ cannot contain amino;
(b) when R¹ is alkyl, L¹ cannot contain amino and oxo in adjacent positions (to form an amido group);
(d) when R¹ is cyano, L¹ must have more than 2 carbons;
(e) R¹L¹ must contain at least 3 carbons;
with respect to compounds, where R¹ or R² is cycloheteroalkyl, R¹ or R² is exclusive of 1-piperidinyl, 1-pyrrolidinyl, 1-azetidinyl or 1-(2-oxo-pyrrolidinyl), wherein the term
"alkyl" or "alk" as employed herein alone or as part of another group includes both straight and branched chain hydrocarbons, containing 1 to 40 carbons, the various branched chain isomers thereof, as well as such groups including 1 to 4 substituents which may be any of the R³ groups, or the R¹ substituents set out herein;
the term "cycloalkyl" as employed herein alone or as part of another group includes saturated or partially unsaturated (containing 1 or 2 double bonds) cyclic hydrocarbon groups containing 1 to 3 rings, including monocyclicalkyl, bicyclicalkyl and tricyclicalkyl, containing a total of 3 to 20 carbons forming the rings, preferably 4 to 12 carbons, forming the ring and which may be fused to 1 aromatic ring as described for aryl,
the term "cycloalkenyl" as employed herein alone or as part of another group refers to cyclic hydrocarbons containing 5 to 20 carbons, and 1 or 2 double bonds;
the term "polycycloalkyl" as employed herein alone or as part of another group refers to a bridged multicyclic group containing 5 to 20 carbons and containing 0 to 3 bridges;
the term "polycycloalkenyl" as employed herein alone or as part of another group refers to a bridged multicyclic group containing 5 to 20 carbons and containing 0 to 3 bridges and containing 1 or 2 double bonds;
the term "aryl" as employed herein alone or as part of another group refers to monocyclic and bicyclic aromatic groups containing 6 to 10 carbons in the ring portion and may optionally include one to three additional rings fused to Ar and may be optionally substituted through available carbon atoms with 1, 2, or 3 groups selected from hydrogen, halo, haloalkyl, alkyl, haloalkyl, alkoxy, haloalkoxy, alkenyl, trifluoromethyl, trifluoromethoxy, alkynyl, cyclo-alkylalkyl, cycloheteroalkyl, cycloheteroalkylalkyl, aryl, heteroaryl, arylalkyl, aryloxy, aryloxyalkyl, arylalkoxy, arylthio, arylazo, heteroarylalkyl, heteroarylalkenyl, heteroarylheteroaryl, heteroaryloxy, hydroxy, nitro, cyano, amino, substituted amino wherein the amino includes 1 or 2 substituents (which are alkyl, aryl or any of the other aryl compounds mentioned in the definitions) , thiol, alkylthio, arylthio, hetero-arylthio, arylthioalkyl, alkoxyarylthio, alkylcarbonyl, arylcarbonyl, alkyl-aminocarbonyl, arylaminocarbonyl, alkoxycarbonyl, aminocarbonyl, alkylcarbonyloxy, arylcarbonyloxy, alkylcarbonylamino, arylcarbonylamino, arylsulfinyl, arylsulfinylalkyl, arylsulfonylamino or arylsulfon-aminocarbonyl or any of the R³ groups, or the R¹ substituents set out herein;
the term "aralkyl", "aryl-alkyl" or "aryllower alkyl" as used herein alone or as part of another group refers to alkyl groups as discussed above having an aryl substituent,
the term "lower alkoxy", "alkoxy", "aryloxy" or "aralkoxy" as employed herein alone or as part of another group includes any of the above alkyl, aralkyl or aryl groups linked to an oxygen atom;
the term "amino" as employed herein alone or as part of another group may optionally be substituted with one or two substituents such as alkyl, aryl, arylalkyl, heteroaryl, heteroarylalkyl, cycloheteroalkyl, cycloheteroalkylalkyl and/or cycloalkyl;
the term "lower alkylthio", alkylthio", "arylthio" or "aralkylthio" as employed herein alone or as part of another group includes any of the above alkyl, aralkyl or aryl groups linked to a sulfur atom;
the term "lower alkylamino", "alkylamino", "arylamino", or "arylalkylamino" as employed herein alone or as part of another group includes any of the above alkyl, aryl or arylalkyl groups linked to a nitrogen atom;
the term "acyl" as employed herein by itself or part of another group, as defined herein, refers to an organic radical linked to a carbonyl 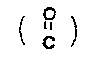 group;
the term "alkanoyl" as used herein alone or as part of another group refers to alkyl linked to a carbonyl group,
the term "alkenyl" as used herein by itself or as part of another group refers to straight or branched chain radicals of 2 to 20 carbons, which include one to six double bonds in the normal chain, and which may be optionally substituted with 1 to 4 substituents, namely, halogen, haloalkyl, alkyl, alkoxy, alkenyl, alkynyl, aryl, arylalkyl, cycloalkyl, amino, hydroxy, heteroaryl, cyclohetero-alkyl, alkanoylamino, alkylamido, arylcarbonylamino, nitro, cyano, thiol, alkylthio or any of the R³ groups, or the R¹ substituents set out herein;
the term "alkynyl" as used herein by itself or as part of another group refers to straight or branched chain radicals of 2 to 20 carbons, which include one triple bond in the normal chain, and which may be optionally substituted with 1 to 4 substituents, namely, halogen, haloalkyl, alkyl, alkoxy, alkenyl, alkynyl, aryl, arylalkyl, cycloalkyl, amino, heteroaryl, cycloheteroalkyl, hydroxy, alkanoylamino, alkylamido, arylcarbonylamino, nitro, cyano, thiol, and/or alkylthio, or any of the R³ groups, or the R¹ substituents set out herein;
the term "alkylene" as employed herein alone or as part of another group refers to alkyl groups as defined above having single bonds for attachment to, other groups at two different carbon atoms and may optionally be substituted as defined above for "alkyl";
the terms "alkenylene" and "alkynylene" as employed herein alone or as part of another group refer to alkenyl groups as defined above and alkynyl groups as defined above, respectively, having single bonds for attachment at two different carbon atoms, and
may optionally include 1, 2, or 3 substituents which include any of the R³ groups, or the R¹ substituents set out herein;
the term "halogen" or "halo" as used herein alone or as part of another group refers to chlorine, bromine, fluorine, and iodine as well as CF₃;
the term "metal ion" refers to alkali metal ions alkaline earth metal ions as well as zinc and aluminum;
the term "cycloheteroalkyl" as used herein alone or as part of another group refers to a 5-, 6- or 7-membered saturated or partially unsaturated ring which includes 1 to 2 hetero atoms, linked through a carbon atom or a heteroatom, where possible, optionally via the linker (CH₂)ₚ (which is defined above);
the term "heteroaryl" as used herein alone or as part of another group refers to a 5- or 6-membered aromatic ring which includes 1, 2, 3 or 4 hetero atoms fused to an aryl, cycloalkyl, heteroaryl or cycloheteroalkyl ring and includes possible N-oxides;
Ar may be either aryl or heteroaryl as defined above;
the term "cycloheteroalkylalkyl" as used herein alone or as part of another group refers to cycloheteroalkyl groups as defined above linked through a C atom or heteroatom to a (CH₂)ₚ chain;
the term "heteroarylalkyl" or "heteroarylalkenyl" as used herein alone or as part of another group refers to a heteroaryl group as defined above linked through a C. atom or heteroatom to a -(CH₂)ₚ-chain, alkylene or alkenylene as defined above and wherein
the term "polyhaloalkyl" as used herein refers to an "alkyl" group as defined above which includes from 2 to 9, halo substituents.

2. The compound as defined in Claim 1 having the formula
wherein B is 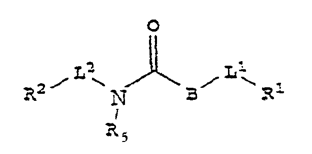 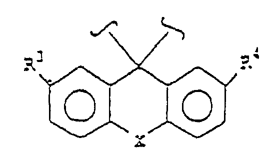 A is NH;
X is a bond, oxygen or sulfur;
R³ and R⁴ are the same or different and are H or F;
R¹ is aryl, phenyl, heteroaryl, imidazolyl, pyridyl, cyclohexyl, PO(R¹³)(R¹⁴), heteroarylthio, benzimidazolyl, indolyl, benzthiazole-2-thio, imidazole-2-thio, alkyl, alkenyl or 1,3-dioxan-2-yl,
R² is alkyl, polyfluoroalkyl, alkenyl, aryl, phenyl, heteroaryl, imidazolyl or pyridyl,
L¹ is a chain containing 1 to 5 atoms in a linear chain;
L² is a bond or alkylene containing up to 12 carbon atoms.

3. The compound as defined in Claim 1 which is N-(phenylmethyl)-9-(3-phenylpropyl)-9H-fluorene-9-carboxamide; 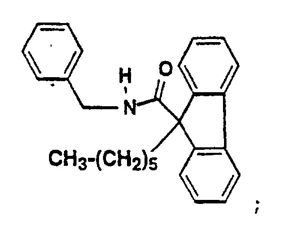 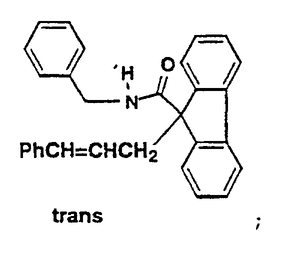 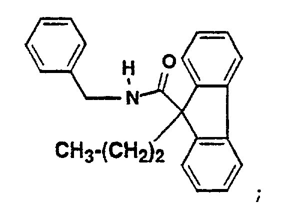 (E)-N-ethyl-9-(3-phenyl-2-propenyl)-9H-fluorene-9-carboxamide; 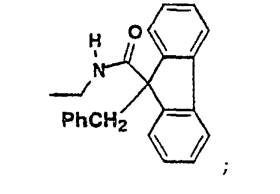 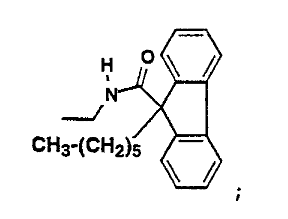 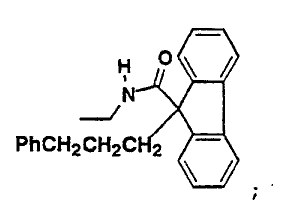 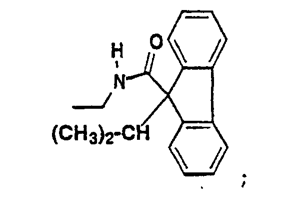 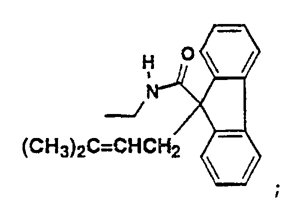 9-[4-(dibutoxyphosphinyl)butyl]-N-propyl-9H-fluorene-9-carboxamide;
(E)-9-(3-phenyl-2-propenyl)-N-propyl-9H-fluorene-9-carboxamide; 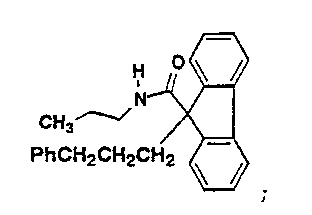 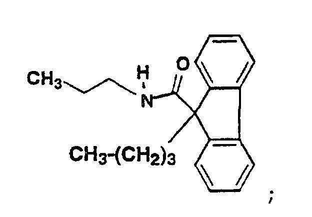 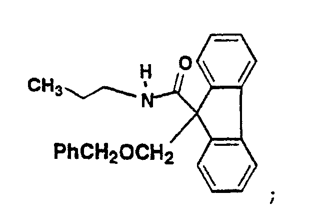 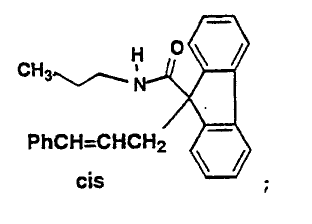 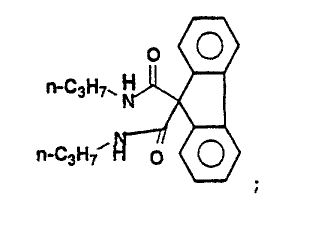 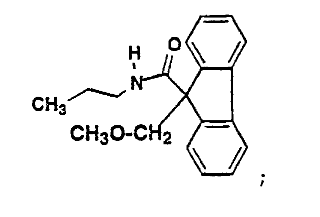 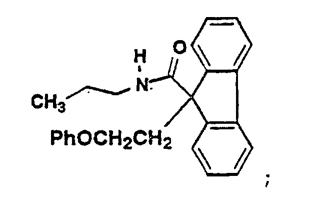 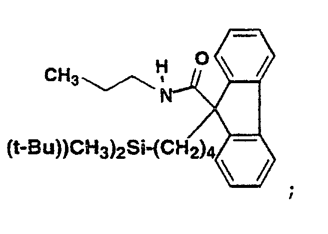 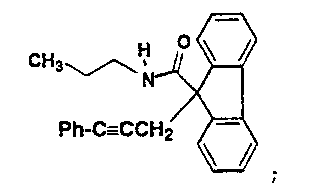 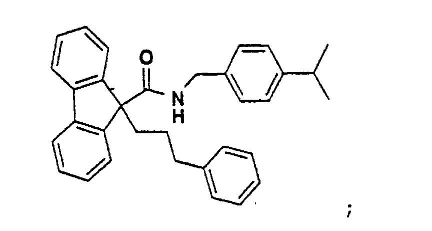 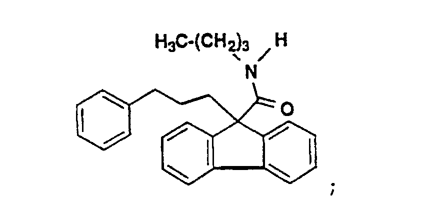 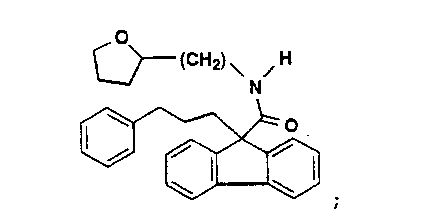 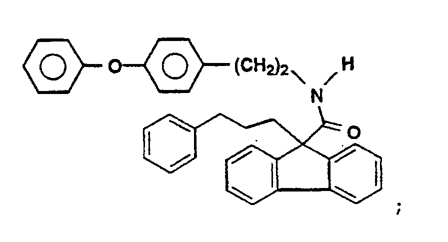 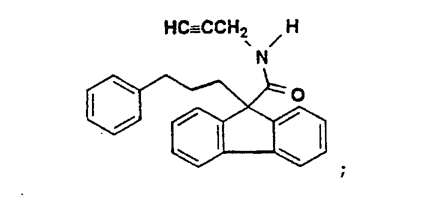 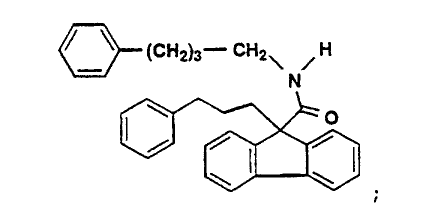 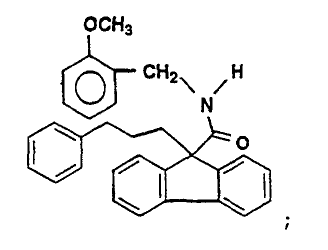 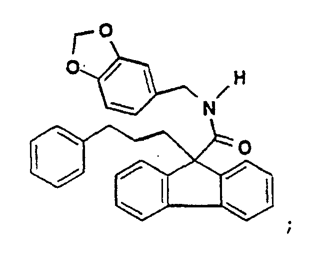 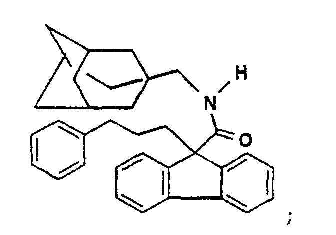 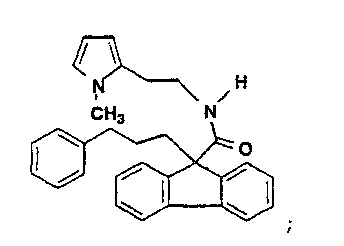 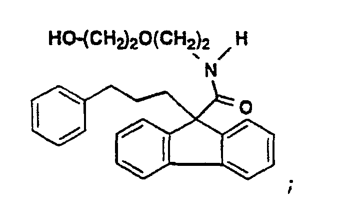 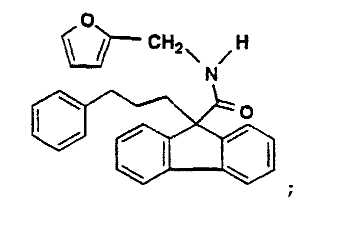 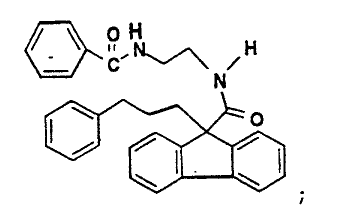 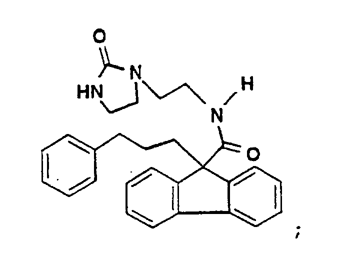 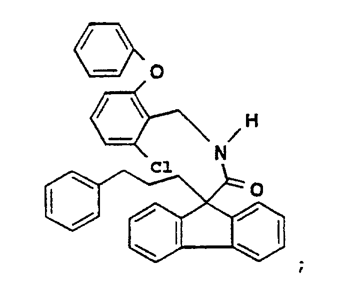 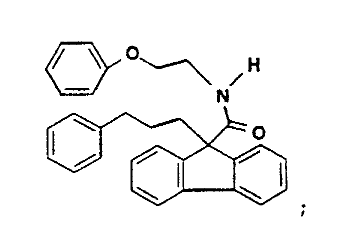 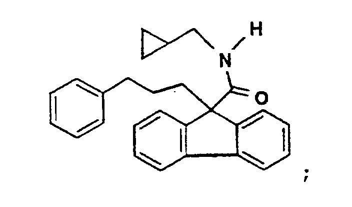 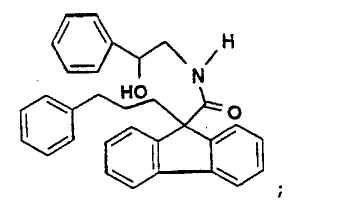 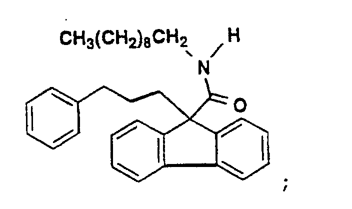 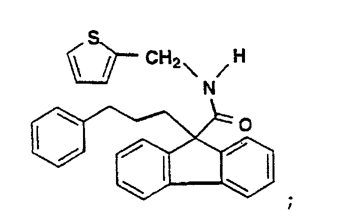 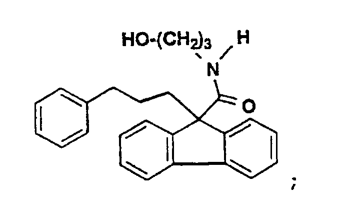 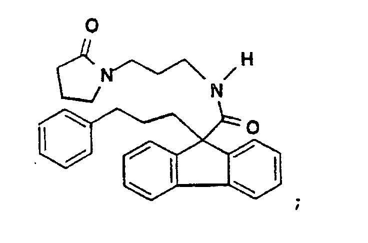 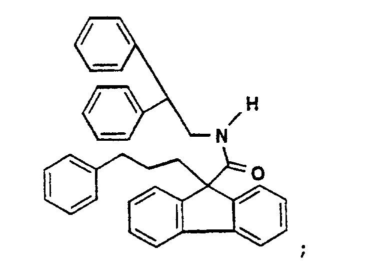 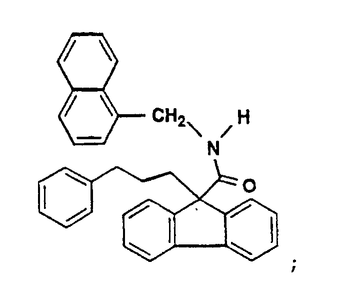 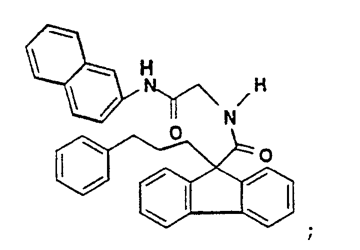 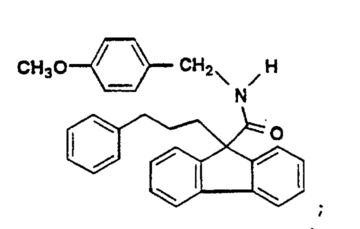 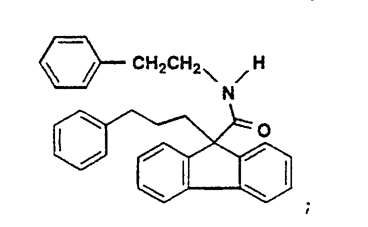 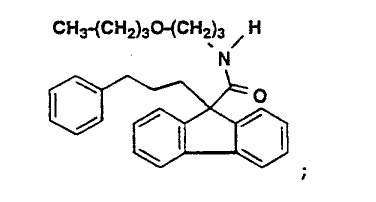 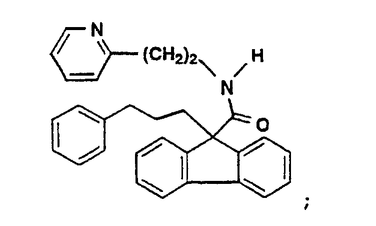 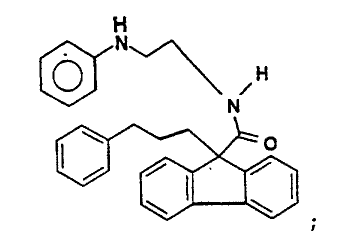 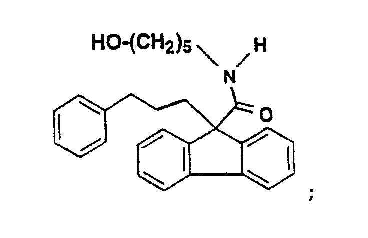 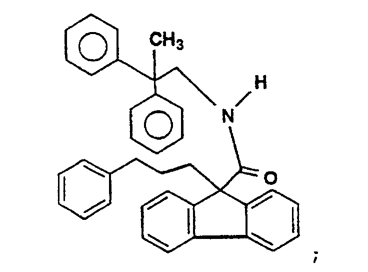 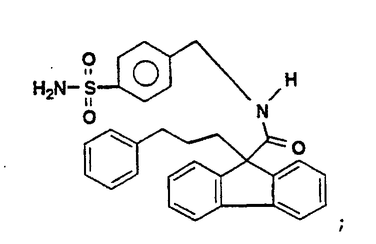 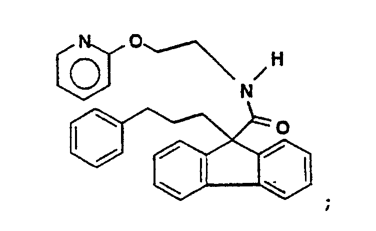 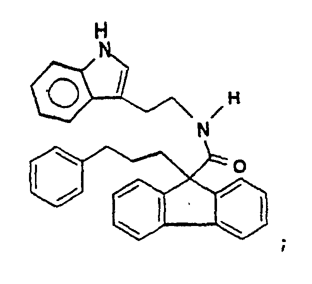 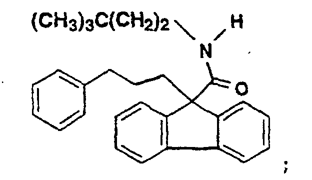 9-(3-phenylpropyl)-N-(2,2,2-trifluoroethyl)-9H-fluorene-9-carboxamide; 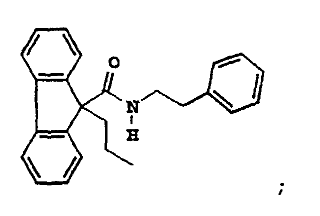 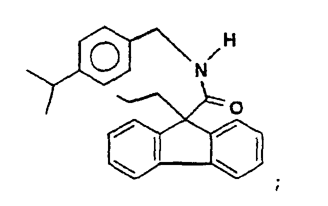 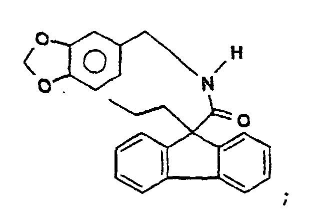 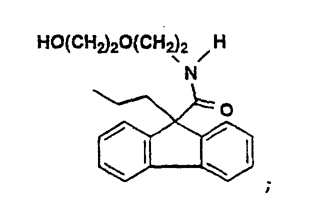 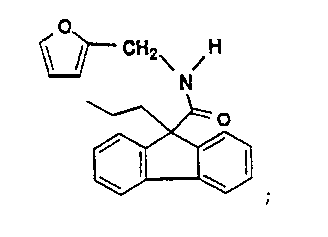 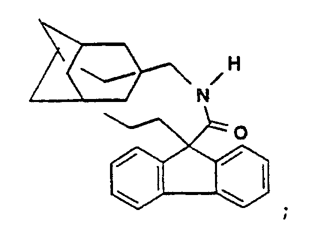 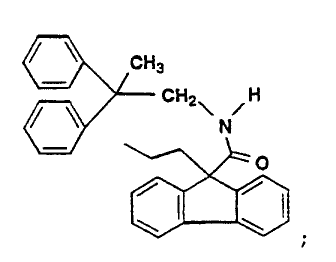 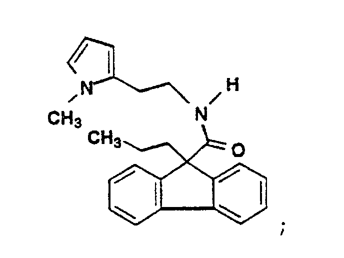 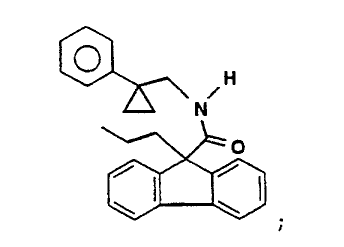 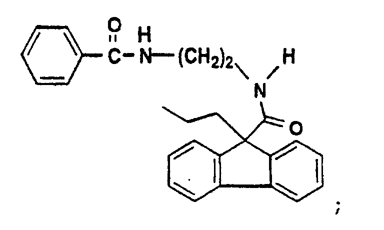 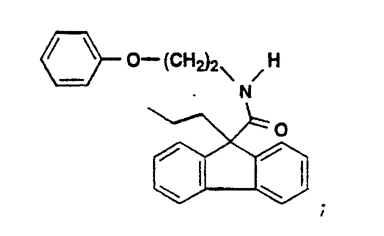 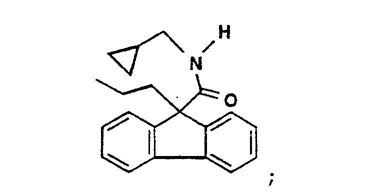 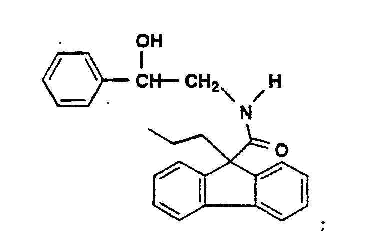 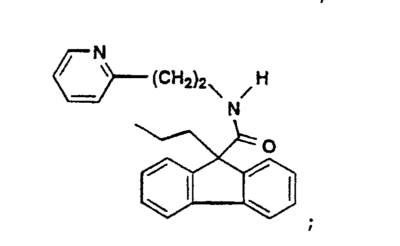 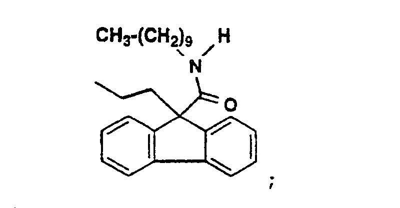 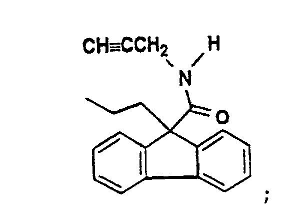 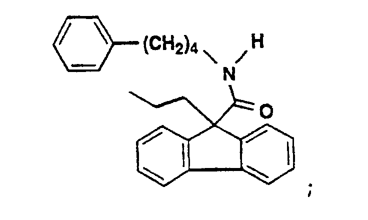 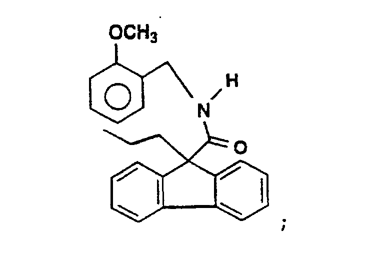 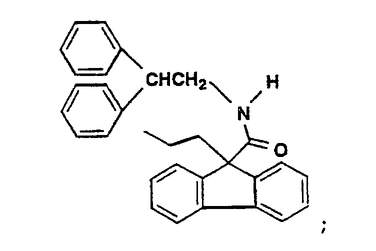 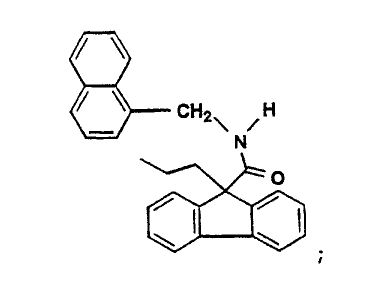 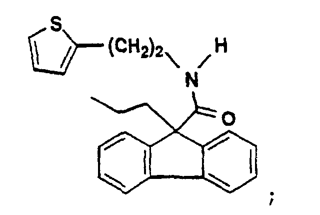 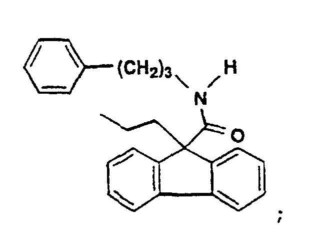 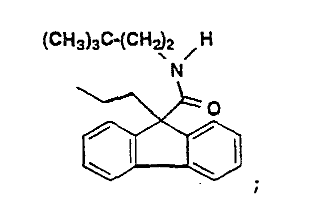 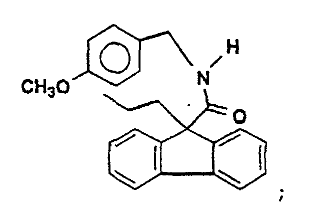 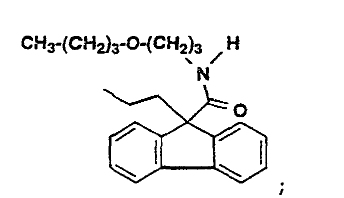 N-methyl-N-(phenylmethyl)-9-propyl-9H-fluorene-9-carboxamide; 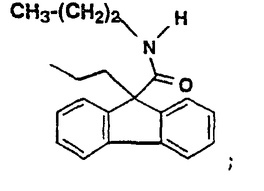 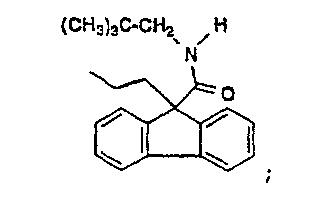 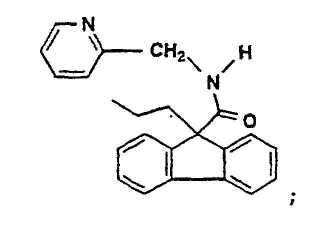 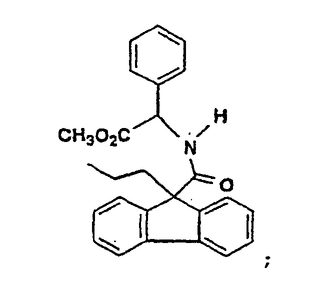 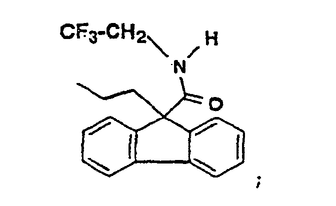 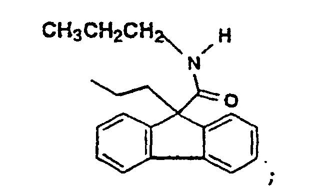 9-(2-propenyl)-N-(2-pyridinylmethyl)-9H-fluorene-9-carboxamide;
N-butyl-9-(2-propenyl)-9H-fluorene-9-carboxamide;
9-[2,2-bis(trifluoromethyl)-1,3-dioxolan-4-yl]methyl-N-ethyl-9H-fluorene-9-carboxamide;
9-(2,3-dihydroxypropyl)-N-ethyl-9H-fluorene-9-carboxamide;
9-(3-phenylpropyl)-N-(3-hydroxy)propyl-9H-xanthene-9-carboxamide; 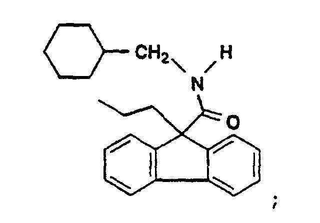 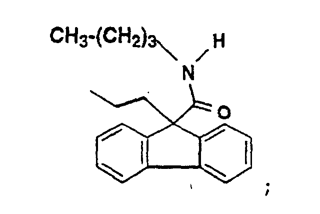 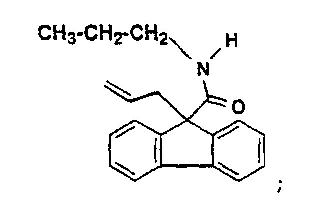 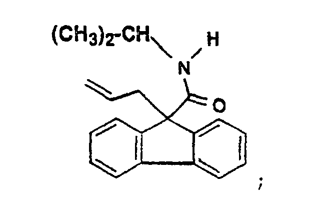 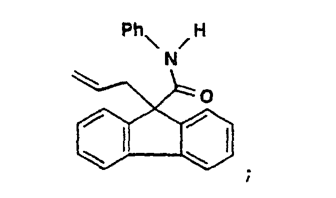 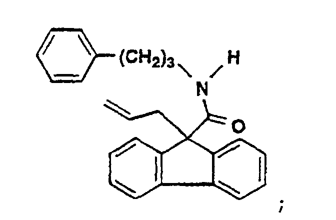 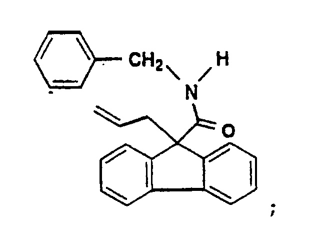 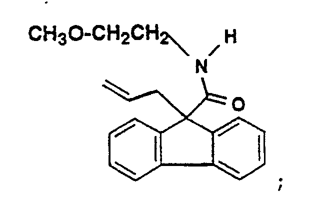 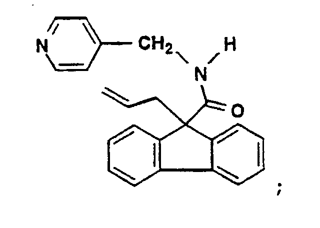 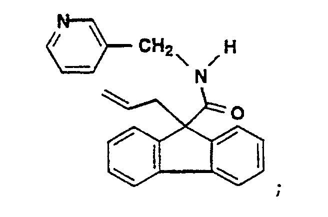 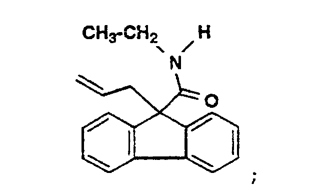 9-(1-piperidinylcarbonyl)-9-(2-propenyl)-9H-fluorene: 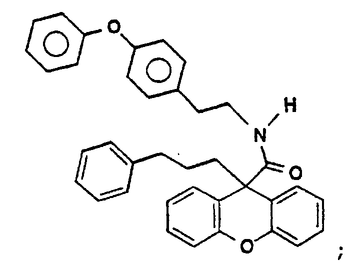 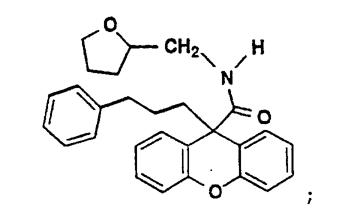 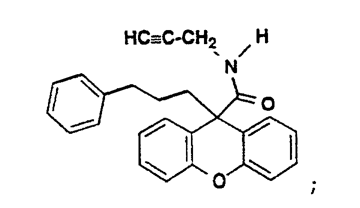 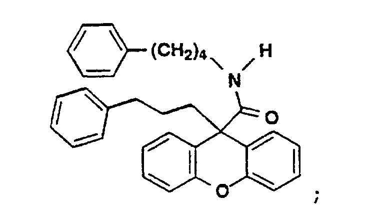 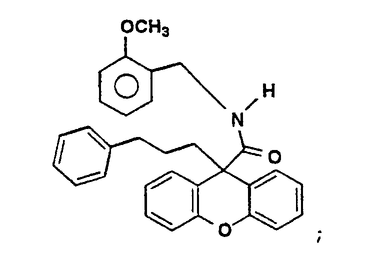 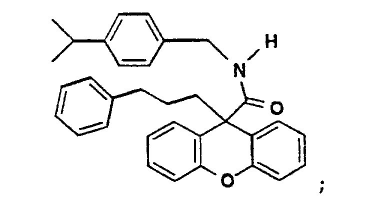 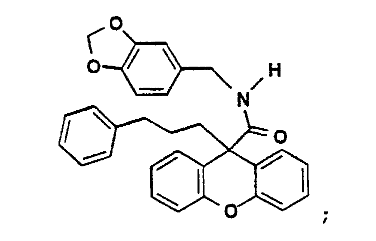 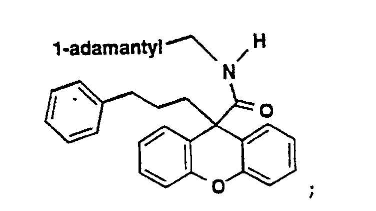 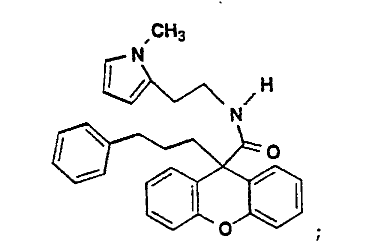 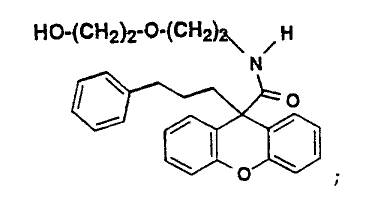 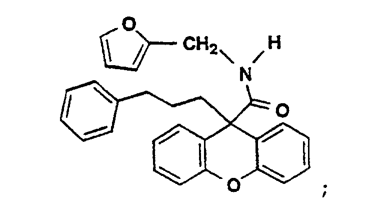 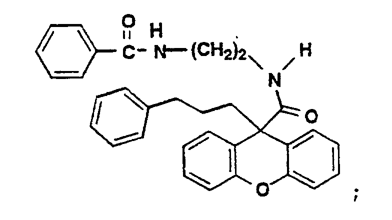 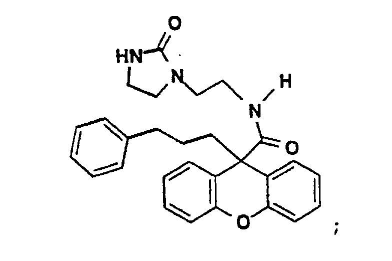 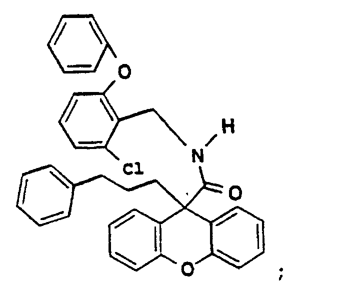 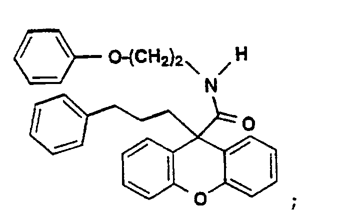 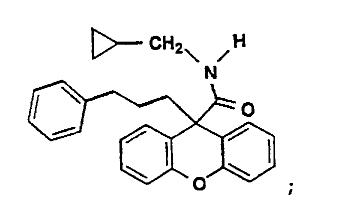 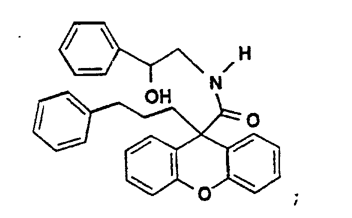 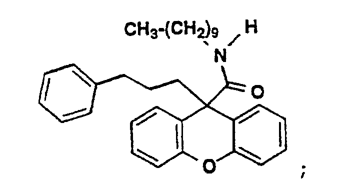 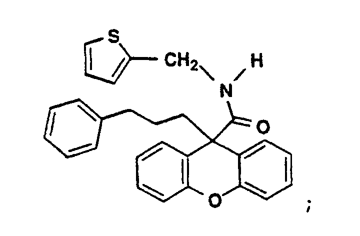 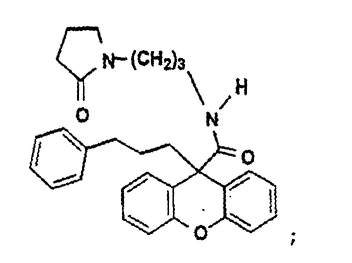 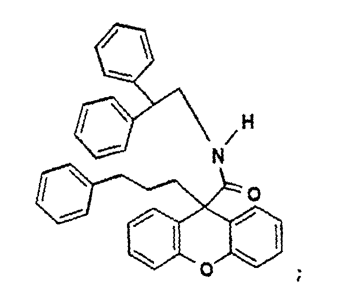 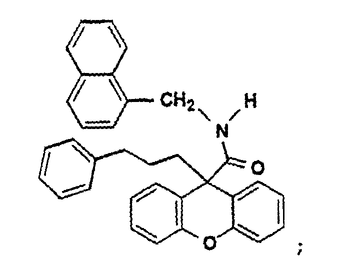 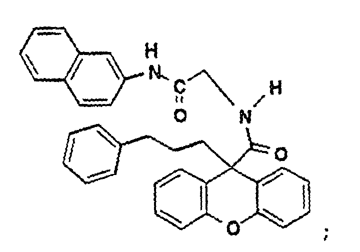 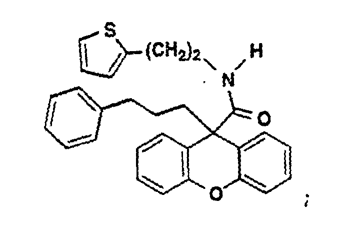 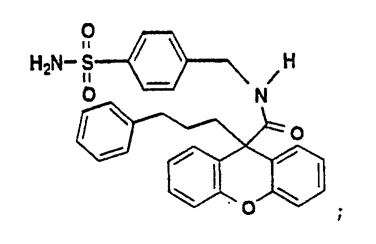 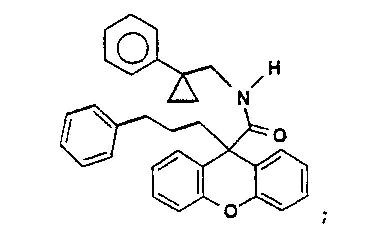 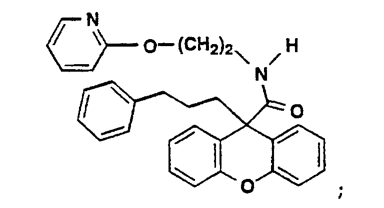 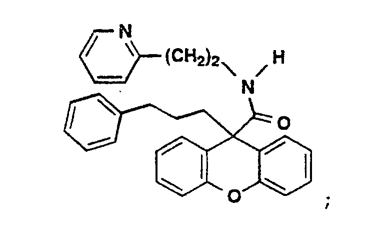 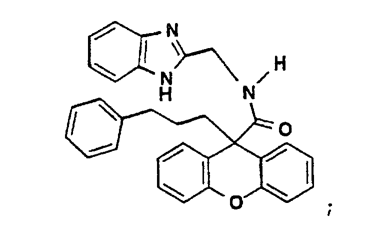 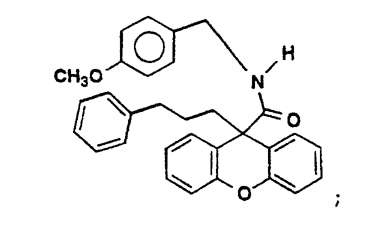 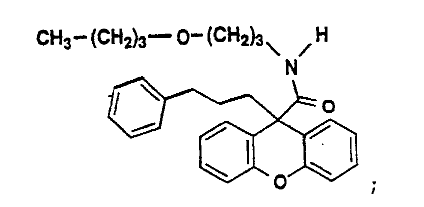 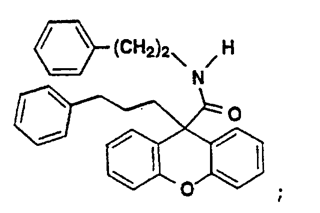 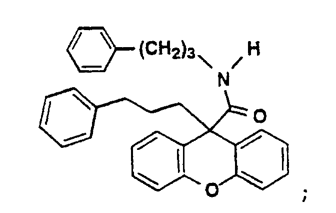 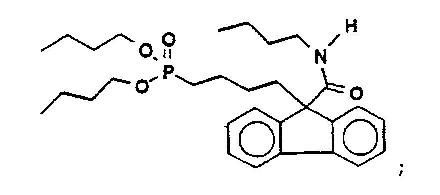 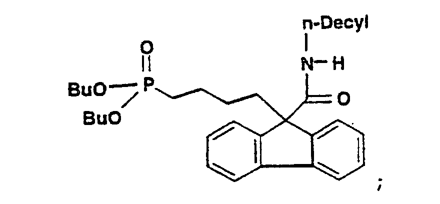 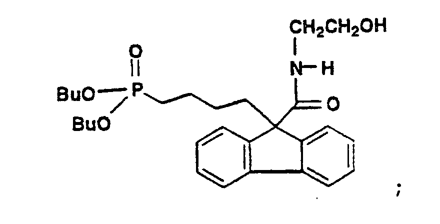 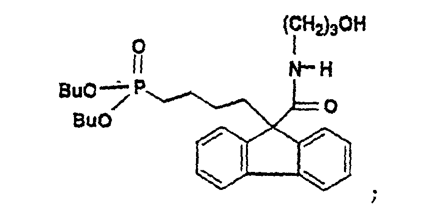 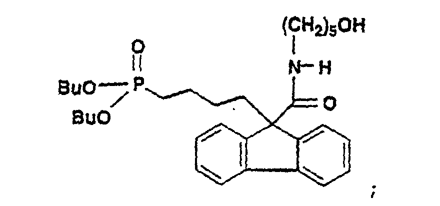 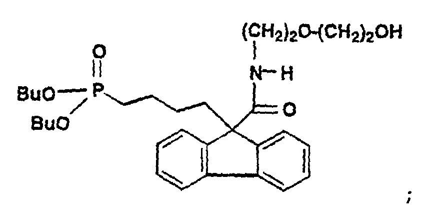 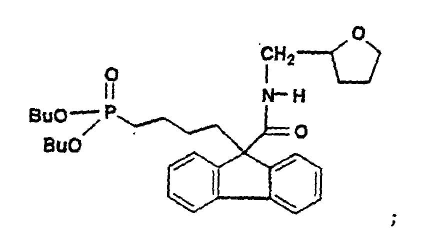 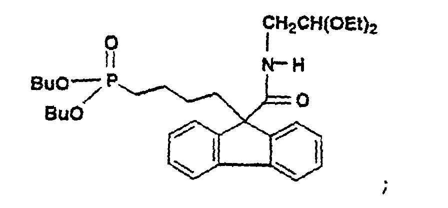 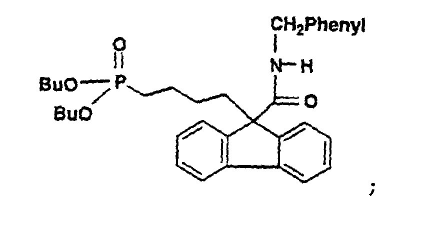 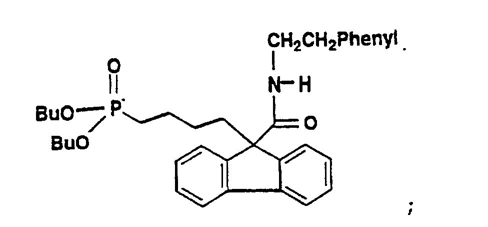 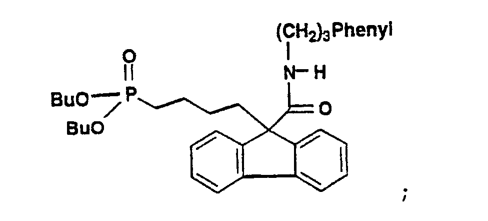 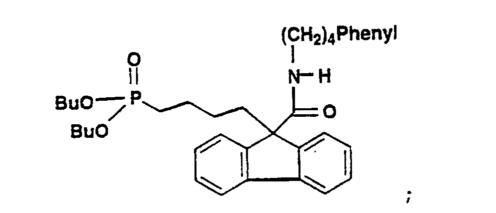 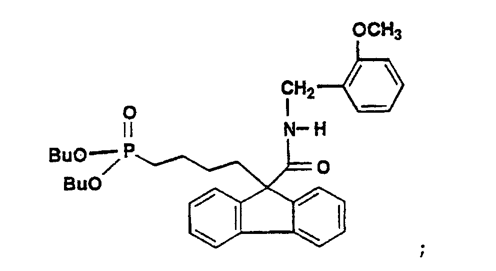 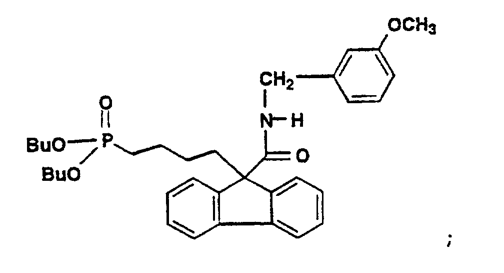 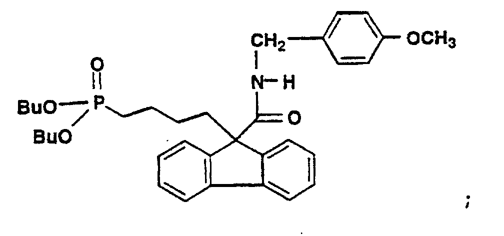 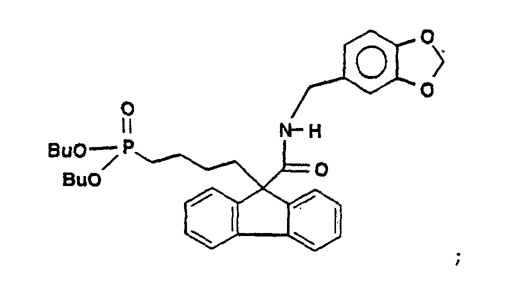 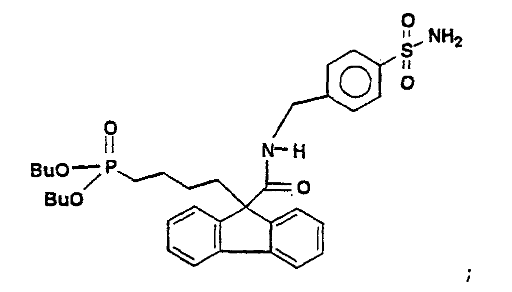 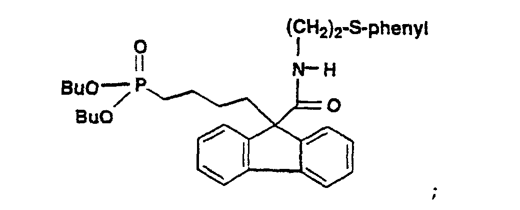 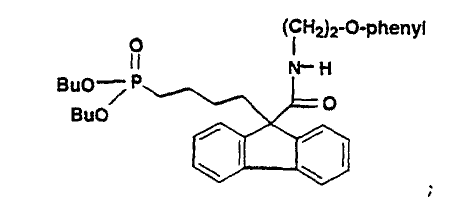 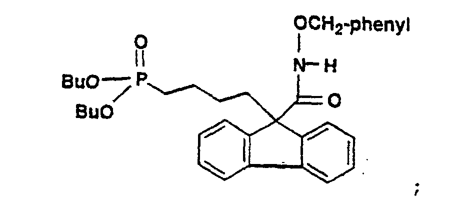 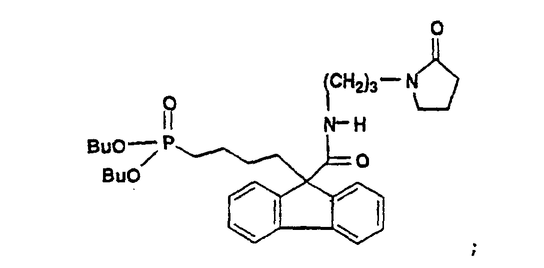 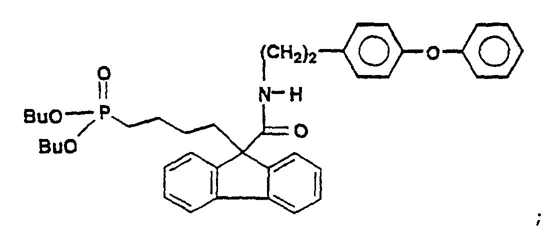 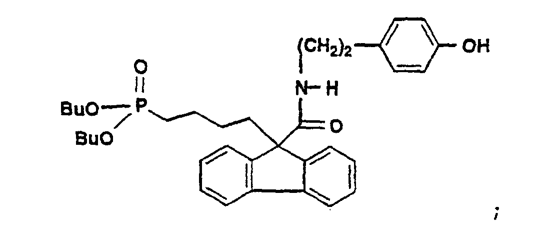 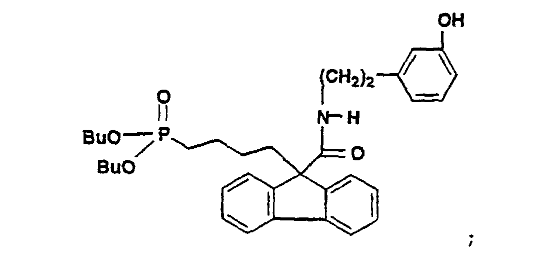 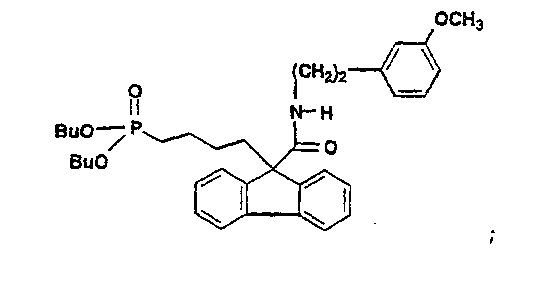 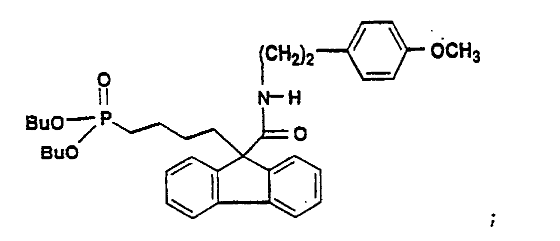 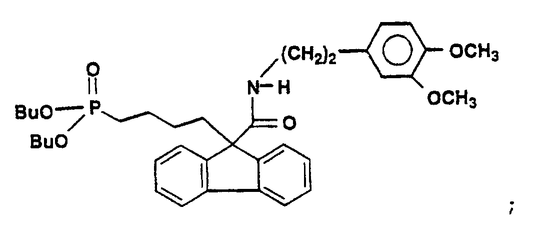 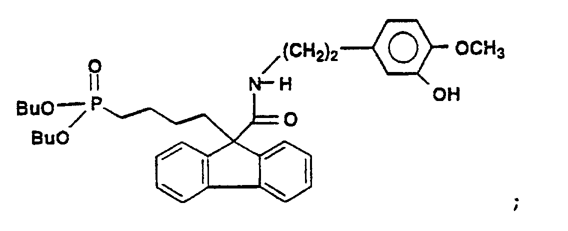 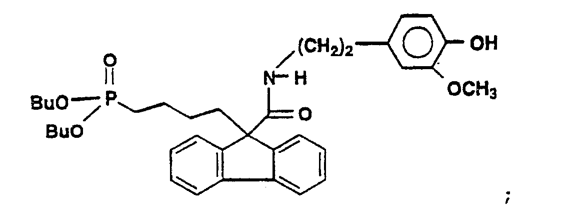 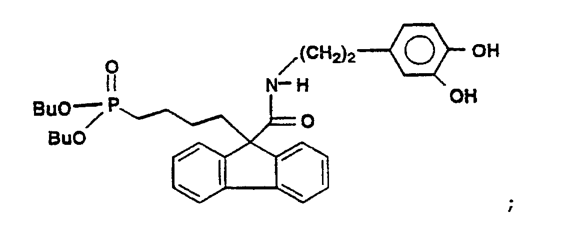 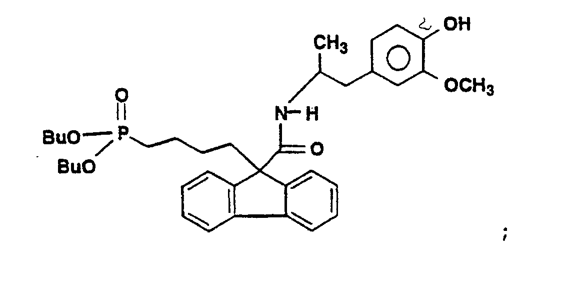 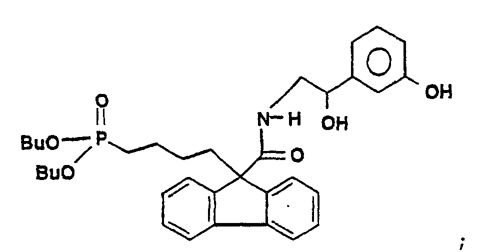 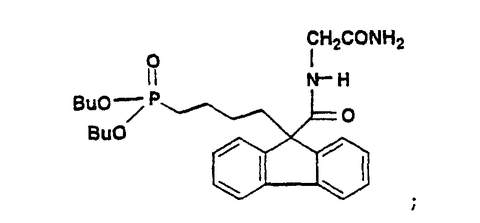 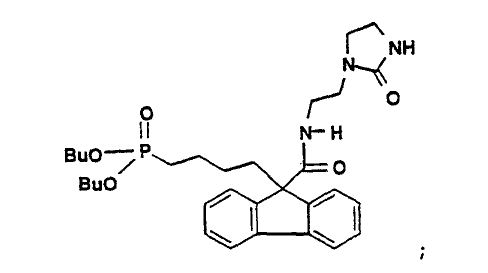 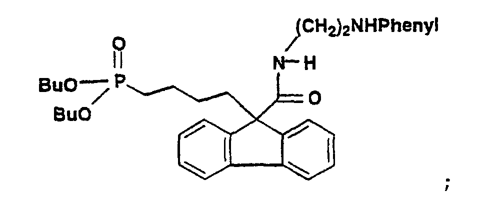 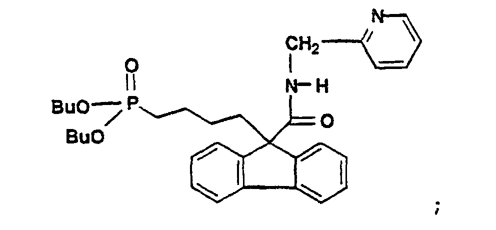 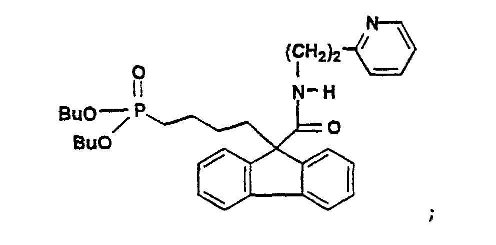 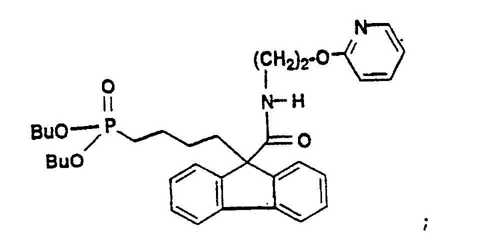 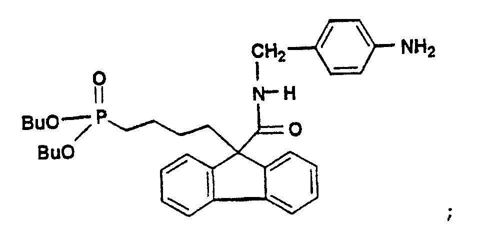 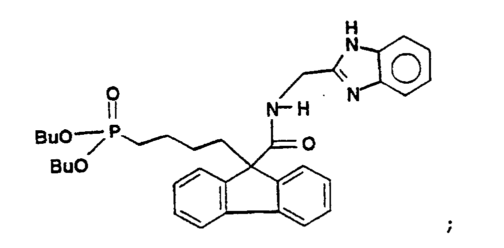 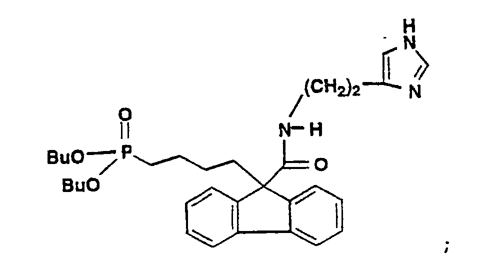 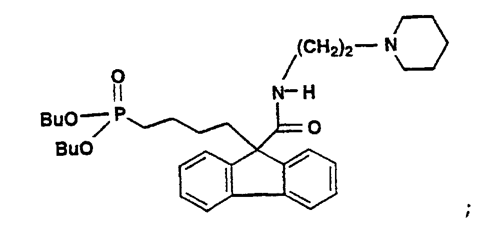 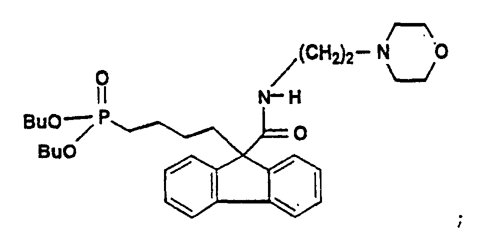 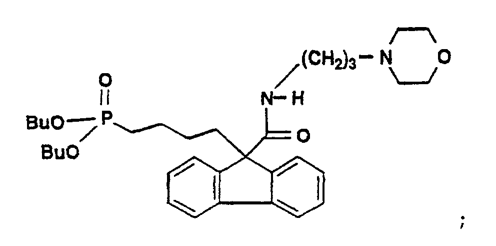 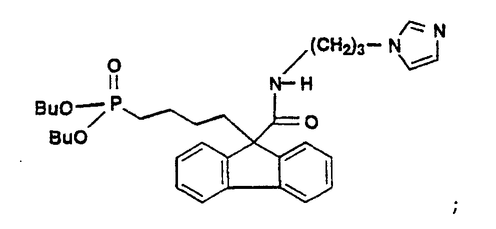 9-[4-(dibutoxyphosphinyl)butyl]-N-(2,2,2-trifluoroethyl)-9H-fluorene-9-carboxamide;
9-(2-propenyl)-9H-fluorene-9-carboxylic acid, ethyl ester;
9-propyl-9H-fluorene-9-carboxaldehyde;
9-(4-cyanobutyl)-N-propyl-9H-fluorene-9-carboxamide;
1-[9-(3-phenylpropyl)-9H-fluorene-9-yl]-1-butanone;
9-(3-phenylpropyl)-α-propyl-9H-fluorene-9-methanol;
4-hydroxy-l-(9-propyl-9H-fluoren-9-yl)-butanone;
N-[3-(dibutoxyphosphinyl)propyl]-9-propyl-9H-fluorene-9-carboxamide;
N-[5-(dibutoxyphosphinyl)pentyl-9-propyl-9H-fluorene-9-carboxamide;
N-[[4-(1,3-dihydro-1-oxo-2H-isoindol-2-yl)phenyl]methyl]-9-propyl-9H-fluorene-9-carboxamide;
(E)-9-[4-(dibutoxyphosphinyl)-2-butenyl]-2,7-difluoro-N-propyl-9H-fluorene-9-carboxamide;
9-[4-(dibutoxyphosphinyl)butyl]-2,7-difluoro-N-propyl-9H-fluorene-9-carboxamide;
9-[4-(diethoxyphosphinyl)butyl]-N-propyl-9H-fluorene-9-carboxamide;
9-[4-(diphenylphosphinyl)butyl]-N-propyl-9H-fluorene-9-carboxamide;
[4-[9-(butylthio)-9H-fluorene-9-yl]butyl]-phosphonic acid, dibutyl ester;
[4-[9-(butylsulfonyl)-9H-fluoren-9-yl]butyl]-phosphinic acid, dibutyl ester;
[4-[9-(butylsulfinyl)-9H-fluoren-9-yl]butyl]-phosphonic acid, dibutyl ester;
5-[4-(dibutoxyphosphinyl)butyl]-N-propyl-5H-indeno[1,2-b]pyridine-5-carboxamide;
(E)-9-[4-(dibutoxyphosphinyl)-2-butenyl]-2,7-difluoro-N-(2,2,2'-trifluoroethyl)-9H-fluorene-9-carboxamide;
9-[4-[4-(1,3-dihydro-1,3-dioxo-2H-isoindol-2-yl)phenyl]butyl]-N-propyl-9H-fluorene-9-carboxamide;
9-[4-[4-[[(2-phenoxyphenyl)carbonyl]amino]-phenyl]butyl]-N-propyl-9H-fluorene-9-carboxamide;
9-[4-[4-(1,3-dihydro-1-oxo-2H-isoindol-2-yl)phenyl]butyl]-N-propyl-9H-fluorene-9-carboxamide;
9-[3-[4-(1,3-dihydro-1,3-dioxo-2H-isoindol-2-yl)phenyl]propyl]-N-propyl-9H-fluorene-9-carboxamide;
9-[3-[4-(benzoylamino)]phenyl]-N-propyl-9H-fluorene-9-carboxamide;
9-[3-[(1,3-dihydro-1-oxo-2H-isoindol-2-yl)-phenyl]propyl]-N-propyl-9H-fluorene-9-carboxamide; 9-[5-[(6-ethoxy-2-benzothiazolyl)thio]-pentyl]-N-propyl-9H-fluorene-9-carboxamide;
9-[4-[4-(benzoylamino)phenyl]butyl]-N-propyl-9H-fluorene-9-carboxamide;
9-[5-(dibutoxyphosphinyl)pentyl]-N-propyl-9H-fluorene-9-carboxamide;
N,N-diethyl-9-(2-propenyl)-9H-fluorene-9-carboxamide;
N-ethyl-9-propyl-9H-fluorene-9-carboxamide;
N-ethyl-9-(2-propenyl)-9H-xanthene-9-carboxamide;
N-ethyl-9-(3-phenylpropyl)-9H-xanthene-9-carboxamide;
9-[(4-morpholinyl)carbonyl]-9-propyl-9H-fluorene;
9-hexyl-N-propyl-9H-xanthene-9-carboxamide;
N-methoxy-N-methyl-9-propyl-9H-fluorene-9-carboxamide;
10, 11-dihydro-5-(3-phenyl-2-propenyl)-N-propyl-5H-dibenzo[a,d]cycloheptene-5-carboxamide;
N-methyl-9-propyl-9H-fluorene-9-carboxamide;
1-(9-propyl-9H-fluorene-9-yl)-1-pentanone;
α-butyl-9-propyl-9H-fluorene-9-methanol;
1-(9-propyl-9H-fluorene-9-yl)-1-butanone;
α,9-dipropyl-9H-fluorene-9-methanol;
10,11-dihydro-5-(2-propenyl)-N-propyl-5H-dibenzo-[a,d]cycloheptene-5-carboxamide;
9-(3-phenylpropyl)-N-propyl-9H-thioxanthene-9-carboxamide;
N,9-dipropyl-9H-thioxanthene-9-carboxamide; 10,11-Dihydro-5-(3-phenylpropyl)-N-propyl-5H-dibenzo-[a,d]cycloheptane-5-carboxamide;
(E)-2,7-difluoro-9-(3-phenyl-2-propenyl)-N-propyl-9H-fluorene-9-carboxamide;
9-(3-phenylpropyl)-N-(2-pyridinylmethyl)-9H-fluorene-9-carboxamide;
2,7-difluoro-9-(3-phenylpropyl)-N-propyl-9H-fluorene-9-carboxamide;
2,7-difluoro-9-(3-phenylpropyl)-N-(4-pyridinylmethyl)-9H-fluorene-9-carboxamide;
9-(butylthio)-9-propyl-9H-fluorene;
9-(butylsulfinyl)-9-propyl-9H-fluorene;
9-(4-hydroxybutyl)-N-propyl-9H-fluorene-9-carboxamide;
9-[4-(phenylthio)butyl]-N-propyl-9H-fluorene-9-carboxamide;
9-[3-(1,3-dioxan-2-yl)propyl]-N-propyl-9H-fluorene-9-carboxamide;
9-[3-(1,3-dioxolan-2-yl)propyl]-N-propyl-9H-fluorene-9-carboxamide;
cis-N,9-dipropyl-1H-thioxanthene-9-carboxamide, 10-oxide;
5-(2-propenyl)-N-propyl-5H-indeno[1,2-b]pyridine-5-carboxamide;
(E)-5-(3-phenyl-2-propenyl)-N-propyl-5H-indeno[1,2-b]pyridine-5-carboxamide;
N-ethyl-N-methyl-9-(2-propenyl)-9H-fluorene-9-carboxamide;
N,9-dipropyl-9H-thioxanthene-9-carboxamide, 10,10-dioxide;
trans-N,9-dipropyl-9H-thioxanthene-9-carboxamide, 10-oxide;
9-[3-(dibutoxyphosphinyl)propyl]-N-(2-pyridinylmethyl)-9H-fluorene-9-carboxamide;
1-(9-propyl-9H-fluorene-9-yl)-2-(1-piperidinyl)ethanone, monohydrochloride;
N-(5-hydroxypentyl)-9-propyl-9H-fluorene-9-carboxamide;
9-(3-cyanopropyl)-N-propyl-9H-fluorene-9-carboxamide;
N-[[4-[[(9-propyl-9H-fluoren-9-yl)carbonyl]amino]phenyl]methyl]-9-propyl-9H-fluorene-9-carboxamide;
N-[4-(4-aminophenyl)methyl]-9-propyl-9H-fluorene-9-carboxamide;
9-[3-(dibutoxyphosphinyl)propyl]-N-propyl-9H-fluorene-9-carboxamide;
4-(1-piperidinyl)-1-(9-propyl-9H-fluoren-9-yl)-1-butanone, monohydrochloride;
N-methyl-9-(3-phenylpropyl)-9H-fluorene-9-carboxamide;
2-(dimethylamino)-9-(3-phenylpropyl)-N-propyl-9H-fluorene-9-carboxamide;
9-[4-(dibutoxyphosphinyl)-2-butenyl]-N-propyl-9H-fluorene-9-carboxamide;
9-[4-(4-nitrophenyl)butyl]-N-propyl-9H-fluorene-9-carboxamide;
9-[3-(4-nitrophenyl)-2-propenyl]-N-propyl-9H-fluorene-9-carboxamide;
5-(3-phenylpropyl)-N-propyl-5H-indeno[1,2-b]pyridine-5-carboxamide;
9-[4-(4-aminophenyl)butyl]-N-propyl-9H-fluorene-9-carboxamide;
9-[3-(4-aminophenyl)propyl]-N-propyl-9H-fluorene-9-carboxamide;
9-[4-(dibutoxyphosphinyl)butyl]-9H-fluorene-9-carboxylic acid, methyl ester;
N,N-dibutyl-9-[(propylamino)carbonyl]-9H-fluorene-9-butanamide;
9-(5-cyanopentyl)-N-propyl-9H-fluorene-9-carboxamide;
9-[2-[[[4-(1,3-dihydro-1,3-dioxo-2H-isoindol-2-yl)phenyl]sulfonyl]amino]ethyl]-N-(2,2,2-trifluoroethyl)-9H-fluorene-9-carboxamide;
(Z)-9-[4-[(6-ethoxy-2-benzothiazolyl)thio]-2-butenyl]-N-propyl-9H-fluorene-9-carboxamide;
9-[4-(dibutoxyphosphinyl)butyl]-N-(2,2,2-trifluoropropyl)-9H-xanthene-9-carboxamide;
9-[4-[butoxy[2-(4-morpholinyl)ethoxy]phosphinyl]butyl]-N-(2,2,2-trifluoroethyl)-9H-fluorene-9-carboxamide; 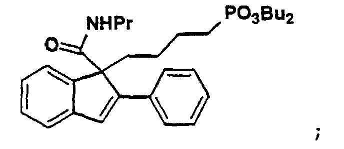 9-[4-(dibutoxyphosphinyl)butyl]-2,7-difluoro-N-(2,2,2-trifluoroethyl)-9H-fluorene-9-carboxamide; 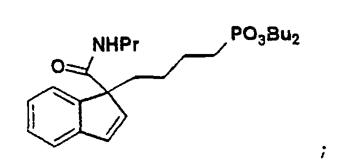 (E)-9-[4-(dibutoxyphosphinyl)-2-butenyl]-N-propyl-9H-fluorene-9-carboxamide;
9-[4-[4-(benzoylamino)-1H-imidazol-1-yl]-butyl]-N-(2,2,2-trifluoroethyl)-9H-fluorene-9-carboxamide;
9-[4-[5-(benzoylamino)-2-pyridinyl]butyl]-N-(2,2,2-trifluoroethyl)-9H-fluorene-9-carboxamide;
9-[4-[4-[(2-phenoxybenzoyl)amino]-1H-imidazol-1-yl]butyl]-N-(2,2,2-trifluoroethyl)-9H-fluorene-9-carboxamide;
9-[4-[(2-bromo-5-pyridinyl)amino]butyl]-N-propyl-9H-fluorene-9-carboxamide;
9-[2-[[[4-(benzoylamino)phenyl]sulfonyl]-amino]ethyl]-N-(2,2,2-trifluoroethyl)-9H-fluorene-9-carboxamide;
9-(4-phenylbutyl)-N-propyl-9H-fluorene-9-carboxamide;
3-[(9-propyl-9H-fluoren-9-yl)sulfonyl]-propanoic acid, methyl ester;
9-[4-[(6-ethoxy-2-benzothiazolyl)thio]-butyl]-N-propyl-9H-fluorene-9-carboxamide;
9-[3-[(6-ethoxy-2-benzothiazolyl)thio]-propyl]-N-propyl-9H-fluorene-9-carboxamide;
(Z)-9-[4-(diethoxyphosphinyl)-2-butenyl]-N-(2,2,2-trifluoroethyl)-9H-fluorene-9-carboxamide;
9-[4-(diethoxyphosphinyl)butyl]-N-(2,2,2-trifluoroethyl)-9H-fluorene-9-carboxamide;
9-[4-(dibutoxyphosphinyl)butyl]-N-(2,2,3,3,3-pentafluoropropyl)-9H-fluorene-9-carboxamide;
9-[4-(dibutoxyphosphinyl)butyl]-N-propyl-9H-xanthene-9-carboxamide;
9-[4-(dibutoxyphosphinyl)butyl]-N-(2,2,3,3,4,4,4-heptafluorobutyl)-9H-fluorene-9-carboxamide;
9-[4-(dibutoxyphosphinyl)butyl]-N-propyl-9H-indeno-[2,1-b]pyridine-9-carboxamide;
9-[4-[4-[(phenylsulfonyl)amino]phenyl]-butyl]-N-(2,2,2-trifluoroethyl)-9H-fluorene-9-carboxamide;
[4-[9-(1-oxopentyl)-9H-fluoren-9-yl]butyl]-phosphonic acid;
9-[5-(dibutoxyphosphinyl)pentyl]-N-(2,2,2-trifluoroethyl)-9H-fluorene-9-carboxamide;
9-[3-[[5-[(2-phenoxybenzoyl)amino]-2-pyridinyl]oxy]propyl]-N-(2,2,2-trifluoroethyl)-9H-fluorene-9-carboxamide;
[6-[9-[[(2,2,2-trifluoroethyl)amino] carbonyl]-9H-fluoren-9-yl]hexyl]-phosphonic acid, dibutyl ester;
9-[4-[5-[(2-phenoxybenzoyl)amino]-2-pyridinyl]butyl]-N-(2,2,2-trifluoroethyl)-9H-fluorene-9-carboxamide;
9-[4-[4-(benzoylamino)-2-methyl-1H-imidazol-1-yl]butyl]-N-(2,2,2-trifluoroethyl)-9H-fluorene-9-carboxamide;
9-[4-[4-[(2-phenoxybenzoyl)amino]-2-methyl-1H-imidazol-1-yl]butyl]-N-(2,2,2-trifluoroethyl)-9H-fluorene-9-carboxamide;
9-[3-[[2-(benzoylamino)-5-pyridinyl]amino]-propyl]-N-(2,2,2-trifluoroethyl)-9H-fluorene-9-carboxamide;
[[4-(benzoylamino)phenyl]methyl][2-[9-[[(2,2,2-trifluoroethyl)amino]carbonyl]-9H-fluoren-9-yl]ethyl]carbamic acid, 1,1-dimethylethyl ester;
9-[2-[[[4-(benzoylamino)phenyl]methyl]-amino]ethyl]-N-(2,2,2-trifluoroethyl)-9H-fluorene-9-carboxamide;
9-[4-[butoxy(tetrahydrofuran-2-ylmethoxy)-phosphinyl]butyl]-N-(2,2,2-trifluoroethyl)-9H-fluorene-9-carboxamide;
9-[4-[butoxy(2-pyridinylmethoxy)-phosphinyl]butyl]-N-(2,2,2-trifluoroethyl)-9H-fluorene-9-carboxamide;
9-[4-(dipropoxyphosphinyl)butyl]-N-(2,2,2-trifluoroethyl)-9H-fluorene-9-carboxamide;
9-[4-(4-[[(4-nitrophenyl)sulfonyl]amino]-phenyl]butyl]-N-(2,2,2-trifluoroethyl)-9H-fluorene-9-carboxamide;
9-[4-[4-[[(2-nitrophenyl)sulfonyl]-amino]phenyl]butyl]-N-(2,2,2-trifluoroethyl)-9H-fluorene-9-carboxamide;
9-[4-(dibutoxyphosphinyl)butyl]-3,6-difluoro-N-(2,2,2-trifluoroethyl)-9H-fluorene-9-carboxamide;
9-[3-[[5-[(2-phenoxybenzoyl)amino]-2-pyridinyl]oxy]propyl]-N-propyl-9H-fluorene-9-carboxamide;
9-[6-[(6-ethoxy-2-benzothiazolyl)thio]-hexyl]-N-(2,2,2-trifluoroethyl)-9H-fluorene-9-carboxamide;
[4-[9-[[(2,2,2-trifluoroethyl)amino]-carbonyl]-9H-fluoren-9-yl]butyl]-phosphonic acid, di(1-methylethyl)ester;
[[4-[(2-phenoxybenzoyl)amino]phenyl]-methyl][2-[9-[[(2,2,2-trifluoroethyl)amino]-carbonyl]-9H-fluoren-9-yl]ethyl]-carbamic acid, 1,1-dimethylethyl ester;
9-[2-[[[4-[(2-phenoxybenzoyl)amino]phenyl]-methyl]amino]ethyl]-N-(2,2,2-trifluoroethyl)-9H-fluorene-9-carboxamide;
[1-[4-[9-[[(2,2,2-trifluoroethyl)amino]-carbonyl]-9H-fluoren-9-yl]butyl]-1H-imidazol-4-yl]-carbamic acid;
9-[4-[(4,5-diphenyl-1H-imidazol-2-yl)thio]-butyl]-N- [2-(4-methoxyphenyl)ethyl]-9H-fluorene-9-carboxamide;
9-[4-[(6-ethoxy-2-benzothiazolyl)thio]-butyl]-N-propyl-9H-fluorene-9-carboxamide;
9-[4-(2-thiazolylthio)butyl]-N-(2,2,2-trifluoroethyl)-9H-fluorene-9-carboxamide; 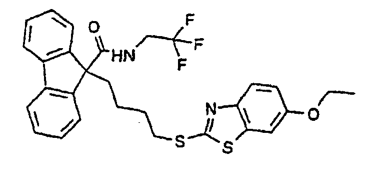 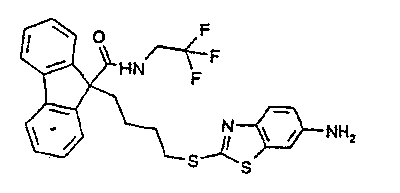 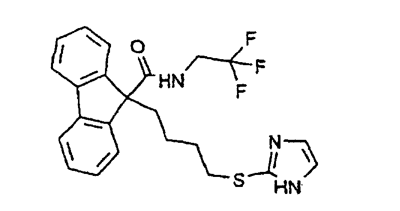 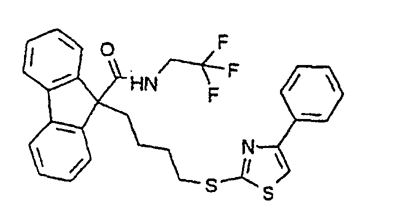 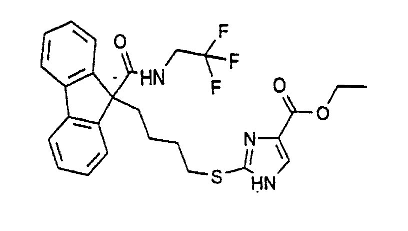 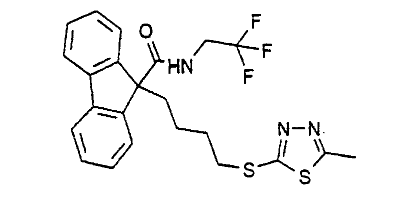 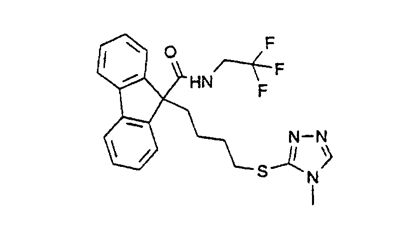 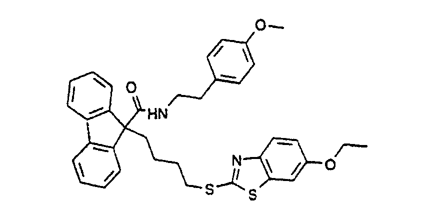 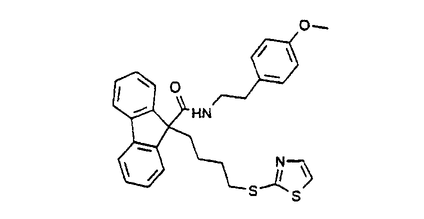 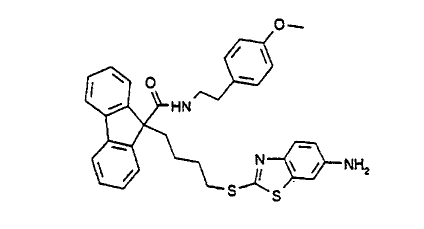 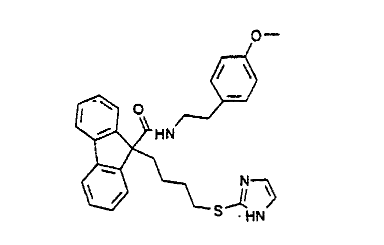 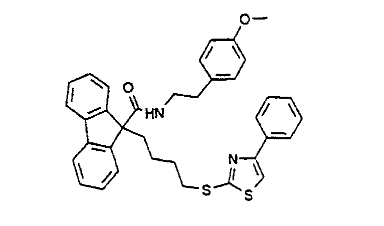 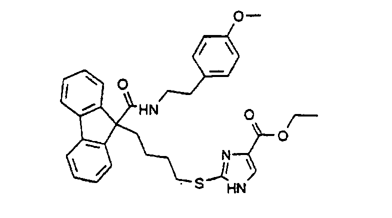 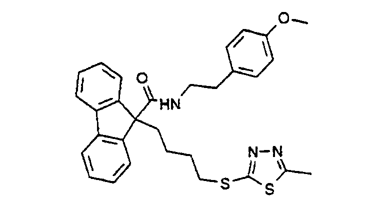 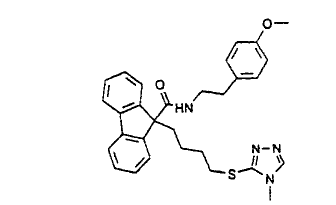 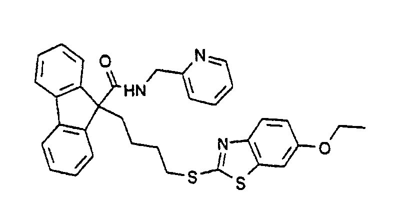 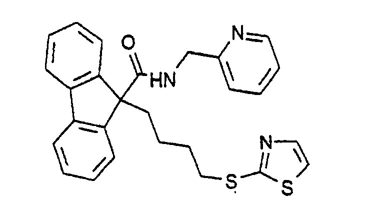 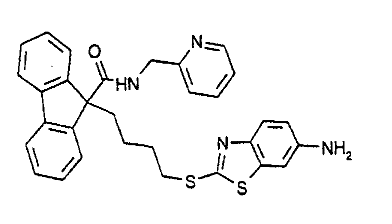 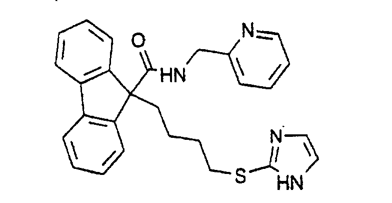 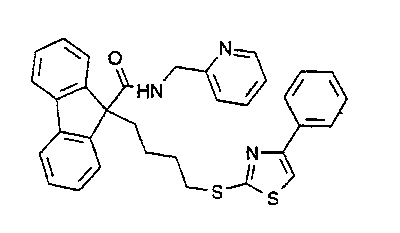 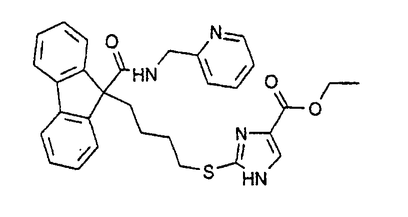 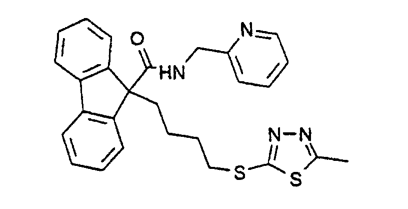 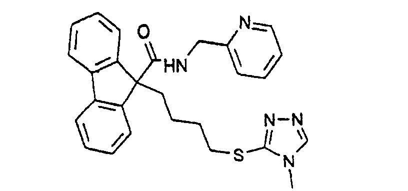 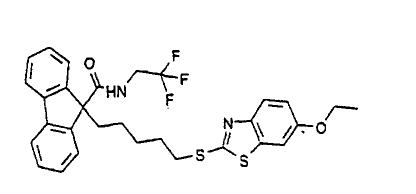 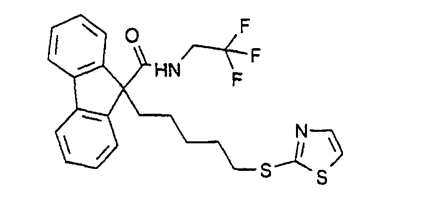 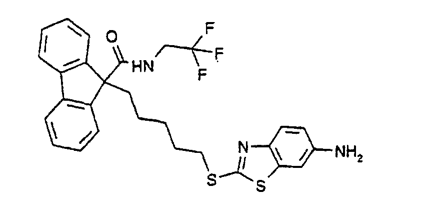 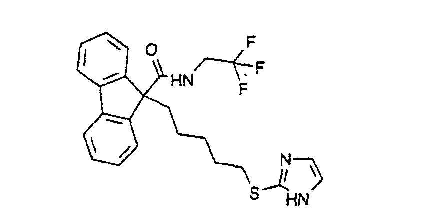 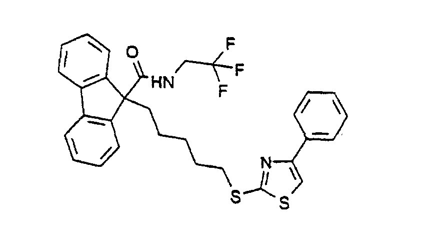 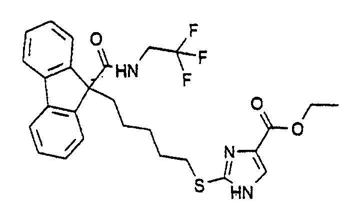 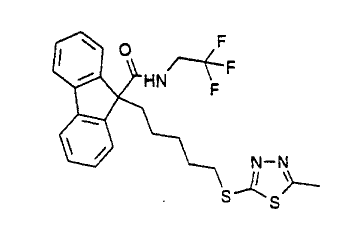 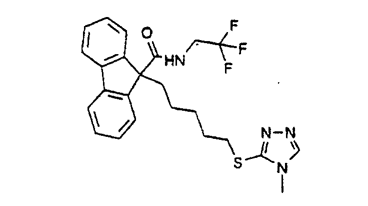 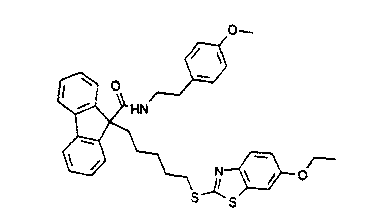 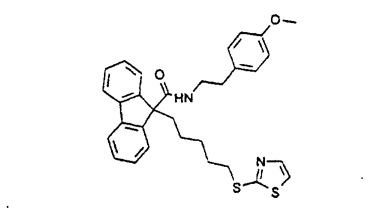 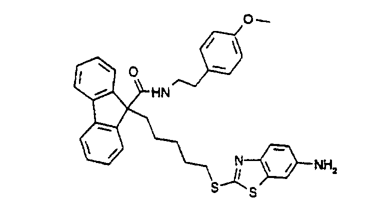 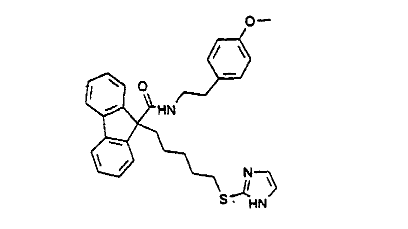 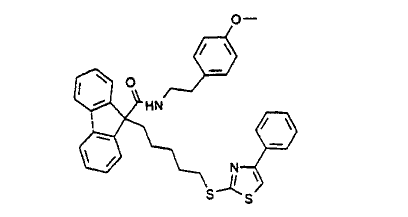 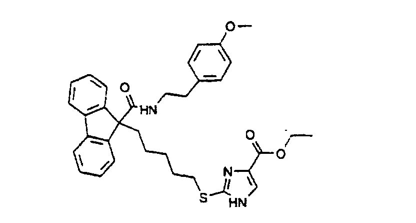 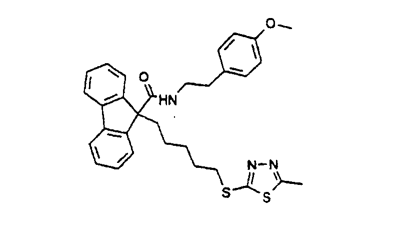 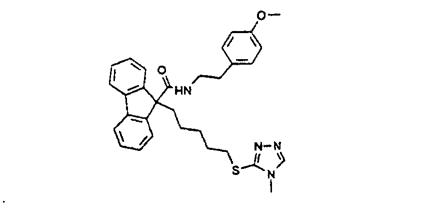 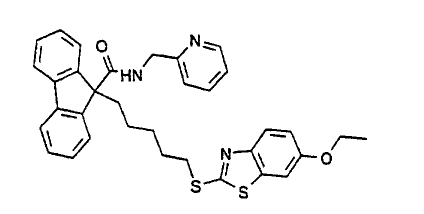 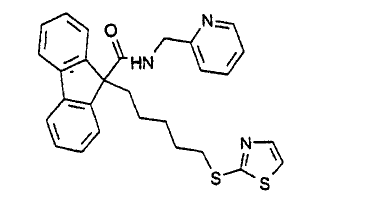 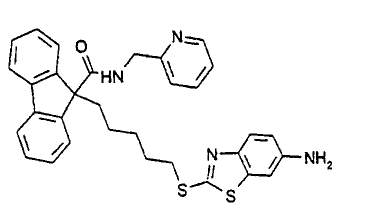 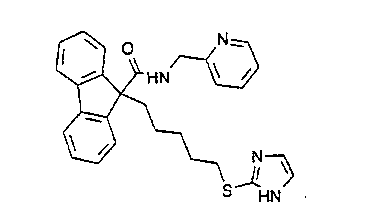 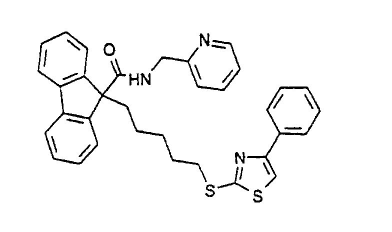 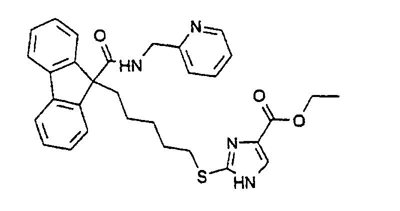 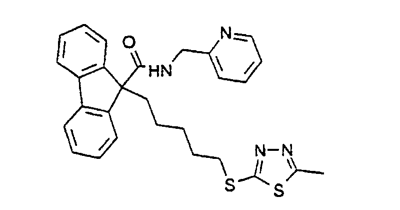 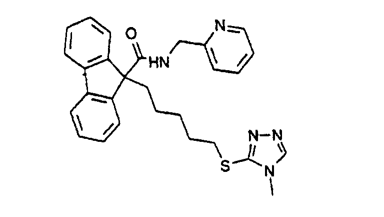 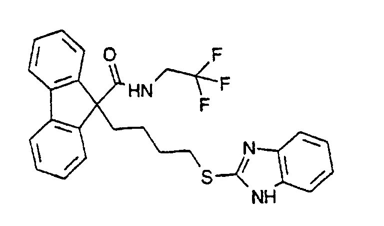 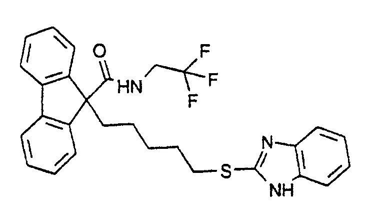 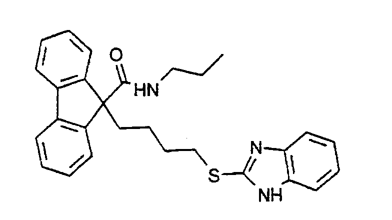 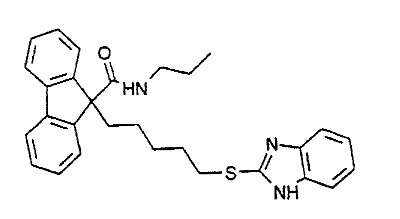 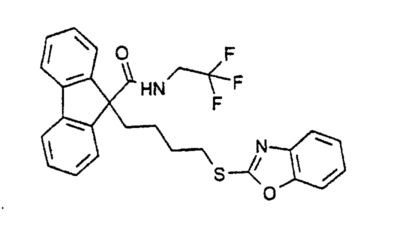 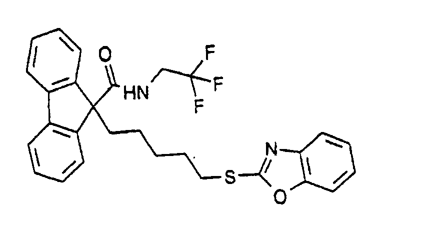 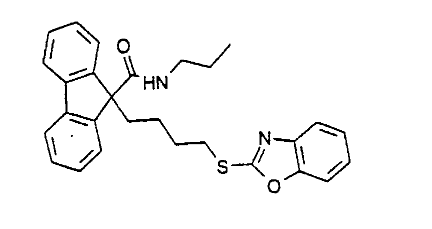 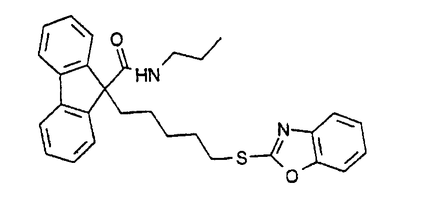 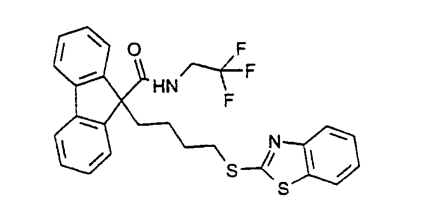 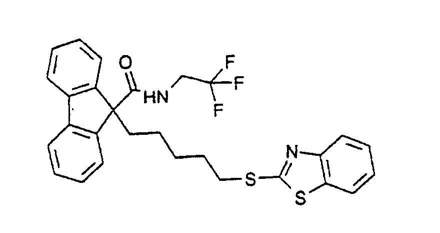 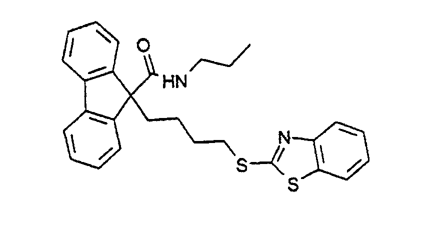 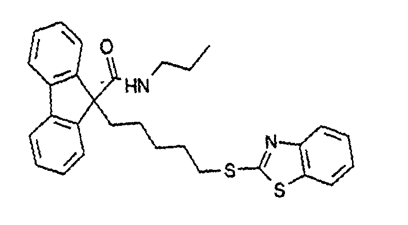 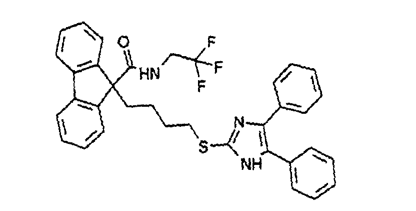 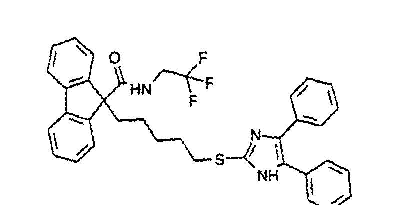 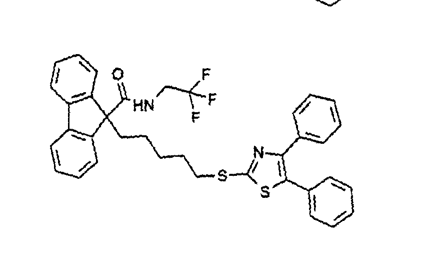 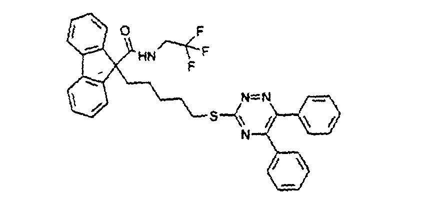 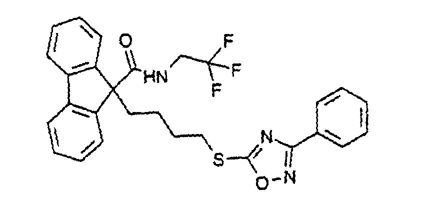 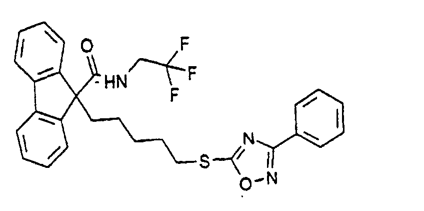 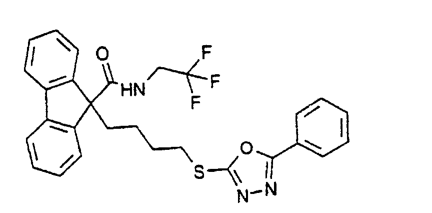 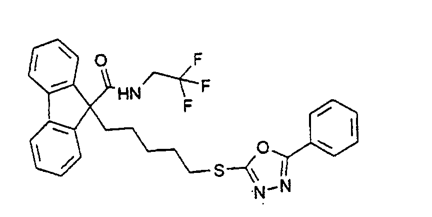 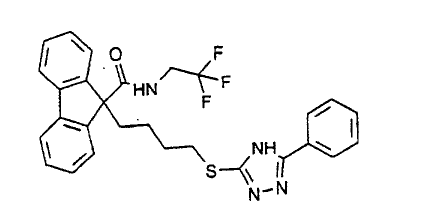 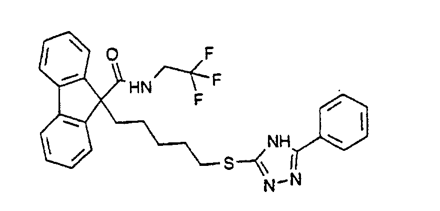 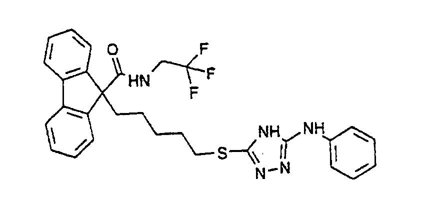 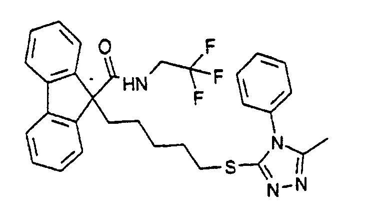 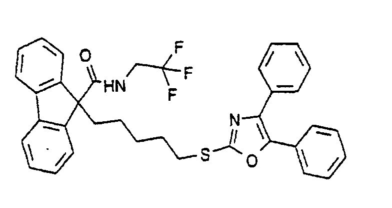 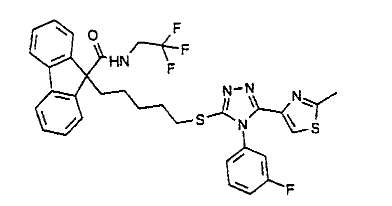 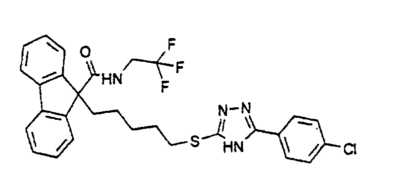 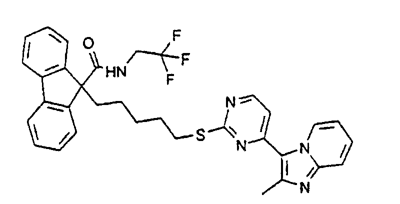 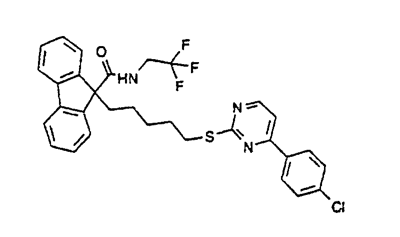 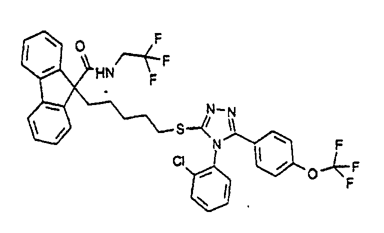 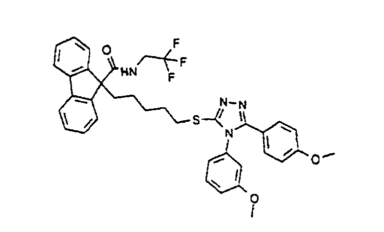 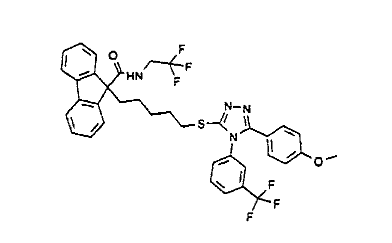 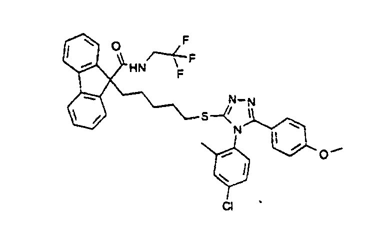 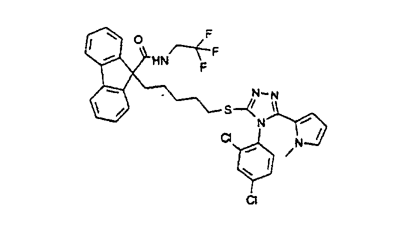 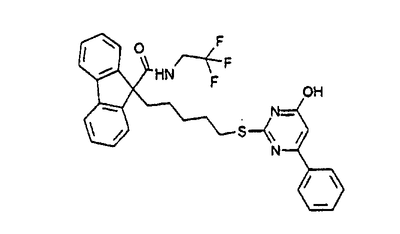 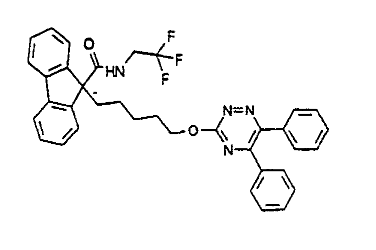 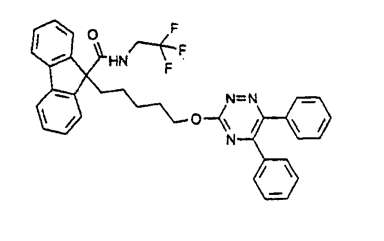 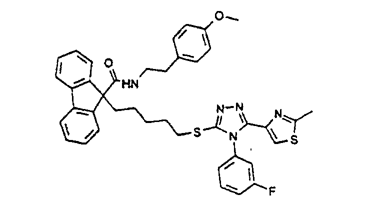 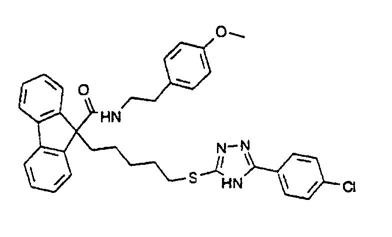 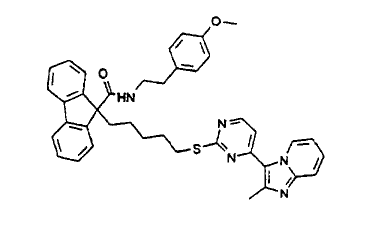 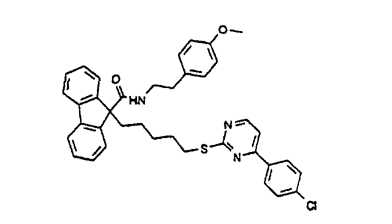 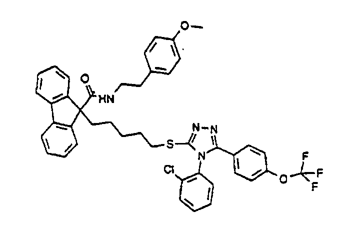 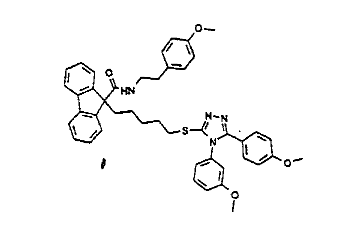 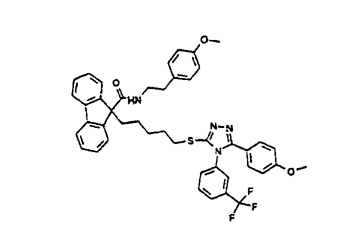 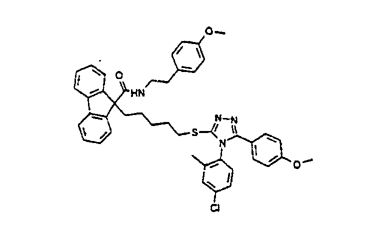 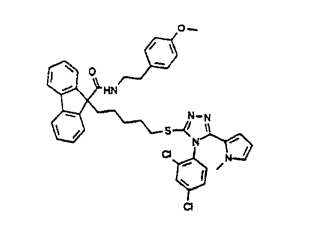 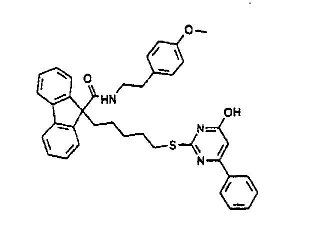 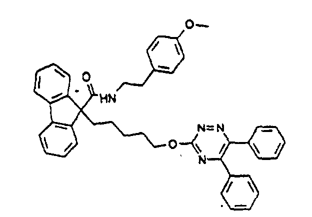 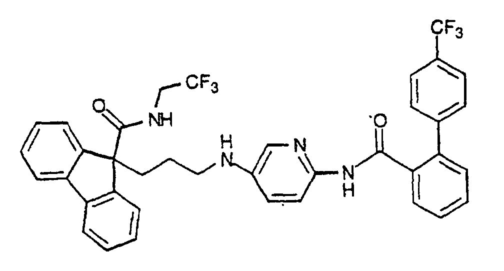 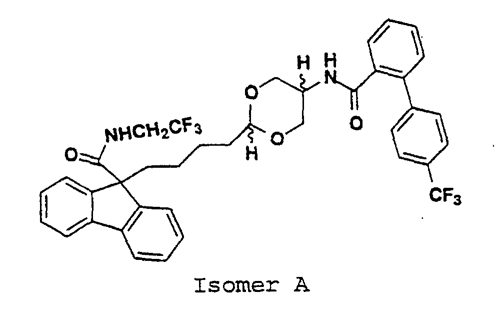 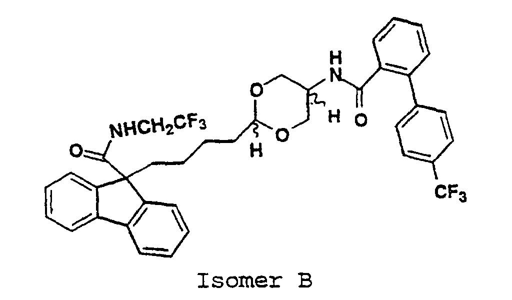 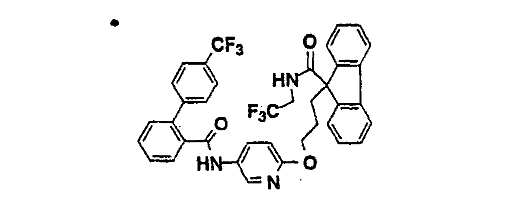 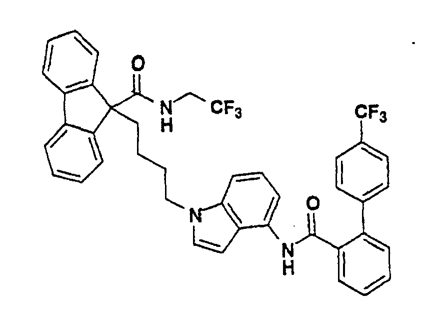 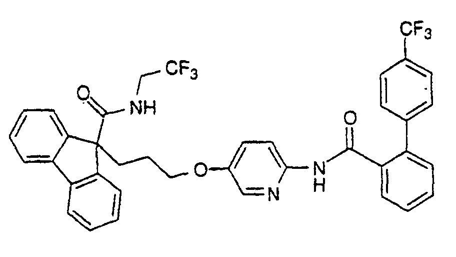 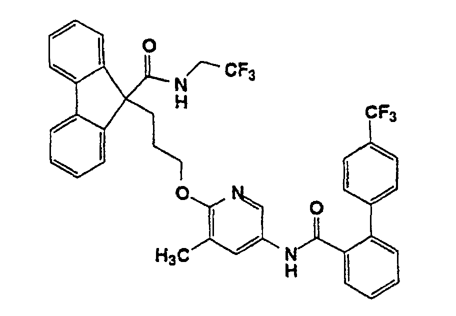 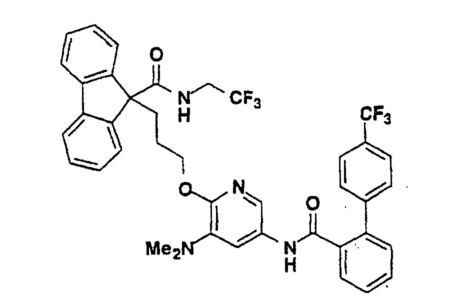 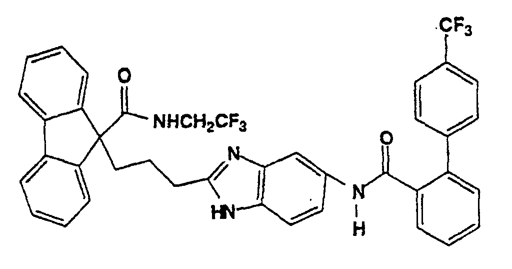 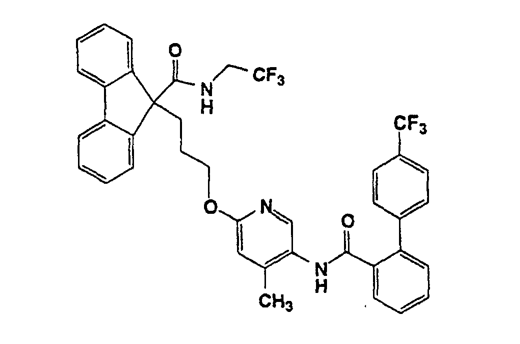 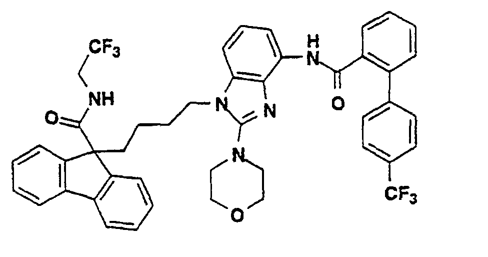 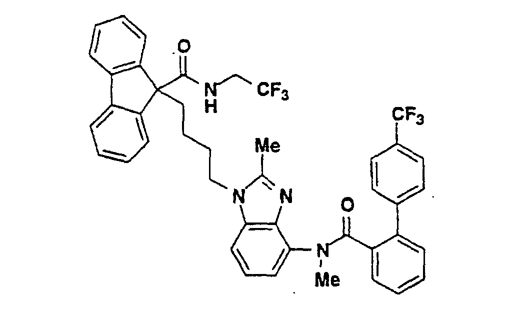 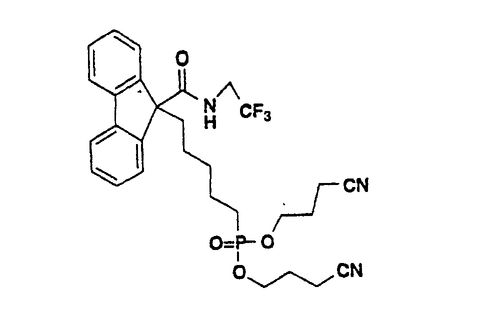 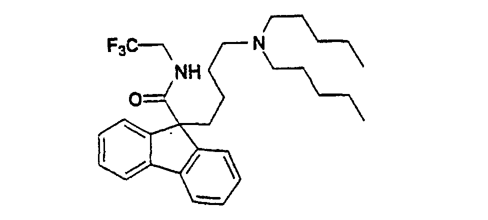 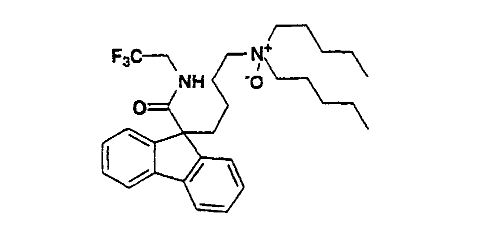 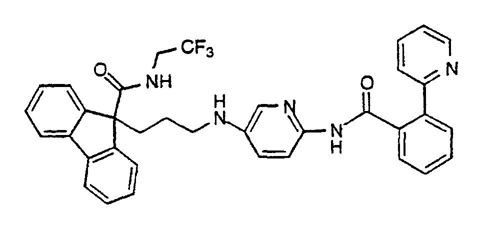 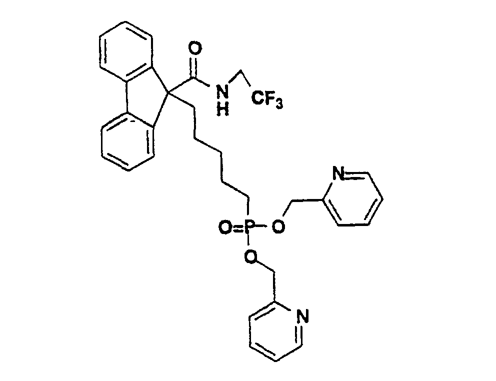 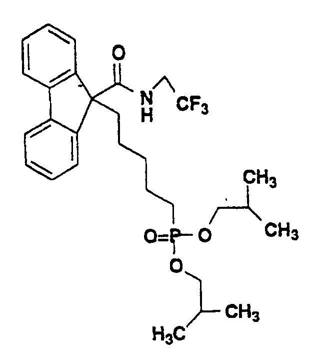 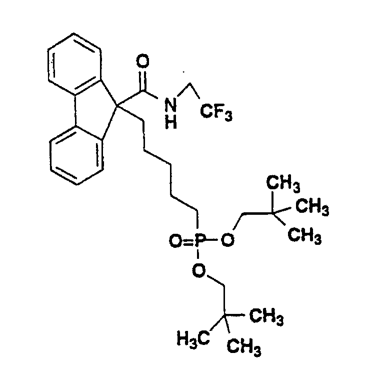 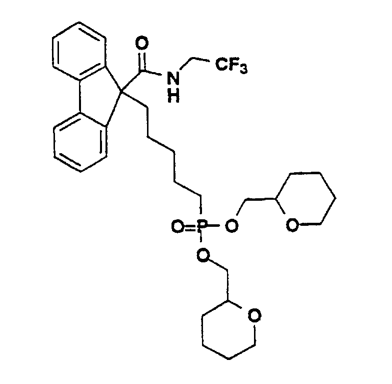 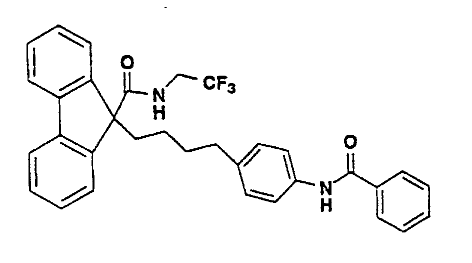 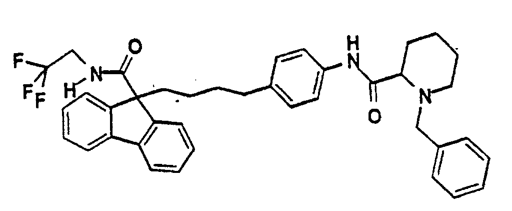 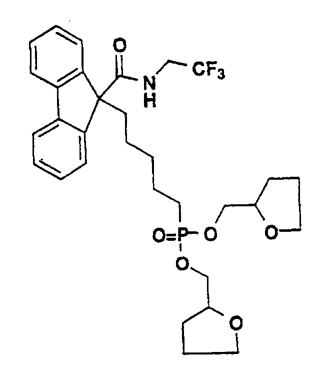 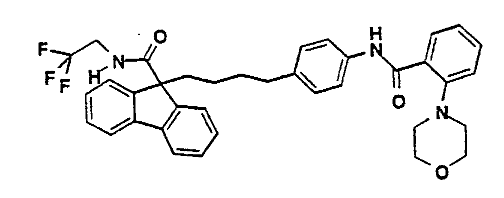 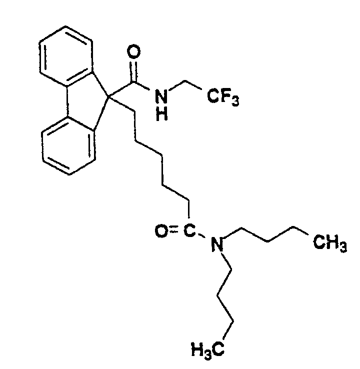 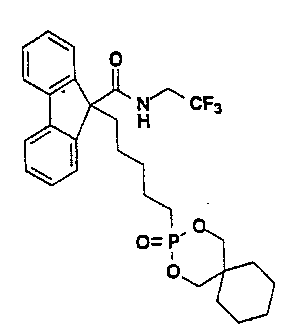 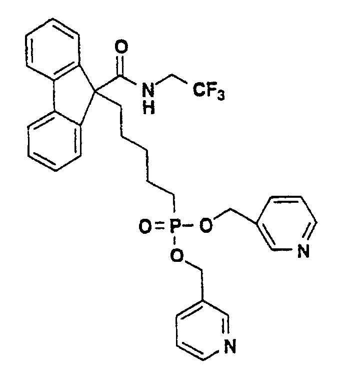 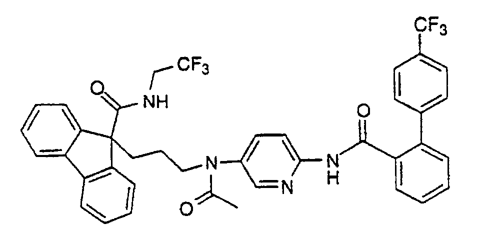 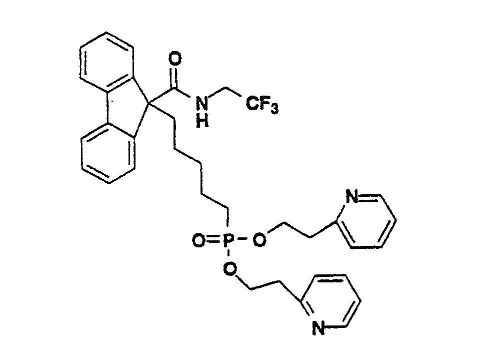 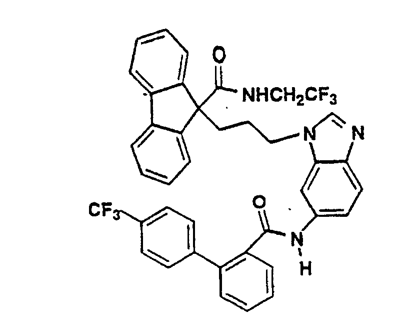 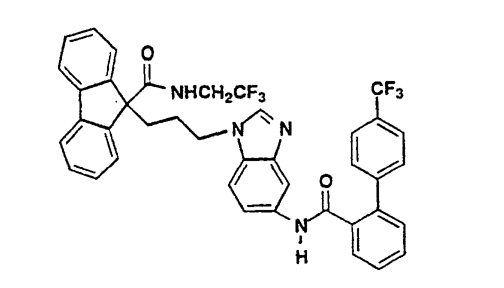 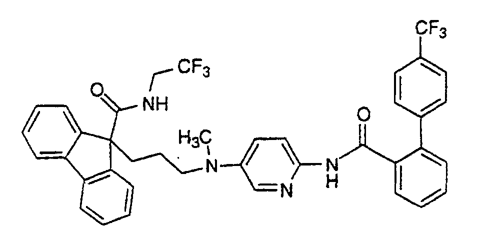 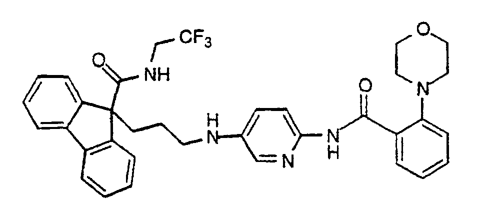 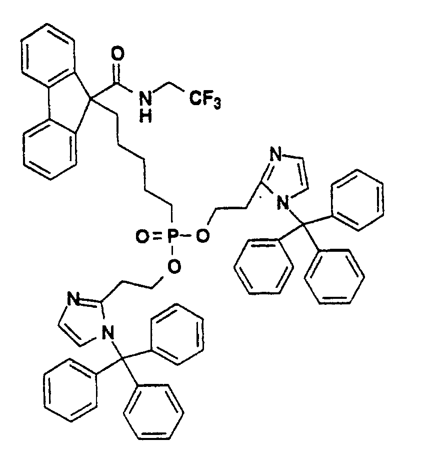 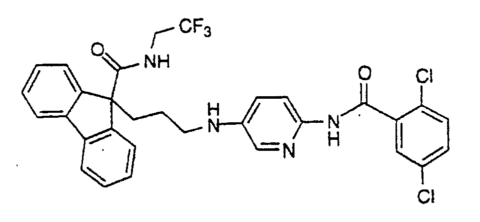 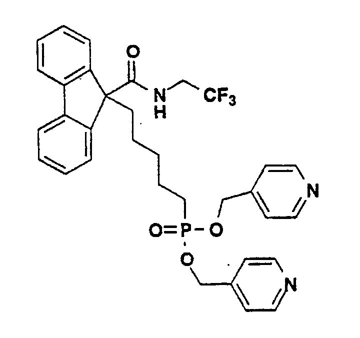 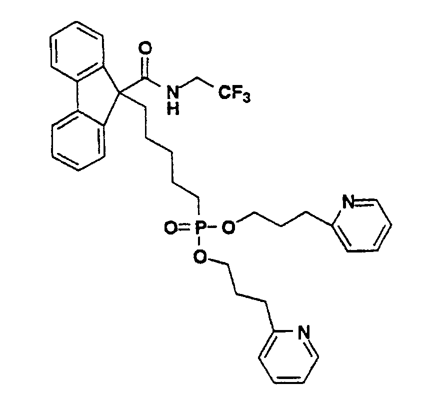 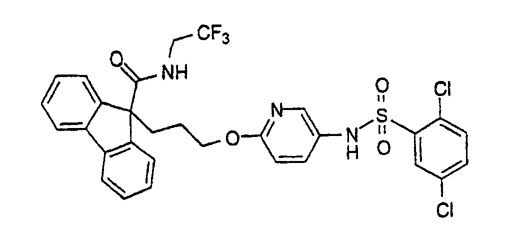 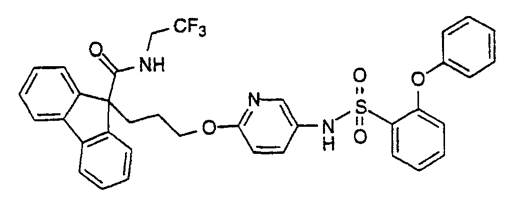 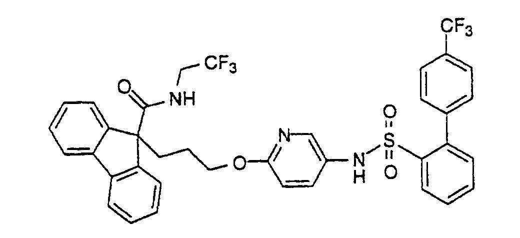 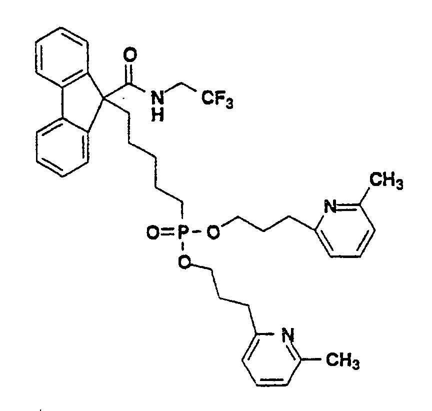 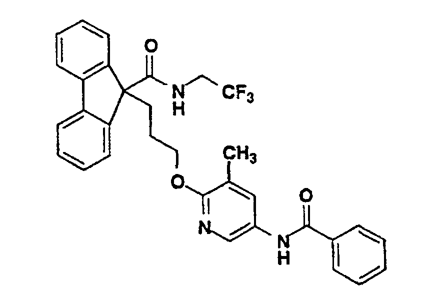 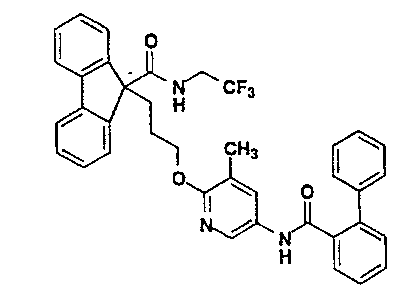 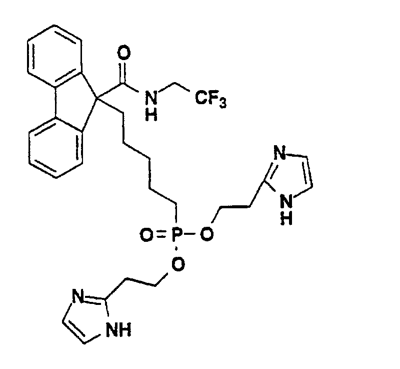 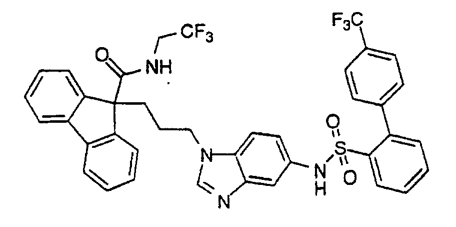 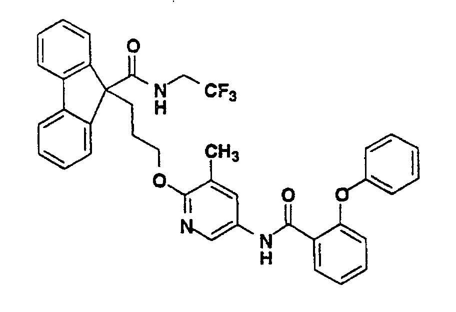 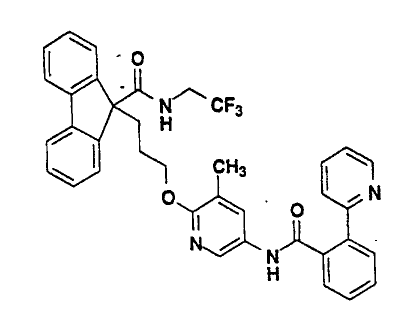 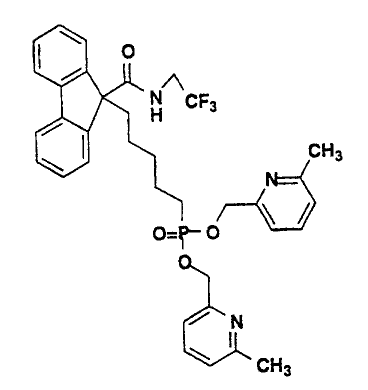 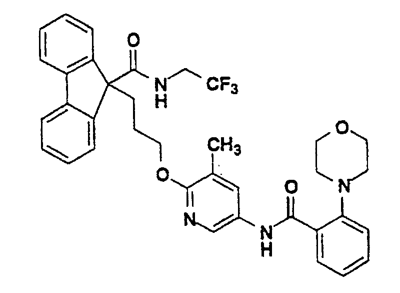 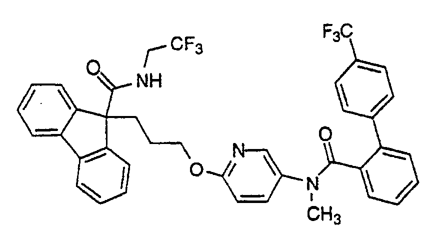 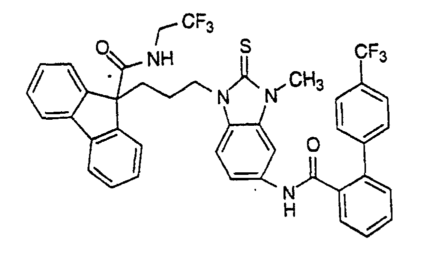 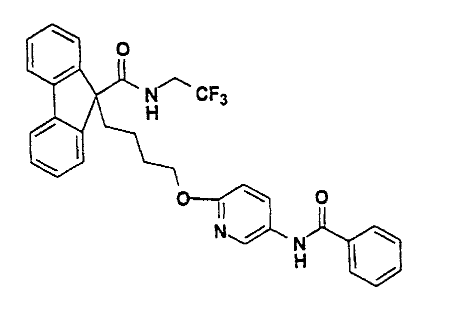 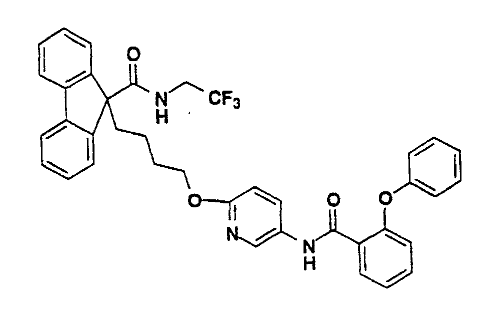 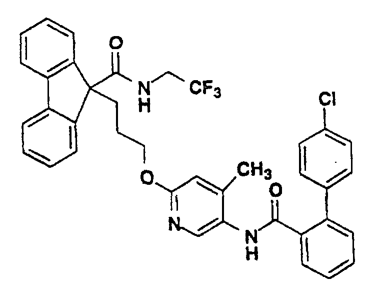 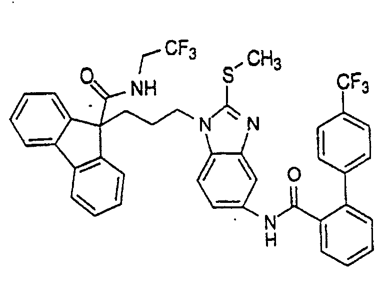 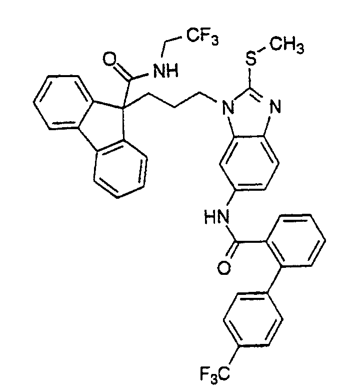 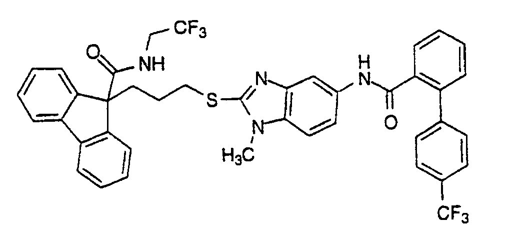 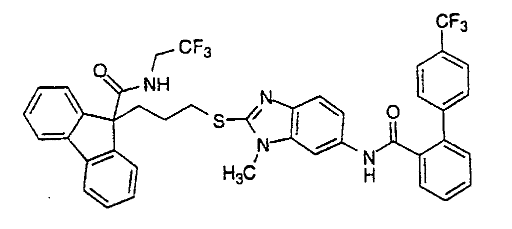 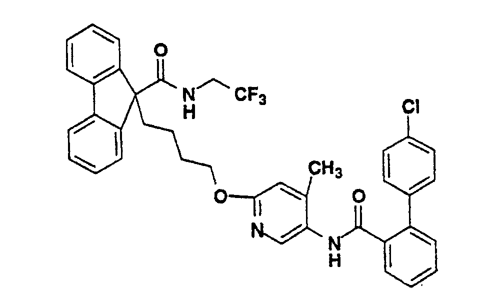 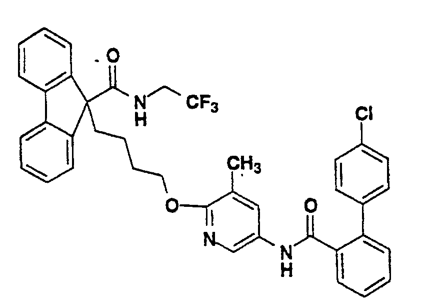 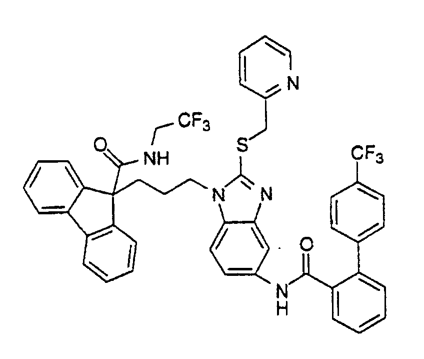 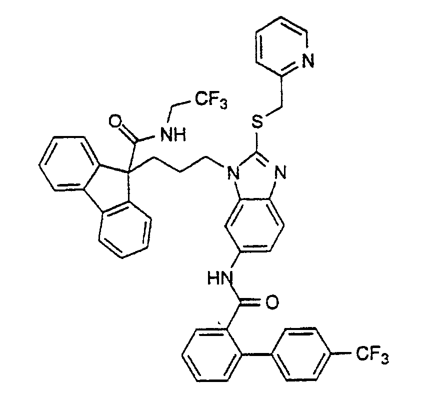 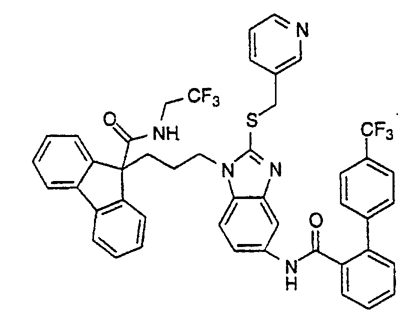 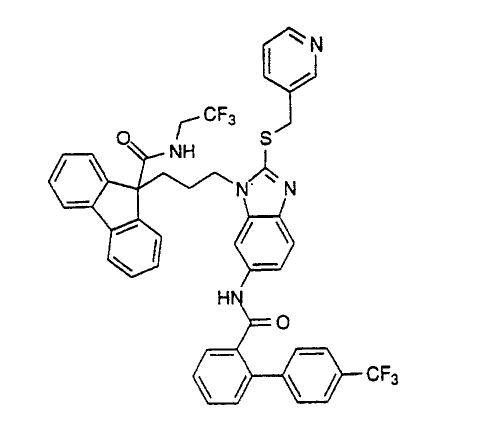 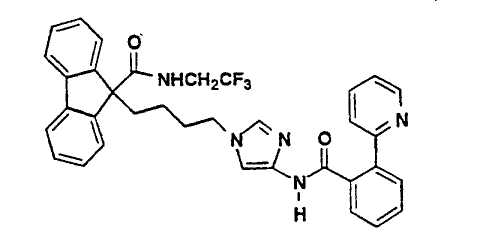 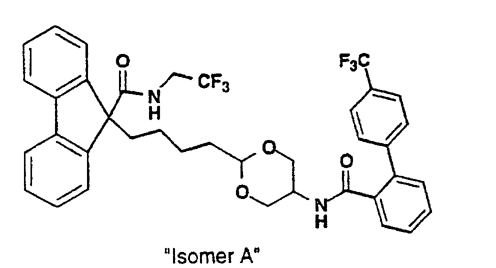 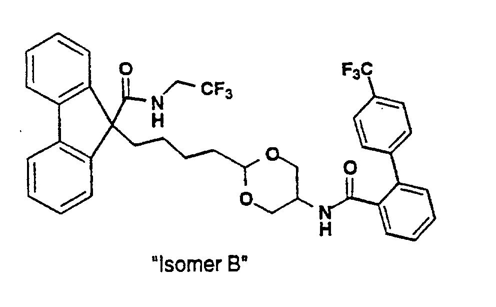 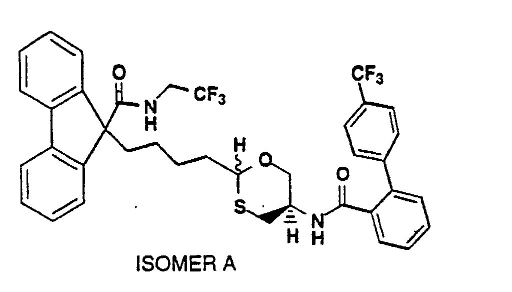 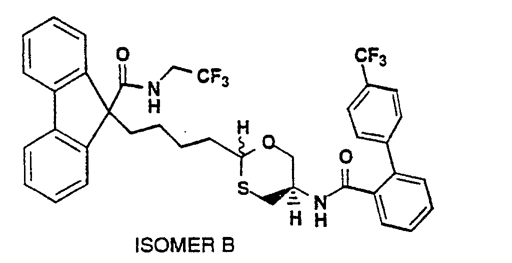 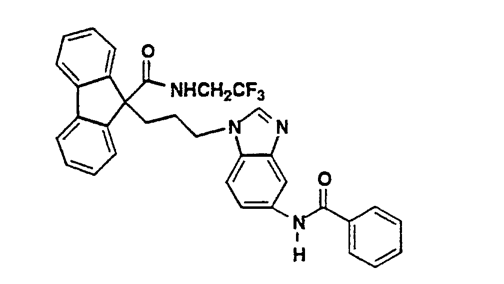 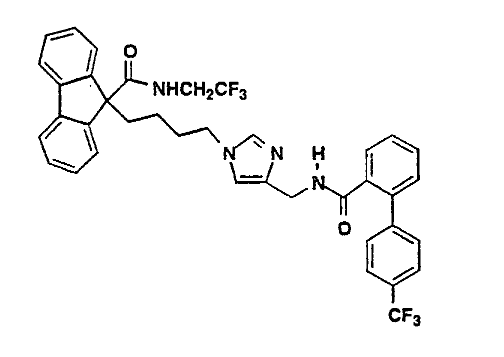 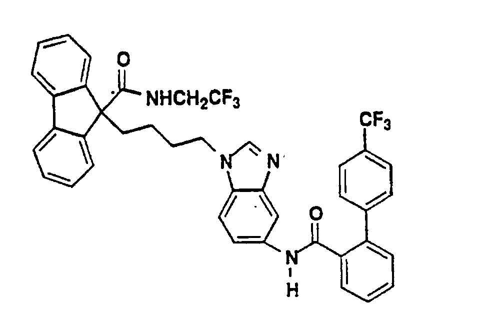 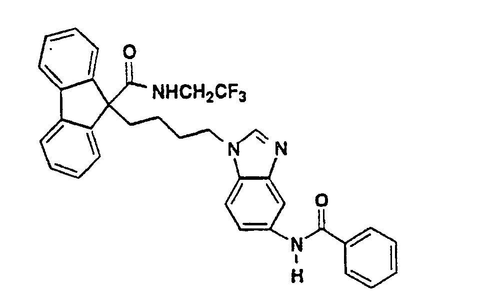 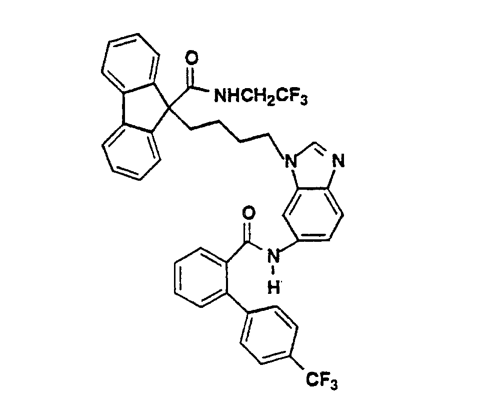 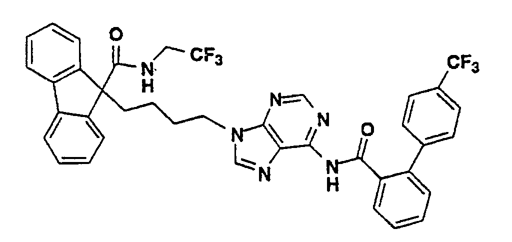 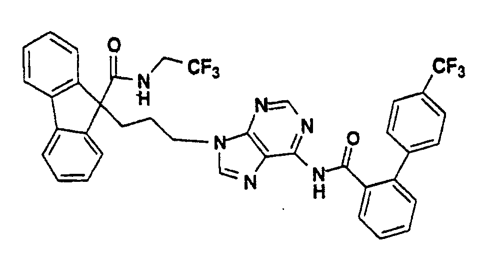 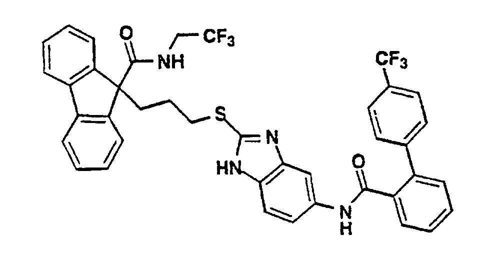 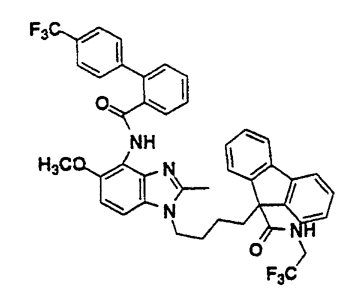 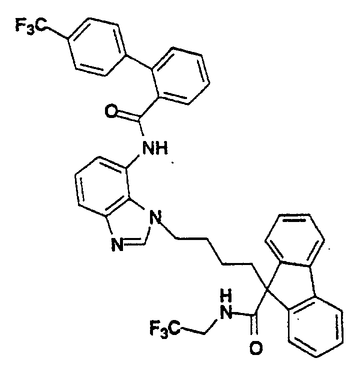 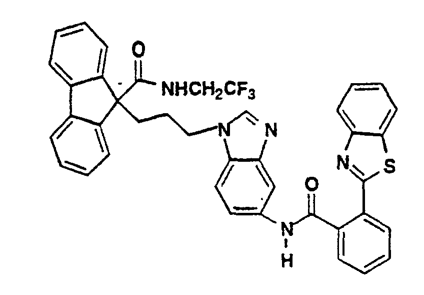 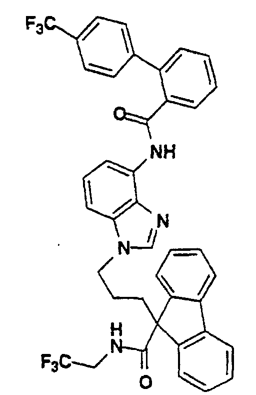 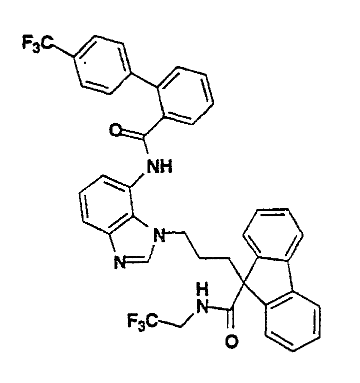 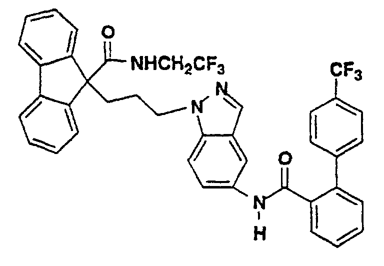 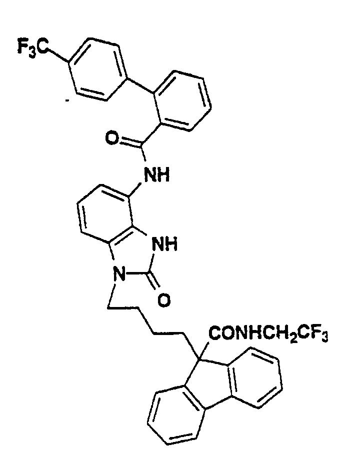 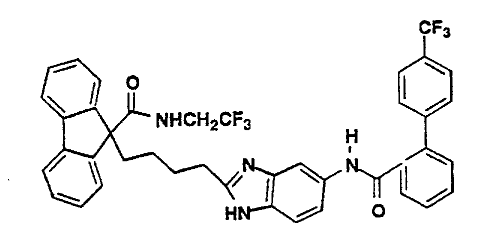 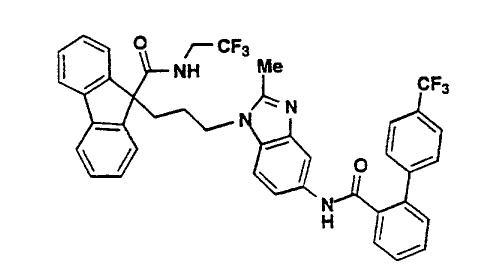 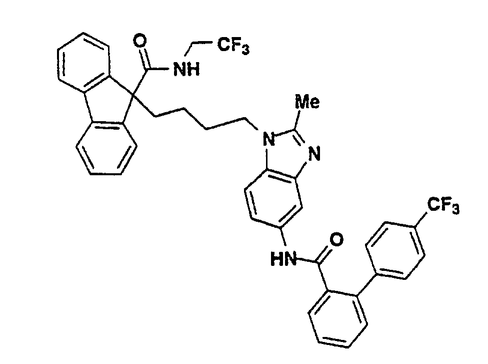 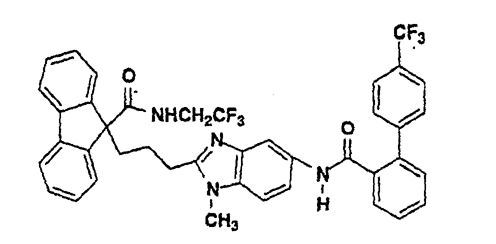 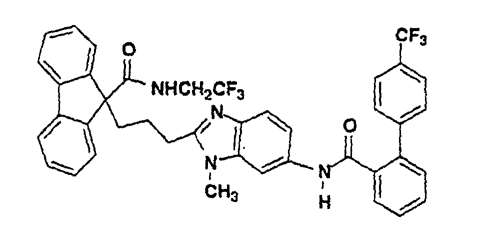 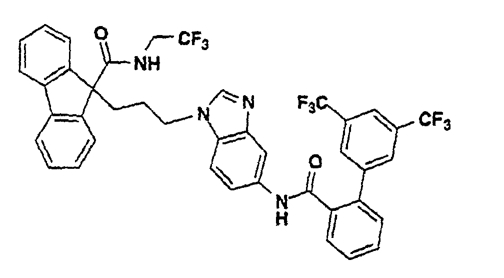 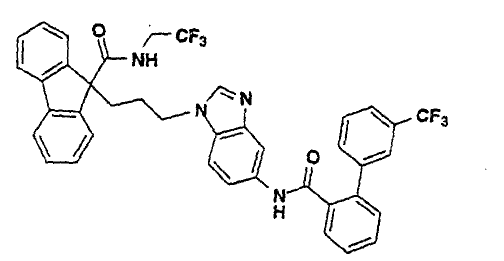 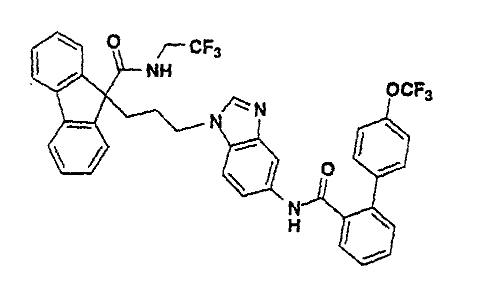 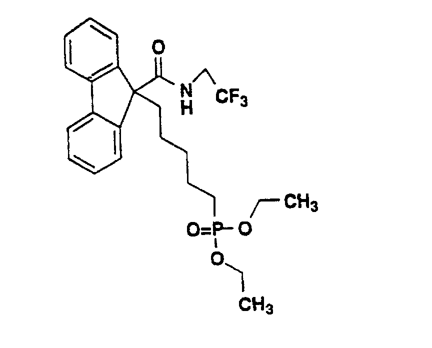 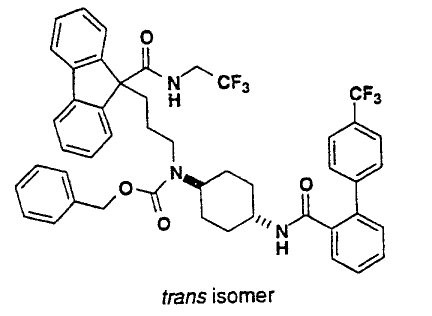 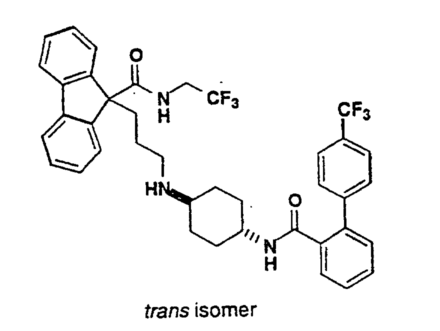 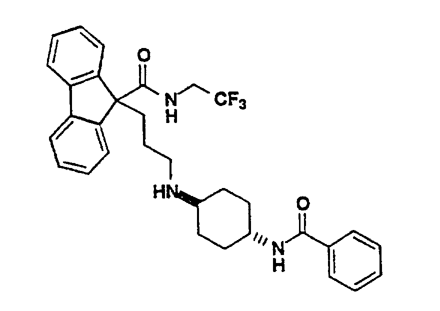 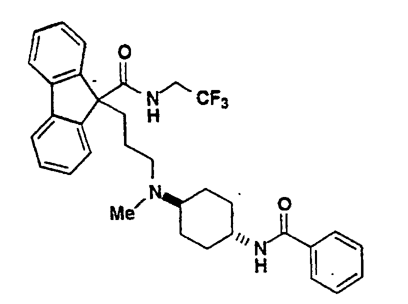 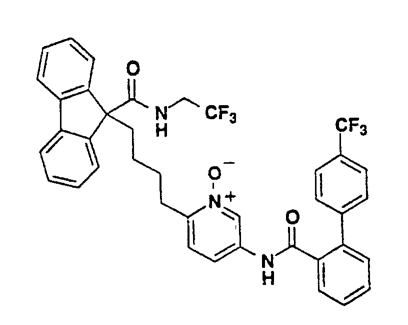 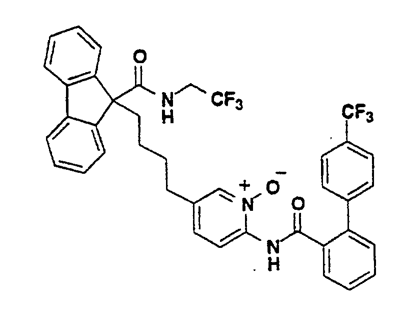 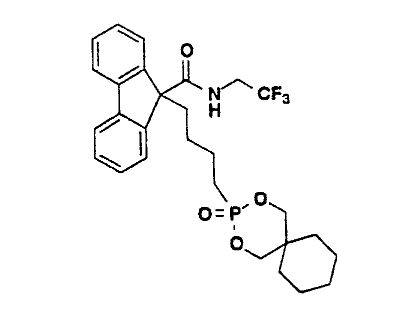 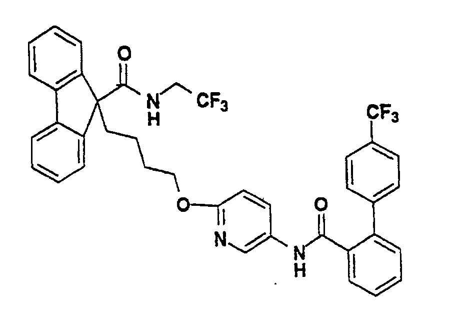 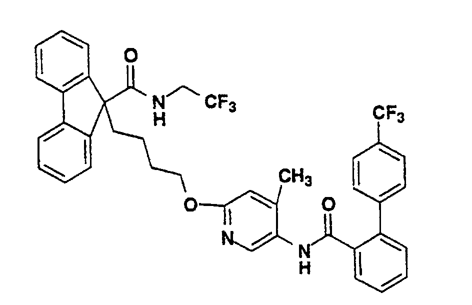 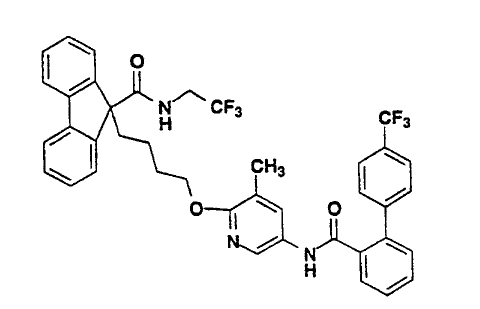 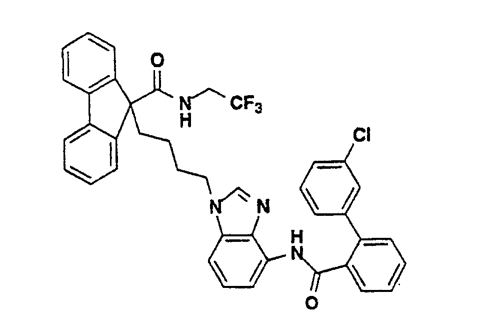 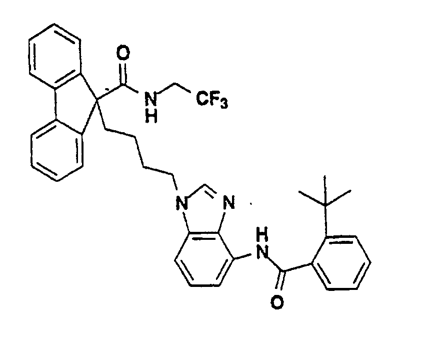 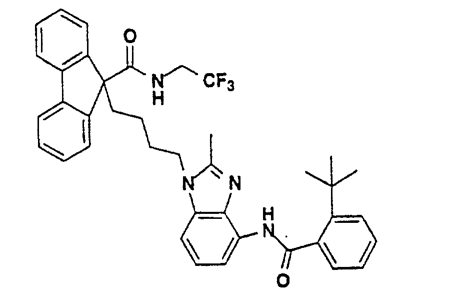 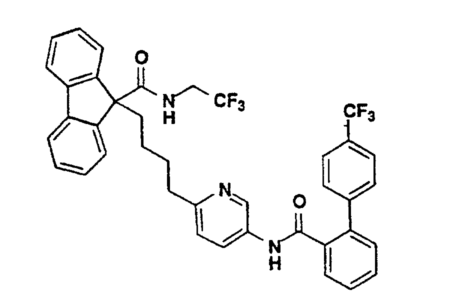 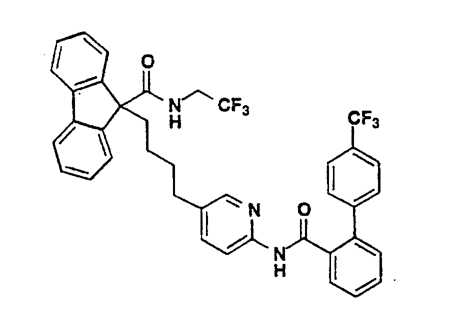 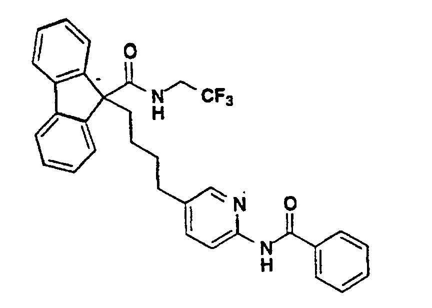 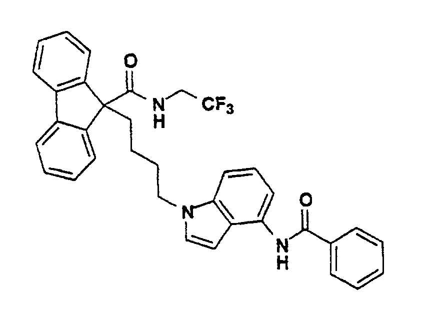 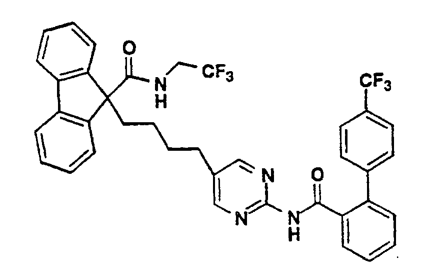 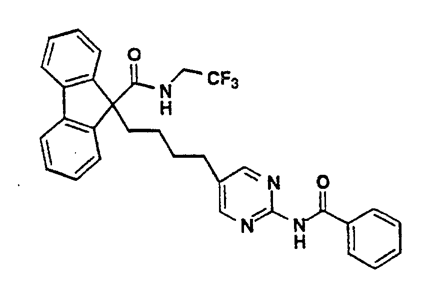 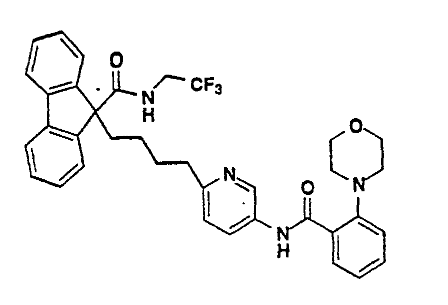 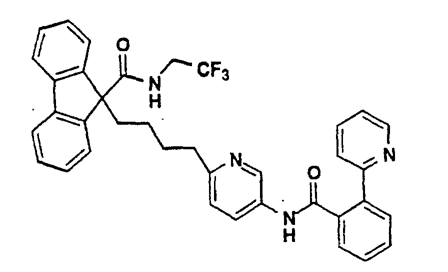 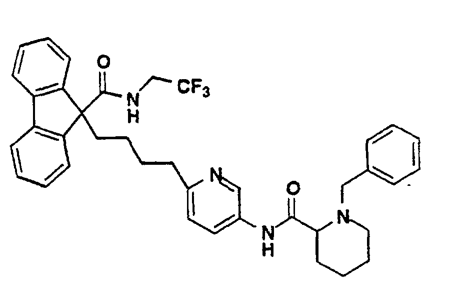 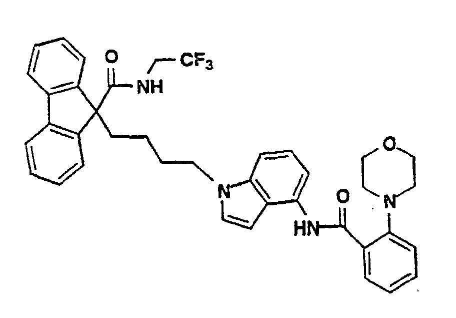 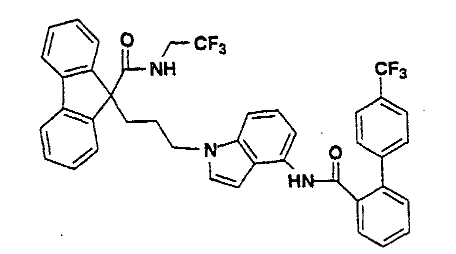 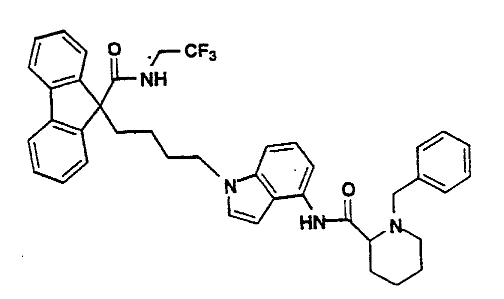 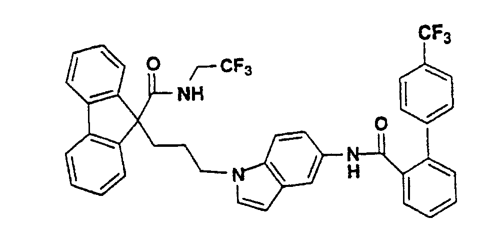 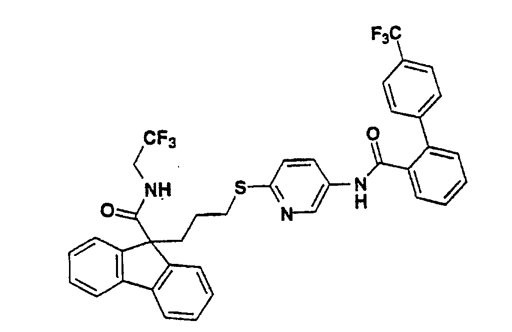 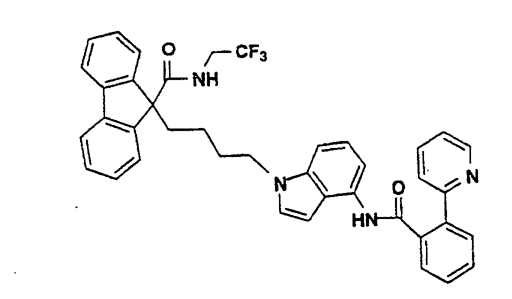 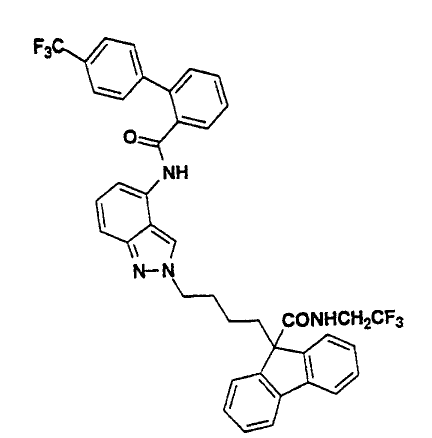 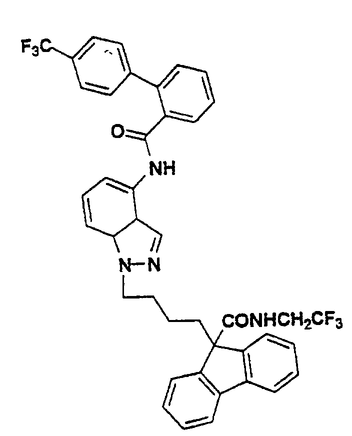 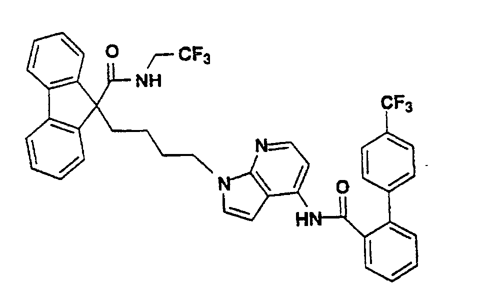 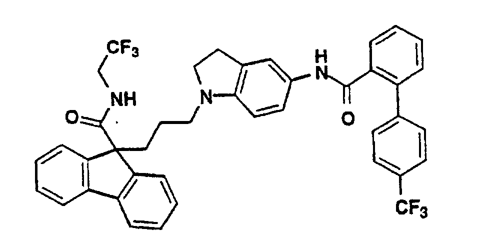 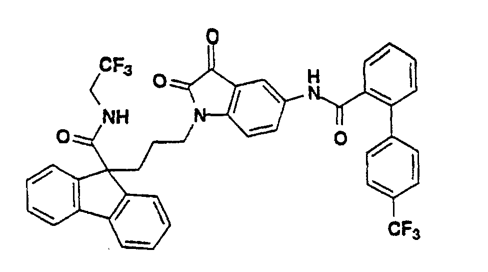 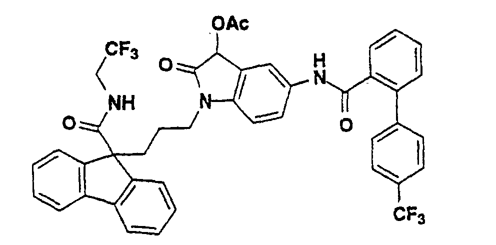 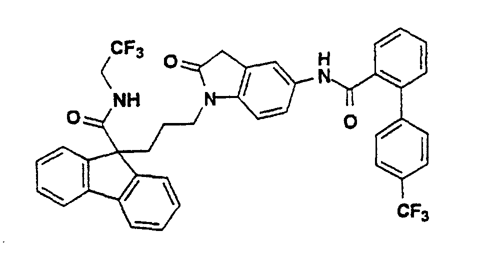 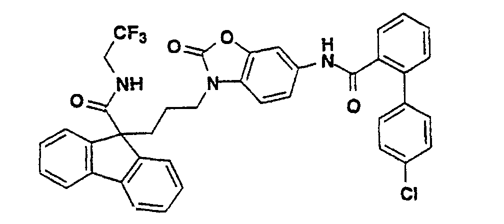 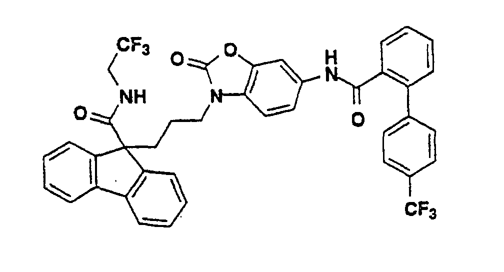 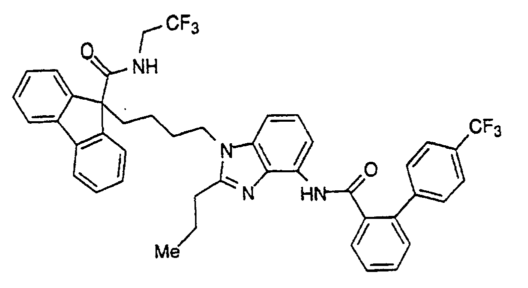 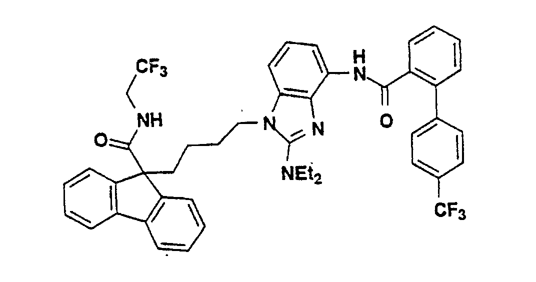 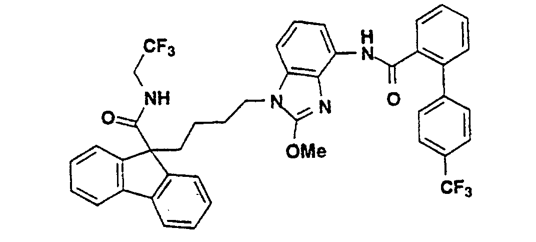 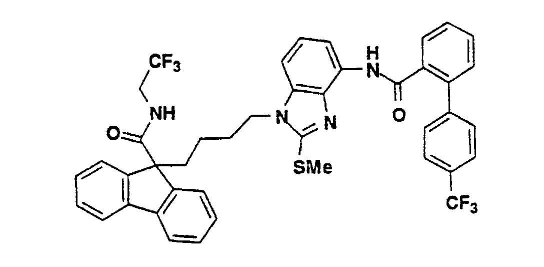 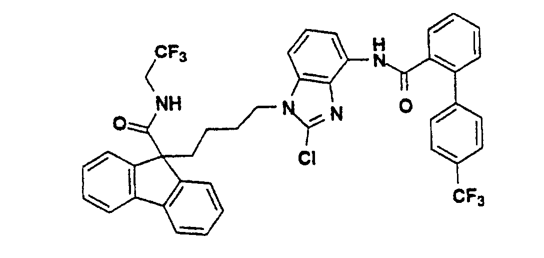 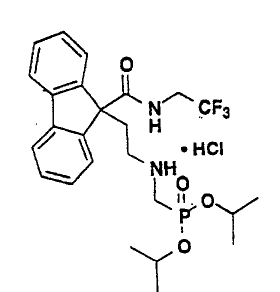 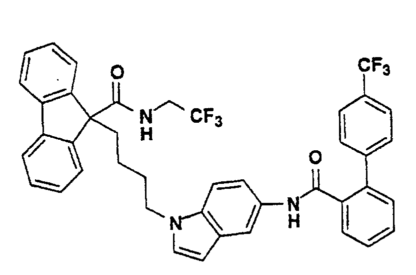 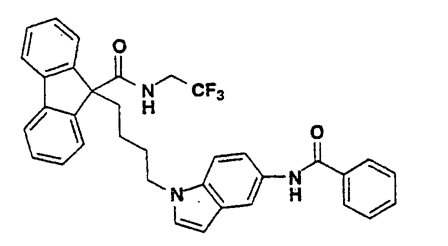 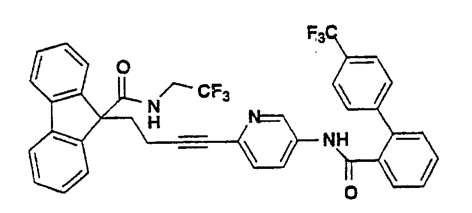 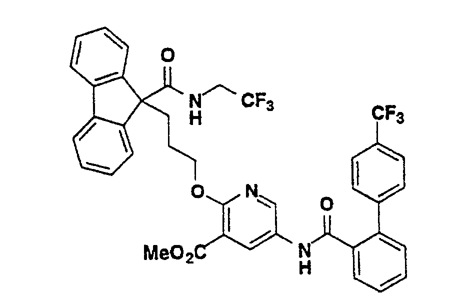 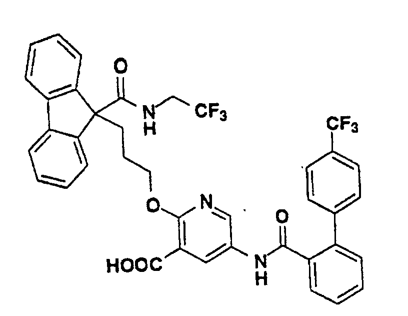 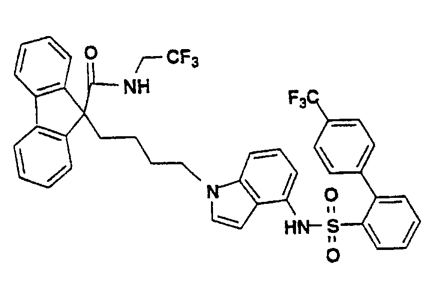 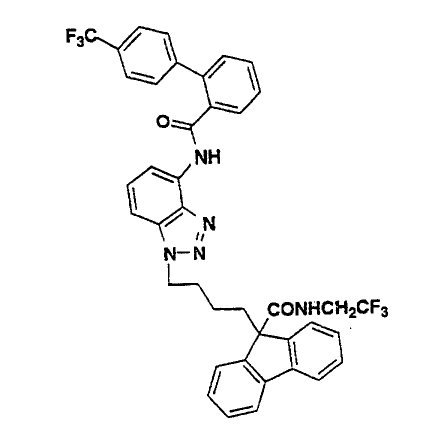 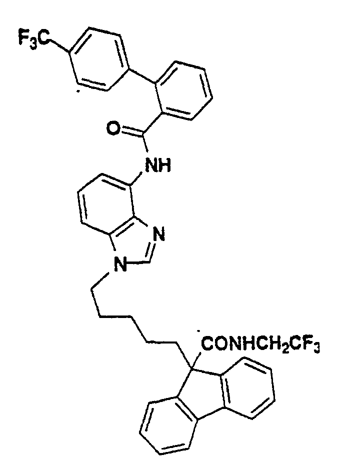 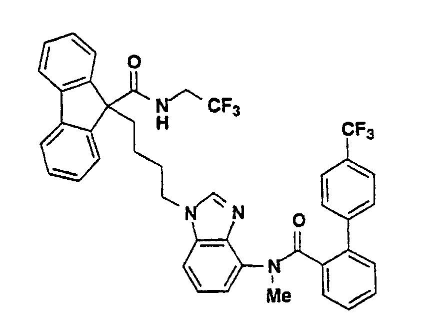 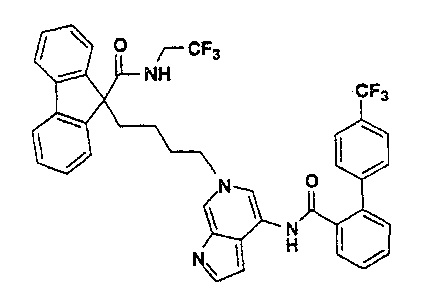 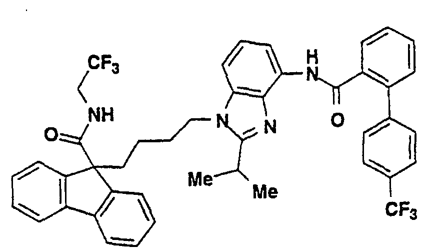 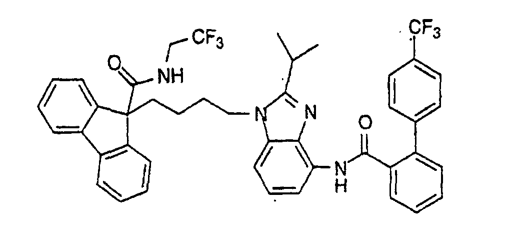 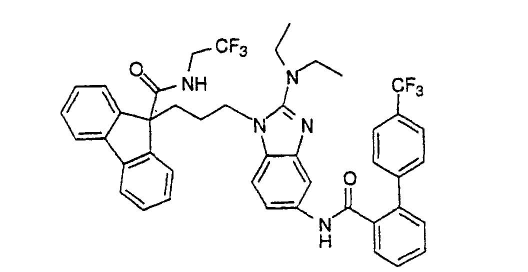 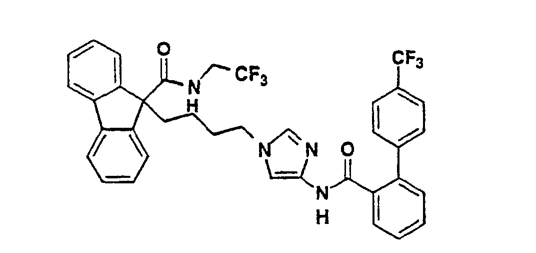 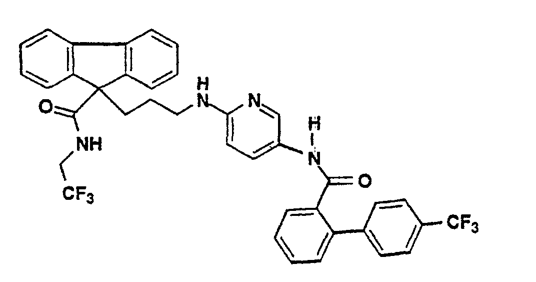 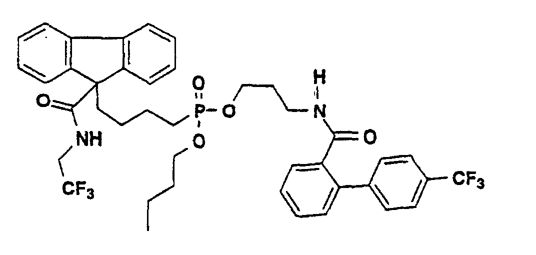 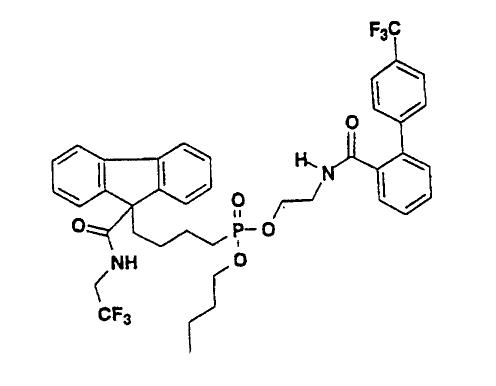 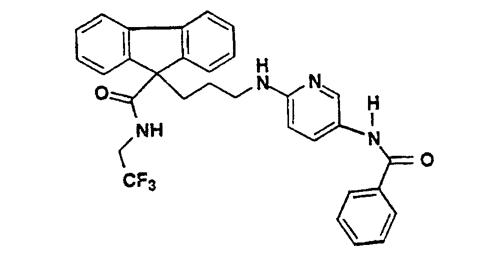 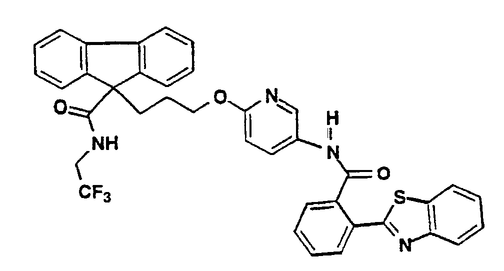 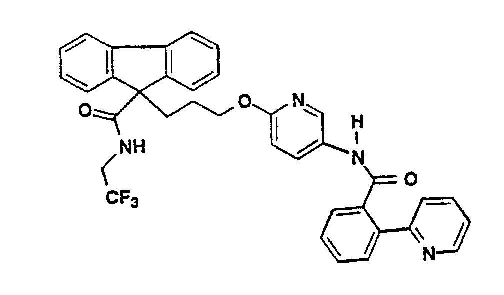 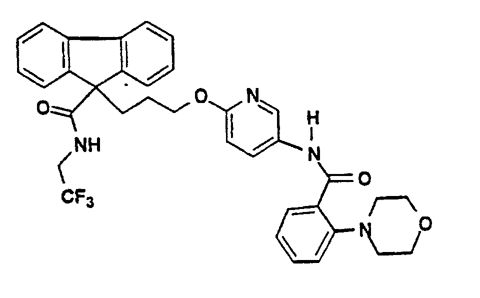 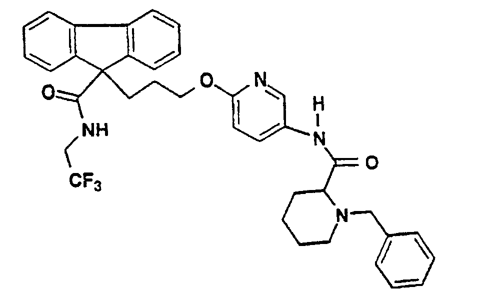 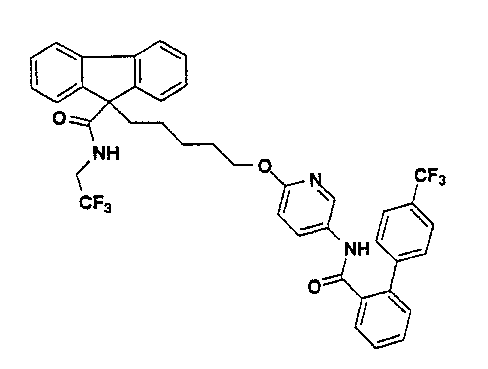 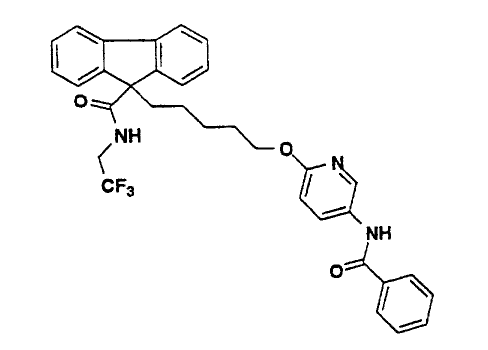 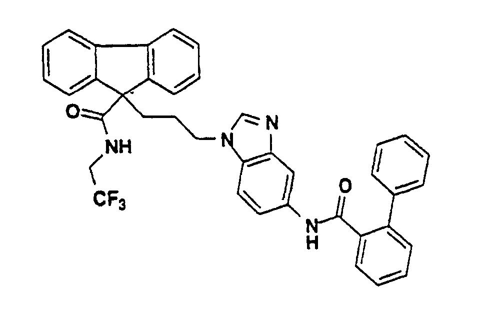 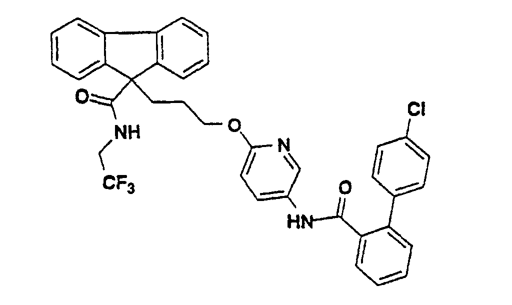 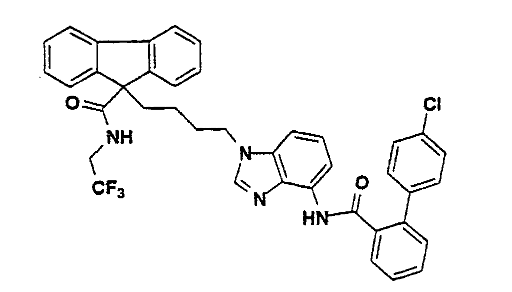 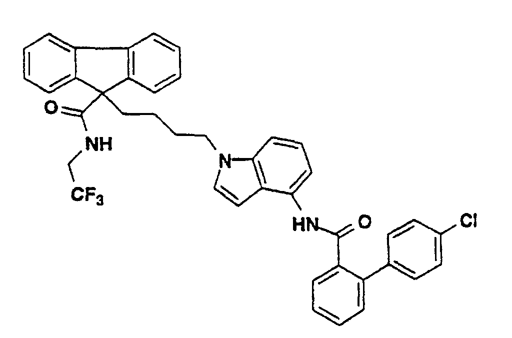 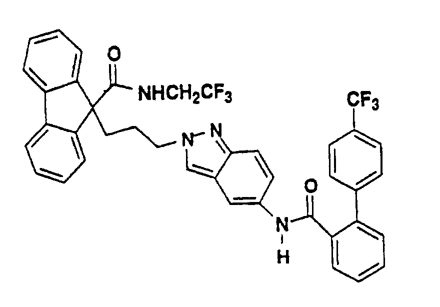 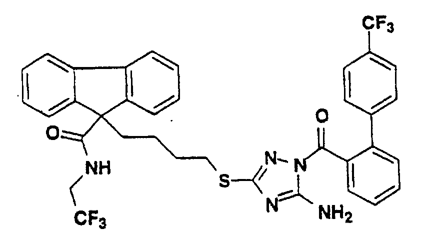 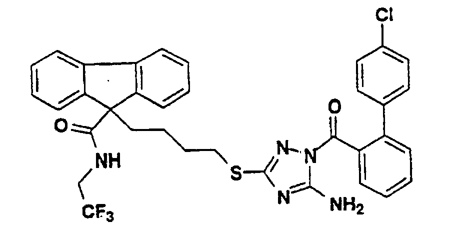 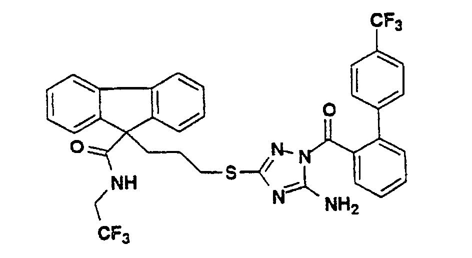 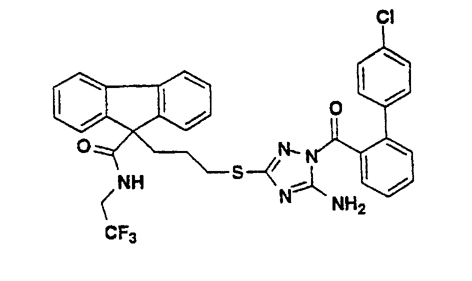 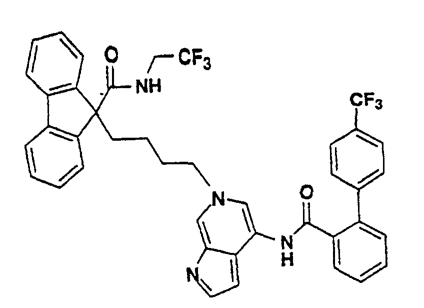 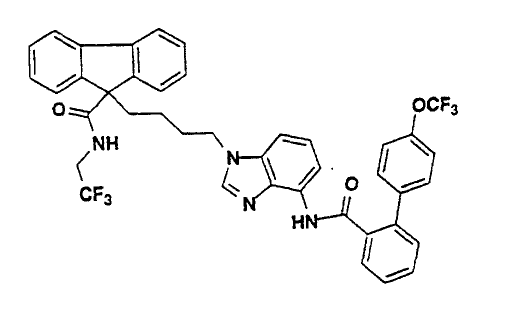 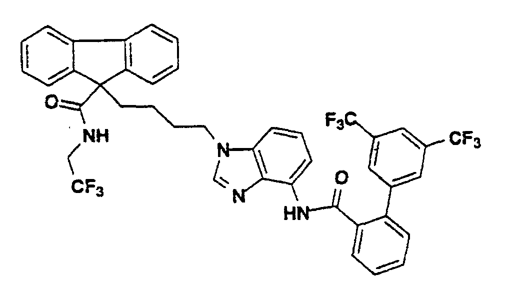 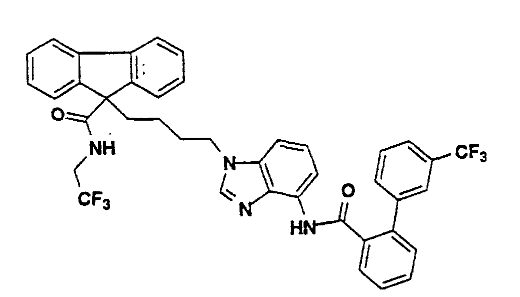 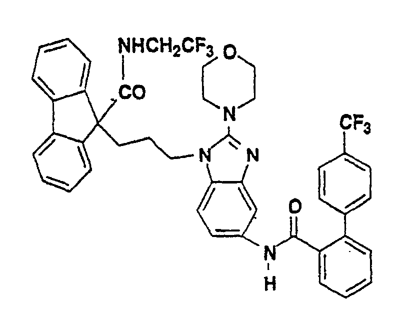 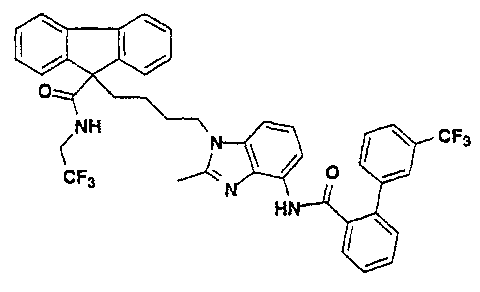 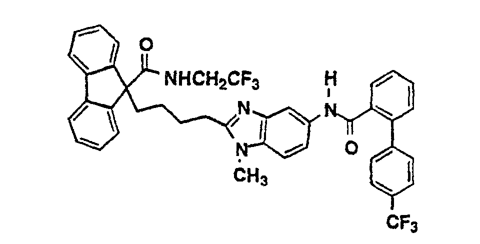 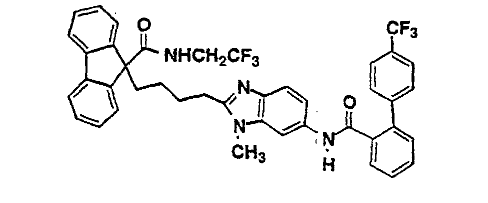 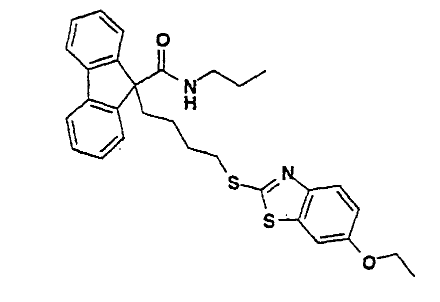 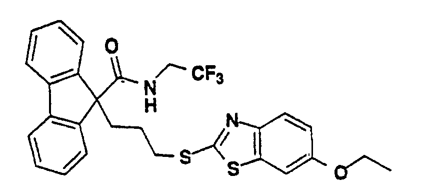 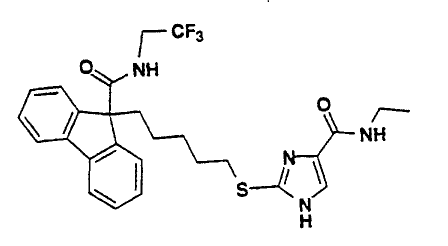 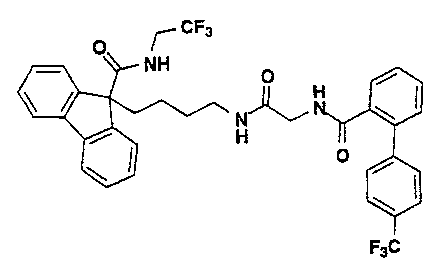 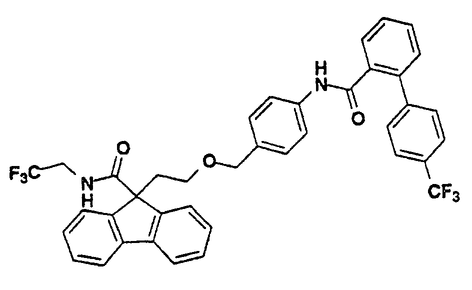 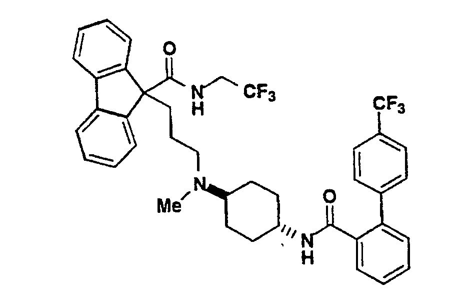 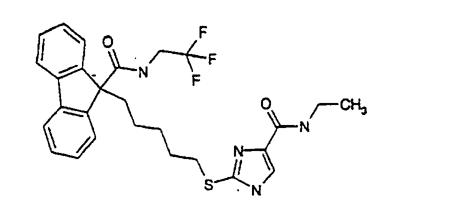 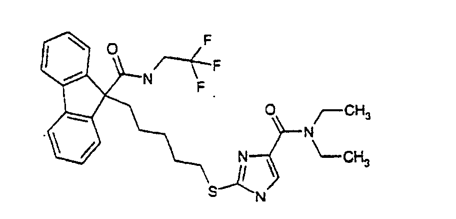 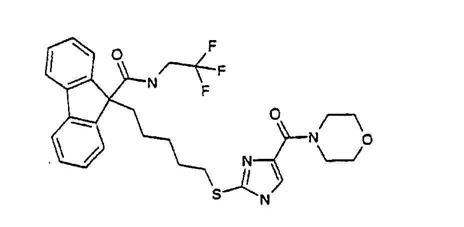 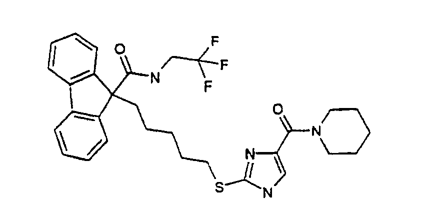 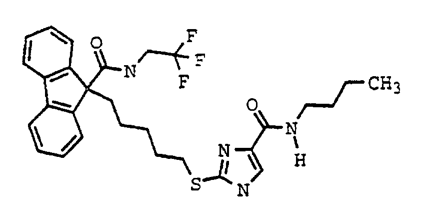 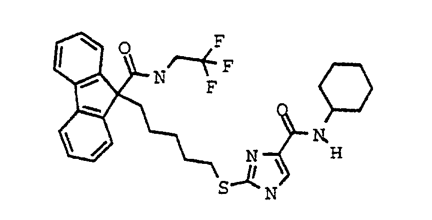 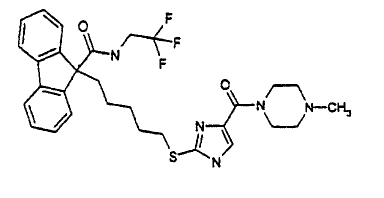 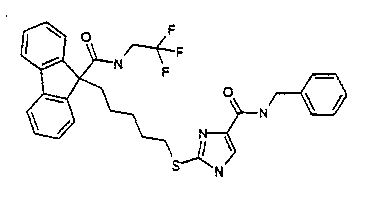 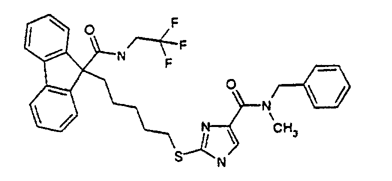 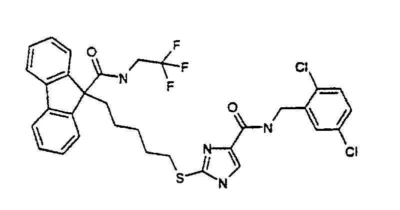 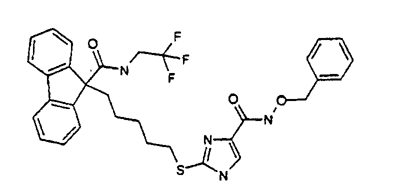 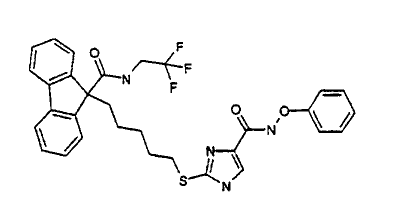 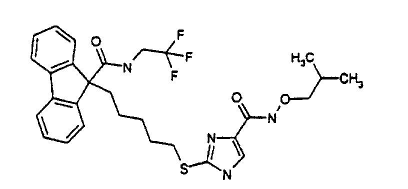 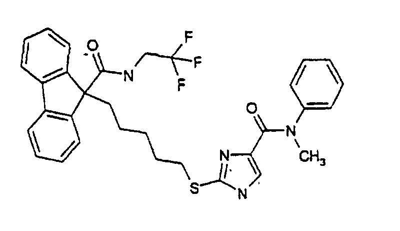 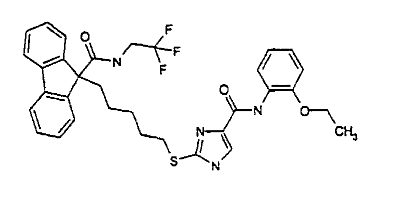 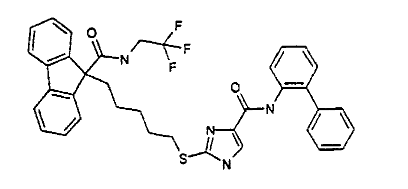 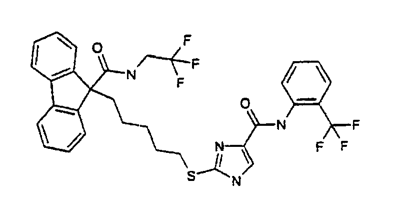 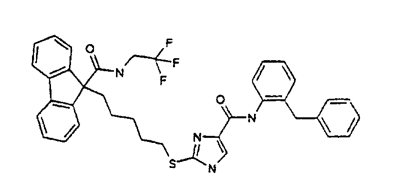 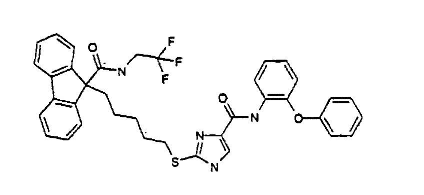 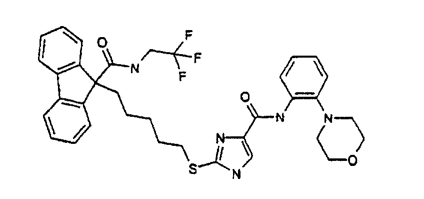 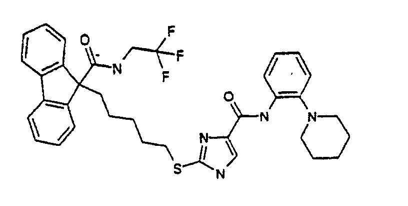 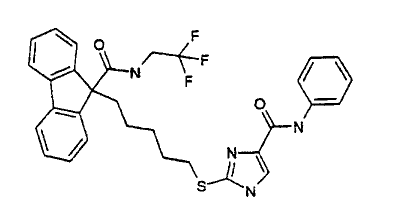 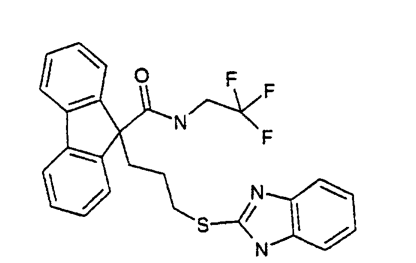 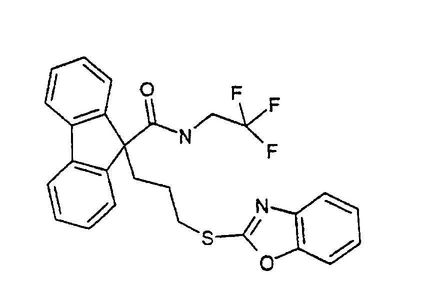 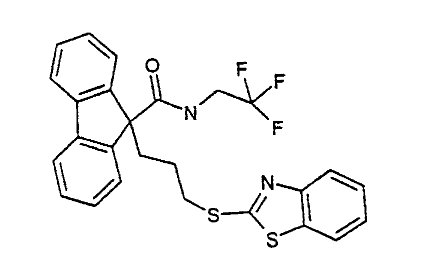 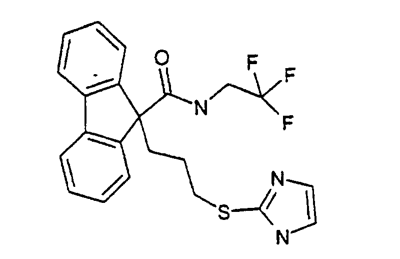 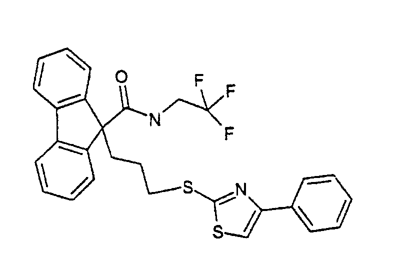 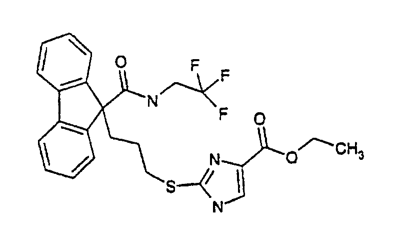 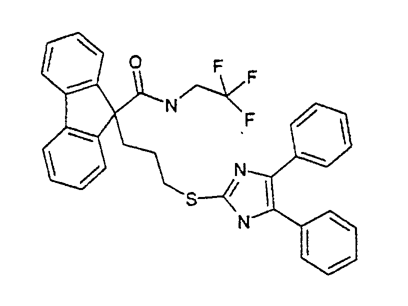 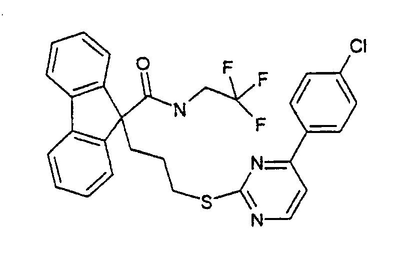 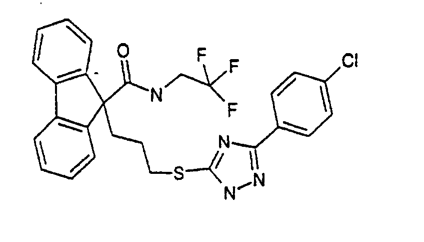 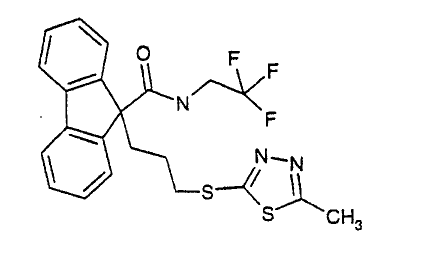 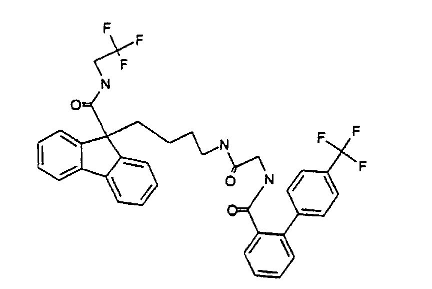 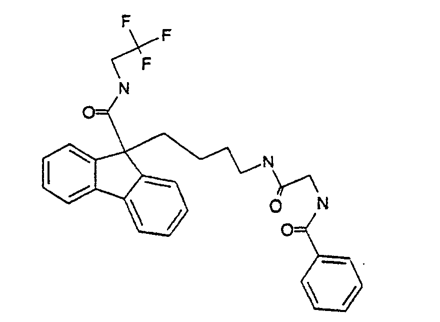 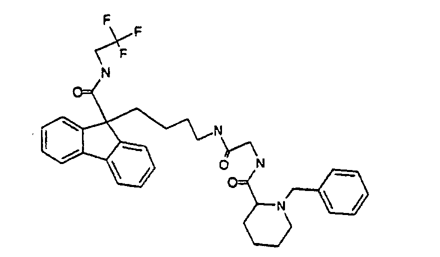 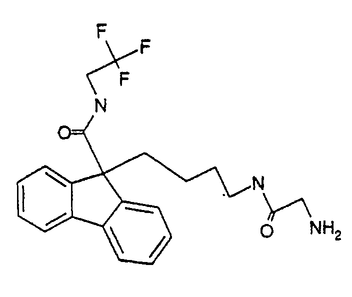 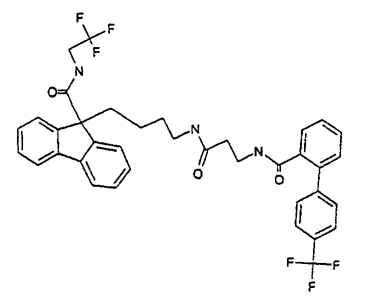 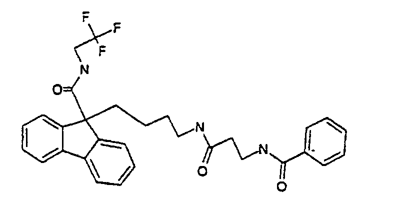 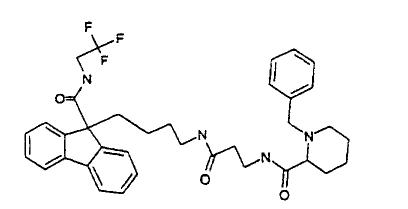 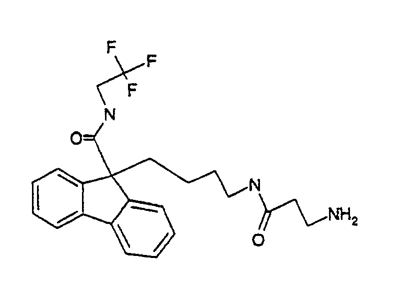 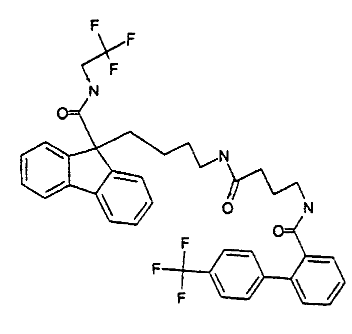 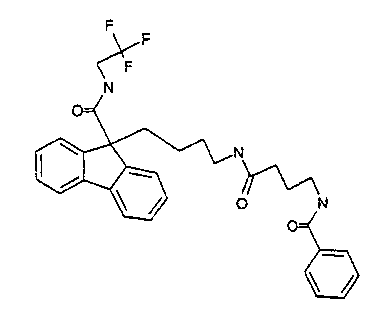 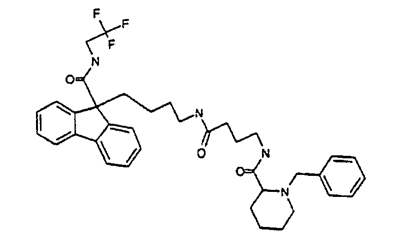 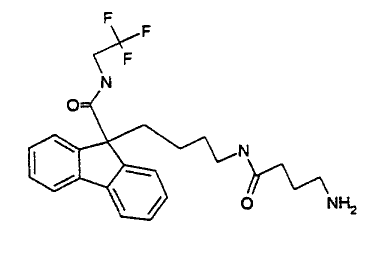 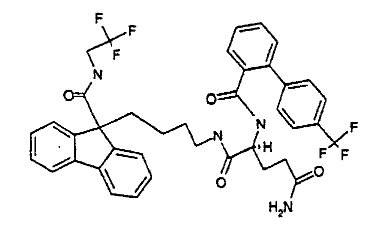 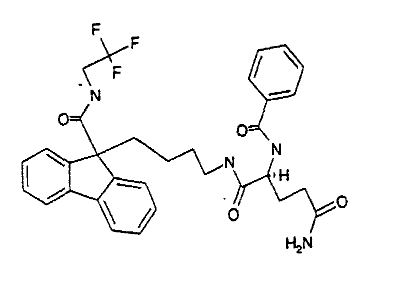 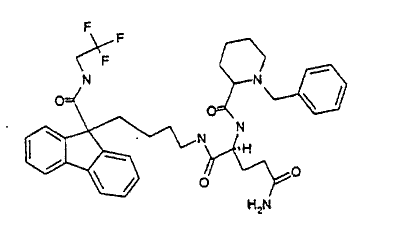 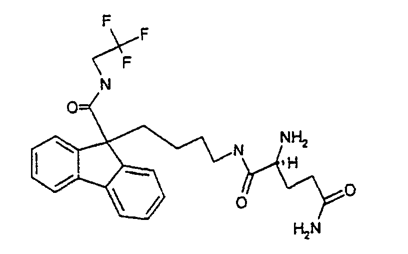 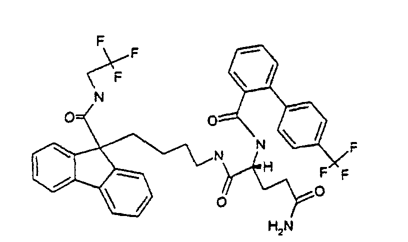 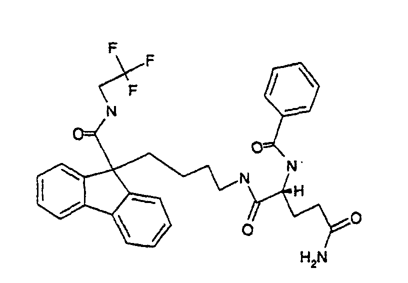 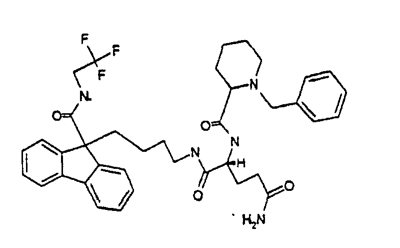 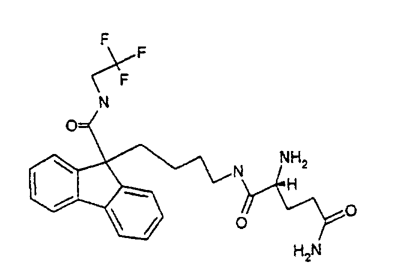 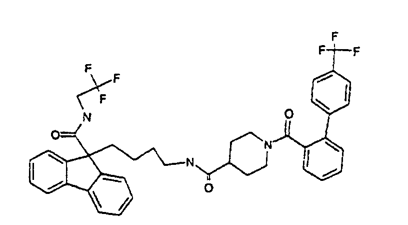 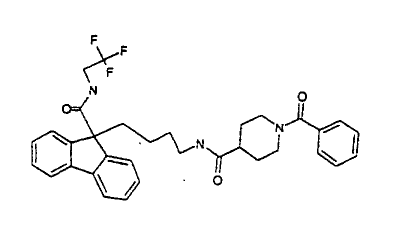 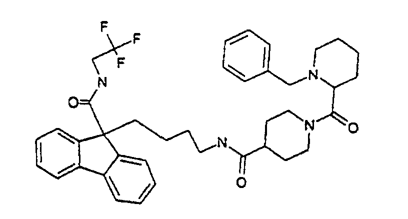 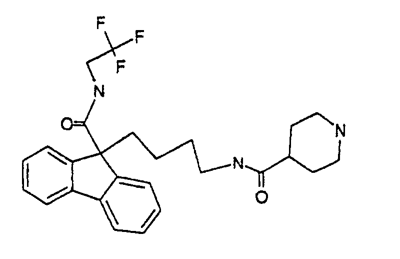 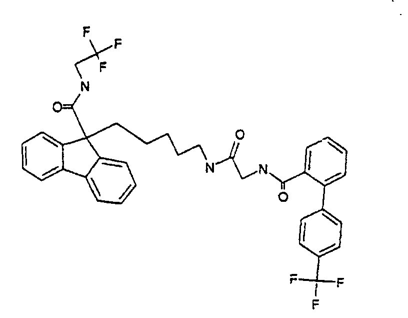 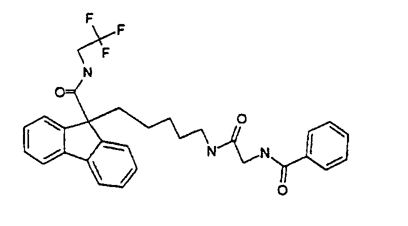 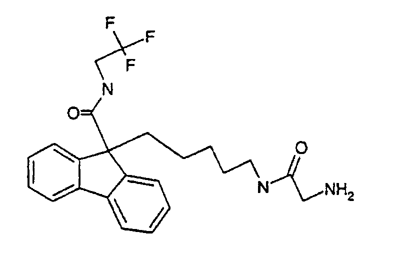 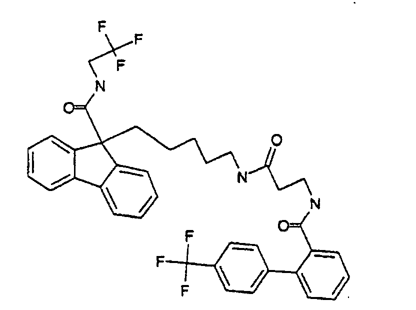 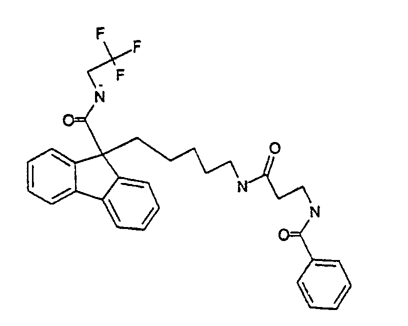 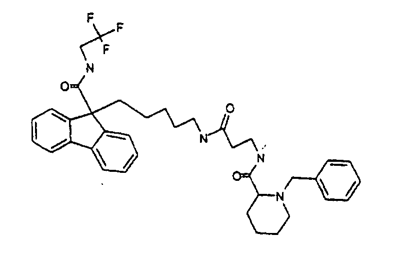 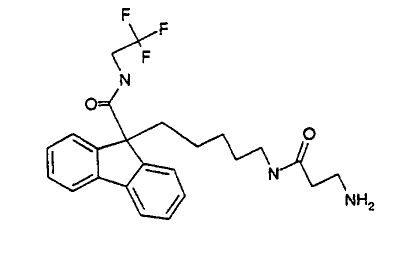 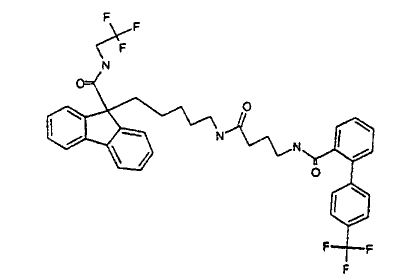 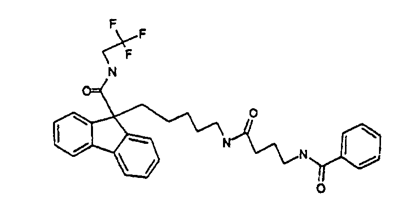 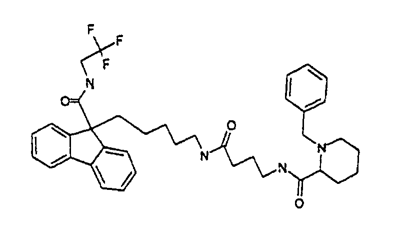 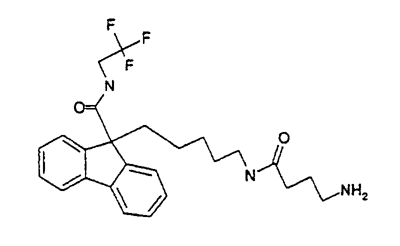 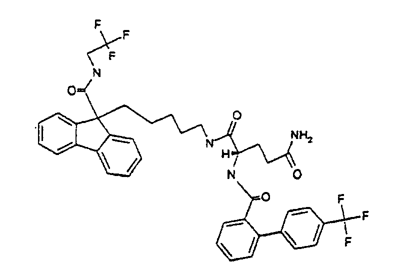 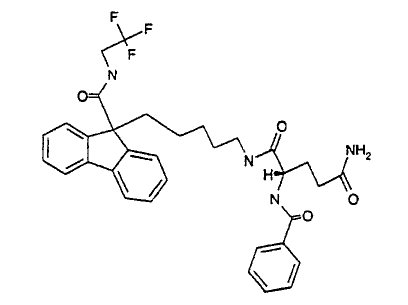 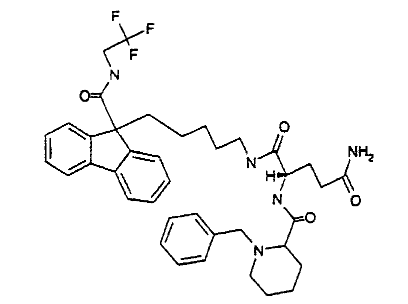 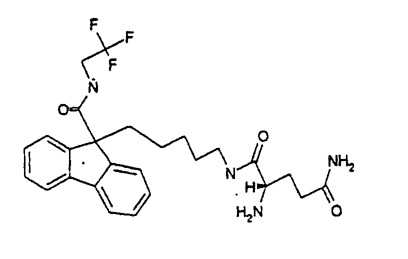 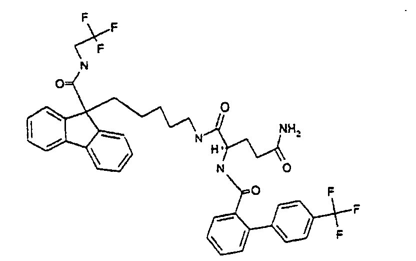 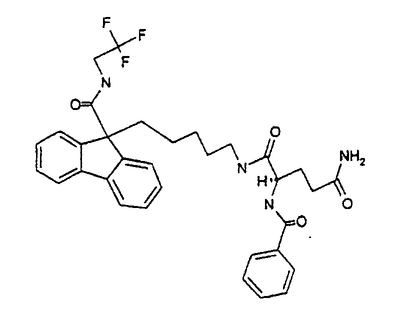 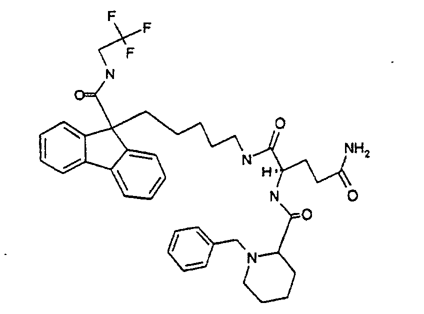 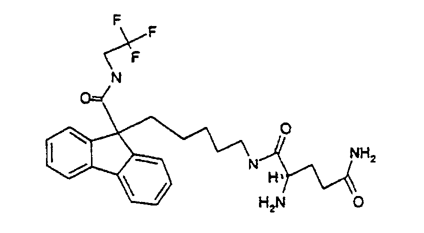 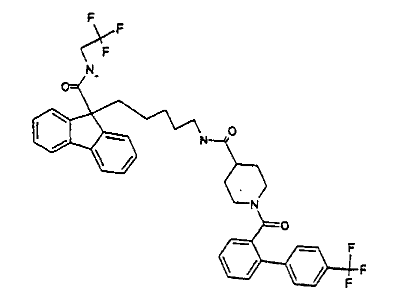 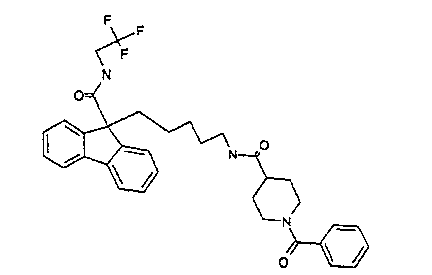 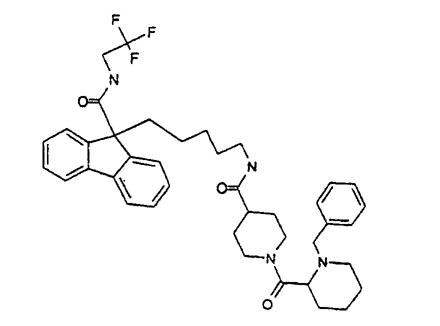 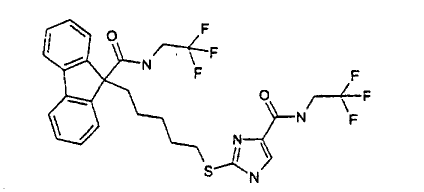 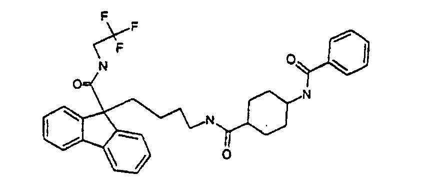 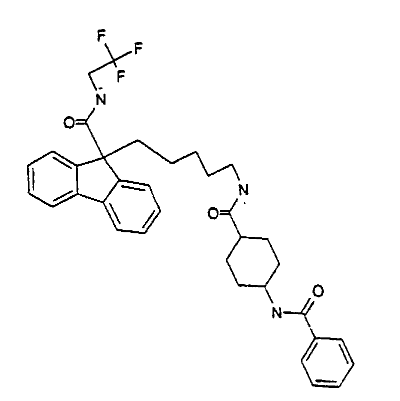 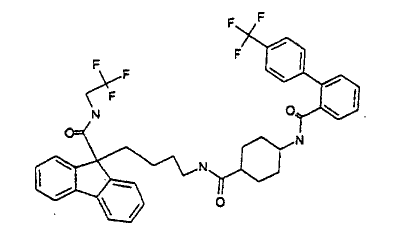 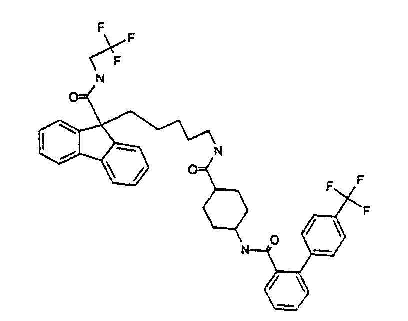 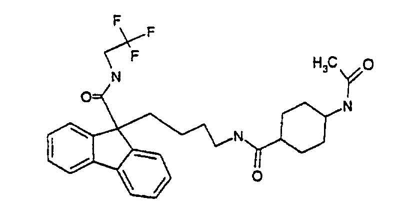 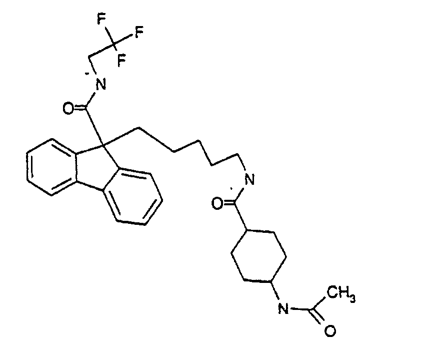 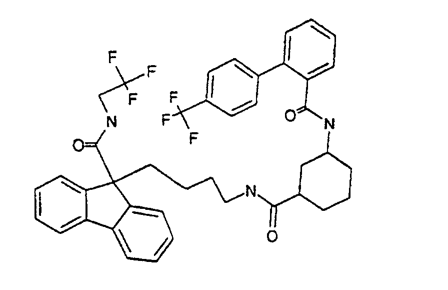 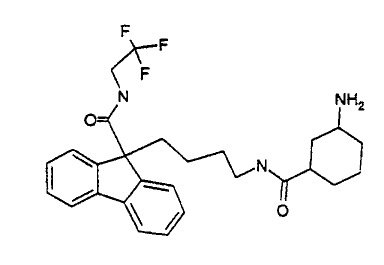 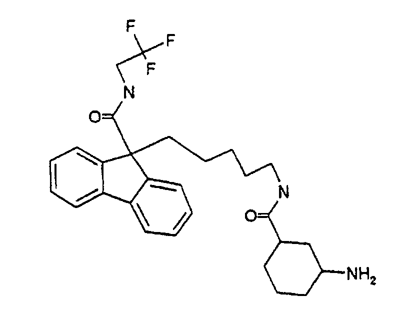 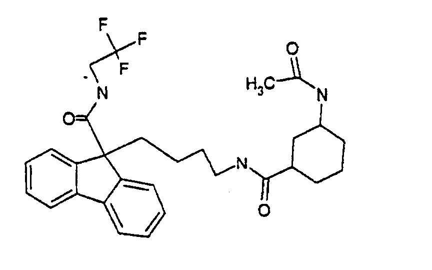 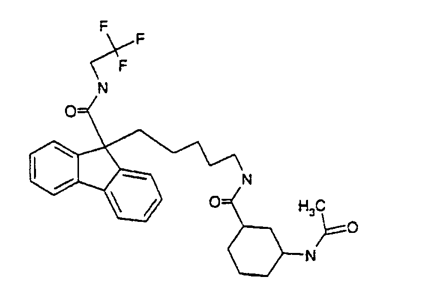 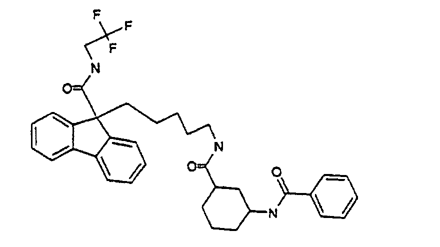 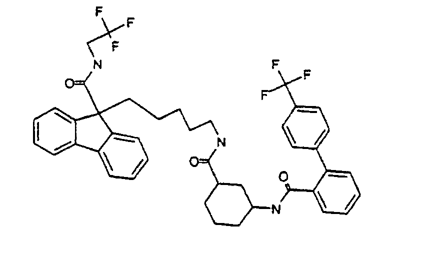 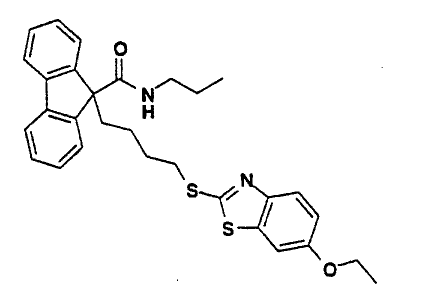 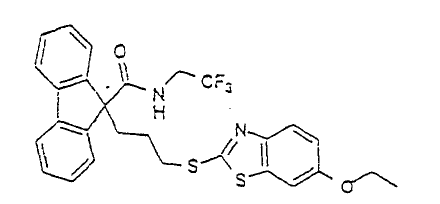 or
pharmaceutically acceptable salts thereof; esters thereof or prodrug esters thereof.

4. The compound as defined in Claim 2 wherein A is NH and R²L² is CF₃CH₂.

5. The compound as defined in Claim 1 having the formula 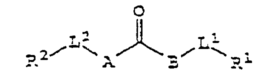 wherein B is 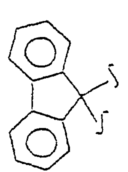 A is NH
L²R² is CH₂CF₃
L¹ is -CH₂CH₂CH₂- or -CH₂CH₂CH₂CH₂-, and
R¹ is heteroaryl which is a 5-membered aromatic ring which includes 2 nitrogen atoms, which ring is fused to an aryl ring and is substituted on the aryl moiety.

6. The compound as dsfined in Claim 1 whersin R¹ is

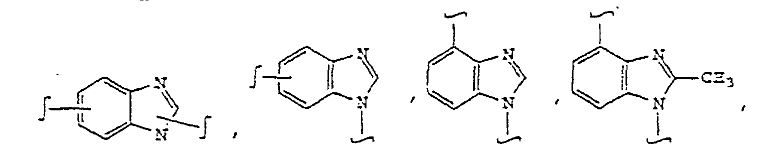

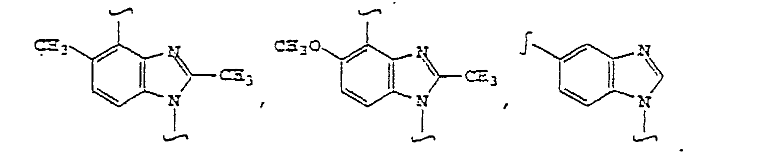

7. The compound ss defined in Claim 1 wherein R¹ is

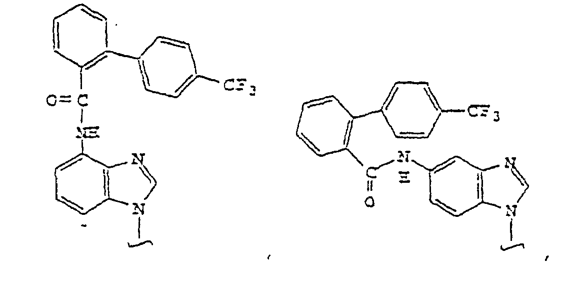

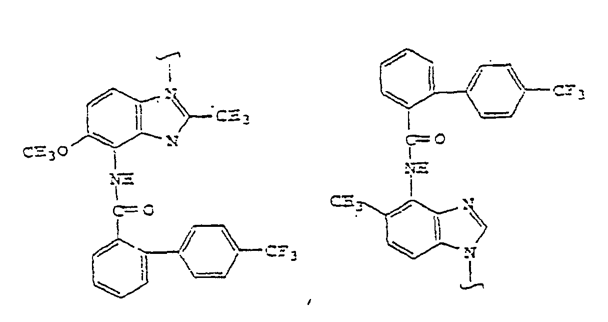

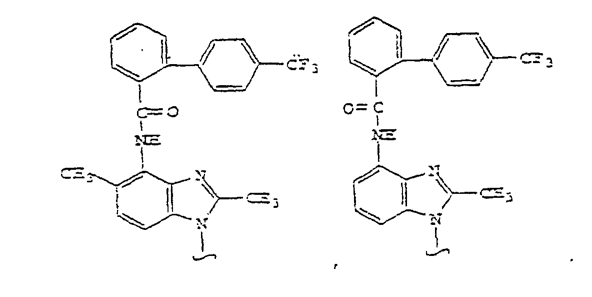

8. The compound as defined in Claim 5 having the structures 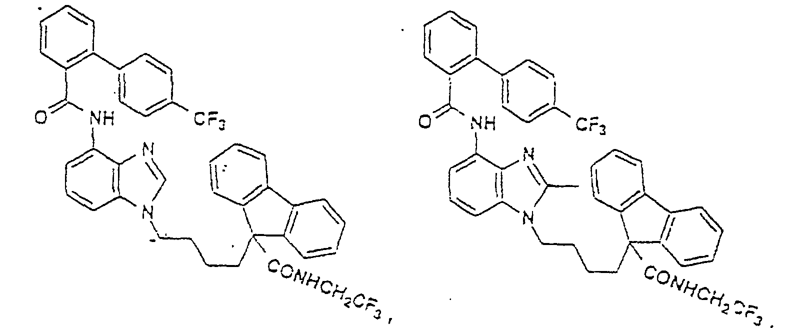 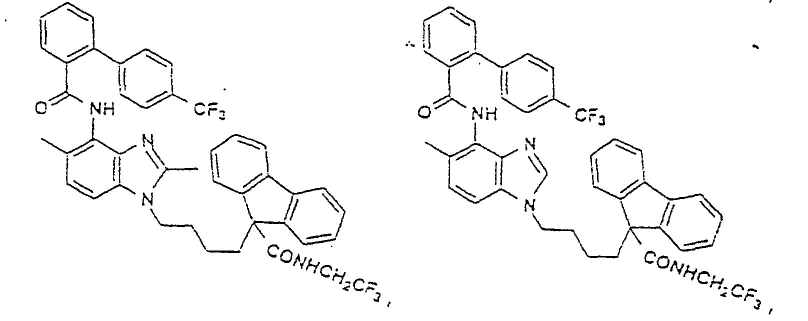 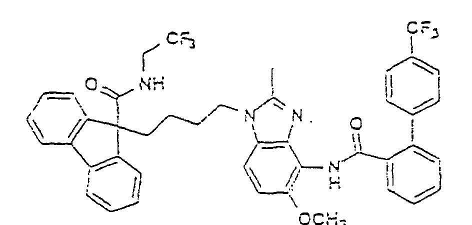 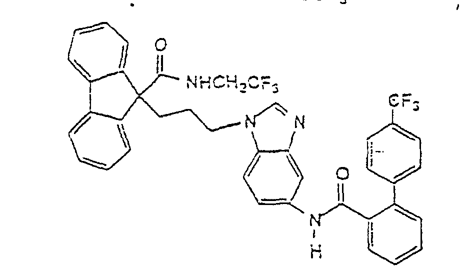 or a pharmaceutically acceptable salt thereof.

9. A pharmaceutical composition comprising a compound as defined in any one of claims 1 to 8 and a pharmaceutically acceptable carrier.

10. A use of a compound as defined in any one of claims 1 to 8 for the preparation of a pharmaceutical composition for decreasing the activity of a microsomal triglyceride transfer protein (MTP) in a mammalian species.

11. A use of a compound as defined in any one of claims 1 to 8 for the preparation or a pharmaceutical composition for lowering serum lipid levels, cholesterol and/or triglycerides, or inhibiting and/or treating hyperlipemia, hyperlipidemia, hyperlipoproteinemia, hypercholesterolemia, hyperglycemia and/or hypertriglyceridemia, and/or preventing, inhibiting or treating atherosclerosis, pancreatitis, noninsulin dependent diabetes, or obesity in a mammalian species.

## Patentansprüche

1. Verbindung, welche die Struktur 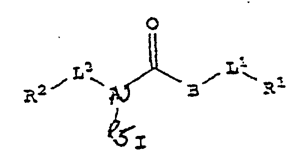 aufweist, einschließlich pharmazeutisch verträglicher Salze und N-Oxide davon,
wobei q 0, 1 oder 2 ist;
wobei R⁵ ein Wasserstoffatom oder ein C₁-C₁₂-Alkylrest ist oder R⁵ zusammen mit R² ein carbocyclisches oder heterocyclisches Ringsystem mit 4 bis 8 Elementen im Ring bildet;
B ein Rest vom Fluorenyltyp mit der Struktur 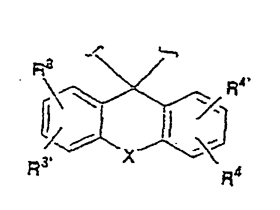 ist;
R¹ ein Wasserstoffatom, ein Alkyl-, Alkenyl-, Alkinyl oder Alkoxylrest, ein (Alkyloder Aryl)₃-Si-Rest (wobei jeder Alkyl- oder Arylrest unabhängig ist), ein Cycloalkyl-, Cycloalkenyl-, substituierter Alkylamino-, substituierter Arylalkylamino-, Aryl-, Arylalkyl-, Arylamino-, Aryloxy-, Heteroaryl-, Heteroarylamino-, Heteroaryloxy-, Arylsulfonylamino-, Heteroarylsulfonylamino-, Arylthio-, Arylsulfinyl-, Arylsulfonyl-, Alkylthio-, Alkylsulfinyl-, Alkylsulfonyl-, Cycloheteroalkyl-, Heteroarylthio-, Heteroarylsulfinyl-, Heteroarylsulfonylrest, -PO(R¹³)(R¹⁴) (wobei R¹³ und R¹⁴ unabhängig ein Alkyl-, Aryl-, Alkoxy-, Aryloxy-, Heteroaryl-, Heteroarylalkyl-, Heteroaryloxy-, Heteroarylalkoxy-, Cycloheteroalkyl-, Cycloheteroalkylalkyl-, Cycloheteroalkoxy- oder ein Cycloheteroalkylalkoxyrest sind), ein Aminocarbonylrest (wobei die Aminogruppe gegebenenfalls mit einem oder zwei Aryl-, Alkyl- oder Heteroarylresten substituiert sein kann), eine Cyanogruppe, ein 1,1-(Alkoxyl oder Aryloxy)₃-alkylrest (wobei die beiden Aryl- oder Alkylsubstituenten unabhängig definiert oder aneinander gebunden sein können, um einen Ring zu bilden, welcher in der 2-Position an L¹ gebunden ist (oder an L² im Falle von R²)), 1,3-Dioxan oder 1,3-Dioxolan, in der 4-Postition gebunden an L¹ (oder an L² im Falle von R²), der Rest R¹ gegebenenfalls mit 1, 2, 3 oder 4 Substituenten substituiert sein kann, welche einer der Reste R³ oder R¹ oder ein Alkylcarbonylamino-, Cycloalkylcarbonylamino-, Arylcarbonylamino-, Heteroarylcarbonylamino-, Alkoxycarbonylamino-, Aryloxycarbonylamino-, Heteroaryloxylcarbonylaminorest, ein Uriedorest (wobei die Uriedo-Stickstoffatome gegebenenfalls mit einem Alkyl-, Aryl- oder Heteroarylrest substituiert sein können), ein Heterocyclylcarbonylaminorest (wobei der Heterocyclus über ein Stickstoff- oder Kohlenstoffatom an die Carbonylgruppe gebunden ist), ein Alkylsulfonylamino-, Arylsulfonylamino-, Heteroarylsulfonylaminorest, 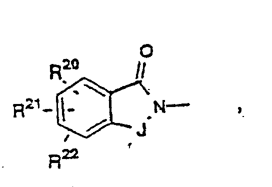 wobei J der Rest CHR²³, 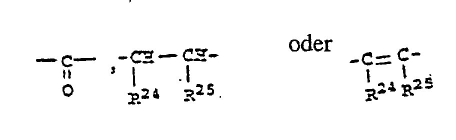 ist;
R²³, R²⁴ und R²⁵ unabhängig ein Wasserstoffatom, ein Alkyl-, Alkenyl-, Alkinyl-, Aryl-, Arylalkyl-, Heteroaryl-, Heteroarylalkyl-, Cycloalkyl- oder ein Cycloalkylalkylrest sind;
R²⁰, R²¹, R²² unabhängig ein Wasserstoff-, Halogenatom, ein Alkyl-, Alkenyl-, Alkoxy-, Aryloxy-, Aryl-, Arylalkylrest, Alkylmercapto-, Arylmercapto-, Cycloalkyl-, Cycloalkylalkyl-, Heteroaryl-, Heteroarylalkylrest, eine Hydroxygruppe oder ein Halogenalkylrest sind und diese Substituenten entweder direkt an R¹ oder über einen Alkylenrest an einer offenen Position gebunden sein können;
R² unabhängig einer für R¹ angegebenen Reste, H, ein Polyhalogenalkyl- oder ein Cycloheteroalkylrest ist und gegebenenfalls mit einem bis vier einer der für R³ definierten Reste substituiert oder für R¹ definierte Substituenten sein kann;
L¹ ein Verbindungsrest, welcher bis zu 10 Kohlenstoffatome in einer linearen Kette, einschließlich einem Alkylen-, Alkenylen- oder Alkinylenrest enthält, welche innerhalb der Verbindungskette folgendes enthalten kann: ein bis zwei Alkene, ein bis zwei Alkine, ein Sauerstoffatom, eine Amino-, Oxogruppe und mit einem bis fünf Alkylresten oder Halogenatomen substituiert sein kann;
L² gleich oder von L¹ verschieden sein kann und unabhängig einen der vorstehend angegebenen L¹-Reste oder eine Einfachbindung sein kann;
R³, R³', R⁴ und R⁴' gleich oder verschieden sein können und unabhängig ausgewählt sind aus H, einem Halogenatom, CF₃, einem Halogenalkylrest, einer Hydroxygruppe, einem Alkoxy-, Alkyl-, Aryl-, Alkenyl-, Alkenyloxy-, Alkinyl-, Alkinyloxy-, Alkanoylrest, einer Nitro-, Aminogruppe, einem Thiol-, Alkylthio-, Alkylsulfinyl-, Alkylsulfonylrest, einer Carboxygruppe, einem Alkoxycarbonyl-, Aminocarbonyl-, Alkylcarbonyloxy-, Alkylcarbonylamino-, Cycloheteroalkyl-, Cycloheteroalkylalkylrest, einer Cyanogruppe, einem Ar-, Ar-Alkylrest, ArO, einer Ar-Aminogruppe, einem Ar-Thio-, Ar-Sulfinyl-, Ar-Sulfonyl-, Ar-Carbonyl-, Ar-Carbonyloxy- oder einem Ar-Carbonylaminorest, wobei Ar ein Aryl- oder Heteroarylrest ist und Ar gegebenenfalls 1, 2 oder 3 an Ar annelierte Ringe einschließen kann;
X eine Bindung oder einer der folgenden Reste ist:
mit den folgenden Maßgaben:
(a) wenn R¹ ein unsubstituierter Alkyl- oder unsubstituierter Arylalkylrest ist, L¹ keine Aminogruppe enthalten kann,
(b) wenn R¹ ein Alkylrest ist, L¹ keine Amino- und Oxogruppe in benachbarten Positionen enthalten kann (um einen Amidorest zu bilden);
(d) wenn R¹ eine Cyanogruppe ist, L¹ mehr als 2 Kohlenstoffatome aufweisen muss;
(e) R¹L¹ mindestens 3 Kohlenstoffatome enthalten muss;
bezogen auf Verbindungen, in denen R¹ oder R² ein Cycloheteroalkylrest, R¹ und R² ausschließlich ein 1-Piperidinyl-, 1-Pyrrolidinyl-, 1-Azetidinyl- oder 1-(2-Oxopyrrolidinyl)-rest sind, wobei der Begriff "Alkylrest" oder "Alk", wie er hier allein oder als Teil eines anderen Restes verwendet wird, sowohl geradkettige als auch verzweigte Kohlenwasserstoffe, welche 1 bis 40 Kohlenstoffatome enthalten, mehrere verzweigte Isomere davon und Reste, welche 1 bis 4 Substituenten einschließen, welche einer der Reste R³ sein können, oder die hier angeführten Substituenten für R¹ einschließt;
der Begriff "Cycloalkylrest", wie er hier allein oder als Teil eines anderen Restes verwendet wird, gesättigte oder teilweise ungesättigte (enthaltend 1 oder 2 Doppelbindungen) cyclische Kohlenwasserstoffreste, welche 1 bis 3 Ringe, einschließlich einem monocyclischen Alkylrest, einem bicyclischen Alkylrest und einem tricyclischen Alkylrest, enthalten, welche insgesamt 3 bis 20 die Ringe bildenden Kohlenstoffatome, bevorzugt 4 bis 12 die Ringe bildenden Kohlenstoffatome enthalten und welche an einen aromatischen Ring wie für den Arylrest beschrieben, anneliert sein können, einschließt;
der Begriff "Cycloalkenylrest", wie er hier allein oder als Teil eines anderen Restes verwendet wird, cyclische Kohlenwasserstoffe, welche 5 bis 20 Kohlenstoffatome und 1 oder 2 Doppelbindungen enthalten, betrifft;
der Begriff "Polycycloalkylrest", wie er hier allein oder als Teil eines anderen Restes verwendet wird, einen verbrückten multicyclischen Rest, welcher 5 bis 20 Kohlenstoffatome und 0 bis 3 Brücken enthält, betrifft;
der Begriff "Polycycloalkenylrest", wie er hier allein oder als Teil eines anderen Restes verwendet wird, einen verbrückten multicyclischen Rest, welcher 5 bis 20 Kohlenstoffatome und 0 bis 3 Brücken und 1 oder 2 Doppelbindungen enthält, betrifft;
der Begriff "Arylrest", wie er hier allein oder als Teil eines anderen Restes verwendet wird, monocyclische und bicyclische aromatische Reste, welche 6 bis 10 Kohlenstoffatome im Ringanteil enthalten, betrifft, und gegebenenfalls ein bis drei zusätzliche an Ar annelierte Ringe einschließt und gegebenenfalls über verfügbare Kohlenstoffatome mit 1, 2 oder 3 Resten substituiert sein können, ausgewählt aus einem Wasserstoff-, Halogenatom, einem Halogenalkyl-, Alkyl-, Halogenalkyl-, Alkoxy-, Halogenalkoxy-, Alkenylrest, einer Trifluormethyl-, Trifluormethoxygruppe, einem Alkinyl-, Cycloalkylalkyl-, Cycloheteroalkyl-, Cycloheteroalkylalkyl-, Aryl-, Heteroaryl-, Arylalkyl-, Aryloxy-, Aryloxyalkyl-, Arylalkoxy-, Arylthio-, Arylazo-, Heteroarylalkyl-, Heteroarylalkenyl-, Heteroarylheteroaryl-, Heteroaryloxyrest, einer Hydroxy-, Nitro-, Cyano-, Aminogruppe, einer substituierten Aminogruppe, wobei die Aminogruppe 1 oder 2 Substituenten (welche ein Alkyl-, Arylrest oder eine andere in den Definitionen erwähnte Arylverbindung sind) einschließt, einem Thiol-, Alkylthio-, Arylthio-, Heteroarylthio-, Arylthioalkyl-, Alkoxyarylthio-, Alkylcarbonyl-, Arylcarbonyl-, Alkylaminocarbonyl-, Arylaminocarbonyl-, Alkoxycarbonyl-, Aminocarbonyl-, Alkylcarbonyloxy-, Arylcarbonyloxy-, Alkylcarbonylamino-, Arylcarbonylamino-, Arylsulfinyl-, Arylsulfinylalkyl-, Arylsulfonylamino- oder einem Arylsulfonaminocarbonylrest oder aus einem der Reste R³ oder der hier angeführten Substituenten für R¹;
der Begriff "Aralkylrest", "Aryl-Alkylrest" oder "Aryl-Niederalkylrest", wie er hier allein oder als Teil eines anderen Restes verwendet wird, wie oben erwähnte Alkylreste mit einem Arylsubstituenten betrifft;
der Begriff "Niederalkoxyrest", "Alkoxyrest", "Aryloxyrest" oder "Aralkoxyrest", wie er hier allein oder als Teil eines anderen Restes verwendet wird, einen der vorstehenden an einem Sauerstoffatom gebundenen Alkyl-, Aralkyl- oder Arylreste einschließt;
der Begriff "Aminogruppe", wie er hier allein oder als Teil eines anderen Restes verwendet wird, gegebenenfalls mit einem oder mehreren Substituenten, wie einem Alkyl-, Aryl-, Arylalkyl-, Heteroaryl-, Heteroarylalkyl-, Cycloheteroalkyl-, Cycloheteroalkylalkyl- und/oder Cycloalkylrest, substituiert sein kann;
der Begriff "Niederalkylthiorest", "Alkylthiorest", "Arylthiorest" oder "Aralkylthiorest", wie er hier allein oder als Teil eines anderen Restes verwendet wird, einen der vorstehenden an einem Schwefelatom gebundenen Alkyl-, Aralkyl- oder Arylreste einschließt;
der Begriff "Niederalkylaminorest", "Alkylaminorest", "Arylaminorest" oder "Arylalkylaminorest", wie er hier allein oder als Teil eines anderen Restes verwendet wird, einen der vorstehenden an einem Stickstoffatom gebundenen Alkyl-, Aryl- oder Arylalkylreste einschließt;
der hier definierte Begiff "Acylrest", wie er hier alleine oder als Teil eines anderen Restes verwendet wird, ein organisches Radikal betrifft, welches an einer Carbonylgruppe 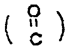 gebunden ist;
der Begriff "Alkanoylrest", wie er hier allein oder als Teil eines anderen Restes verwendet wird, einen an einer Carbonylgruppe gebundenen Alkylrest betrifft;
der Begriff "Alkenylrest", wie er hier alleine oder als Teil eines anderen Restes verwendet wird, geradkettige oder verzweigte Reste mit 2 bis 20 Kohlenstoffatomen betrifft, welche 1 bis 6 Doppelbindungen in der normalen Kette einschließen und welche gegebenenfalls mit 1 bis 4 Substituenten substituiert sein können, nämlich einem Halogenatom, einem Halogenalkyl-, Alkyl-, Alkoxy-, Alkenyl-, Alkinyl-, Aryl-, Arylalkyl-, Cycloalkylrest, einer Amino-, Hydroxygruppe, einem Heteroaryl-, Cycloheteroalkyl-, Alkanoylamino-, Alkylamido-, Arylcarbonylaminorest, einer Nitro-, Cyanogruppe, einem Thiol-, Alkylthio-, oder einem der Reste R³, oder den hier angeführten Substituenten für R¹;
der Begriff "Alkinylrest", wie er hier alleine oder als Teil eines anderen Restes verwendet wird, geradkettige oder verzweigte Reste mit 2 bis 20 Kohlenstoffatomen betrifft, welche eine Dreifachbindung in der normalen Kette einschließen und welche gegebenenfalls mit 1 bis 4 Substituenten substituiert sein können, nämlich einem Halogenatom, einem Halogenalkyl-, Alkyl-, Alkoxy-, Alkenyl-, Alkinyl-, Aryl-, Arylalkyl-, Cycloalkylrest, einer Aminogruppe, einem Heteroaryl-, Cycloheteroalkylrest, einer Hydroxygruppe, einem Alkanoylamino-, Alkylamido-, Arylcarbonylaminorest, einer Nitro-, Cyanogruppe, einem Thiolund/oder einem Alkylthiorest, oder einem der Reste R³, oder den hier angeführten Substituenten für R¹;
der Begriff "Alkylenrest", wie er hier allein oder als Teil eines anderen Restes verwendet wird, wie vorstehend definierte Alkylreste betrifft, welche Einzelbindungen für die Bindung an andere Reste an zwei verschiedenen Kohlenstoffatomen aufweisen und gegebenenfalls wie vorstehend für "Alkylrest" definiert, substituiert sein können;
die Begriffe "Alkenylenrest" und "Alkinylenrest", wie sie hier allein oder als Teil eines anderen Restes verwendet werden, wie oben definierte Alkenylreste beziehungsweise Alkinylreste betreffen, welche Einzelbindungen für die Bindung an andere Reste an zwei verschiedenen Kohlenstoffatomen aufweisen und gegebenenfalls 1, 2 oder 3 Substituenten einschließen, die einen der Reste R³ oder die hier angeführten Substituenten für R¹ umfassen;
der Begriff "Halogenatom", wie er hier allein oder als Teil eines anderen Restes verwendet wird, ein Chlor-, Brom-, Fluor- und Iodatom sowie CF₃ betrifft;
der Begriff "Metallion" Alkalimetallionen, Erdalkalimetallionen sowie Zink und Aluminium betrifft;
der Begriff "Cycloheteroalkylrest", wie er hier allein oder als Teil eines anderen Restes verwendet wird, einen 5-, 6- oder 7-gliedrigen gesättigten oder teilweise ungesättigten Ring betrifft, welcher 1 oder 2 Heteroatome einschließt, welche, wo möglich, über ein Kohlenstoffatom oder ein Heteroatom, gegebenenfalls über den Linker (CH₂)ₚ (welcher vorstehend definiert ist) gebunden sind;
der Begriff "Heteroarylrest", wie er hier allein oder als Teil eines anderen Restes verwendet wird, einen 5- oder 6-gliedrigen aromatischen Ring betrifft, welcher 1, 2, 3 oder 4 Heteroatome einschließt, welche an einen Aryl-, Cycloalkyl-, Heteroaryl- oder Cycloheteroalkylring anneliert sind und mögliche N-Oxide einschließt;
Ar entweder ein Aryl- oder Heteroarylrest, wie vorstehend definiert, ist;
der Begriff "Cycloheteroalkylalkylrest", wie er hier allein oder als Teil eines anderen Restes verwendet wird, wie vorstehend definierte Cycloheteroalkylreste betrifft, welche über ein C-Atom oder ein Heteroatom an eine -(CH₂)ₚ-Kette gebunden sind;
der Begriff "Heteroarylalkylrest" oder "Heteroarylalkenylrest", wie er hier allein oder als Teil eines anderen Restes verwendet wird, einen wie vorstehend definierten Heteroarylrest betrifft, welcher über ein C-Atom oder ein Heteroatom an eine wie vorstehend definierte -(CH₂)ₚ-Kette, einen Alkylen- oder Alkenylenrest gebunden ist und wobei
der Begriff "Polyhalogenalkylrest", wie er hier verwendet wird, einen wie vorstehend definierten "Alkyl"-Rest betrifft, welcher 2 bis 9 Halogensubtituenten einschließt.

2. Verbindung, wie in Anspruch 1 definiert, mit der Formel 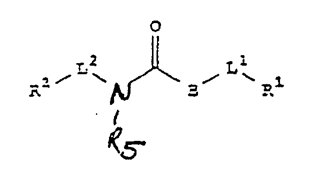 wobei B 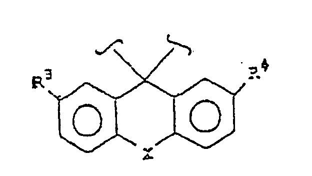 ist;
A eine NH-Gruppe ist,
X eine Bindung, ein Sauerstoff- oder Schwefelatom ist;
R³ und R⁴ gleich oder verschieden sind und H oder F sind;
R¹ ein Aryl-, Phenyl-, Heteroaryl-, Imidazolyl-, Pyridyl-, Cyclohexylrest, PO(R¹³)(R¹⁴), ein Heteroarylthio-, Benzimidazolyl-, Indolyl-, Benzthiazol-2-thio-, Imidazol-2-thio-, Alkyl-, Alkenyl- oder ein 1,3-Dioxan-2-ylrest ist;
R² ein Alkyl-, Polyfluoralkyl-, Alkenyl-, Aryl-, Phenyl-, Heteroaryl-, Imidazolyl- oder ein Pyridylrest ist;
L¹ ein Kette ist, welche 1 bis 5 Atome in einer linearen Kette enthält;
L² ein Bindung oder ein Alkylenrest ist, welcher bis zu 12 Kohlenstoffatome enthält.

3. Verbindung, wie in Anspruch 1 definiert, nämlich
N-(Phenylmethyl)-9-(3-phenylpropyl)-9H-fluoren-9-carboxamid; 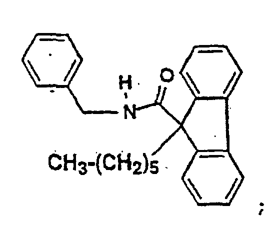 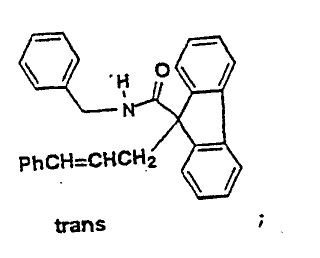 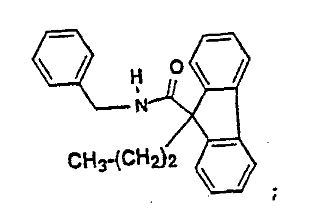 (E)-N-Ethyl-9-(3-phenyl-2-propenyl)-9H-fluoren-9-carboxamid; 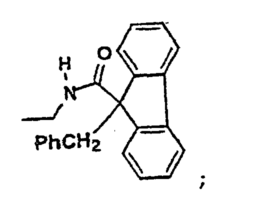 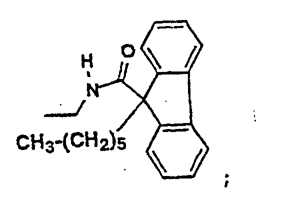 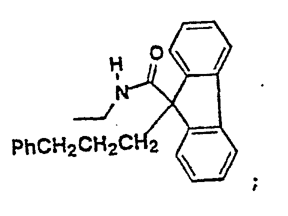 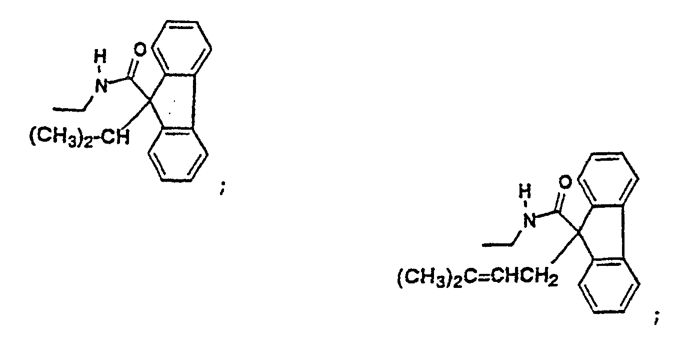 9-[4-(Dibutoxyphosphinyl)butyl]-N-propyl-9H-fluoren-9-carboxamid; (E)-9-(3-phenyl-2-propenyl)-N-propyl-9H-fluoren-9-carboxamid; 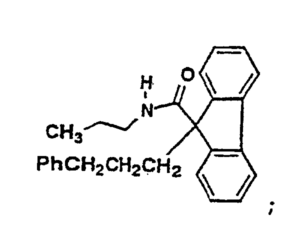 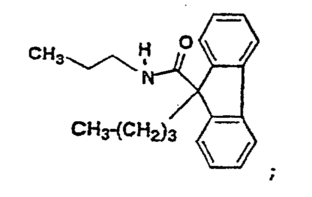 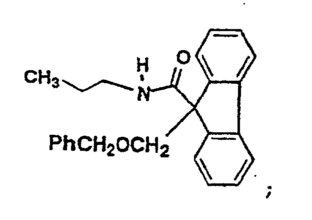 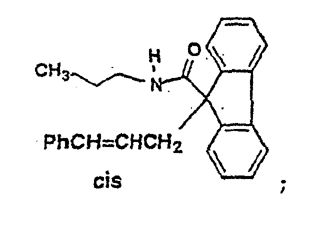 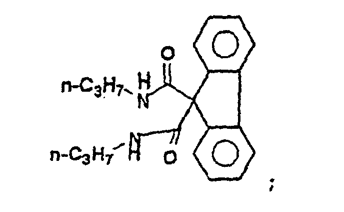 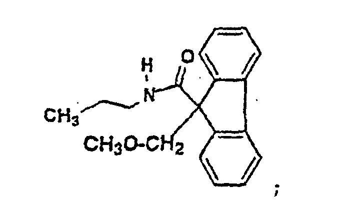 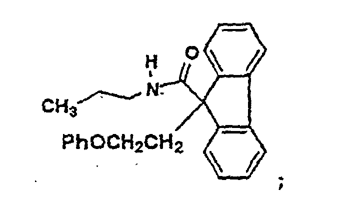 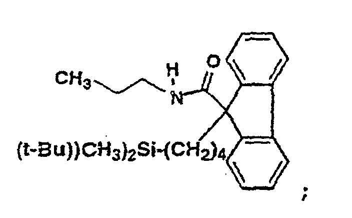 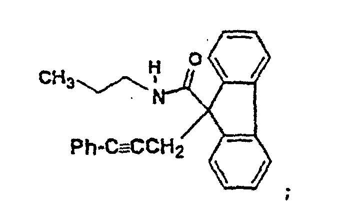 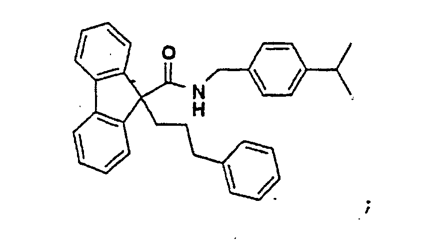 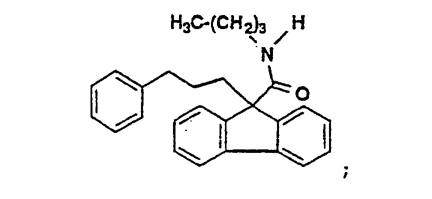 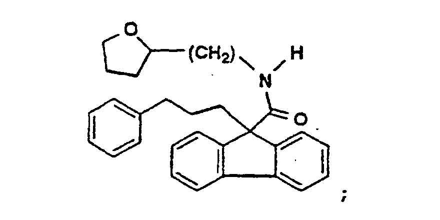 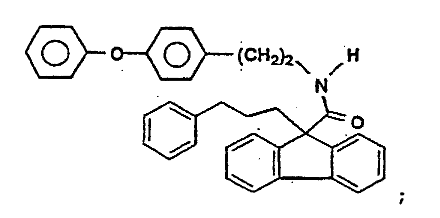 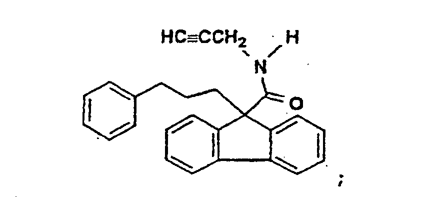 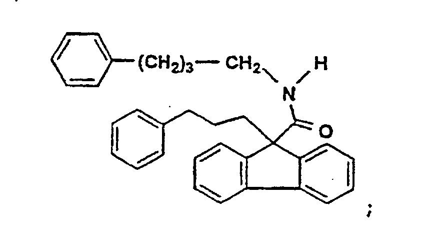 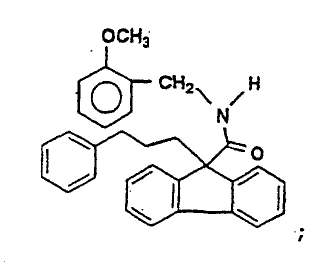 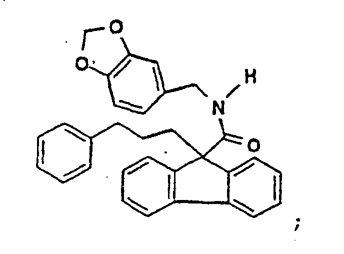 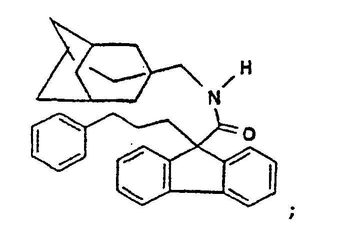 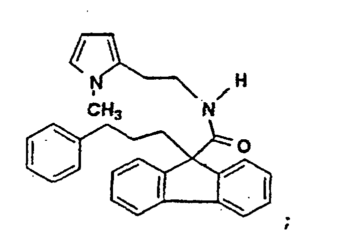 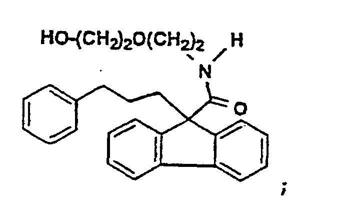 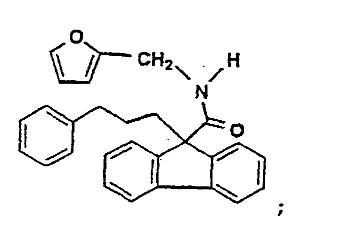 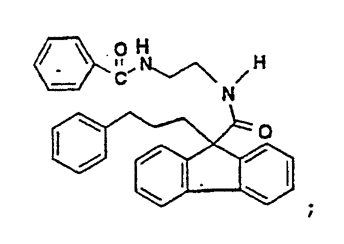 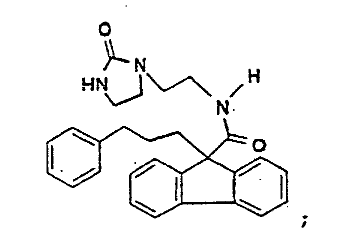 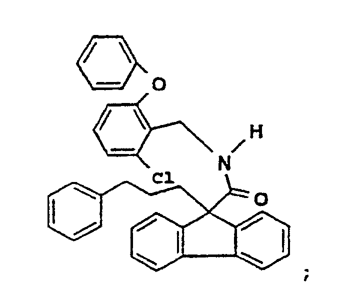 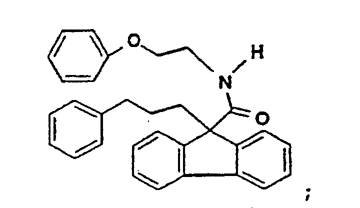 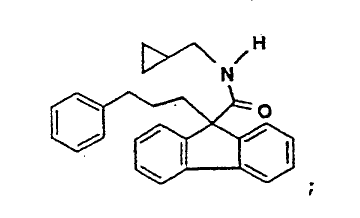 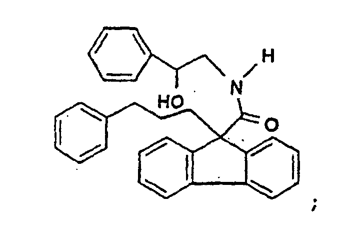 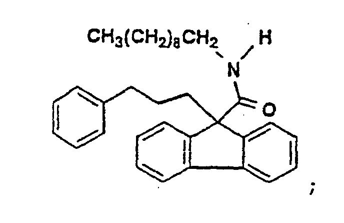 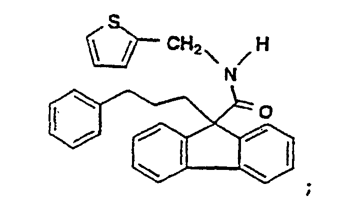 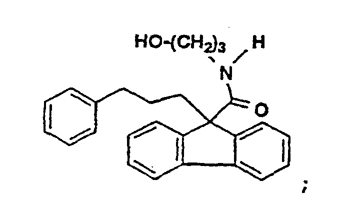 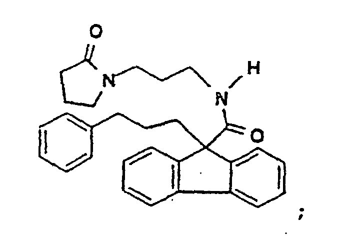 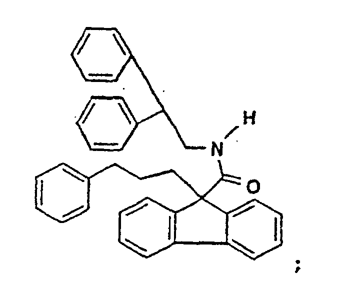 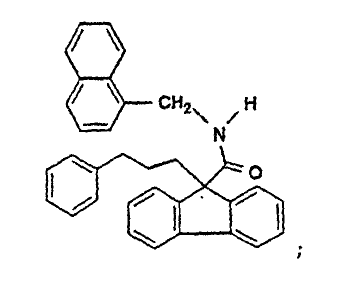 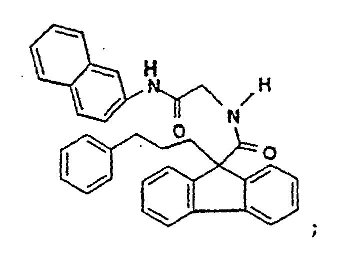 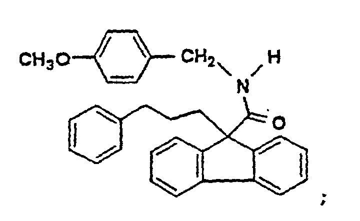 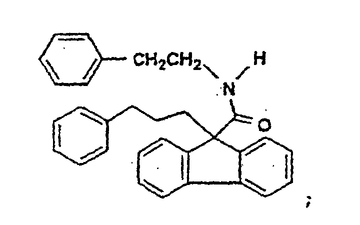 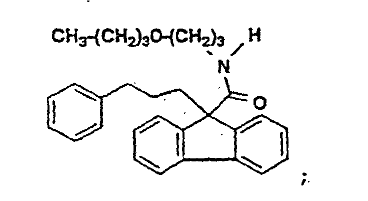 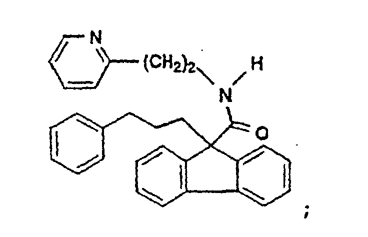 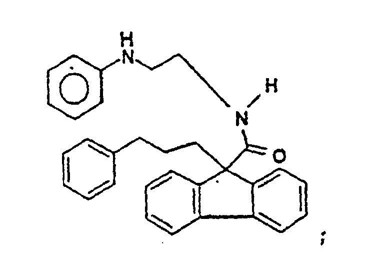 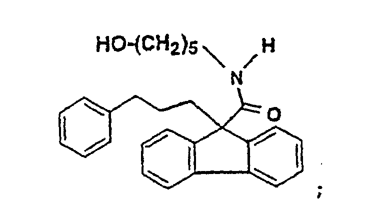 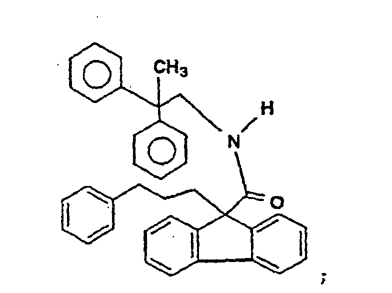 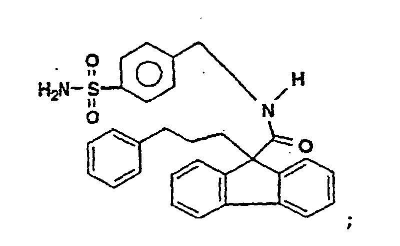 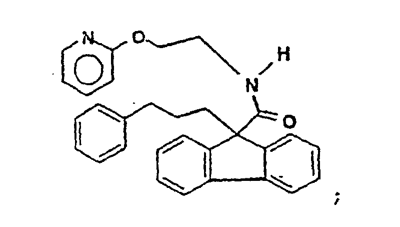 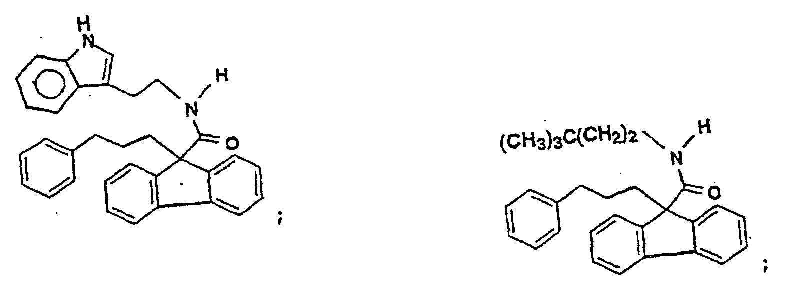 9-(3-Phenylpropyl)-N-(2,2,2-trifluorethyl)-9H-fluoren-9-carboxamid; 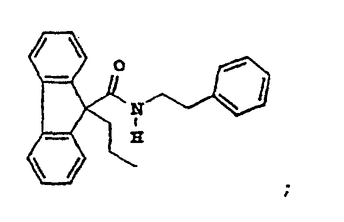 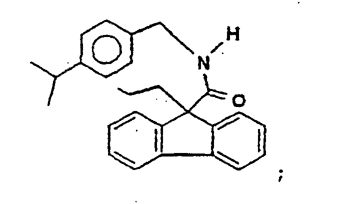 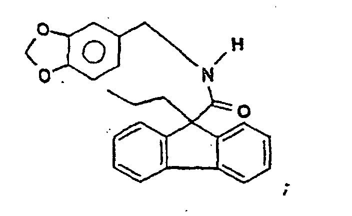 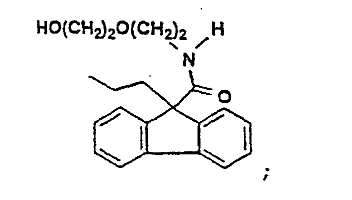 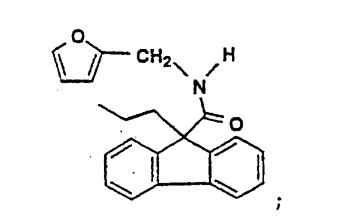 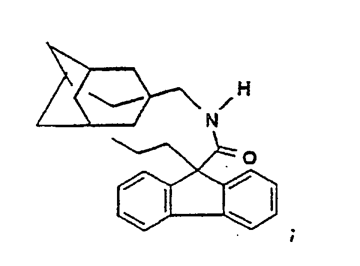 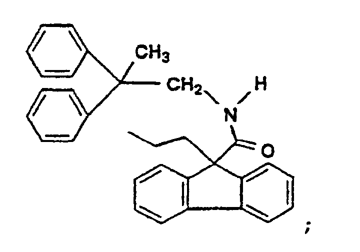 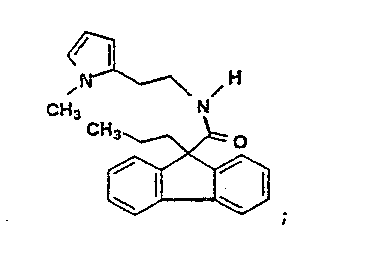 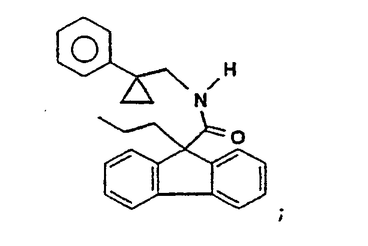 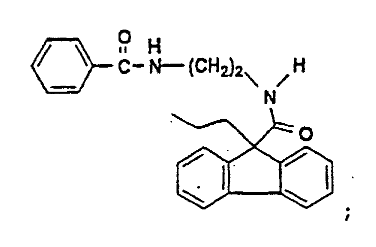 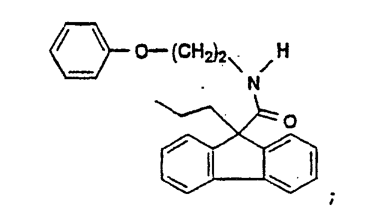 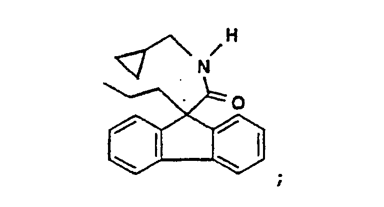 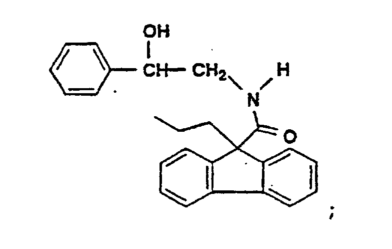 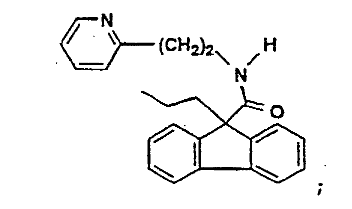 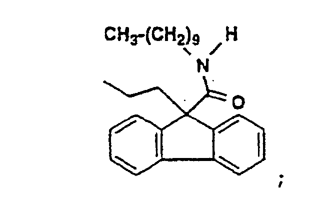 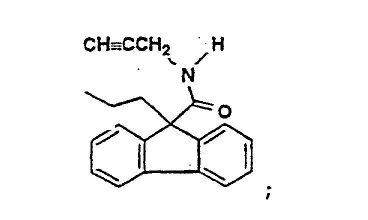 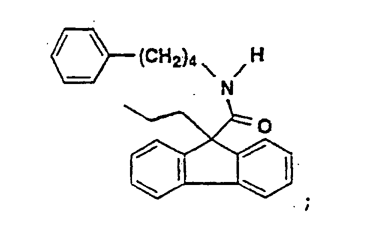 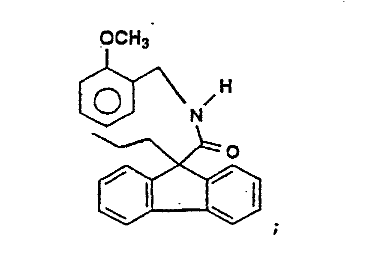 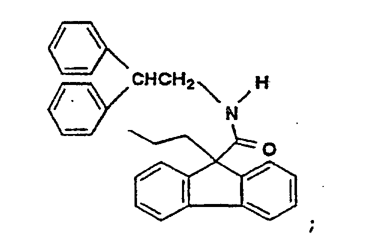 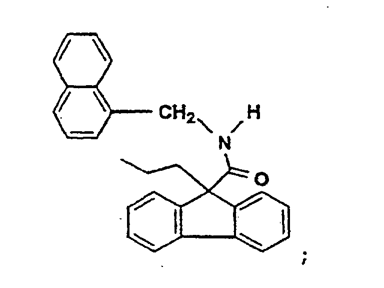 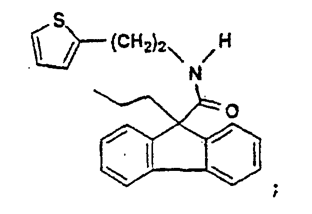 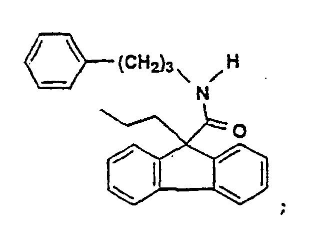 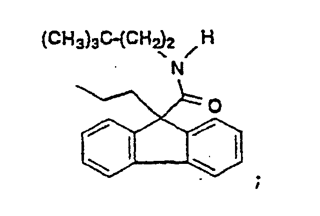 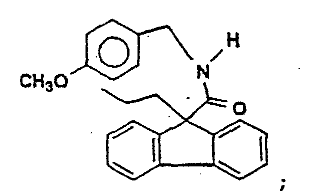 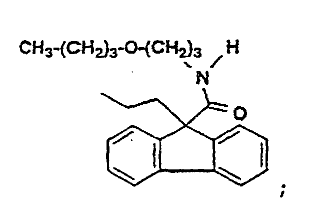 N-Methyl-N-(phenylmethyl)-9-propyl-9H-fluoren-9-carboxamid; 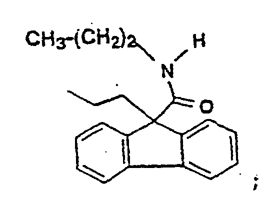 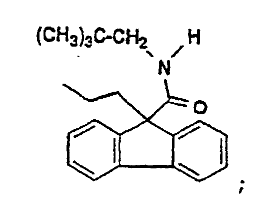 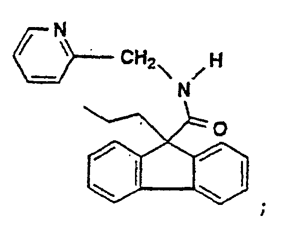 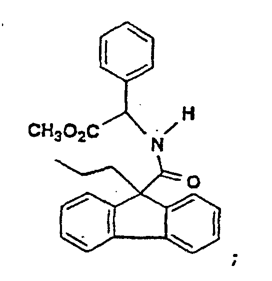 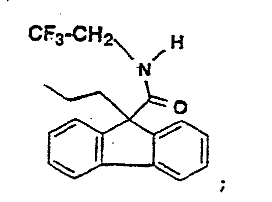 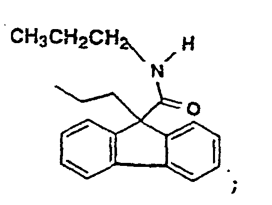 9-(2-Propenyl)-N-(2-pyridinylmethyl)-9H-fluoren-9-carboxamid;
N-Butyl-9-(2-propenyl)-9H-fluoren-9-carboxamid;
9-[2,2-Bis(trifluormethyl)-1,3-dioxolan-4-yl]methyl-N-ethyl-9H-fluoren-9-carboxamid;
9-(2,3-Dihydroxypropyl)-N-ethyl-9H-fluoren-9-carboxamid;
9-(3-Phenylpropyl)-N-(3-hydroxy)propyl-9H-xanthen-9-carboxamid; 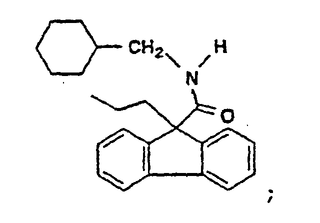 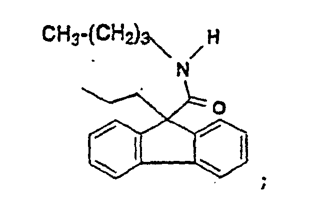 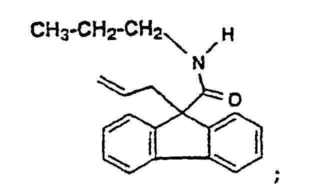 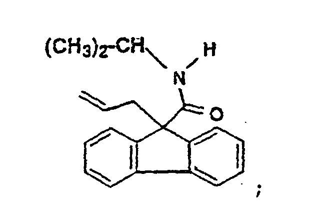 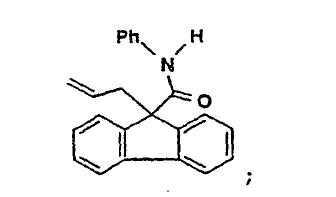 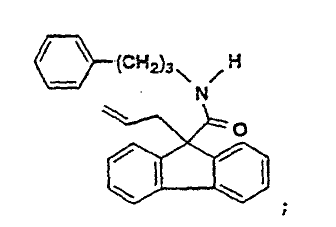 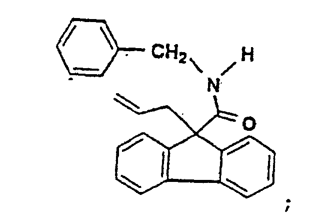 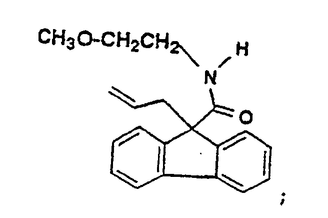 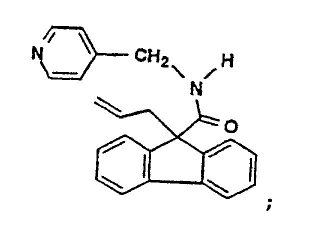 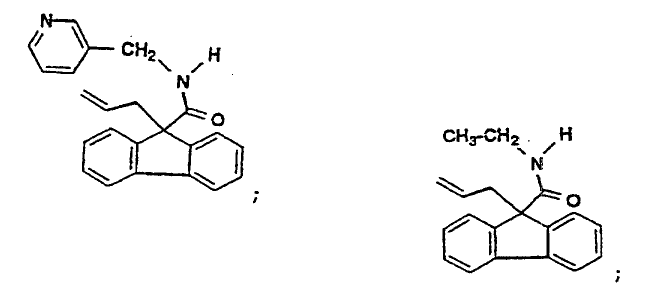 9-(1-Piperidinylcarbonyl)-9-(2-propenyl)-9H-fluoren; 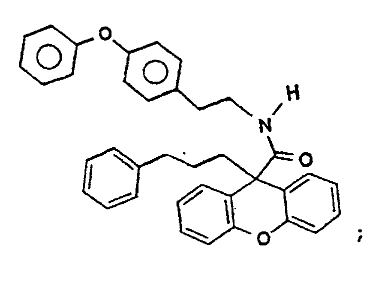 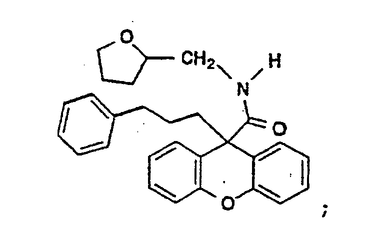 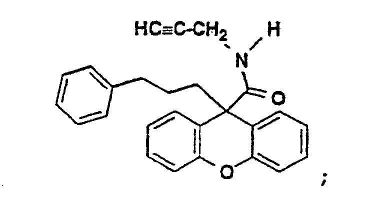 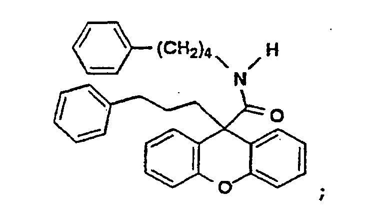 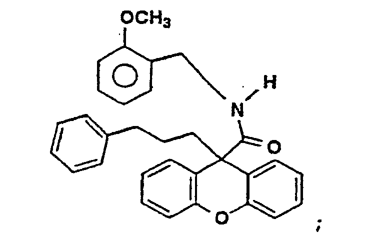 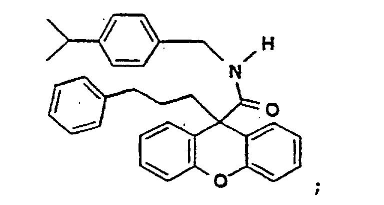 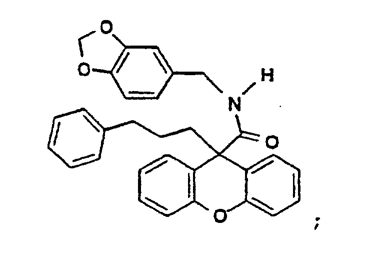 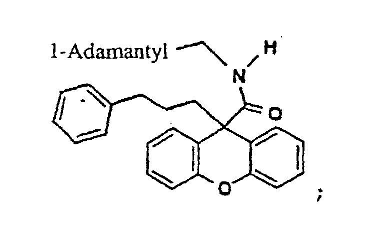 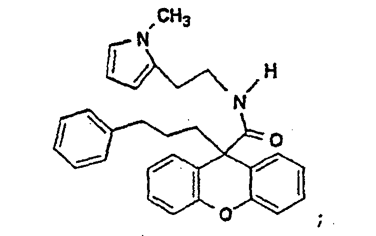 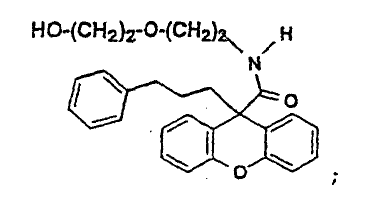 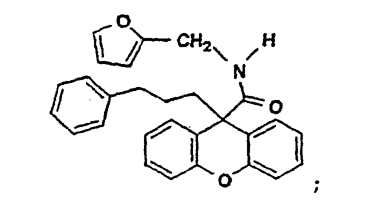 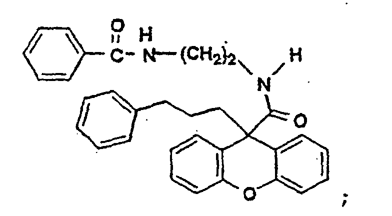 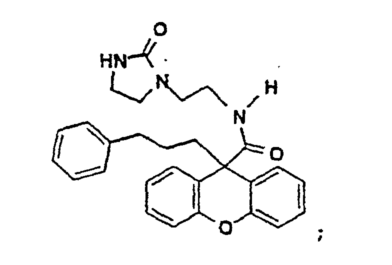 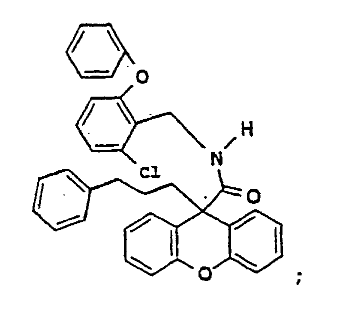 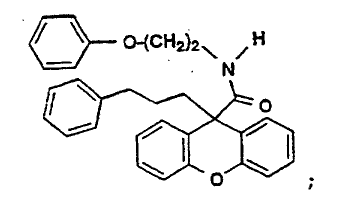 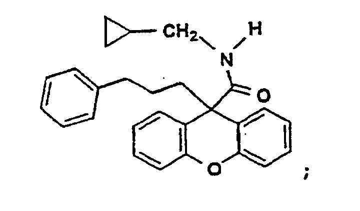 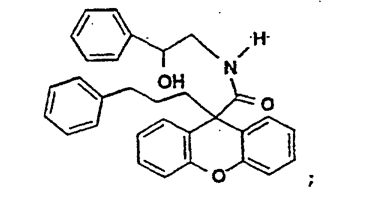 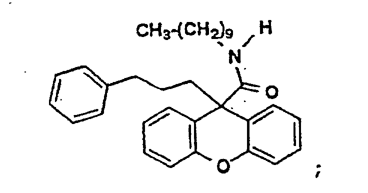 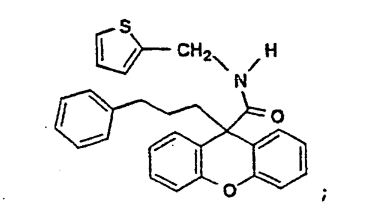 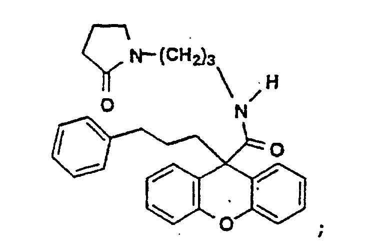 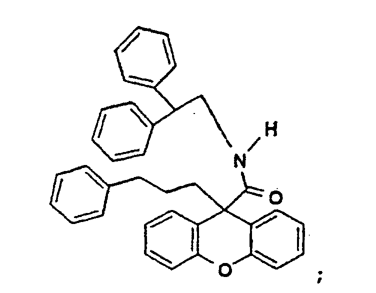 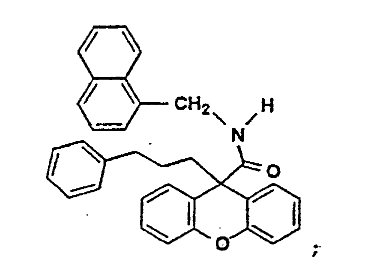 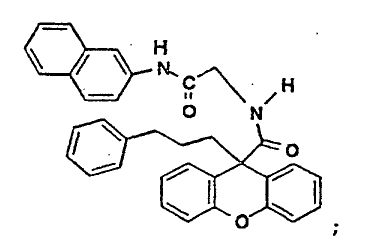 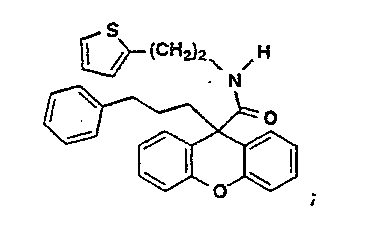 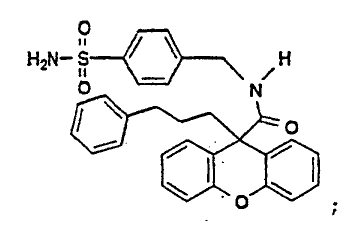 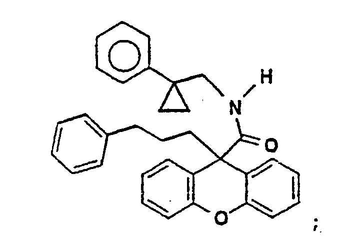 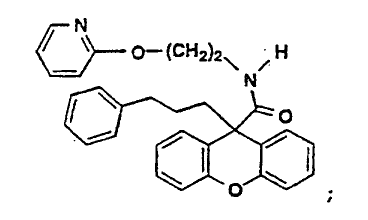 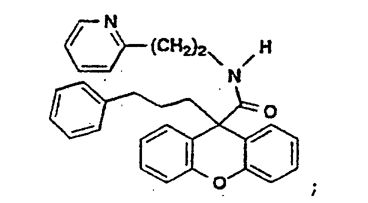 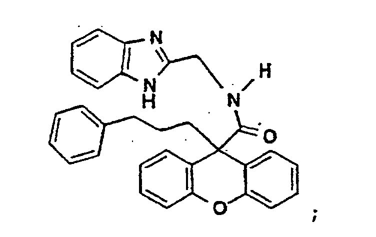 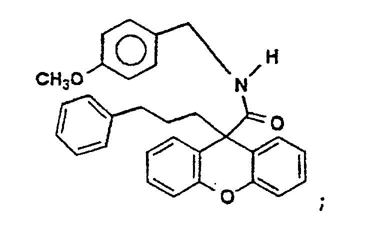 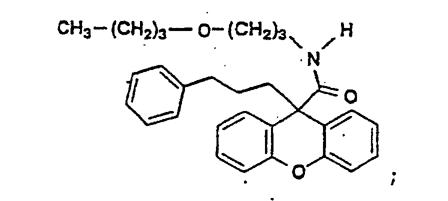 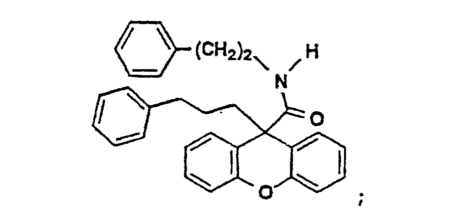 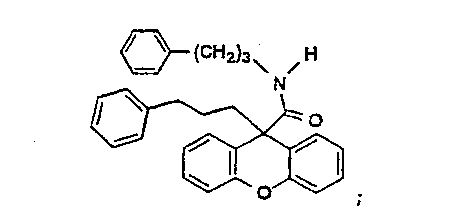 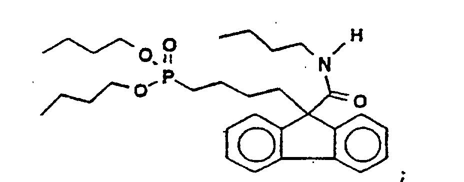 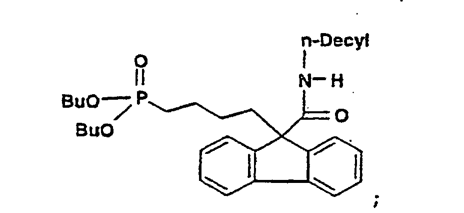 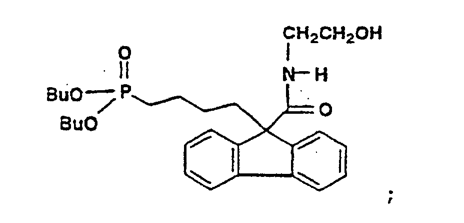 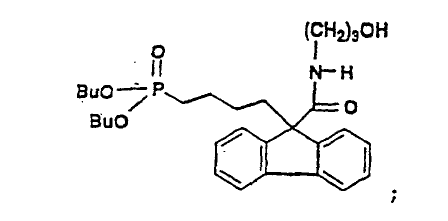 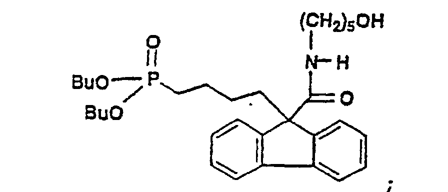 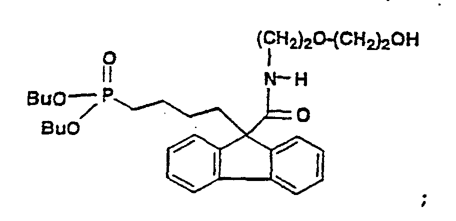 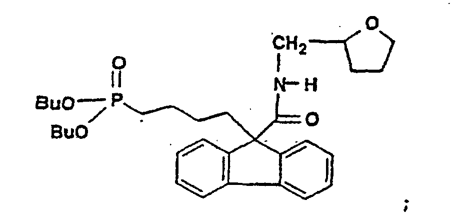 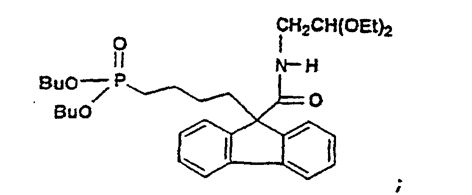 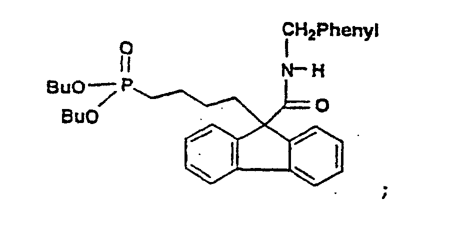 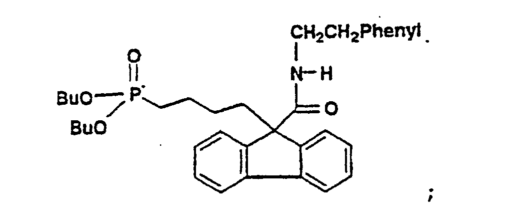 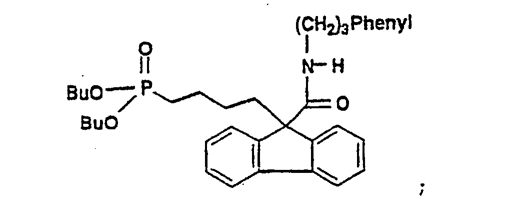 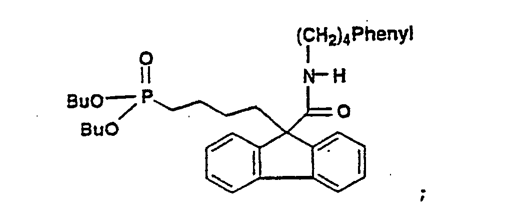 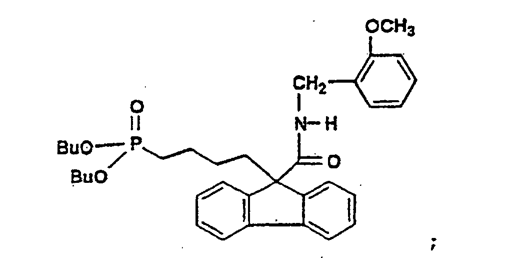 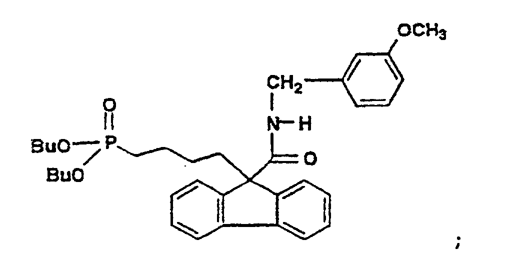 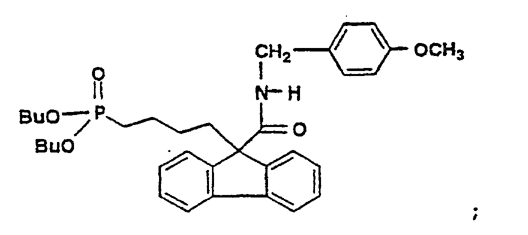 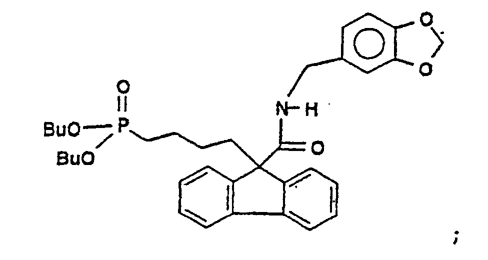 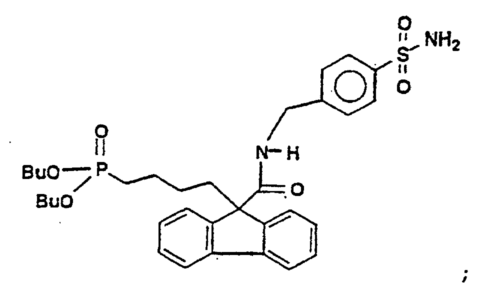 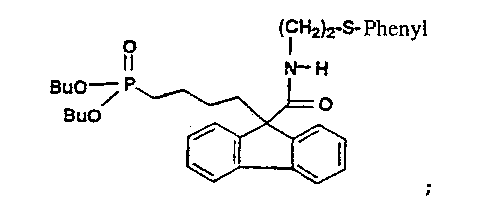 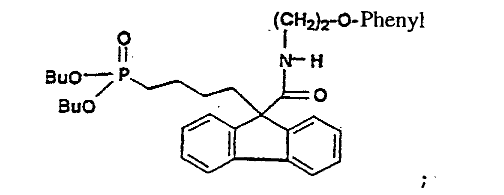 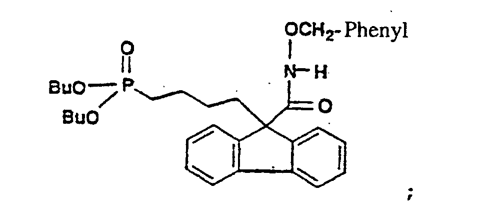 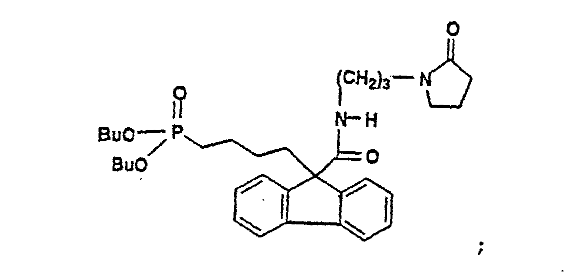 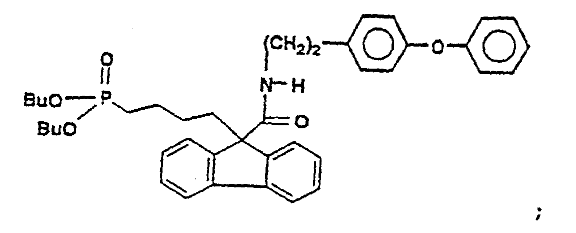 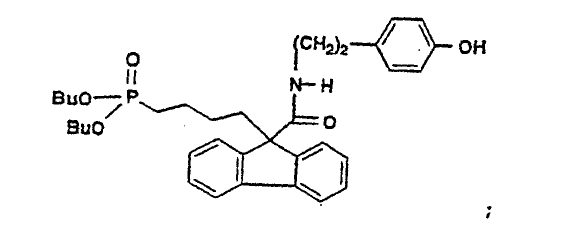 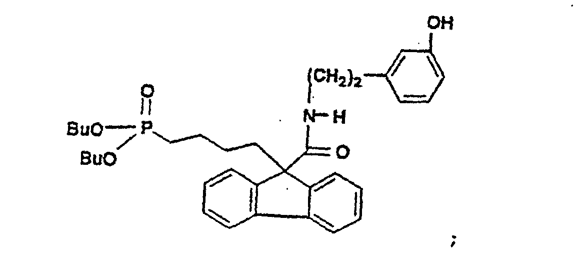 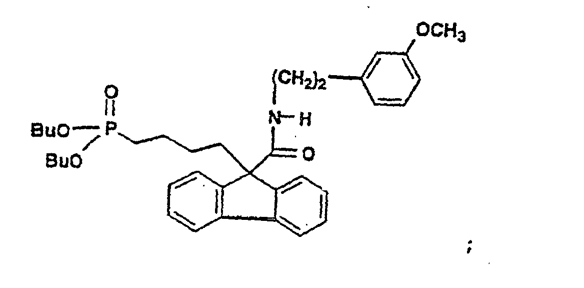 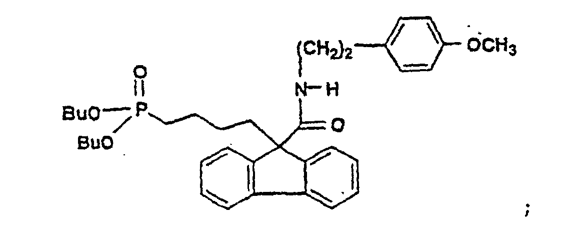 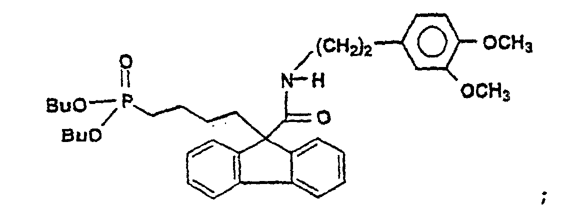 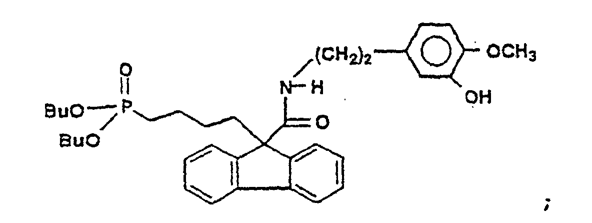 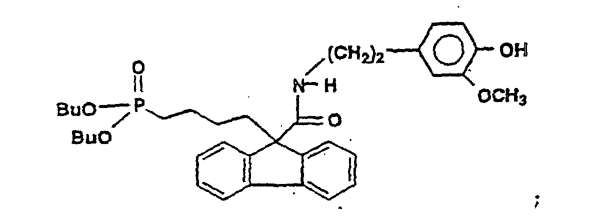 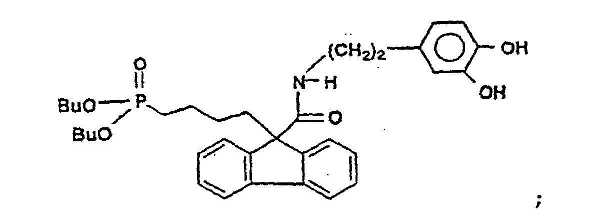 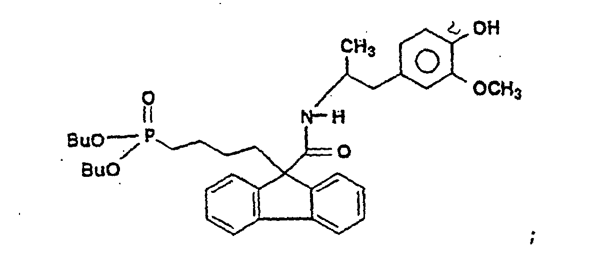 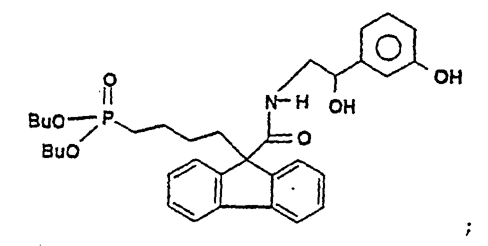 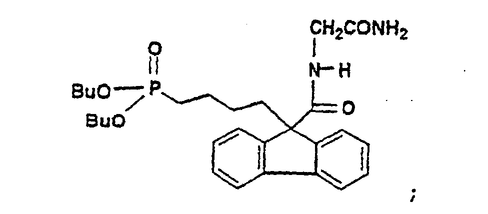 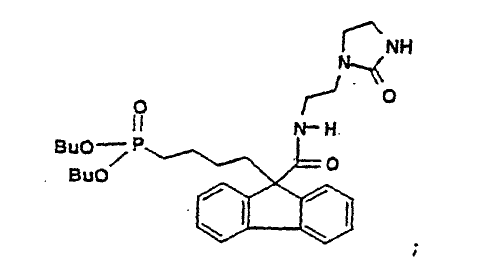 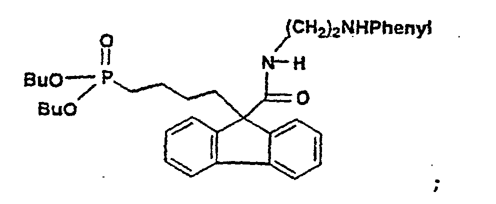 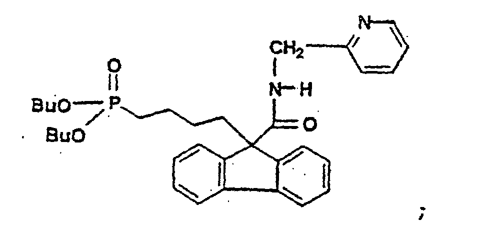 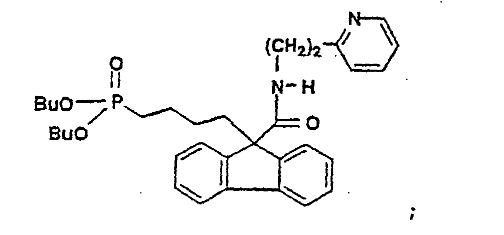 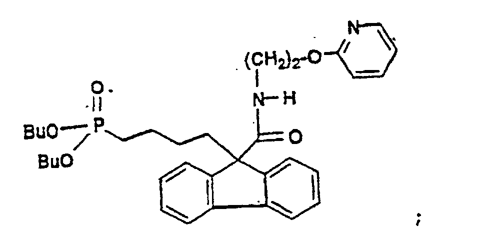 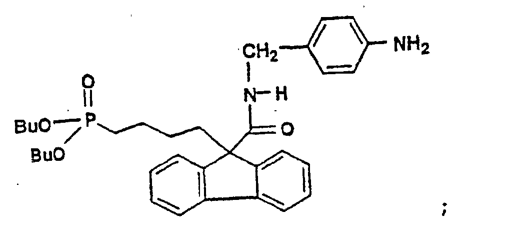 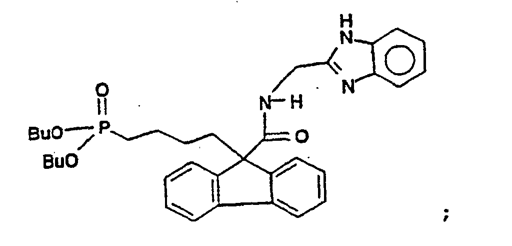 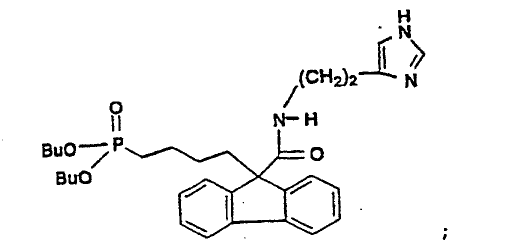 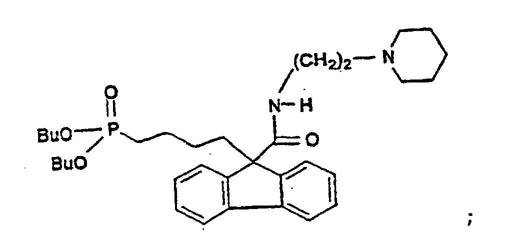 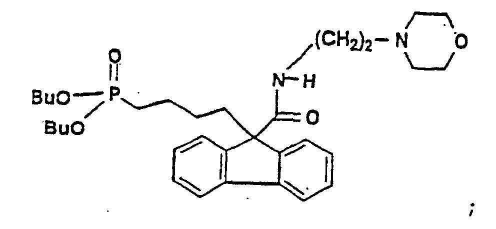 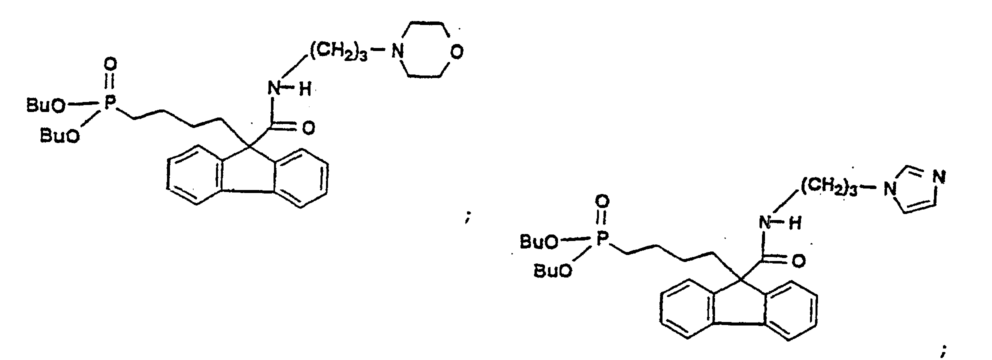 9-[4-(Dibutoxyphosphinyl)butyl]-N-(2,2,2-trifluorethyl)-9H-fluoren-9-carboxamid;
9-(2-Propenyl)-9H-fluoren-9-carbonsäure, Ethylester;
9-Propyl-9H-fluoren-9-carboxaldehyd;
9-(4-Cyanobutyl)-N-propyl-9H-fluoren-9-carboxamid;
1-[9-(3-Phenylpropyl)-9H-fluoren-9-yl]-1-butanon;
9-(3-Phenylpropyl)-α-propyl-9H-fluoren-9-methanol;
4-Hydroxy-1-(9-propyl-9H-fluoren-9-yl)-butanon;
N-[3-(Dibutoxyphospinyl)propyl]-9-propyl-9H-fluoren-9-carboxamid;
N-[5-(Dibutoxyphosphinyl)pentyl]-9-propyl-9H-fluoren-9-carboxamid;
N-[[4-(1,3-Dihydro-1-oxo-2H-isoindol-2-yl)phenyl]methyl]-9-propyl-9H-fluoren-9-carboxamid;
(E)-9-[4-(Dibutoxyphosphinyl)-2-butenyl]-2,7-difluor-N-propyl-9H-fluoren-9-carboxamid;
9-[4-(Dibutoxyphosphinyl)butyl]-2,7-difluor-N-propyl-9H-fluoren-9-carboxamid;
9-[4-(Diethoxyphosphinyl)butyl]-N-propyl-9H-fluoren-9-carboxamid;
9-[4-(Diphenylphosphinyl)butyl]-N-propyl-9H-fluoren-9-carboxamid;
[4-[9-(Butylthio)-9H-fluoren-9-yl]butyl]phosphonsäure, Dibutylester;
[4-(9-(Butylsulfonyl)-9H-fluoren-9-yl]butyl]phosphinsäure, Dibutylester;
[4-[9-(Butylsulfinyl)-9H-fluoren-9-yl]butyl]phosphonsäure, Dibutylester;
5-[4-(Dibutoxyphosphinyl)butyl]-N-propyl-5H-indeno[1,2-b]pyridin-5-carboxamid;
(E)-9-[4-(Dibutoxyphosphinyl)-2-butenyl]-2,7-difluor-N-(2,2,2-trifluorethyl)-9Hfluoren-9-carboxamid;
9- [4-[4-(1,3-Dihydro-1,3-dioxo-2H-isoindol-2-yl)phenyl]butyl]-N-propyl-9H-fluoren-9-carboxamid;
9-[4-[4-[[(2-Phenoxyphenyl)carbonyl]amino]phenyl]butyl]-N-propyl-9H-fluoren-9-carboxamid;
9-[4-[4-(1,3-Dihydro-1-oxo-2H-isoindol-2-yl)phenyl]butyl]-N-propyl-9H-fluoren-9-carboxamid;
9-[3-[4-(1,3-Dihydro-1,3-dioxo-2H-isoindol-2-yl)phenyl]propyl]-N-propyl-9Hfluoren-9-carboxamid;
9-[3-[4-(Benzoylamino)phenyl]-N-propyl-9H-fluoren-9-carboxamid;
9-[3-[(1,3-Dihydro-1-oxo-2H-isoindol-2-yl)phenyl]propyl]-N-propyl-9H-fluoren-9-carboxamid;
9-[5-[(6-Ethoxy-2-benzothiazolyl)thio]pentyl]-N-propyl-9H-fluoren-9-carboxamid;
9-[4-[4-(Benzoylamino)phenyl]butyl]-N-propyl-9H-fluoren-9-carboxamid;
9-[5-(Dibutoxyphosphinyl)pentyl]-N-propyl-9H-fluoren-9-carboxamid;
N,N-Diethyl-9-(2-propenyl)-9H-fluoren-9-carboxamid;
N-Ethyl-9-propyl-9H-fluoren-9-carboxamid;
N-Ethyl-9-(2-propenyl)-9H-xanthen-9-carboxamid;
N-Ethyl-9-(3-phenylpropyl)-9H-xanthen-9-carboxamid;
9-[(4-Morpholinyl)carbonyl]-9-propyl-9H-fluoren;
9-Hexyl-N-propyl-9H-xanthen-9-carboxamid;
N-Methoxy-N-methyl-9-propyl-9H-fluoren-9-carboxamid;
10, 11-Dihydro-5-(3-phenyl-2-propenyl)-N-propyl-5H-dibenzo[a,d]cyclohepten-5-carboxamid;
N-Methyl-9-propyl-9H-fluoren-9-carboxamid;
1-(9-Propyl-9H-fluoren-9-yl)-1-pentanon;
α-Butyl-9-propyl-9H-fluoren-9-methanol;
1-(9-Propyl-9H-fluoren-9-yl)-1-butanon;
α,9-Dipropyl-9H-fluoren-9-methanol;
10,11-Dihydro-5-(2-propenyl)-N-propyl-5H-dibenzo[a,d]cyclohepten-5-carboxamid;
9-(3-Phenylpropyl)-N-propyl-9H-thioxanthen-9-carboxamid;
N,9-Dipropyl-9H-thioxanthen-9-carboxamid;
10,11-Dihydro-5-(3-phenylpropyl)-N-propyl-5H-dibenzo[a,d]cycloheptan-5-carboxamid;
(E)-2,7-Difluor-9-(3-phenyl-2-propenyl)-N-propyl-9H-fluoren-9-carboxamid;
9-(3-Phenylpropyl)-N-(2-pyridinylmethyl)-9H-fluoren-9-carboxamid;
2,7-Difluor-9-(3-phenylpropyl)-N-propyl-9H-fluoren-9-carboxamid;
2,7-Difluor-9-(3-phenylpropyl)-N-(4-pyridinylmethyl)-9H-fluoren-9-carboxamid;
9-(Butylthio)-9-propyl-9H-fluoren;
9-(Butylsulfinyl)-9-propyl-9H-fluoren;
9-(4-Hydroxybutyl)-N-propyl-9H-fluoren-9-carboxamid;
9-[4-(Phenylthio)butyl]-N-propyl-9H-fluoren-9-carboxamid;
9-[3-(1,3-Dioxan-2-yl)propyl]-N-propyl-9H-fluoren-9-carboxamid;
9-[3-(1,3-Dioxolan-2-yl)propyl]-N-propyl-9H-fluoren-9-carboxamid;
cis-N,9-Dipropyl-1H-thioxanthen-9-carboxamid, 10-Oxid;
5-(2-Propenyl)-N-propyl-5H-indeno[1,2-b]pyridin-5-carboxamid;
(E)-5-(3-Phenyl-2-propenyl)-N-propyl-5H-indeno[1,2-b]pyridin-5-carboxamid;
N-Ethyl-N-methyl-9-(2-propenyl)-9H-fluoren-9-carboxamid;
N,9-Dipropyl-9H-thioxanthen-9-carboxamid, 10,10-Dioxid;
trans-N,9-Dipropyl-9H-thioxanthen-9-carboxamid, 10-Oxid;
9-[3-(Dibutoxyphosphinyl)propyl]-N-(2-pyridinylmethyl)-9H-fluoren-9-carboxamid;
1-(9-Propyl-9H-fluoren-9-yl)-2-(1-piperidinyl)ethanon, Monohydrochlorid;
N-(5-Hydroxypentyl)-9-propyl-9H-fluoren-9-carboxamid;
9-(3-Cyanopropyl)-N-propyl-9H-fluoren-9-carboxamid;
N-[[4-[[(9-Propyl-9H-fluoren-9-yl)carbonyl]amino]phenyl]methyl]-9-propyl-9Hfluoren-9-carboxamid;
N-[4-(4-Aminophenyl)methyl]-9-propyl-9H-fluoren-9-carboxamid;
9-[3-(Dibutoxyphosphinyl)propyl]-N-propyl-9H-fluoren-9-carboxamid;
4-(1-Piperidinyl)-1-(9-Propyl-9H-fluoren-9-yl)-1-butanon, Monohydrochlorid;
N-Methyl-9-(3-phenylpropyl)-9H-fluoren-9-carboxamid;
2-(Dimethylamino)-9-(3-phenylpropyl)-N-propyl-9H-fluoren-9-carboxamid;
9-[4-(Dibutoxyphosphinyl)-2-butenyl]-N-propyl-9H-fluoren-9-carboxamid;
9-[4-(4-Nitrophenyl)butyl]-N-propyl-9H-fluoren-9-carboxamid;
9-[3-(4-Nitrophenyl)-2-propenyl]-N-propyl-9H-fluoren-9-carboxamid;
5-(3-Phenylpropyl)-N-propyl-5H-indeno[1,2-b]pyridin-5-carboxamid;
9-[4-(4-Aminophenyl)butyl]-N-propyl-9H-fluoren-9-carboxamid;
9-[3-(4-Aminophenyl)propyl]-N-propyl-9H-fluoren-9-carboxamid;
9-[4-(Dibutoxyphosphinyl)butyl]-9H-fluoren-9-carbonsäure, Methylester;
N,N-Dibutyl-9-[(propylamino)carbonyl]-9H-fluoren-9-butanamid;
9-(5-Cyanopentyl)-N-propyl-9H-fluoren-9-carboxamid;
9-[2-[[[4-(1,3-Dihydro-1,3-dioxo-2H-isoindol-2-yl)phenyl]sulfonyl]amino]ethyl]-N-(2,2,2-trifluorethyl)-9H-fluoren-9-carboxamid;
(Z)-9-[4-[(6-Ethoxy-2-benzothiazolyl)thio]-2-butenyl]-N-propyl-9H-fluoren-9-carboxamid;
9-[4-(Dibutoxyphosphinyl)butyl]-N-(2,2,2-trifluorpropyl)-9H-xanthen-9-carboxamid;
9-[4-[Butoxy[2-(4-morpholinyl)ethoxy]phosphinyl]butyl]-N-(2,2,2-trifluorethyl)-9Hfluoren-9-carboxamid; 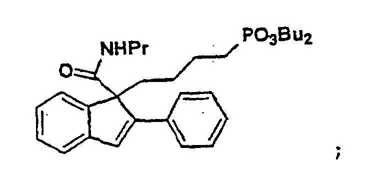 9-[4-(Dibutoxyphosphinyl)butyl]-2,7-difluor-N-(2,2,2-trifluorethyl)-9H-fluoren-9-carboxamid; 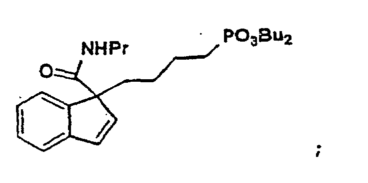 (E)-9-[4-(Dibutoxyphosphinyl)-2-butenyl]-N-propyl-9H-fluoren-9-carboxamid;
9-[4-[4-(Benzoylamino)-1H-imidazol-1-yl]butyl]-N-(2,2,2-trifluorethyl)-9H-fluoren-9-carboxamid;
9-[4-[5-(Benzoylamino)-2-pyridinyl]butyl]-N-(2,2,2-trifluorethyl)-9H-fluoren-9-carboxamid;
9-[4-[4-[(2-Phenoxybenzoyl)amino]-1H-imidazol-1-yl]butyl]-N-(2,2,2-trifluorethyl)-9H-fluoren-9-carboxamid;
9-[4-[(2-Brom-5-pyridinyl)amino]butyl]-N-propyl-9H-fluoren-9-carboxamid;
9-[2-[[[4-(Benzoylamino)phenyl]sulfonyl]amino]ethyl]-N-(2,2,2-trifluorethyl)-9Hfluoren-9-carboxamid;
9-(4-Phenylbutyl)-N-propyl-9H-fluoren-9-carboxamid;
3-[(9-Propyl-9H-fluoren-9-yl)sulfonyl]-propansäure, Methylesther;
9-[4-[(6-Ethoxy-2-benzothiazolyl)thio]butyl]-N-propyl-9H-fluoren-9-carboxamid;
9-[3-[(6-Ethoxy-2-benzothiazolyl)thio]propyl]-N-propyl-9H-fluoren-9-carboxamid;
(Z)-9-[4-(Diethoxyphosphinyl)-2-butenyl]-N-(2,2,2-trifluorethyl)-9H-fluoren-9-carboxamid;
9-[4-(Diethoxyphosphinyl)butyl]-N-(2,2,2-trifluorethyl)-9H-fluoren-9-carboxamid;
9-[4-(Dibutoxyphosphinyl)butyl]-N-(2,2,3,3,3-pentafluorpropyl)-9H-fluoren-9-carboxamid;
9-[4-(Dibutoxyphosphinyl)butyl]-N-propyl-9H-xanthen-9-carboxamid;
9-[4-(Dibutoxyphosphinyl)butyl]-N-(2,2,3,3,4,4,4-heptafluorbutyl)-9H-fluoren-9-carboxamid;
9-[4-(Dibutoxyphosphinyl)butyl]-N-propyl-9H-indeno-[2,1-b]pyridin-9-carboxamid;
9-[4-[4-[(Phenylsulfonyl)amino]phenyl]butyl]-N-(2,2,2-trifluorethyl)-9H-fluoren-9-carboxamid;
[4-[9-(1-Oxopentyl)- 9H-fluoren-9-yl]butyl]-phosphonsäure;
9-[5-(Dibutoxyphosphinyl)pentyl]-N-(2,2,2-trifluorethyl)-9H-fluoren-9-carboxamid;
9-[3-[[5-[(2-Phenoxyhenzoyl)amino]-2-pyridinyl]oxy]propyl]-N-(2,2,2-trifluorethyl)-9H-fluoren-9-carboxamid;
[6-[9-[[(2,2,2-Trifluorethyl)amino]carbonyl]-9H-fluoren-9-yl]hexyl]-phosphonsäure, Dibutylester;
9-[4-[5-[(2-Phenoxybenzoyl)amino]-2-pyridinyl]butyl]-N-(2,2,2-trifluorethyl)-9Hfluoren-9-carboxamid;
9-[4-[4-(Benzoylamino)-2-methyl-1H-imidazol-1-yl]butyl]-N-(2,2,2-trifluorethyl)-9Hfluoren-9-carboxamid;
9-[4-[4-[(2-Phenoxybenzoyl)amino]-2-methyl-1H-imidazol-1-yl]butyl]-N-(2,2,2-trifluorethyl)-9H-fluoren-9-carboxamid;
9-[3-[[2-(Benzoylamino)-5-pyridinyl]amino]propyl]-N-(2,2,2-trifluorethyl)-9Hfluoren-9-carboxamid;
[[4-(Benzoylamino)phenyl]methyl][2-[9-[[2,2,2-trifluorethyl)amino]carbonyl]-9Hfluoren-9-yl]ethyl]-carbaminsäure, 1,1-Dimethylethylester;
9-[2-[[[4-(Benzoylamino)phenyl]methyl]amino]ethyl]-N-(2,2,2-trifluorethyl)-9Hfluoren-9-carboxamid;
9-[4-[Butoxy(tetrahydrofuran-2-ylmethoxy)-phosphinyl]butyl]-N-(2,2,2-trifluorethyl)-9H-fluoren-9-carboxamid;
9-[4-[Butoxy(2-pyridinylmethoxy)phosphinyl]butyl]-N-(2,2,2-trifluorethyl)-9Hfluoren-9-carboxamid;
9-[4-(Dipropoxyphosphinyl)butyl]-N-(2,2,2-trifluorethyl)-9H-fluoren-9-carboxamid;
9-[4-[4-[[(4-Nitrophenyl)sulfonyl]amino]phenyl]butyl]-N-(2,2,2-trifluorethyl)-9Hfluoren-9-carboxamid;
9-[4-[4-[[(2-Nitrophenyl)sulfonyl]amino]phenyl]butyl]-N-(2,2,2-trifluorethyl)-9Hfluoren-9-carboxamid;
9-[4-(Dibutoxyphosphinyl)butyl]-3,6-difluor-N-(2,2,2-trifluorethyl)-9H-fluoren-9-carboxamid;
9-[3-[[5-[(2-Phenoxybenzoyl)amino]-2-pyridinyl]oxy]propyl]-N-propyl-9H-fluoren-9-carboxamid;
9-[6-[(6-Ethoxy-2-benzothiazolyl)thio]hexyl]-N-(2,2,2-trifluorethyl)-9H-fluoren-9-carboxamid;
[4-[9-[[(2,2,2-Trifluorethyl)amino]carbonyl]-9H-fluoren-9-yl]butyl]-phosphonsäure, Di(1-Methylethyl)ester;
[[4-[(2-Phenoxybenzoyl)amino]phenyl]methyl][2-[9-[[(2,2,2-trifluorethyl)amino] carbonyl]-9H-fluoren-9-yl]ethyl]-carbaminsäure, 1,1-Dimethylethylester;
9-[2-[[[4-[(2-Phenoxybenzoyl)amino]phenyl]methyl]amino]ethyl]-N-(2,2,2-trifluorethyl)-9H-fluoren-9-carboxamid;
[1-[4-[9-[[(2,2,2-Trifluorethyl)amino]carbonyl]-9H-fluoren-9-yl]butyl]-1H-imidazol-4-yl]carbaminsäure;
9-[4-[(4,5-Diphenyl-1H-imidazol-2-yl)thio]butyl]-N-[2-(4-methoxyphenyl)ethyl]-9Hfluoren-9-carboxamid;
9-[4-[(6-Ethoxy-2-benzothiazolyl)thio]butyl]-N-propyl-9H-fluoren-9-carboxamid;
9-[4-(2-Thiazolylthio)butyl]-N-(2,2,2-trifluorethyl)-9H-fluoren-9-carboxamid; 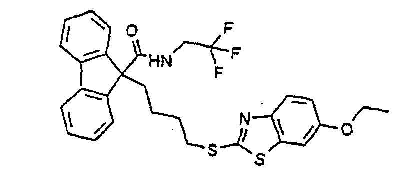 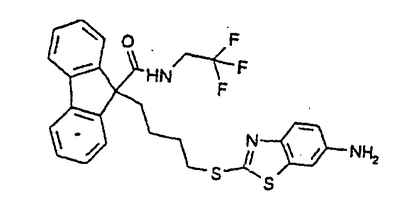 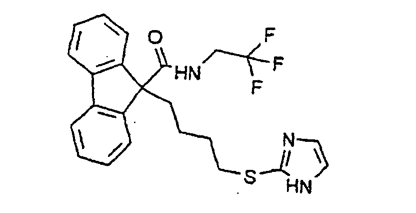 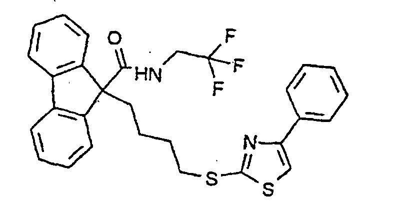 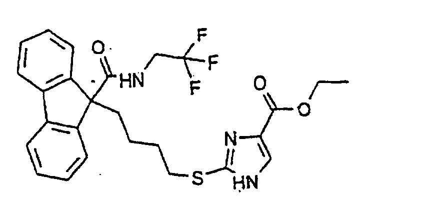 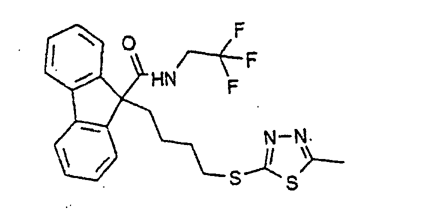 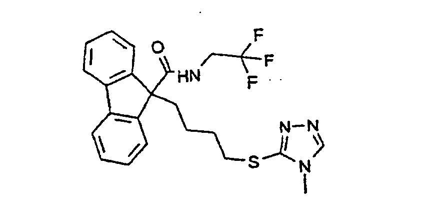 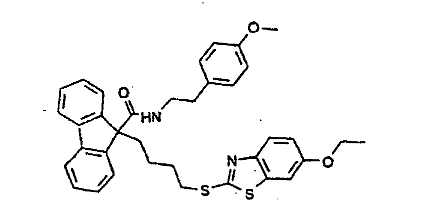 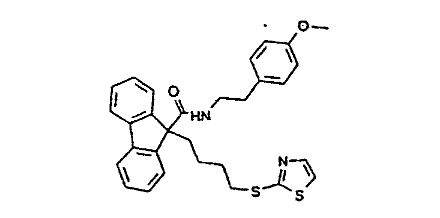 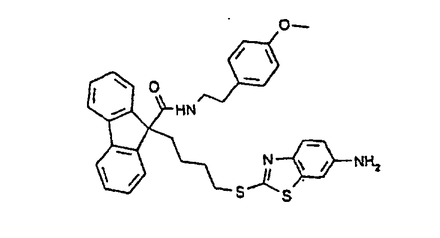 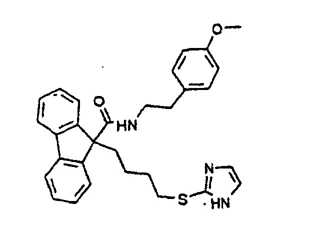 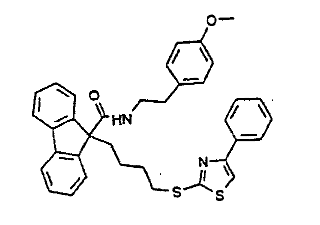 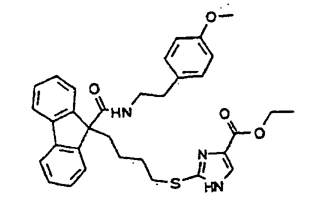 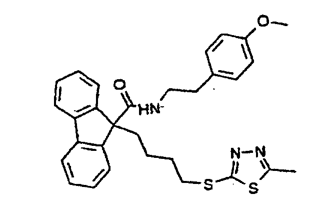 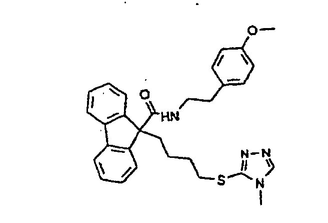 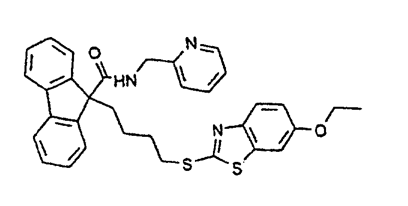 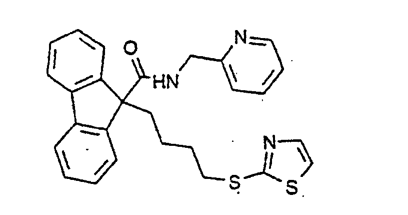 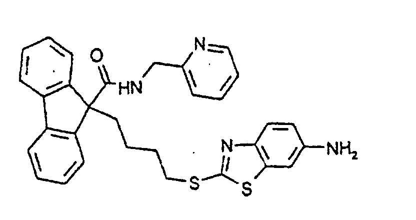 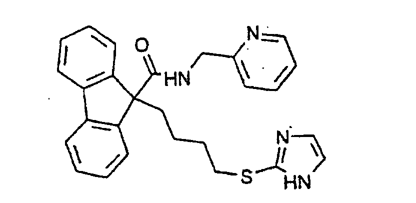 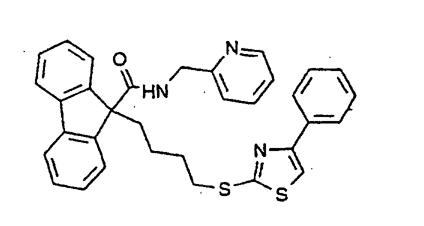 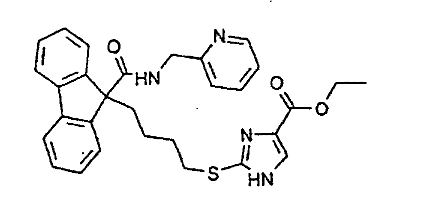 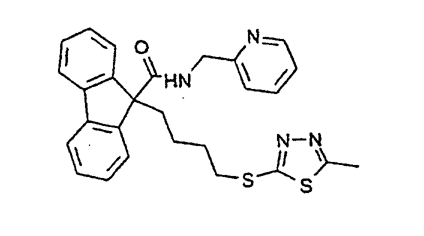 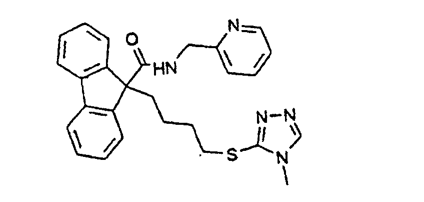 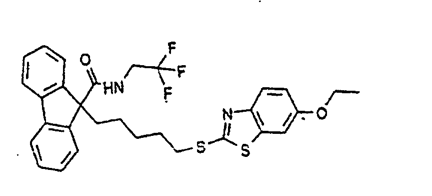 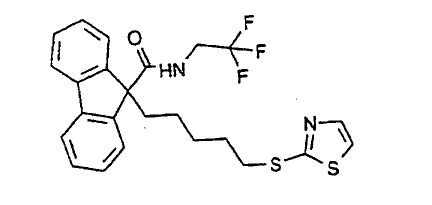 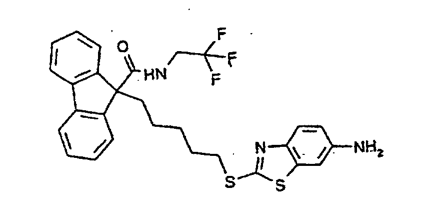 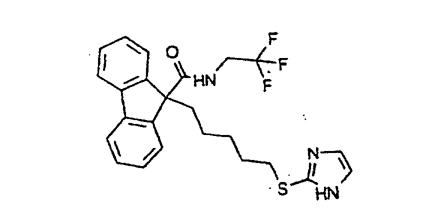 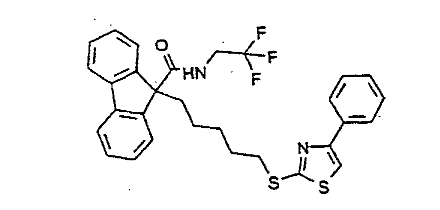 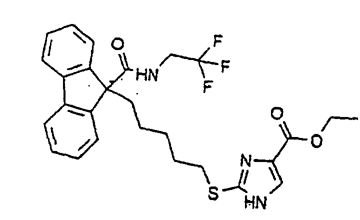 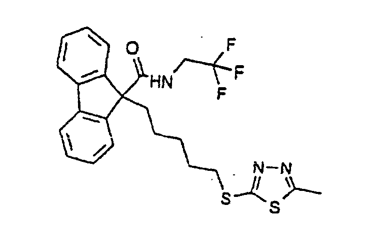 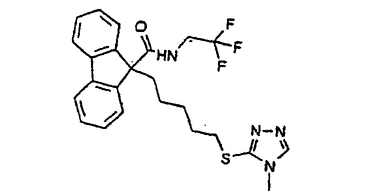 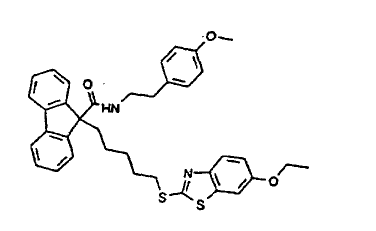 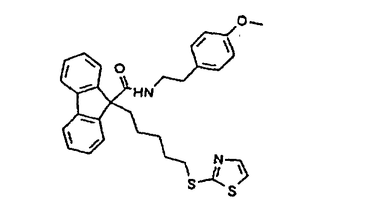 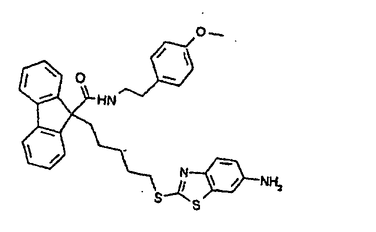 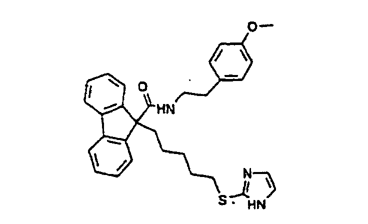 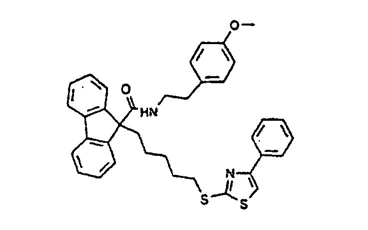 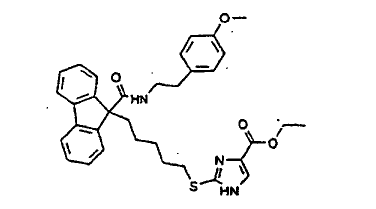 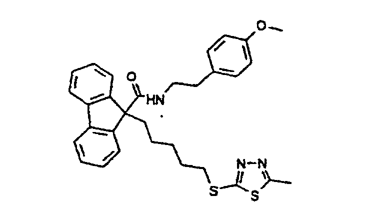 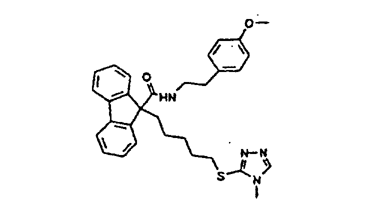 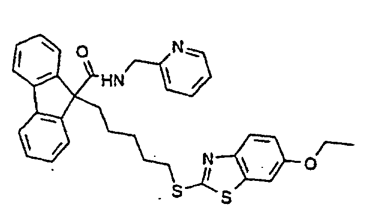 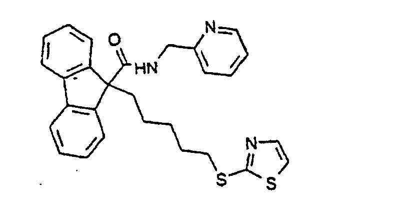 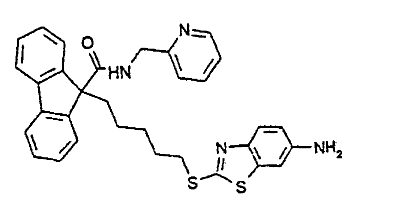 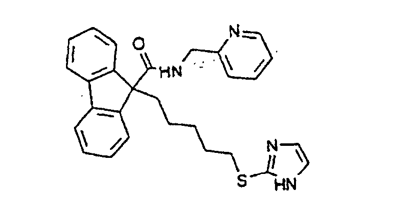 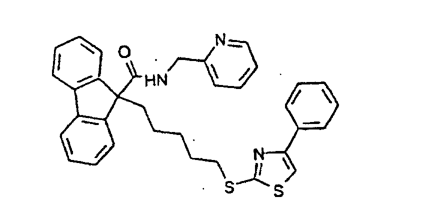 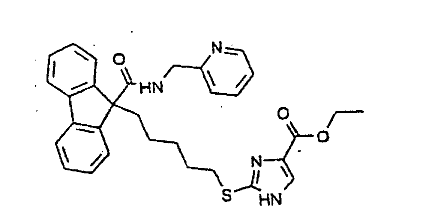 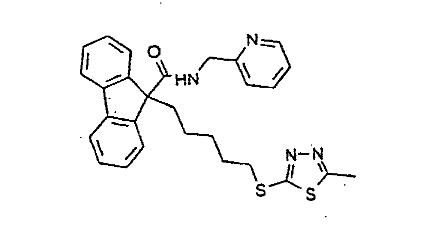 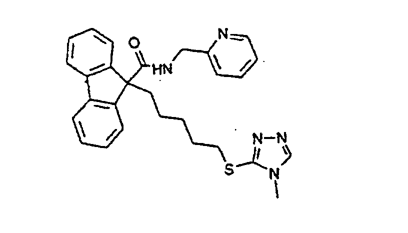 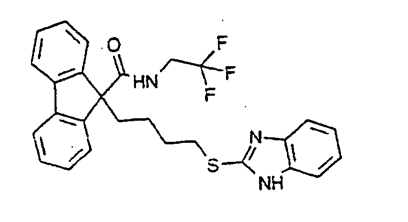 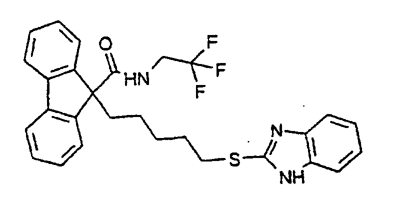 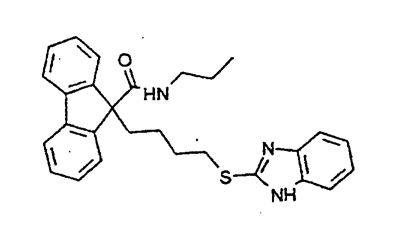 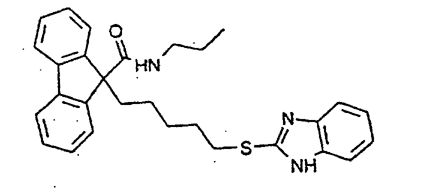 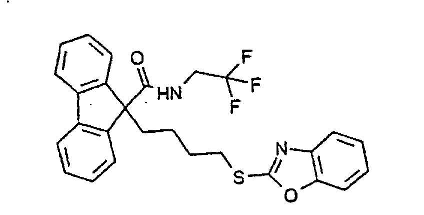 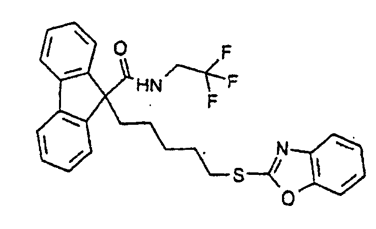 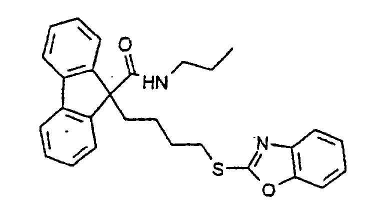 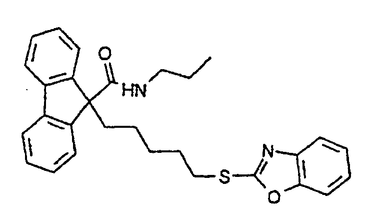 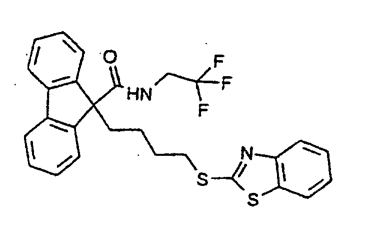 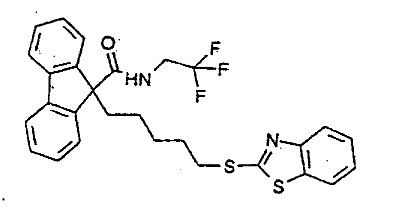 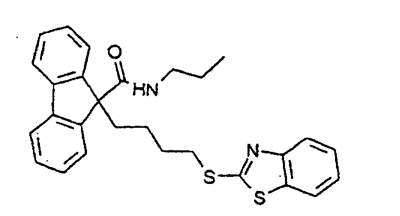 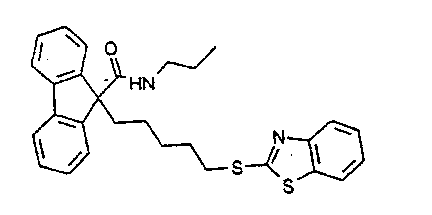 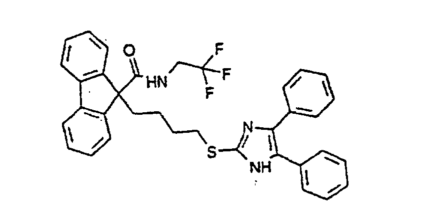 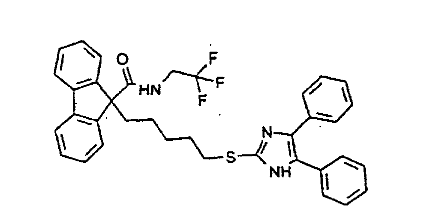 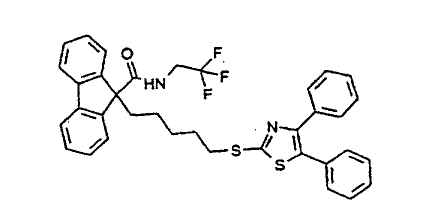 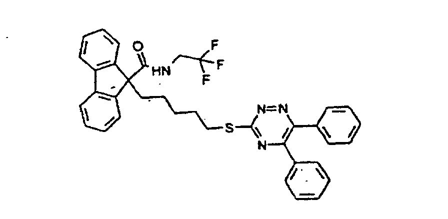 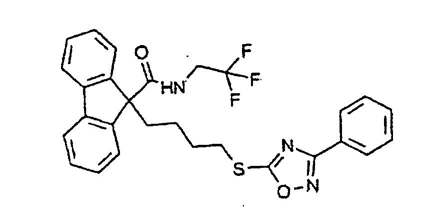 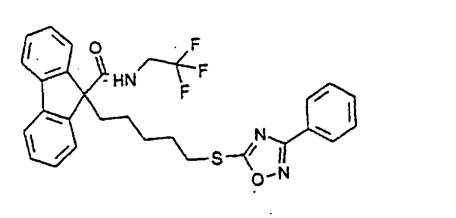 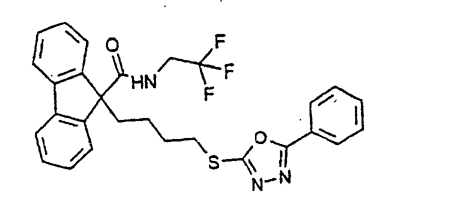 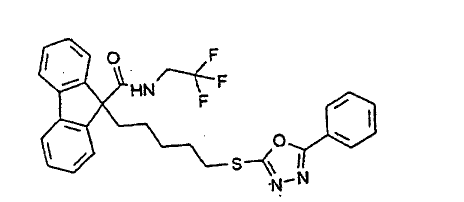 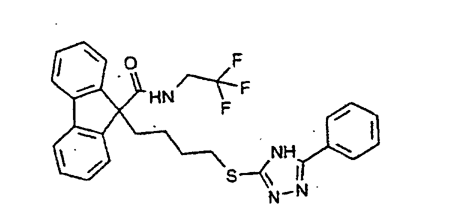 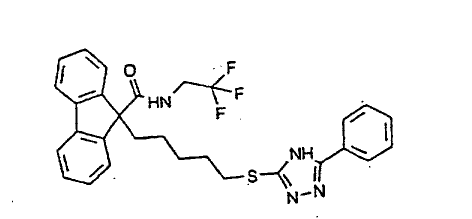 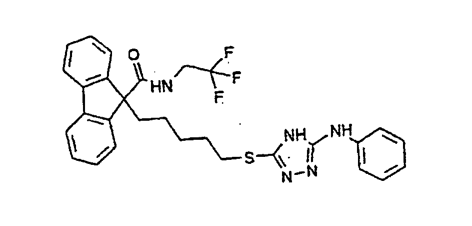 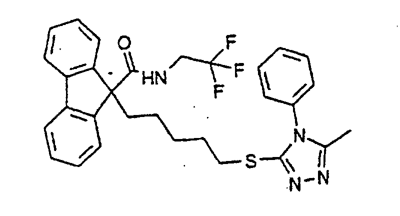 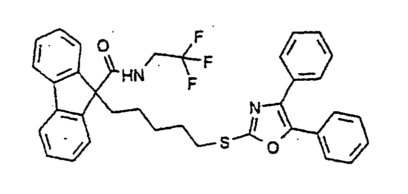 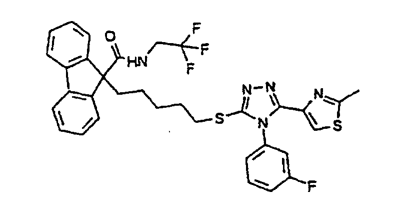 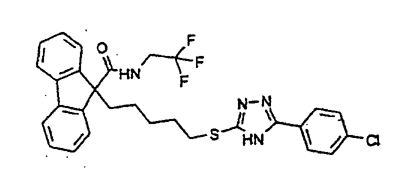 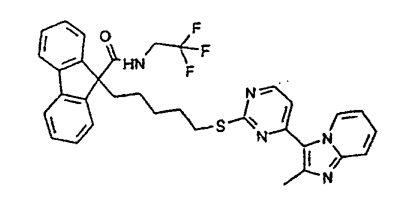 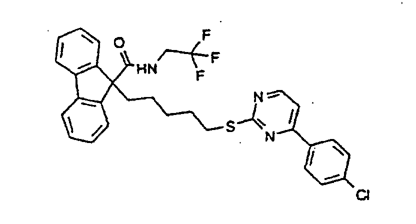 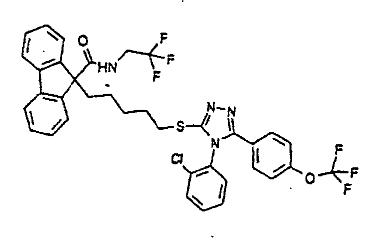 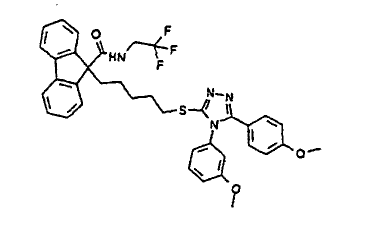 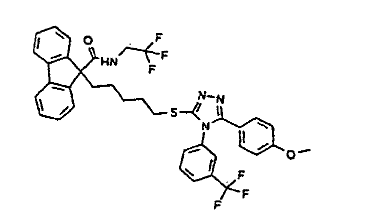 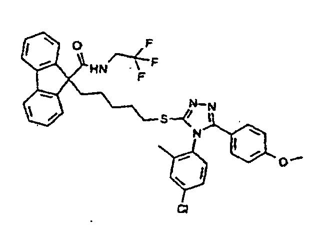 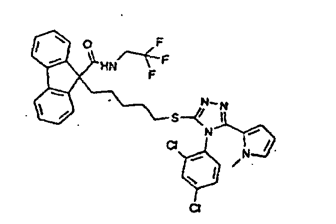 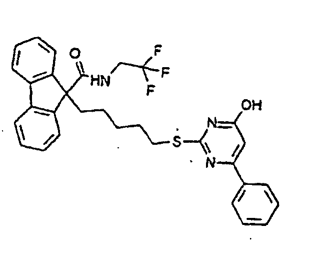 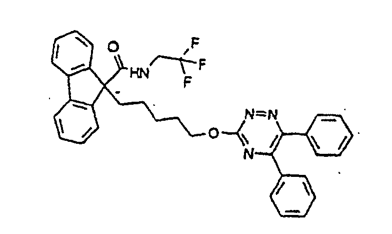 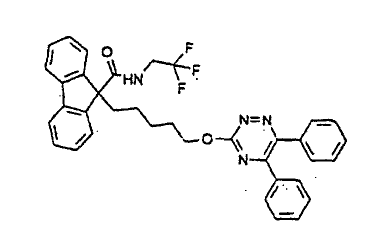 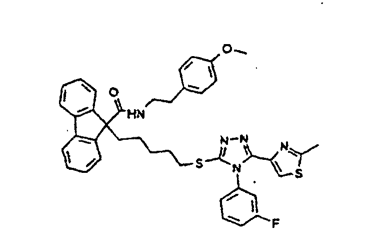 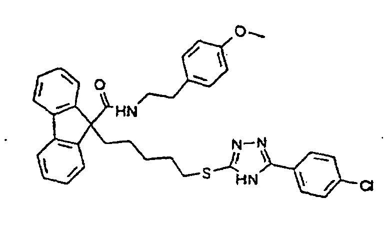 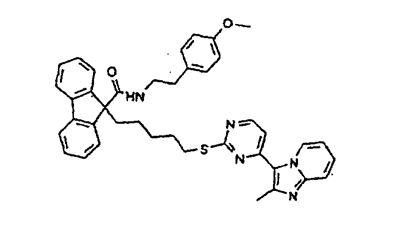 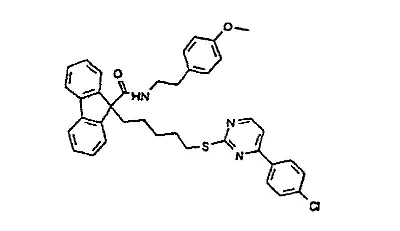 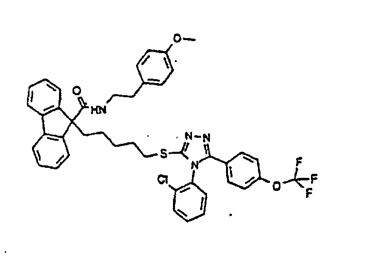 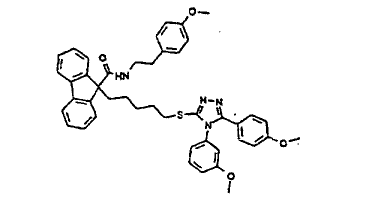 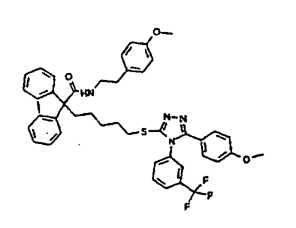 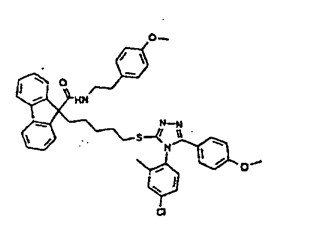 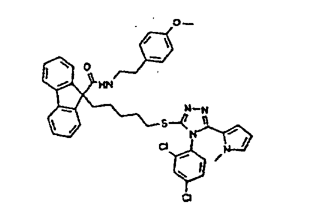 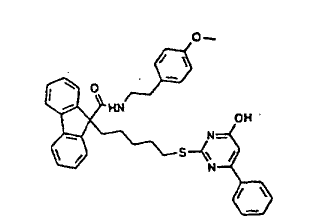 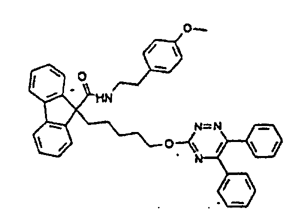 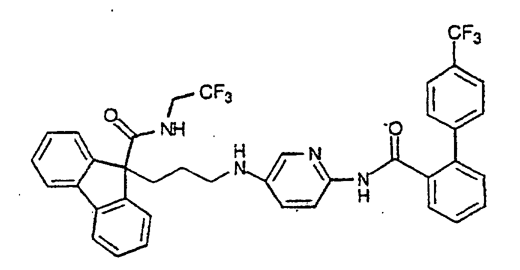 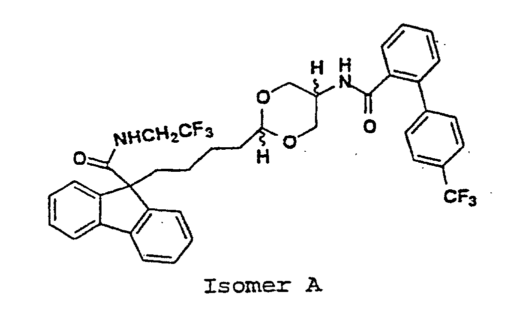 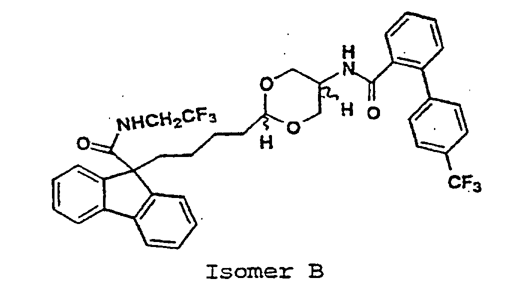 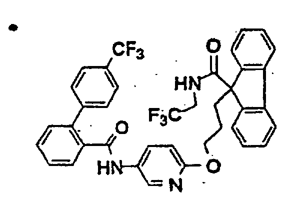 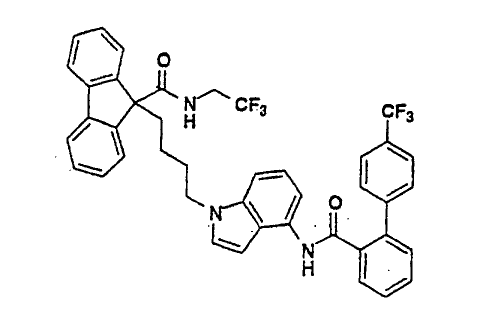 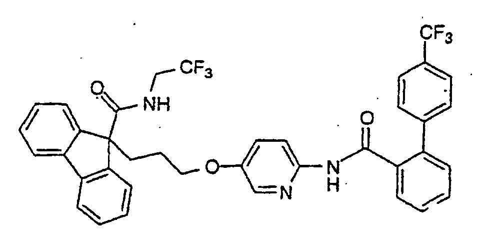 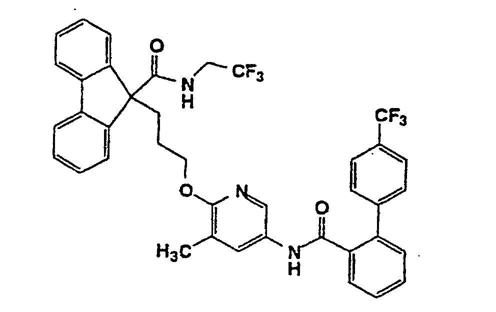 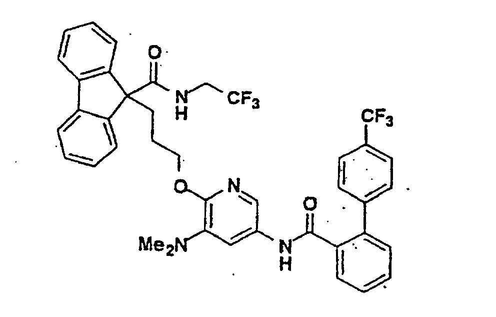 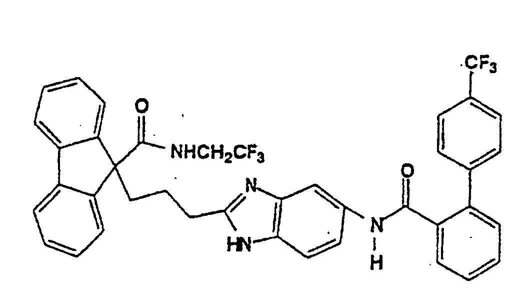 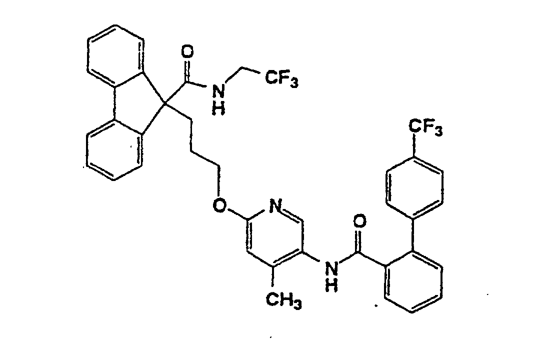 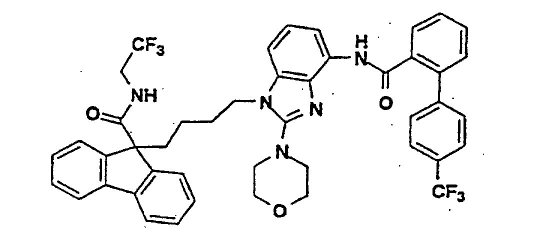 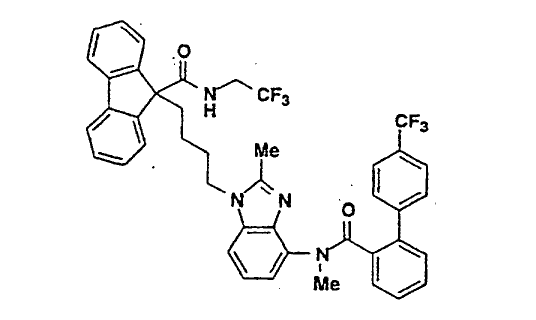 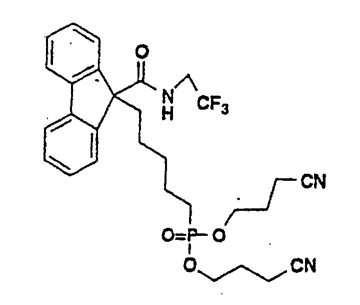 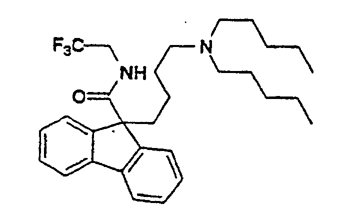 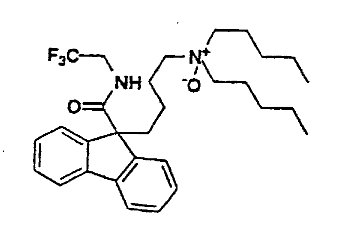 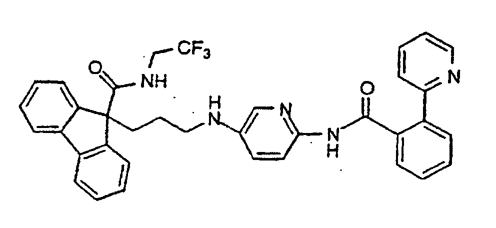 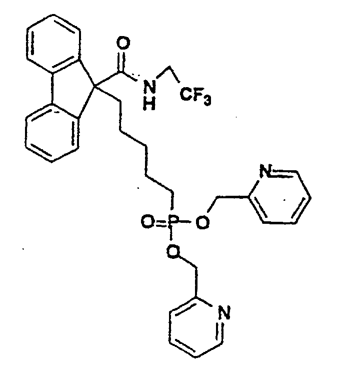 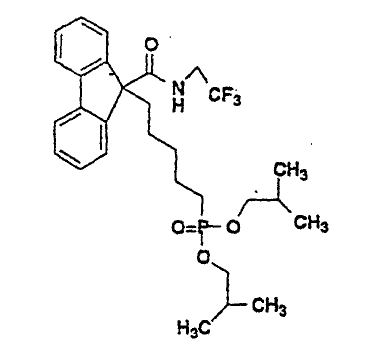 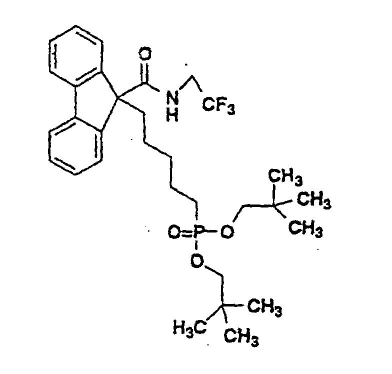 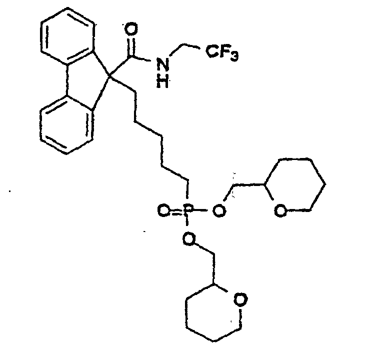 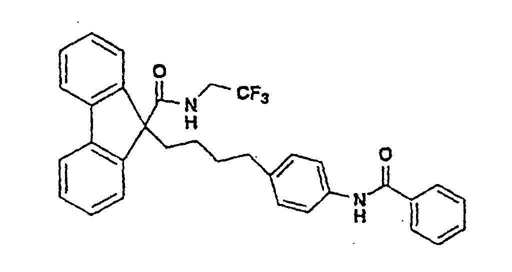 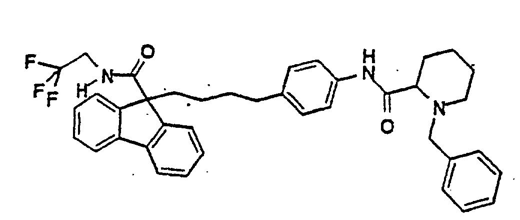 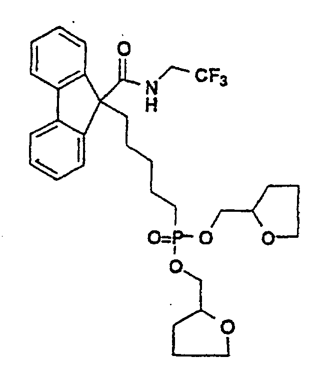 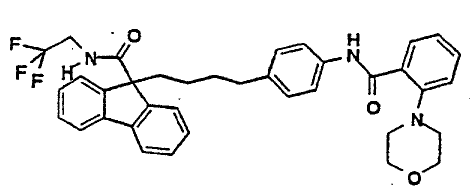 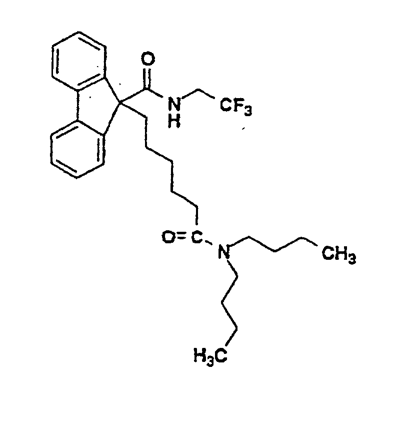 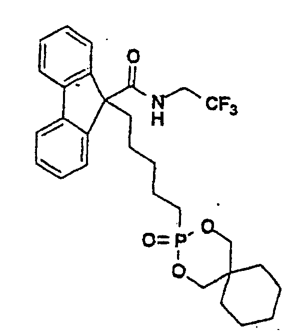 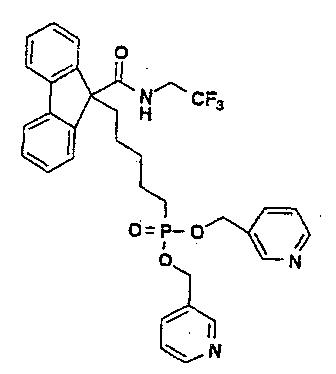 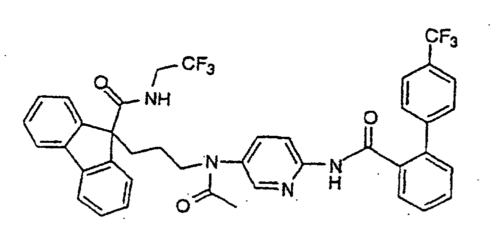 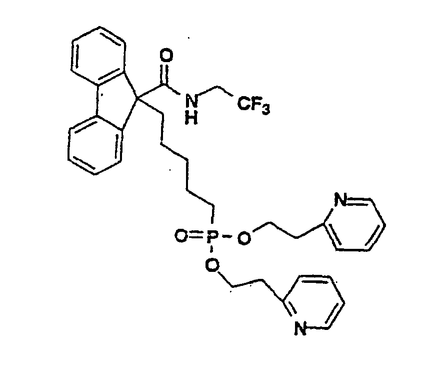 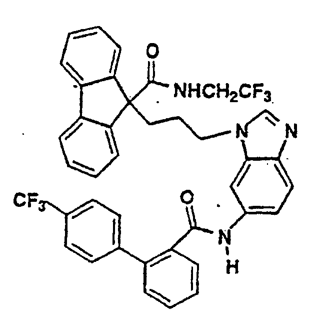 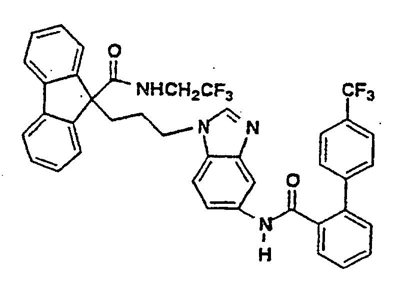 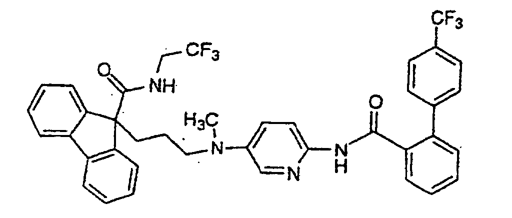 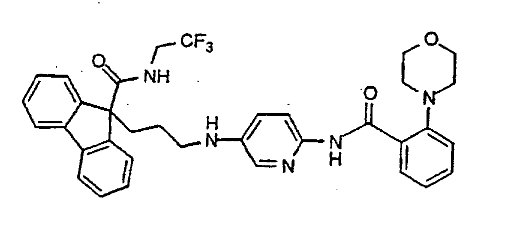 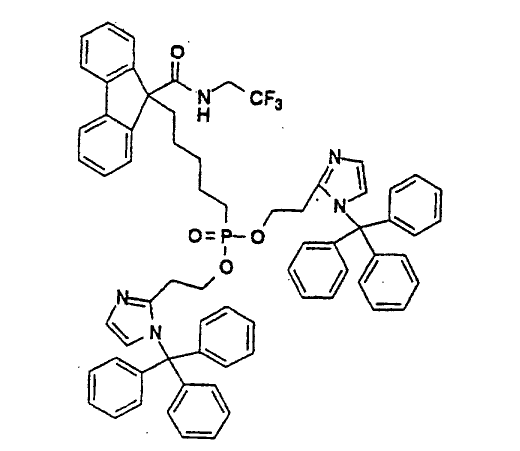 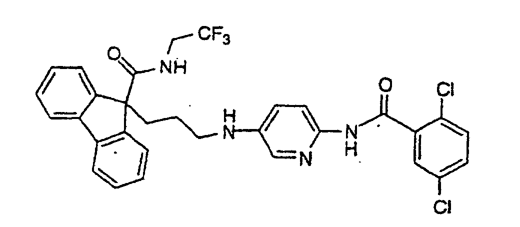 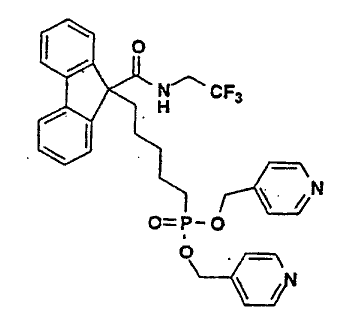 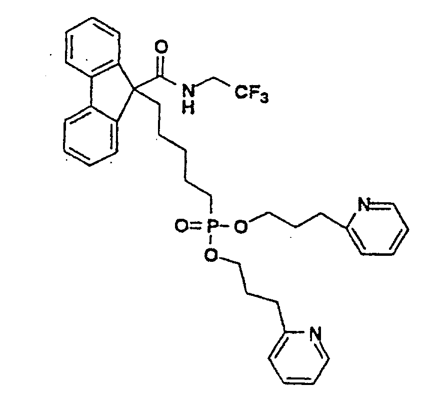 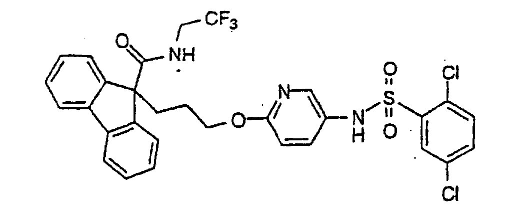 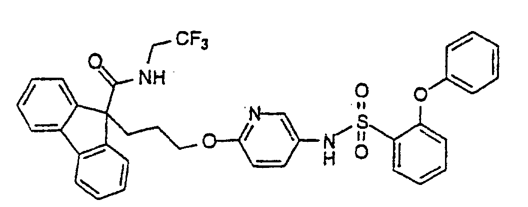 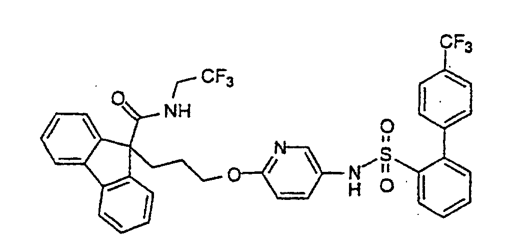 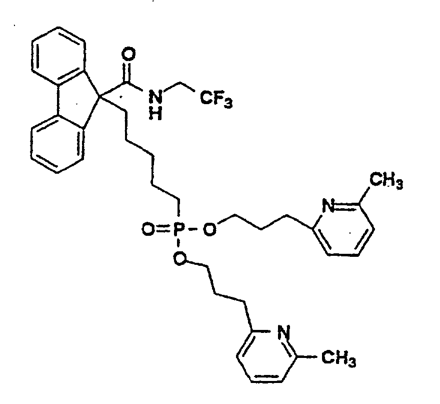 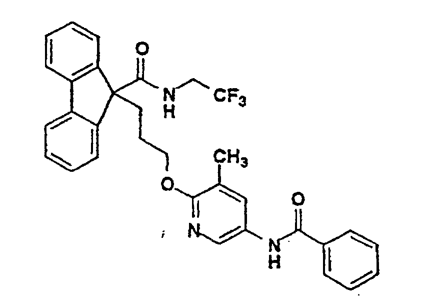 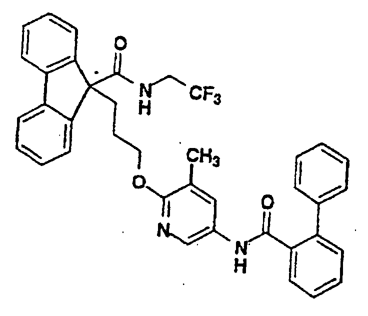 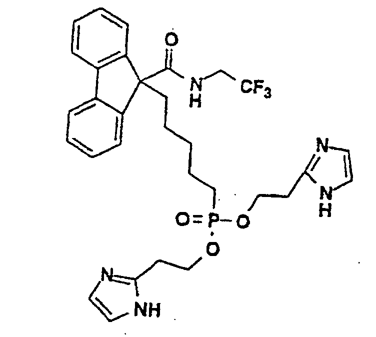 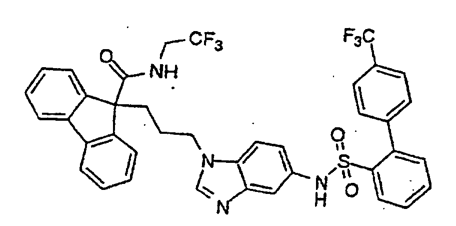 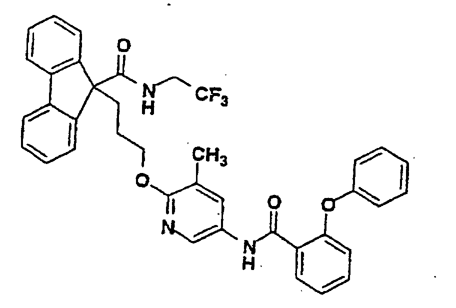 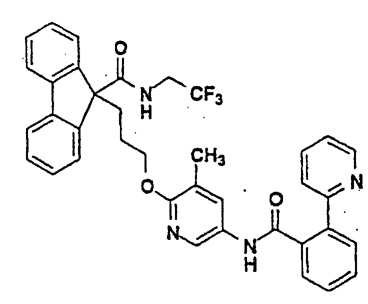 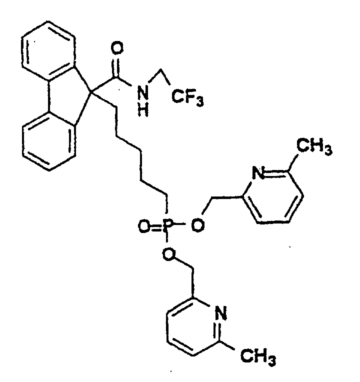 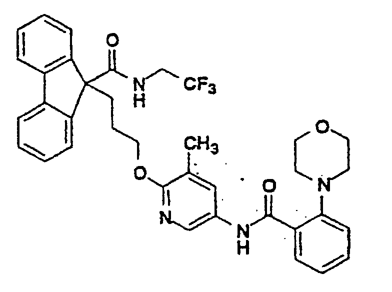 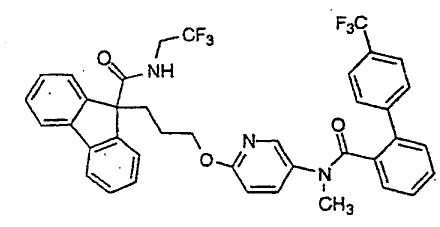 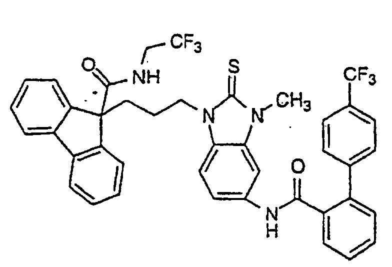 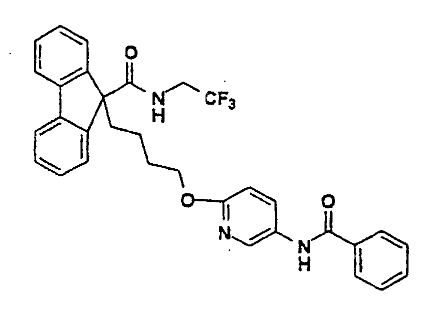 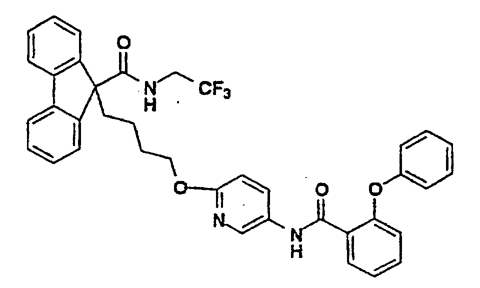 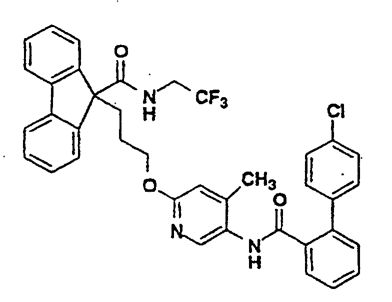 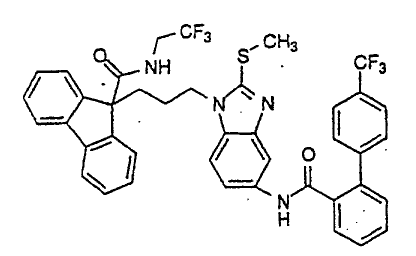 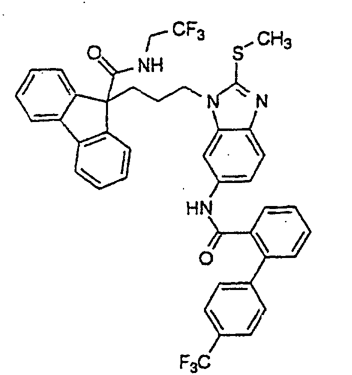 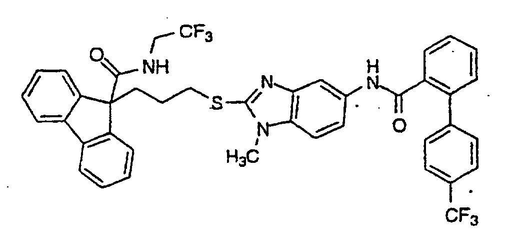 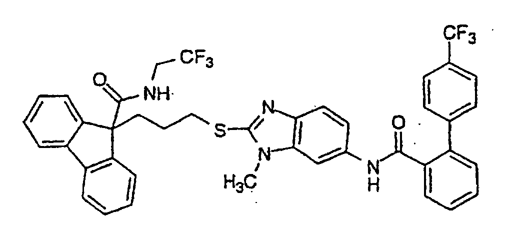 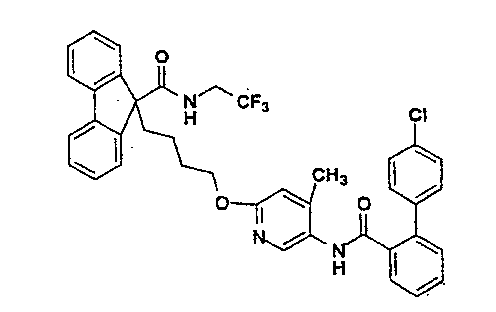 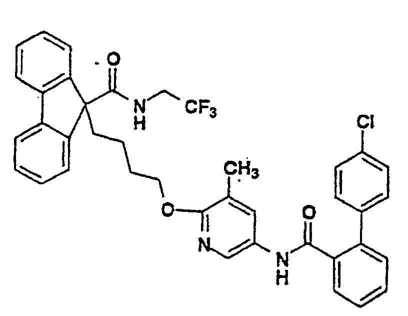 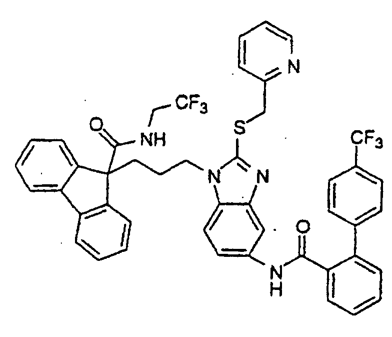 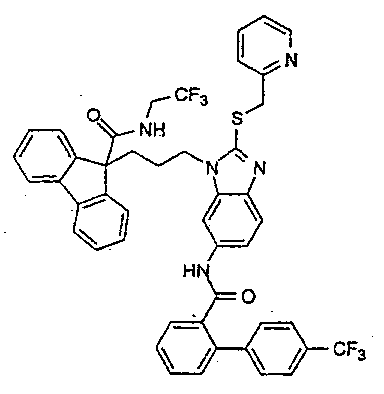 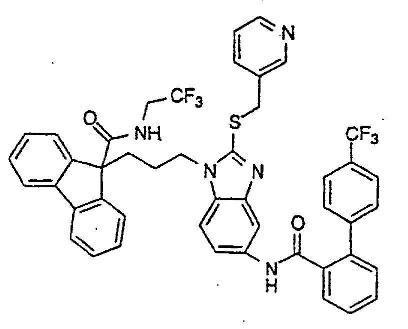 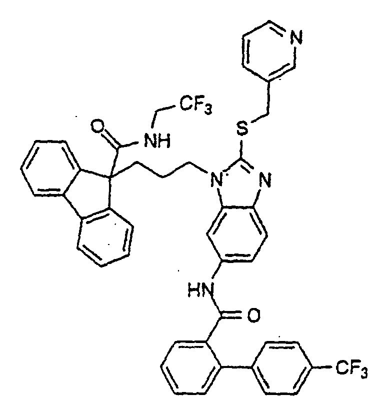 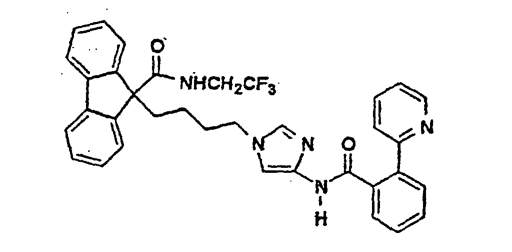 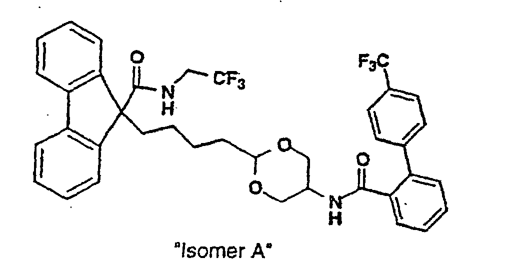 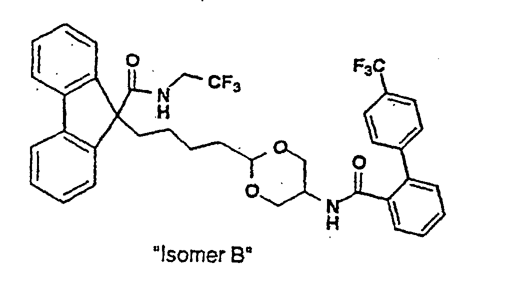 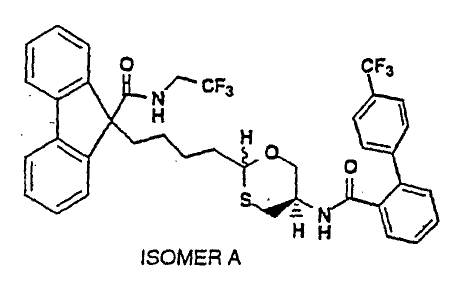 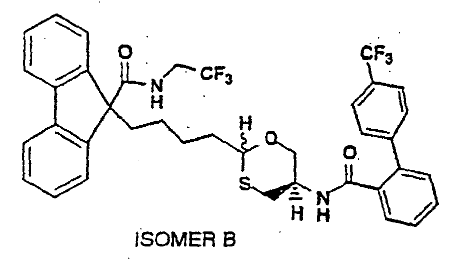 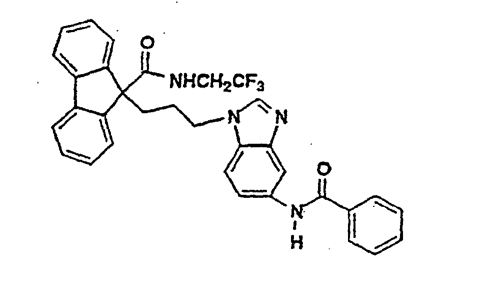 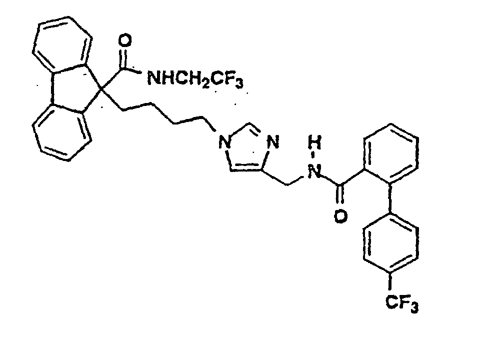 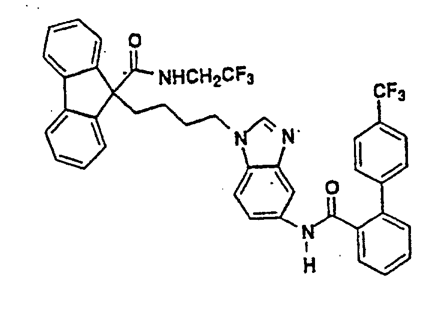 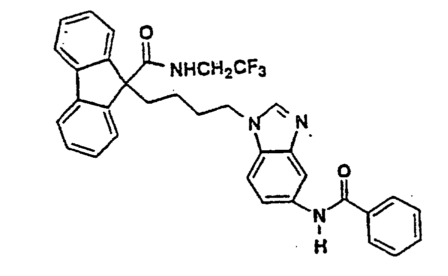 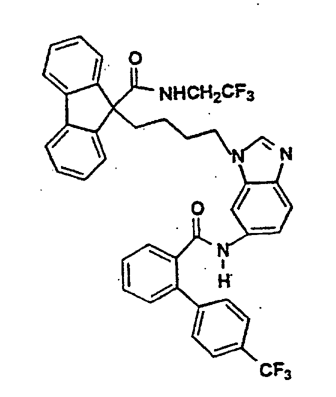 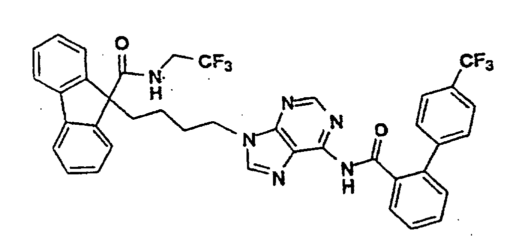 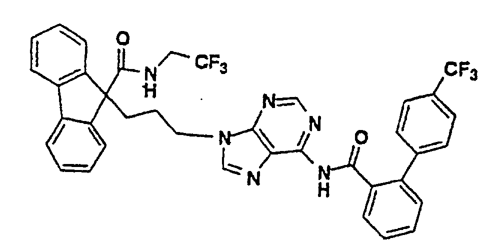 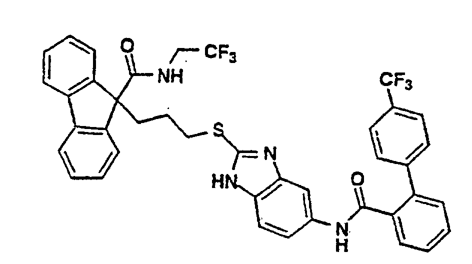 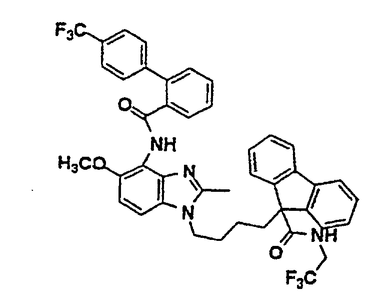 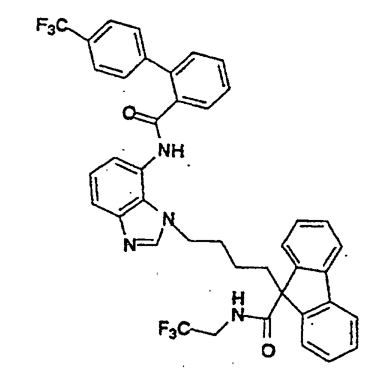 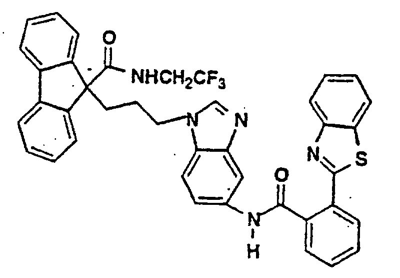 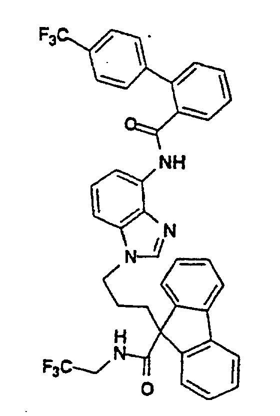 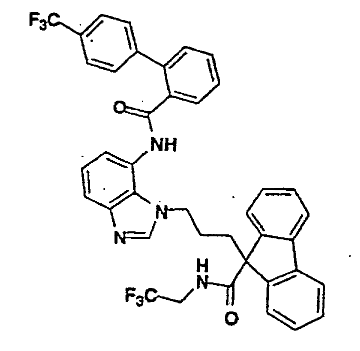 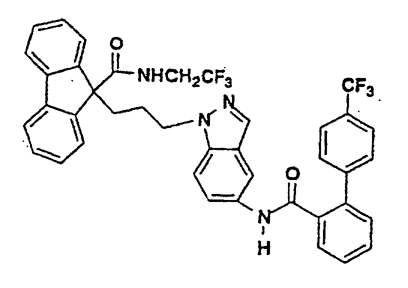 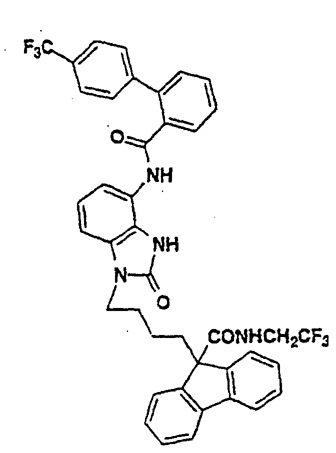 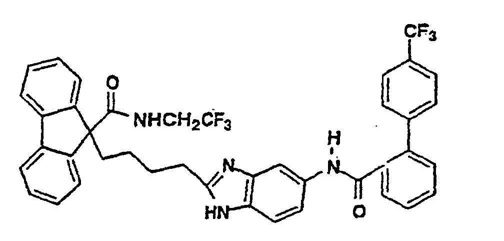 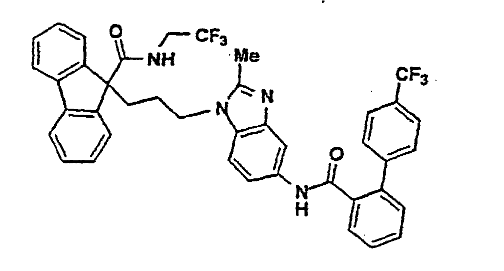 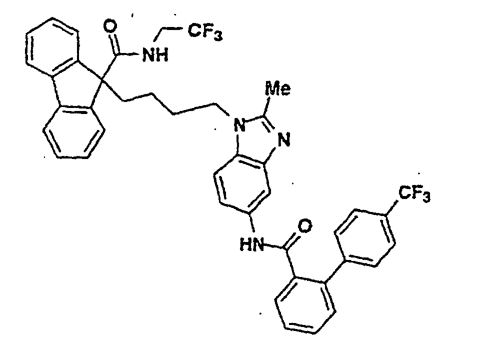 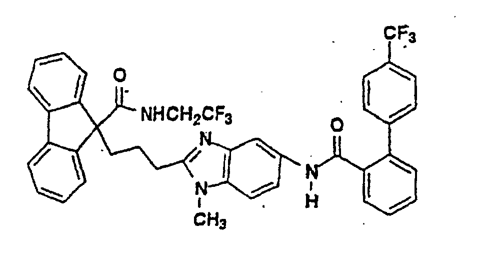 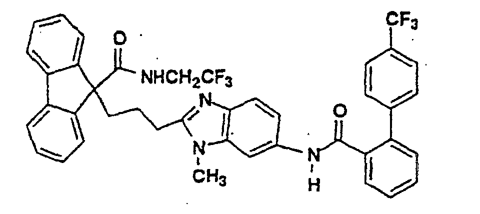 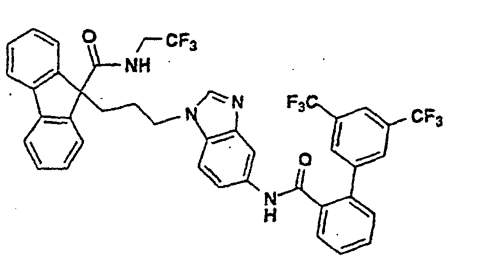 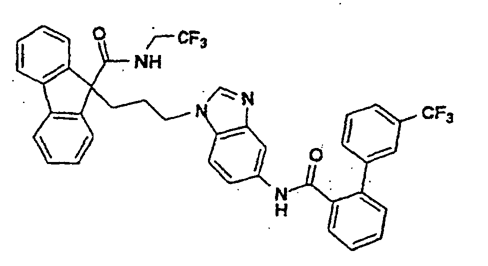 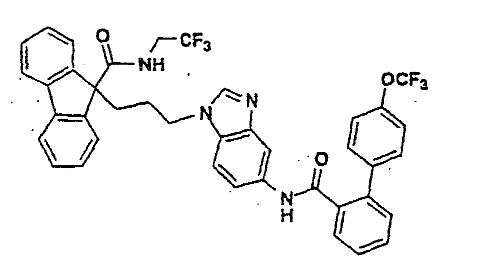 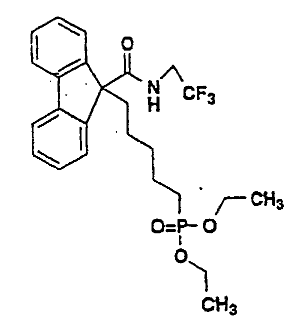 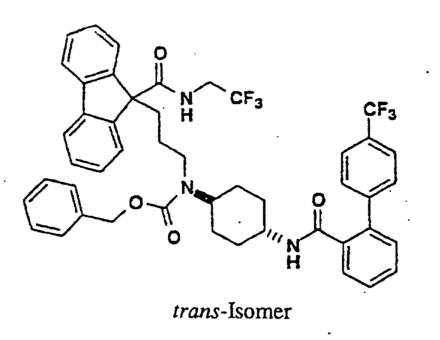 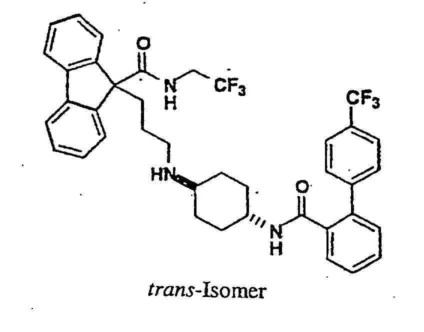 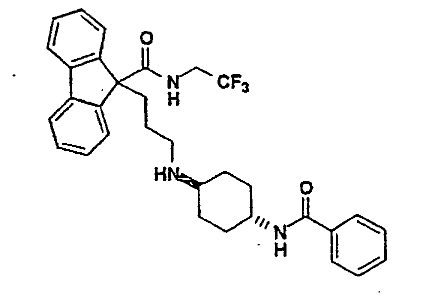 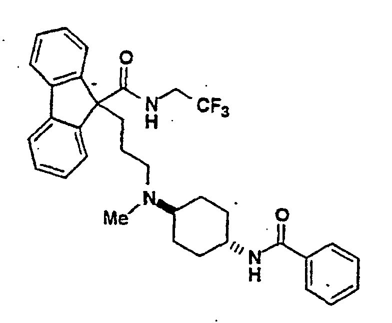 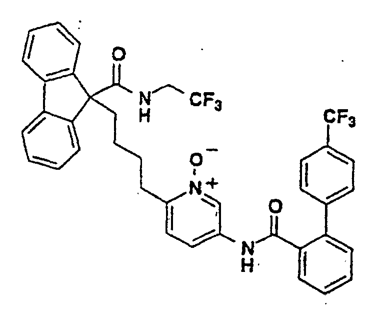 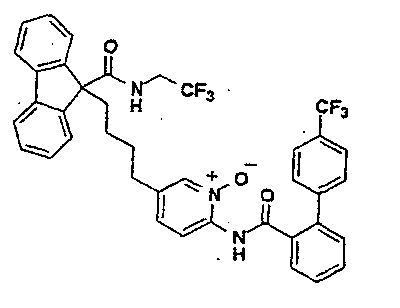 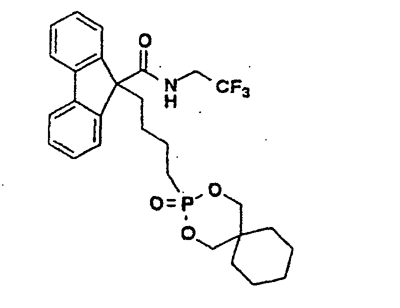 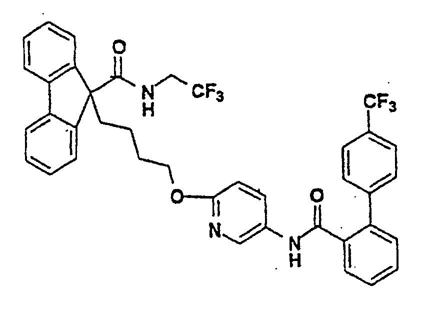 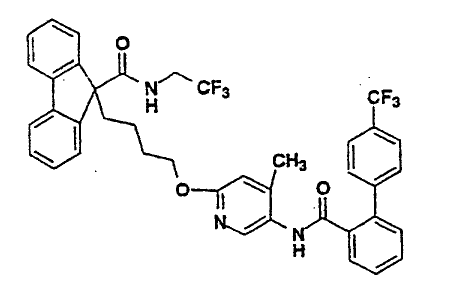 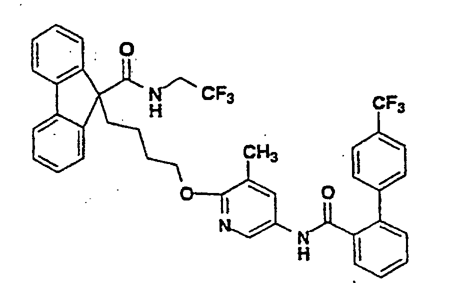 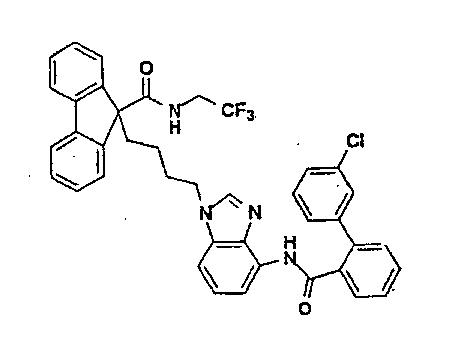 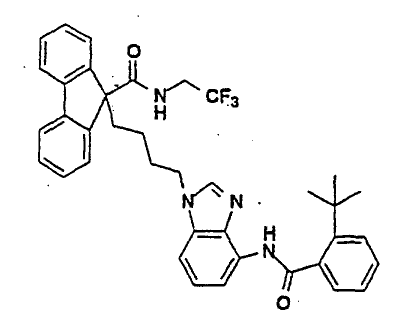 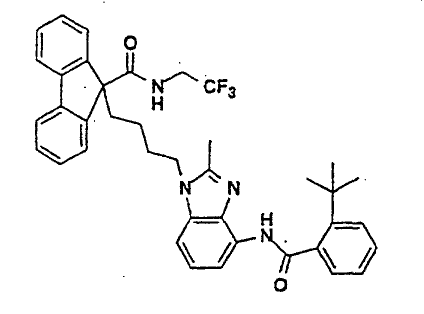 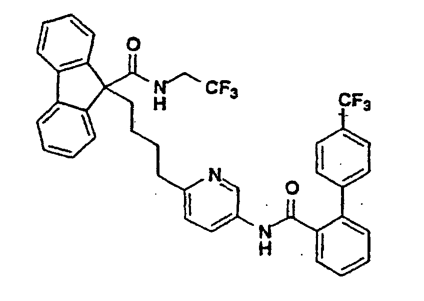 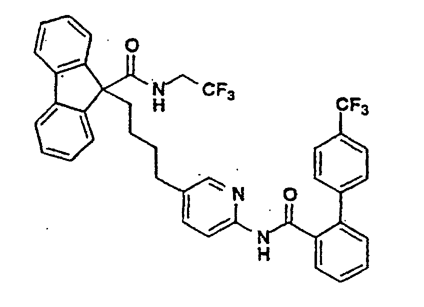 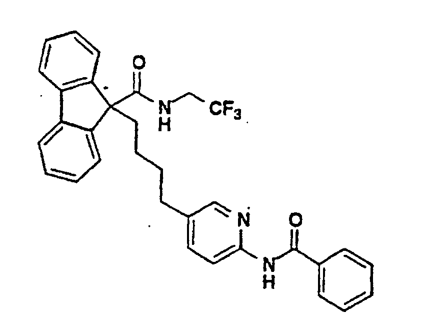 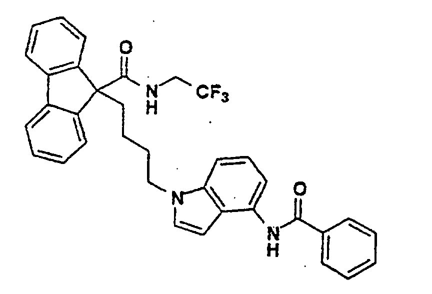 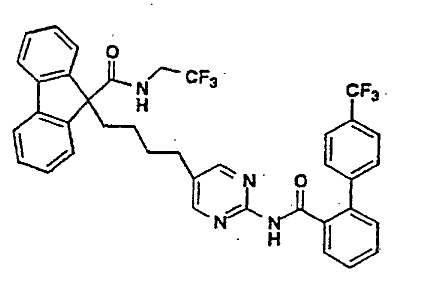 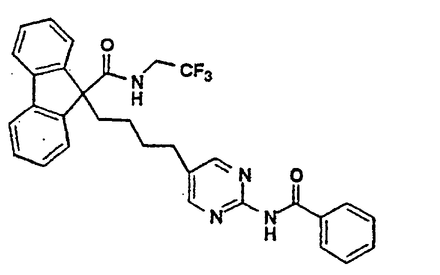 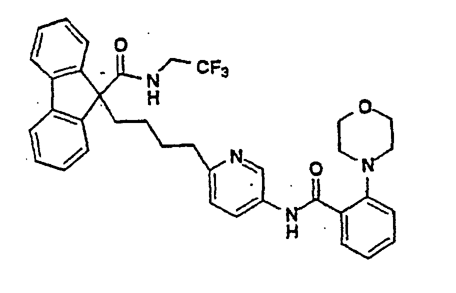 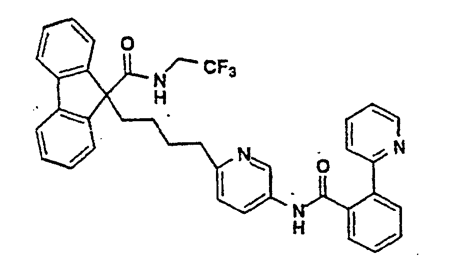 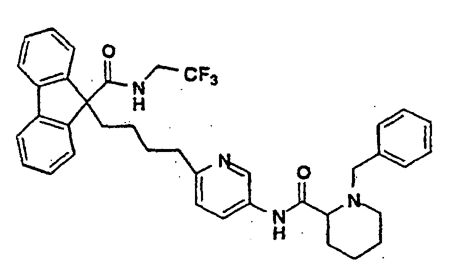 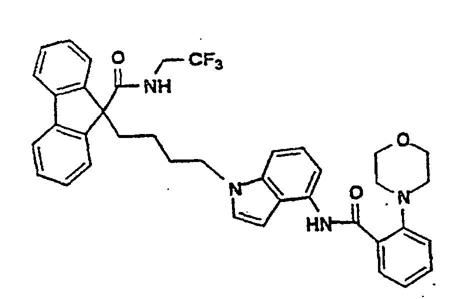 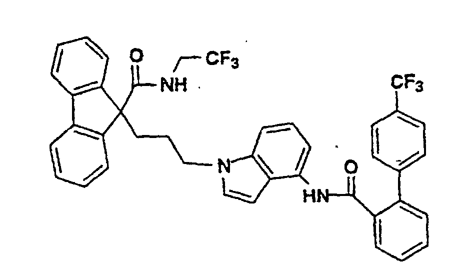 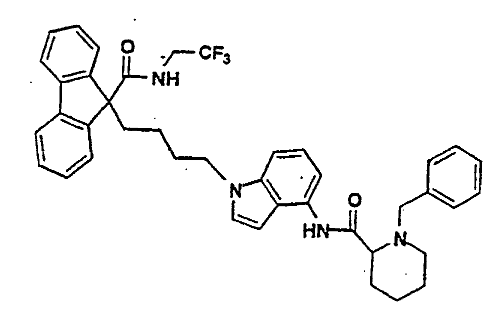 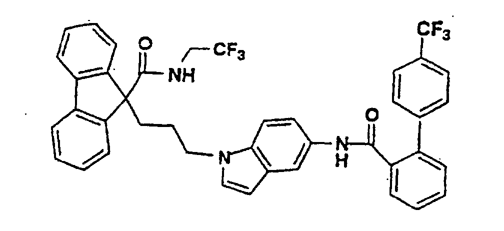 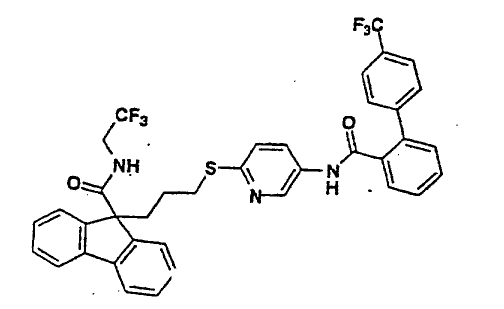 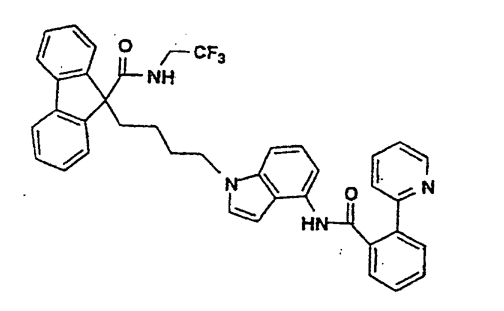 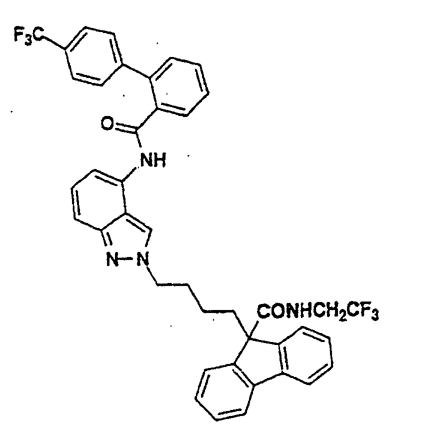 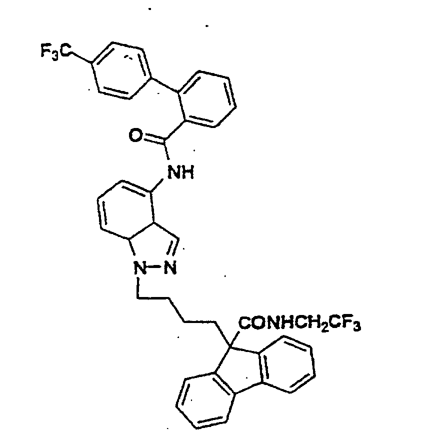 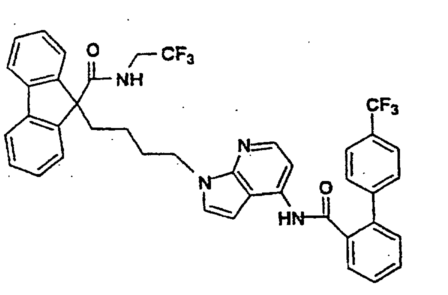 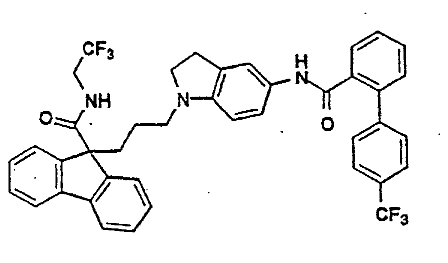 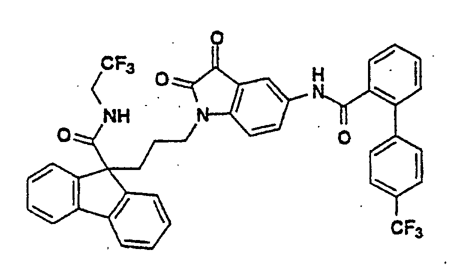 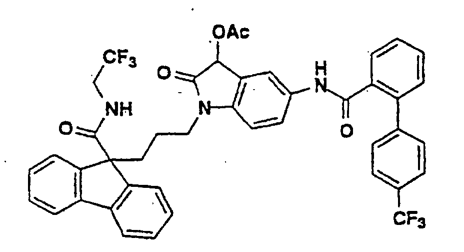 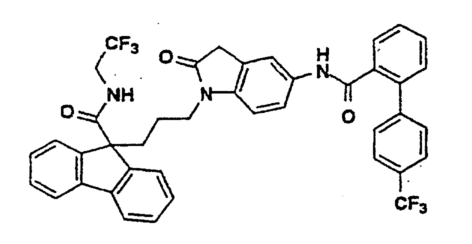 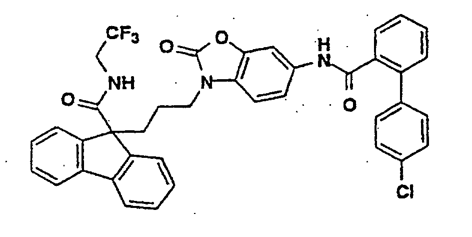 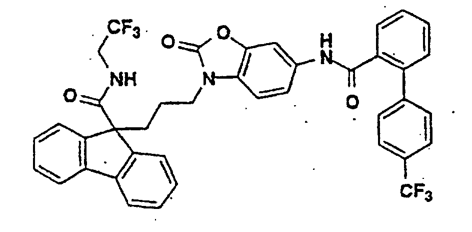 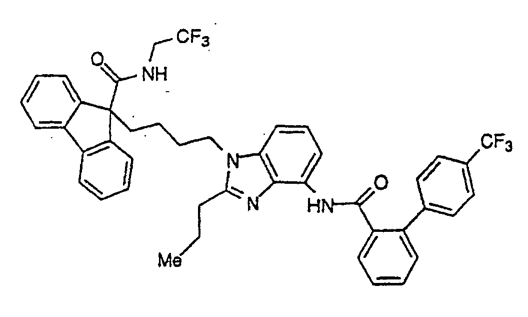 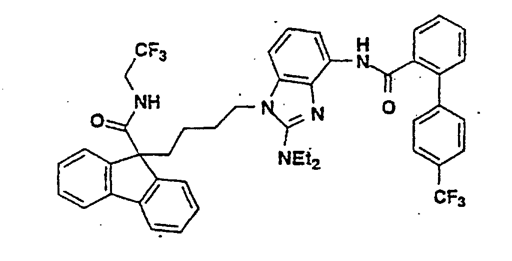 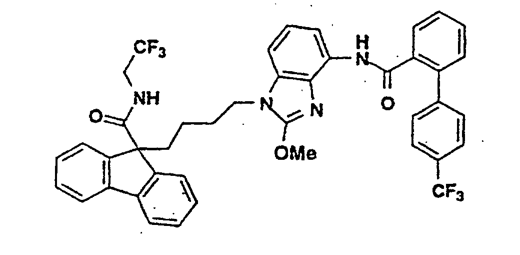 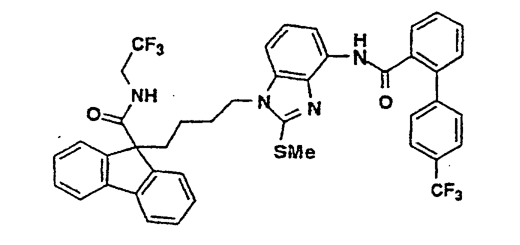 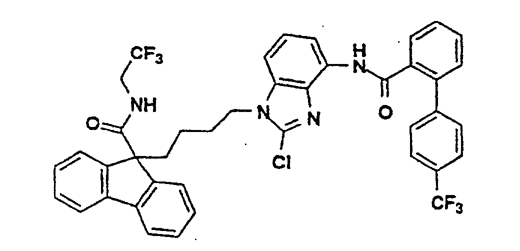 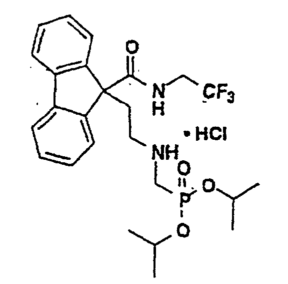 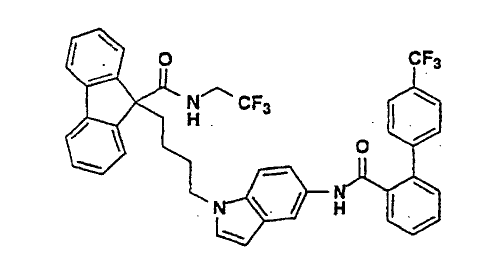 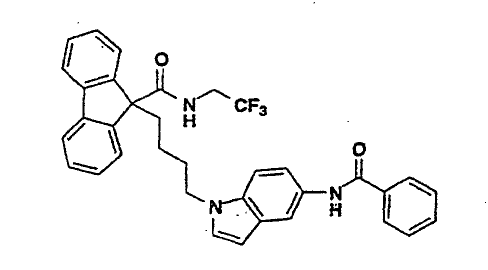 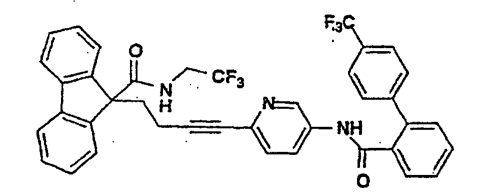 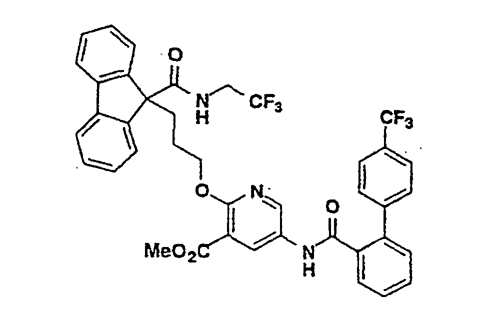 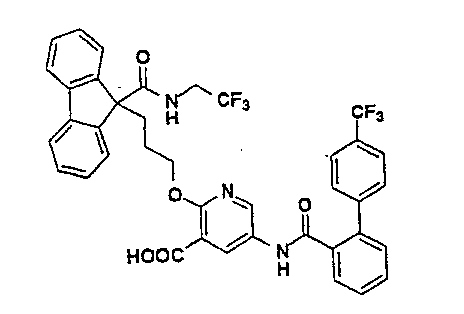 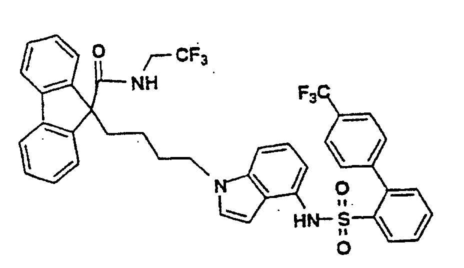 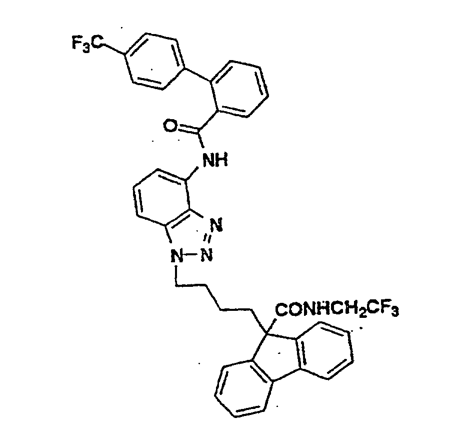 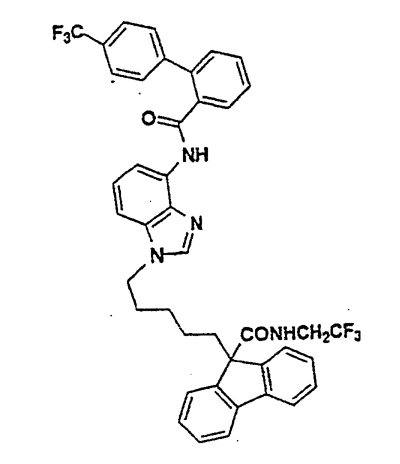 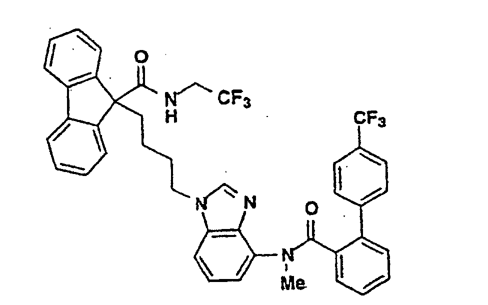 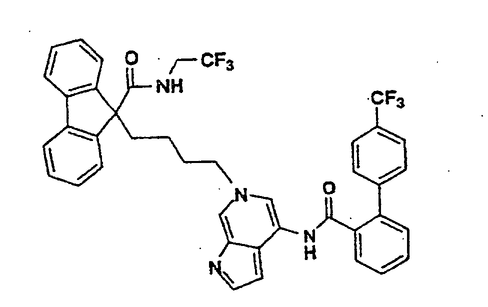 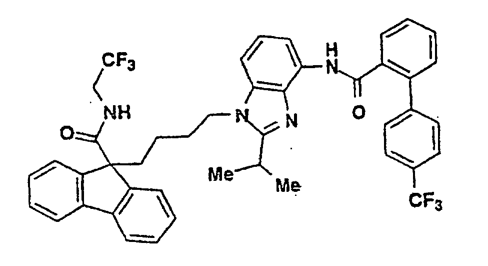 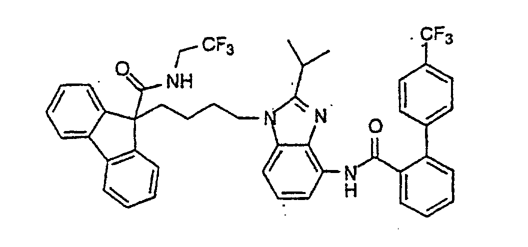 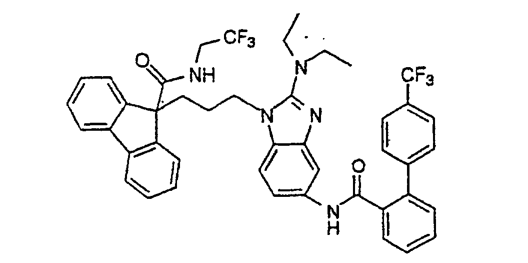 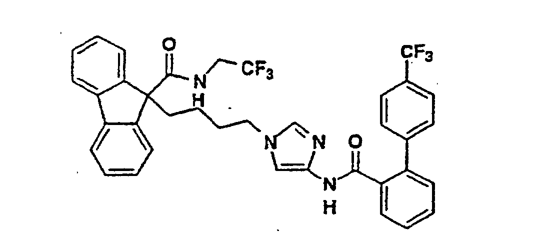 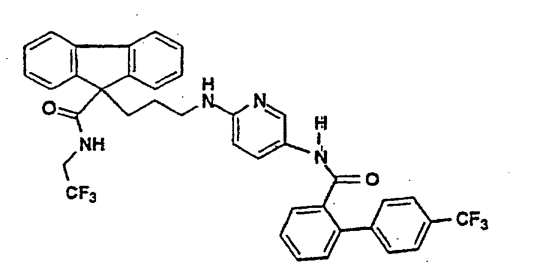 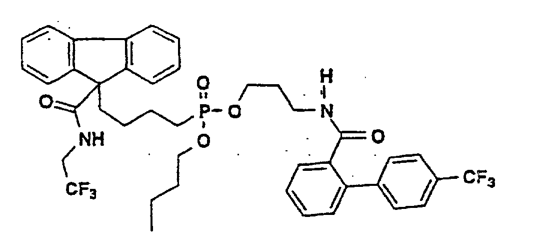 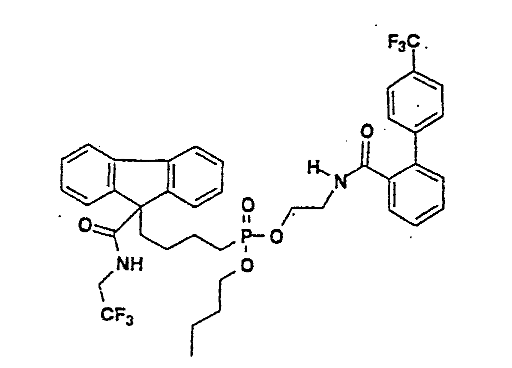 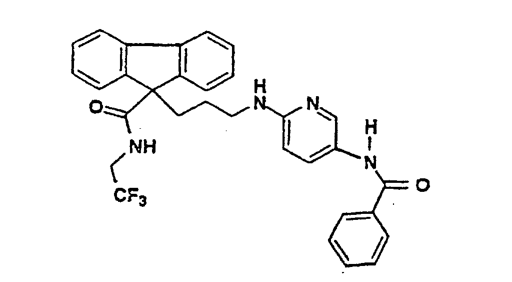 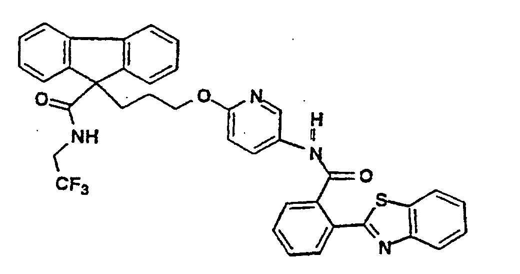 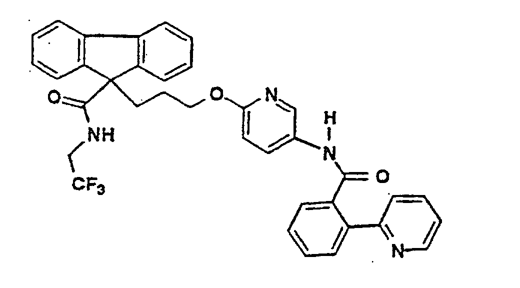 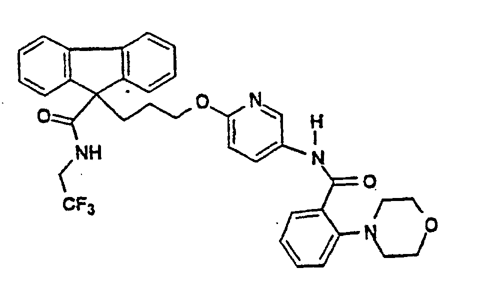 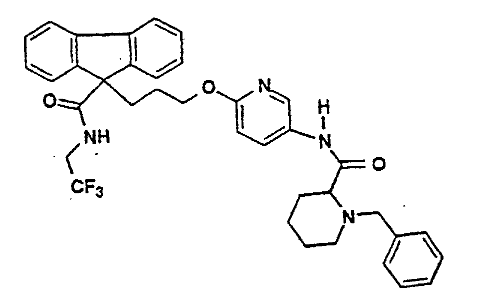 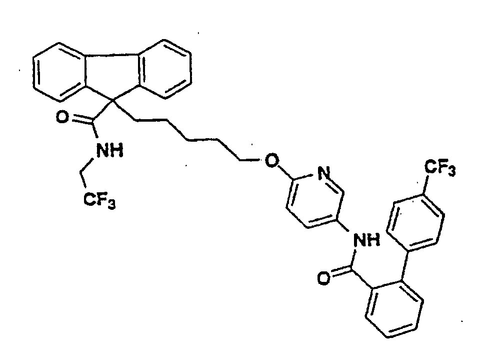 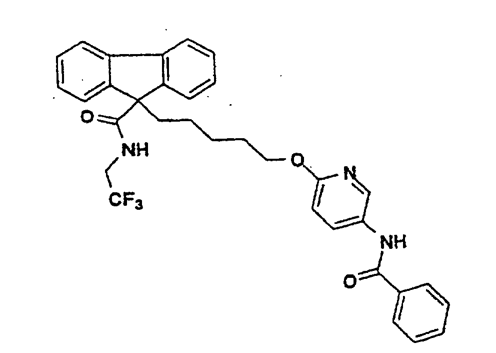 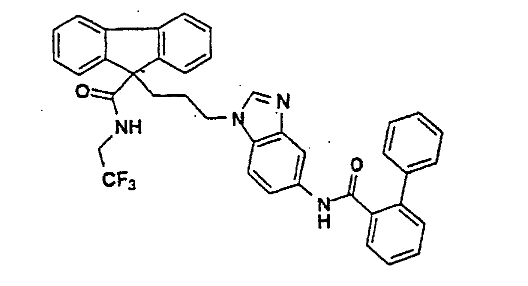 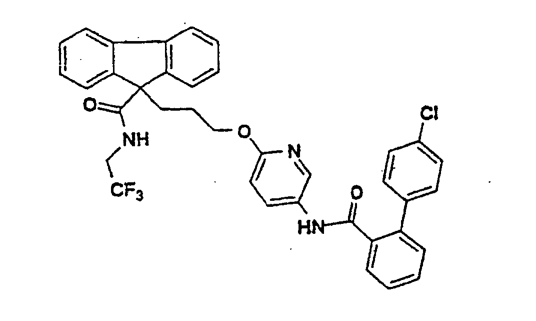 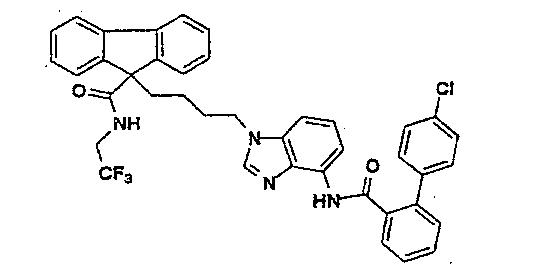 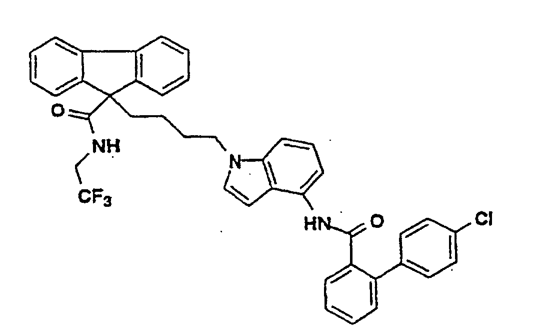 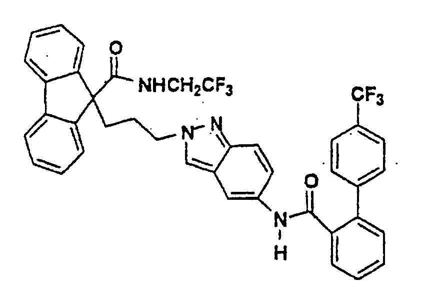 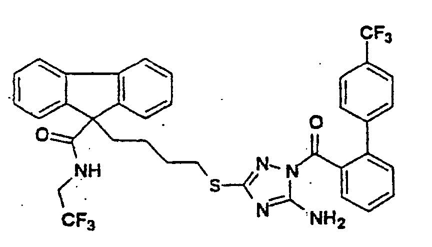 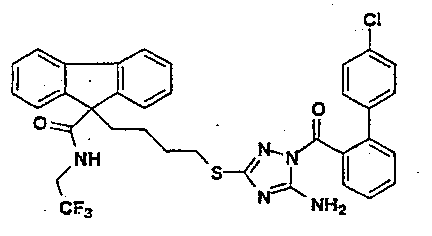 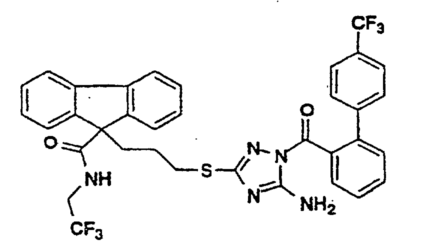 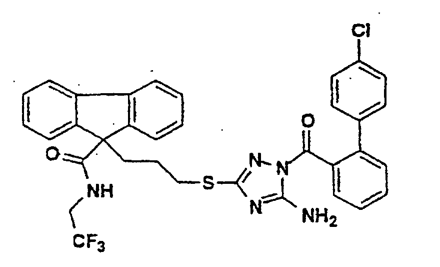 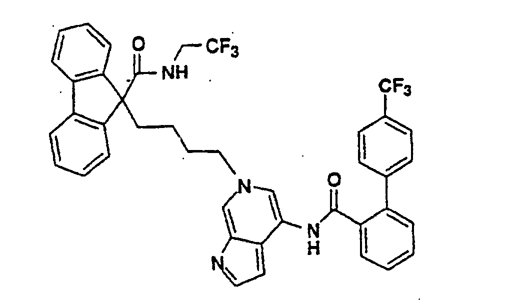 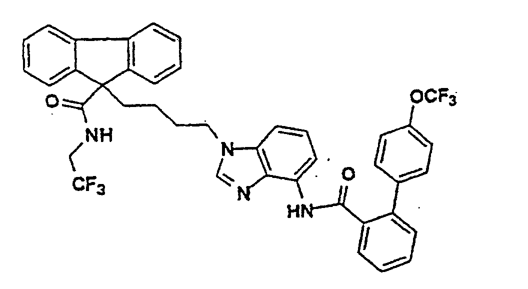 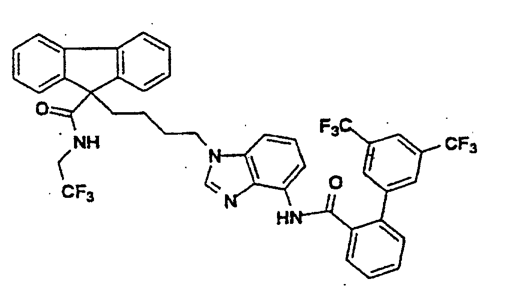 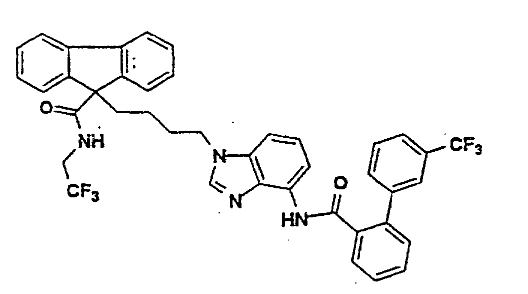 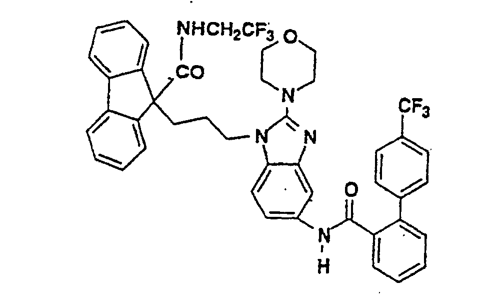 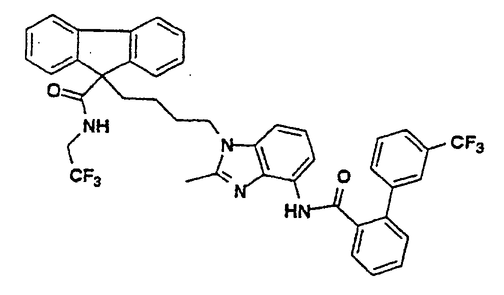 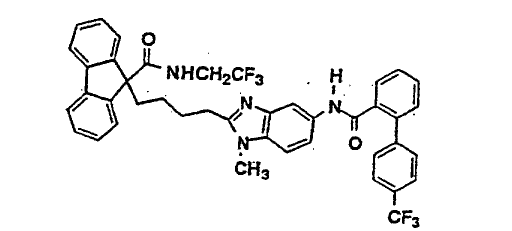 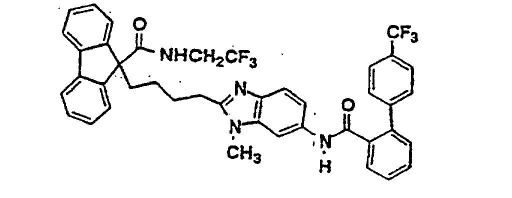 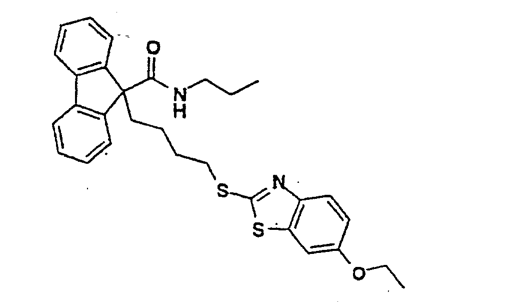 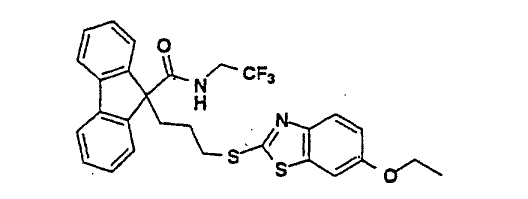 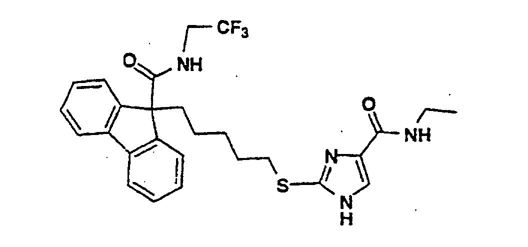 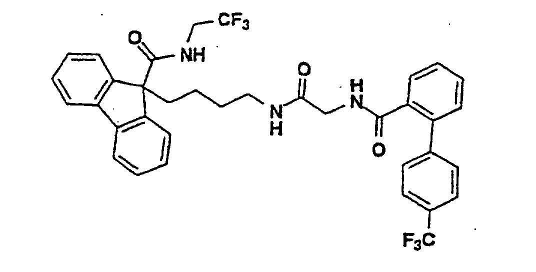 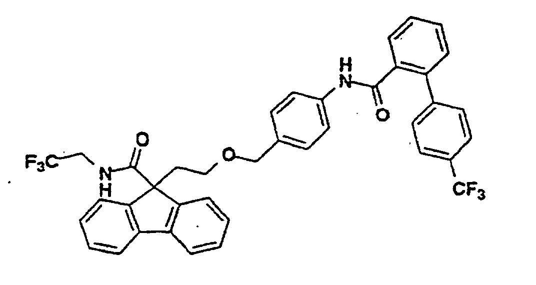 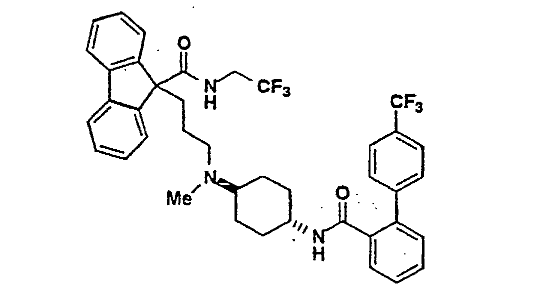 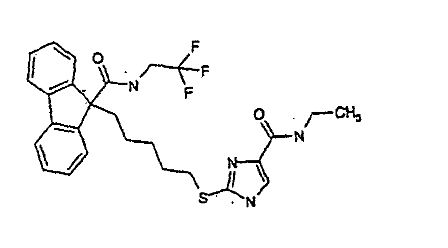 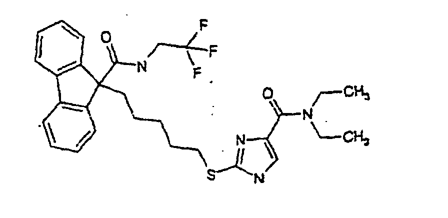 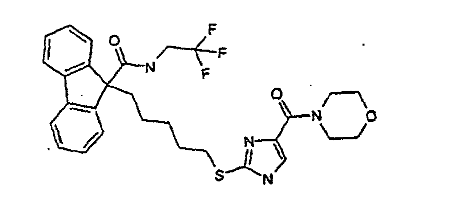 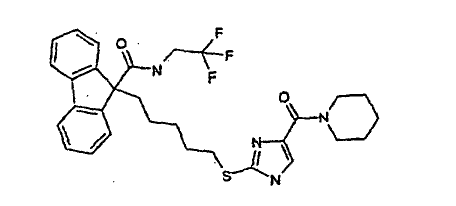 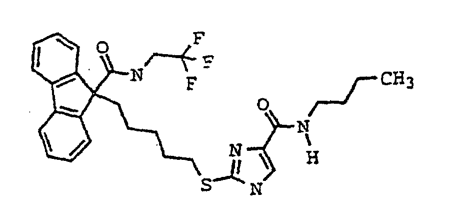 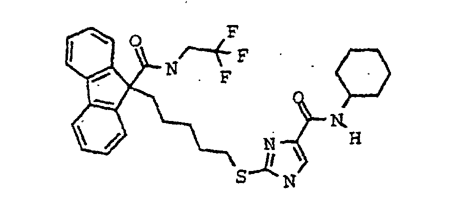 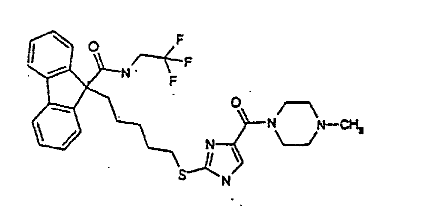 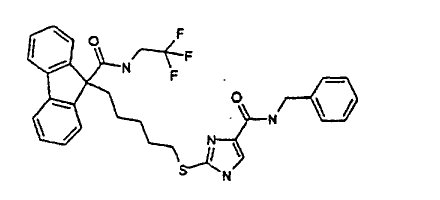 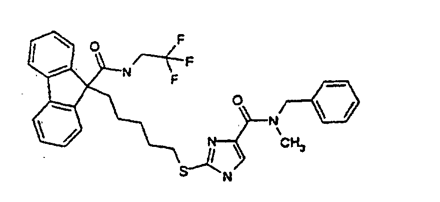 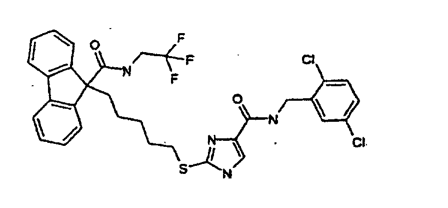 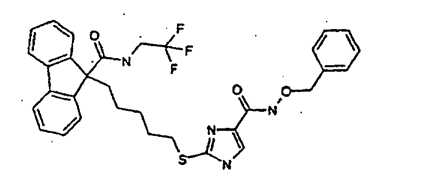 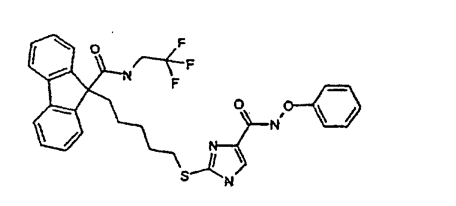 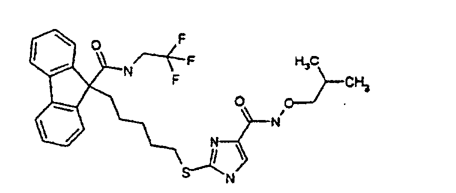 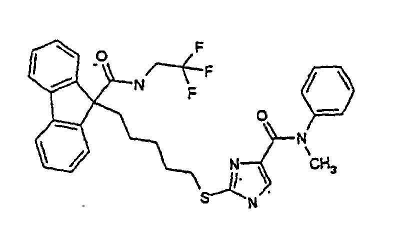 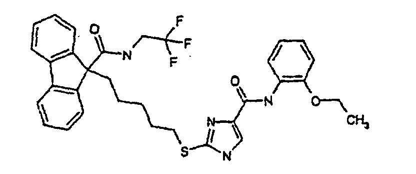 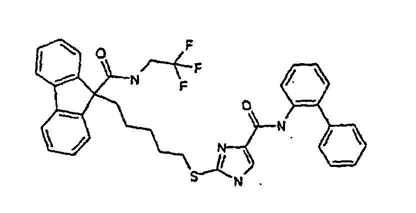 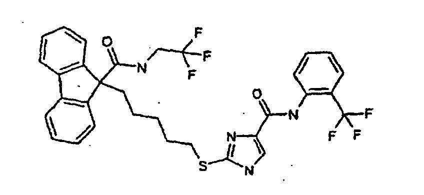 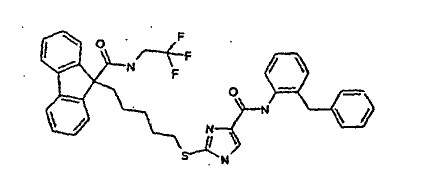 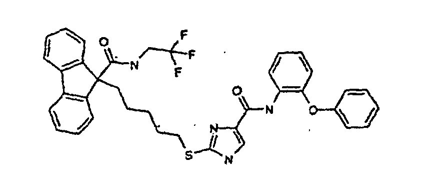 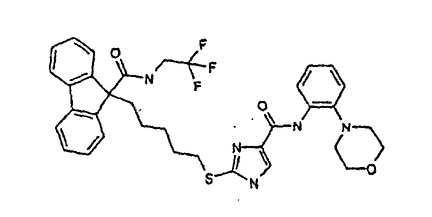 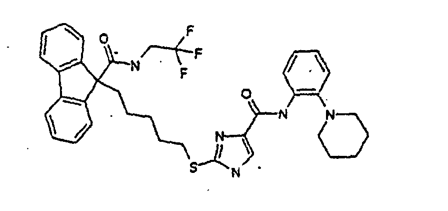 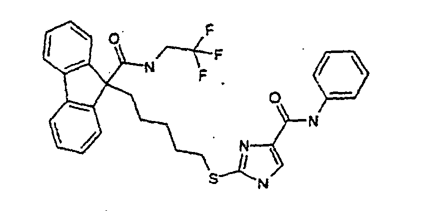 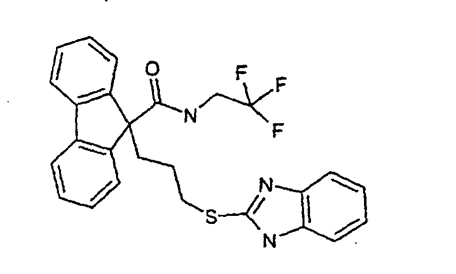 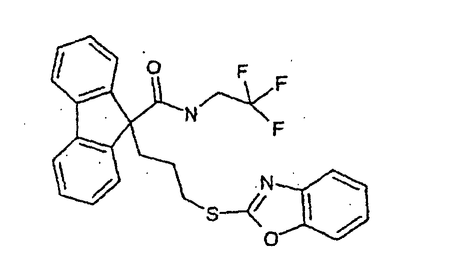 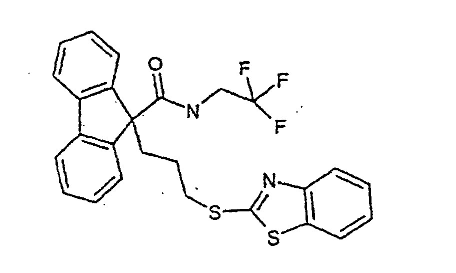 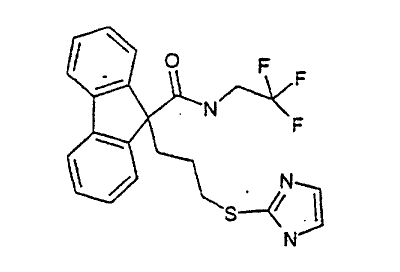 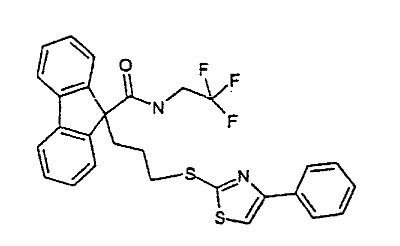 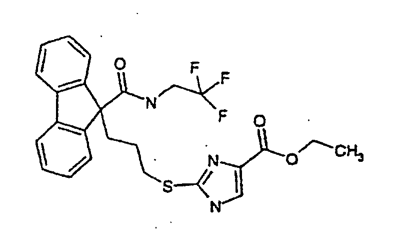 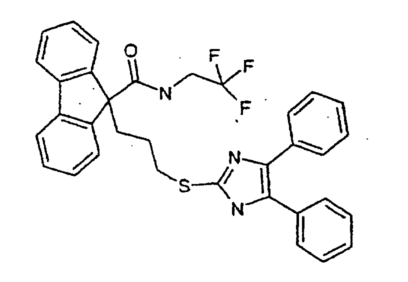 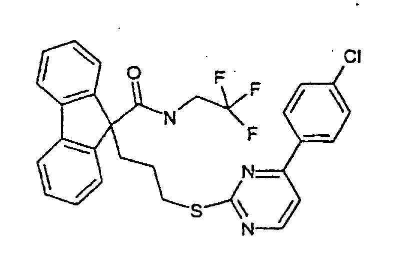 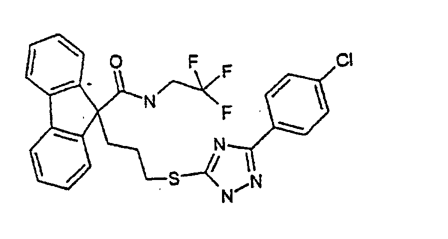 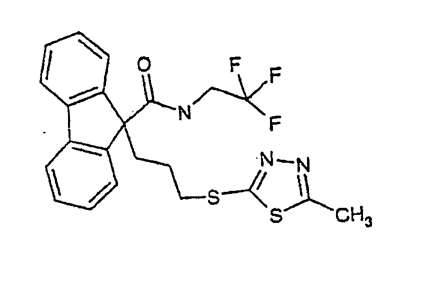 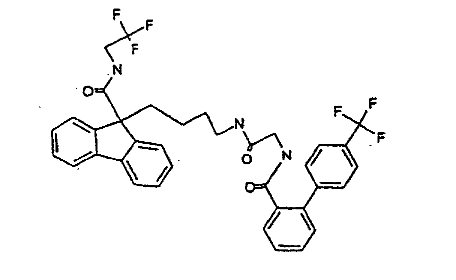 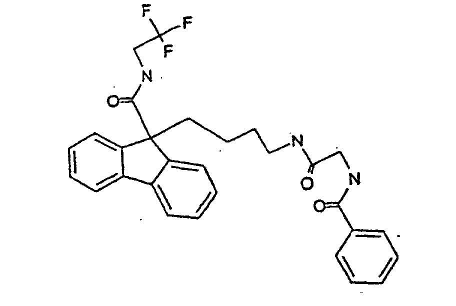 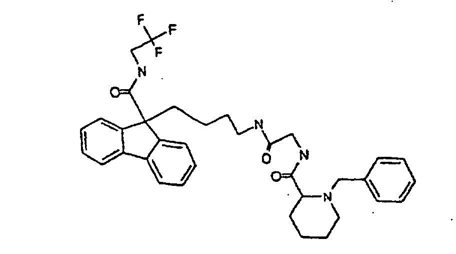 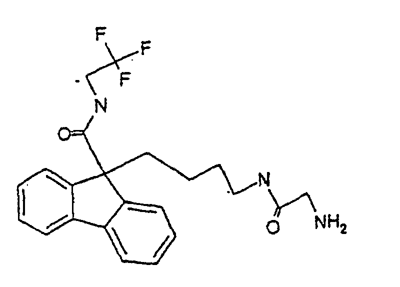 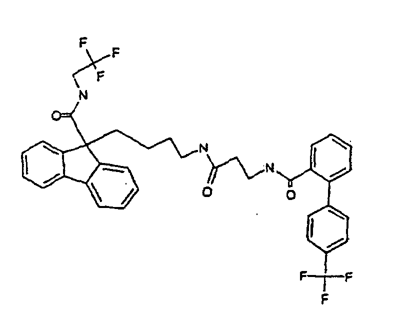 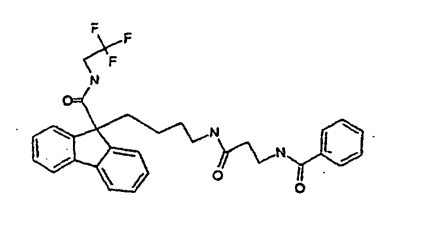 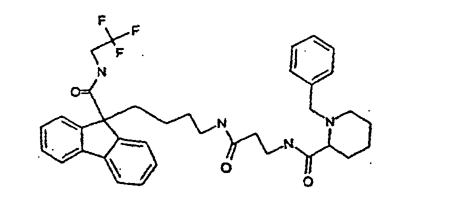 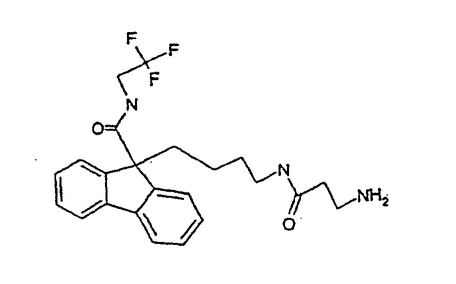 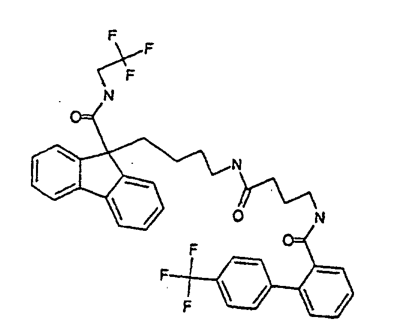 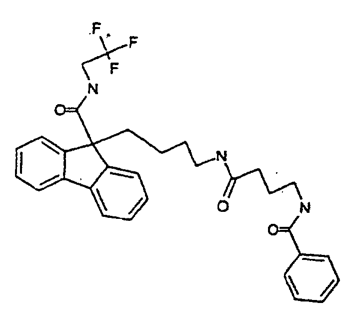 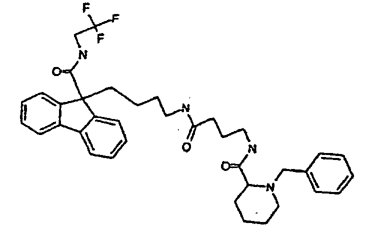 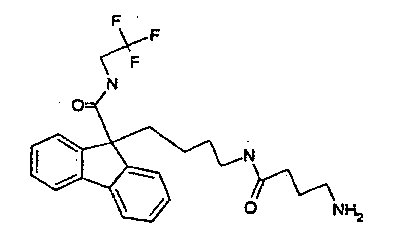 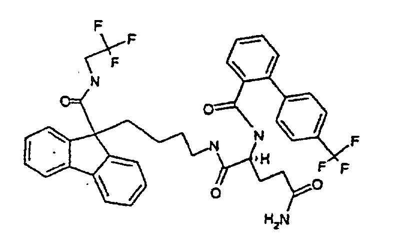 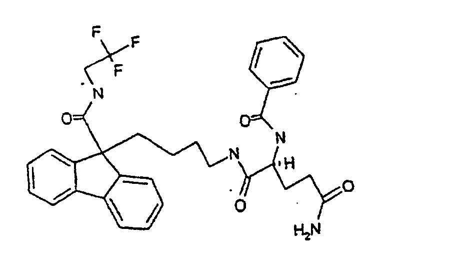 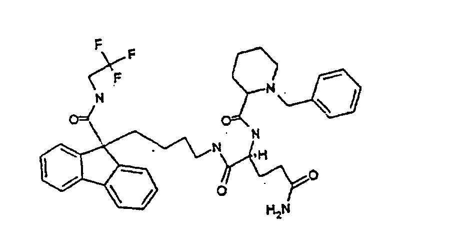 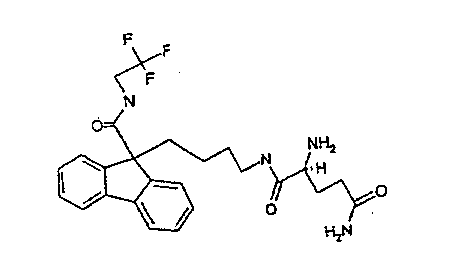 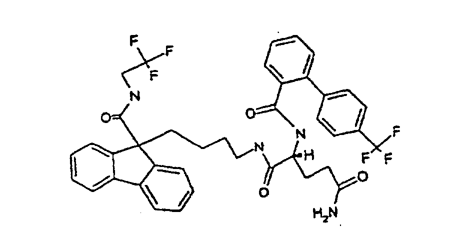 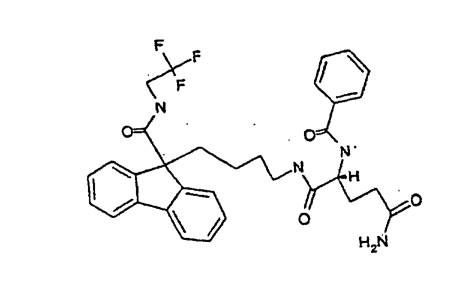 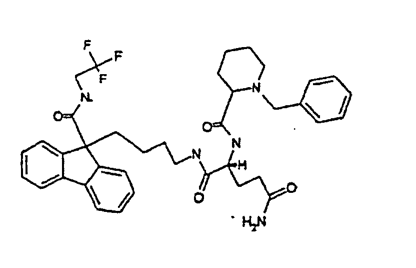 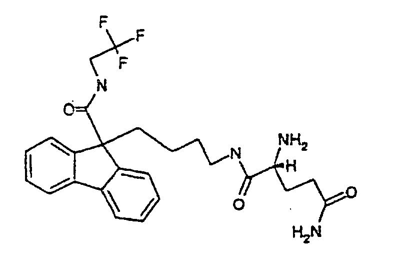 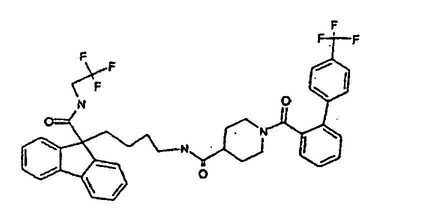 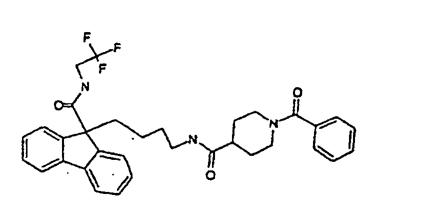 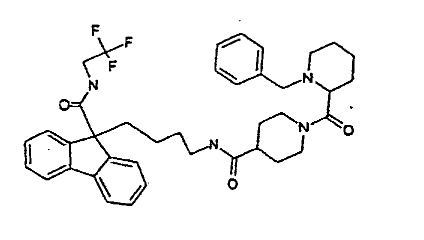 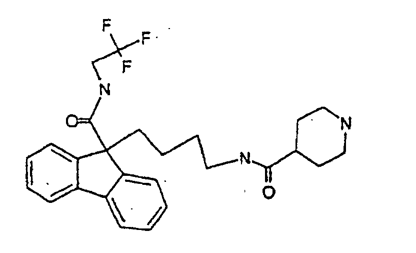 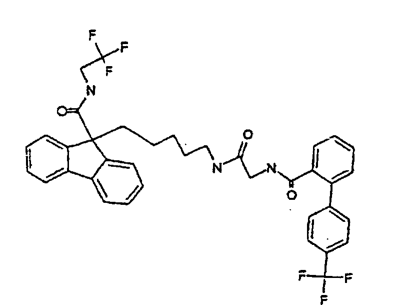 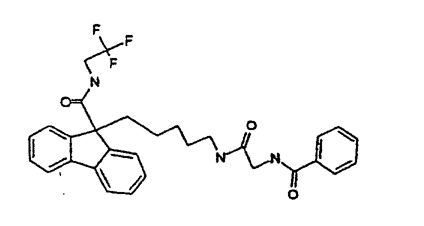 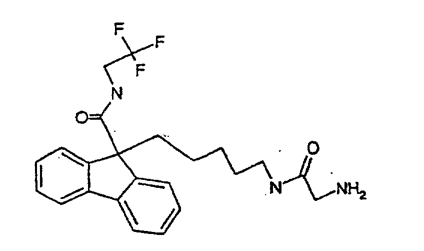 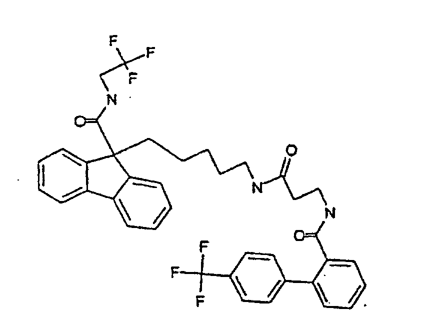 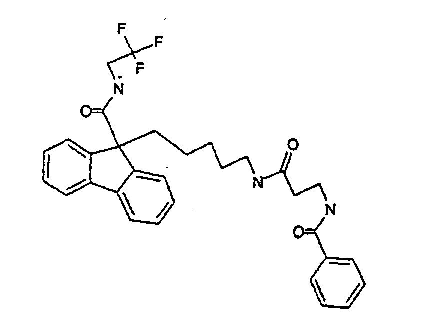 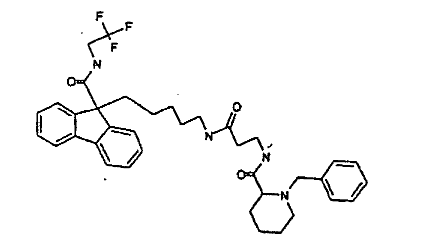 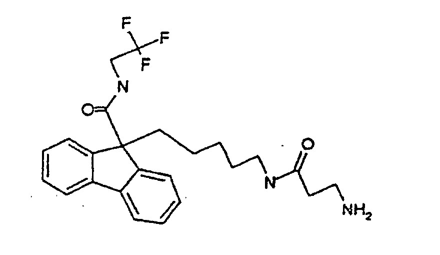 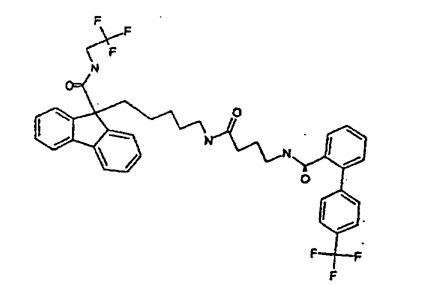 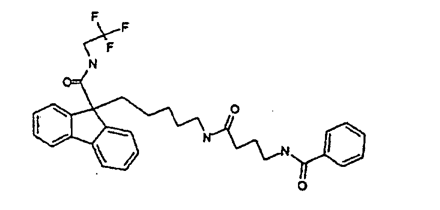 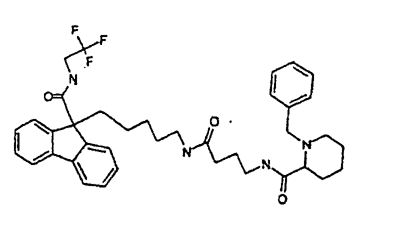 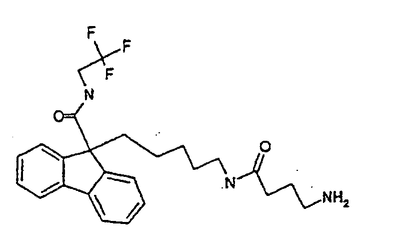 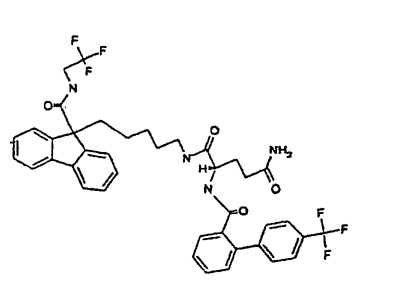 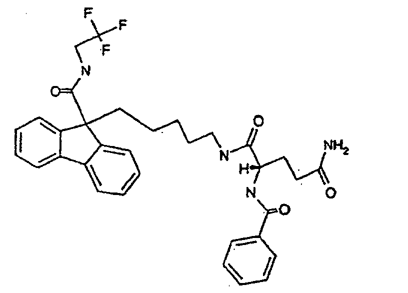 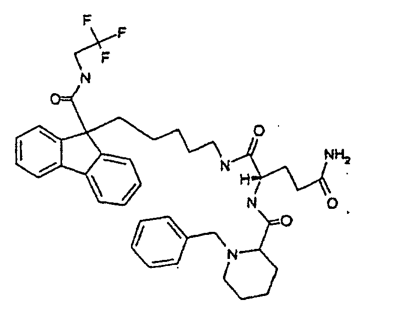 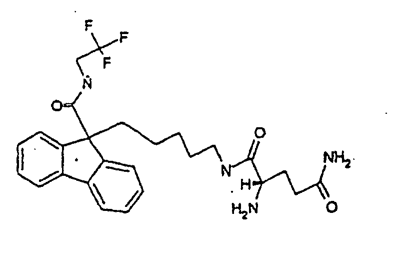 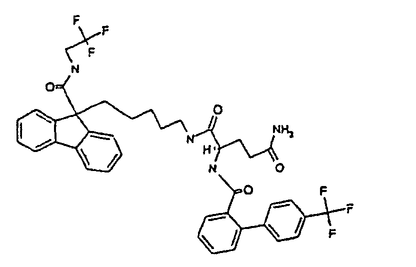 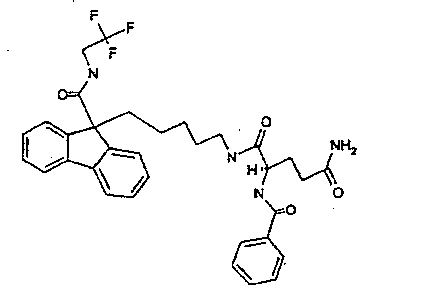 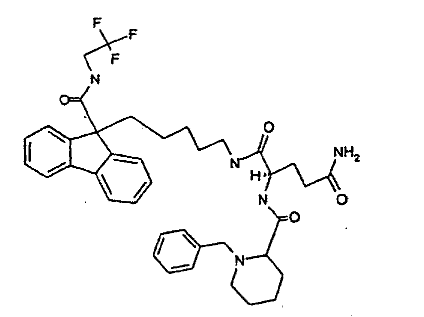 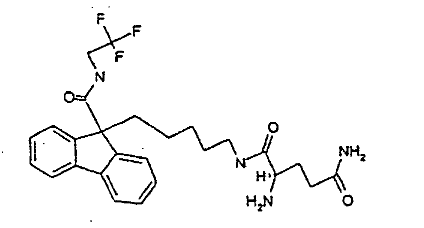 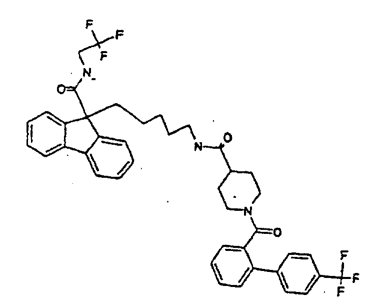 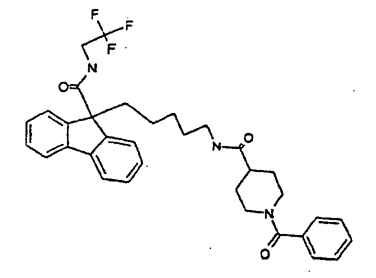 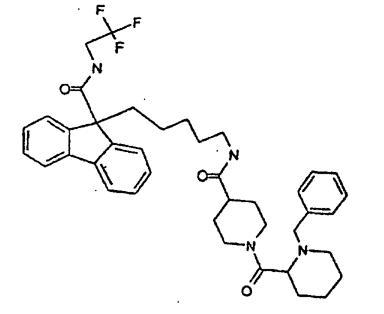 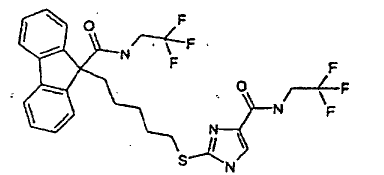 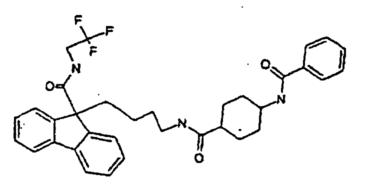 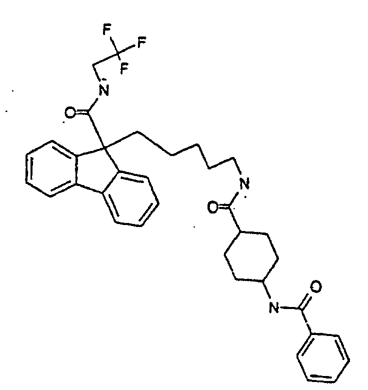 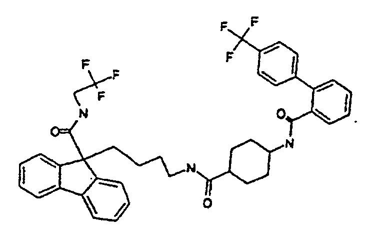 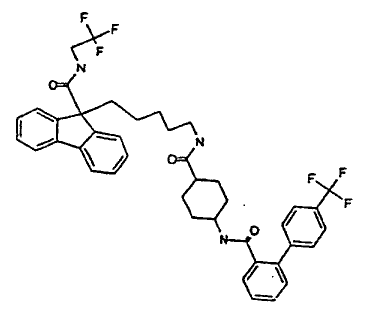 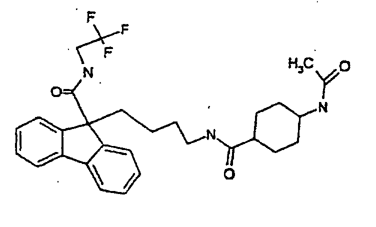 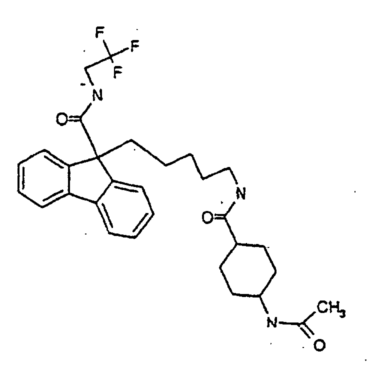 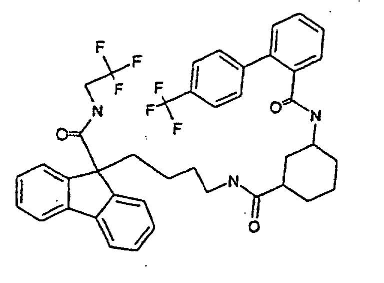 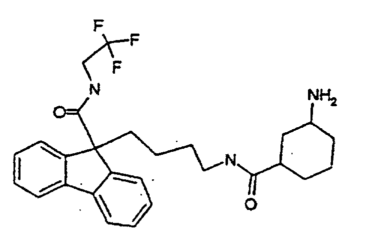 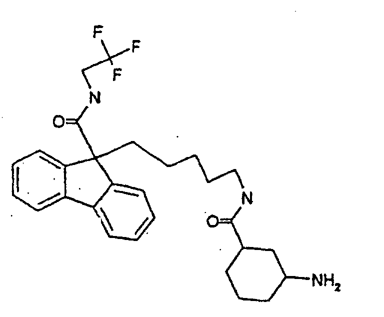 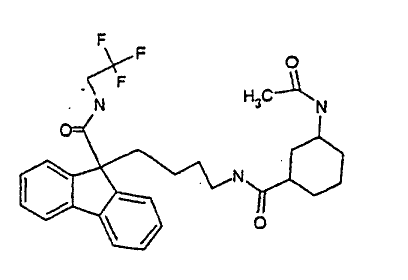 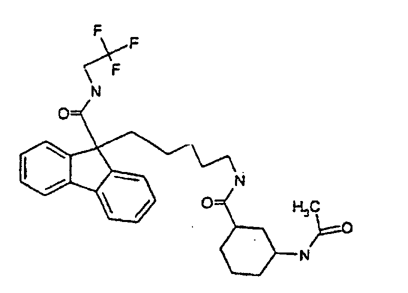 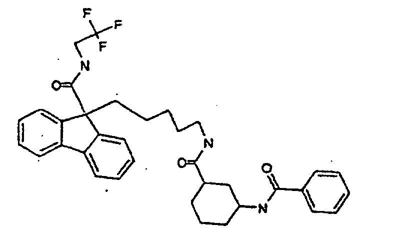 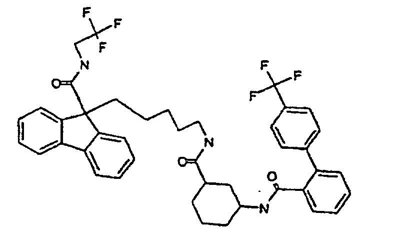 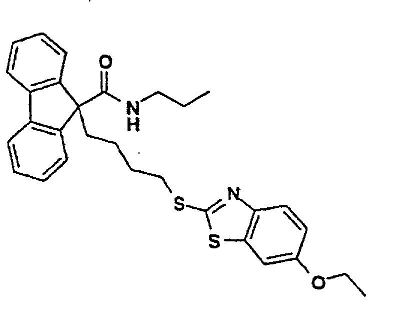 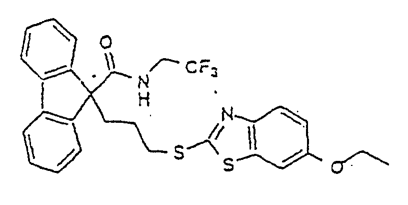 oder pharmazeutisch verträgliche Salze, Ester oder Prodrug-Ester davon.

4. Verbindung wie in Anspruch 2 definiert, wobei A eine NH- und R²L² eine CF₃CH₂-Gruppe ist.

5. Verbindung wie in Anspruch 1 definiert, mit der Formel 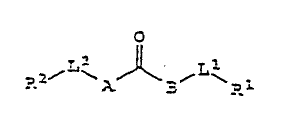 wobei B 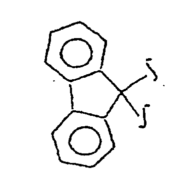 ist,
A eine NH- Gruppe ist,
L²R² eine CH₂CF₃-Gruppe ist,
L¹ eine -CH₂CH₂CH₂- oder eine -CH₂CH₂CH₂CH₂-Gruppe ist und R¹ ein Heteroarylrest ist, welcher ein 5-gliedriger aromatischer Ring ist, der 2 Stickstoffatome einschließt, wobei der Ring an einen Arylring anneliert ist und an der Aryleinheit substituiert ist.

6. Verbindung wie in Anspruch 1 definiert, wobei R¹ 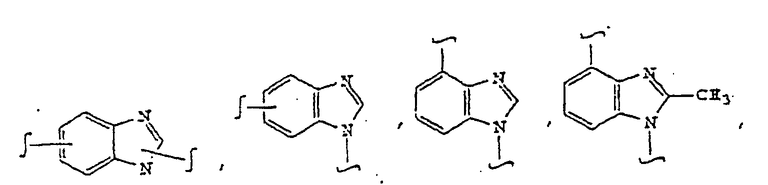 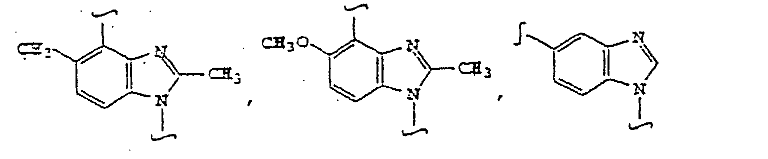 ist.

7. Verbindung wie in Anspruch 1 definiert, wobei R¹ 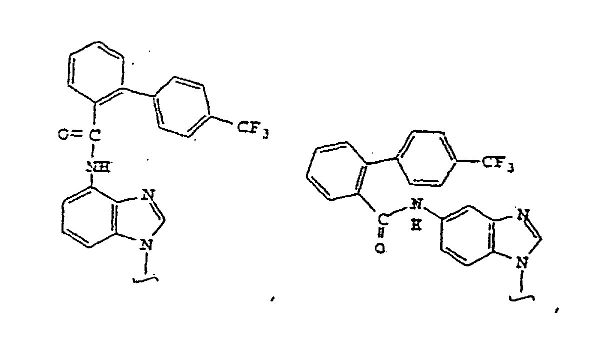 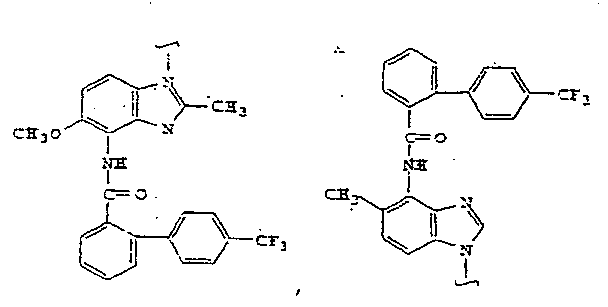 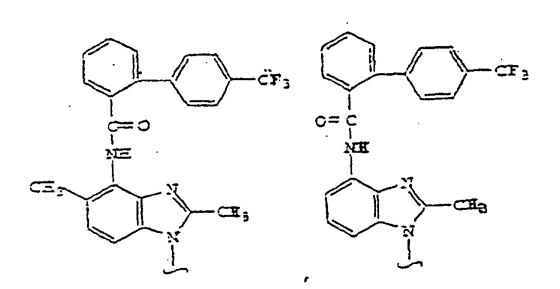 ist.

8. Verbindung gemäß Anspruch 5, welche die Strukur 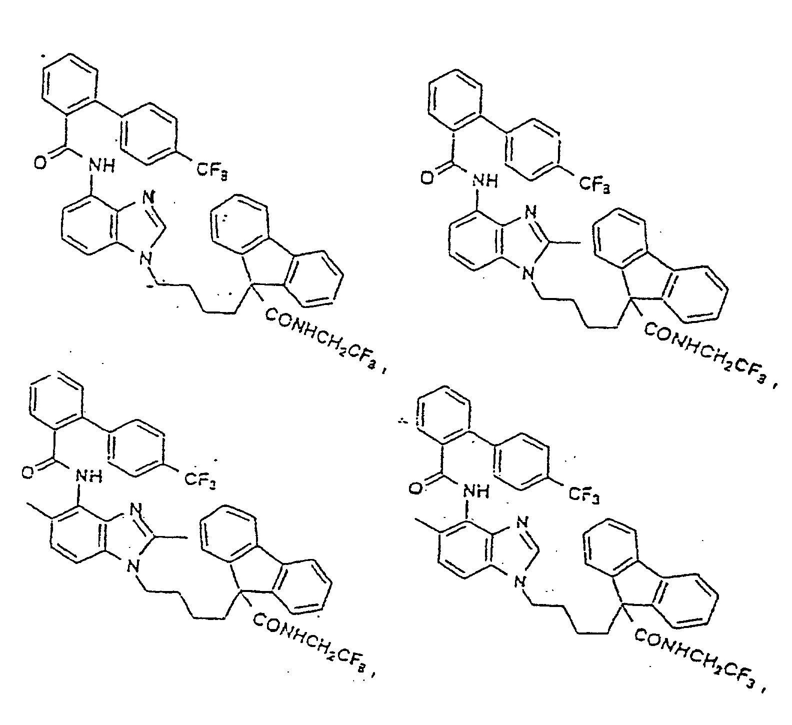 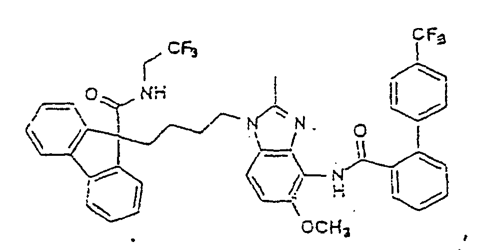 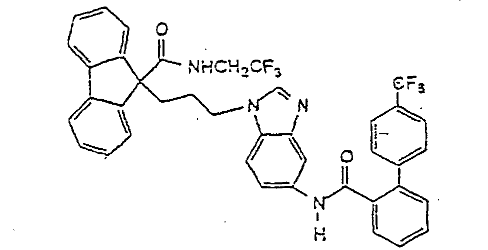 aufweist, oder ein pharmazeutisch verträgliches Salz davon.

9. Arzneimittel, umfassend eine Verbindung wie in einem der Ansprüche 1 bis 8 definiert und einen pharmazeutisch verträglichen Träger.

10. Verwendung einer Verbindung wie in einem der Ansprüche 1 bis 8 definiert für die Herstellung eines Arzneimittels zur Senkung der Aktivität eines mikrosomalen Triglyceridtransferproteins (MTP) in einem Säuger.

11. Verwendung einer Verbindung wie in einem der Ansprüche 1 bis 8 definiert für die Herstellung eines Arzneimittels zur Senkung des Serumlipid-, Cholesterin- und/oder Triglyceridspiegels oder zur Hemmung und/oder Behandlung von Hyperlipämie, Hyperlipidämie, Hyperlipoproteinämie, Hypercholesterinämie, Hyperglykämie und/oder Hypertriglyceridämie und/oder Verhinderung, Hemmung oder Behandlung von Atherosklerose, Pankreatitis, nicht insulinabhängigem Diabetes, oder Fettsucht in einem Säuger.

## Revendications

1. Composé ayant pour structure 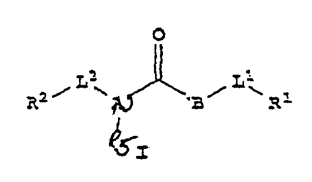 y compris ses sels pharmaceutiquement acceptables, ses N-oxydes,
dans laquelle q est 0, 1 ou 2 ;
où R⁵ est H ou un alkyle en C₁ à C₁₂, ou bien R⁵ forme avec R² un système cyclique carbocyclique ou hétérocyclique contenant 4 à 8 chaînons dans le cycle ;
B est un groupe de type fluorényle ayant pour structure 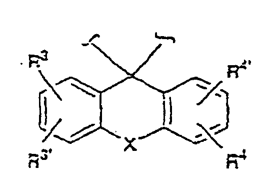 R¹ est H, un alkyle, alcényle, alcynyle, alcoxyl, (alkyl ou aryl)₃Si (où chaque groupe alkyle ou aryle est indépendant), cycloalkyle, cycloalcényle, alkylamino substitué, arylalkylamino substitué, aryle, arylalkyle, arylamino, aryloxy, hétéroaryle, hétéroarylamino, hétéroaryloxy, arylsulfonylamino, hétéroarylsulfonylamino, arylthio, arylsulfinyle, arylsulfonyle, alkylthio, alkylsulfinyle, alkylsulfonyle, cyclohétéroalkyle, hétéroarylthio, hétéroarylsulfinyle, hétéroarylsulfonyle, -PO(R¹³)(R¹⁴), (où R¹³ et R¹⁴ sont indépendamment un alkyle, aryle, alcoxy, aryloxy, hétéroaryle, hétéroarylalkyle, hétéroaryloxy, hétéroarylalcoxy, cyclohétéroalkyle, cyclohétéroalkylalkyle, cyclohétéroalcoxy ou cyclohétéroalkylalcoxy) ; aminocarbonyle (où le groupe amino peut éventuellement être substitué par un ou deux groupes aryle, alkyle ou hétéroaryle) ; cyano, 1,1-(alcoxy ou aryloxy)₃alkyle (où les deux substituants aryle ou alkyle peuvent être indépendamment définis ou liés l'un à l'autre pour former un cycle relié à L¹ (ou L² dans le cas de R²) en position 2) ; 1,3-dioxane ou 1,3-dioxolane relié à L¹ (ou L² dans le cas de R²) en position 4 ; le groupe R¹ peut éventuellement être substitué par 1, 2, 3 ou 4 substituants, qui peuvent être l'un quelconque des groupes R³ ou R¹ ou un alkylcarbonylamino, cycloalkylcarbonylamino, arylcarbonylamino, hétéroarylcarbonylamino, alcoxycarbonylamino, aryloxycarbonylamino, hétéroaryloxylcarbonylamino, uriédo (où les azotes uriédo peuvent éventuellement être substitués par un alkyle, aryle ou hétéroaryle), hétérocyclylcarbonylamino (où l'hétérocycle est relié au groupe carbonyle par l'intermédiaire d'un atome d'azote ou de carbone), alkylsulfonylamino, arylsulfonylamino, hétéroarylsulfonylamino, 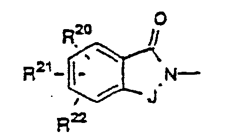 où J est : CHR²³, 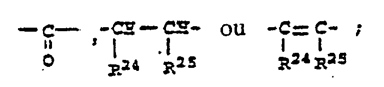 R²³, R²⁴ et R²⁵ sont indépendamment un hydrogène, alkyle, alcényle, alcynyle, aryle, arylalkyle, hétéroaryle, hétéroarylalkyle, cycloalkyle ou cycloalkylalkyle ;
R²⁰, R²¹, R²² sont indépendamment un hydrogène, halo, alkyle, alcényle, alcoxy, aryloxy, aryle, arylalkyle, alkylmercapto, arylmercapto, cycloalkyle, cycloalkylalkyle, hétéroaryle, hétéroarylalkyle, hydroxy ou haloalkyle ; et ces substituants peuvent être soit directement reliés à R¹, soit reliés par l'intermédiaire d'un alkylène en position ouverte ;
R² est indépendamment l'un quelconque des groupes cités pour R¹, H, polyhaloalkyle ou cyclohétéroalkyle, et peut éventuellement être substitué par un à quatre parmi les groupes définis pour R³ ou les substituants définis pour R¹ ;
L¹ est un groupe de liaison contenant jusqu'à 10 atomes de carbone dans une chaîne linéaire comprenant l'alkylène, l'alcénylène ou l'alcynylène, qui peuvent contenir, dans la chaîne de liaison, l'un des composés suivants : un ou deux alcènes; un ou deux alcynes, un oxygène, un groupe amino, un groupe oxo, et peut être substitué par un à cinq groupes alkyle ou halo ;
L² peut être identique ou différent de L¹ et peut indépendamment être l'un quelconque des groupes L¹ cités ci-dessus ou une liaison simple ;
R³, R^{3'}, R⁴ et R^{4'} peuvent être identiques ou différents et sont indépendamment choisis parmi H, un halogène, CF₃, un haloalkyle, hydroxy, alcoxy, alkyle, aryle, alcényle, alcényloxy, alcynyle, alcynyloxy, alcanoyl, nitro, amino, thiol, alkylthio, alkylsulfinyle, alkylsulfonyle, carboxy, alcoxycarbonyle, aminocarbonyle, alkylcarbonyloxy, alkylcarbonylamino, cyclohétéroalkyle, cyclohétéroalkylalkyle, cyano, Ar-, Ar-alkyle, ArO, Ar-amino, Ar-thio, Ar-sulfinyle, Ar-sulfonyle, Ar-carbonyle, Ar-carbonyloxy ou Ar-carbonylamino, où Ar est un aryle ou hétéroaryle, et Ar peut éventuellement comprendre 1, 2 ou 3 cycles supplémentaires fusionnés à Ar;
X est une liaison, ou est l'un des groupes suivants :
(2) -O- ;
avec les conditions suivantes
(a) quand R¹ est un alkyle non substitué ou un arylalkyle non substitué, L¹ ne peut pas contenir d'amino ;
(b) quand R¹ est un alkyle, L¹ ne peut pas contenir d'amino et d'oxo en positions adjacentes (pour former un groupe amido) ;
(d) quand R¹ est un cyano, L¹ doit avoir plus de 2 carbones ;
(e) R¹L¹ doit contenir au moins 3 carbones ;
en ce qui concerne les composés où R¹ ou R² est un cyclohétéroalkyle, R¹ ou R² exclut le 1-pipéridinyle, le 1-pyrrolidinyle, le 1-azétidinyle ou le 1-(2-oxo-pyrrolidinyle), où les termes "alkyle" ou "alk" , tels qu'ils sont utilisés, seuls ou comme partie d'un autre groupe, comprennent à la fois les hydrocarbures à chaîne linéaire et ramifiée, contenant 1 à 40 carbones, leurs divers isomères à chaîne ramifiée, ainsi que les groupes comprenant 1 à 4 substituants qui peuvent être l'un quelconque des groupes R³, ou les substituants de R¹ cités ici ;
le terme "cycloalkyle", tel qu'il est utilisé ici, seul ou comme partie d'un autre groupe, comprend les groupes hydrocarbure cyclique saturés ou partiellement insaturés (contenant 1 ou 2 doubles liaisons) contenant 1 à 3 cycles, y compris les alkyles monocycliques, les alkyles bicycliques et les alkyles tricycliques, contenant un total de 3 à 20 carbones formant les cycles, de préférence de 4 à 12 carbones formant le cycle, et qui peuvent être fusionnés pour donner 1 cycle aromatique comme décrit pour l'aryle,
le terme "cycloalcényle", tel qu'il est utilisé ici présentes, seul ou comme partie d'un autre groupe, désigne les hydrocarbures cycliques contenant 5 à 20 carbones, et 1 ou 2 doubles liaisons ;
le terme "polycycloalkyle", tel qu'il est utilisé ici, seul ou comme partie d'un autre groupe, désigne un groupe multicyclique ponté contenant 5 à 20 carbones et contenant 0 à 3 ponts ;
le terme "polycycloalcényle", tel qu'il est utilisé ici, seul ou comme partie d'un autre groupe, désigne un groupe multicyclique ponté contenant 5 à 20 carbones et contenant 0 à 3 ponts et contenant 1 ou 2 doubles liaisons ;
le terme "aryle", tel qu'il est utilisé ici, seul ou comme partie d'un autre groupe, désigne des groupes aromatiques monocycliques et bicycliques contenant 6 à 10 carbones dans la partie de cycle et peut éventuellement comprendre un à trois cycles supplémentaires fusionnés à Ar, et peut éventuellement être substitué à travers des atomes de carbone disponibles par 1, 2 ou 3 groupes choisis parmi les groupes hydrogène, halo, haloalkyle, alkyle, haloalkyle, alcoxy, haloalcoxy, alcényle, trifluorométhyle, trifluorométhoxy, alcynyle, cyclo-alkylalkyle, cyclohétéroalkyle, cyclohétéroalkylalkyle, aryle, hétéroaryle, arylalkyle, aryloxy, aryloxyalkyle, arylalcoxy, arylthio, arylazo, hétéroarylalkyle, hétéroarylalcényle, hétéroarylhétéroaryle, hétéroaryloxy, hydroxy, nitro, cyano, amino, amino substitué, où l'amino comprend 1 ou 2 substituants (qui sont un alkyle, un aryle ou l'un quelconque des autres composés aryle cités dans les définitions), thiol, alkylthio, arylthio, hétéroarylthio, arylthioalkyle, alcoxyarylthio, alkylcarbonyle, arylcarbonyle, alkylaminocarbonyle, arylaminocarbonyle, alcoxycarbonyle, aminocarbonyle, alkylcarbonyloxy, arylcarbonyloxy, alkylcarbonylamino, arylcarbonylamino, arylsulfinyle, arylsulfinylalkyle, arylsulfonylamino ou arylsulfonaminocarbonyle ou l'un quelconque des groupes R³, ou les substituants de R¹ cités ici ;
les termes "aralkyle", "arylalkyle" ou "alkyle aryl inférieur", tels qu'ils sont utilisés ici, seuls ou comme partie d'un autre groupe, désignent des groupes alkyle décrits ci-dessus ayant un substituant aryle ;
les termes "alcoxy inférieur", "alcoxy", "aryloxy" ou "aralcoxy", tels qu'ils sont utilisés ici, seuls ou comme partie d'un autre groupe, comprennent l'un quelconque des groupes alkyle, aralkyle ou aryle ci-dessus lié à un atome d'oxygène ;
le terme "amino", tel qu'il est utilisé ici, seul ou comme partie d'un autre groupe, peut éventuellement être substitué par un ou deux substituants tels qu'un alkyle, aryle, arylalkyle, hétéroaryle, hétéroarylalkyle, cyclohétéroalkyle, cyclohétéroalkylalkyle et/ou cycloalkyle ;
les termes "alkylthio inférieur", "alkylthio", "arylthio" ou "aralkylthio", tels qu'ils sont utilisés ici, seuls ou comme partie d'un autre groupe, comprennent l'un quelconque des groupes alkyle, aralkyle ou aryle ci-dessus lié à un atome de soufre ;
les termes "alkylamino inférieur", "alkylamino", "arylamino" ou "arylalkylamino", tels qu'ils sont utilisés ici, seuls ou comme partie d'un autre groupe, comprennent l'un quelconque des groupes alkyle, aryle ou arylalkyle ci-dessus lié à un atome d'azote ;
le terme "acyle", tel qu'il est utilisé ici, seul ou comme partie d'un autre groupe, comme défini ici. désigne un radical organique lié à un groupe carbonyle 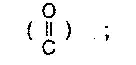 le terme "alcanoyl", tel qu'il est utilisé ici, seul ou comme partie d'un autre groupe, désigne un alkyle lié à un groupe carbonyle,
le terme "alcényle", tel qu'il est utilisé ici, seul ou comme partie d'un autre groupe, désigne des radicaux à chaîne linéaire ou ramifiée de 2 à 20 carbones, qui comprennent une à six doubles liaisons dans la chaîne principale, et qui peuvent éventuellement être substitués par 1 à 4 substituants, à savoir un halogène, haloalkyle, alkyle, alcoxy, alcényle, alcynyle, aryle, arylalkyle, cycloalkyle, amino, hydroxy, hétéroaryle, cyclohétéroalkyle, alcanoylamino, alkylamido, arylcarbonylamino, nitro, cyano, thiol, alkylthio ou l'un quelconque des groupes R³, ou les substituants R¹ cités ici ;
le terme "alcynyle", tel qu'il est utilisé ici, seul ou comme partie d'un autre groupe, désigne des radicaux à chaîne linéaire ou ramifiée de 2 à 20 carbones, qui comprennent une triple liaison dans la chaîne principale, et qui peuvent éventuellement être substitués par 1 à 4 substituants, à savoir un halogène, haloalkyle, alkyle, alcoxy, alcényle, alcynyle, aryle, arylalkyle, cycloalkyle, amino, hétéroaryle, cyclohétéroalkyle, hydroxy, alcanoylamino, alkylamido, arylcarbonylamino, nitro, cyano, thiol et/ou alkylthio, ou l'un quelconque des groupes R³, ou les substituants R¹ cités ici;
le terme "alkylène", tel qu'il est utilisé ici, seul ou comme partie d'un autre groupe, désigne des groupes alkyle tels que définis ci-dessus, ayant des liaisons uniques pour la fixation à d'autres groupes sur deux atomes de carbone différents et peut éventuellement être substitué comme un « alkyle» tel que défini ci-dessus;
les termes "alcénylène" et "alcynylène", tels qu'ils sont utilisés ici, seuls ou comme partie d'un autre groupe, désignent respectivement des groupes alcényle tels que définis ci-dessus et des groupes alcynyle tels que définis ci-dessus, ayant des liaisons simples pour la fixation sur deux atomes de carbone différents, et peuvent éventuellement comprendre 1, 2 ou 3 substituants qui comprennent l'un des groupes R³, ou les substituants R¹ cités ici;
les termes "halogène" ou "halo", tels qu'ils sont utilisés ici, seuls ou comme partie d'un autre groupe, désignent le chlore, le brome, le fluor et l'iode, ainsi que CF₃ ;
le terme "ion métal" désigne des ions de métaux alcalins, des ions de métaux alcalinoterreux ainsi que le zinc et l'aluminium ;
le terme "cyclohétéroalkyle", tel qu'il est utilisé ici, seul ou comme partie d'un autre groupe, désigne un cycle à 5, 6 ou 7 éléments, saturé ou partiellement insaturé, qui comprend 1 ou 2 atomes hétéro liés par l'intermédiaire d'un atome de carbone ou d'un hétéroatome, si possible, éventuellement par l'intermédiaire de l'agent de liaison (CH₂)ₚ (qui est défini ci-dessus) ;
le terme "hétéroaryle", tel qu'il est utilisé ici, seul ou comme partie d'un autre groupe, désigne un cycle aromatique à 5 ou 6 éléments qui comprend 1, 2, 3 ou 4 hétéroatomes fusionnés à un cycle aryle, cycloalkyle, hétéroaryle ou cyclohétéroalkyle, et comprend des N-oxydes éventuels ;
Ar peut être soit un aryle soit un hétéroaryle, comme défini ci-dessus ;
le terme "cyclohétéroalkylalkyle", tel qu'il est utilisé ici, seul ou comme partie d'un autre groupe, désigne des groupes cyclohétéroalkyle comme définis ci-dessus, liés par l'intermédiaire d'un atome de C ou d'un hétéroatome à une chaîne de (CH₂)ₚ ;
les termes "hétéroarylalkyle" ou "hétéroarylalcényle", tel qu'ils sont utilisés ici, seuls ou comme partie d'un autre groupe, désignent un groupe hétéroaryle comme défini ci-dessus, lié par l'intermédiaire d'un atome de C ou d'un hétéroatome à une chaîne -(CH₂)ₚ-, à un alkylène ou un alcénylène, comme défini ci-dessus, et dans lequel
le terme "polyhaloalkyle", tel qu'il est utilisé ici, désigne un groupe "alkyle" comme défini ci-dessus, qui comprend de 2 à 9 substituants halo.

2. Composé selon la revendication 1 ayant pour formule 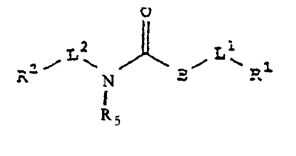 dans laquelle B est 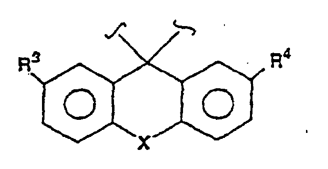 A est NH ;
X est une liaison, un oxygène ou un soufre ;
R³ et R⁴ sont identiques ou différents, et sont H ou F ;
R¹ est un aryle, phényle, hétéroaryle, imidazolyle, pyridyle, cyclohexyle, PO(R¹³)(R¹⁴), hétéroarylthio, benzimidazolyle, indolyle, benzothiazole-2-thio, imidazole-2-thio, alkyle, alcényle ou 1,3-dioxane-2-yle ;
R² est un alkyle, polyfluoroalkyle, alcényle, aryle, phényle, hétéroaryle, imidazolyle ou pyridyle ;
L¹ est une chaîne contenant 1 à 5 atomes dans une chaîne linéaire ;
L² est une liaison ou un alkylène contenant jusqu'à 12 atomes de carbone.

3. Composé selon la revendication 1 qui est le N-(phénylméthyl)-9-(3-phénylpropyl)-9H-fluorène-9-carboxamide ; 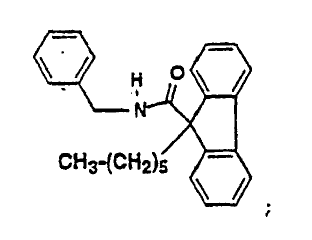 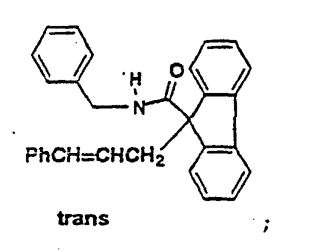 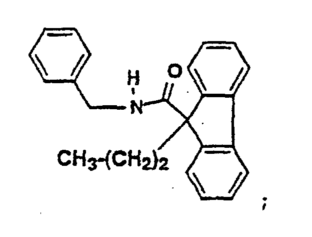 le (E)-N-éthyl-9-(3-phényl-2-propényl)-9H-fluorène-9-carboxamide ; 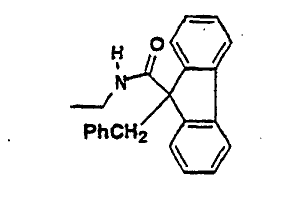 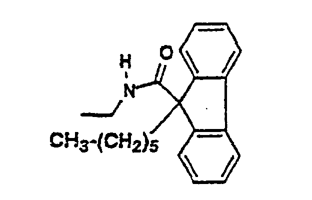 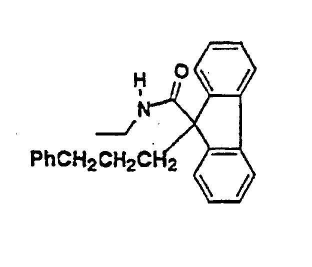 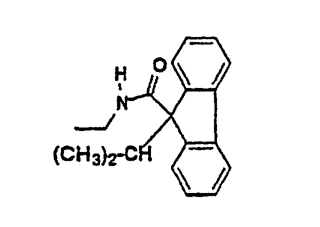 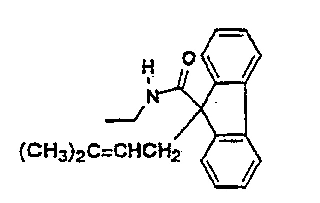 le 9-[4-dibutoxyphosphinyl)butyl]-N-propyl-9H-fluorène-9-carboxamide ;
le (E)-9-(3-phényl-2-propényl)-N-propyl-9H-fluorène-9-carboxamide ; 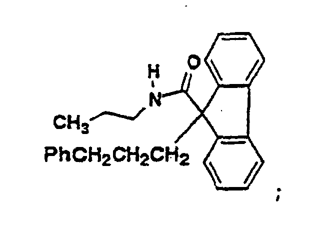 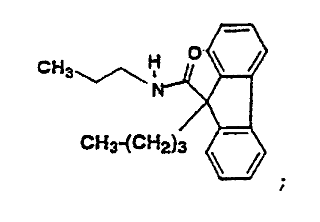 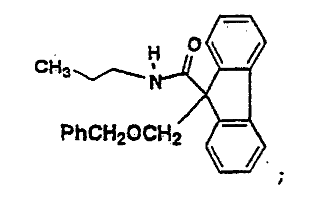 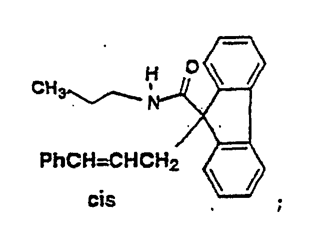 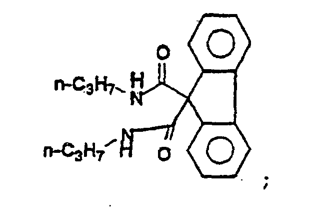 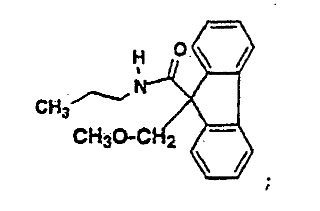 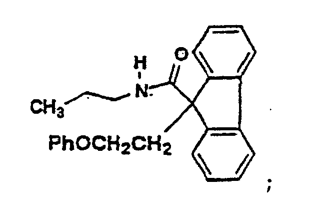 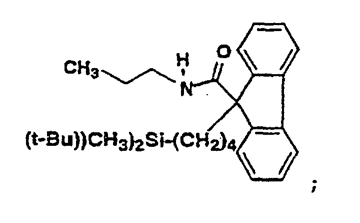 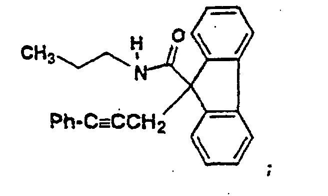 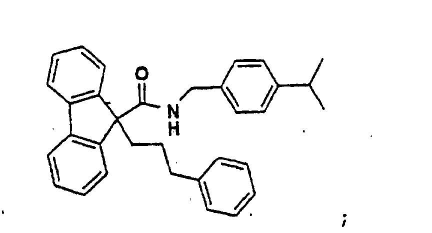 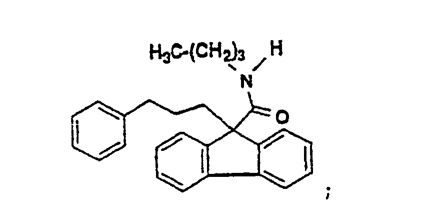 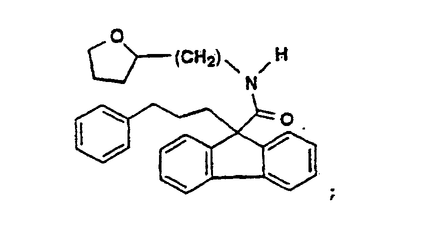 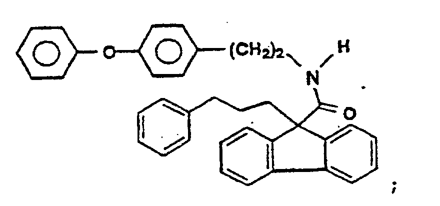 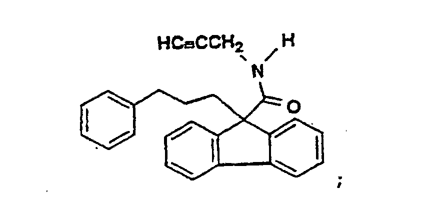 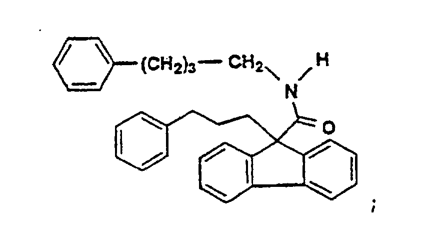 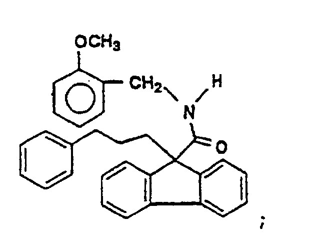 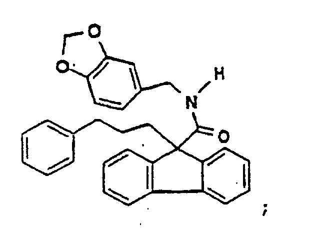 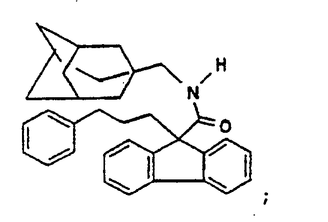 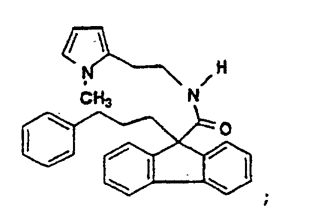 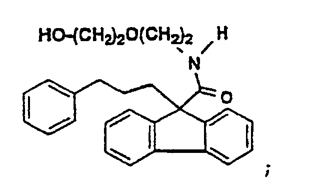 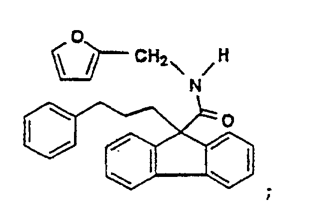 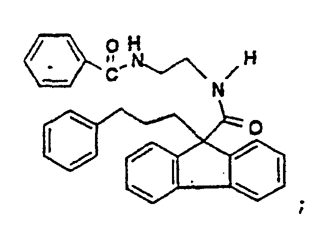 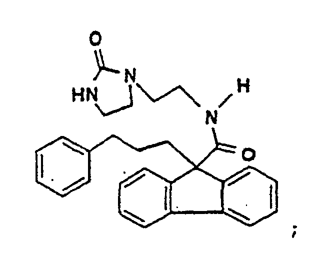 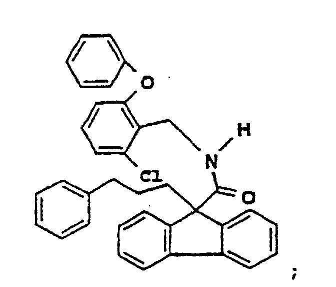 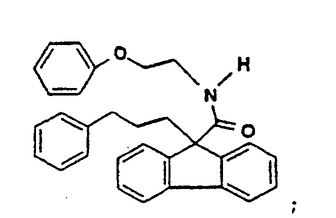 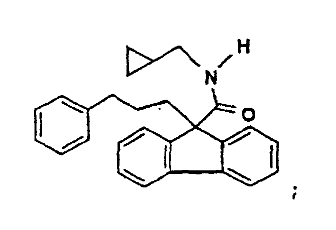 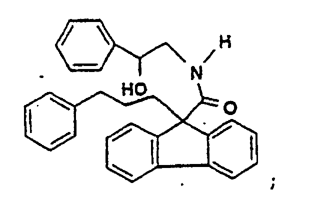 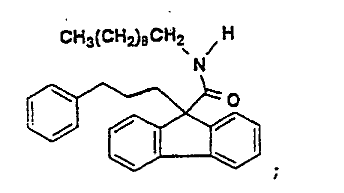 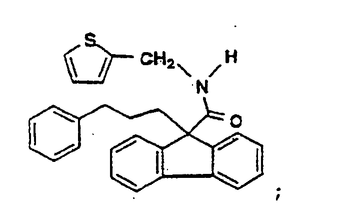 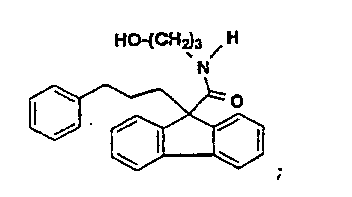 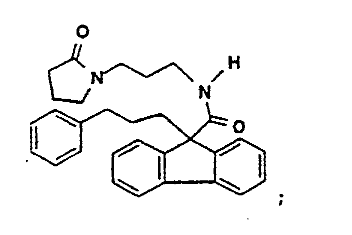 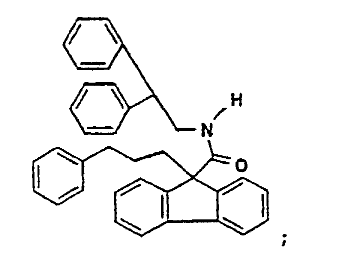 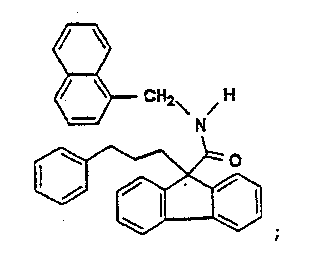 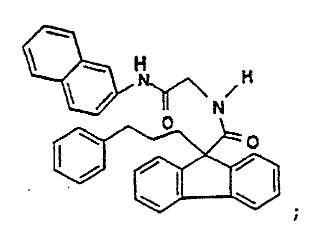 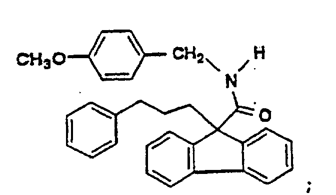 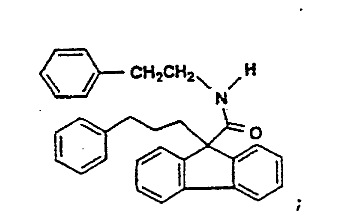 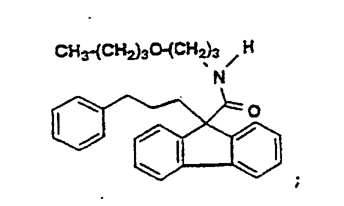 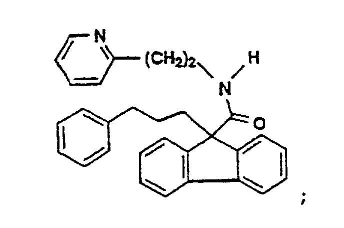 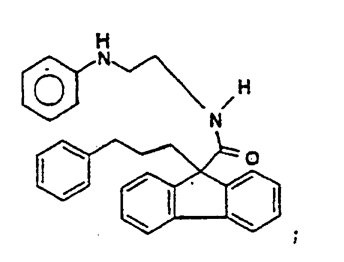 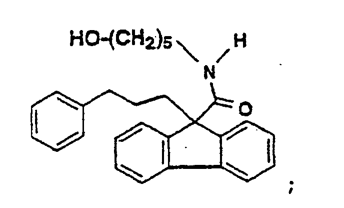 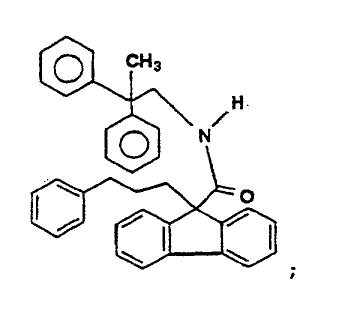 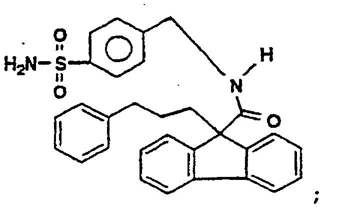 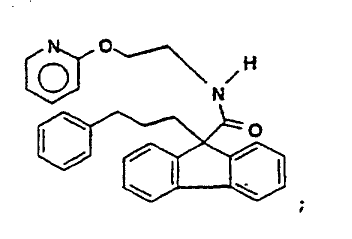 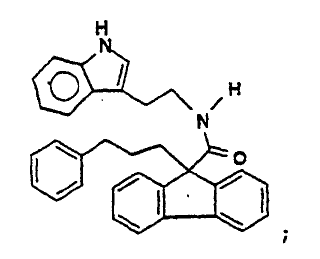 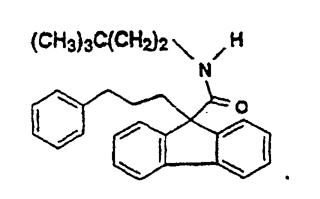 le 9-(3-phénylpropyl)-N-(2,2,2-trifluoroéthyl)-9H-fluorène-9-carboxamide ; 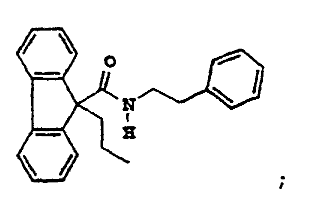 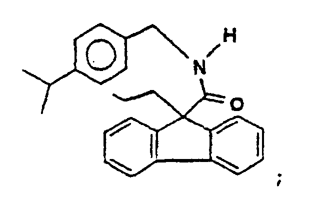 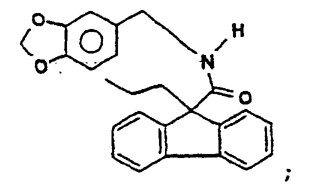 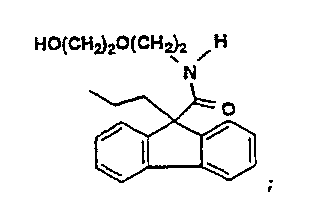 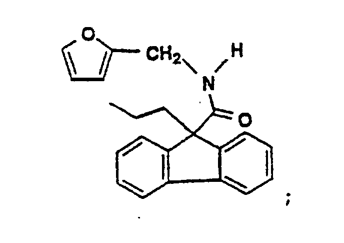 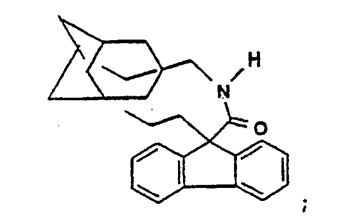 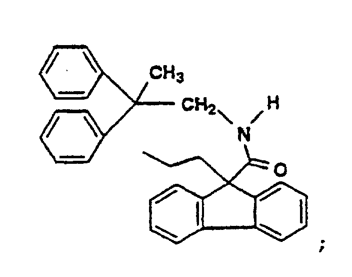 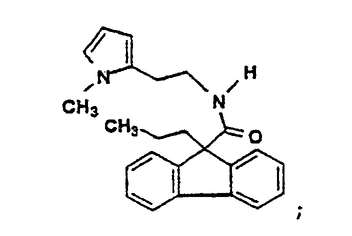 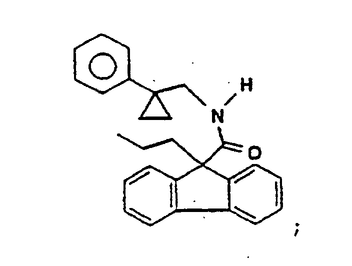 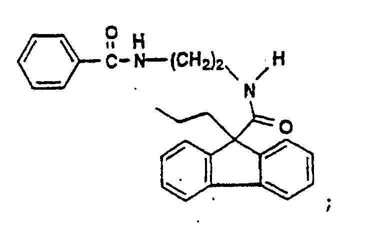 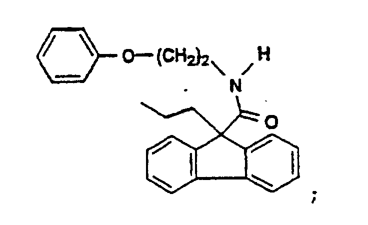 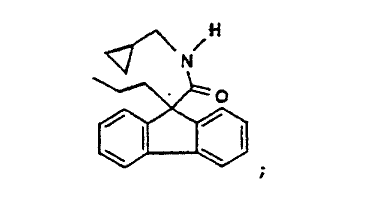 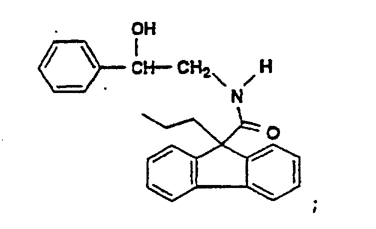 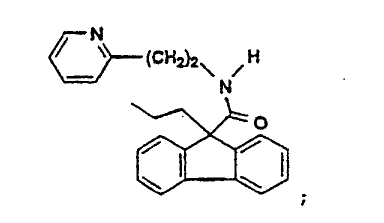 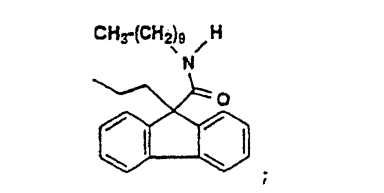 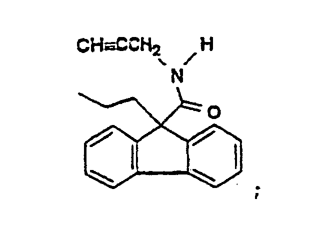 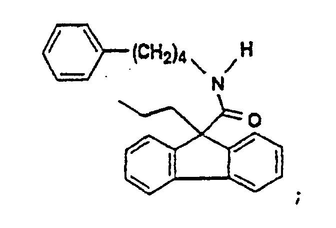 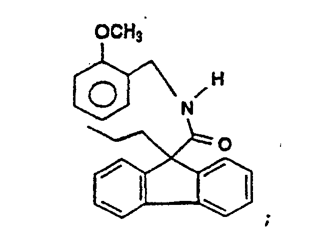 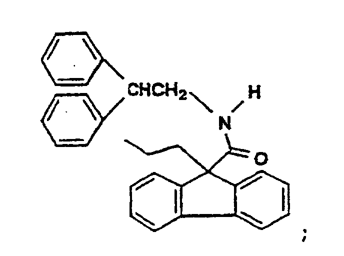 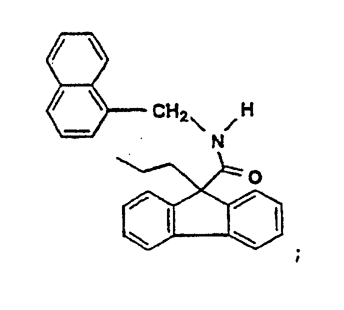 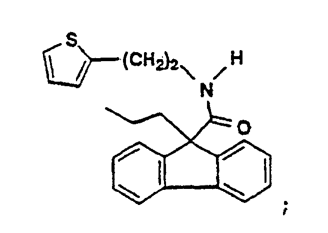 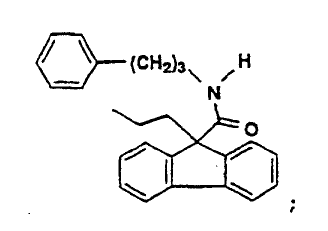 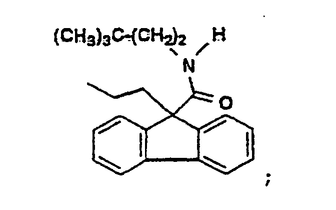 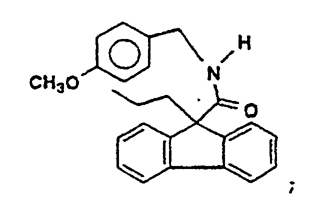 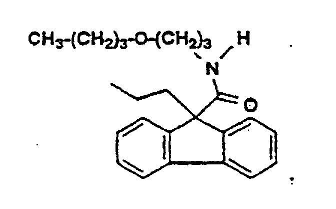 le N-méthyl-N-(phénylméthyl)-9-propyl-9H-fluorène-9-carboxamide ; 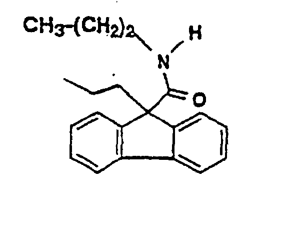 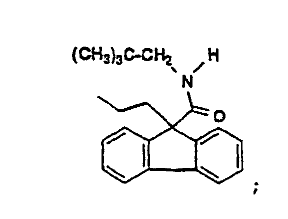 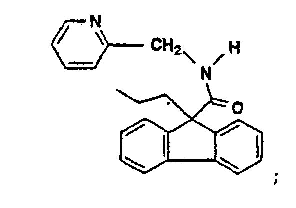 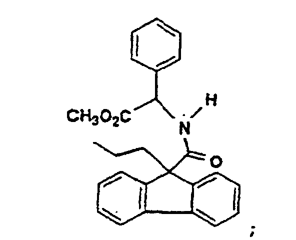 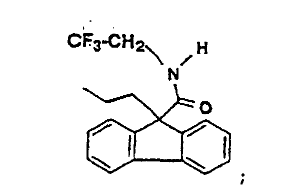 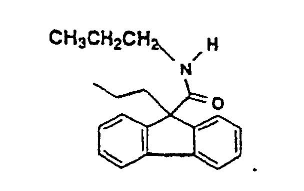 le 9-(2-propényl)-N-(2-pyridinylméthyl)-9H-fluorène-9-carboxamide ;
le N-butyl-9-(2-propényl)-9H-fluorène-9-carboxamide ;
le 9-[2,2-bis(triflourométhyl)-1,3-dioxolan-4-yl]méthyl-N-éthyl-9H-fluorène-9-carboxamide ;
le 9-(2,3-dihydroxypropyl)-N-éthyl-9H-fluorène-9-carboxamide ;
le 9-(3-phénylpropyl)-N-(3-hydroxy)propyl-9H-xanthène-9-carboxamide ; 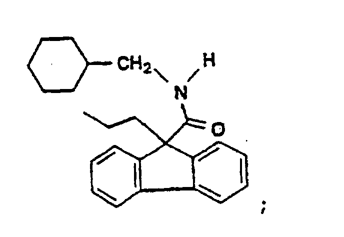 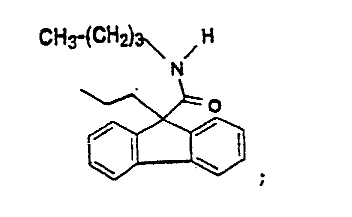 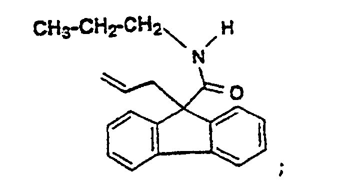 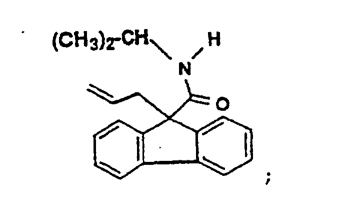 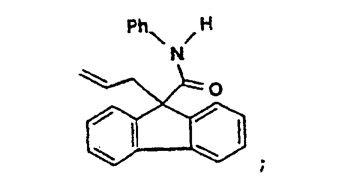 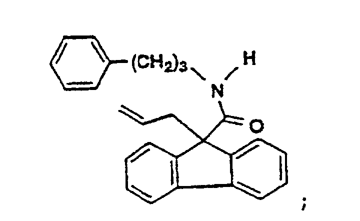 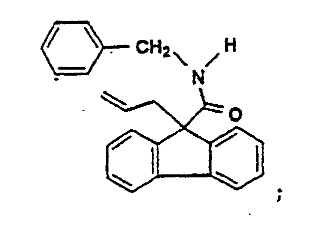 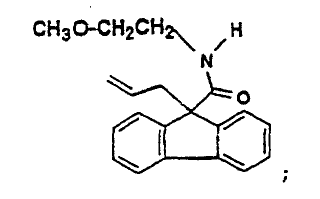 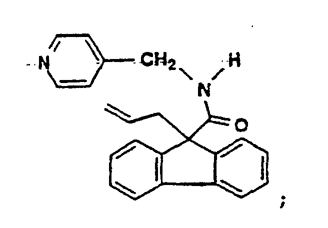 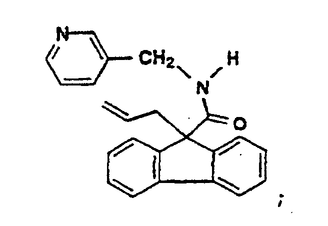 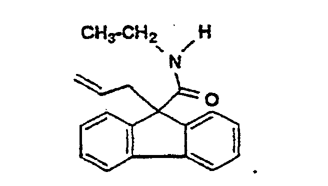 le 9-(1-pipéridinylcarbonyl)-9-(2-propényl)-9H-fluorène ; 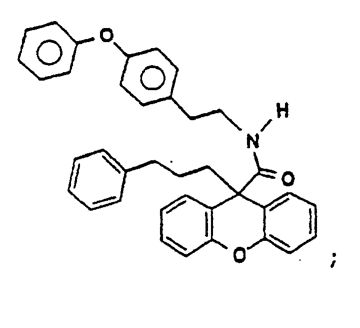 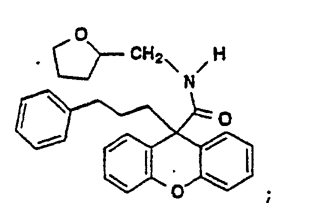 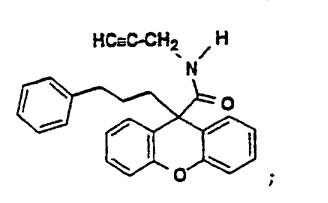 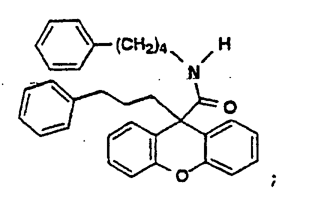 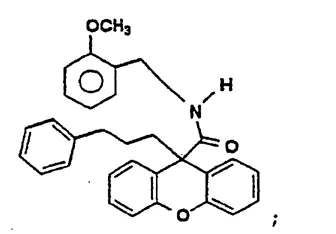 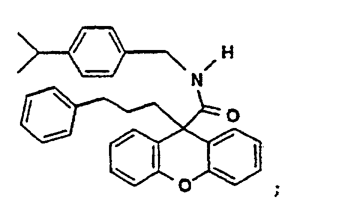 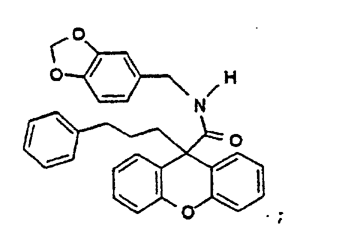 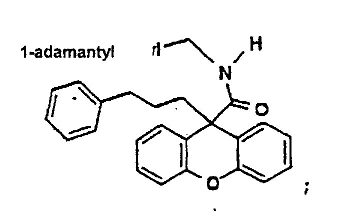 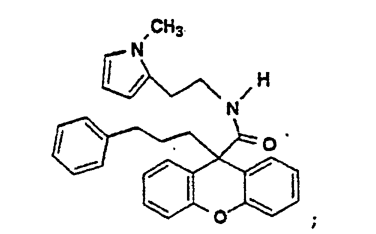 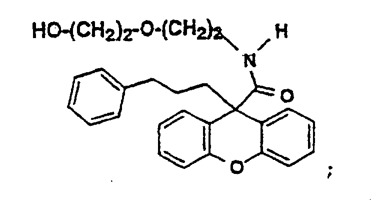 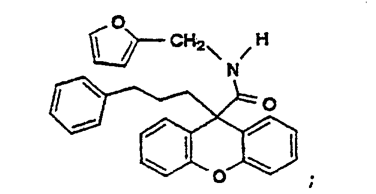 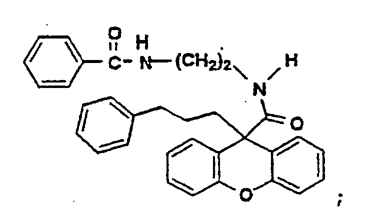 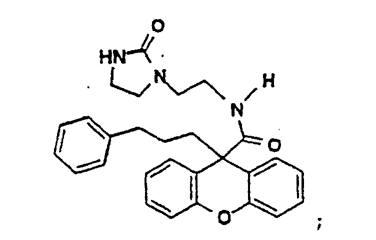 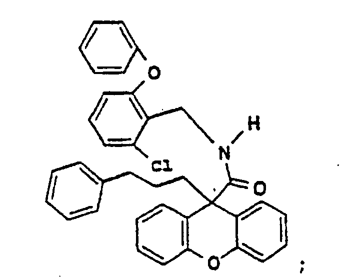 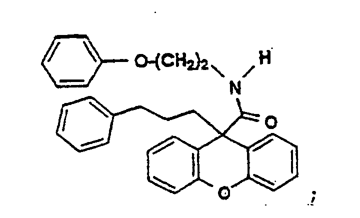 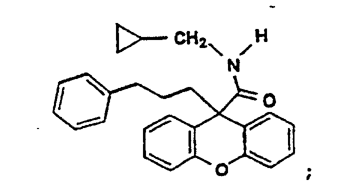 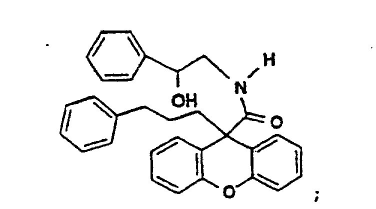 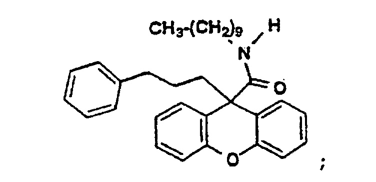 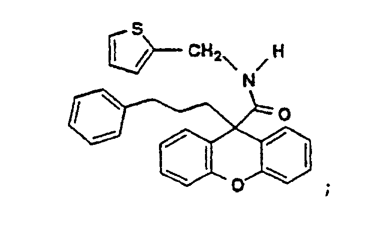 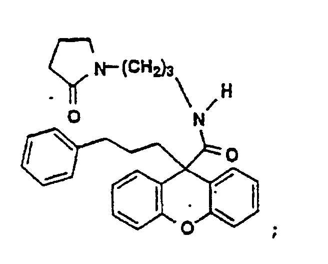 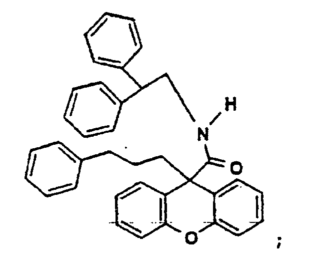 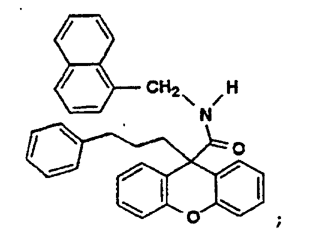 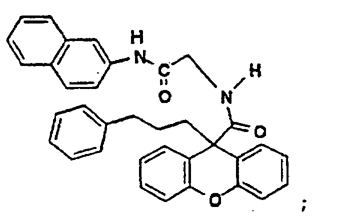 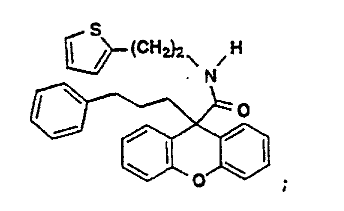 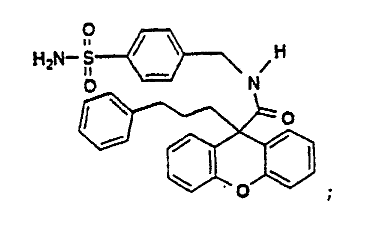 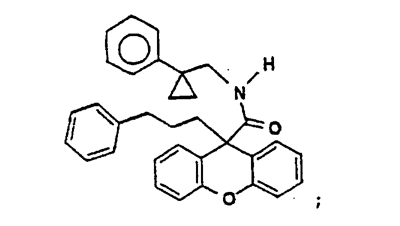 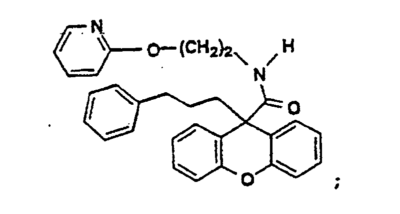 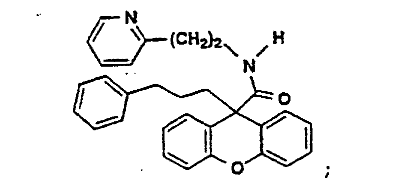 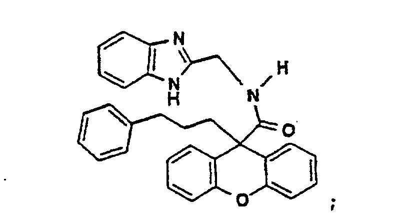 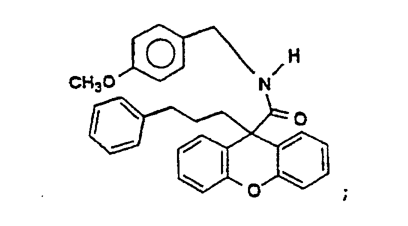 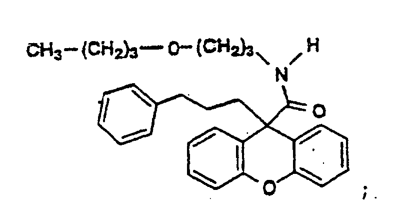 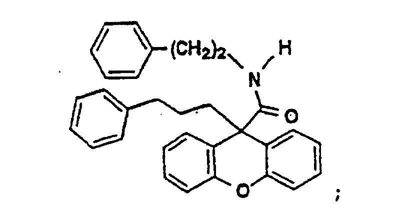 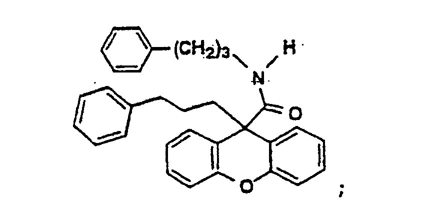 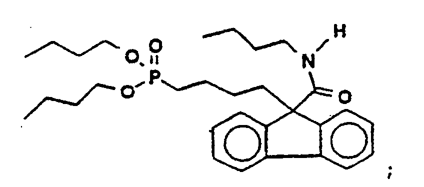 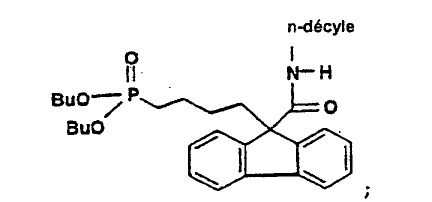 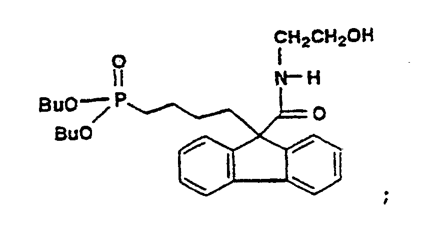 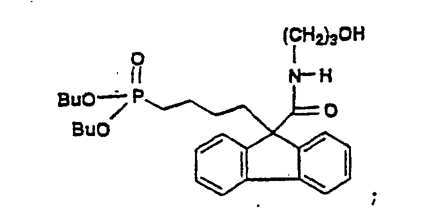 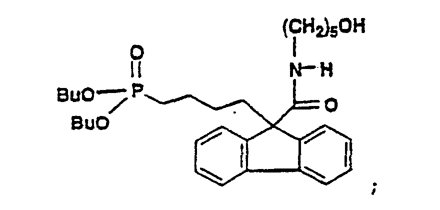 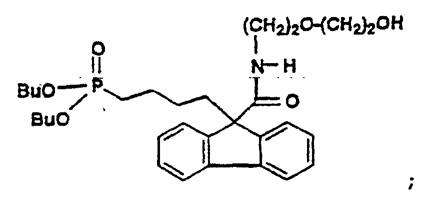 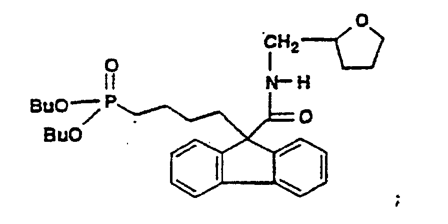 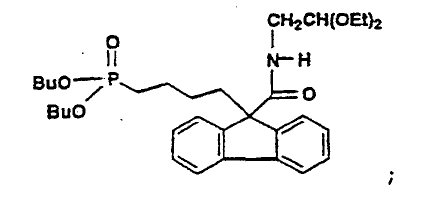 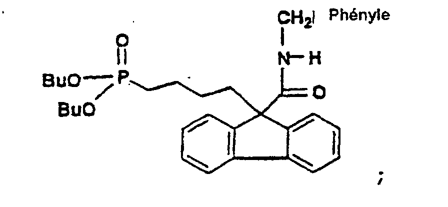 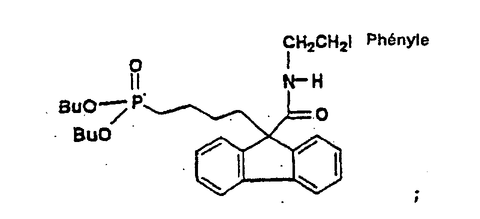 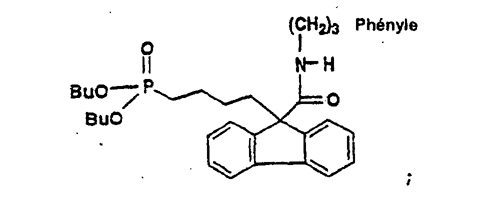 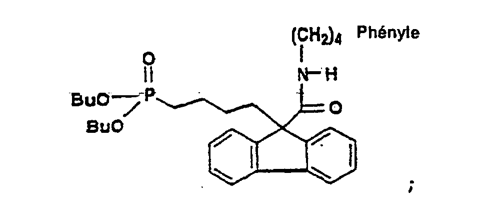 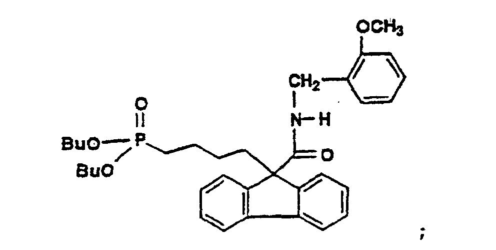 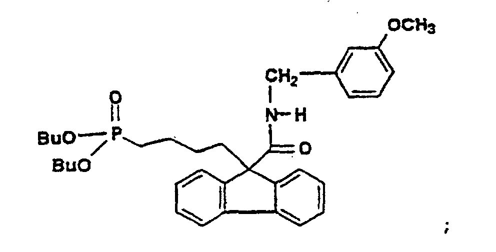 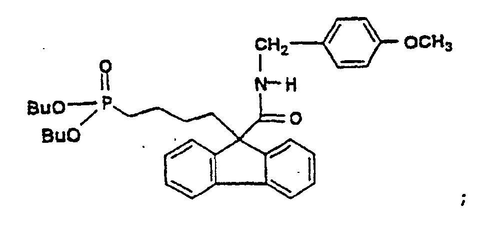 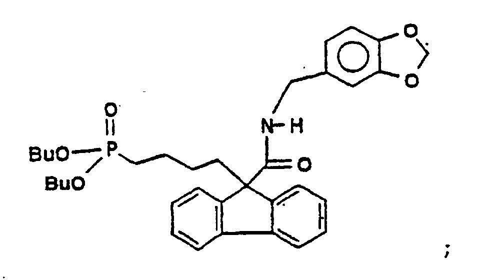 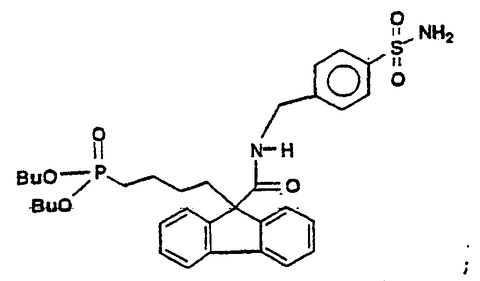 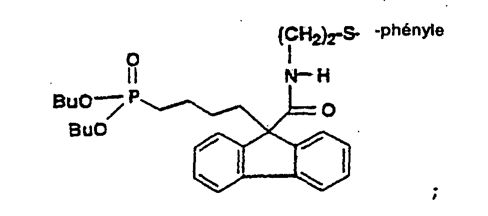 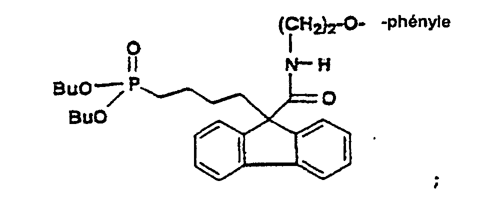 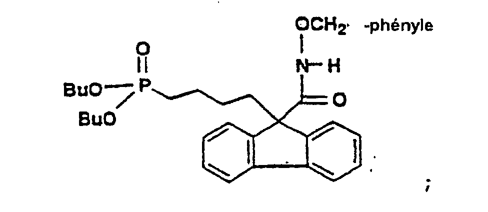 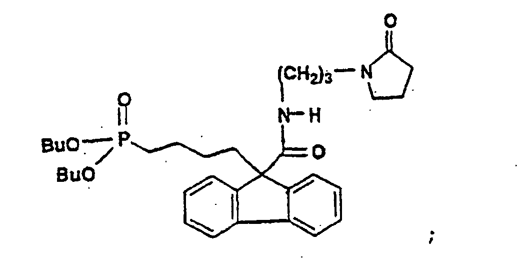 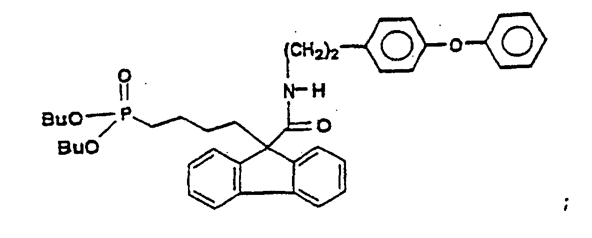 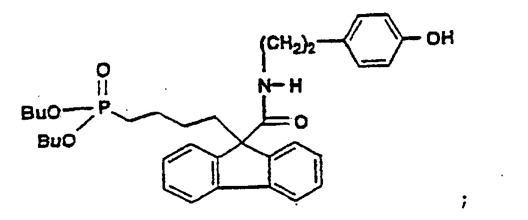 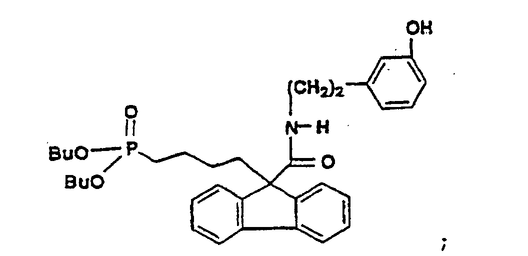 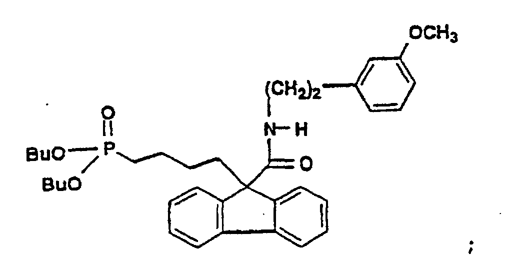 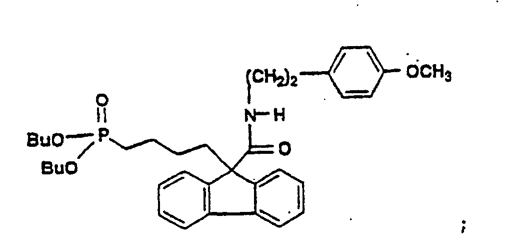 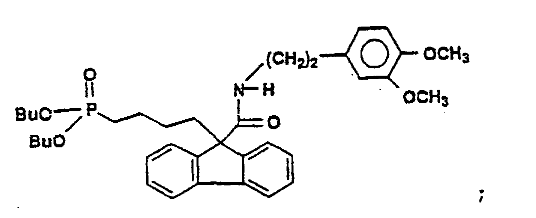 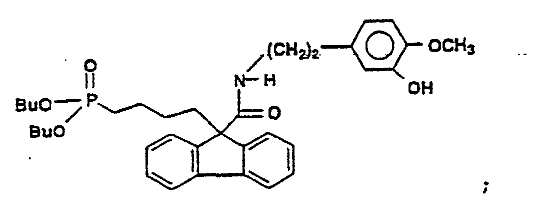 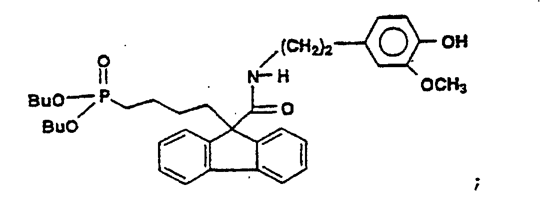 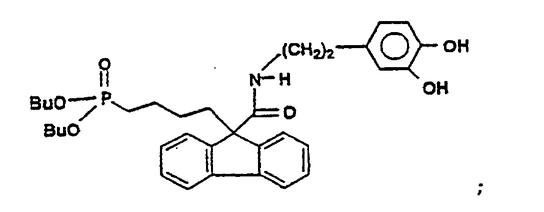 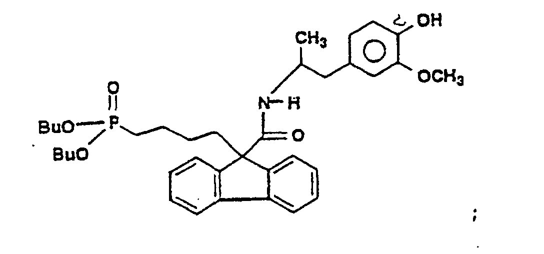 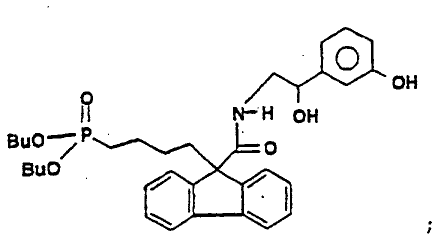 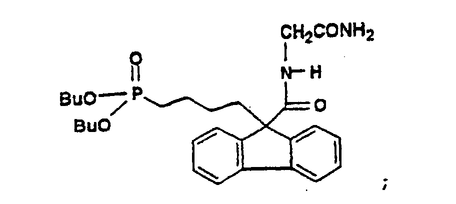 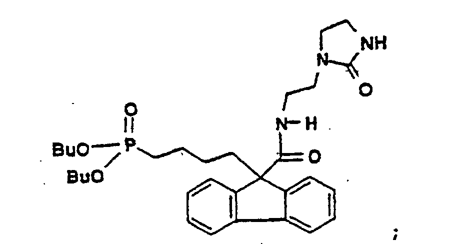 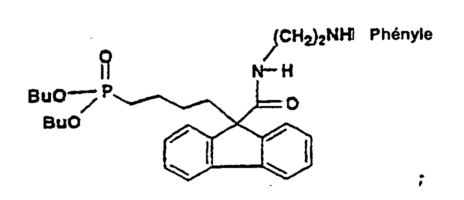 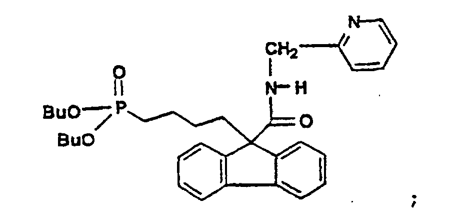 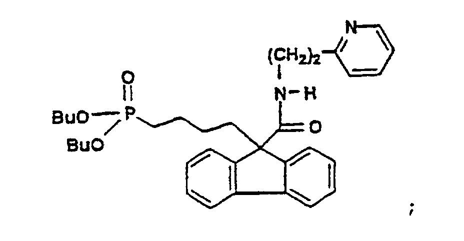 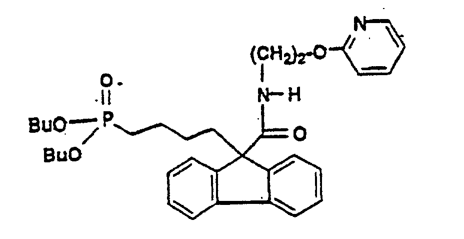 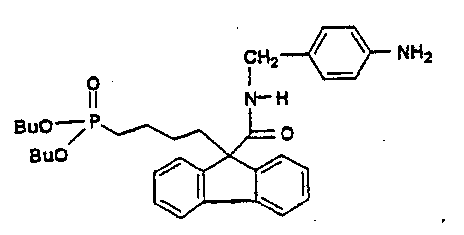 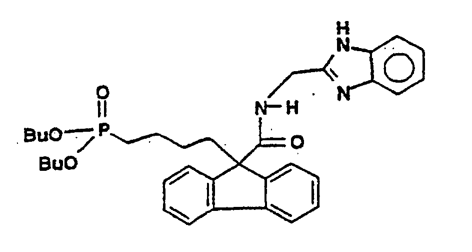 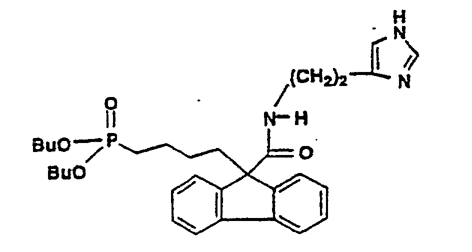 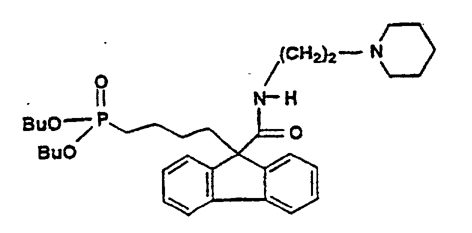 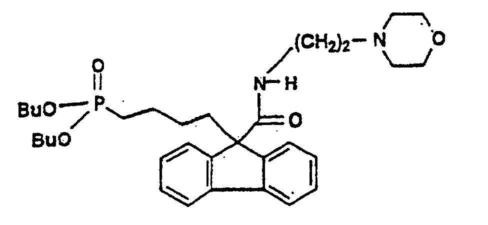 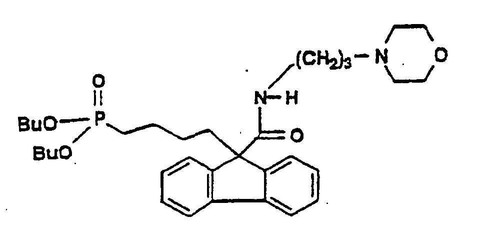 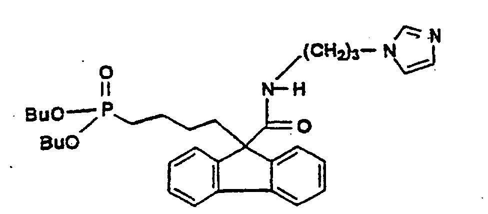 le 9-[4-(dibutaxyphosphinyl)butyl]-N-(2,2,2-trifluoroéthyl)-9H-fluorène-9-carboxamide ;
le 9-(2-propényl)-9H-fluorène-9-acide carboxylique, éthylester ;
le 9-propyl-9H-fluorène-9-carboxaldéhyde ;
le 9-(4-cyanobutyl)-N-propyl-9H-fluorène-9-carboxamide ;
le 1-[9-(3-phénylpropyl)-9H-fluorène-9-yl]-1-butanone ;
le 9-(3-phénylpropyl)-α-propyl-9H-fluorène-9-méthanol ;
le 4-hydroxy-1-(9-propyl-9H-fluorène-9-yl)-butanone ;
le N-[3-(dibutoxyphosphinyl)propyl]-9-propyl-9H-fluorène-9-carboxamide ;
le N-[5-(dibutoxyphosphinyl)pentyl]-9-propyl-9H-fluorène-9-carboxamide ;
le N-[[4-(1,3-dihydro-1-oxo-2H-isoindol-2-yl)phényl]méthyl]-9-propyl-9H-fluorène-9-carboxamide ;
le (E)-9-[4-(dibutoxyphosphinyl)-2-butényl]-2,7-difluoro-N-propyl-9H-fluorène-9-carboxamide ;
le 9-[4-(dibutoxyphosphinyl)butyl]-2,7-difluoro-N-propyl-9H-fluorène-9-carboxamide ;
le 9-[4-(diéthoxyphosphinyl)butyl]-N-propyl-9H-fluorène-9-carboxamide ;
le 9-[4-(diphénylphosphinyl)butyl]-N-propyl-9H-fluorène-9-carboxamide ;
le [4-[9-(butylthio)-9H-fluorène-9-yl]butyl]-acide phosphonique, dibutylester ;
le [4-[9-(butylsulfonyl)-9H-fluorène-9-yl]butyl]-acide phosphinique, dibutylester ;
le [4-[9-(butylsulfinyl)-9H-fluorène-9-yl]butyl]-acide phosphonique, dibutylester ;
le 5-[4-(dibutoxyphosphinyl)butyl]-N-propyl-5H-indéno[1,2-b]pyridine-5-carboxamide ;
le (E)-9-[4-(dibutoxyphosphinyl)-2-butényl]-2,7-difluoro-N-(2,2,2-trifluoroéthyl)-9H-flourène-9-carboxamide ;
le 9-[4-[4-(1,3-dihydro-1,3-dioxo-2H-isoindol-2-yl)phényl]butyl]-N-propyl-9H-fluorène-9-carboxamide ;
le 9-[4-[4-[[(2-phénoxyphényl)carbonyl]amino]-phényl]butyl]-N-propyl-9H-fluorène-9-carboxamide ;
le 9-[4-[4-(1,3-dihydro-1-oxo-2H-isoindol-2-yl)-phényl]butyl]-N-propyl-9H-fluorène-9-carboxamide ;
le 9-[3-[4-(1,3-dihydro-1,3-dioxo-2H-isoindol-2-yl)phényl]propyl]-N-propyl-9H-fluorène-9-carboxamide ;
le 9-[3-[4-(benzoylamino)phényl]-N-propyl-9H-fluorène-9-carboxamide ;
le 9-[3-[(1,3-dihydro-1-oxo-2H-isoindol-2-yl)phényl]propyl]-N-propyl-9H-fluorène-9-carboxamide ;
le 9-[5-[(6-éthoxy-2-benzothiazolyl)thio]-pentyl]-N-propyl-9H-fluorène-9-carboxamide ;
le 9-[4-[4-(benzoylamino)phényl]butyl]-N-propyl-9H-fluorène-9-carboxamide ;
le 9-[5-(dibutoxyphosphinyl)pentyl]-N-propyl-9H-fluorène-9-carboxamide ;
le N,N-diéthyl-9-(2-propényl)-9H-fluorène-9-carboxamide ;
le N-éthyl-9-propyl-9H-fluorène-9-carboxamide ;
le N-éthyl-9-(2-propényl)-9H-xanthène-9-carboxamide ;
le N-éthyl-9-(3-phénylpropyl)-9H-xanthène-9-carboxamide ;
le 9-[(4-morpholinyl)carbonyl]-9-propyl-9H-fluorène ;
9-hexyl-N-propyl-9H-xanthène-9-carboxamide ;
le N-méthoxy-N-méthyl-9-propyl-9H-fluorène-9-carboxamide ;
le 10,11-dihydro-5-(3-phényl-2-propényl)-N-propyl-5H-dibenzo[a,d]cycloheptène-5-carboxamide ;
le N-méthyl-9-propyl-9H-fluorène-9-carboxamide ;
le 1-(9-propyl-9H-fluorène-9-yl)-1-pentanone ;
α-butyl-9-propyl-9H-fluorène-9-méthanol ;
le 1-(9-propyl-9H-fluorène-9-yl)-1-butanone ;
α,9-dipropyl-9H-fluorène-9-méthanol ;
le 10,11-dihydro-5-(2-propényl)-N-propyl-5H-dibenzo-[a,d]cycloheptène-5-carboxamide ;
le 9-(3-phénylpropyl)-N-propyl-9H-thioxanthène-9-carboxamide ;
le N,9-dipropyl-9H-thioxanthène-9-carboxamide ;
le 10,11-dihydro-5-(3-phénylpropyl)-N-propyl-5H-dibenzo-[a,d]cycloheptane-5-carboxamide ;
le (E)-2,7-difluoro-9-(3-phényl-2-propényl)-N-propyl-9H-fluorène-9-carboxamide ;
le 9-(3-phénylpropyl)-N-(2-pyridinylméthyl)-9H-fluorène-9-carboxamide ;
le 2,7-difluoro-9-(3-phénylpropyl)-N-propyl-9H-fluorène-9-carboxamide ;
le 2,7-difluoro-9-(3-phénylpropyl)-N-(4-pyridinylméthyl)-9H-fluorène-9-carboxamide ;
le 9-(butylthio)-9-propyl-9H-fluorène ;
le 9-(butylsulfinyl)-9-propyl-9H-fluorène ;
le 9-(4-hydroxybutyl)-N-propyl-9H-fluorène-9-carboxamide ;
le 9-[4(phénylthio)butyl]-N-propyl-9H-fluorène-9-carboxamide ;
le 9-[3-(1,3-dioxan-2-yl)propyl]-N-propyl-9H-fluorène-9-carboxamide ;
le 9-[3-(1,3-dioxolan-2-yl)propyl]-N-propyl-9H-fluorène-9-carboxamide ;
le cis-N,9-dipropyl-1H-thioxanthène-9-carboxamide, 10-oxyde ;
le 5-(2-propényl)-N-propyl-5H-indéno[1,2-b]pyridine-5-carboxamide ;
le (E)-5-(3-phényl-2-propényl)-N-propyl-5H-indéno[1,2-b]pyridine-5-carboxamide ;
le N-éthyl-N-méthyl-9-(2-propényl)-9H-fluorène-9-carboxamide ;
le N,9-dipropyl-9H-thioxanthène-9-carboxamide, 10,10-dioxyde ;
le trans-N,9-dipropyl-9H-thioxanthène-9-carboxamide, 10-oxyde ;
le 9-[3-(dibutoxyphosphinyl)propyl]-N-(2-pyridinylméthyl)-9H-fluorène-9-carboxamide ;
le 1-(9-propyl-9H-fluorène-9-yl)-2-(1-pipéridinyl)éthanone, monohydrochlorure ;
le N-(5-hydroxypentyl)-9-propyl-9H-fluorène-9-carboxamide ;
le 9-(3-cyanopropyl)-N-propyl-9H-fluorène-9-carboxamide ;
le N-[[4-[[(9-propyl-9H-fluorène-9-yl)carbonyl]amino]phényl]méthyl]-9-propyl-9H-fluorène-9-carboxamide ;
le N-[4-(4-aminophényl)méthyl]-9-propyl-9H-fluorène-9-carboxamide ;
le 9-[3-(dibutoxyphosphinyl)propyl]-N-propyl-9H-fluorène-9-carboxamide ;
le 4-(1-pipéridinyl)-1-(9-propyl-9H-fluorène-9-yl)-1-butanone, monohydrochlorure ;
le N-méthyl-9-(3-phénylpropyl)-9H-fluorène-9-carboxamide ;
le 2-(diméthylamino)-9-(3-phénylpropyl)-N-propyl)-9H-fluorène-9-carboxamide ;
le 9-[4-(dibutoxyphosphinyl)-2-butényl]-N-propyl-9H-fluorène-9-carboxamide ;
le 9-[4-(4-nitrophényl)butyl]-N-propyl-9H-flourène-9-carboxamide ;
le 9-[3-(4-nitrophényl)-2-propényl]-N-propyl-9H-fluorène-9-carboxamide ;
le 5-(3-phénylpropyl)-N-propyl-5H-indéno[1,2-b]pyridine-5-carboxamide ;
le 9-[4-(4-aminophényl)butyl]-N-propyl-9H-fluorène-9-carboxamide ;
le 9-[3-(4-aminophényl)propyl]-N-propyl-9H-fluorène-9-carboxamide ;
le 9-[4-(dibutoxyphosphinyl)butyl]-9H-fluorène-9-acide carboxylique, méthyl-ester ;
le N,N-dibutyl-9-[(propylamino)carbonyl]-9H-fluorène-9-butanamide ;
le 9-(5-cyanopentyl)-N-propyl-9H-fluorène-9-carboxamide ;
le 9-[2-[[[4-(1,3-dihydro-1,3-dioxo-2H-isoindol-2-yl)phényl]sulfonyl]amino]-éthyl]-N-(2,2,2-trifluoroéthyl)-9H-fluorène-9-carboxamide ;
le (Z)-9-[4-[(6-éthoxy-2-benzothiazolyl)thio]-2-butényl]-N-propyl-9H-fluorène-9-carboxamide ;
le 9-[4-(dibutoxyphosphinyl)butyl]-N-(2,2,2-trifluoropropyl)-9H-xanthène-9-carboxamide ;
le 9-[4-[butoxy[2-(4-morpholinyl)éthoxy]phosphinyl]butyl]-N-(2,2,2-trifluoroéthyl)-9H-fluorène-9-carboxamide ; 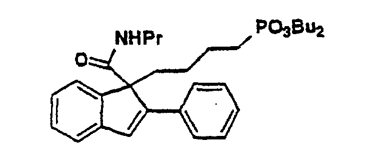 le 9-[4-(dibutoxyphosphinyl)butyl]-2,7-difluoro-N-(2,2,2-trifluoroéthyl)-9H-fluorène-9-carboxamide ; 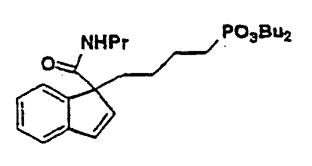 le (E)-9-[4-(dibutoxyphosphinyl)-2-butényl]-N-propyl-9H-fluorène-9-carboxamide ;
le 9-[4-[4-(benzoylamino)-1H-imidazol-1-yl]butyl]-N-(2,2,2-trifluoroéthyl)-9H-fluorène-9-carboxamide ;
le 9-[4-[5-(benzoylamino)-2-pyridinyl]butyl]-N-(2,2,2-trifluoroéthyl)-9H-fluorène-9-carboxamide ;
le 9-[4-[4-[(2-phénoxybenzoyl)amino]-1H-imidazol-1-yl]butyl]-N-(2,2,2-trifluoroéthyl)-9H-fluorène-9-carboxamide ;
le 9-[4-[(2-bromo-5-pyridinyl)amino]butyl]-N-propyl-9H-fluorène-9-carboxamide ;
le 9-[2-[[[4(benzoylamino)phényl]sulfonyl]amino]éthyl]-N-(2,2,2-trifluoroéthyl)-9H-fluorène-9-carboxamide ;
le 9-(4-phénylbutyl)-N-propyl-9H-fluorène-9-carboxamide ;
le 3-[(9-propyl-9H-fluorène-9-yl)sulfonyl]-acide propanoïque, méthylester ;
le 9-[4-[(6-éthoxy-2-benzothiazolyl)thio]butyl]-N-propyl-9H-fluorène-9-carboxamide ;
le 9-[3-[(6-éthoxy-2-benzothiazolyl)thio]propyl]-N-propyl-9H-fluorène-9-carboxamide ;
le (Z)-9-[4-(diéthoxyphosphinyl)-2-butényl]-N-(2,2,2-trifluoroéthyl)-9H-fluorène-9-carboxamide ;
le 9-[4-(diéthoxyphosphinyl)butyl]-N-(2,2,2-trifiuoroéthyl)-9H-fluorène-9-carboxamide ;
le 9-[4-(dibutoxyphosphinyl)butyl]-N-(2,2,3,3,3-pentafluoropropyl)-9H-fluorène-9-carboxamide ;
le 9-[4-(dibutoxyphosphinyl)butyl]-N-propyl-9H-xanthène-9-carboxamide ;
le 9-[4-(dibutoxyphosphinyl)butyl]-N-(2,2,3,3,4,4,4-heptafluorobutyl)-9H-fluorène-9-carboxamide ;
le 9-[4-(dibutoxyphosphinyl)butyl]-N-propyl-9H-indéno-[2,1-b]pyridine-9-carboxamide ;
le 9-[4-[4-[(phénylsulfonyl)amino]phényl]butyl]-N-(2,2,2-trifluoroéthyl)-9H-fluorène-9-carboxamide ;
le [4-[9-(1-oxopentyl)-9H-fluorène-9-yl]butyl]-acide phosphonique ;
le 9-[5-(dibutoxyphosphinyl)pentyl]-N-(2,2,2-trifluoroéthyl)-9H-fluorène-9-carboxamide ;
le 9-[3-[[5-[(2-phénoxybenzoyl)amino]-2-pyridinyl]oxy]propyl]-N-(2,2,2-trifluoroéthyl)-9H-fluorène-9-carboxamide ;
le [6-[9-[[(2,2,2-trifluoroéthyl)amino]-carbonyl]-9H-fluorène-9-yl]hexyl]acide phosphonique, dibutylester ;
le 9-[4-[5-[(2-phénoxybenzoyl)amino]-2-pyridinyl]butyl]-N-(2,2,2-trifluoroéthyl)-9H-fluorène-9-carboxamide ;
le 9-[4-[4-(benzoylamino)-2-méthyl-1H-imidazol-1-yl]butyl]-N-(2,2,2-trifluoroéthyl)-9H-fluorène-9-carboxamide ;
le 9-[4-[4-[(2-phénoxybenzoyl)amino]-2-méthyl-1H-imidazol-1-yl]butyl]-N-(2,2,2-trifluoroéthyl)-9H-fluorène-9-carboxamide ;
le 9-[3-[[2-(benzoylamino)-5-pyridinyl]amino]propyl]-N-(2,2,2-trifluoroéthyl)-9H-fluorène-9-carboxamide ;
le [[4-(benzoylamino)phényl]méthyl][2-[9-[[(2,2,2-trifluoroéthyl)amino]-carbonyl]-9H-fluorène-9-yl]éthyl]acide carbamique, 1,1-diméthyléthylester ;
le 9-[2-[[[4-(benzoylamino)phényl]méthyl]amino]éthyl]-N-(2,2,2-trifluoroéthyl)-9H-fluorène-9-carboxamide ;
le 9-[4-[butoxy(tétrahydrofurane-2-ylméthoxy)phosphinyl]butyl]-N-(2,2,2-trifluoroéthyl)-9H-fluorène-9-carboxamide ;
le 9-[4-[butoxy(2-pyridinylméthoxy)phosphinyl]butyl]-N-(2,2,2-trifluoroéthyl)-9H-fluorène-9-carboxamide ;
le 9-[4-(dipropoxyphosphinyl)butyl]-N-(2,2,2-trifluoroéthyl)-9H-fluorène-9-carboxamide ;
le 9-[4-[4-[[(4-nitrophényl)sulfonyl]amino]phényl]butyl]-N-(2,2,2-trifluoroéthyl)-9H-fluorène-9-carboxamide ;
le 9-[4-[4-[[(2-nitrophényl)sulfonyl]amino]phényl]butyl]-N-(2,2,2-trifluoroéthyl)-9H-fluorène-9-carboxamide ;
le 9-[4-(dibutoxyphosphinyl)butyl]-3,6-difluoro-N-(2,2,2-trifluoroéthyl)-9H-fluorène-9-carboxamide ;
le 9-[3-[[5-[(2-phénoxybenzoyl)amino]-2-pyridinyl]oxy]propyl]-N-propyl-9H-fluorène-9-carboxamide ;
le 9-[6-[(6-éthoxy-2-benzothiazolyl)thio]hexyl]-N-(2,2,2-trifluoroéthyl)-9H-fluorène-9-carboxamide ;
le [4-[9-[[(2,2,2-trifluoroéthyl)amino]carbonyl]-9H-fluorène-9-yl]butyl]acide phosphonique, di(1-méthyl-éthyl)ester ;
le [[4-[(2-phénoxybenzoyl)amino]phényl]méthyl][2-[9-[[(2,2,2-trifluoroéthyl)amino]carbonyl]-9H-fluorène-9-yl]éthyl]acide carbamique, 1,1-diméthyléthylester ;
le 9-[2-[[[4-[(2-phénoxybenzoyl)amino]phényl]méthyl]amino]éthyl]-N-(2,2,2-trifluoroéthyl)-9H-fluorène-9-carboxamide ;
le [1-[4-[9-[[(2,2,2-trifluoroéthyl)amino]carbonyl]-9H-fluorène-9-yl]butyl]-1H-imidazol-4-yl]-acide carbamique ;
le 9-[4-[(4,5-diphényl-1H-imidazol-2-yl)thio]-butyl]-N-[2-(4-méthoxyphényl)éthyl]-9H-fluorène-9-carboxamide ;
le 9-[4-[(6-éthoxy-2-benzothiazolyl)thio]butyl]-N-propyl-9H-fluorène-9-carboxamide ;
le 9-[4-(2-thiazolylthio)butyl]-N-(2,2,2-trifluoroéthyl)-9H-fluorène-9-carboxamide ; 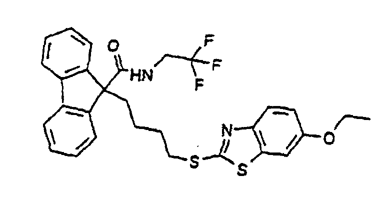 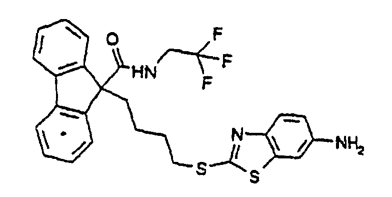 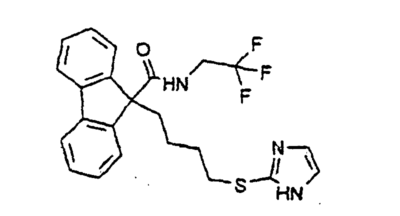 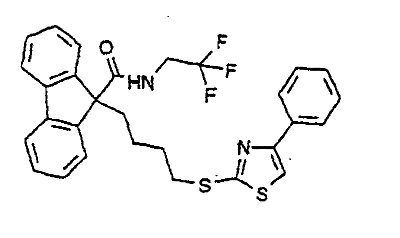 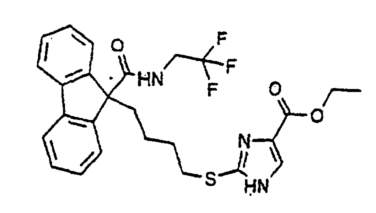 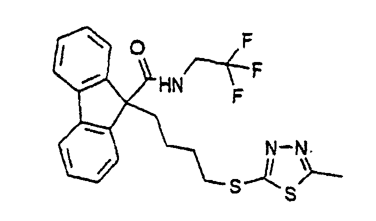 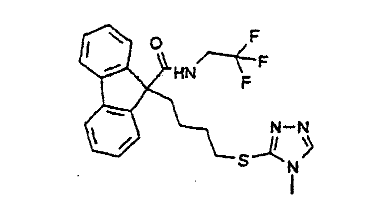 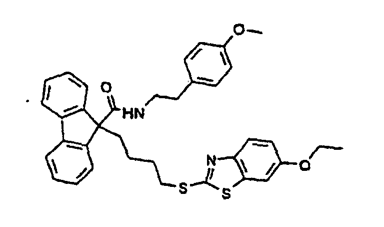 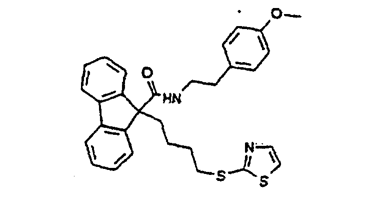 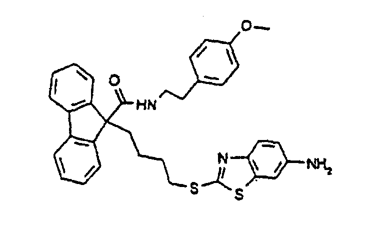 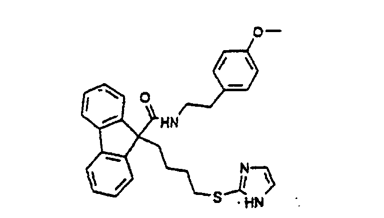 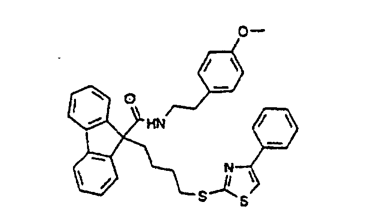 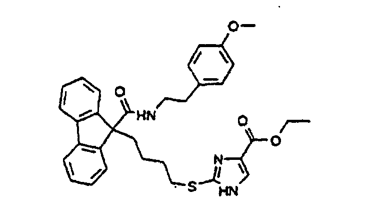 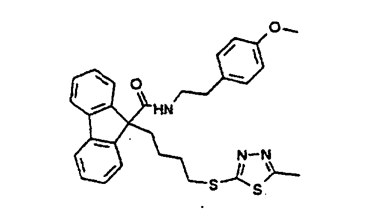 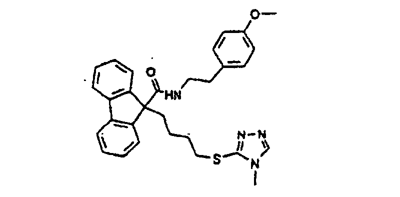 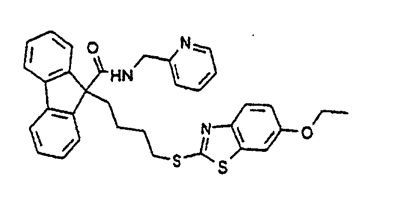 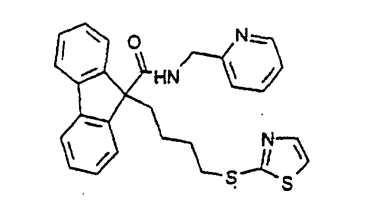 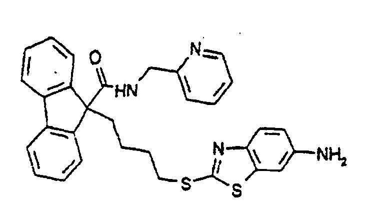 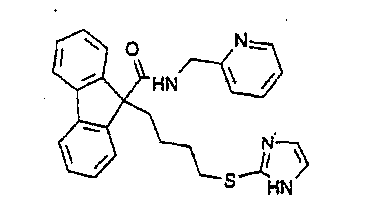 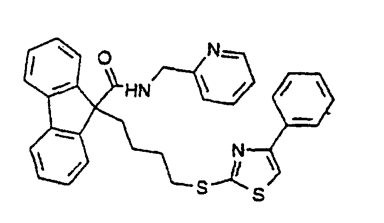 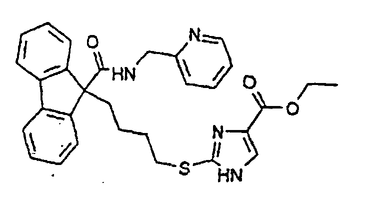 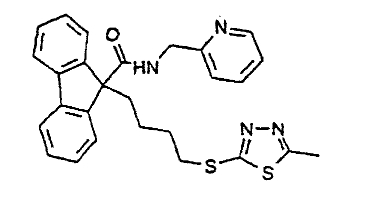 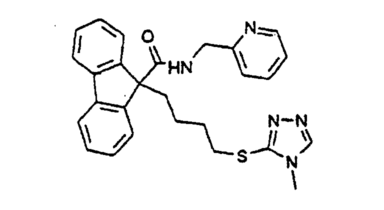 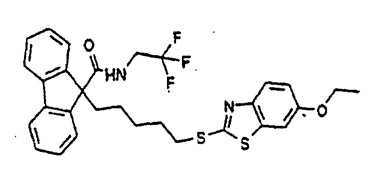 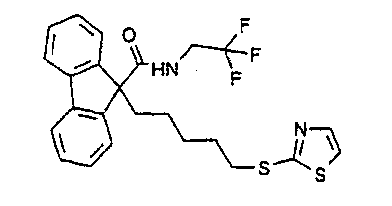 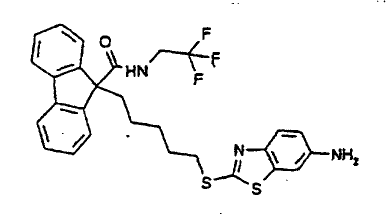 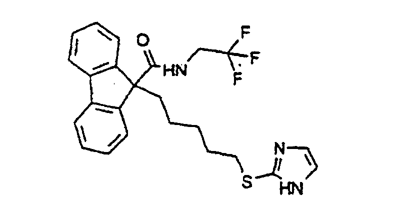 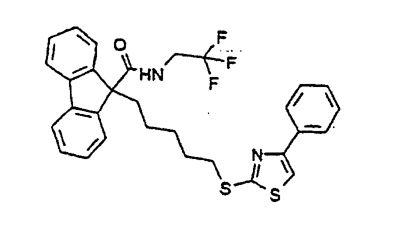 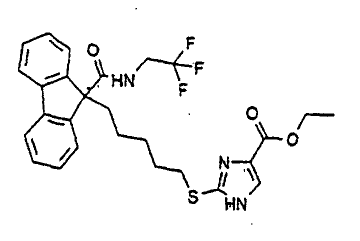 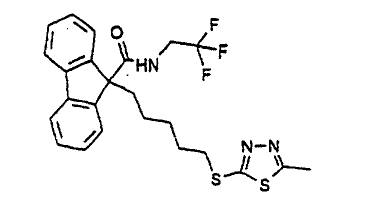 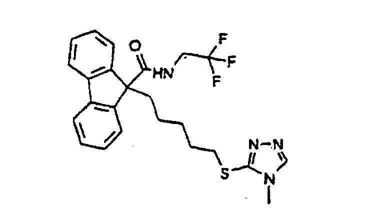 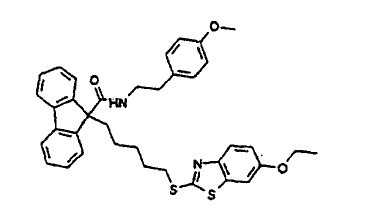 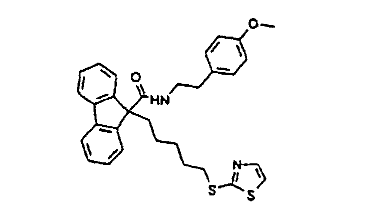 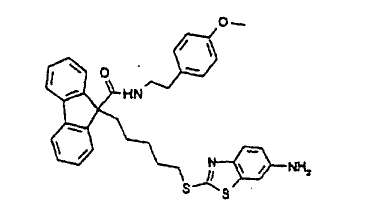 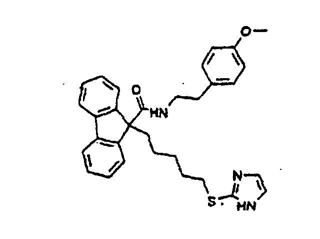 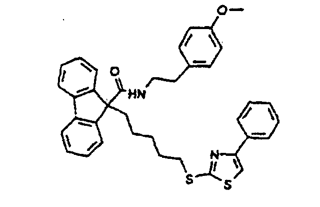 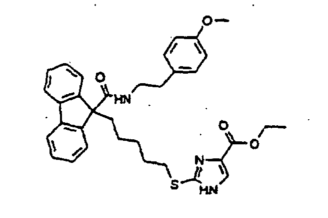 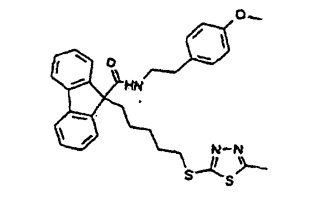 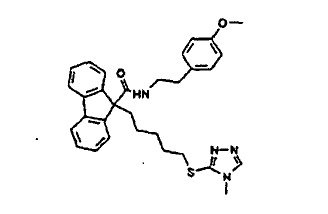 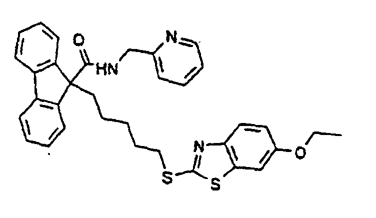 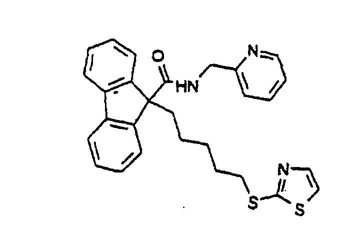 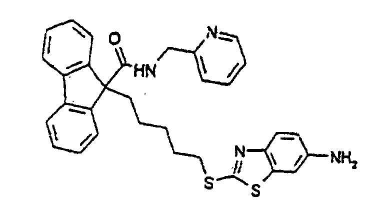 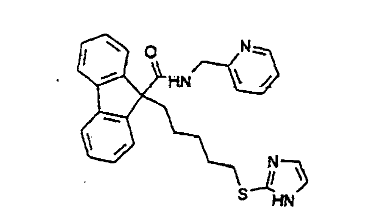 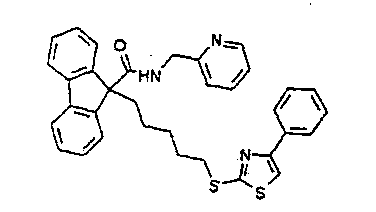 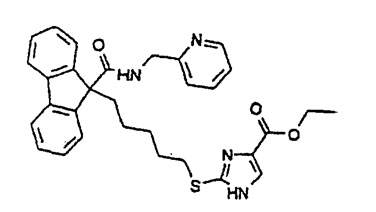 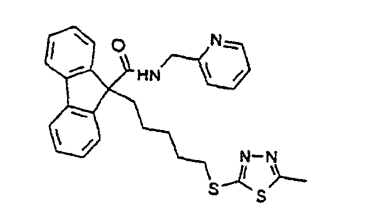 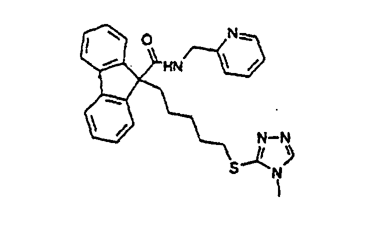 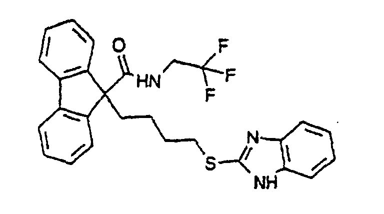 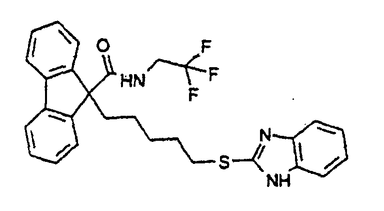 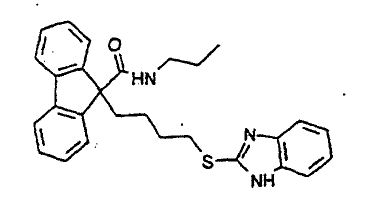 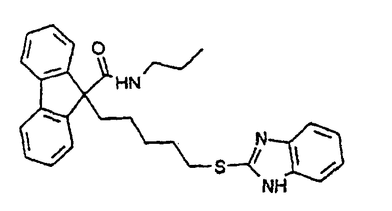 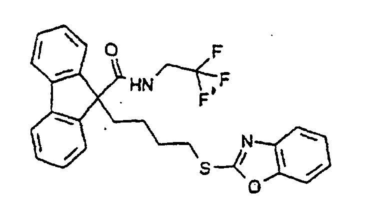 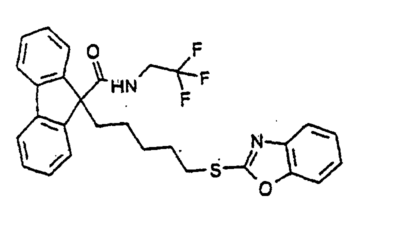 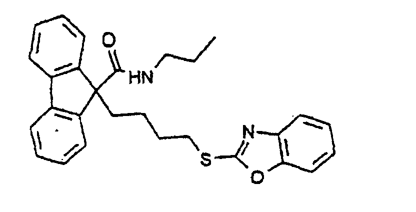 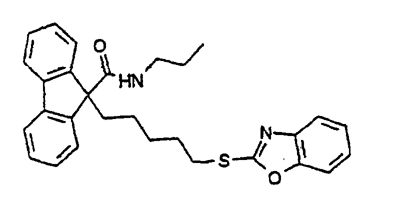 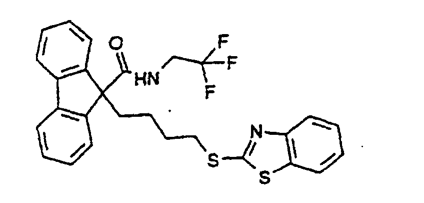 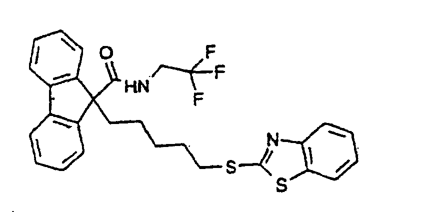 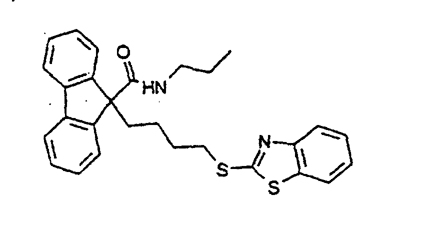 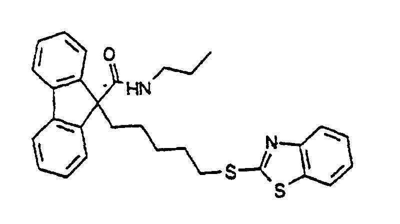 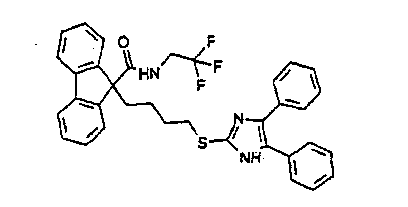 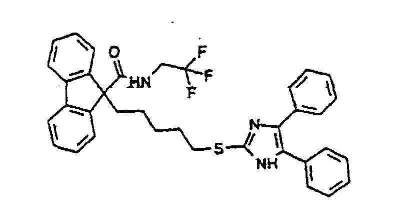 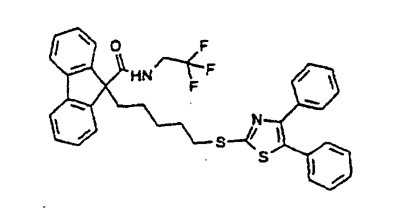 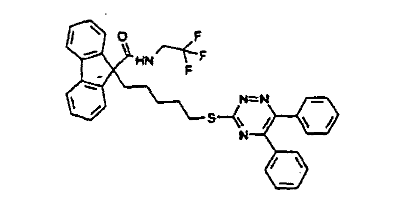 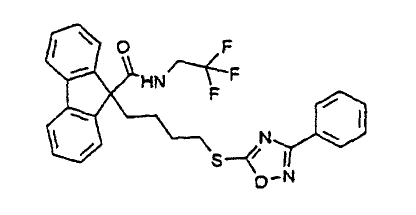 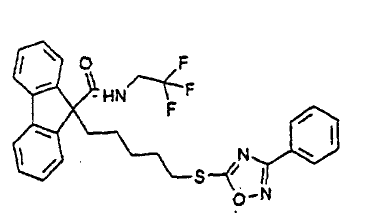 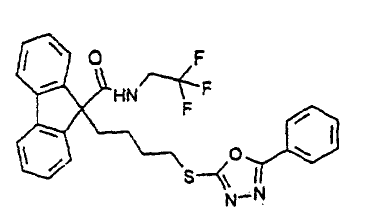 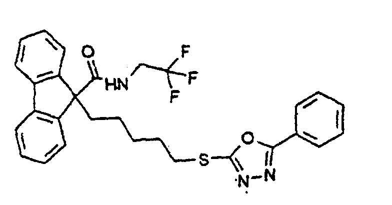 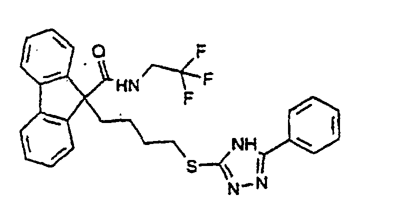 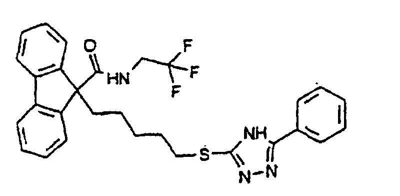 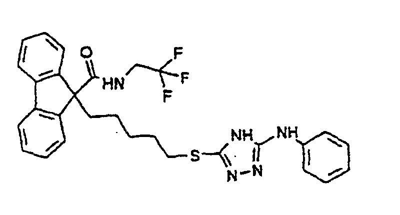 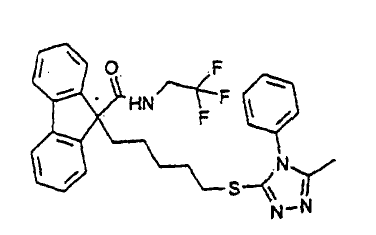 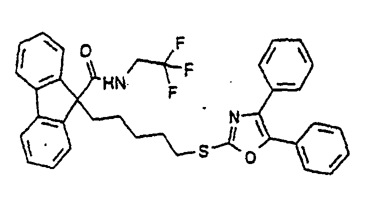 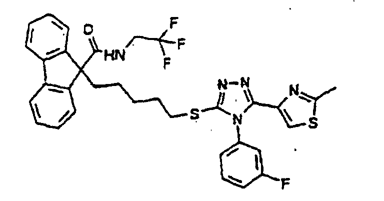 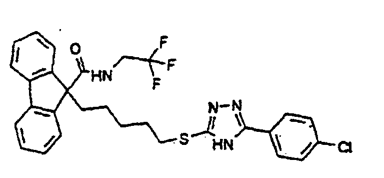 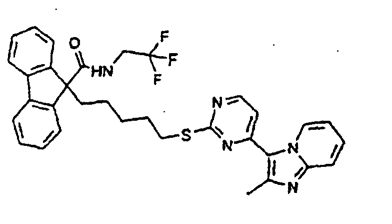 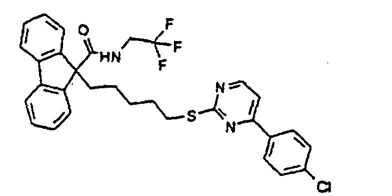 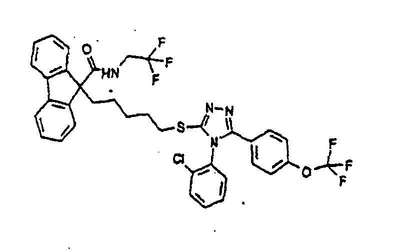 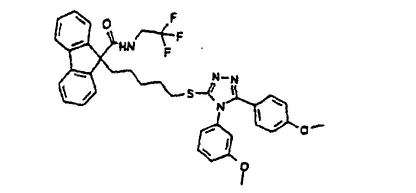 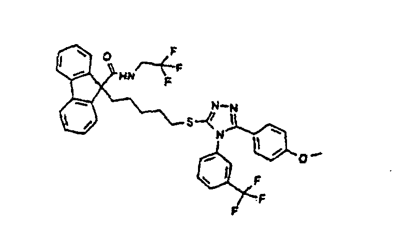 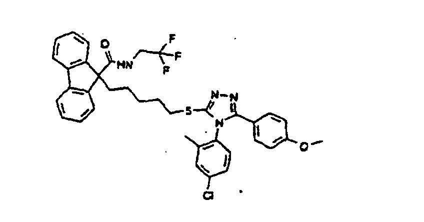 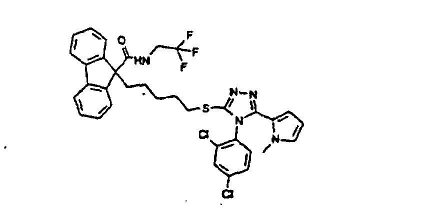 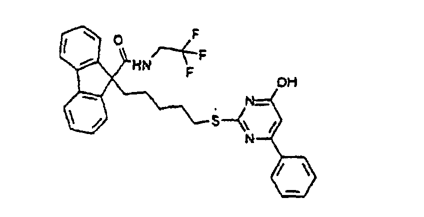 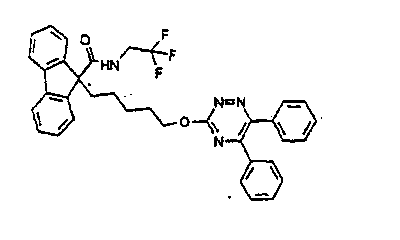 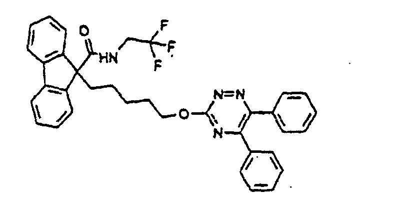 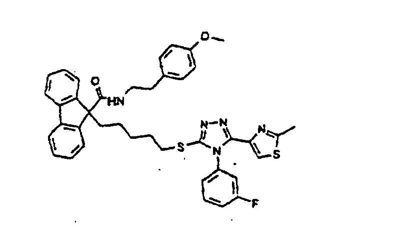 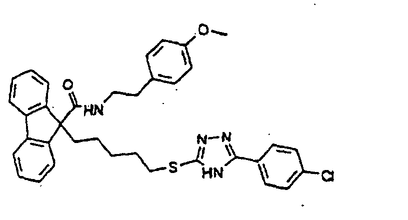 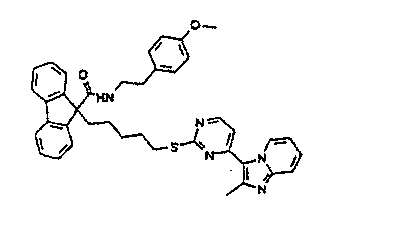 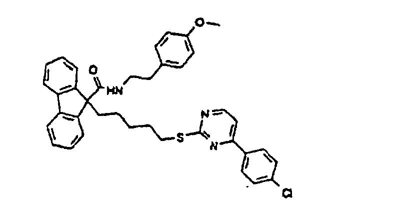 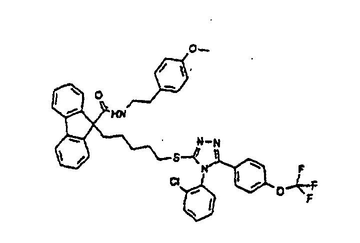 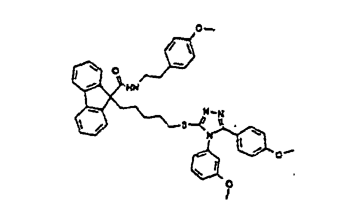 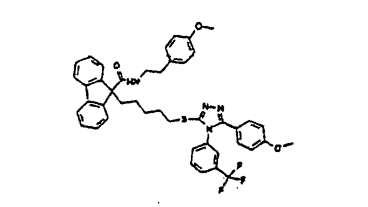 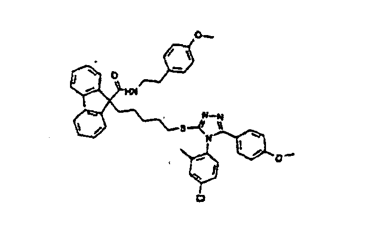 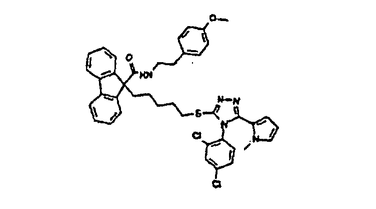 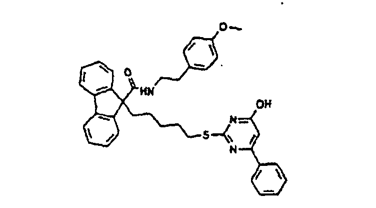 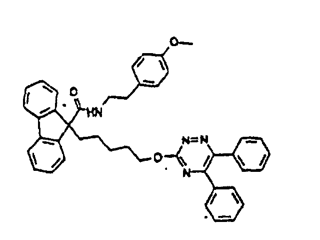 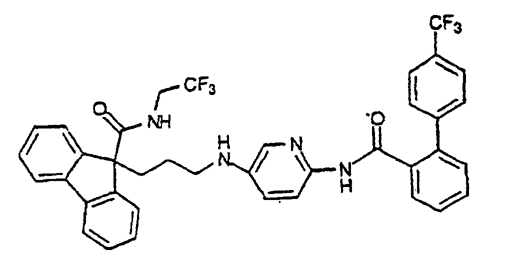 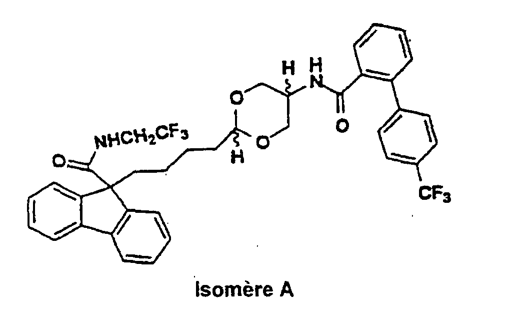 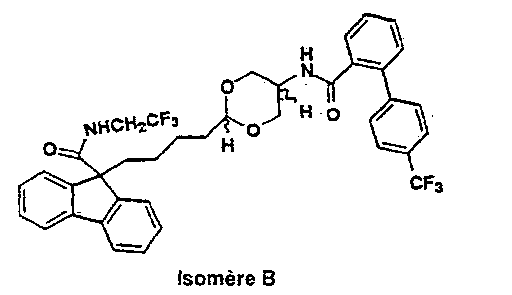 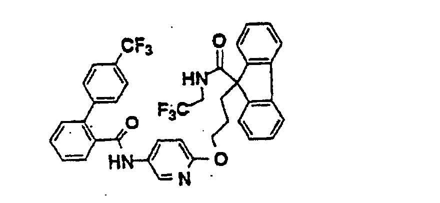 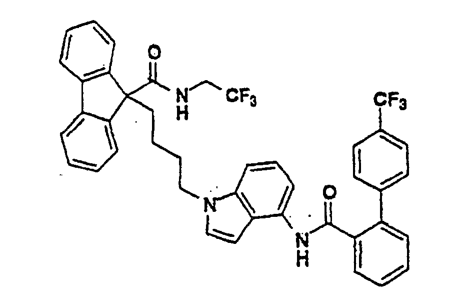 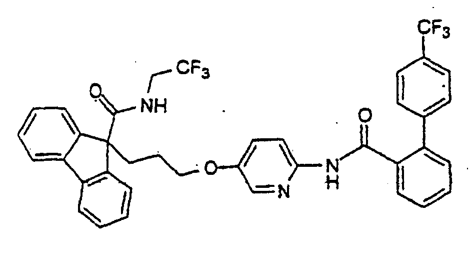 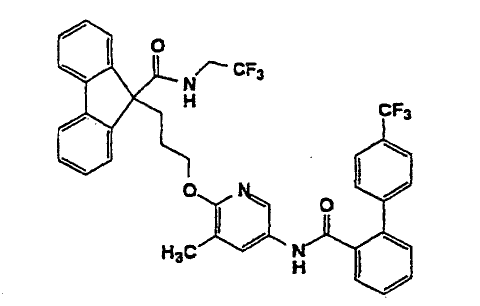 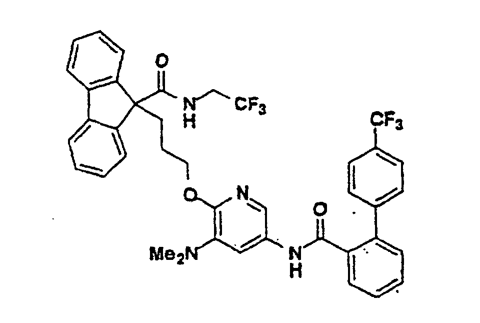 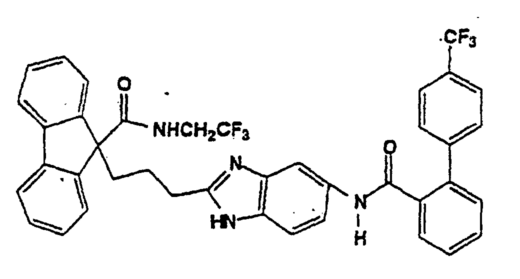 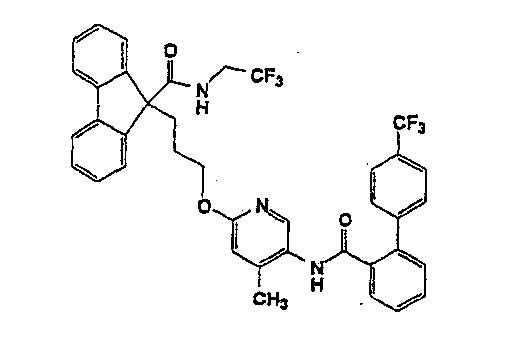 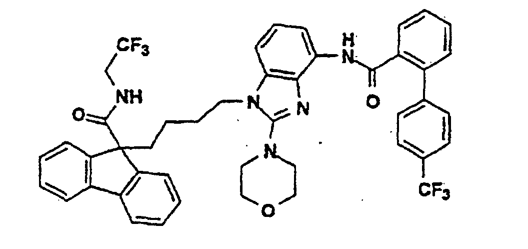 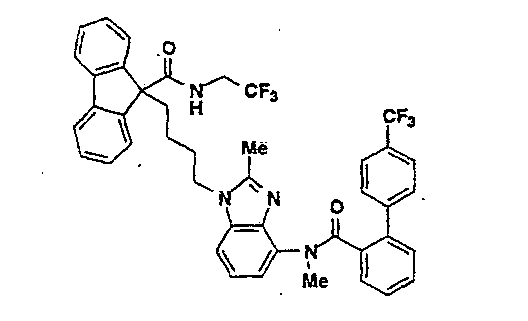 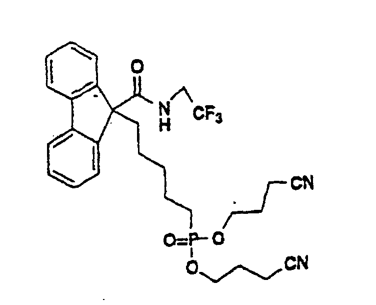 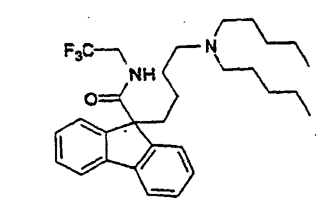 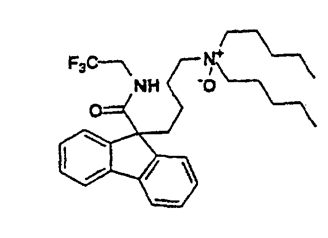 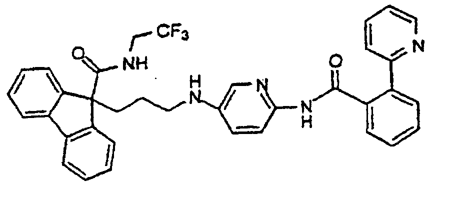 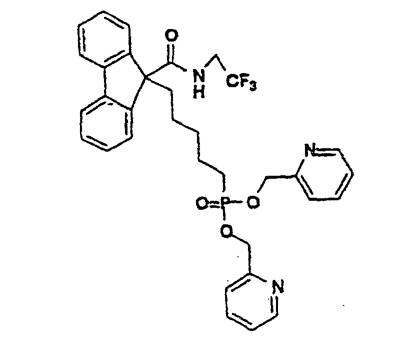 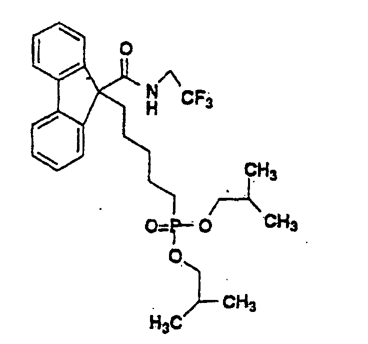 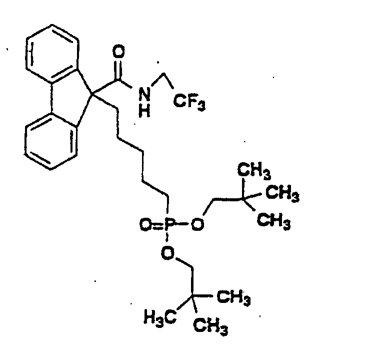 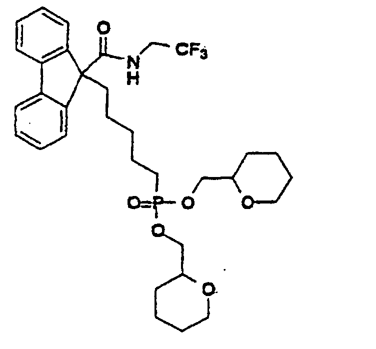 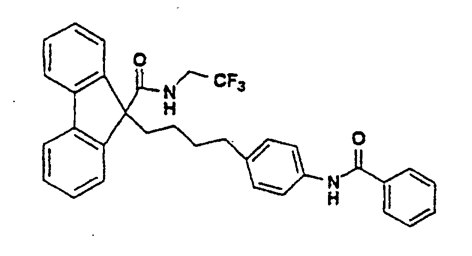 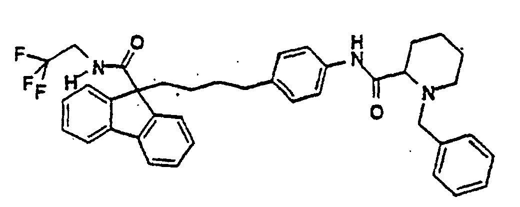 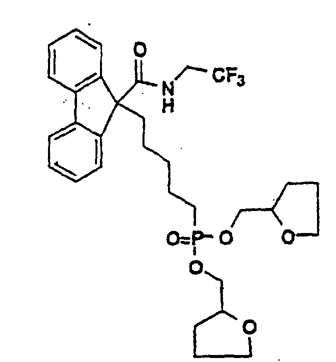 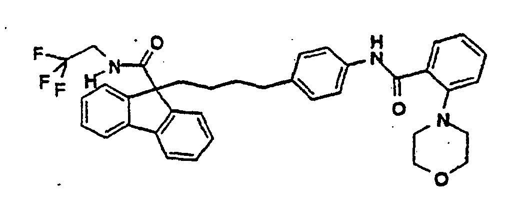 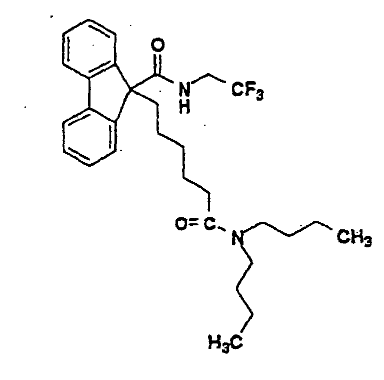 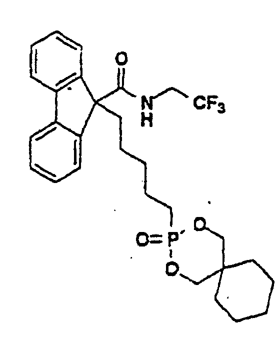 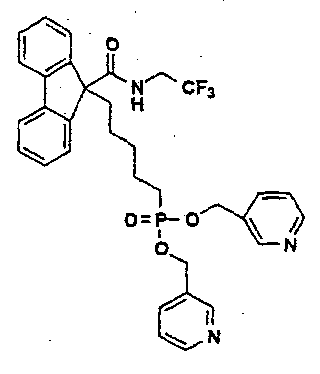 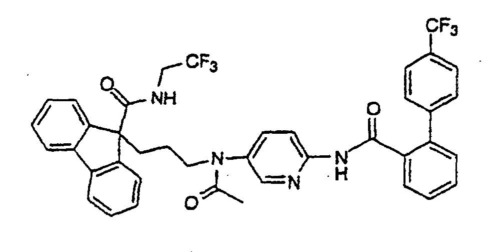 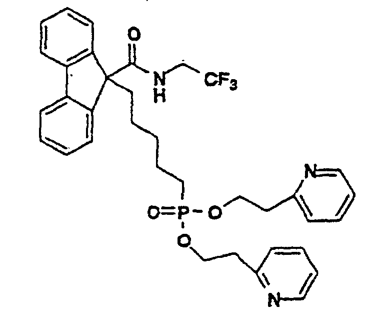 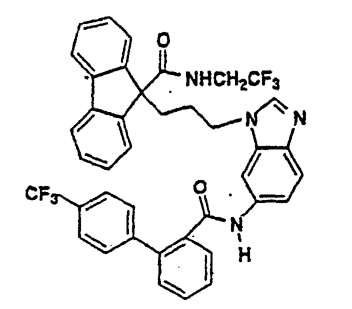 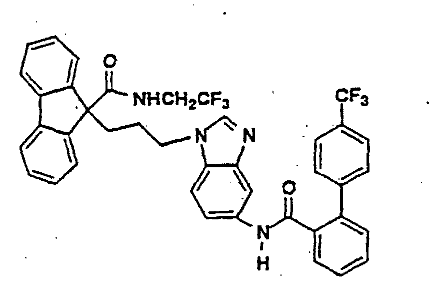 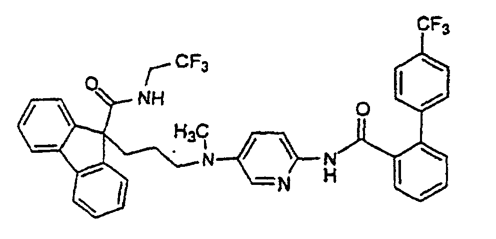 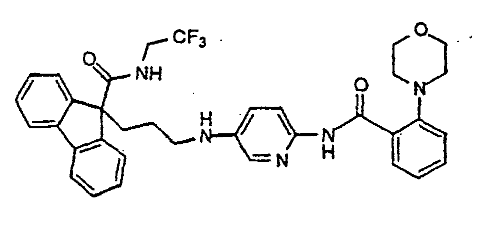 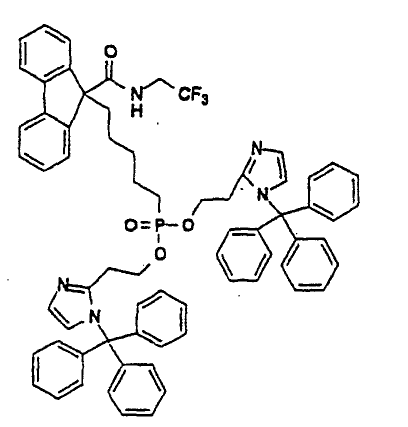 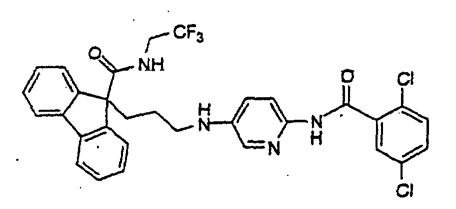 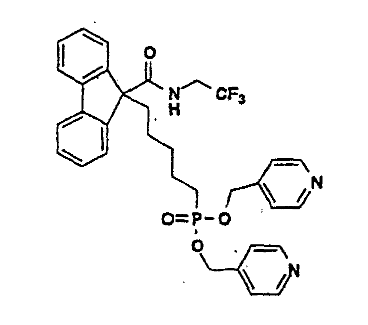 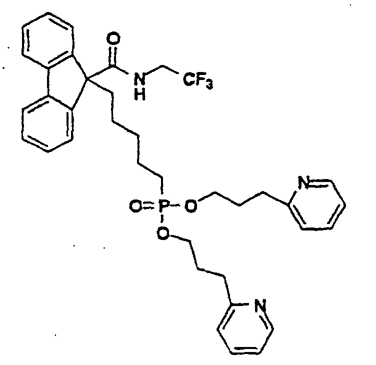 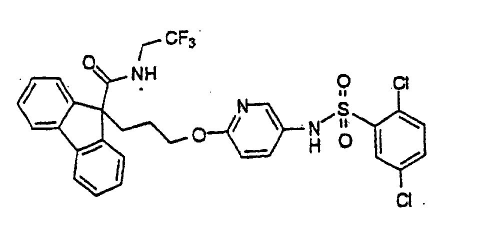 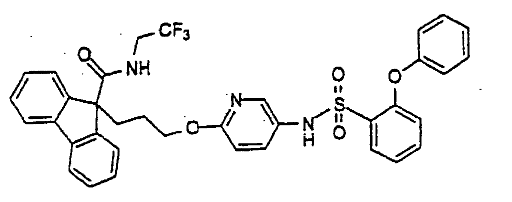 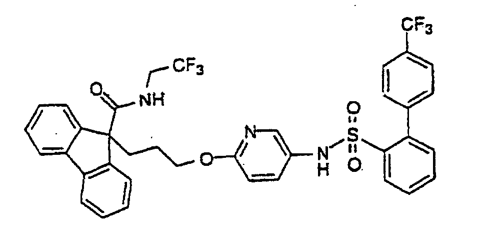 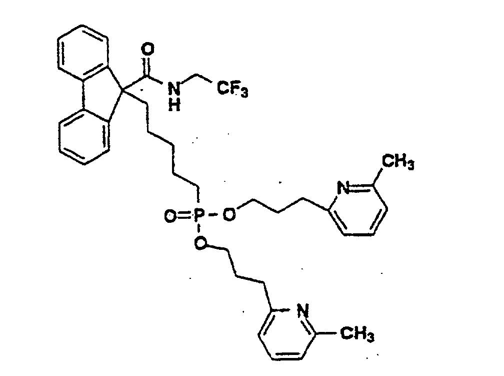 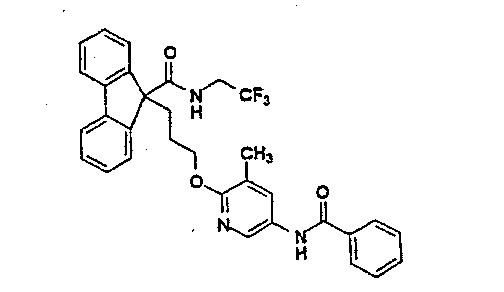 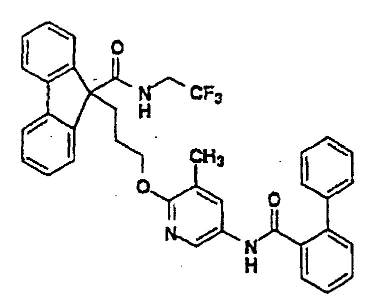 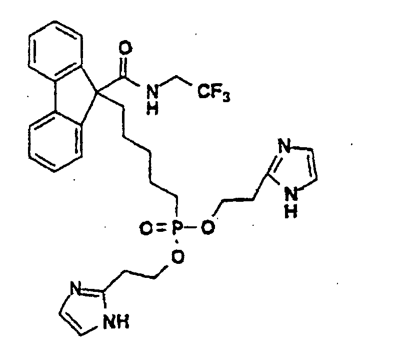 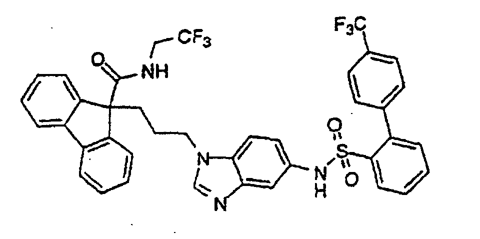 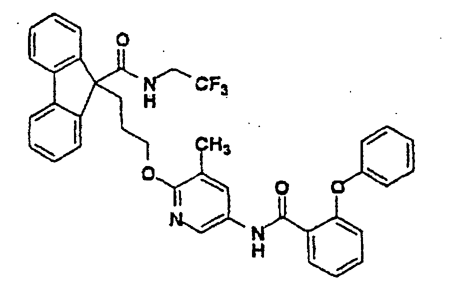 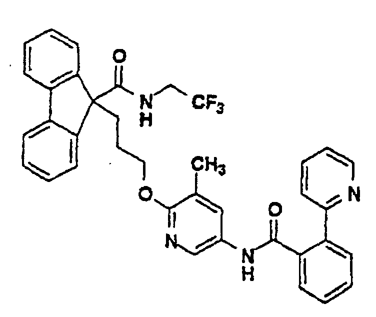 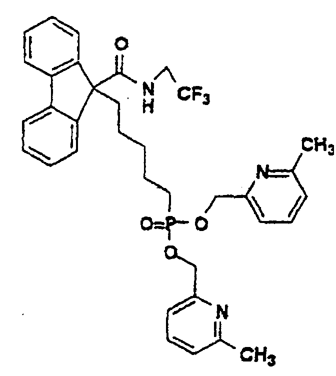 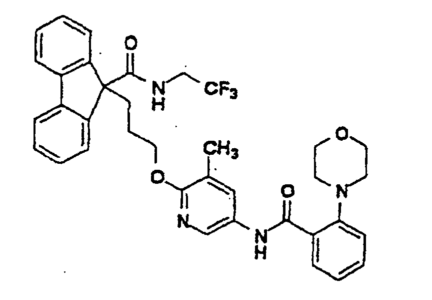 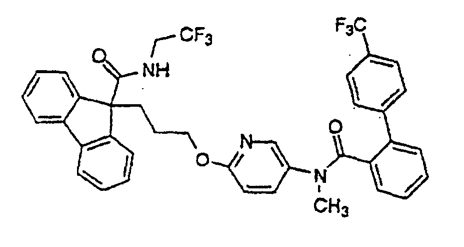 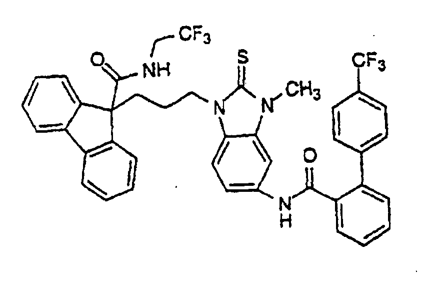 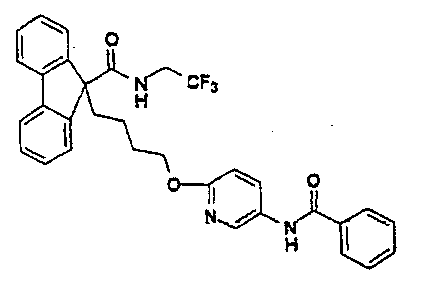 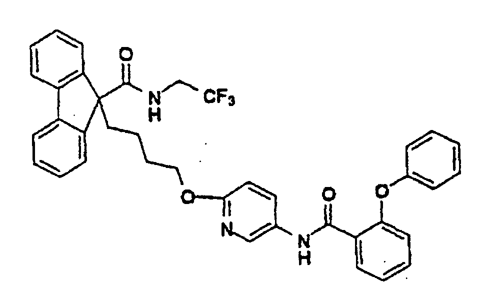 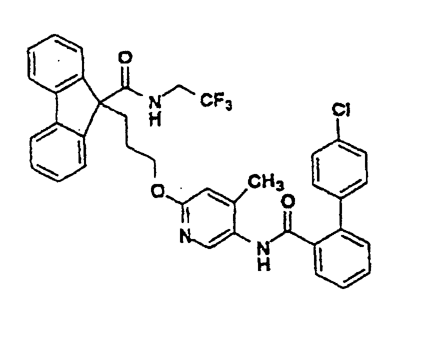 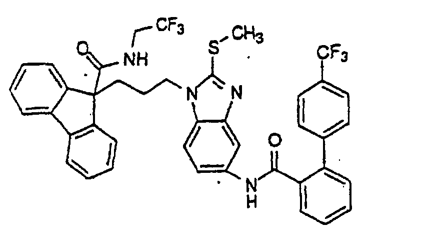 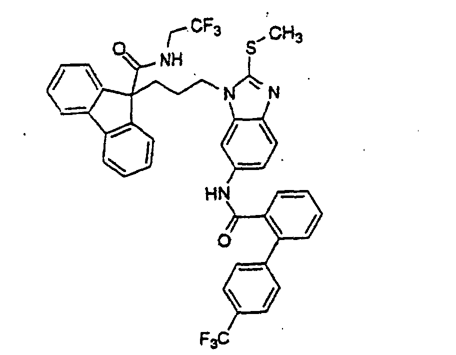 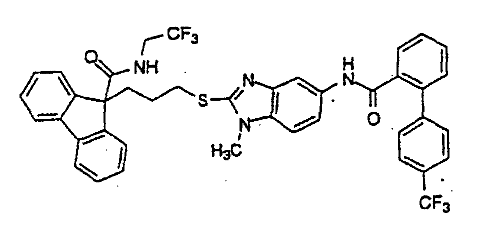 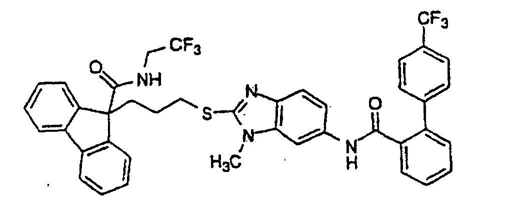 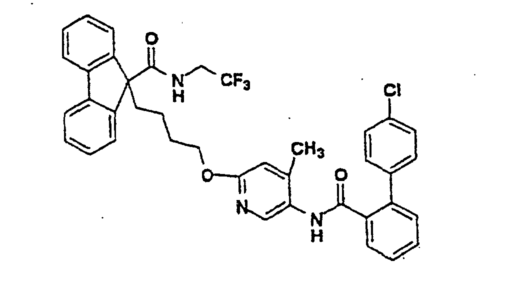 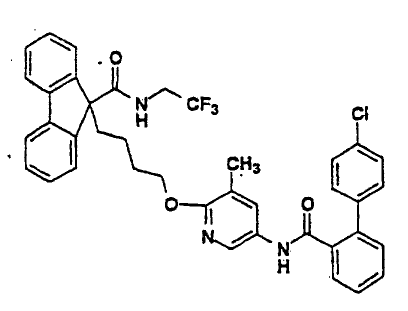 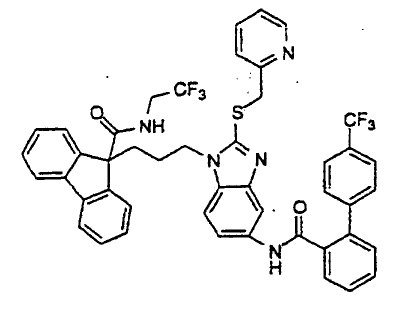 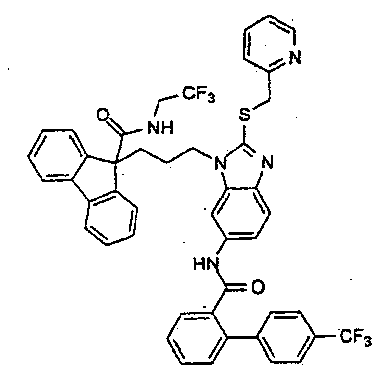 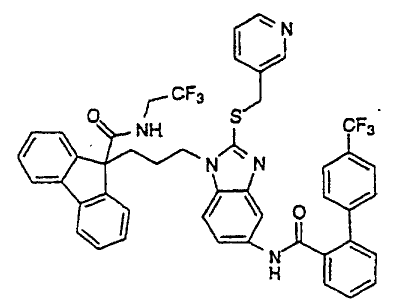 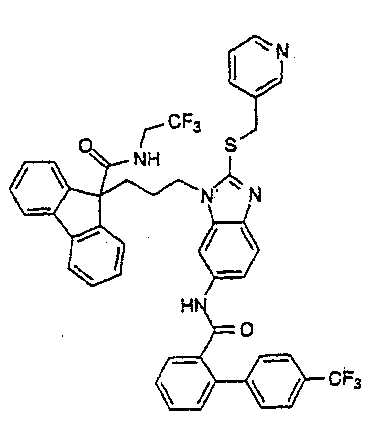 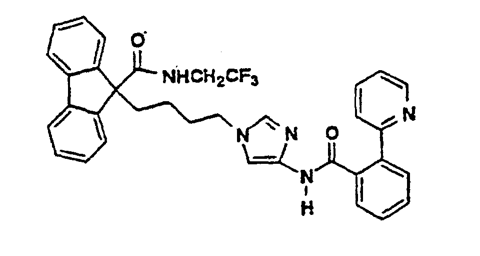 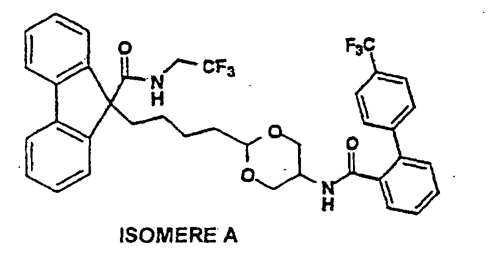 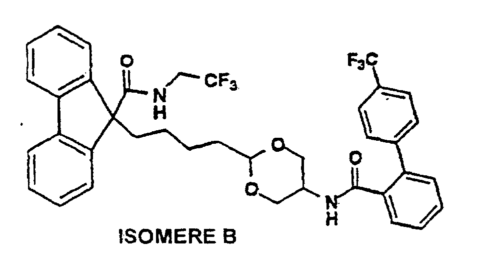 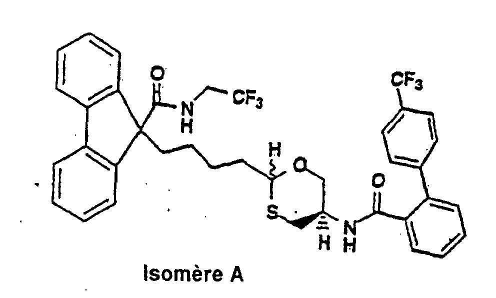 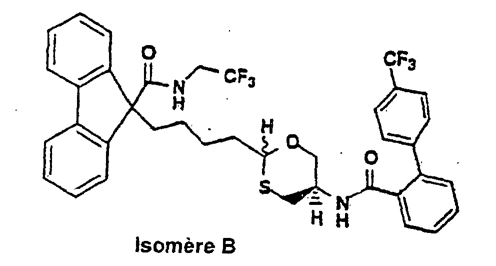 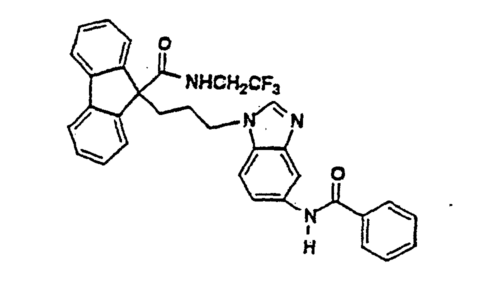 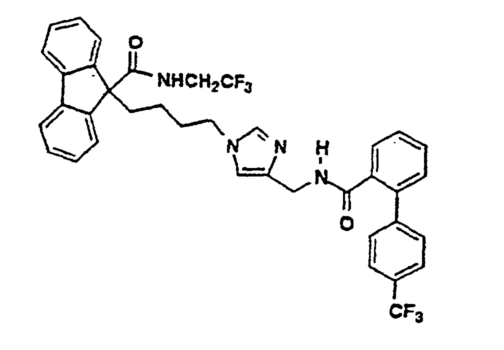 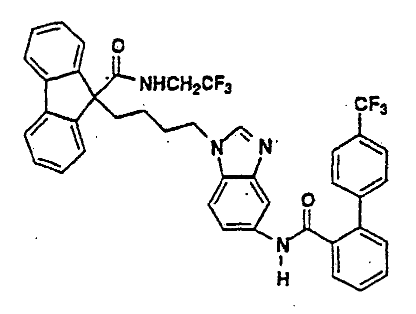 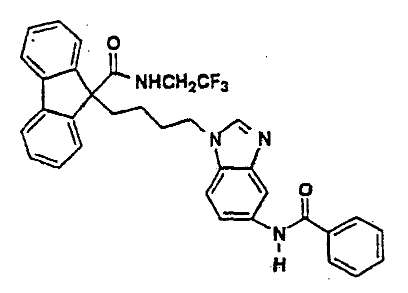 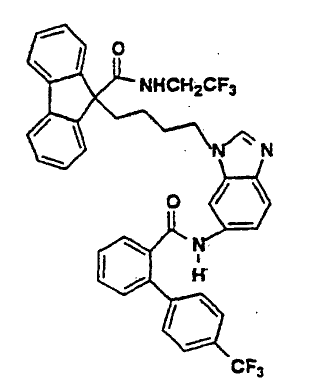 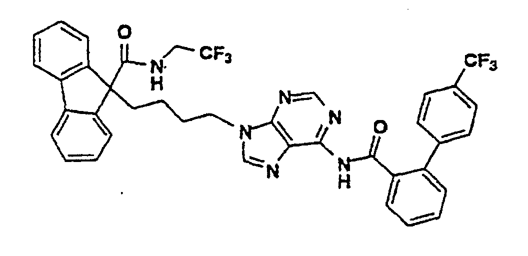 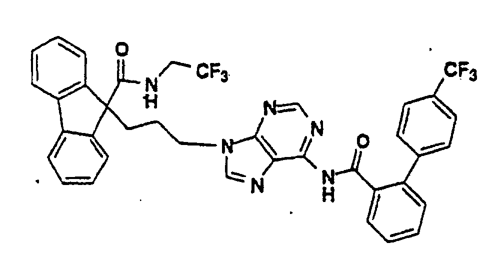 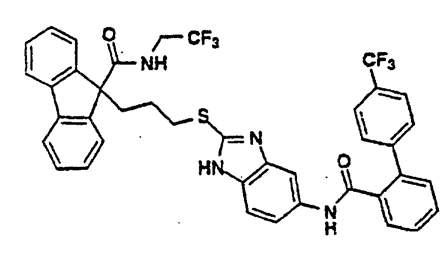 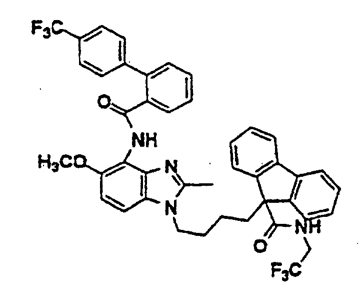 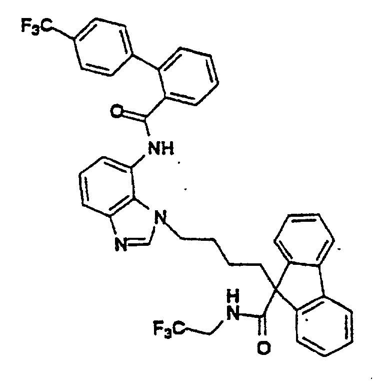 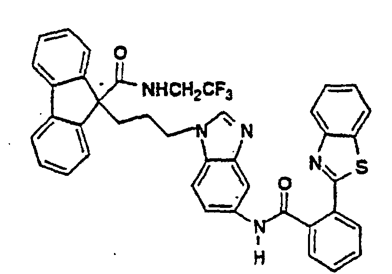 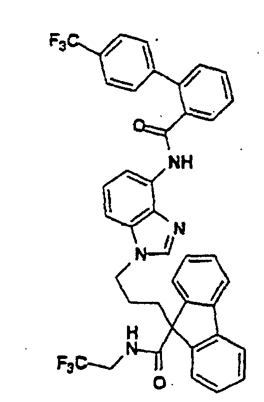 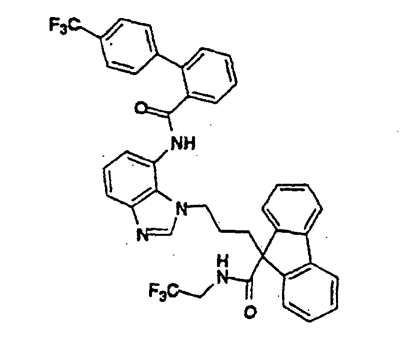 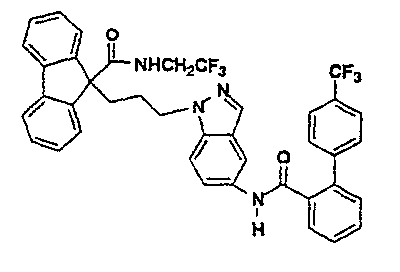 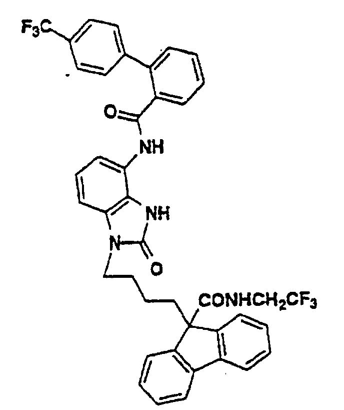 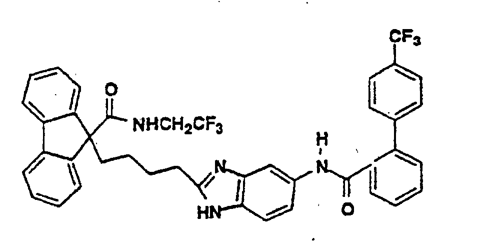 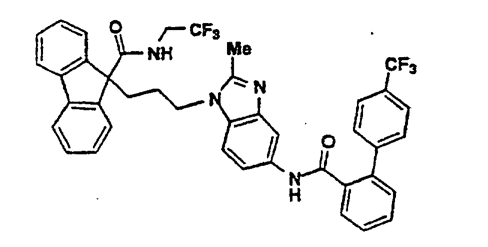 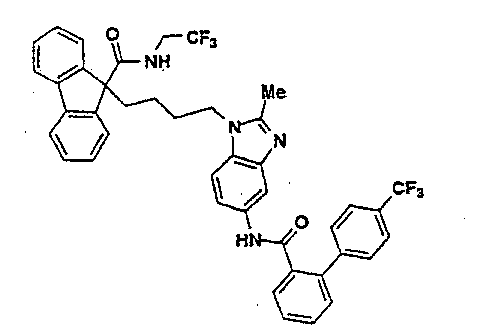 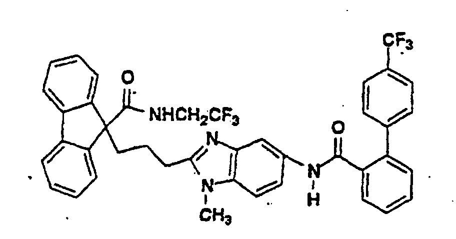 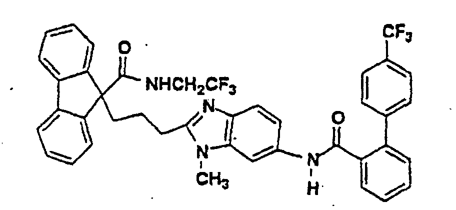 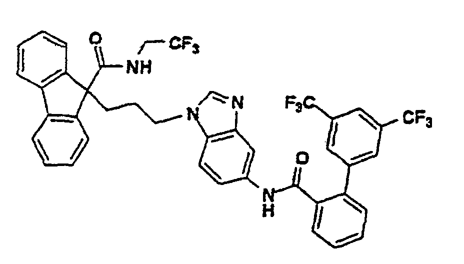 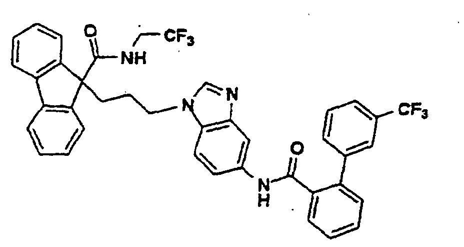 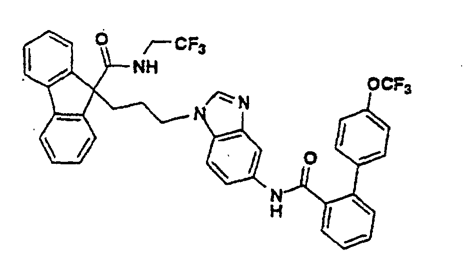 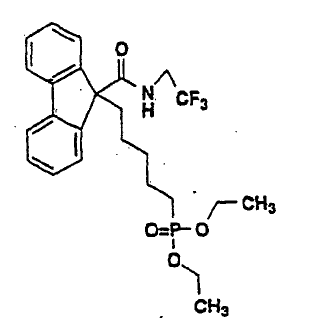 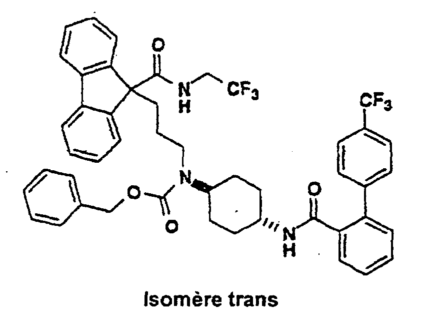 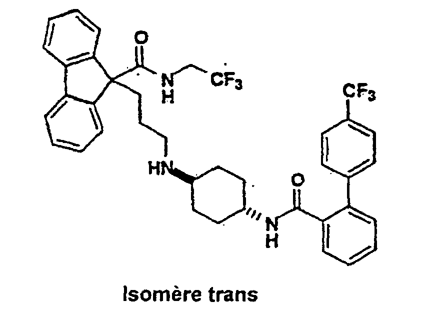 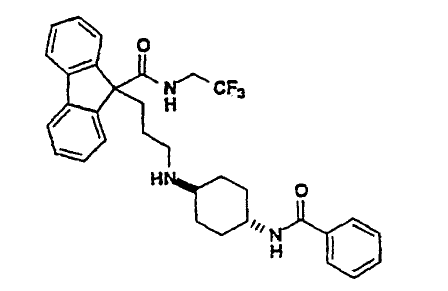 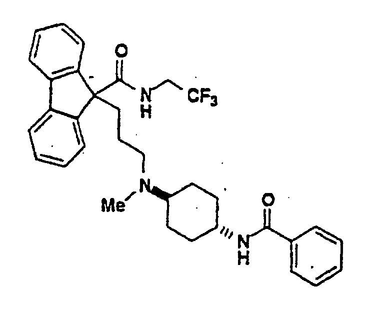 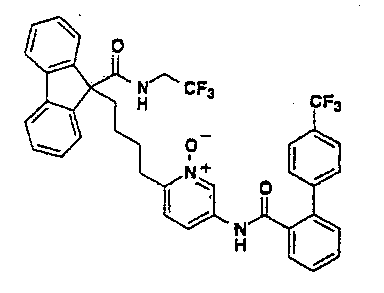 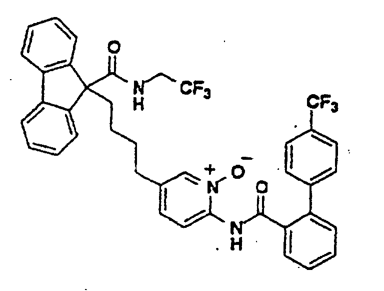 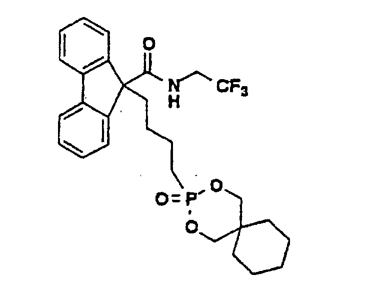 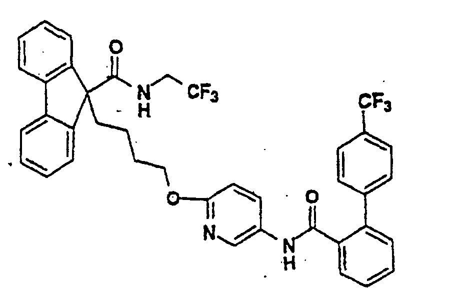 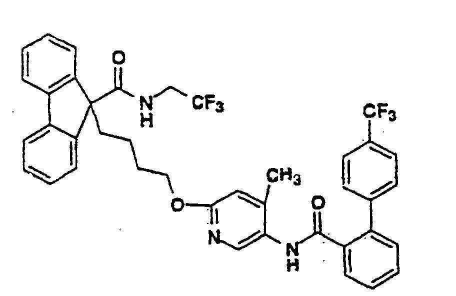 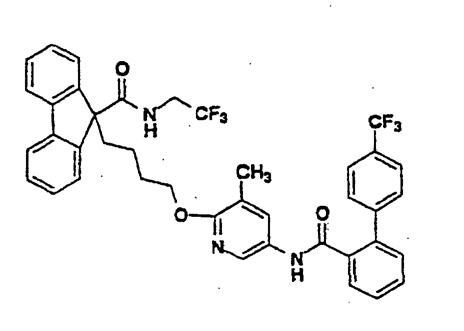 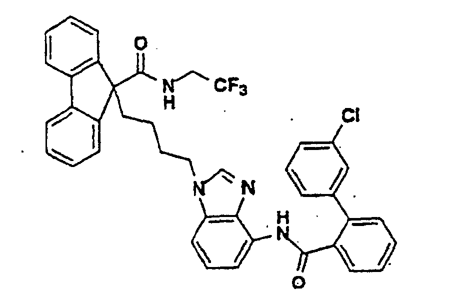 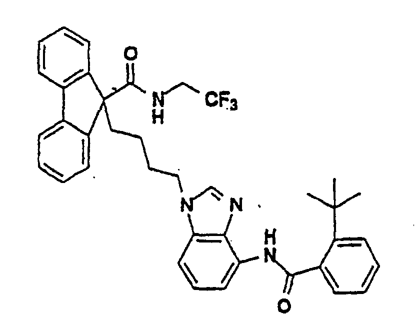 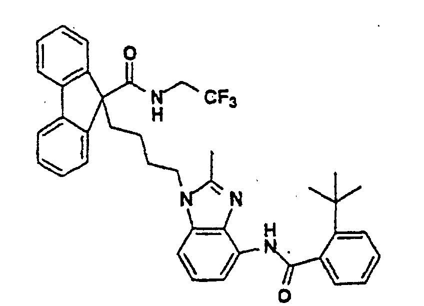 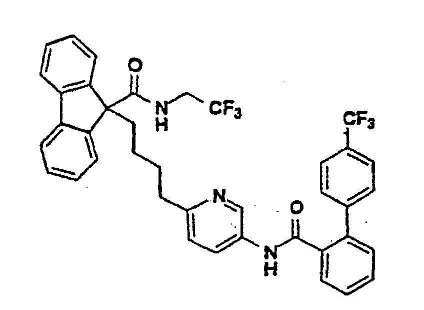 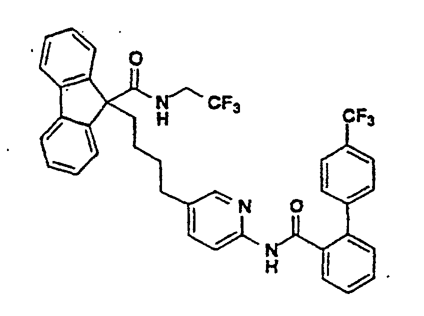 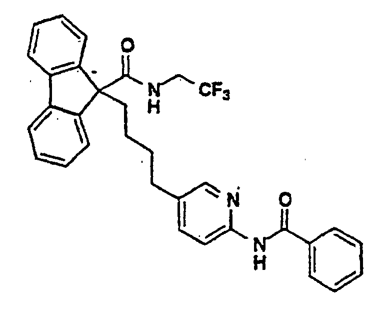 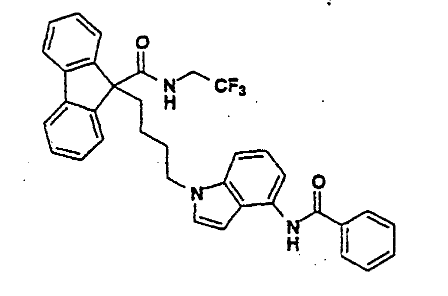 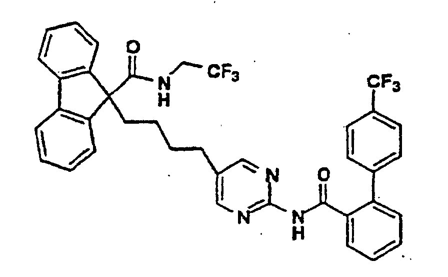 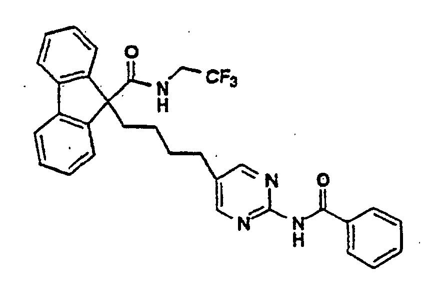 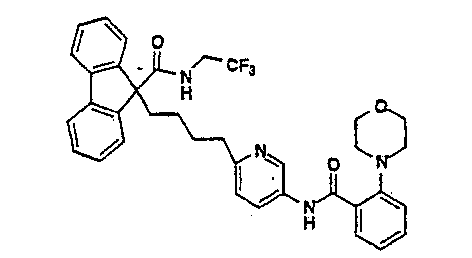 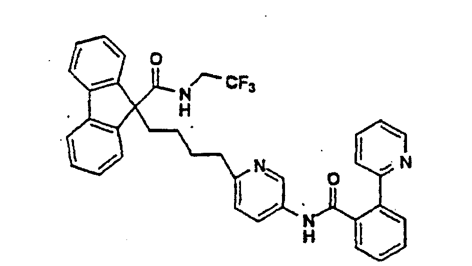 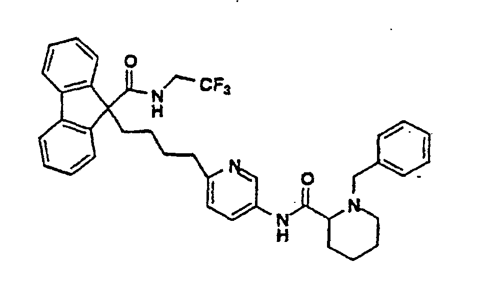 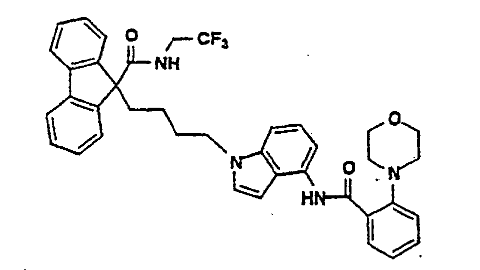 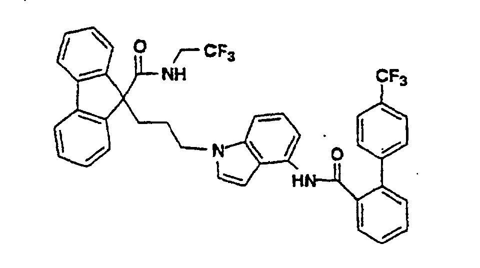 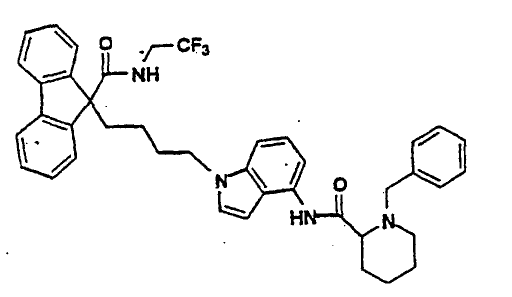 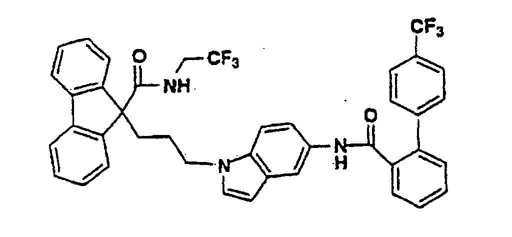 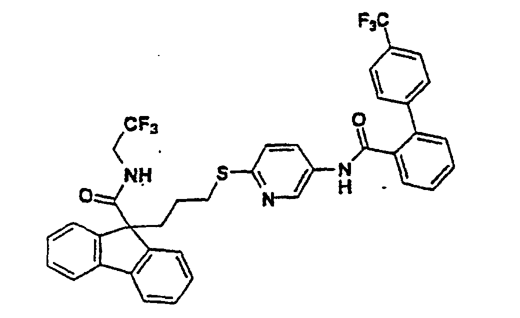 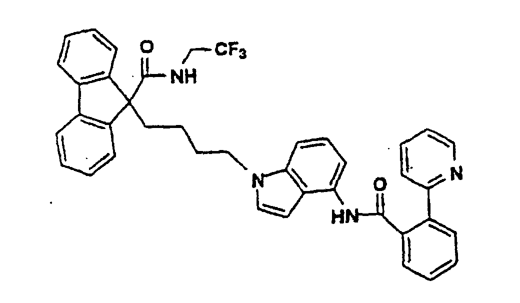 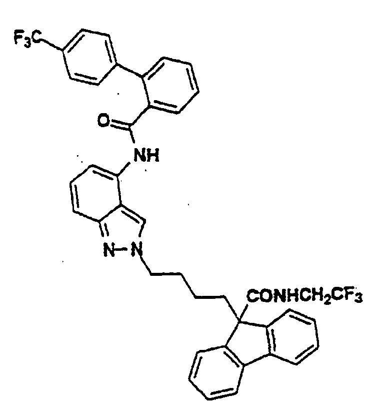 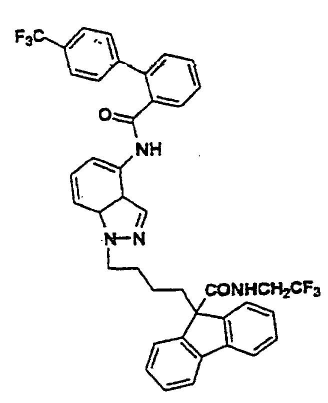 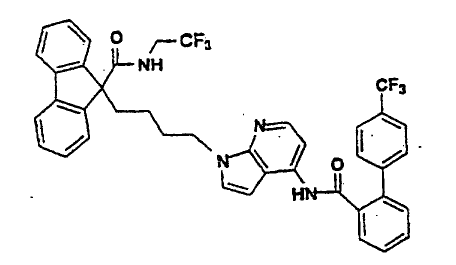 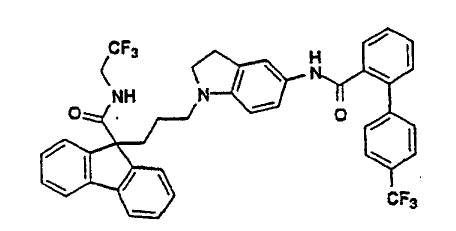 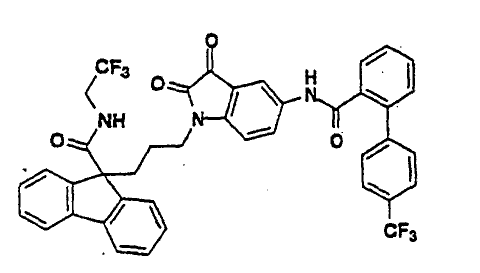 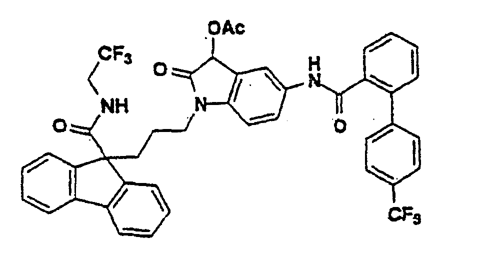 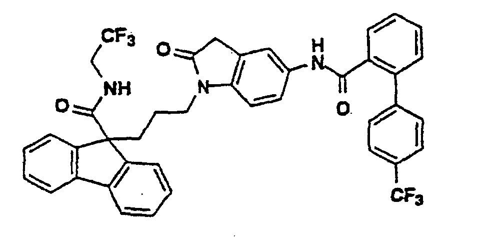 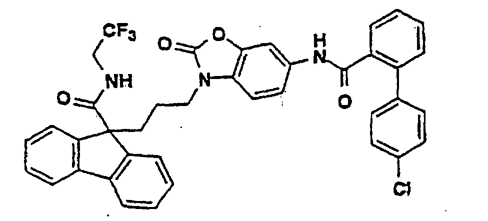 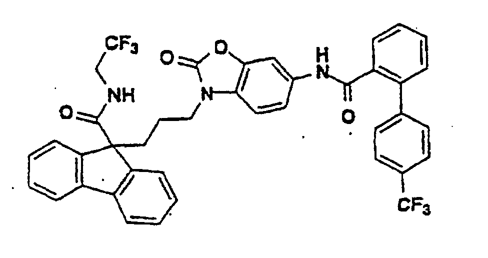 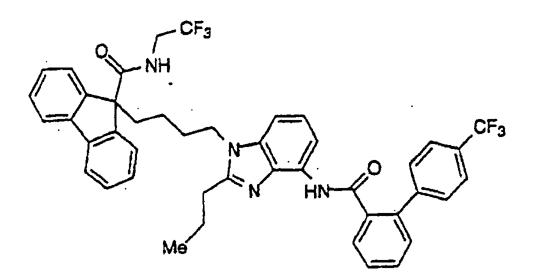 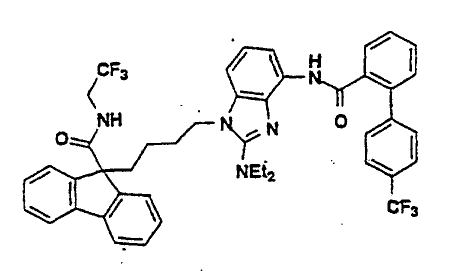 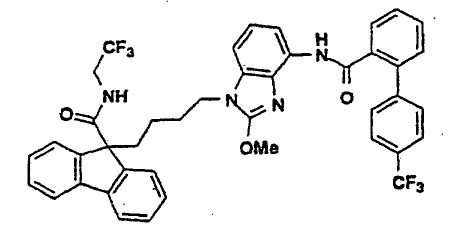 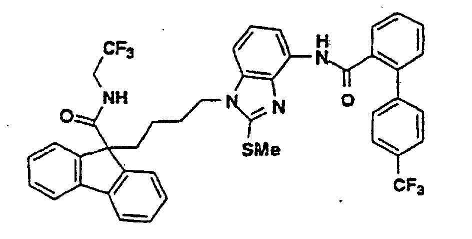 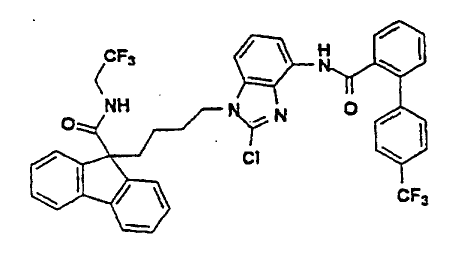 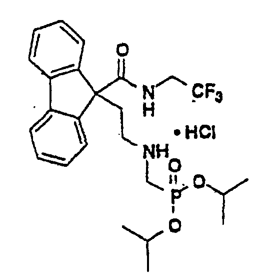 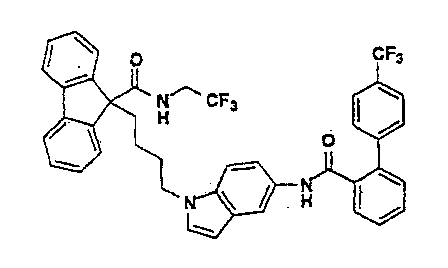 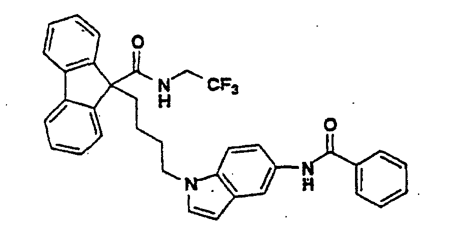 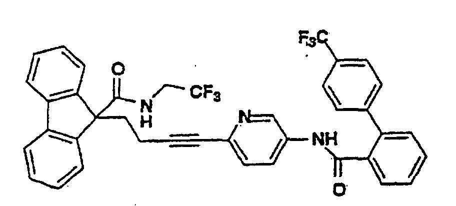 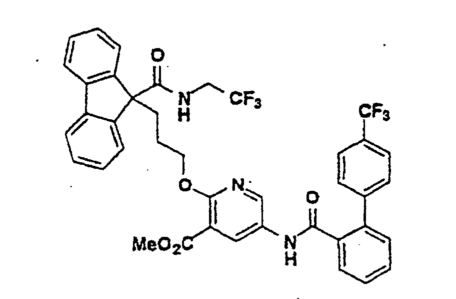 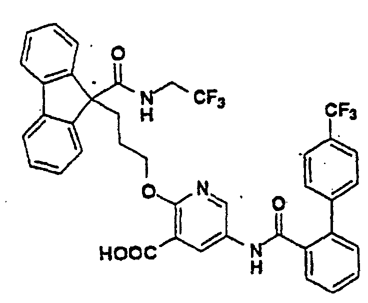 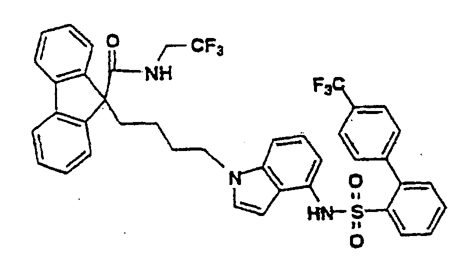 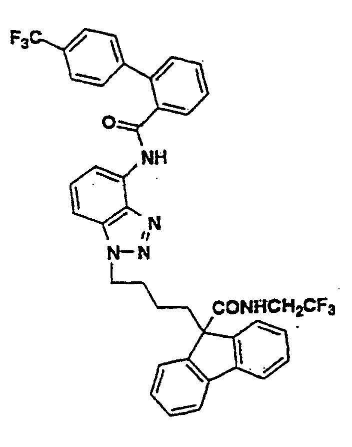 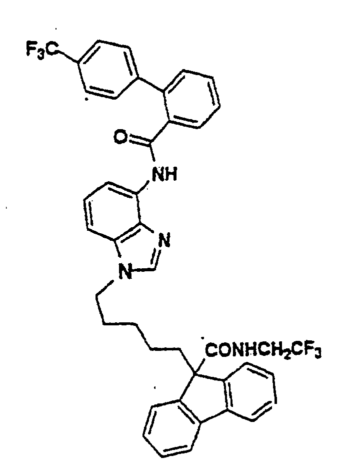 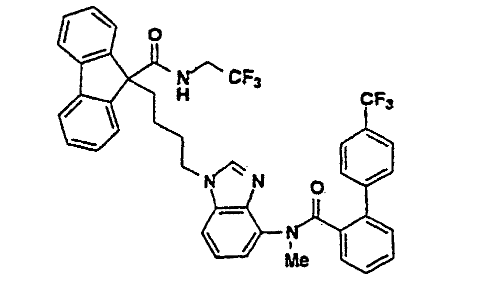 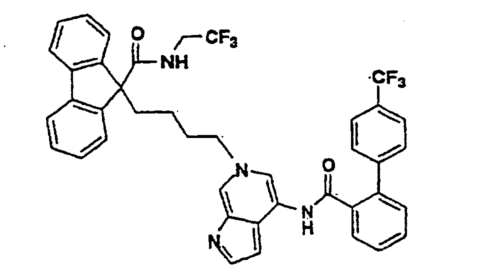 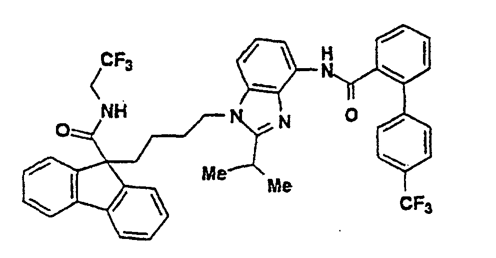 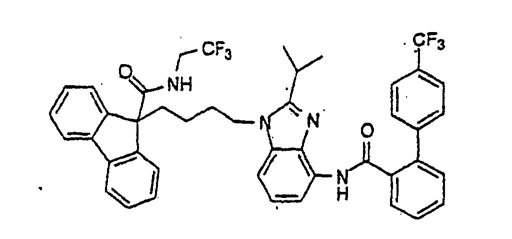 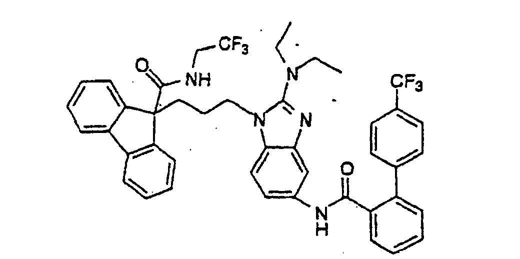 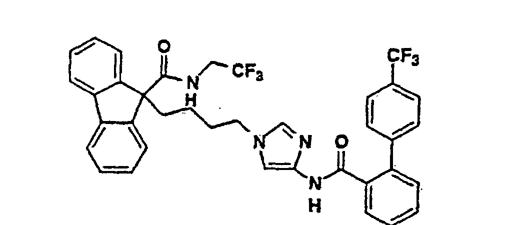 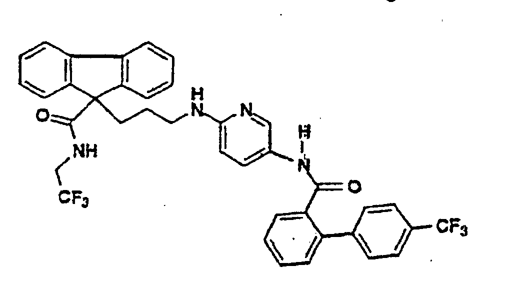 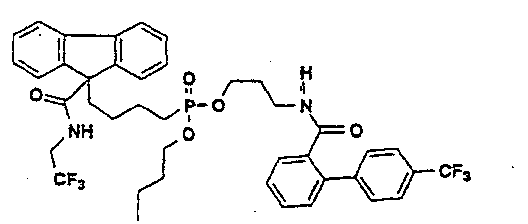 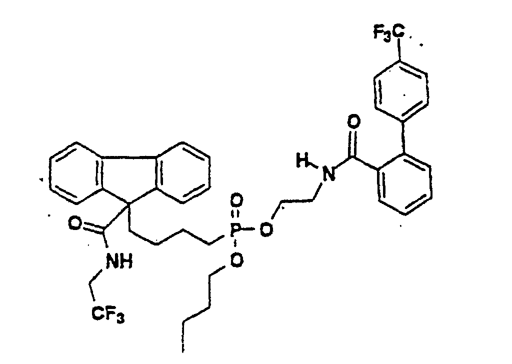 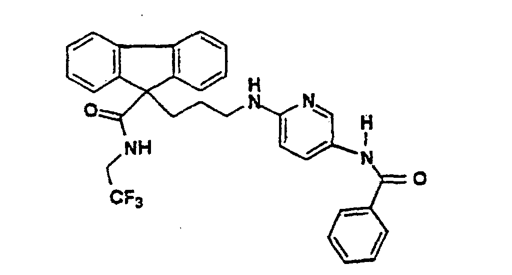 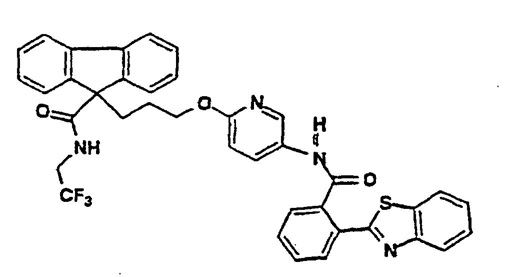 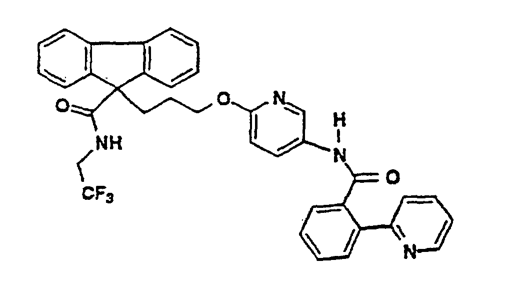 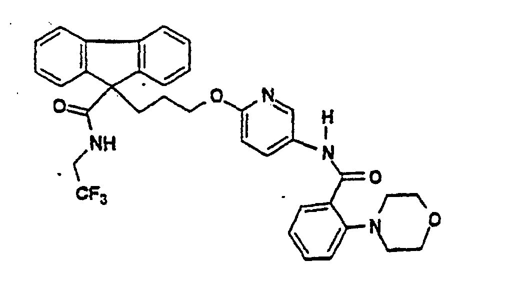 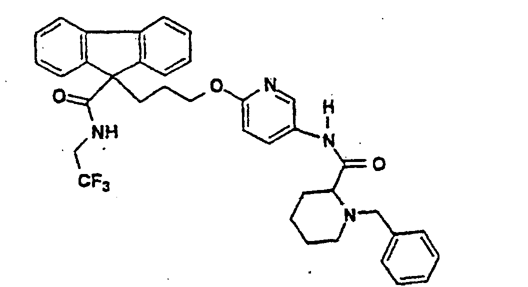 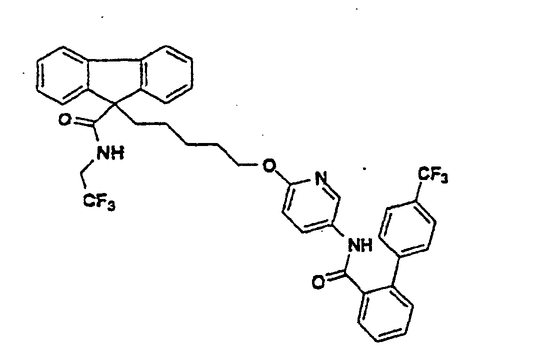 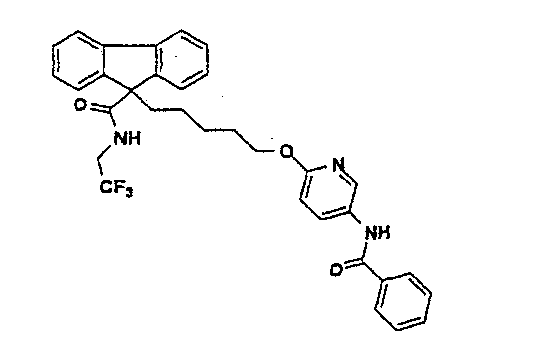 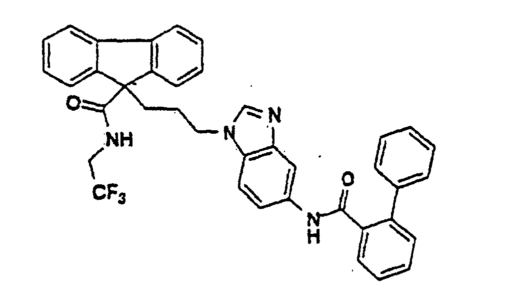 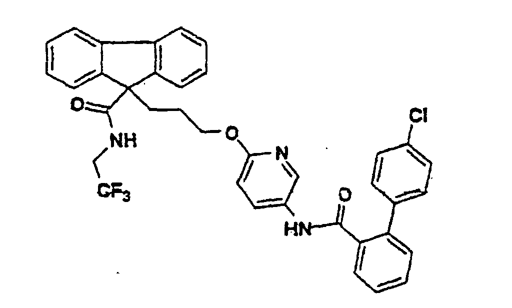 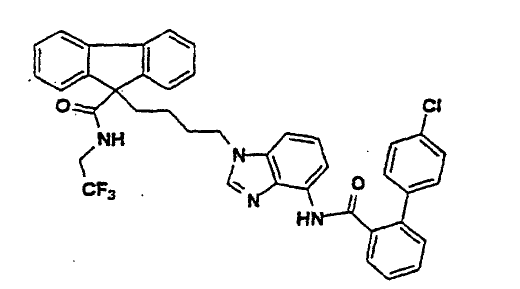 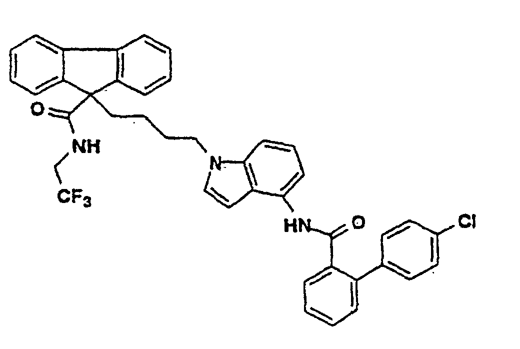 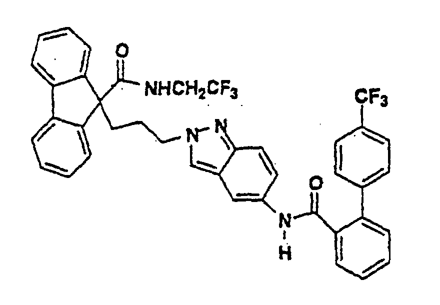 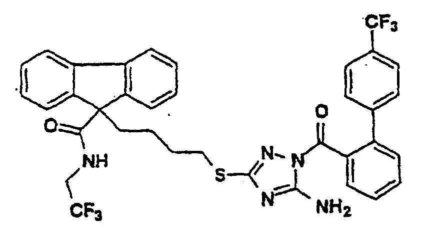 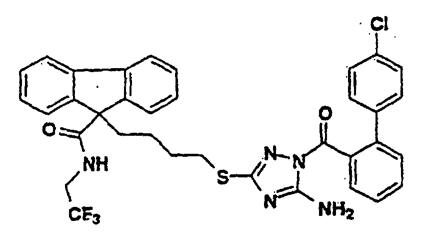 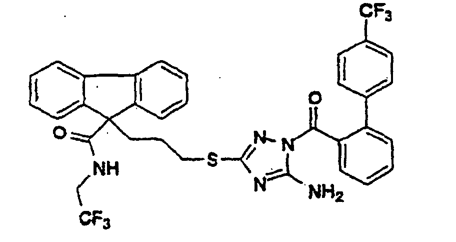 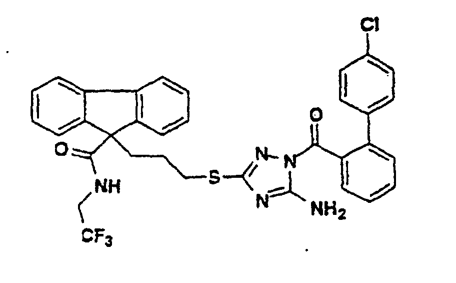 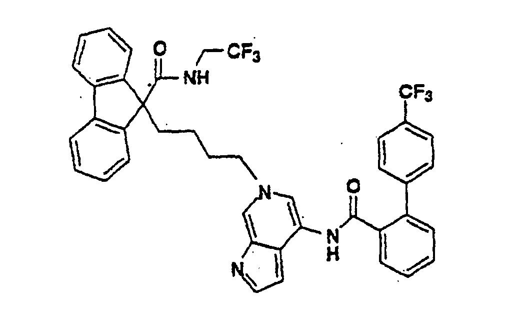 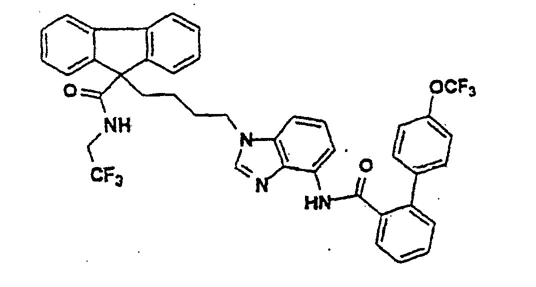 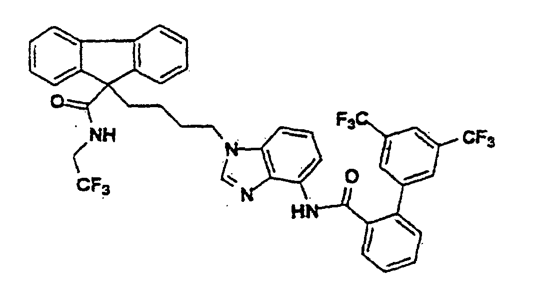 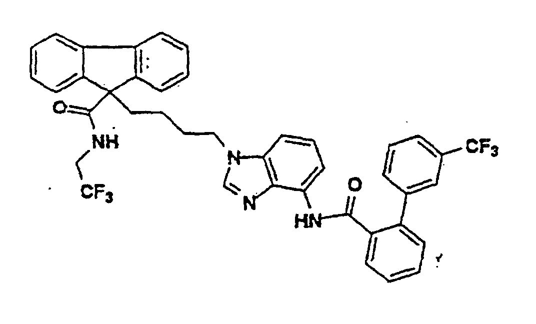 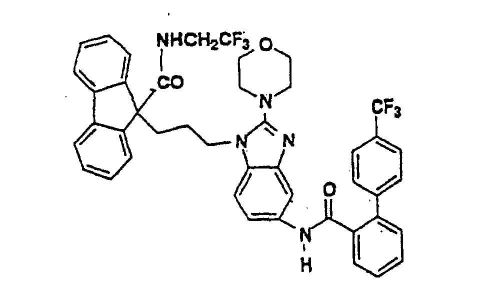 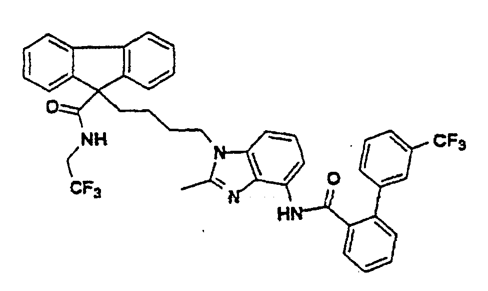 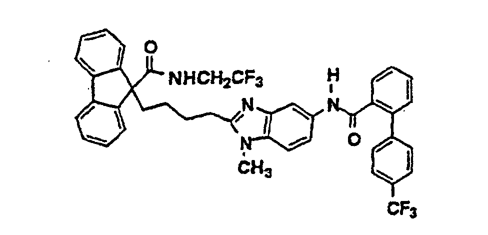 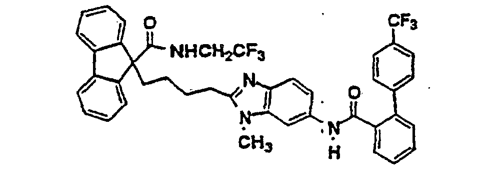 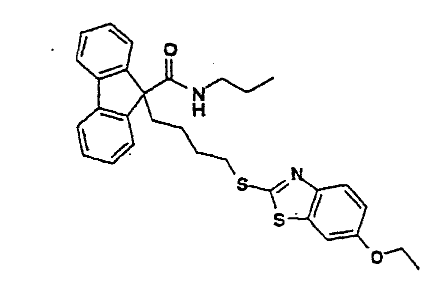 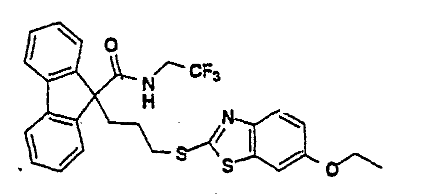 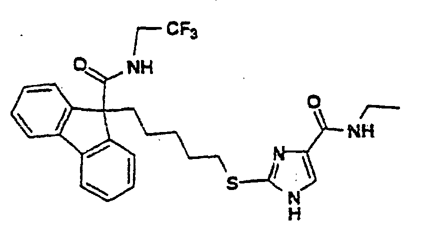 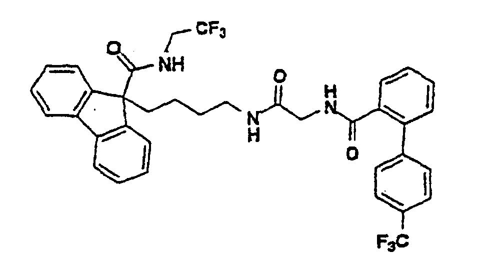 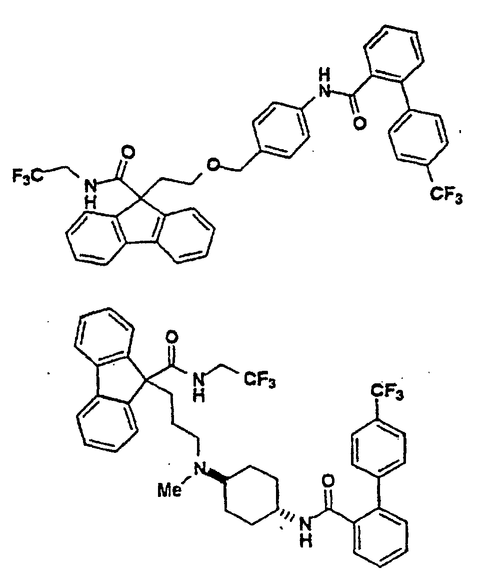 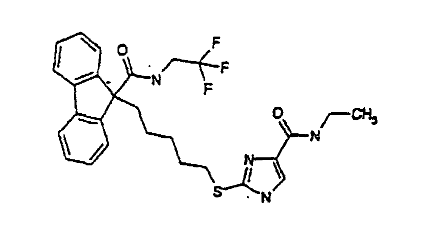 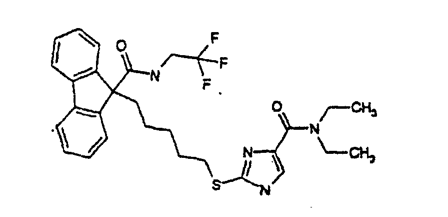 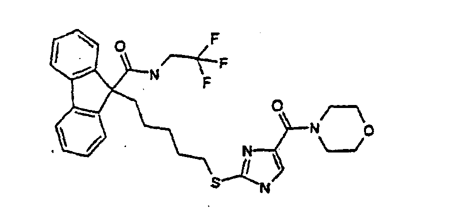 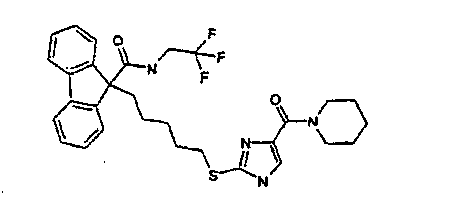 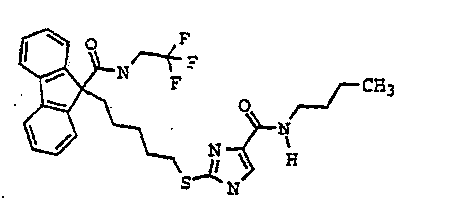 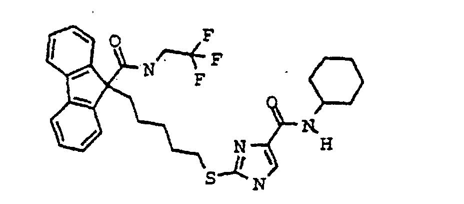 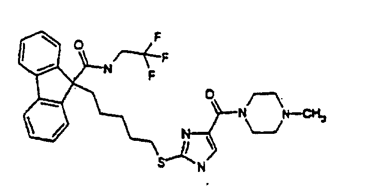 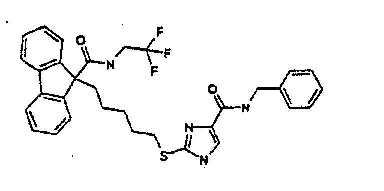 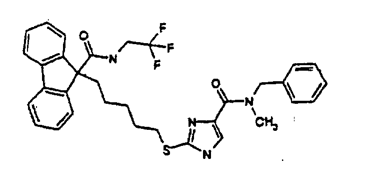 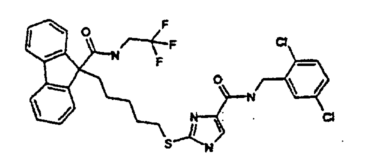 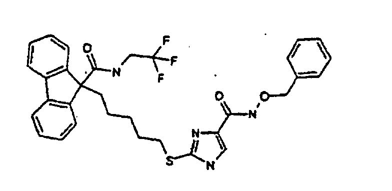 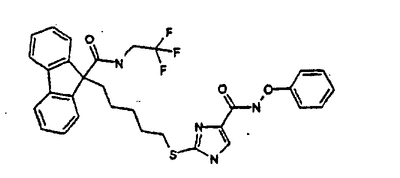 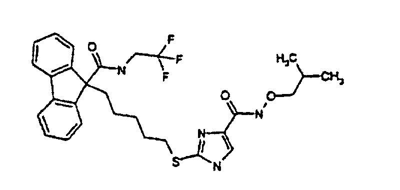 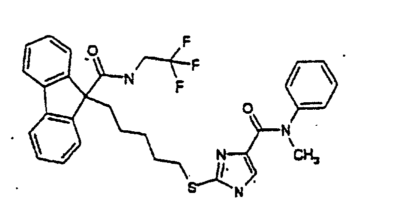 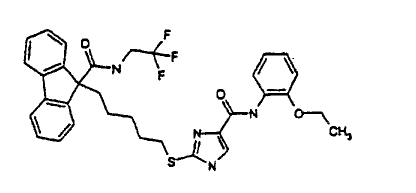 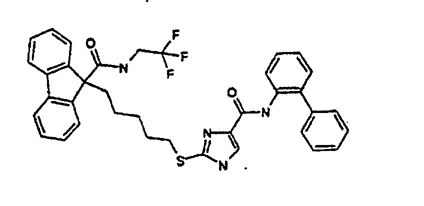 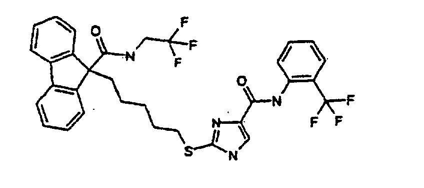 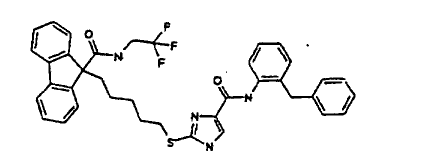 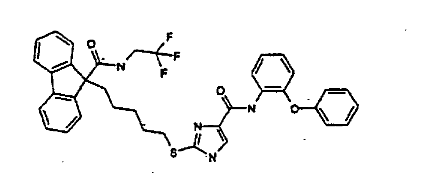 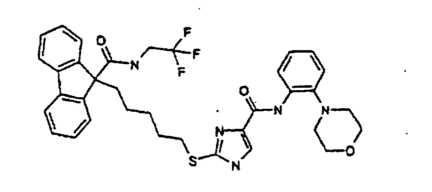 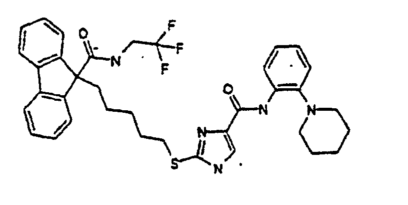 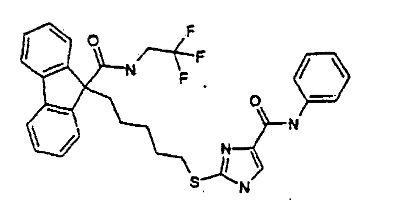 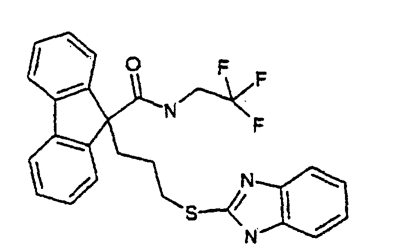 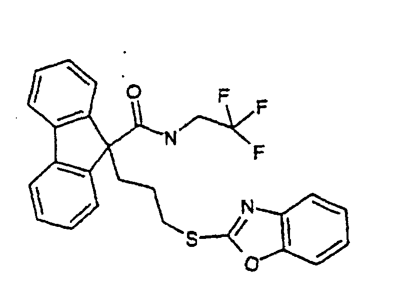 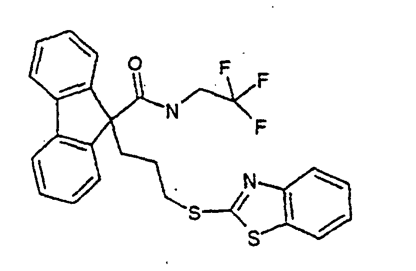 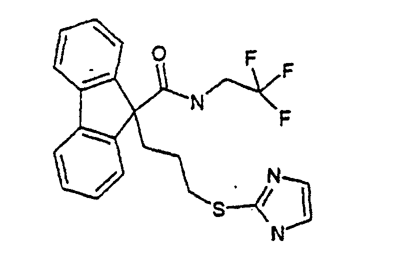 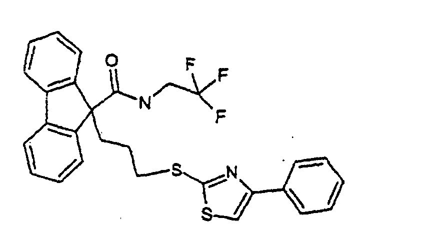 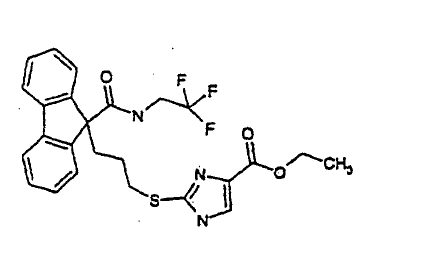 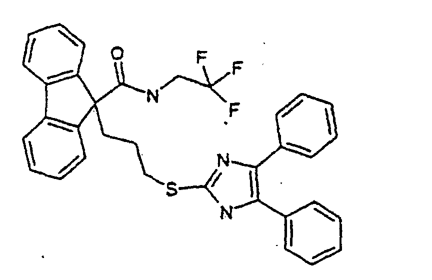 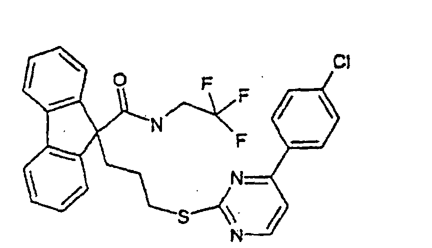 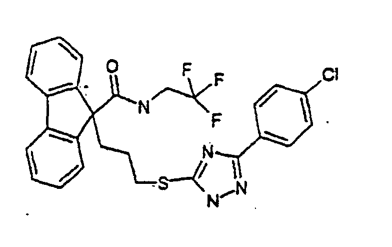 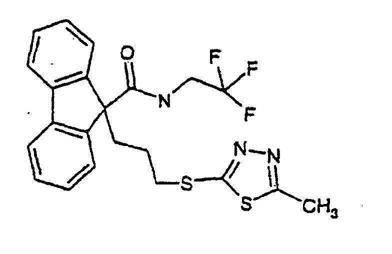 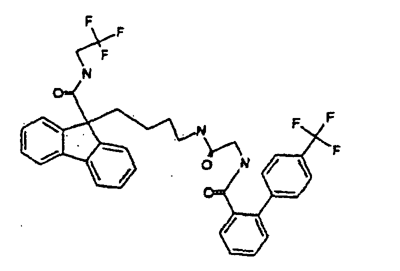 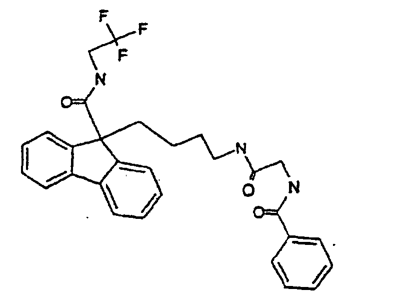 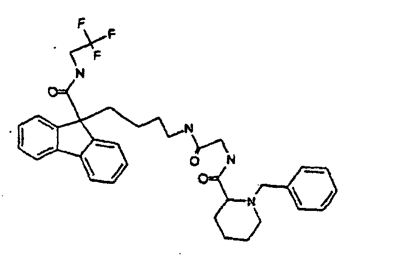 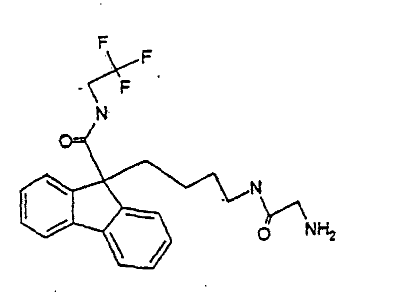 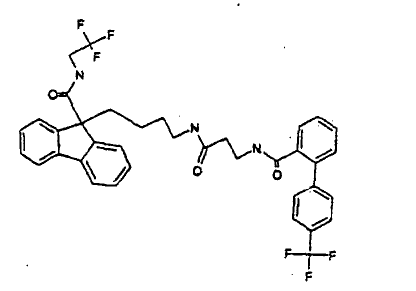 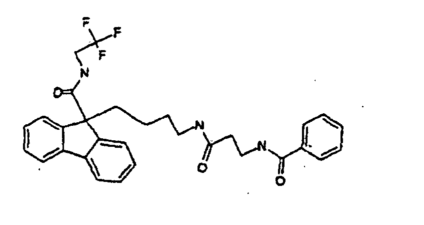 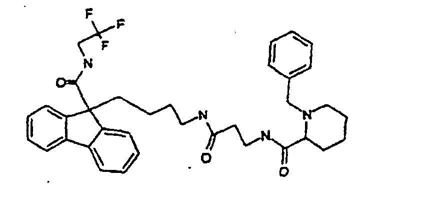 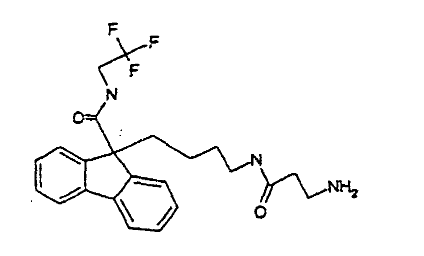 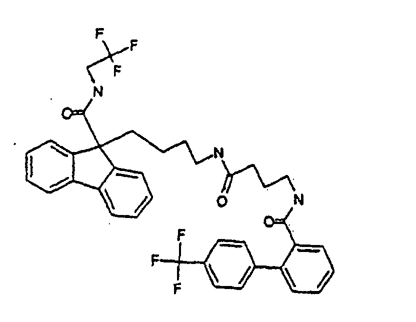 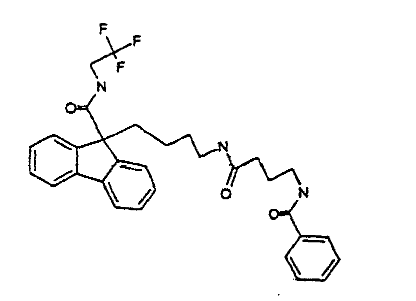 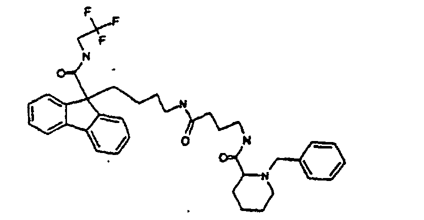 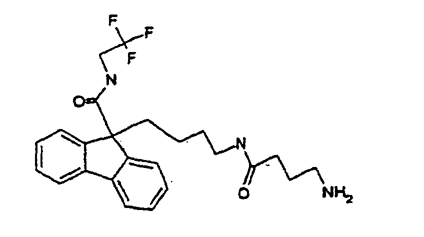 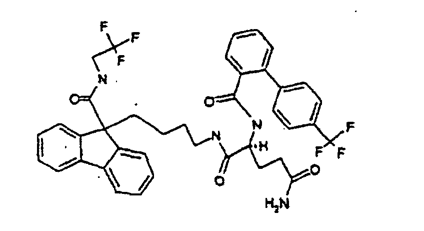 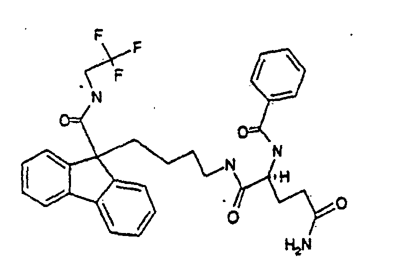 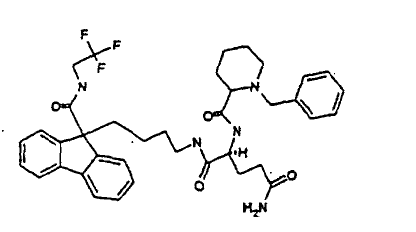 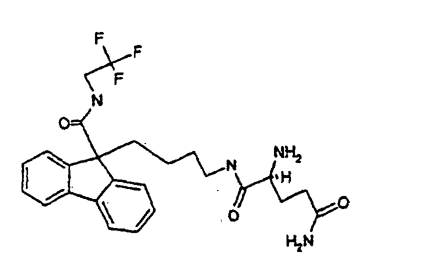 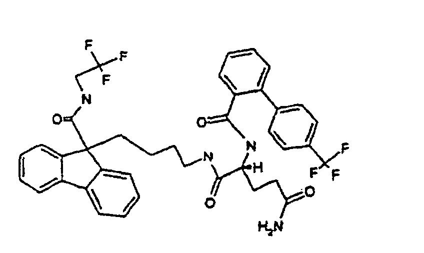 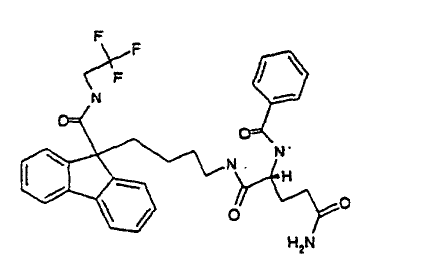 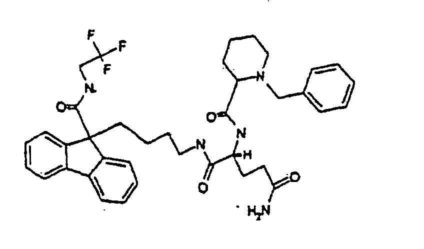 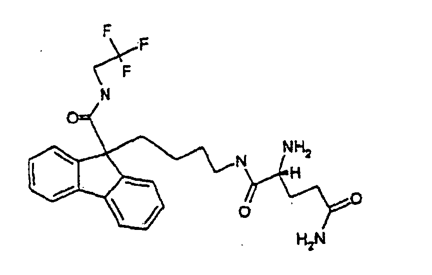 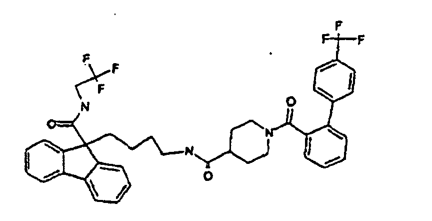 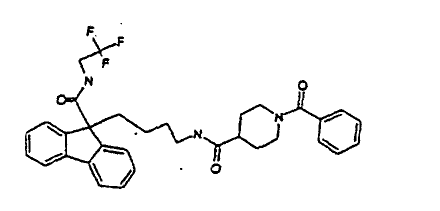 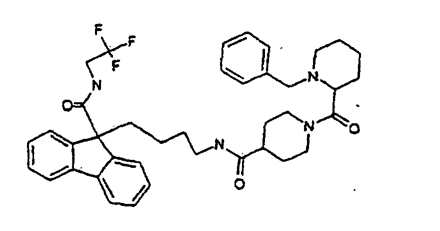 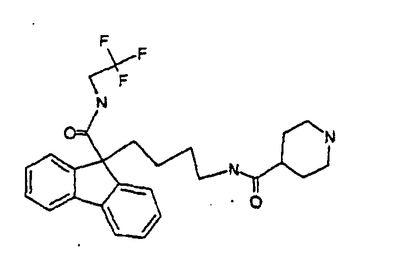 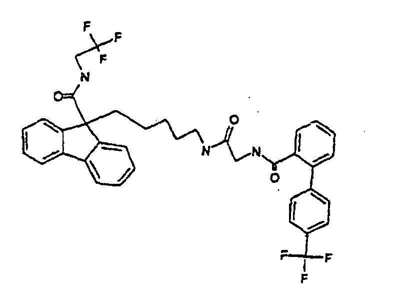 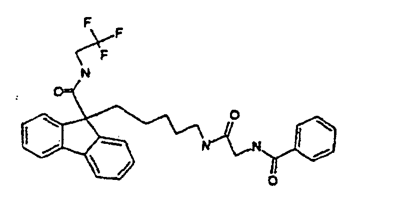 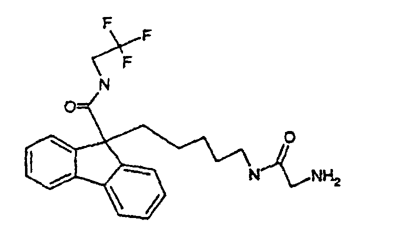 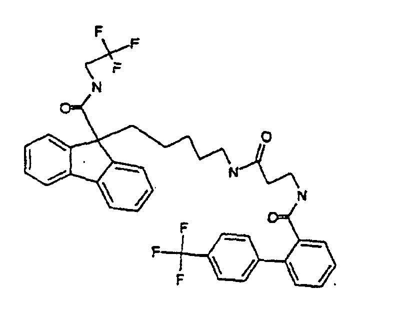 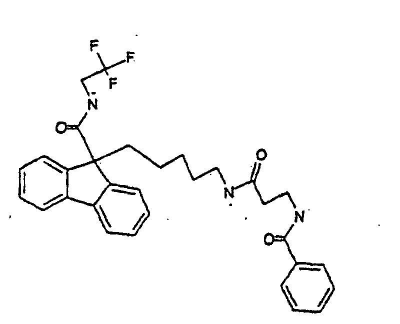 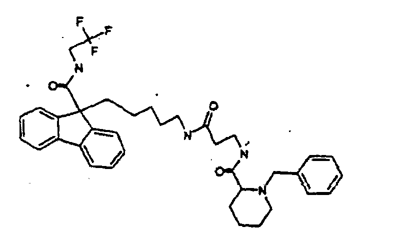 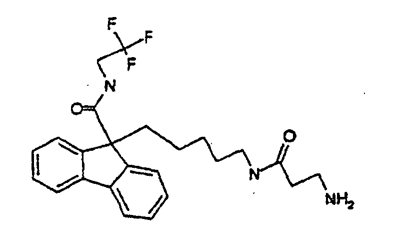 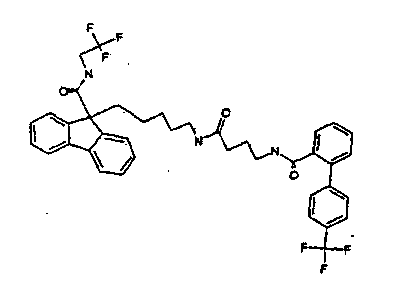 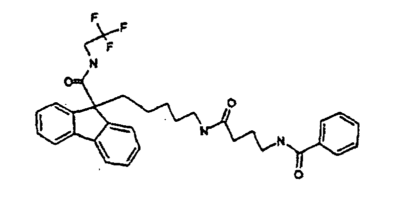 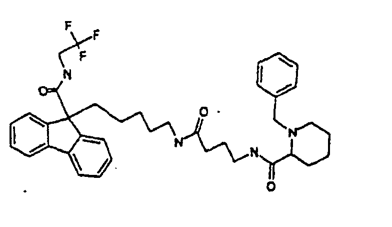 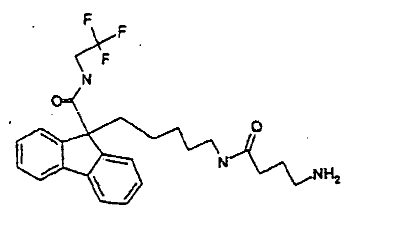 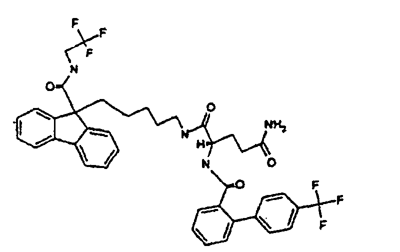 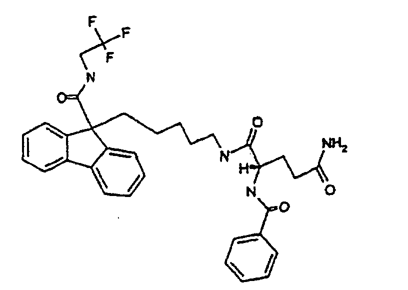 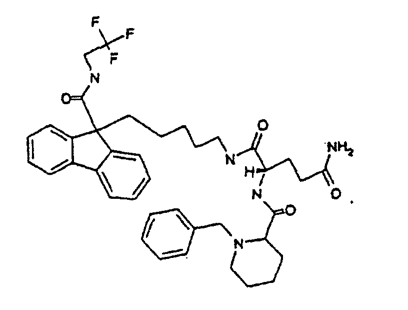 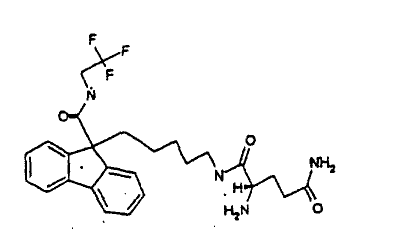 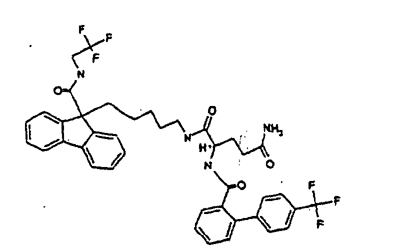 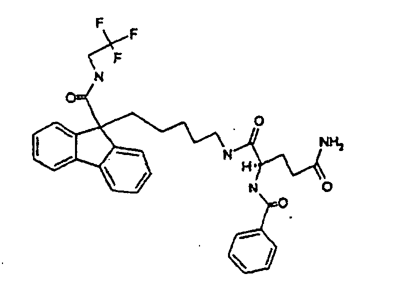 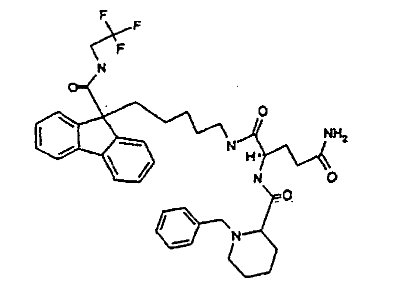 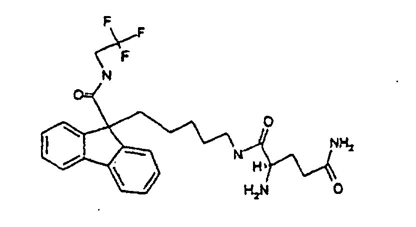 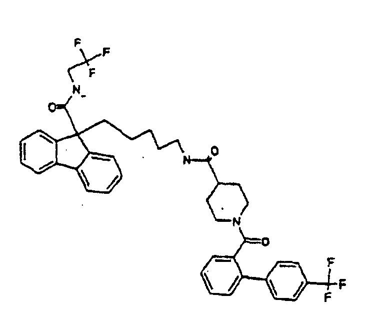 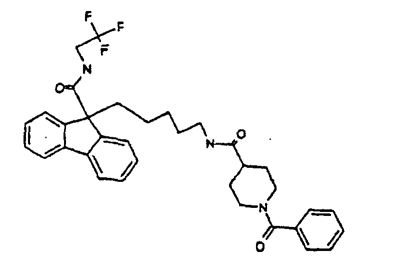 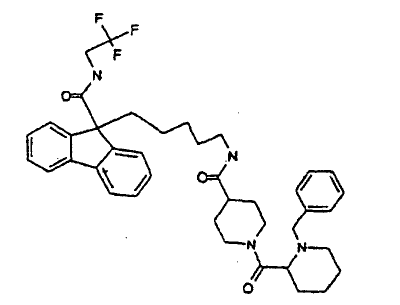 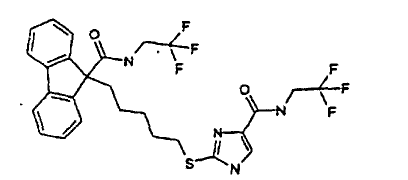 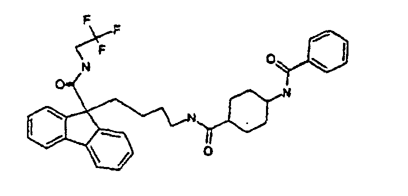 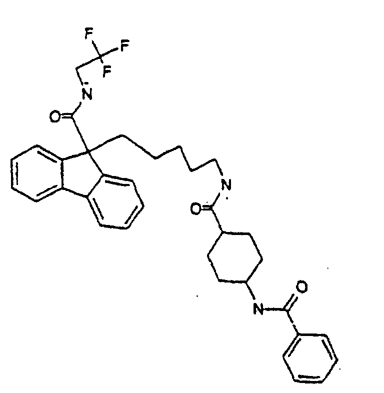 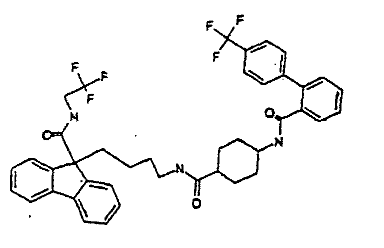 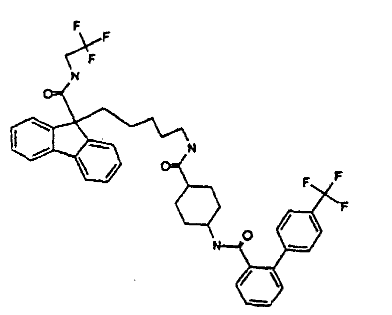 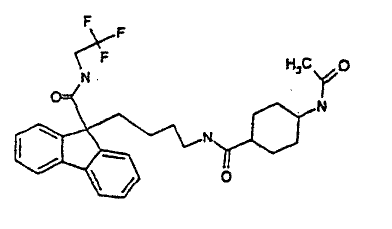 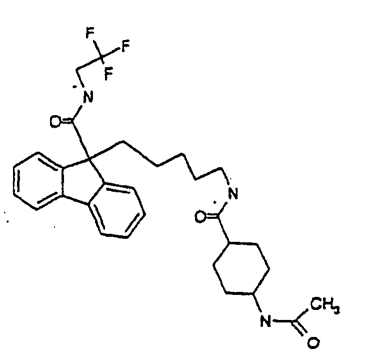 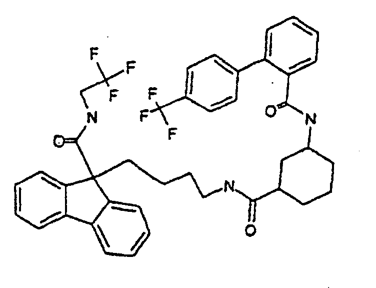 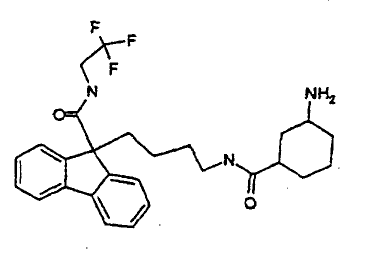 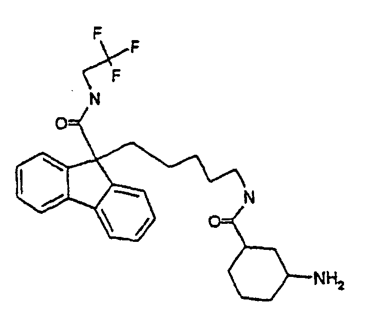 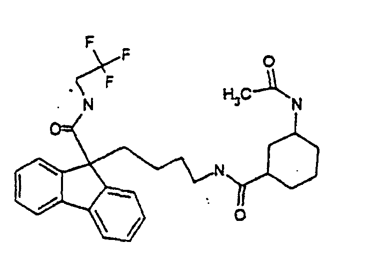 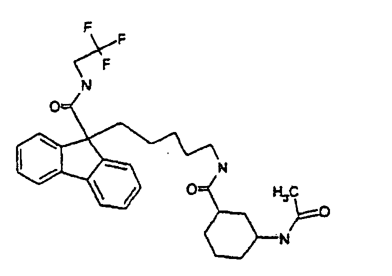 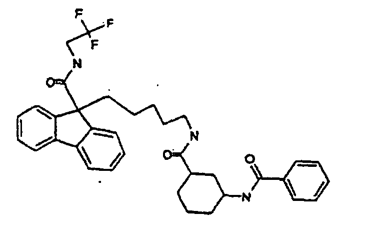 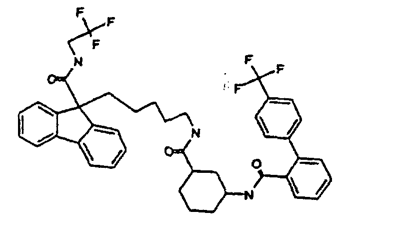 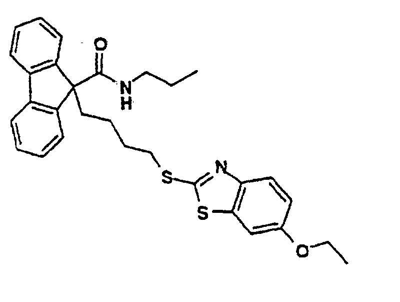 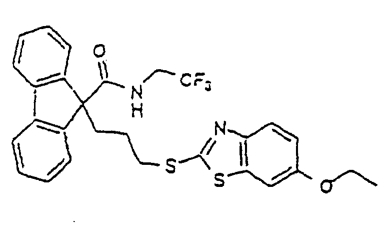 ou
les sels pharmaceutiquement acceptables de ces composés; les esters ou les esters promédicaments (« prodrugs ») de ces composés.

4. Composé selon la revendication 2, dans lequel A est NH et R²L² est CF₃CH₂.

5. Composé selon la revendication 1 ayant pour formule 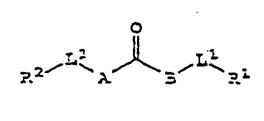 dans laquelle B est 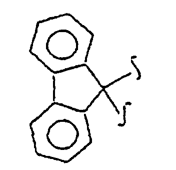 A est NH
L²R² est CH₂CF₃
L¹ est -CH₂CH₂CH₂- ou -CH₂CH₂CH₂CH₂-, et
R¹ est un hétéroaryle qui est un cycle aromatique à 5 chaînes qui comprend 2 atomes d'azote, lequel cycle est fusionné à un cycle aryle et est substitué sur la partie aryle.

6. Composé selon la revendication 1 dans lequel R¹ est

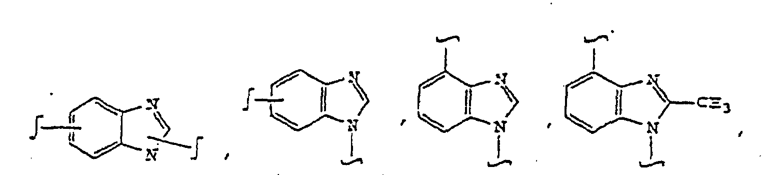

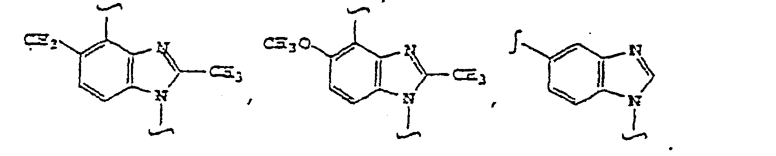

7. Composé selon la revendication 1 dans lequel R¹ est

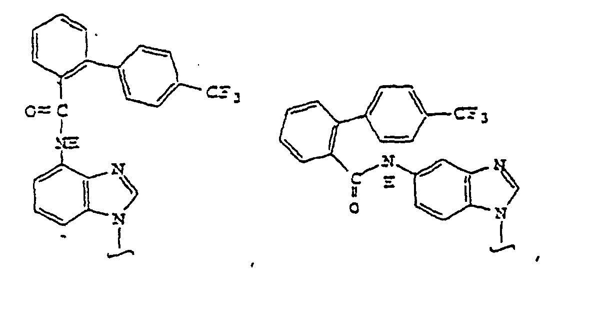

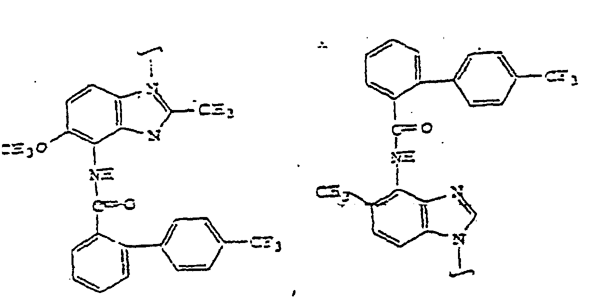

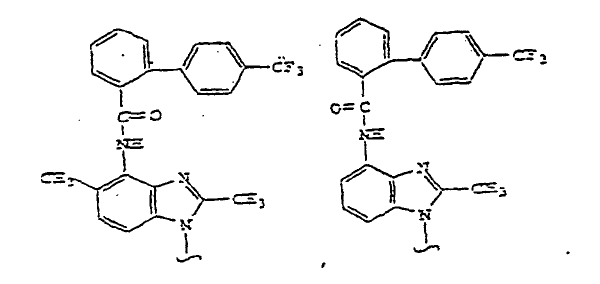

8. Composé selon la revendication 5 ayant pour structures 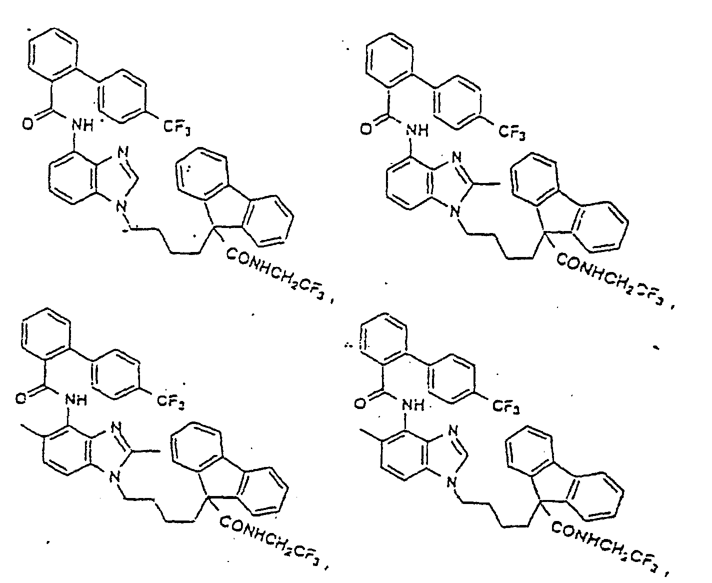 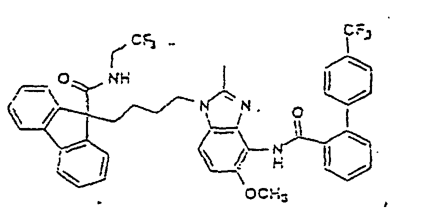 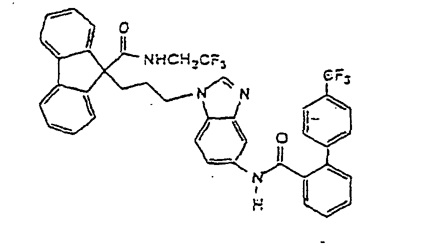 ,ou un sel pharmaceutiquement acceptable de ces structures.

9. Composition pharmaceutique comprenant un composé selon l'une quelconque des revendications 1 à 8 et un véhicule pharmaceutiquement acceptable.

10. Utilisation d'un composé selon l'une quelconque des revendications 1 à 8 pour la préparation d'une composition pharmaceutique pour réduire l'activité d'une protéine microsomique de transfert des triglycérides (MTP) chez une espèce mammifère.

11. Utilisation d'un composé selon l'une quelconque des revendications 1 à 8 pour la préparation d'une composition pharmaceutique pour réduire les taux de lipides sériques, de cholestérol et/ou de triglycérides, ou inhiber et/ou traiter l'hyperlipémie, l'hyperlipidémie, l'hyperlipoprotéinémie, l'hypercholestérolémie, l'hyperglycémie et/ou l'hvpertriglycéridémie et/ou empêcher, inhiber ou traiter l'athérosclérose, la pancréatite, les diabètes non insulinodépendants ou l'obésité chez une espèce mammifère.
